(19) Europäisches Patentamt
European Patent Office
Office européen des brevets

(11) EP 2 520 570 A1

(12) **EUROPEAN PATENT APPLICATION**
published in accordance with Art. 153(4) EPC

(43) Date of publication:
**07.11.2012 Bulletin 2012/45**

(21) Application number: **10839533.6**

(22) Date of filing: **24.12.2010**

(51) Int Cl.:
*C07D 213/74* (2006.01) *A61K 31/44* (2006.01)
*A61K 31/4427* (2006.01) *A61K 31/4436* (2006.01)
*A61K 31/4439* (2006.01) *A61K 31/444* (2006.01)
*A61P 11/00* (2006.01) *A61P 11/06* (2006.01)
*A61P 11/08* (2006.01) *A61P 27/06* (2006.01)
*A61P 43/00* (2006.01) *C07D 401/12* (2006.01)
*C07D 405/12* (2006.01) *C07D 409/12* (2006.01)
*C07D 409/14* (2006.01) *C07D 417/12* (2006.01)

(86) International application number:
**PCT/JP2010/073274**

(87) International publication number:
**WO 2011/078303 (30.06.2011 Gazette 2011/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.12.2009 JP 2009294744**
**25.12.2009 JP 2009294745**
**29.10.2010 JP 2010242885**

(71) Applicant: **Ube Industries, Ltd.**
**Ube-shi**
**Yamaguchi 755-8633 (JP)**

(72) Inventors:
- **IWAMURA, Ryo**
  **Ube-shi,**
  **Yamaguchi (JP)**
- **TANAKA, Masayuki**
  **Ube-shi**
  **Yamaguchi (JP)**
- **KATSUBE, Tetsushi**
  **Ube-shi,**
  **Yamaguchi (JP)**
- **SHIBAKAWA, Nobuhiko**
  **Ube-shi**
  **Yamaguchi (JP)**
- **SHIGETOMI, Manabu**
  **Ube-shi**
  **Yamaguchi (JP)**
- **OKANARI, Eiji**
  **Ube-shi**
  **Yamaguchi (JP)**
- **KANDA, Tomoko**
  **Ube-shi**
  **Yamaguchi (JP)**
- **TOKUNAGA, Yasunori**
  **Ube-shi,**
  **Yamaguchi (JP)**
- **FUJIWARA, Hiroshi**
  **Ube-shi,**
  **Yamaguchi (JP)**

(74) Representative: **HOFFMANN EITLE**
**Patent- und Rechtsanwälte**
**Arabellastrasse 4**
**81925 München (DE)**

# (54) AMINOPYRIDINE COMPOUND

(57) The present invention relates to a compound represented by the formula (I):

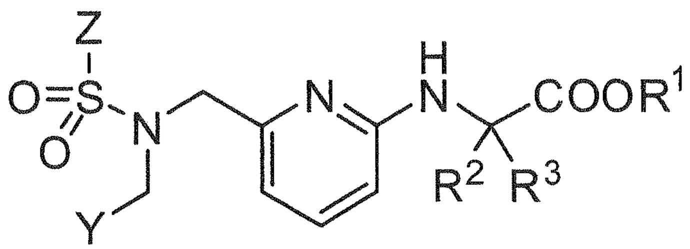

(I)

EP 2 520 570 A1

or a pharmaceutically acceptable salt thereof, a medical composition containing the same, and a medical composition for the treatment or prophylaxis of respiratory diseases or glaucoma.

**Description**

TECHNICAL FIELD

The present invention relates to a novel aminopyridine compound or a pharmaceutically acceptable salt thereof useful as a medicine. More specifically, the aminopyridine compound according to the present invention has an EP2 agonistic action so that it is useful as a therapeutic and/or prophylactic agent against, for example, respiratory diseases such as asthma or chronic obstructive pulmonary disease (hereinafter abbreviated to as COPD), etc., or eye diseases (in particular, glaucoma).

BACKGROUND ART

Prostaglandin $E_2$ (hereinafter abbreviated as $PGE_2$), which is administered by inhalation, has been reported to inhibit immediate-type and late-type asthmatic responses in asthma patients (see Non-Patent Literature 1). In addition, PGE2 is known to act as an agonist against receptors such as EP1, EP2, EP3 and EP4, and its agonistic action against an EP2 receptor in particular has been suggested to be intimately involved with bronchodilatory action (see Non-Patent Literature 2).

On the other hand, glaucoma is an eye disease which is eye function disorder characterized in that aqueous humor is accumulated due to circulatory disorder of aqueous humor, an intraocular pressure is continuously increased, optic nerve is pressed, whereby it causes temporal or permanent visual field defect or low vision, and a cause thereof is said to be an increase of an intraocular pressure. In human eye tissues, the above-mentioned 4 receptors are expressed together (see Non-Patent Literatures 3 and 4), but each role has not yet been clarified. However, it has been clarified that an EP2 agonist has ocular hypotensive effects (see Non-Patent Literature 5), and EP2 is considered to participate in at least a part of the ocular hypotensive effects of $PGE_2$. The EP2 agonist has functions not only to promote drainage of the aqueous humor through an uveoscleral pathway, but also to promote drainage of the aqueous humor through a trabecular meshwork pathway. Further, the EP2 agonist does not act on melanocytes, so that it can avoid side effects such as strengthening chromatosis in the iris, particularly in the iris with pale brown color, etc., and worsening in uveites, etc. appeared in $PGF_{2\alpha}$ derivatives (Latanoprost and isopropyl unostone) known to as a FP agonist. Accordingly, it has been desired to develop an agonist having high selectivity on EP2 receptor in the viewpoints of medical effects and safety.

Until now, a sulfonamide compound having similar structure to that of the present invention has been known to have an EP2 agonist action (see Patent Literatures 1 to 5). Among these, the compound described in Example 14e of Patent Literature 2 has been reported to increase a concentration of the cyclic adenosine monophosphate (hereinafter abbreviated to as cAMP) according to its EP2 agonistic action, and have an action that accelerates healing of fractures (see Non-Patent Literature 6). Also, it has disclosed that the compound described in Patent Literature 5 has bronchodilation based on the EP2 agonistic action, and has excellent EP2 receptor selectivity (see Patent Literature 6). However, in either of the above-mentioned literatures, there is no specific disclosure about the sulfonamide compound having an aromatic ring group or a 5- to 6-membered heteroaromatic ring group substituted by a specific substituent, and a pyridylaminoacetic acid and an ester thereof as a partial structure according to the compound of the present invention.

PRIOR ART LITERATURES

PATENT LITERATURE

[Patent Literature 1] WO98/28264A
[Patent Literature 2] WO99/19300A
[Patent Literature 3] WO2004/078169A
[Patent Literature 4] WO2008/015517A
[Patent Literature 5] WO2009/113600A
[Patent Literature 6] WO2010/113957A


NON-PATENT LITERATURE

[Non-Patent Literature 1] American Journal of Respiratory and Critical Care Medicine, 159,31(1999)
[Non-Patent Literature 2] American Journal of Physiology-Lung Cellular and Molecular Physiology, 284, L599 (2003)

[Non-Patent Literature 3] Prostaglandins, Leukotrienes and Essential Fatty Acids, 71, 277(2004)
[Non-Patent Literature 4] Invest. Ophthalmol. Vis. Sci., 43, 1475(2002)
[Non-Patent Literature 5] Invest. Ophthalmol. Vis. Sci., 24, 312(1983)
[Non-Patent Literature 6] Proceedings of the National Academy of Sciences of the United States of America, 100, 6736(2003)

SUMMARY OF THE INVENTION

PROBLEMS TO BE SOLVED BY THE INVENTION

The present inventors have carried out intensive studies on various sulfonamide compounds to develop a superior therapeutic agent or prophylactic agent for respiratory diseases or eye diseases, and as a result, they have found that a novel aminopyridine compound having a specific structure has potent EP2 selective agonistic action, while also having superior properties in terms of tissue distribution, bioavailability (BA), fast-acting pharmacological effect, sustained pharmacological effect, solubility, physical stability, drug interaction, toxicity and the like, and is particularly useful as a therapeutic and/or prophylactic agent (preferably a therapeutic agent) for respiratory diseases such as asthma and COPD or eye diseases (in particular, glaucoma), thereby leading to completion of the present invention.
The present invention is to provide a novel aminopyridine compound or a pharmaceutically acceptable salt thereof, that has potent EP2 selective agonistic action, and is particularly useful as a therapeutic and/or prophylactic agent (and preferably a therapeutic agent) for respiratory diseases such as asthma and COPD or eye diseases (in particular, glaucoma).

MEANS TO SOLVE THE PROBLEMS

The "aminopyridine compound" in the present invention means the compound represented by the following formula (I).
A compound represented by the formula:

[Formula 1]

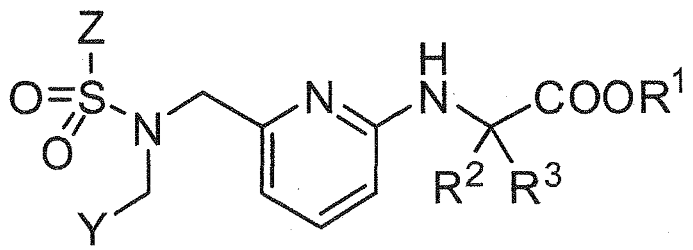

(I)

[wherein
$R^1$, $R^2$ and $R^3$ are each independently represent a hydrogen atom or $C_1$-$C_6$ alkyl group,
Y represents the formula (II):
[Formula 2]

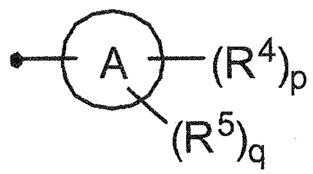

(II)

(wherein Ring A represents an aromatic ring group or a 5- to 6-membered heteroaromatic ring group, $R^4$ represents a $C_1$-$C_{12}$ alkyl group, halogeno-$C_1$-$C_8$ alkyl group, $C_1$-$C_6$ alkoxy group, halogeno-$C_1$-$C_6$ alkoxy group, halogeno-$C_1$-$C_6$ alkoxy-$C_1$-$C_6$ alkyl group, $C_3$-$C_6$ cycloalkyl-$C_1$-$C_6$ alkyl group, $C_2$-$C_6$ alkenyl group or the formula (III):
[Formula 3]

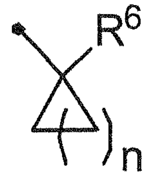

(III)

(wherein $R^6$ represents a $C_1$-$C_{12}$ alkyl group, a $C_1$-$C_6$ alkoxy group, a $C_7$-$C_{18}$ aralkyl group, a $C_1$-$C_6$ alkoxy-$C_1$-$C_6$ alkyl group or a $C_7$-$C_{18}$ aralkyloxy-$C_1$-$C_6$ alkyl group, n is an integer of 1 to 4.), p is an integer of 1 to 3, $R^5$ represents a halogeno group or an amino group which may be substituted by a $C_1$-$C_6$ alkyl group, q is an integer of 0 to 2.),
Z represents an aromatic ring group or a 5- to 6-membered heteroaromatic ring group each of which may be substituted by a group(s) selected from the group consisting of a halogeno group, a $C_1$-$C_6$ alkyl group, a halogeno-$C_1$-$C_6$ alkyl group, a $C_1$-$C_6$ alkoxy group and a halogeno-$C_1$-$C_6$ alkoxy group.]
or a pharmaceutically acceptable salt thereof.

## EFFECTS OF THE INVENTION

The aminopyridine compound represented by the formula (I) or a pharmaceutically acceptable salt thereof of the present invention demonstrates a potent EP2 selective agonistic action, and also has superior properties in terms of tissue distribution, bioavailability (BA), fast-acting pharmaceutically effect, sustained pharmaceutically effect, solubility, physical stability, drug interaction, toxicity and the like. Thus, the present invention is able to provide a novel compound having superior properties as a therapeutic and/or prophylactic agent for respiratory diseases (such as asthma, COPD, bronchitis, emphysema, pulmonary fibrosis, acute respiratory distress syndrome (ARDS), cystic fibrosis and pulmonary hypertension) or eye diseases (in particular, glaucoma). Moreover, the compound represented by the formula (I) of the present invention is also useful as a therapeutic and/or prophylactic agent for diseases for which EP2 agonistic action is thought to be useful (such as bone diseases, gastric ulcer, hypertension, etc).

## EMBODIMENTS TO CARRY OUT THE INVENTION

In the compound represented by the above-mentioned formula (I), the "$C_1$-$C_6$ alkyl group" shown by $R^1$; the "$C_1$-$C_6$ alkyl group" show by $R^2$; the "$C_1$-$C_6$ alkyl group" shown by $R^3$; the "$C_1$-$C_6$ alkyl portion" of the $C_1$-$C_6$ alkoxy-$C_1$-$C_6$ alkyl group shown by $R^6$ in the formula (III) shown by $R^4$ in the formula (II) shown by Y, the "$C_1$-$C_6$ alkyl portion" of the $C_7$-$C_{18}$ aralkyloxy-$C_1$-$C_6$ alkyl group; the "$C_1$-$C_6$ alkyl group" as a substituent for the aromatic ring group or the 5- to 6-membered heteroaromatic ring group shown by Z, and the "$C_1$-$C_6$ alkyl portion" of the halogeno-$C_1$-$C_6$ alkyl group each means the "$C_1$-$C_6$ alkyl group" having the same meanings, and such a "$C_1$-$C_6$ alkyl group" may be mentioned, for example, a linear or branched $C_1$-$C_6$ alkyl group such as a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a 1-methylbutyl group, a 2-methylbutyl group, a 1-ethylpropyl group, a 1,2-dimethylpropyl group, a hexyl group, a 1-methylpentyl group, a 2-methylpentyl group, a 3-methylpentyl group, a 4-methylpentyl group, a 1-ethylbutyl group, a 2-ethylbutyl group, a 1,1-dimethylbutyl group, a 1,2-dimethylbutyl group, a 1,3-dimethylbutyl group, a 2,2-dimethylbutyl group, a 2,3-dimethylbutyl group, a 3,3-dimethylbutyl group, a 1-ethyl-1-methylpropyl group, a 1-ethyl-2-methylpropyl group, a 1,1,2-trimethylpropyl group and a 1,2,2-trimethylpropyl group, etc., preferably a $C_1$-$C_4$ alkyl group, further preferably a methyl group, an ethyl group, a propyl group, an isopropyl group or a tert-butyl group, particularly preferably a methyl group or an ethyl group.

$R^1$ is preferably a hydrogen atom, a methyl group, an ethyl group, a propyl group, an isopropyl group or a tert-butyl group, further preferably a hydrogen atom, a methyl group, an ethyl group or an isopropyl group, and particularly preferablya hydrogen atom or an ethyl group.

$R^2$ is preferably a hydrogen atom, a methyl group, an ethyl group, a propyl group or an isopropyl group, further preferably a hydrogen atom or a methyl group, and particularly preferably a hydrogen atom.

$R^3$ is preferably a hydrogen atom, a methyl group, an ethyl group, a propyl group or an isopropyl group, further preferably a hydrogen atom or a methyl group, and particularly preferably a hydrogen atom.

The "$C_1$-$C_{12}$ alkyl group" shown by $R^4$ may be mentioned, for example, a linear or branched $C_1$-$C_{12}$ alkyl group such as a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a 1-methylbutyl group, a 2-methylbutyl group, a 1,2-dimethylpropyl group, a 1-ethylpropyl group, a hexyl group, a 1-methylpentyl group, a 2-methylpentyl group, a 3-methylpentyl group, a 4-methylpentyl group, a 1-ethylbutyl group, a 2-ethylbutyl group, a

1,1-dimethylbutyl group, a 2,2-dimethylbutyl group, a 3,3-dimethylbutyl group, a 1,3-dimethylbutyl group, a 2,3-dimethylbutyl group, a 1-ethyl-1-methylpropyl group, a heptyl group, a 1-methylhexyl group, a 2-methylhexyl group, a 3-methylhexyl group, a 4-methylhexyl group, a 5-methylhexyl group, a 1-ethylpentyl group, a 2-ethylpentyl group, a 3-ethylpentyl group, a 1,2-dimethylpentyl group, a 1,3-dimethylpentyl group, a 1, 4-dimethylpentyl group, a 2,3-dimethylpentyl group, a 2,4-dimethylpentyl group, a 3,4-dimethylpentyl group, a 1,1-dimethylpentyl group, a 2,2-dimethylpentyl group, a 3,3-dimethylpentyl group, a 4,4-dimethylpentyl group, a 1-methyl-2-ethylbutyl group, an octyl group, a 1-methylheptyl group, a 2-methylheptyl group, a 3-methylheptyl group, a 4-methylheptyl group, a 5-methylheptyl group, a 6-methylheptyl group, a 1-ethylhexyl group, a 2-ethylhexyl group, a 3-ethylhexyl group, a 4-ethylhexyl group, a 1,1-dimethylhexyl group, a 2,2-dimethylhexyl group, a 3,3-dimethylhexyl group, a 4,4-dimethylhexyl group, a 5,5-dimethylhexyl group, a 1-propylpentyl group, a 2-propylpentyl group, a 1,1,3,3-tetramethylbutyl group, a 1,1,4-trimethylpentyl group, a nonyl group, a 1-methyloctyl group, a 2-methyloctyl group, a 3-methyloctyl group, a 4-methyloctyl group, a 5-methyloctyl group, a 6-methyloctyl group, a 7-methyloctyl group, a 1-ethylheptyl group, a 2-ethylheptyl group, a 3-ethylheptyl group, a 4-ethylheptyl group, a 5-ethylheptyl group, a 1,1-dimethylheptyl group, a 2,2-dimethylheptyl group, a 3,3-dimethylheptyl group, a 4,4-dimethylheptyl group, a 5,5-dimethylheptyl group, a 1-propylhexyl group, a 2-propylhexyl group, a 3-propylhexyl group, a decyl group, a 1-methylnonyl group, a 2-methylnonyl group, a 3-methylnonyl group, a 4-methylnonyl group, a 5-methylnonyl group, a 6-methylnonyl group, a 7-methylnonyl group, a 8-methylnonyl group, a 1,1-dimethyloctyl group, a 2,2-dimethyloctyl group, a 3,3-dimethyloctyl group, a 4,4-dimethyloctyl group, a 5,5-dimethyloctyl group, a 6,6-dimethyloctyl group, a 1-ethyloctyl group, a 2-ethyloctyl group, a 3-ethyloctyl group, a 4-ethyloctyl group, a 5-ethyloctyl group, a 6-ethyloctyl group, a 1-propylheptyl group, a 2-propylheptyl group, a 3-propylheptyl group, a 4-propylheptyl group, a undecyl group, a 1-methyldecyl group, a 2-methyldecyl group, a 3-methyldecyl group, a 4-methyldecyl group, a 5-methyldecyl group, a 6-methyldecyl group, a 7-methyldecyl group, a 8-methyldecyl group, a 9-methyldecyl group, a 1,1-dimethylnonyl group, a 2,2-dimethylnonyl group, a 3,3-dimethylnonyl group, a 4,4-dimethylnonyl group, a 5,5-dimethylnonyl group, a 6,6-dimethylnonyl group, a 7,7-dimethylnonyl group, a 8,8-dimethylnonyl group, a 1-ethylnonyl group, a 2-ethylnonyl group, a 3-ethylnonyl group, a 4-ethylnonyl group, a 5-ethylnonyl group, a 6-ethylnonyl group, a 7-ethylnonyl group, a 1-propyloctyl group, a 2-propyloctyl group, a 3-propyloctyl group, a 4-propyloctyl group, a 5-propyloctyl group, a dodecyl group, a 1-methylundecyl group, a 2-methylundecyl group, a 3-methylundecyl group, a 4-methylundecyl group, a 5-methylundecyl group, a 6-methylundecyl group, a 7-methylundecyl group, a 8-methylundecyl group, a 9-methylundecyl group, a 10-methylundecyl group, a 1,1-dimethyldecyl group, a 2,2-dimethyldecyl group, a 3,3-dimethyldecyl group, a 4,4-dimethyldecyl group, a 5,5-dimethyldecyl group, a 6,6-dimethyldecyl group, a 7,7-dimethyldecyl group, a 8,8-dimethyldecyl group, a 9,9-dimethyldecyl group, a 1-ethyldecyl group, a 2-ethyldecyl group, a 3-ethyldecyl group, a 4-ethyldecyl group, a 5-ethyldecyl group, a 6-ethyldecyl group, a 7-ethyldecyl group, a 8-ethyldecyl group, a 1-propylnonyl group, a 2-propylnonyl group, a 3-propylnonyl group, a 4-propylnonyl group, a 5-propylnonyl group and a 6-propylnonyl group, etc.,

preferably a $C_1$-$C_8$ alkyl group, further preferably an ethyl group, a propyl group, an isopropyl group, a butyl group, a sec-butyl group, a tert-butyl group, a pentyl group, an isopentyl group, a neopentyl group, a tert-pentyl group, a 1-methylbutyl group, a 1-ethylpropyl group, a hexyl group, a 1-methylpentyl group, a 1-ethylbutyl group, a 1,1-dimethylbutyl group, a 2,2-dimethylbutyl group, a 1-ethyl-1-methylpropyl group, a 1-methylhexyl group, a 1,1-dimethylpentyl group, a 2,2-dimethylpentyl group, a 1,1-dimethylhexyl group, a 1,1,3,3-tetramethylbutyl group or a 1,1,4-trimethylpentyl group, particularly preferably an ethyl group, a butyl group, a tert-butyl group, a pentyl group, a neopentyl group, a tert-pentyl group, a 1-ethylpropyl group, a hexyl group, a 1-methylpentyl group, a 1,1-dimethylbutyl group, a 1-ethyl-1-methylpropyl group, a 1,1-dimethylpentyl group, a 2,2-dimethylpentyl group, a 1,1-dimethylhexyl group, a 1,1,3,3-tetramethylbutyl group or a 1,1,4-trimethylpentyl group.

The "halogeno portion" of the halogeno-$C_1$-$C_8$ alkyl group shown by $R^4$, the "halogeno portion" of the halogeno-$C_1$-$C_6$ alkoxy group, the "halogeno portion" of the halogeno-$C_1$-$C_6$ alkoxy-$C_1$-$C_6$ alkyl group; the "halogeno group" shown by $R^5$; the "halogeno group" as a substituent for the aromatic ring group or the 5- to 6-membered heteroaromatic ring group shown by Z, the "halogeno portion" of the halogeno-$C_1$-$C_6$ alkyl group, and the "halogeno portion" of the halogeno-$C_1$-$C_6$ alkoxy group each means the "halogeno group" having the same meanings, and such a "halogeno group" may be mentioned, for example, a fluoro group, a chloro group, a bromo group or an iodo group preferably a fluoro group, a chloro group or a bromo group, particularly preferably a fluoro group or a chloro group.

The "$C_1$-$C_8$ alkyl portion" of the halogeno-$C_1$-$C_8$ alkyl group shown by $R^4$ may be mentioned, for example, a linear or branched $C_1$-$C_8$ alkyl group such as a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a 1-methylbutyl group, a 2-methylbutyl group, a 1,2-dimethylpropyl group, a 1-ethylpropyl group, a hexyl group, a 1-methylpentyl group, a 2-methylpentyl group, a 3-methylpentyl group, a 4-methylpentyl group, a 1-ethylbutyl group, a 2-ethylbutyl group, a 1,1-dimethylbutyl group, a 2,2-dimethylbutyl group, a 3,3-dimethylbutyl group, a 1,3-dimethylbutyl group, a 2,3-dimethylbutyl group, a 1-ethyl-1-methylpropyl group, a heptyl group, a 1-methylhexyl group, a 2-methylhexyl group, a 3-methylhexyl group, a 4-methylhexyl group, a 5-methylhexyl group, a 1-ethylpentyl group, a 2-ethylpentyl group, a 3-ethylpentyl group, a 1,2-dimethylpentyl group, a 1,3-dimethylpentyl group, a 1, 4-dimethylpentyl group, a 2,3-dimethylpentyl group, a 2,4-dimethylpentyl group, a 3,4-dimethylpentyl group, a 1,1-dimethylpentyl

group, a 2,2-dimethylpentyl group, a 3,3-dimethylpentyl group, a 4,4-dimethylpentyl group, a 1-methyl-2-ethylbutyl group, an octyl group, a 1-methylheptyl group, a 2-methylheptyl group, a 3-methylheptyl group, a 4-methylheptyl group, a 5-methylheptyl group, a 6-methylheptyl group, a 1-ethylhexyl group, a 2-ethylhexyl group, a 3-ethylhexyl group, a 4-ethylhexyl group, a 1,1-dimethylhexyl group, a 2,2-dimethylhexyl group, a 3,3-dimethylhexyl group, a 4,4-dimethylhexyl group, a 5,5-dimethylhexyl group, a 1-propylpentyl group, a 2-propylpentyl group, a 1,1,3,3-tetramethylbutyl group and a 1,1,4-trimethylpentyl group, etc., preferably a methyl group, an ethyl group, a propyl group, a butyl group, a pentyl group, a tert-pentyl group, a 1-methylbutyl group, a hexyl group, a 1-methylpentyl group, a 1,1-dimethylbutyl group, a 1,1-dimethylpentyl group, a 2,2-dimethylpentyl group, a 1-methylhexyl group or a 1,1-dimethylhexyl group, further preferably a methyl group, a butyl group, a pentyl group, a hexyl group, a 1-methylpentyl group, a 1,1-dimethylbutyl group, a 1,1-dimethylpentyl group or a 2,2-dimethylpentyl group, particularly preferably a methyl group, a pentyl group, a 1-methylpentyl group, a 1,1-dimethylbutyl group or a 1,1-dimethylpentyl group.

The "halogeno-$C_1$-$C_8$ alkyl group" shown by $R^4$ may be mentioned, for example, a linear or branched halogeno-$C_1$-$C_8$ alkyl group such as a fluoromethyl group, a difluoromethyl group, a trifluoromethyl group, a chloromethyl group, a dichloromethyl group, a trichloromethyl group, a 2-fluoroethyl group, a 2,2,2-trifluoroethyl group, a pentafluoroethyl group, a 2-chloroethyl group, a 2,2,2-trichloroethyl group, a 2-bromoethyl group, a 3-fluoropropyl group, a 3,3,3-trifluoropropyl group, a heptafluoropropyl group, a 3-chloropropyl group, a 1,2,2,2-tetrafluoro-1-trifluoromethylethyl group, a 2,2,2-trifluoro-1-methylethyl group, a 2-fluoro-1-methylethyl group, a 2-chloro-I-methylethyl group, a 4-fluorobutyl group, a 4,4,4-trifluorobutyl group, a perfluorobutyl group, a perfluoro-tert-butyl group, a 2,2,2-trifluoro-1,1-dimethylethyl group, a 2-fluoro-1,1-dimethylethyl group, a 4-chlorobutyl group, a 2-chloro-1,1-dimethylethyl group, a 5,5,5-trifluoropentyl group, a perfluoropentyl group, a 4,4,4-trifluoro-1-methylbutyl group, a 1-ethyl-3,3,3-trifluoropropyl group, a 6,6,6-trifluorohexyl group, a perfluorohexyl group, a 5,5,5-trifluoro-1-methylpentyl group, a 1-ethyl-4,4,4-trifluorobutyl group, a 4,4,4-trifluoro-1,1-dimethylbutyl group, a 4,4,4-trifluoro-2,2-dimethylbutyl group, a 7, 7, 7-trifluoroheptyl group, a perfluoroheptyl group, a 6,6,6-trifluoro-1-methylhexyl group, a 5,5,5-trifluoro-1,1-dimethylpentyl group, a 5,5,5-trifluoro-2,2-dimethylpentyl group, a 8,8,8-trifluorooctyl group, a perfluorooctyl group and a 6,6,6-trifluoro-1,1-dimethylhexyl group, etc., preferably a fluoro-$C_1$-$C_8$ alkyl group, further preferably a trifluoromethyl group, a 4,4,4-trifluorobutyl group, a 5,5,5-trifluoropentyl group, a 6,6,6-trifluorohexyl group, a 5,5,5-tri-fluoro-1-methylpentyl group, a 4,4,4-trifluoro-1,1-dimethylbutyl group, a 5,5,5-tri-fluoro-1,1-dimethylpentyl group or a 5,5,5-trifluoro-2,2-dimethylpentyl group, particularly preferably a trifluoromethyl group, a 5,5,5-trifluoropentyl group, a 5,5,5-trifluoro-1-methylpentyl group, a 4,4,4-trifluoro-1,1-dimethylbutyl group or a 5,5,5-trifluoro-1,1-dimethylpentyl group.

The "$C_1$-$C_6$ alkoxy group" shown by $R^4$; and the "$C_1$-$C_6$ alkoxy portion" of the $C_1$-$C_6$ alkoxy-$C_1$-$C_6$ alkyl group shown by $R^6$ each means the "$C_1$-$C_6$ alkoxy group" having the same meanings, and such a "$C_1$-$C_6$ alkoxy group" may be mentioned, for example, a linear or branched $C_1$-$C_6$ alkoxy group such as a methoxy group, an ethoxy group, a propoxy group, an isopropoxy group, a butoxy group, an isobutoxy group, a sec-butoxy group, a tert-butoxy group, a pentyloxy group, an isopentyloxy group, a neopentyloxy group, a tert-pentyloxy group, a 1-methylbutoxy group, a 2-methylbutoxy group, a 1-ethylpropoxy group, a 1,2-dimethylpropoxy group, a hexyloxy group, a 1-methylpentyloxy group, a 2-methylpentyloxy group, a 3-methylpentyloxy group, a 4-methylpentyloxy group, a 1-ethylbutoxy group, a 2-ethylbutoxy group, a 1,1-dimethylbutoxy group, a 1,2-dimethylbutoxy group, a 1,3-dimethylbutoxy group, a 2,2-dimethylbutoxy group, a 2,3-dimethylbutoxy group, a 3,3-dimethylbutoxy group, a 1-ethyl-1-methylpropoxy group, a 1-ethyl-2-methylpropoxy group, a 1,1,2-trimethylpropoxy group and a 1,2,2-trimethylpropoxy group, etc., preferably a $C_1$-$C_4$ alkoxy group, further preferably a propoxy group, a butoxy group or an isobutoxy group, particularly preferably a propoxy group or a butoxy group.

The "$C_1$-$C_6$ alkoxy portion" of the halogeno-$C_1$-$C_6$ alkoxy group shown by $R^4$, the "$C_1$-$C_6$ alkoxy portion" of the halogeno-$C_1$-$C_6$ alkoxy-$C_1$-$C_6$ alkyl group; the "$C_1$-$C_6$ alkoxy group" shown by $R^6$; the "$C_1$-$C_6$ alkoxy group" as a substituent for the aromatic ring group or the 5- to 6-membered heteroaromatic ring group shown by Z, and the "$C_1$-$C_6$ alkoxy portion" of the halogeno-$C_1$-$C_6$ alkoxy group each means the "$C_1$-$C_6$ alkoxy group" having the same meanings, preferably a $C_1$-$C_4$ alkoxy group, further preferably a methoxy group, an ethoxy group, a propoxy group or an isopropoxy group, particularly preferably a methoxy group.

The "halogeno-$C_1$-$C_6$ alkoxy group" shown by $R^4$, the "halogeno-$C_1$-$C_6$ alkoxy portion" of the halogeno-$C_1$-$C_6$ alkoxy-$C_1$-$C_6$ alkyl group; and the "halogeno-$C_1$-$C_6$ alkoxy group" as a substituent for the aromatic ring group or the 5- to 6-membered heteroaromatic ring group shown by Z may be mentioned, for example, a linear or branched halogeno-$C_1$-$C_6$ alkoxy group such as a trifluoromethoxy group, a difluoromethoxy group, a trichloromethoxy group, a dichloromethoxy group, a perfluoroethoxy group, a 2,2,2-trifluoroethoxy group, a 2-fluoroethoxy group, a 2,2,2-trichloroethoxy group, a 2-chloroethoxy group, a 2-bromoethoxy group, a perfluoropropoxy group, a 3,3,3-trifluoropropoxy group, a 3-fluoropropoxy group, a 3-chloropropoxy group, a 1,2,2,2-tetrafluoro-1-trifluoromethylethoxy group, a 2,2,2-trifluoro-1-methylethoxy group, a 2-fluoro-1-methylethoxy group, a 2-chloro-1-methylethoxy group, a perfluorobutoxy group, a 4,4,4-trifluorobutoxy group, a 4-fluorobutoxy group, a 4-chlorobutoxy group, a perfluoro-tert-butoxy group, a 2,2,2-trifluoro-1,1-dimethylethoxy group, a 2-fluoro-1,1-dimethylethoxy group, a 2-chloro-1,1-dimethylethoxy group, a perfluoropentyloxy group and a perfluorohexyloxy group, etc., preferably a halogeno-$C_1$-$C_4$ alkoxy group, further preferably a

trifluoromethoxy group, a difluoromethoxy group, a trichloromethoxy group or a dichloromethoxy group, particularly preferably a difluoromethoxy group.

The "$C_1$-$C_6$ alkyl portion" of the halogeno-$C_1$-$C_6$ alkoxy-$C_1$-$C_6$ alkyl group shown by $R^4$, and the "$C_1$-$C_6$ alkyl portion" of the $C_3$-$C_6$ cycloalkyl-$C_1$-$C_6$ alkyl group each means the "$C_1$-$C_6$ alkyl group" having the same meanings as mentioned above, preferably a $C_3$-$C_6$ alkyl group, further preferably a propyl group, a sec-butyl group, a tert-pentyl group or a 1,1-dimethylbutyl group, particularly preferably a sec-butyl group or a tert-pentyl group.

The "halogeno-$C_1$-$C_6$ alkoxy-$C_1$-$C_6$ alkyl group" shown by $R^4$ may be mentioned, for example, a difluoromethoxymethyl group, a trifluoromethoxymethyl group, a dichloromethoxymethyl group, a 2-fluoroethoxymethyl group, a 2-chloroethoxymethyl group, a 2,2,2-trifluoroethoxymethyl group, a 2,2,2-trichloroethoxymethyl group, a 3,3,3-trifluoropropoxymethyl group, a 4,4,4-trifluorobutoxymethyl group, a 5,5,5-trifluoropentyloxymethyl group, a 6,6,6-trifluorohexyloxymethyl group, a 2-difluoromethoxyethyl group, a 2-trifluoromethoxyethyl group, a 2-(2,2,2-trifluoroethoxy)ethyl group, a 2-(3,3,3-trifluoropropoxy)ethyl group, a 2-(4,4,4-trifluorobutoxy)-ethyl group, a 2-(5,5,5-trifluoropentyloxy)ethyl group, a 2-(6,6,6-trifluorohexyloxy)-ethyl group, a 3-difluoromethoxypropyl group, a 3-trifluoromethoxypropyl group, a 3-(2,2,2-trifluoroethoxy)propyl group, a 3-(3,3,3-trifluoropropoxy)propyl group, a 3-(4,4,4-trifluorobutoxy)propyl group, a 2-difluoromethoxy-1-methylethyl group, a 4-difluoromethoxybutyl group, a 4-trifluoromethoxybutyl group, a 4-(2,2,2-trifluoroethoxy)butyl group, a 4-(3,3,3-trifluoropropoxy)butyl group, a 4-(4,4,4-trifluorobutoxy)butyl group, a 3-difluoromethoxy-1-methylpropyl group, a 2-difluoromethoxy-1,1-dimethyl ethyl group, a 5-difluoromethoxypentyl group, a 5-trifluoromethoxypentyl group, a 4-difluoromethoxy- I -methylbutyl group, a 3-difluoromethoxy-1,1-dimethylpropyl group, a 3-trifluoromethoxy-1,1-dimethylpropyl group, a 3-difluoromethoxy-2,2-dimethylpropyl group, a 6-difluoromethoxyhexyl group, a 6-trifluoromethoxyhexyl group, a 5-difluoromethoxy-1-methylpentyl group, a 4-difluoromethoxy-1,1-dimethylbutyl group, a 4-trifluoromethoxy-1,1-dimethylbutyl group or a 4-difluoromethoxy-2,2-dimethylbutyl group, etc., preferably a fluoro-$C_1$-$C_4$ alkoxy-$C_3$-$C_6$ alkyl group, further preferably a 3-difluoromethoxypropyl group, a 3-difluoromethoxy-1-methylpropyl group, a 3-difluoromethoxy-1,1-dimethylpropyl group or a 4-difluoromethoxy-1,1-dimethylbutyl group, particularly preferably a 3-difluoromethoxy-1-methylpropyl group or a 3-difluoromethoxy-1,1-dimethylpropyl group.

The "$C_3$-$C_6$cyclo alkyl portion" of the $C_3$-$C_6$ cycloalkyl-$C_1$-$C_6$ alkyl group shown by $R^4$ may be mentioned a cyclopropyl group, a cyclobutyl group, a cyclopentyl group or a cyclohexyl group, preferably a cyclopropyl group, a cyclobutyl group or a cyclopentyl group, particularly preferably a cyclopropyl group or a cyclobutyl group.

The "$C_3$-$C_6$ cycloalkyl-$C_1$-$C_6$ alkyl group" shown by $R^4$ may be mentioned, for example, a cyclopropylmethyl group, a cyclobutylmethyl group, a cyclopentylmethyl group, a 1-cyclopropylethyl group, a 2-cyclopropylethyl group, a 2-cyclobutylethyl group, a 2-cyclopentylethyl group, a 3-cyclopropylpropyl group, a 3-cyclobutylpropyl group, a 3-cyclopentylpropyl group, a 2-cyclopropyl-1-methylethyl group, a 2-cyclobutyl-1-methylethyl group, a 4-cyclopropylbutyl group, a 4-cyclobutylbutyl group, a 4-cyclopentylbutyl group, a 2-cyclopropyl-1,1-dimethylethyl group, a 2-cyclobutyl-1,1-dimethylethyl group, a 3-cyclopropyl-1-methylpropyl group, a 3-cyclobutyl-1-methylpropyl group, a 5-cyclopropylpentyl group, a 5-cyclobutylpentyl group, a 3-cyclopropyl-1,1-dimethylpropyl group, a 3-cyclobutyl-1,1-dimethylpropyl group, a 4-cyclopropyl-1-methylbutyl group, a 4-cyclobutyl-1-methylbutyl group, a 6-cyclopropylhexyl group, a 6-cyclobutylhexyl group, a 4-cyclopropyl-1,1-dimethylbutyl group, a 4-cyclobutyl-1,1-dimethylbutyl group, a 5-cyclopropyl-1-methylpentyl group, a 5-cyclobutyl-1-methylpentyl group or a 4-cyclopropyl-2,2-dimethylbutyl group, etc., preferably a $C_3$-$C_4$ cycloalkyl-$C_3$-$C_6$ alkyl group, further preferably a 3-cyclopropylpropyl group, a 3-cyclopropyl-1-methylpropyl group, a 3-cyclopropyl-1,1-dimethylpropyl group or a 4-cyclopropyl-1,1-dimethylbutyl group, particularly preferably a 3-cyclopropyl-1-methylpropyl group or a 3-cyclopropyl-1,1-dimethylpropyl group.

The "$C_2$-$C_6$ alkenyl group" shown by $R^4$ may be mentioned, for example, a linear or branched $C_2$-$C_6$ alkenyl group such as a vinyl, a 1-propenyl group, a 2-propenyl group, an isopropenyl group, a 1-butenyl group, a 2-butenyl group, a 3-butenyl group, a 1-methyl-1-propenyl group, a 2-methyl-1-propenyl group, a 1-methyl-2-propenyl group, a 2-methyl-2-propenyl group, a 1-ethylvinyl group, a 1-pentenyl group, a 2-pentenyl group, a 3-pentenyl group, a 4-pentenyl group, a 1-methyl-1-butenyl group, a 2-methyl-1-butenyl group, a 3-methyl-1-butenyl group, a 1-methyl-2-butenyl group, a 2-methyl-2-butenyl group, a 3-methyl-2-butenyl group, a 1-methyl-3-butenyl group, a 2-methyl-3-butenyl group, a 3-methyl-3-butenyl group, a 1-ethyl-1-propenyl group, a 1-ethyl-2-propenyl group, a 1,1-dimethyl-2-propenyl group, a 1-hexenyl group, a 2-hexenyl group, a 3-hexenyl group, a 4-hexenyl group, a 5-hexenyl group, a 1-methyl-1-pentenyl group, a 2-methyl-1-pentenyl group, a 3-methyl-1-pentenyl group, a 4-methyl-1-pentenyl group, a 1-methyl-2-pentenyl group, a 2-methyl-2-pentenyl group, a 3-methyl-2-pentenyl group, a 4-methyl-2-pentenyl group, a 1-methyl-3-pentenyl group, a 2-methyl-3-pentenyl group, a 3-methyl-3-pentenyl group, a 4-methyl-3-pentenyl group, a 1-methyl-4-pentenyl group, a 2-methyl-4-pentenyl group, a 3-methyl-4-pentenyl group, a 4-methyl-4-pentenyl group, a 1-ethyl-1-butenyl group, a 2-ethyl-1-butenyl group, a 1-ethyl-2-butenyl group, a 2-ethyl-2-butenyl group, a 1-ethyl-3-butenyl group, a 2-ethyl-3-butenyl group, a 1,1-dimethyl-2-butenyl group and a 1,1-dimethyl-3-butenyl group, etc., preferably a $C_3$-$C_6$ alkenyl group, further preferably an isopropenyl group, a 1-methyl-1-propenyl group, a 1-methyl-1-butenyl group or a 1-methyl-1-pentenyl group, particularly preferably a 1-methyl-1-pentenyl group.

The "$C_1$-$C_6$ alkyl group" as a substituent for the amino group shown by $R^5$ means the "$C_1$-$C_6$ alkyl group" having the same meanings as mentioned above, preferably a $C_1$-$C_3$ alkyl group, further preferably a methyl group or an ethyl

group. The amino group may be substituted by 1 or 2 $C_1$-$C_6$ alkyl groups.

The "amino group which may be substituted by a $C_1$-$C_6$ alkyl group" shown by $R^5$ may be mentioned, for example, an amino group, a methylamino group, a dimethylamino group, an ethylamino group, a diethylamino group, a propylamino group, a dipropylamino group, a diisopropylamino group, a N-ethyl-N-methylamino group, a butylamino group, a dibutylamino group, an isobutylamino group, a sec-butylamino group, a N-methyl-N-propylamino group, a tert-butylamino group, a pentylamino group, an isopentylamino group, a neopentylamino group, a tert-pentylamino group or a hexylamino group, preferably an amino group which may be substituted by a $C_1$-$C_3$ alkyl group, further preferably an amino group, a methylamino group, a dimethylamino group or ethylamino group, particularly preferably a methylamino group or a dimethylamino group.

The "$C_1$-$C_{12}$ alkyl group" shown by $R^6$ means the "$C_1$-$C_{12}$ alkyl group" having the same meanings as mentioned above, preferably a $C_1$-$C_{10}$ alkyl group, further preferably a methyl group, an ethyl group, a propyl group, an isopropyl group, a butyl group, an isobutyl group, a sec-butyl group, a tert-butyl group, a pentyl group, a hexyl group, a heptyl group, an octyl group, a nonyl group or a decyl group, particularly preferably a methyl group, an ethyl group, a propyl group, an isopropyl group, a butyl group or a nonyl group.

The "$C_7$-$C_{18}$ aralkyl group" shown by $R^6$ may be mentioned, for example, a benzyl group, a 1-phenylethyl group, a 2-phenylethyl group, a 1-phenylpropyl group, a 2-phenylpropyl group, a 3-phenylpropyl group, a 4-phenylbutyl group, a 5-phenylpentyl group, a 6-phenylhexyl group, a 7-phenylheptyl group, a 8-phenyloctyl group, a 9-phenylnonyl group, a 10-phenyldecyl group, a 11-phenylundecyl group, a 12-phenyldodecyl group, a naphthalen-1-ylmethyl group, a naphthalen-2-ylmethyl group, a 1-(naphthalen-1-yl)ethyl group, a 2-(naphthalen-1-yl)ethyl group, a 1-(naphthalen-2-yl)ethyl group, a 2-(naphthalen-2-yl)ethyl group, a 1-(naphthalen-1-yl)propyl group, a 2-(naphthalen-1-yl)propyl group, a 3-(naphthalen-1-yl)propyl group, a 1-(naphthalen-2-yl)propyl group, a 2-(naphthalen-2-yl)propyl group, a 3-(naphthalen-2-yl)propyl group, a 4-(naphthalen-1-yl)butyl group, a 4-(naphthalen-2-yl)butyl group, a 5-(naphthalen-1-yl)pentyl group, a 5-(naphthalen-2-yl)pentyl group, a 6-naphthalen-1-yl)hexyl group, a 6-(naphthalen-2-yl)hexyl group, a 7-(naphthalen-1-yl)heptyl group, a 7-(naphthalen-2-yl)heptyl group, a 8-(naphthalen-1-yl)octyl group or a 8-(naphthalen-2-yl)octyl group, etc., preferably a $C_7$-$C_{12}$ aralkyl group, further preferably a benzyl group, a 1-phenylethyl group, a 2-phenylethyl group, a naphthalen-1-ylmethyl group or a naphthalen-2-ylmethyl group, particularly preferably a benzyl group or a 2-phenylethyl group.

The "$C_1$-$C_6$ alkoxy-$C_1$-$C_6$ alkyl group" shown by $R^6$ may be mentioned, for example, a methoxymethyl group, an ethoxymethyl group, a propoxymethyl group, an isopropoxymethyl group, a butoxymethyl group, an isobutoxymethyl group, a sec-butoxymethyl group, a tert-butoxymethyl group, a pentyloxymethyl group, an isopentyloxymethyl group, a neopentyloxymethyl group, a tert-pentyloxymethyl group, a hexyloxymethyl group, a 1-methoxyethyl group, a 2-methoxyethyl group, a 2-ethoxyethyl group, a 2-propoxyethyl group, a 2-isopropoxyethyl group, a 2-butoxyethyl group, a 2-isobutoxyethyl group, a 2-(sec-butoxy)ethyl group, a 2-(tert-butoxy)ethyl group, a 2-hexyloxyethyl group, a 3-methoxypropyl group, a 3-ethoxypropyl group, a 4-methoxybutyl group, a 4-ethoxybutyl group, a 5-methoxypentyl group, a 5-ethoxypentyl group, a 6-methoxyhexyl group or a 6-ethoxyhexyl group, etc., preferably a $C_1$-$C_4$ alkoxy-$C_1$-$C_4$ alkyl group, further preferably an ethoxymethyl group, a propoxymethyl group, a butoxymethyl group, a 2-ethoxyethyl group or a 2-propoxyethyl group, particularly preferably a propoxymethyl group, a butoxymethyl group or a 2-propoxyethyl group.

The "$C_7$-$C_{18}$ aralkyloxy portion" of the "$C_7$-$C_{18}$ aralkyloxy-$C_1$-$C_6$ alkyl group" shown by $R^6$ may be mentioned, for example, a benzyloxy group, a 1-phenylethoxy group, a 2-phenylethoxy group, a 1-phenylpropoxy group, a 2-phenylpropoxy group, a 3-phenylpropoxy group, a 4-phenylbutoxy group, a 5-phenylpentoxy group, a 6-phenylhexyloxy group, a 7-phenylheptyloxy group, a 8-phenyloctyloxy group, a 9-phenylnonyloxy group, a 10-phenyldecyloxy group, a 11-phenylundecyloxy group, a 12-phenyldodecyloxy group, a naphthalen-1-ylmethoxy group, a naphthalen-2-ylmethoxy group, a 1-(naphthalen-1-yl)ethoxy group, a 2-(naphthalen-1-yl)ethoxy group, a 1-(naphthalen-2-yl)ethoxy group, a 2-(naphthalen-2-yl)ethoxy group, a 1-(naphthalen-1-yl)propoxy group, a 2-(naphthalen-1-yl)propoxy group, a 3-(naphthalen-1-yl)propoxy group, a 1-(naphthalen-2-yl)propoxy group, a 2-(naphthalen-2-yl)propoxy group, a 3-(naphthalen-2-yl)propoxy group, a 4-(naphthalen-1-yl)butoxy group, a 4-(naphthalen-2-yl)butoxy group, a 5-(naphthalen-1-yl)pentoxy group, a 5-(naphthalen-2-yl)pentoxy group, a 6-(naphthalen-1-yl)hexyloxy group, a 6-(naphthalen-2-yl)hexyloxy group, a 7-(naphthalen-1-yl)heptyloxy group, a 7-(naphthalen-2-yl)heptyloxy group, a 8-(naphthalen-l-yl)octyloxy group or a 8-(naphthalen-2-yl)octyloxy group, etc., preferably a $C_7$-$C_{12}$ aralkyloxy group, further preferably a benzyloxy group, a 1-phenylethoxy group, a 2-phenylethoxy group, a naphthalen-1-ylmethoxy group or a naphthalen-2-ylmethoxy group, particularly preferably a benzyloxy group or a 2-phenylethoxy group.

The "$C_7$-$C_{18}$ aralkyloxy-$C_1$-$C_6$ alkyl group" shown by $R^6$ may be mentioned, for example, a benzyloxymethyl group, a 2-benzyloxyethyl group, a 3-benzyloxypropyl group, a 4-benzyloxybutyl group, a 5-benzyloxypentyl group, a 6-benzyloxyhexyl group, a 1-phenylethoxymethyl group, a 2-phenylethoxymethyl group, a 1-(1-phenylethoxy)ethyl group, a 2-(1-phenylethoxy)ethyl group, a 1-2-phenylethoxy)ethyl group, a 2-(2-phenylethoxy)ethyl group, a 3-(2-phenylethoxy)propyl group, a 4-(2-phenylethoxy)butyl group, a 1-phenylpropoxymethyl group, a 2-phenylpropoxymethyl group, a 3-phenylpropoxymethyl group, a 4-phenylbutoxymethyl group, a 5-phenylpentoxymethyl group, a 6-phenylhexyloxymethyl group, a 7-phenylheptyloxymethyl group, a 8-phenyloctyloxymethyl group, a 9-phenylnonyloxymethyl group,

a 10-phenyldecyloxymethyl group, a 11-phenylundecyloxymethyl group, a 12-phenyldodecyloxymethyl group, a naphthalen-1-ylmethoxymethyl group or a naphthalen-2-ylmethoxymethyl group, etc., preferably a $C_7$-$C_{12}$ aralkyloxy-$C_1$-$C_4$ alkyl group, further preferably a benzyloxymethyl group, a 2-benzyloxyethyl group, a 1-phenylethoxymethyl group, a 2-phenylethoxymethyl group, a 1-(1-phenylethoxy)ethyl group or a 2-(2-phenylethoxy)-ethyl group, particularly preferably a benzyloxymethyl group, a 2-benzyloxyethyl group or a 2-phenylethoxymethyl group.

The "halogeno-$C_1$-$C_6$ alkyl group" as a substituent for the aromatic ring group or the 5- to 6-membered heteroaromatic ring group shown by Z may be mentioned, for example, a linear or branched halogeno-$C_1$-$C_6$ alkyl group such as a fluoromethyl group, a difluoromethyl group, a trifluoromethyl group, a chloromethyl group, a dichloromethyl group, a trichloromethyl group, a pentafluoroethyl group, a 2,2,2-trifluoroethyl group, a 2-fluoroethyl group, a 2,2,2-trichloroethyl group, a 2-chloroethyl group, a 2-bromoethyl group, a heptafluoropropyl group, a 3,3,3-trifluoropropyl group, a 3-fluoropropyl group, a 3-chloropropyl group, a 1,2,2,2-tetrafluoro-1-trifluoromethylethyl group, a 2,2,2-trifluoro-1-methylethyl group, a 2-fluoro-1-methylethyl group, a 2-chloro-1-methylethyl group, a perfluorobutyl group, a 4,4,4-trifluorobutyl group, a 4-fluorobutyl group, a 4-chlorobutyl group, a perfluoro-tert-butyl group, a 2,2,2-trifluoro-1,1-dimethylethyl group, a 2-fluoro-1,1-dimethylethyl group, a 2-chloro-1,1-dimethylethyl group, a perfluoropentyl group and a perfluorohexyl group, etc., preferably a halogeno-$C_1$-$C_4$ alkyl group, further preferably a trifluoromethyl group, a difluoromethyl group, a trichloromethyl group, a dichloromethyl group, a 2,2,2-trifluoroethyl group or a 2,2,2-trichloroethyl group, particularly preferably a trifluoromethyl group.

p is preferably 1 or 2.

q is preferably a 0 or 1.

n in the formula (III) is preferably a 1 to 3, further preferablyl or 2.

$R^6$ is preferably a $C_1$-$C_{10}$ alkyl group, a $C_1$-$C_4$ alkoxy group, a $C_7$-$C_{12}$ aralkyl group, a $C_1$-$C_4$ alkoxy-$C_1$-$C_4$ alkyl group or a $C_7$-$C_{12}$ aralkyloxy-$C_1$-$C_4$ alkyl group, further preferably a methyl group, an ethyl group, a propyl group, an isopropyl group, a butyl group, an isobutyl group, a sec-butyl group, a tert-butyl group, a pentyl group, a hexyl group, a heptyl group, an octyl group, a nonyl group, a decyl group, a methoxy group, an ethoxy group, a propoxy group, an isopropoxy group, a benzyl group, a 1-phenylethyl group, a 2-phenylethyl group, a naphthalen-1-ylmethyl group, a naphthalen-2-ylmethyl group, an ethoxymethyl group, a propoxymethyl group, a butoxymethyl group, a 2-ethoxyethyl group, a 2-propoxyethyl group, a benzyloxymethyl group, a 2-benzyloxyethyl group, a 1-phenylethoxymethyl group, a 2-phenylethoxymethyl group, a 1-(1-phenylethoxy)ethyl group or a 2-(2-phenylethoxy)ethyl group, particularly preferably a methyl group, an ethyl group, a propyl group, an isopropyl group, a butyl group, a nonyl group, a methoxy group, a benzyl group, a 2-phenylethyl group, a propoxymethyl group, a butoxymethyl group, a 2-propoxyethyl group, a benzyloxymethyl group, a 2-benzyloxyethyl group or a 2-phenylethoxymethyl group.

$R^4$ is preferably a $C_1$-$C_8$ alkyl group, a fluoro-$C_1$-$C_8$ alkyl group, a $C_1$-$C_4$ alkoxy group, a halogeno-$C_1$-$C_4$ alkoxy group, a fluoro-$C_1$-$C_4$ alkoxy-$C_3$-$C_6$ alkyl group, a $C_3$-$C_4$ cycloalkyl-$C_3$-$C_6$ alkyl group, a $C_3$-$C_6$ alkenyl group or the formula (III):

[Formula 4]

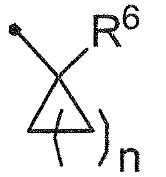

(III)

(wherein $R^6$ represents a $C_1$-$C_{10}$ alkyl group, a $C_1$-$C_4$ alkoxy group, a $C_7$-$C_{12}$ aralkyl group, a $C_1$-$C_4$ alkoxy-$C_1$-$C_4$ alkyl group or a $C_7$-$C_{12}$ aralkyloxy-$C_1$-$C_4$ alkyl group, and n is an integer of 1 to 3.),

further preferably an ethyl group, a propyl group, an isopropyl group, a butyl group, a sec-butyl group, a tert-butyl group, a pentyl group, an isopentyl group, a neopentyl group, a tert-pentyl group, a 1-methylbutyl group, a 1-ethylpropyl group, a hexyl group, a 1-methylpentyl group, a 1-ethylbutyl group, a 1,1-dimethylbutyl group, a 2,2-dimethylbutyl group, a 1-ethyl-1-methylpropyl group, a 1-methylhexyl group, a 1,1-dimethylpentyl group, a 2,2-dimethylpentyl group, a 1,1-dimethylhexyl group, a 1,1,3,3-tetramethylbutyl group, a 1,1,4-trimethylpentyl group, a trifluoromethyl group, a 4,4,4-trifluorobutyl group, a 5,5,5-trifluoropentyl group, a 6,6,6-trifluorohexyl group, a 5,5,5-trifluoro-1-methylpentyl group, a 4,4,4-trifluoro-1,1-dimethylbutyl group, a 5,5,5-trifluoro-1,1-dimethylpentyl group, a 5,5,5-trifluoro-2,2-dimethylpentyl group, a propoxy group, a butoxy group, an isobutoxy group, a trifluoromethoxy group, a difluoromethoxy group, a trichloromethoxy group, a dichloromethoxy group, a 3-difluoromethoxypropyl group, a 3-difluoromethoxy-1-methylpropyl group, a 3-difluoromethoxy-1,1-dimethylpropyl group, a 4-difluoromethoxy-1,1-dimethylbutyl group, a 3-cyclopropylpropyl group, a 3-cyclopropyl-1-methylpropyl group, a 3-cyclopropyl-1,1-dimethylpropyl group, a 4-cyclopropyl-1,1-dimethylbutyl group, an isopropenyl group, a 1-methyl-1-propenyl group, a 1-methyl-1-butenyl group, a 1-methyl-1-pentenyl group or the formula (III):

[Formula 5]

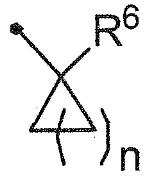

(III)

(wherein $R^6$ represents a methyl group, an ethyl group, a propyl group, an isopropyl group, a butyl group, an isobutyl group, a sec-butyl group, a tert-butyl group, a pentyl group, a hexyl group, a heptyl group, an octyl group, a nonyl group, a decyl group, a methoxy group, an ethoxy group, a propoxy group, an isopropoxy group, a benzyl group, a 1-phenylethyl group, a 2-phenylethyl group, a naphthalen-1-ylmethyl group, a naphthalen-2-ylmethyl group, an ethoxymethyl group, a propoxymethyl group, a butoxymethyl group, a 2-ethoxyethyl group, a 2-propoxyethyl group, a benzyloxymethyl group, a 2-benzyloxyethyl group, a 1-phenylethoxymethyl group, a 2-phenylethoxymethyl group, a 1-(1-phenylethoxy)ethyl group or a 2-(2-phenylethoxy)ethyl group, and n is an integer of 1 or 2.),

further more preferably an ethyl group, a propyl group, an isopropyl group, a butyl group, a sec-butyl group, a tert-butyl group, a pentyl group, an isopentyl group, a neopentyl group, a tert-pentyl group, a 1-methylbutyl group, a 1-ethylpropyl group, a hexyl group, a 1-methylpentyl group, a 1-ethylbutyl group, a 1,1-dimethylbutyl group, a 2,2-dimethylbutyl group, a 1-ethyl-1-methylpropyl group, a 1-methylhexyl group, a 1,1-dimethylpentyl group, a 2,2-dimethylpentyl group, a 1,1-dimethylhexyl group, a 1,1,3,3-tetramethylbutyl group, a 1,1,4-trimethylpentyl group, a trifluoromethyl group, a 4,4,4-trifluorobutyl group, a 5,5,5-trifluoropentyl group, a 6,6,6-trifluorohexyl group, a 5,5,5-trifluoro-1-methylpentyl group, a 4,4,4-trifluoro-1,1-dimethylbutyl group, a 5,5,5-trifluoro-1,1-dimethylpentyl group, a 5,5,5-trifluoro-2,2-dimethylpentyl group, a propoxy group, a butoxy group, an isobutoxy group, a trifluoromethoxy group, a difluoromethoxy group, a trichloromethoxy group, a dichloromethoxy group, a 3-difluoromethoxypropyl group, a 3-difluoromethoxy-1-methylpropyl group, a 3-difluoromethoxy-1,1-dimethylpropyl group, a 4-difluoromethoxy-1,1-dimethylbutyl group, a 3-cyclopropylpropyl group, a 3-cyclopropyl-1-methylpropyl group, a 3-cyclopropyl-1,1-dimethylpropyl group, a 4-cyclopropyl-1,1-dimethylbutyl group, an isopropenyl group, a 1-methyl-1-propenyl group, a 1-methyl-1-butenyl group, a 1-methyl-1-pentenyl group, a 1-methylcyclopropyl group, a 1-ethylcyclopropyl group, a 1-isopropylcyclopropyl group, a 1-butylcyclopropyl group, a 1-nonylcyclopropyl group, a 1-methoxycyclopropyl group, a 1-benzylcyclopropyl group, a 1-(2-phenylethyl)cyclopropyl group, a 1-propoxymethylcyclopropyl group, a 1-butoxymethylcyclopropyl group, a 1-(2-propoxyethyl)cyclopropyl group, a 1-benzyloxymethylcyclopropyl group, a 1-(2-benzyloxyethyl)cyclopropyl group, a 1-(2-phenylethoxymethyl)cyclopropyl group, a 1-ethylcyclobutyl group, a 1-isopropylcyclobutyl group, a 1-butylcyclobutyl group, a 1-nonylcyclobutyl group, a 1-methoxycyclobutyl group, a 1-benzylcyclobutyl group, a 1-propoxymethylcyclobutyl group, a 1-benzyloxymethylcyclobutyl group or a 2-benzyloxymethylcyclobutyl group,

particularly preferably an ethyl group, a butyl group, a tert-butyl group, a pentyl group, a neopentyl group, a tert-pentyl group, a 1-ethylpropyl group, a hexyl group, a 1-methylpentyl group, a 1,1-dimethylbutyl group, a 1-ethyl-1-methylpropyl group, a 1,1-dimethylpentyl group, a 2,2-dimethylpentyl group, a 1,1-dimethylhexyl group, a 1,1,3,3-tetramethylbutyl group, a 1,1,4-trimethylpentyl group, a trifluoromethyl group, a 5,5,5-trifluoropentyl group, a 5,5,5-trifluoro-1-methylpentyl group, a 4,4,4-trifluoro-1,1-dimethylbutyl group, a 5,5,5-trifluoro-1,1-dimethylpentyl group, a propoxy group, a butoxy group, a difluoromethoxy group, a 3-difluoromethoxy-1-methylpropyl group, a 3-difluoromethoxy-1,1-dimethylpropyl group, a 3-cyclopropyl-1-methylpropyl group, a 3-cyclopropyl-1,1-dimethylpropyl group, a 1-methyl-1-pentenyl group, a 1-methylcyclopropyl group, a 1-ethylcyclopropyl group, a 1-isopropylcyclopropyl group, a 1-butylcyclopropyl group, a 1-nonylcyclopropyl group, a 1-methoxycyclopropyl group, a 1-benzylcyclopropyl group, a 1-propoxymethylcyclopropyl group, a 1-benzyloxymethylcyclopropyl group, a 1-ethylcyclobutyl group or a 1-butylcyclobutyl group.

$R^5$ is preferably a fluoro group, a chloro group or an amino group which may be substituted by a $C_1$-$C_3$ alkyl group, further preferably a fluoro group, a chloro group, an amino group, a methylamino group, a dimethylamino group or ethylamino group, and particularly preferably a fluoro group, a methylamino group or dimethylamino group.

The "aromatic ring group" shown by Ring A; and the "aromatic ring group" shown by Z each means the "aromatic ring group" having the same meanings, and such an "aromatic ring group" may be mentioned, for example, a phenyl group or a naphthyl group, preferably a phenyl group.

The "5- to 6-membered heteroaromatic ring group" shown by Ring A means a completely unsaturated 5- to 6-membered cyclic group containing 1 to 4 hetero atoms (in case of a plural number, each independently) selected from

the group consisting of an oxygen atom, a nitrogen atom and a sulfur atom as a constitutional element(s) of the ring, and may be mentioned, for example, a pyrrolyl group, a furyl group, a thienyl group, a pyrazolyl group, an imidazolyl group, an oxazolyl group, a thiazolyl group, a pyridyl group, a pyridazinyl group, a pyrimidinyl group or a pyrazinyl group, etc., preferably a thienyl group, a thiazolyl group, a pyridyl group or a pyrimidinyl group, particularly preferably a thienyl group or a pyridyl group.

Ring A is preferably a phenyl group, a thienyl group, a thiazolyl group, a pyridyl group or a pyrimidinyl group, further preferably a phenyl group, a thienyl group or a pyridyl group.

Y is preferably the formula (II):

[Formula 6]

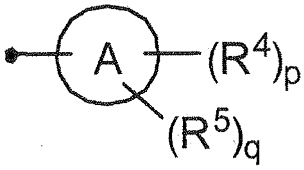

(II)

(wherein Ring A represents a phenyl group, a thienyl group, a thiazolyl group, a pyridyl group or a pyrimidinyl group, $R^4$ represents a $C_1$-$C_8$ alkyl group, a fluoro-$C_1$-$C_8$ alkyl group, a $C_1$-$C_4$ alkoxy group, a halogeno-$C_1$-$C_4$ alkoxy group, a fluoro-$C_1$-$C_4$ alkoxy-$C_3$-$C_6$ alkyl group, a $C_3$-$C_4$ cycloalkyl-$C_3$-$C_6$ alkyl group, a $C_3$-$C_6$ alkenyl group or the formula (III):

[Formula 7]

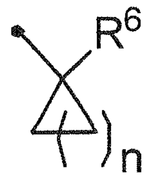

(III)

(wherein $R^6$ represents a $C_1$-$C_{10}$ alkyl group, a $C_1$-$C_4$ alkoxy group, a $C_7$-$C_{12}$ aralkyl group, a $C_1$-$C_4$ alkoxy-$C_1$-$C_4$ alkyl group or a $C_7$-$C_{12}$ aralkyloxy-$C_1$-$C_4$ alkyl group, and n is an integer of 1 to 3.), p is an integer of 1 or 2, $R^5$ represents a fluoro group, a chloro group or an amino group which may be substituted by a $C_1$-$C_3$ alkyl group, and q is an integer of 0 or 1.), further preferably the formula (II):

[Formula 8]

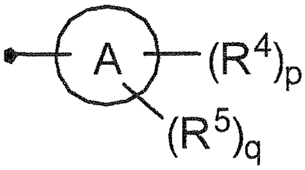

(II)

(wherein Ring A represents a phenyl group, a thienyl group, a thiazolyl group, a pyridyl group or a pyrimidinyl group, $R^4$ represents an ethyl group, a propyl group, an isopropyl group, a butyl group, a sec-butyl group, a tert-butyl group, a pentyl group, an isopentyl group, a neopentyl group, a tert-pentyl group, a 1-methylbutyl group, a 1-ethylpropyl group, a hexyl group, a 1-methylpentyl group, a 1-ethylbutyl group, a 1,1-dimethylbutyl group, a 2,2-dimethylbutyl group, a 1-ethyl-1-methylpropyl group, a 1-methylhexyl group, a 1,1-dimethylpentyl group, a 2,2-dimethylpentyl group, a 1,1-dimethylhexyl group, a 1,1,3,3-tetramethylbutyl group, a 1,1,4-trimethylpentyl group, a trifluoromethyl group, a 4,4,4-trifluorobutyl group, a 5,5,5-trifluoropentyl group, a 6,6,6-trifluorohexyl group, a 5,5,5-trifluoro-1-methylpentyl group, a 4,4,4-trifluoro-1,1-dimethylbutyl group, a 5,5,5-trifluoro-1,1-dimethylpentyl group, a 5,5,5-trifluoro-2,2-dimethylpentyl group, a propoxy group, a butoxy group, an isobutoxy group, a trifluoromethoxy group, a difluoromethoxy group, a

trichloromethoxy group, a dichloromethoxy group, a 3-difluoromethoxypropyl group, a 3-difluoromethoxy-l-methylpropyl group, a 3-difluoromethoxy-1,1-dimethylpropyl group, a 4-difluoromethoxy-1,1-dimethylbutyl group, a 3-cyclopropylpropyl group, a 3-cyclopropyl-1-methylpropyl group, a 3-cyclopropyl-1,1-dimethylpropyl group, a 4-cyclopropyl-1,1-dimethylbutyl group, an isopropenyl group, a 1-methyl-1-propenyl group, a 1-methyl-1-butenyl group, a 1-methyl-1-pentenyl group or the formula (III):

[Formula 9]

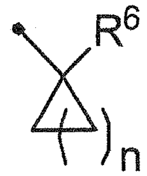

(III)

(wherein $R^6$ represents a methyl group, an ethyl group, a propyl group, an isopropyl group, a butyl group, an isobutyl group, a sec-butyl group, a tert-butyl group, a pentyl group, a hexyl group, a heptyl group, an octyl group, a nonyl group, a decyl group, a methoxy group, an ethoxy group, a propoxy group, an isopropoxy group, a benzyl group, a 1-phenylethyl group, a 2-phenylethyl group, a naphthalen-1-ylmethyl group, a naphthalen-2-ylmethyl group, an ethoxymethyl group, a propoxymethyl group, a butoxymethyl group, a 2-ethoxyethyl group, a 2-propoxyethyl group, a benzyloxymethyl group, a 2-benzyloxyethyl group, a 1-phenylethoxymethyl group, a 2-phenylethoxymethyl group, a 1-(1-phenylethoxy)ethyl group or a 2-(2-phenylethoxy)ethyl group, and n is an integer of 1 or 2.), p is an integer of 1 or 2, $R^5$ represents a fluoro group, a chloro group, an amino group, a methylamino group, a dimethylamino group or an ethylamino group, and q is an integer of 0 or 1.),

further more preferably a 4-ethylphenyl group, a 3,4-diethylphenyl group, a 4-propylphenyl group, a 4-isopropylphenyl group, a 3-butylphenyl group, a 4-butylphenyl group, a 3-tert-butylphenyl group, a 4-tert-butylphenyl group, a 5-tert-butylthiophen-2-yl group, a 3-pentylphenyl group, a 4-pentylphenyl group, a 3-dimethylamino-4-pentylphenyl group, a 4-isopentylphenyl group, a 4-neopentylphenyl group, a 4-tert-pentylphenyl group, a 5-tert-pentylthiophen-2-yl group, a 4-(1-ethylpropyl)phenyl group, a 4-(1-methylbutyl)phenyl group, a 3-hexylphenyl group, a 4-(1-ethylbutyl)phenyl group, a 4-(1-methylpentyl)phenyl group, a 5-(1-methylpentyl)thiophen-2-yl group, a 3-(1,1-dimethylbutyl)phenyl group, a 4-(1,1-dimethylbutyl)phenyl group, a 2-fluoro-4-(1,1-dimethylbutyl)phenyl group, a 3-fluoro-4-(1,1-dimethylbutyl)phenyl group, a 4-(2,2-dimethylbutyl)phenyl group, a 5-(1,1-dimethylbutyl)thiophen-2-yl group, a 4-(1-methyl-1-methylpropyl)phenyl group, a 3-(1,1-dimethylpentyl)phenyl group, a 4-(1,1-dimethylpentyl)phenyl group, a 2-fluoro-4-(1,1-dimethylpentyl)phenyl group, a 3-fluoro-4-(1,1-dimethylpentyl)phenyl group, a 3-amino-4-(1,1-dimethylpentyl)phenyl group, a 3-methylamino-4-(1,1-dimethylpentyl)phenyl group, a 3-dimethylamino-4-(1,1-dimethylpentyl)phenyl group, a 4-(1,1-dimethylpentyl)thiophen-2-yl group, a 5-(1,1-dimethylpentyl)thiophen-2-yl group, a 5-(1,1-dimethylpentyl)thiazol-2-yl group, a 5-(1,1-dimethylpentyl)pyridin-2-yl group, a 6-(1,1-dimethylpentyl)pyridin-3-yl group, a 5-(1,1-dimethylpentyl)pyrimidin-2-yl group, a 2-(1,1-dimethylpentyl)pyrimidin-5-yl group, a 3-(2,2-dimethylpentyl)phenyl group, a 4-(2,2-dimethylpentyl)phenyl group, a 4-(1-methylhexyl)phenyl group, a 4-(1,1,3,3-tetramethylbutyl)phenyl group, a 4-(1,1,4-trimethylpentyl)phenyl group, a 4-(1,1-dimethylhexyl)phenyl group, a 4-trifluoromethylphenyl group, a 4-(4,4,4-trifluorobutyl)phenyl group, a 4-(5,5,5-trifluoropentyl)-phenyl group, a 4-(6,6,6-trifluorohexyl)phenyl group, a 4-(5,5,5-trifluoro-1-methylpentyl)phenyl group, a 4-(4,4,4-trifluoro-1,1-dimethylbutyl)phenyl group, a 4-(5,5,5-trifluoro-1,1-dimethylpentyl)phenyl group, a 4-(5,5,5-trifluoro-2,2-dimethylpentyl)-phenyl group, a 5-(5,5,5-trifluoro-1,1-dimethylpentyl)thiophen-2-yl group, a 4-propoxyphenyl group, a 4-butoxyphenyl group, a 4-isobutoxyphenyl group, a 4-difluoromethoxyphenyl group, a 4-trifluoromethoxyphenyl group, a 4-(3-difluoromethoxy-1-methylpropyl)phenyl group, a 4-(3-difluoromethoxy-1,1-dimethylpropyl)phenyl group, a 4-(3-cyclopropyl-1-methylpropyl)phenyl group, a 4-(3-cyclopropyl-1,1-dimethylpropyl)phenyl group, a 4-isopropenylphenyl group, a 4-(1-methyl-1-propenyl)phenyl group, a 4-(1-methyl-1-butenyl)phenyl group, a 4-(1-methyl-1-pentenyl)phenyl group, a 4-(1-methylcyclopropyl)phenyl group, a 4-(1-ethylcyclopropyl)phenyl group, a 4-(1-propylcyclopropyl)phenyl group, a 4-(1-isopropylcyclopropyl)phenyl group, a 3-(1-buty1cyclopropyl)phenyl group, a 4-(1-butylcyclopropyl)phenyl group, a 2-fluoro-4-(1-butylcyclopropyl)phenyl group, a 3-fluoro-4-(1-butylcyclopropyl)phenyl group, a 3-dimethylamino-4-(1-butylcyclopropyl)phenyl group, a 5-(1-butylcyclopropyl)thiophen-2-yl group, a 4-(1-hexylcyclopropyl)phenyl group, a 4-(1-nonylcyclopropyl)phenyl group, a 4-(1-methoxycyclopropyl)phenyl group, a 4-(1-ethoxycyclopropyl)phenyl group, a 4-(l -propoxycyclopropyl)phenyl group, a 4-(1-benzylcyclopropyl)phenyl group, a 4-{1-(1-phenylethyl)cyclopropyl}phenyl group, a 4-{1-(2-phenylethyl)cyclopropyl}phenyl group, a 4-(1-ethoxymethylcyclopropyl)phenyl group, a 4-(1-propoxymethylcyclopropyl)phenyl group, a 5-(1-propoxymethylcyclopropyl)thiophen-2-yl group, a 4- {1-(2-ethoxyethyl)cyclopropyl}phenyl group, a 4-{1-(2-propoxyethyl)-cyclopropyl}phenyl group, a 4-(1-butoxymethylcyclopropyl)phenyl group, a 4-{1-benzyloxymethylcyclopropyl)phenyl group, a 4-{1-(2-benzyloxyethyl)cyclopropyl phenyl group, a 4-{1-(1-phenylethoxymethyl)cyclopropyl}phenyl group, a 4-{1-(2-phenylethoxymethyl)cyclopropyl}phenyl group, a 4-(1-ethylcyclobutyl)phenyl group, a 4-(1-butylcyclobutyl)phenyl group or a 5-(1-butylcyclobutyl)thiophen-2-yl group,

particularly preferably a 3,4-diethylphenyl group, a 3-butylphenyl group, a 4-butylphenyl group, a 3-tert-butylphenyl group, a 4-tert-butylphenyl group, a 5-tert-butylthiophen-2-yl group, a 4-pentylphenyl group, a 3-dimethylamino-4-pentylphenyl group, a 4-isopentylphenyl group, a 4-neopentylphenyl group, a 4-tert-pentylphenyl group, a 5-tert-pentylthiophen-2-yl group, a 4-(1-ethylpropyl)phenyl group, a 4-(1-methylbutyl)phenyl group, a 3-hexylphenyl group, a 4-(1-methylpentyl)phenyl group, a 5-(1-methylpentyl)thiophen-2-yl group, a 3-(1,1-dimethylbutyl)phenyl group, a 4-(1,1-dimethylbutyl)phenyl group, a 2-fluoro-4-(1,1-dimethylbutyl)phenyl group, a 3-fluoro-4-(1,1-dimethylbutyl)phenyl group, a 5-(1,1-dimethylbutyl)thiophen-2-yl group, a 4-(1-ethyl-1-methylpropyl)phenyl group, a 3-(1,1-dimethylpentyl)phenyl group, a 4-(1,1-dimethylpentyl)phenyl group, a 2-fluoro-4-(1,1-dimethylpentyl)phenyl group, a 3-fluoro-4-(l,l-dimethylpentyl)phenyl group, a 3-dimethylamino-4-(1,1-dimethylpentyl)-phenyl group, a 5-(1,1-dimethylpentyl)thiophen-2-yl group, a 4-(2,2-dimethylpentyl)-phenyl group, a 4-(1,1,3,3-tetramethylbutyl)phenyl group, a 4-(1,1,4-trimethylpentyl)-phenyl group, a 4-(1,1-dimethylhexyl)phenyl group, a 4-trifluoromethylphenyl group, a 4-(4,4,4-trifluoro-1,1-dimethylbutyl)phenyl group, a 4-(5,5,5-trifluoro-1,1-dimethylpentyl)phenyl group, a 5-(5,5,5-trifluoro-1,1-dimethylpentyl)thiophen-2-yl group, a 4-butoxyphenyl group, a 4-difluoromethoxyphenyl group, a 4-(3-difluoromethoxy-1,1-dimethylpropyl)phenyl group, a 4-(3-cyclopropyl-1,1-dimethylpropyl)phenyl group, a 4-(1-methyl-1-butenyl)phenyl group, a 4-(1-methyl-1-pentenyl)phenyl group, a 4-(1-methylcyclopropyl)phenyl group, a 4-(1-ethylcyclopropyl)phenyl group, a 4-(1-propylcyclopropyl)phenyl group, a 4-(1-isopropylcyclopropyl)phenyl group, a 4-(1-butylcyclopropyl)phenyl group, a 2-fluoro-4-(1-butylcyclopropyl)phenyl group, a 3-fluoro-4-(1-butylcyclopropyl)phenyl group, a 5-(1-butylcyclopropyl)thiophen-2-yl group, a 4-(1-nonylcyclopropyl)phenyl group, a 4-(1-methoxycyclopropyl)phenyl group, a 4-(1-benzylcyclopropyl)phenyl group, a 4-{1-(2-phenylethyl)cyclopropyl}phenyl group, a 4-(1-propoxymethylcyclopropyl)phenyl group, a 5-(1-propoxymethylcyclopropyl)thio-phen-2-yl group, a 4-(1-benzyloxymethylcyclopropyl)phenyl group, a 4-(1-ethylcyclobutyl)phenyl group or a 4-(1-butylcyclobutyl)phenyl group,

most preferably a 3,4-diethylphenyl group, a 3-butylphenyl group, a 3-tert-butylphenyl group, a 4-tert-butylphenyl group, a 4-pentylphenyl group, a 3-dimethylamino-4-pentylphenyl group, a 4-neopentylphenyl group, a 4-tert-pentylphenyl group, a 5-tert-pentylthiophen-2-yl group, a 4-(1-ethylpropyl)phenyl group, a 3-hexylphenyl group, a 4-(1-methylpentyl)phenyl group, a 4-(1,1-dimethylbutyl)phenyl group, a 4-(1-ethyl-1-methylpropyl)phenyl group, a 4-(1,1-dimethylpentyl)phenyl group, a 2-fluoro-4-(1,1-dimethylpentyl)phenyl group, a 3-fluoro-4-(1,1-dimethylpentyl)phenyl group, a 5-(1,1-dimethylpentyl)thiophen-2-yl group, a 4-(2,2-dimethylpentyl)phenyl group, a 4-(1,1,3,3-tetramethylbutyl)phenyl group, a 4-(1,1,4-trimethylpentyl)phenyl group, a 4-(1,1-dimethylhexyl)phenyl group, a 4-trifluoromethylphenyl group, a 4-(5,5,5-trifluoro-1,1-dimethylpentyl)phenyl group, a 4-butoxyphenyl group, a 4-difluoromethoxyphenyl group, a 4-(3-difluoromethoxy-1,1-dimethylpropyl)phenyl group, a 4-(1-methyl-1-pentenyl)phenyl group, a 4-(l-methylcyclopropyl)phenyl group, a 4-(1-ethylcyclopropyl)phenyl group, a 4-(1-isopropylcyclopropyl)phenyl group, a 4-(1-butylcyclopropyl)phenyl group, a 4-(1-nonylcyclopropyl)phenyl group, a 4-(1-methoxycyclopropyl)phenyl group, a 4-(1-benzylcyclopropyl)phenyl group, a 4-(1-propoxymethylcyclopropyl)phenyl group, a 4-(1-benzyloxymethylcyclopropyl)phenyl group, a 4-(1-ethylcyclobutyl)phenyl group or a 4-(1-butylcyclobutyl)phenyl group.

The "5- to 6-membered heteroaromatic ring group" shown by Z means the "5-to 6-membered heteroaromatic ring group" having the same meanings as mentioned above, preferably a furyl group, a thienyl group, an imidazolyl group, a thiazolyl group, a pyridyl group, a pyridazinyl group or a pyrimidinyl group, further preferably a furyl group, a thienyl group, an imidazolyl group, a thiazolyl group, a pyridyl group or a pyrimidinyl group, particularly preferably a furyl group, a thienyl group, an imidazolyl group, a thiazolyl group or a pyridyl group.

The substituent(s) for the aromatic ring group or the 5- to 6-membered heteroaromatic ring group shown by Z is preferably a fluoro group, a chloro group, a bromo group, a methyl group, an ethyl group, a propyl group, an isopropyl group, a tert-butyl group, a trifluoromethyl group, a difluoromethyl group, a trichloromethyl group, a dichloromethyl group, a 2,2,2-trifluoroethyl group, a 2,2,2-trichloroethyl group, a methoxy group, an ethoxy group, a propoxy group, an isopropoxy group, a trifluoromethoxy group, a difluoromethoxy group, a trichloromethoxy group or a dichloromethoxy group, further preferably a fluoro group, a chloro group, a methyl group, an ethyl group, a trifluoromethyl group, a methoxy group or a difluoromethoxy group, particularly preferably a fluoro group, a chloro group or a methoxy group.

A number of the substituent(s) on the aromatic ring group or the 5- to 6-membered heteroaromatic ring group shown by Z is, for example, 1 to 4, preferably 1 to 3, particularly preferably 1 or 2, and in case of a plural number, they may be the same or different from each other.

The "aromatic ring group which may be substituted by a group(s) selected from the group consisting of a halogeno group, a $C_1$-$C_6$ alkyl group, a halogeno-$C_1$-$C_6$ alkyl group, a $C_1$-$C_6$ alkoxy group and a halogeno-$C_1$-$C_6$ alkoxy group" shown by Z may be mentioned, for example, a phenyl group, a 1-naphthyl group, a 2-naphthyl group, a 2-fluorophenyl group, a 3-fluorophenyl group, a 4-fluorophenyl group, a 3-fluoro-1-naphthyl group, a 4-fluoro-1-naphthyl group, a 4-fluoro-2-naphthyl group, a 2,3-difluorophenyl group, a 2,4-difluorophenyl group, a 2,5-difluorophenyl group, a 2,6-difluorophenyl group, a 3,4-difluorophenyl group, a 3,5-difluorophenyl group, a 2,3,4-trifluorophenyl group, a 2,3,5-trifluorophenyl group, a 2,3,6-trifluorophenyl group, a 2,4,5-trifluorophenyl group, a 2,4,6-trifluorophenyl group, a 3,4,5-trifluorophenyl group, a 2,3,4,5,6-pentafluorophenyl group, a 2-chlorophenyl group, a 3-chlorophenyl group, a 4-chlorophenyl group, a 3-chloro-1-naphthyl group, a 4-chloro-1-naphthyl group, a 4-chloro-2-naphthyl group, a 2,3-dichlorophenyl group, a 2,4-dichlorophenyl group, a 2,5-dichlorophenyl group, a 2,6-dichlorophenyl group, a 3,4-dichlorophenyl group, a 3,5-dichlorophenyl group, a 4-chloro-2-fluorophenyl group, a 4-chloro-3-fluorophenyl group, a 4-chloro-2,3-difluorophenyl group, a 4-chloro-2,5-difluorophenyl group, a 4-chloro-2,6-difluorophenyl group, a 4-chloro-3,5-difluorophenyl group, a 3-bromophenyl group, a 4-bromophenyl group, a 4-iodophenyl group, a 2-methylphenyl group, a 3-methylphenyl group, a 4-methylphenyl group, a 3-fluoro-4-methylphenyl group, a 2-ethylphenyl group, a 3-ethylphenyl group, a 4-ethylphenyl group, a 4-ethyl-3-fluorophenyl group, a 3-propylphenyl group, a 4-propylphenyl group, a 3-isopropylphenyl group, a 4-isopropylphenyl group, a 4-butylphenyl group, a 4-isobutylphenyl group, a 4-sec-butylphenyl group, a 3-tert-butylphenyl group, a 4-tert-butylphenyl group, a 4-pentylphenyl group, a 4-hexylphenyl group, a 2-trifluoromethylphenyl group, a 3-trifluoromethylphenyl group, a 4-trifluoromethylphenyl group, a 3-fluoro-4-trifluoromethylphenyl group, a 3-difluoromethylphenyl group, a 4-difluoromethylphenyl group, a 3-trichloromethylphenyl group, a 4-trichloromethylphenyl group, a 3-dichloromethylphenyl group, a 4-dichloromethylphenyl group, a 3-(2,2,2-trifluoroethyl)phenyl group, a 4-(2,2,2-trifluoroethyl)phenyl group, a 3-(2,2,2-trichloroethyl)phenyl group, a 4-(2,2,2-trichloroethyl)phenyl group, a 2-methoxyphenyl group, a 3-methoxyphenyl group, a 4-methoxyphenyl group, a 3-methoxy-1-naphthyl group, a 4-ethoxy-1-naphthyl group, a 4-methoxy-2-naphthyl group, a 2-fluoro-4-methoxyphenyl group, a 3-fluoro-4-methoxyphenyl group, a 2,3-difluoro-4-methoxyphenyl group, a 2,5-difluoro-4-methoxyphenyl group, a 2,6-difluoro-4-methoxyphenyl group, a 3,5-difluoro-4-methoxyphenyl group, a 3-ethoxyphenyl group, a 4-ethoxyphenyl group, a 3-propoxyphenyl group, a 4-propoxyphenyl group, a 3-isopropoxyphenyl group, a 4-isopropoxyphenyl group, a 4-butoxyphenyl group, a 4-isobutoxyphenyl group, a 4-sec-butoxyphenyl group, a 3-tert-butoxyphenyl group, a 4-tert-butoxyphenyl group, a 4-pentyloxyphenyl group, a 4-hexyloxyphenyl group, a 3-trifluoromethoxyphenyl group, a 4-trifluoromethoxyphenyl group, a 2-difluoromethoxyphenyl group, a 3-difluoromethoxyphenyl group, a 4-difluoromethoxyphenyl group, a 4-difluoromethoxy-3-fluorophenyl group, a 3-trichloromethoxyphenyl group, a 4-trichloromethoxyphenyl group, a 3-dichloromethoxyphenyl group or a 4-dichloromethoxyphenyl group, etc.,

preferably a phenyl group, a 2-fluorophenyl group, a 3-fluorophenyl group, a 4-fluorophenyl group, a 3,4-difluorophenyl group, a 3,5-difluorophenyl group, a 3,4,5-trifluorophenyl group, a 2-chlorophenyl group, a 3-chlorophenyl group, a 4-chlorophenyl group, a 2,6-dichlorophenyl group, a 4-chloro-3-fluorophenyl group, a 4-chloro-3,5-difluorophenyl group, a 4-bromophenyl group, a 4-methylphenyl group, a 3-fluoro-4-methylphenyl group, a 4-ethylphenyl group, a 4-ethyl-3-fluorophenyl group, a 4-propylphenyl group, a 4-isopropylphenyl group, a 4-tert-butylphenyl group, a 4-trifluoromethylphenyl group, a 3-fluoro-4-trifluoromethylphenyl group, a 4-difluoromethylphenyl group, a 4-trichloromethylphenyl group, a 4-dichloromethylphenyl group, a 4-(2,2,2-trifluoroethyl)phenyl group, a 4-(2,2,2-trichloroethyl)phenyl group, a 4-methoxyphenyl group, a 3-fluoro-4-methoxyphenyl group, a 4-ethoxyphenyl group, a 4-propoxyphenyl group, a 4-isopropoxyphenyl group, a 4-trifluoromethoxyphenyl group, a 4-difluoromethoxyphenyl group, a 4-difluoromethoxy-3-fluorophenyl group, a 4-trichloromethoxyphenyl group or a 4-dichloromethoxyphenyl group, further preferably a phenyl group, a 2-fluorophenyl group, a 3-fluorophenyl group, a 4-fluorophenyl group, a 3,4-difluorophenyl group, a 3,5-difluorophenyl group, a 2-chlorophenyl group, a 3-chlorophenyl group, a 4-chlorophenyl group, a 2,6-dichlorophenyl group, a 4-chloro-3-fluorophenyl group, a 4-methylphenyl group, a 3-fluoro-4-methylphenyl group, a 4-ethylphenyl group, a 4-ethyl-3-fluorophenyl group, a 4-trifluoromethylphenyl group, a 3-fluoro-4-trifluoromethylphenyl group, a 4-methoxyphenyl group, a 3-fluoro-4-methoxyphenyl group, a 4-difluoromethoxyphenyl group or a 4-difluoromethoxy-3-fluorophenyl group, particularly preferably a phenyl group, a 2-fluorophenyl group, a 3-fluorophenyl group, a 4-fluorophenyl group, a 3-chlorophenyl group, a 4-chlorophenyl group or a 4-methoxyphenyl group.

The "5- to 6-membered heteroaromatic ring group which may be substituted by a group(s) selected from the group consisting of a halogeno group, a $C_1$-$C_6$ alkyl group, a halogeno-$C_1$-$C_6$ alkyl group, a $C_1$-$C_6$ alkoxy group and a halogeno-$C_1$-$C_6$ alkoxy group" shown by Z may be mentioned, for example, a pyrrol-1-yl group, a furan-2-yl group, a furan-3-yl group, a 5-methylfuran-2-yl group, a thiophen-2-yl group, a thiophen-3-yl group, a 5-fluorothiophen-2-yl group, a 5-chlorothiophen-2-yl group, a 5-methylthiophen-2-yl group, a 5-ethylthiophen-2-yl group, a 5-trifluoromethylthiophen-2-yl group, a 5-methoxythiophen-2-yl group, a 5-difluoromethoxythiophen-2-yl group, a pyrazol-1-yl group, a 1-methyl-1H-imidazol-4-yl group, a 1-ethyl-1H-imidazol-4-yl group, a oxazol-2-yl group, a thiazol-2-yl group, a pyridin-2-yl group, a 5-fluoropyridin-2-yl group, a 5-chloropyridin-2-yl group, a 5-bromopyridin-2-yl group, a 5-methylpyridin-2-yl group, a 5-ethylpyridin-2-yl group, a 5-propylpyridin-2-yl group, a 5-isopropylpyridin-2-yl group, a 5-butylpyridin-2-yl group, a 5-isobutylpyridin-2-yl group, a 5-sec-butylpyridin-2-yl group, a 5-tert-butylpyridin-2-yl group, a 5-pentylpyridin-2-yl group, a 5-hexylpyridin-2-yl group, a 5-trifluoromethylpyridin-2-yl group, a 5-difluoromethylpyridin-2-yl group, a 5-trichloromethylpyridin-2-yl group, a 5-dichloromethylpyridin-2-yl group, a 5-(2,2,2-trifluoroethyl)pyridin-2-yl group, a 5-(2,2,2-trichloroethyl)pyridin-2-yl group, a 5-methoxypyridin-2-yl group, a 5-ethoxypyridin-2-yl group, a 5-propoxypyridin-2-yl group, a 5-isopropoxypyridin-2-yl group, a 5-tert-butoxypyridin-2-yl group, a 5-trifluoromethoxypyridin-2-yl group, a 5-difluoro-methoxypyridin-2-yl group, a 5-trichloromethoxypyridin-2-yl group, a 5-dichloro-methoxypyridin-2-yl group, a pyridin-3-yl group, a 6-fluoropyridin-3-yl group, a 6-chloropyridin-3-yl group, a 6-bromopyridin-3-yl group, a 6-methylpyridin-3-yl group, a 6-ethylpyridin-3-yl group, a 6-propylpyridin-3-yl group, a 6-isopropylpyridin-3-yl group, a 6-butylpyridin-3-yl group, a 6-isobutylpyridin-3-yl group, a 6-sec-butylpyridin-3-yl group, a 6-tert-butylpyridin-3-yl group, a 6-pentylpyridin-3-yl group, a 6-hexylpyridin-3-yl group, a 6-trifluoromethylpyridin-3-yl group, a 6-difluoromethylpyridin-3-yl group, a 6-trichloromethylpyridin-3-yl group, a 6-dichloromethylpyridin-3-yl group, a 6-(2,2,2-trifluoroethyl)pyridin-3-yl group, a 6-(2,2,2-trichloroethyl)pyridin-3-yl group, a 6-methoxypyridin-3-yl group, a 6-ethoxypyridin-3-yl group, a 6-propoxypyridin-3-yl group, a 6-isopropoxypyridin-3-yl group, a 6-tert-butoxypyridin-3-yl group, a 6-tri-fluoromethoxypyridin-3-yl group, a 6-difluoromethoxypyridin-3-yl group, a 6-trichloromethoxypyridin-3-yl group, a 6-dichloromethoxypyridin-3-yl group, a pyridin-4-yl group, a pyridazin-3-yl group, a pyridazin-4-yl group, a pyrimidin-2-yl group, a pyrimidin-4-yl group, a pyrimidin-5-yl group or a pyrazin-2-yl group, etc.,

preferably a furan-2-yl group, a 5-methylfuran-2-yl group, a thiophen-2-yl group, a thiophen-3-yl group, a 5-chlorothiophen-2-yl group, a 1-methyl-1H-imidazol-4-yl group, a thiazol-2-yl group, a pyridin-2-yl group, a 5-fluoropyridin-2-yl group, a 5-chloropyridin-2-yl group, a 5-methylpyridin-2-yl group, a 5-ethylpyridin-2-yl group, a 5-trifluoromethylpyridin-2-yl group, a 5-methoxypyridin-2-yl group, a 5-difluoromethoxypyridin-2-yl group, a pyridin-3-yl group, a 6-fluoropyridin-3-yl group, a 6-chloropyridin-3-yl group, a 6-methylpyridin-3-yl group, a 6-ethylpyridin-3-yl group, a 6-trifluoromethylpyridin-3-yl group, a 6-methoxypyridin-3-yl group, a 6-difluoromethoxypyridin-3-yl group, a pyridin-4-yl group or a pyrimidin-2-yl group, further preferably a furan-2-yl group, a 5-methylfuran-2-yl group, a thiophen-2-yl group, a thiophen-3-yl group, a 1-methyl-1H-imidazol-4-yl group, a thiazol-2-yl group, a pyridin-2-yl group, a 5-fluoropyridin-2-yl group, a 5-chloropyridin-2-yl group, a 5-methoxypyridin-2-yl group, a pyridin-3-yl group, a 6-fluoropyridin-3-yl group, a 6-chloropyridin-3-yl group, a 6-methoxypyridin-3-yl group or a pyridin-4-yl group, particularly preferably a furan-2-yl group, a 5-methylfuran-2-yl group, a thiophen-2-yl group, a 1-methyl-1H-imidazol-4-yl group, a thiazol-2-yl group, a pyridin-2-yl group, a pyridin-3-yl group or a pyridin-4-yl group.

Z is preferably a phenyl group, a furyl group, a thienyl group, an imidazolyl group, a thiazolyl group, a pyridyl group or a pyrimidinyl group each of which may be substituted by a group(s) selected from the group consisting of a halogeno group, a $C_1$-$C_4$ alkyl group, a halogeno-$C_1$-$C_4$ alkyl group, a $C_1$-$C_4$ alkoxy group and a halogeno-$C_1$-$C_4$ alkoxy group, further preferably a phenyl group, a furyl group, a thienyl group, an imidazolyl group, a thiazolyl group, a pyridyl group or a pyrimidinyl group each of which may be substituted by a group(s) selected from the group consisting of a fluoro group, a chloro group, a bromo group, a methyl group, an ethyl group, a propyl group, an isopropyl group, a tert-butyl group, a trifluoromethyl group, a difluoromethyl group, a trichloromethyl group, a dichloromethyl group, a 2,2,2-trifluoroethyl group, a 2,2,2-trichloroethyl group, a methoxy group an ethoxy group, a propoxy group, an isopropoxy group, a trifluoromethoxy group, a difluoromethoxy group, a trichloromethoxy group and a dichloromethoxy group,

further more preferably a phenyl group, a 2-fluorophenyl group, a 3-fluorophenyl group, a 4-fluorophenyl group, a 3,4-difluorophenyl group, a 3,5-difluorophenyl group, a 3,4,5-trifluorophenyl group, a 2-chlorophenyl group, a 3-chlorophenyl group, a 4-chlorophenyl group, a 2,6-dichlorophenyl group, a 4-chloro-3-fluorophenyl group, a 4-chloro-3,5-difluorophenyl group, a 4-bromophenyl group, a 4-methylphenyl group, a 3-fluoro-4-methylphenyl group, a 4-ethylphenyl group, a 4-ethyl-3-fluorophenyl group, a 4-propylphenyl group, a 4-isopropylphenyl group, a 4-tert-butylphenyl group, a 4-trifluoromethylphenyl group, a 3-fluoro-4-trifluoromethylphenyl group, a 4-difluoromethylphenyl group, a 4-trichloromethylphenyl group, a 4-dichloromethylphenyl group, a 4-(2,2,2-trifluoroethyl)phenyl group, a 4-(2,2,2-trichloroethyl)phenyl group, a 4-methoxyphenyl group, a 3-fluoro-4-methoxyphenyl group, a 4-ethoxyphenyl group, a 4-propoxyphenyl group, a 4-isopropoxyphenyl group, a 4-trifluoromethoxyphenyl group, a 4-difluoromethoxyphenyl group, a 4-difluoromethoxy-3-fluorophenyl group, a 4-trichloromethoxyphenyl group, a 4-dichloromethoxyphenyl group, a furan-2-yl group, a 5-methylfuran-2-yl group, a thiophen-2-yl group, a thiophen-3-yl group, a 5-chlorothiophen-2-yl group, a 1-methyl-1H-imidazol-4-yl group, a thiazol-2-yl group, a pyridin-2-yl group, a 5-fluoropyridin-2-yl group, a 5-chloropyridin-2-yl group, a 5-methylpyridin-2-yl group, a 5-ethylpyridin-2-yl group, a 5-trifluoromethylpyridin-2-yl group, a 5-methoxypyridin-2-yl group, a 5-difluoromethoxypyridin-2-yl group, a pyridin-3-yl group, a 6-fluoropyridin-3-yl group, a 6-chloropyridin-3-yl group, a 6-methylpyridin-3-yl group, a 6-ethylpyridin-3-yl group, a 6-trifluoromethylpyridin-3-yl group, a 6-methoxypyridin-3-yl group, a 6-difluoromethoxypyridin-3-yl group, a pyridin-4-yl group or a pyrimidin-2-yl group,

particularly preferably a phenyl group, a 2-fluorophenyl group, a 3-fluorophenyl group, a 4-fluorophenyl group, a 3,4-difluorophenyl group, a 3,5-difluorophenyl group, a 2-chlorophenyl group, a 3-chlorophenyl group, a 4-chlorophenyl group, a 2,6-dichlorophenyl group, a 4-chloro-3-fluorophenyl group, a 4-methylphenyl group, a 3-fluoro-4-methylphenyl group, a 4-trifluoromethylphenyl group, a 3-fluoro-4-trifluoromethylphenyl group, a 4-methoxyphenyl group, a 3-fluoro-4-methoxyphenyl group, a 4-difluoromethoxyphenyl group, a 4-difluoromethoxy-3-fluorophenyl group, a furan-2-yl group, a 5-methylfuran-2-yl group, a thiophen-2-yl group, a thiophen-3-yl group, a 1-methyl-1H-imidazol-4-yl group, a thiazol-2-yl group, a pyridin-2-yl group, a 5-fluoropyridin-2-yl group, a 5-chloropyridin-2-yl group, a 5-methoxypyridin-2-yl group, a pyridin-3-yl group, a 6-fluoropyridin-3-yl group, a 6-chloropyridin-3-yl group, a 6-methoxypyridin-3-yl group or a pyridin-4-yl group, most preferably a phenyl group, a 2-fluorophenyl group, a 3-fluorophenyl group, a 4-fluorophenyl group, a 4-chlorophenyl group, a 4-methoxyphenyl group, a furan-2-yl group, a 5-methylfuran-2-yl group, a thiophen-2-yl group, a 1-methyl-1H-imidazol-4-yl group, a thiazol-2-yl group, a pyridin-2-yl group, a pyridin-3-yl group or a pyridin-4-yl group.

In the substituent(s) mentioned in the present invention, each atom or each ring is also contained.
When there is a geometric isomer or an optical isomer in the compound represented by the formula (I) of the present invention, such isomers are also included in the scope of the present invention, and when there is a proton tautomer, such tautomer is also included in the present invention.
The compound represented by the formula (I) of the present invention is easily converted into a pharmaceutically acceptable salt by treating it with an acid. Examples of such a salt include, for example, inorganic acid salts such as hydrochloride, hydrobromide, hydroiodide, nitrate, sulfate and phosphate; or organic acid salts such as acetate, trifluoroacetate, benzoate, oxalate, malonate, succinate, maleate, fumarate, tartrate, citrate, methanesulfonate, ethanesulfonate, trifluoromethanesulfonate, benzenesulfonate, p-toluenesulfonate, glutamate and aspartate, preferably a hydrochloride or hydrobromide.
The compound represented by the formula (I) of the present invention is easily converted into a pharmaceutically acceptable basic salt by treating it with a base when $R^1$ is a hydrogen atom. Examples of such a salt include, for example, metal salts such as a sodium salt, a potassium salt, a calcium salt or a magnesium salt: inorganic salts such as an ammonium salt: or organic amine salts such as a triethylamine salt and a guanidine salt.
Further, the compound or a pharmaceutically acceptable salt thereof represented by the formula (I) of the present invention can be present as a hydrate or solvate, and they are also included in the present invention.
The compound represented by the formula (I) of the present invention is, preferably,

(1) a compound wherein $R^1$ is a hydrogen atom, a methyl group, an ethyl group, a propyl group, an isopropyl group or a tert-butyl group,
(2) a compound wherein $R^1$ is a hydrogen atom, a methyl group, an ethyl group or an isopropyl group,
(3) a compound wherein $R^1$ is a hydrogen atom or an ethyl group,
(4) a compound wherein $R^2$ and $R^3$ are each independently a hydrogen atom, a methyl group, an ethyl group, a propyl group or an isopropyl group,
(5) a compound wherein $R^2$ and $R^3$ are each independently a hydrogen atom or a methyl group,
(6) a compound wherein $R^2$ and $R^3$ are both hydrogen atom,

(7) a compound wherein Y is the formula (II):
[Formula 10]

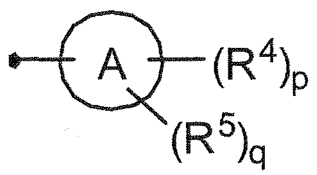

(II)

(wherein Ring A represents a phenyl group, a thienyl group, a thiazolyl group, a pyridyl group or a pyrimidinyl group, $R^4$ represents a $C_1$-$C_8$ alkyl group, a fluoro-$C_1$-$C_8$ alkyl group, a $C_1$-$C_4$ alkoxy group, a halogeno-$C_1$-$C_4$ alkoxy group, a fluoro-$C_1$-$C_4$ alkoxy-$C_3$-$C_6$ alkyl group, a $C_3$-$C_4$ cycloalkyl-$C_3$-$C_6$ alkyl group, a $C_3$-$C_6$ alkenyl group or the formula (III):
[Formula 11]

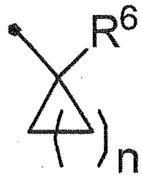

(III)

(wherein $R^6$ represents a $C_1$-$C_{10}$ alkyl group, a $C_1$-$C_4$ alkoxy group, a $C_7$-$C_{12}$ aralkyl group, a $C_1$-$C_4$ alkoxy-$C_1$-$C_4$ alkyl group or a $C_7$-$C_{12}$ aralkyloxy-$C_1$-$C_4$ alkyl group, and n is an integer of 1 to 3.), p is an integer of 1 or 2, $R^5$ represents a fluoro group, a chloro group or an amino group which may be substituted by a $C_1$-$C_3$ alkyl group, and q is an integer

of 0 or 1.),

(8) a compound wherein Y is the formula (II):

[Formula 12]

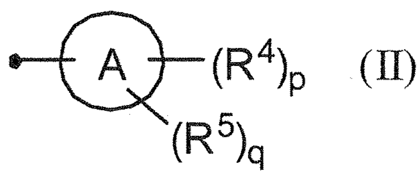

(wherein Ring A represents a phenyl group, a thienyl group, a thiazolyl group, a pyridyl group or a pyrimidinyl group, $R^4$ represents an ethyl group, a propyl group, an isopropyl group, a butyl group, a sec-butyl group, a tert-butyl group, a pentyl group, an isopentyl group, a neopentyl group, a tert-pentyl group, a 1-methylbutyl group, a 1-ethylpropyl group, a hexyl group, a 1-methylpentyl group, a 1-ethylbutyl group, a 1,1-dimethylbutyl group, a 2,2-dimethylbutyl group, a 1-ethyl-1-methylpropyl group, a 1-methylhexyl group, a 1,1-dimethylpentyl group, a 2,2-dimethylpentyl group, a 1,1-dimethylhexyl group, a 1,1,3,3-tetramethylbutyl group, a 1,1,4-trimethylpentyl group, a trifluoromethyl group, a 4,4,4-trifluorobutyl group, a 5,5,5-trifluoropentyl group, a 6,6,6-trifluorohexyl group, a 5,5,5-trifluoro-1-methylpentyl group, a 4,4,4-trifluoro-1,1-dimethylbutyl group, a 5,5,5-trifluoro-1,1-dimethylpentyl group, a 5,5,5-trifluoro-2,2-dimethylpentyl group, a propoxy group, a butoxy group, an isobutoxy group, a trifluoromethoxy group, a difluoromethoxy group, a trichloromethoxy group, a dichloromethoxy group, a 3-difluoromethoxypropyl group, a 3-difluoromethoxy-1-methylpropyl group, a 3-difluoromethoxy-1,1-dimethylpropyl group, a 4-difluoromethoxy-1,1-dimethylbutyl group, a 3-cyclopropylpropyl group, a 3-cyclopropyl-1-methylpropyl group, a 3-cyclopropyl-1,1-dimethylpropyl group, a 4-cyclopropyl-1,1-dimethylbutyl group, an isopropenyl group, a 1-methyl-1-propenyl group, a 1-methyl-1-butenyl group, a 1-methyl-1-pentenyl group or the formula (III):

[Formula 13]

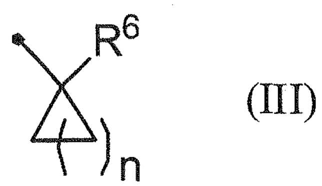

(wherein $R^6$ represents a methyl group, an ethyl group, a propyl group, an isopropyl group, a butyl group, an isobutyl group, a sec-butyl group, a tert-butyl group, a pentyl group, a hexyl group, a heptyl group, an octyl group, a nonyl group, a decyl group, a methoxy group, an ethoxy group, a propoxy group, an isopropoxy group, a benzyl group, a 1-phenylethyl group, a 2-phenylethyl group, a naphthalen-1-ylmethyl group, a naphthalen-2-ylmethyl group, an ethoxymethyl group, a propoxymethyl group, a butoxymethyl group, a 2-ethoxyethyl group, a 2-propoxyethyl group, a benzyloxymethyl group, a 2-benzyloxyethyl group, a 1-phenylethoxymethyl group, a 2-phenylethoxymethyl group, a 1-(1-phenylethoxy)ethyl group or a 2-(2-phenylethoxy)ethyl group, and n is an integer of 1 or 2.), p is an integer of 1 or 2, $R^5$ represents a fluoro group, a chloro group, an amino group, a methylamino group, a dimethylamino group or an ethylamino group, and q is an integer of 0 or 1.),

(9) a compound wherein Y is a 4-ethylphenyl group, a 3,4-diethylphenyl group, a 4-propylphenyl group, a 4-isopropylphenyl group, a 3-butylphenyl group, a 4-butylphenyl group, a 3-tert-butylphenyl group, a 4-tert-butylphenyl group, a 5-tert-butylthiophen-2-yl group, a 3-pentylphenyl group, a 4-pentylphenyl group, a 3-dimethylamino-4-pentylphenyl group, a 4-isopentylphenyl group, a 4-neopentylphenyl group, a 4-tert-pentylphenyl group, a 5-tert-pentylthiophen-2-yl group, a 4-(1-ethylpropyl)phenyl group, a 4-(1-methylbutyl)phenyl group, a 3-hexylphenyl group, a 4-(1-ethylbutyl)phenyl group, a 4-(1-methylpentyl)phenyl group, a 5-(1-methylpentyl)thiophen-2-yl group, a 3-(1,1-dimethylbutyl)phenyl group, a 4-(1,1-dimethylbutyl)phenyl group, a 2-fluoro-4-(1,1-dimethylbutyl)phenyl group, a 3-fluoro-4-(1,1-dimethylbutyl)phenyl group, a 4-(2,2-dimethylbutyl)phenyl group, a 5-(1,1-dimethylbutyl)thiophen-2-yl group, a 4-(1-ethyl-1-methylpropyl)phenyl group, a 3-(1,1 -dimethylpentyl)phenyl group, a 4-(1,1-dimethylpentyl)phenyl group, a 2-fluoro-4-(1,1-dimethylpentyl)phenyl group, a 3-fluoro-4-(1,1-dimethylpentyl)phenyl group, a 3-amino-4-(1,1-dimethylpentyl)phenyl group, a 3-methylamino-4-(1,1-dimethylpentyl)phenyl group, a 3-dimethylamino-4-(1,1-dimethylpentyl)phenyl group, a 4-(1,1-dimethylpentyl)thiophen-2-yl group, a 5-(1,1-dimethylpentyl)thiophen-2-yl group, a 5-(1,1-dimethylpentyl)thiazol-2-yl group, a 5-(1,1-dimethylpentyl)pyridin-2-yl group, a 6-(1,1-dimethylpentyl)pyridin-3-yl group, a 5-(1,1-dimethylpentyl)pyrimidin-2-yl group, a 2-(1,1-dimethylpentyl)pyrimidin-5-yl group, a 3-(2,2-dimethylpentyl)phenyl group, a 4-(2,2-dimethylpentyl)phenyl group, a 4-(1-methylhexyl)phenyl group, a 4-(1,1,3,3-tetramethylbutyl)phenyl group, a 4-(1,1,4-trimethylpentyl)phenyl group, a 4-(1,1-dimethylhexyl)phenyl group, a 4-trifluoromethylphenyl group, a 4-(4,4,4-trifluorobutyl)phenyl group, a 4-(5,5,5-trifluoropentyl)-phenyl group, a 4-(6,6,6-trifluorohexyl)phenyl group, a 4-(5,5,5-trifluoro-1-methylpentyl)phenyl group, a 4-(4,4,4-trifluoro-1,1-dimethylbutyl)phenyl group, a 4-(5,5,5-trifluoro-1,1-dimethylpentyl)phenyl group, a 4-(5,5,5-trifluoro-2,2-dimethylpentyl)-phenyl group, a 5-(5,5,5-trifluoro-1,1-dimethylpentyl)thiophen-2-yl group, a 4-propoxyphenyl group, a 4-butoxyphenyl group, a 4-isobutoxyphenyl group, a 4-difluoromethoxyphenyl group, a 4-trifluoromethoxyphenyl group, a 4-(3-difluoromethoxy-1-methylpropyl)phenyl group, a 4-(3-difluoromethoxy-1,1-dimethylpropyl)phenyl group, a 4-(3-cyclopropyl-1-methylpropyl)phenyl group, a 4-(3-cyclopropyl-1,1-dimethylpropyl)phenyl group, a 4-isopropenylphenyl group, a 4-(1-methyl-1-propenyl)phenyl group, a 4-(1-methyl-1-butenyl)phenyl group, a 4-(1-methyl-1-pentenyl)phenyl group, a 4-(1-methylcyclopropyl)phenyl group, a 4-(1-ethylcyclopropyl)phenyl group, a 4-(1-propylcyclopropyl)phenyl group, a 4-(1-isopropylcyclopropyl)phenyl group, a 3-(1-butylcyclopropyl)phenyl group, a 4-(1-butylcyclopropyl)phenyl group, a 2-fluoro-4-(1-butylcyclopropyl)phenyl group, a 3-fluoro-4-(1-butylcyclopropyl)phenyl group, a 3-dimethylamino-4-(1-butylcyclopropyl)phenyl group, a 5-(1-butylcyclopropyl)thiophen-2-yl group, a 4-(1-hexylcyclopropyl)phenyl group, a 4-(-nonylcyclopropyl)phenyl group, a 4-(1-methoxycyclopropyl)phenyl group, a 4-(1-ethoxycyclopropyl)phenyl group, a 4-(1-propoxycyclopropyl)phenyl group, a 4-(1-benzylcyclopropyl)phenyl group, a 4-{1-(1-phenylethyl)cyclopropyl)phenyl group, a4-{1-(2-phenylethyl)cyclopropyl}phenyl group, a 4-(1-ethoxymethylcyclopropyl)phenyl group, a 4-(1-propoxymethylcyclopropyl)phenyl group, a 5-(1-propoxymethylcyclopropyl)thiophen-2-yl group, a 4- {1-(2-ethoxyethyl)cyclopropyl }phenyl group, a 4-{1-(2-propoxyethyl)-cyclopropyl}phenyl group, a 4-(1-butoxymethylcyclopropyl)phenyl group, a 4-(1-benzyloxymethylcyclopropyl)phenyl group, a 4-{1-(2-benzyloxyethyl)cyclopropyl}-phenyl group, a 4-{1-(1-phenylethoxymethyl)cyclopropyl}phenyl group, a 4-{1-(2-phenylethoxymethyl)cyclopropyl}phenyl group, a 4-(1-ethylcyclobutyl)phenyl group, a 4-(1-butylcyclobutyl)phenyl group or a 5-(1-butylcyclobutyl)thiophen-2-yl group,

(10) a compound wherein Y is a 3,4-diethylphenyl group, a 3-butylphenyl group, a 4-butylphenyl group, a 3-tert-butylphenyl group, a 4-tert-butylphenyl group, a 5-tert-butylthiophen-2-yl group, a 4-pentylphenyl group, a 3-dimethylamino-4-pentylphenyl group, a 4-isopentylphenyl group, a 4-neopentylphenyl group, a 4-tert-pentylphenyl group, a 5-tert-pentylthiophen-2-yl group, a 4-(1-ethylpropyl)phenyl group, a 4-(1-methylbutyl)phenyl group, a 3-hexylphenyl group, a 4-(1-methylpentyl)phenyl group, a 5-(1-methylpentyl)thiophen-2-yl group, a 3-(1,1-dimethylbutyl)phenyl group, a 4-(1,1-dimethylbutyl)phenyl group, a 2-fluoro-4-(1,1-dimethylbutyl)phenyl group, a 3-fluoro-4-(1,1-dimethylbutyl)phenyl group, a 5-(1,1-dimethylbutyl)thiophen-2-yl group, a 4-(1-ethyl-1-methylpropyl)phenyl group, a 3-(1,1-dimethylpentyl) phenyl group, a 4-(1,1-dimethylpentyl)phenyl group, a 2-fluoro-4-(1,1-dimethylpentyl)phenyl group, a 3-fluoro-4-(1,1-dimethylpentyl)phenyl group, a 3-dimethylamino-4-(1,1-dimethylpentyl)-phenyl group, a 5-(1,1-dimethylpentyl)thiophen-2-yl group, a 4-(2,2-dimethylpentyl)-phenyl group, a 4-(1,1,3,3-tetramethylbutyl)phenyl group, a 4-(1,1,4-trimethylpentyl)-phenyl group, a 4-(1,1-dimethylhexyl)phenyl group, a 4-trifluoromethylphenyl group, a 4-(4,4,4-trifluoro-1,1-dimethylbutyl)phenyl group, a 4-(5,5,5-trifluoro-1,1-dimethylpentyl)phenyl group, a 5-(5,5,5-trifluoro-1,1-dimethylpentyl)thiophen-2-yl group, a 4-butoxyphenyl group, a 4-difluoromethoxyphenyl group, a 4-(3-difluoromethoxy-1,1-dimethylpropyl)phenyl group, a 4-(3-cyclopropyl-1,1-dimethylpropyl)phenyl group, a 4-(1-methyl-1-butenyl)phenyl group, a 4-(1-methyl-1-pentenyl)phenyl group, a 4-(1-methylcyclopropyl)phenyl group, a 4-(1-ethylcyclopropyl)phenyl group, a 4-(1-propylcyclopropyl)phenyl group, a 4-(1-isopropylcyclopropyl)phenyl group, a 4-(1-butylcyclopropyl)phenyl group, a 2-fluoro-4-(1-butylcyclopropyl)phenyl group, a 3-fluoro-4-(1-butylcyclopropyl)phenyl group, a 5-(1-butylcyclopropyl)thiophen-2-yl group, a 4-(1-nonylcyclopropyl)phenyl group, a 4-(1-methoxycyclopropyl)phenyl group, a 4-(1-benzylcyclopropyl)phenyl group, a 4-{1-(2-phenylethyl)cyclopropyl}phenyl group, a 4-(1-propoxymethylcyclopropyl)phenyl group, a 5-(1-propoxymethylcyclopropyl)-thiophen-2-yl group, a 4-(1-benzyloxymethylcyclopropyl)phenyl group, a 4-(1-ethylcyclobutyl)phenyl group or a 4-(1-butylcyclobutyl)phenyl group,

(11) a compound wherein Y is a 3,4-diethylphenyl group, a 3-butylphenyl group, a 3-tert-butylphenyl group, a 4-tert-butylphenyl group, a 4-pentylphenyl group, a 3-dimethylamino-4-pentylphenyl group, a 4-neopentylphenyl group, a 4-tert-pentylphenyl group, a 5-tert-pentylthiophen-2-yl group, a 4-(1-ethylpropyl)phenyl group, a 3-hexylphenyl group, a 4-(1-methylpentyl)phenyl group, a 4-(1,1-dimethylbutyl)phenyl group, a 4-(1-ethyl-1-methylpropyl)phenyl group, a 4-(1,1-dimethylpentyl)phenyl group, a 2-fluoro-4-(1,1-dimethylpentyl)phenyl group, a 3-fluoro-4-(1,1-dimethylpentyl)phenyl group, a 5-(1,1-dimethylpentyl)thiophen-2-yl group, a 4-(2,2-dimethylpentyl)phenyl group, a 4-(1,1,3,3-tetramethylbutyl)phenyl group, a 4-(1,1,4-trimethylpentyl)phenyl group, a 4-(1,1-dimethylhexyl)phenyl group, a 4-trifluoromethylphenyl group, a 4-(5,5,5-trifluoro-1,1-dimethylpentyl)phenyl group, a 4-butoxyphenyl group, a 4-difluoromethoxyphenyl group, a 4-(3-difluoromethoxy-1,1-dimethylpropyl)phenyl group, a 4-(1-methyl-1-pentenyl)phenyl group, a 4-(1-methylcyclopropyl)phenyl group, a 4-(1-ethylcyclopropyl)phenyl group, a 4-(1-isopropylcyclopropyl)phenyl group, a 4-(1-butylcyclopropyl)phenyl group, a 4-(1-nonylcyclopropyl)phenyl group, a 4-(1-methoxycyclopropyl)phenyl group, a 4-(1-benzylcyclopropyl)phenyl group, a 4-(1-propoxymethylcyclopropyl)phenyl group, a 4-(1-benzyloxymethylcyclopropyl)phenyl

group, a 4-(1-ethylcyclobutyl)phenyl group or a 4-(1-butylcyclobutyl)phenyl group,

(12) a compound wherein Z is a phenyl group, a furyl group, a thienyl group, an imidazolyl group, a thiazolyl group, a pyridyl group or a pyrimidinyl group each of which may be substituted by a group(s) selected from the group consisting of a halogeno group, a $C_1$-$C_4$ alkyl group, a halogeno-$C_1$-$C_4$ alkyl group, a $C_1$-$C_4$ alkoxy group and a halogeno-$C_1$-$C_4$ alkoxy group,

(13) a compound wherein Z is a phenyl group, a furyl group, a thienyl group, an imidazolyl group, a thiazolyl group, a pyridyl group or a pyrimidinyl group each of which may be substituted by a group(s) selected from the group consisting of a fluoro group, a chloro group, a bromo group, a methyl group, an ethyl group, a propyl group, an isopropyl group, a tert-butyl group, a trifluoromethyl group, a difluoromethyl group, a trichloromethyl group, a dichloromethyl group, a 2,2,2-trifluoroethyl group, a 2,2,2-trichloroethyl group, a methoxy group, an ethoxy group, a propoxy group, an isopropoxy group, a trifluoromethoxy group, a difluoromethoxy group, a trichloromethoxy group and a dichloromethoxy group,

(14) a compound wherein Z is a phenyl group, a 2-fluorophenyl group, a 3-fluorophenyl group, a 4-fluorophenyl group, a 3,4-difluorophenyl group, a 3,5-difluorophenyl group, a 3,4,5-trifluorophenyl group, a 2-chlorophenyl group, a 3-chlorophenyl group, a 4-chlorophenyl group, a 2,6-dichlorophenyl group, a 4-chloro-3-fluorophenyl group, a 4-chloro-3,5-difluorophenyl group, a 4-bromophenyl group, a 4-methylphenyl group, a 3-fluoro-4-methylphenyl group, a 4-ethylphenyl group, a 4-ethyl-3-fluorophenyl group, a 4-propylphenyl group, a 4-isopropylphenyl group, a 4-tert-butylphenyl group, a 4-trifluoromethylphenyl group, a 3-fluoro-4-trifluoromethylphenyl group, a 4-difluoromethylphenyl group, a 4-trichloromethylphenyl group, a 4-dichloromethylphenyl group, a 4-(2,2,2-trifluoroethyl)phenyl group, a 4-(2,2,2-trichloroethyl)phenyl group, a 4-methoxyphenyl group, a 3-fluoro-4-methoxyphenyl group, a 4-ethoxyphenyl group, a 4-propoxyphenyl group, a 4-isopropoxyphenyl group, a 4-trifluoromethoxyphenyl group, a 4-difluoromethoxyphenyl group, a 4-difluoromethoxy-3-fluorophenyl group, a 4-trichloromethoxyphenyl group, a 4-dichloromethoxyphenyl group, a furan-2-yl group, a 5-methylfuran-2-yl group, a thiophen-2-yl group, a thiophen-3-yl group, a 5-chlorothiophen-2-yl group, a 1-methyl-1H-imidazol-4-yl group, a thiazol-2-yl group, a pyridin-2-yl group, a 5-fluoropyridin-2-yl group, a 5-chloropyridin-2-yl group, a 5-methylpyridin-2-yl group, a 5-ethylpyridin-2-yl group, a 5-trifluoromethylpyridin-2-yl group, a 5-methoxypyridin-2-yl group, a 5-difluoromethoxypyridin-2-yl group, a pyridin-3-yl group, a 6-fluoropyridin-3-yl group, a 6-chloropyridin-3-yl group, a 6-methylpyridin-3-yl group, a 6-ethylpyridin-3-yl group, a 6-trifluoromethylpyridin-3-yl group, a 6-methoxypyridin-3-yl group, a 6-difluoromethoxypyridin-3-y1 group, a pyridin-4-yl group or a pyrimidin-2-yl group,

(15) a compound wherein Z is a phenyl group, a 2-fluorophenyl group, a 3-fluorophenyl group, a 4-fluorophenyl group, a 3,4-difluorophenyl group, a 3,5-difluorophenyl group, a 2-chlorophenyl group, a 3-chlorophenyl group, a 4-chlorophenyl group, a 2,6-dichlorophenyl group, a 4-chloro-3-fluorophenyl group, a 4-methylphenyl group, a 3-fluoro-4-methylphenyl group, a 4-trifluoromethylphenyl group, a 3-fluoro-4-trifluoromethylphenyl group, a 4-methoxyphenyl group, a 3-fluoro-4-methoxyphenyl group, a 4-difluoromethoxyphenyl group, a 4-difluoromethoxy-3-fluorophenyl group, a furan-2-yl group, a 5-methylfuran-2-yl group, a thiophen-2-yl group, a thiophen-3-yl group, a 1-methyl-1H-imidazol-4-yl group, a thiazol-2-yl group, a pyridin-2-yl group, a 5-fluoropyridin-2-yl group, a 5-chloropyridin-2-yl group, a 5-methoxypyridin-2-yl group, a pyridin-3-yl group, a 6-fluoropyridin-3-yl group, a 6-chloropyridin-3-yl group, a 6-methoxypyridin-3-yl group or a pyridin-4-yl group, or,

(16) a compound wherein Z is a phenyl group, a 2-fluorophenyl group, a 3-fluorophenyl group, a 4-fluorophenyl group, a 4-chlorophenyl group, a 4-methoxyphenyl group, a furan-2-yl group, a 5-methylfuran-2-yl group, a thiophen-2-yl group, a 1-methyl-1H-imidazol-4-yl group, a thiazol-2-yl group, a pyridin-2-yl group, a pyridin-3-yl group or a pyridin-4-yl group.

Further, in the above-mentioned groups of (1)-(3); (4)-(6), (7)-(11) and (12)-(16), as the number becomes larger, a more preferred compound is indicated, and a compound obtained by arbitrarily selecting $R^1$ from the groups (1)-(3), $R^2$ and $R^3$ from the groups (4)-(6), Y from the groups (7)-(11), and Z from the groups (12)-(16), or by arbitrarily combining them is also a preferred compound.

Examples of such compound include:

(17) a compound wherein $R^1$ is a hydrogen atom, a methyl group, an ethyl group, a propyl group, an isopropyl group or tert-butyl group,

$R^2$ and $R^3$ are each independently a hydrogen atom or a methyl group,

Y is the formula (II):

[Formula 14]

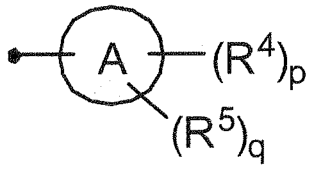 (II)

(wherein Ring A represents a phenyl group, a thienyl group, a thiazolyl group, a pyridyl group or a pyrimidinyl group, $R^4$ represents a $C_1$-$C_8$ alkyl group, a fluoro-$C_1$-$C_8$ alkyl group, a $C_1$-$C_4$ alkoxy group, a halogeno-$C_1$-$C_4$ alkoxy group, a fluoro-$C_1$-$C_4$ alkoxy-$C_3$-$C_6$ alkyl group, a $C_3$-$C_4$ cycloalkyl-$C_3$-$C_6$ alkyl group, a $C_3$-$C_6$ alkenyl group or the formula (III):

[Formula 15]

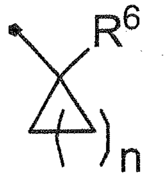 (III)

(wherein $R^6$ represents a $C_1$-$C_{10}$ alkyl group, a $C_1$-$C_4$ alkoxy group, a $C_7$-$C_{12}$ aralkyl group, a $C_1$-$C_4$ alkoxy-$C_1$-$C_4$ alkyl group or a $C_7$-$C_{12}$ aralkyloxy-$C_1$-$C_4$ alkyl group, and n is an integer of 1 to 3.), p is an integer of 1 or 2, $R^5$ represents a fluoro group, a chloro group or an amino group which may be substituted by a $C_1$-$C_3$ alkyl group, and q is an integer of 0 or 1.),

Z is a phenyl group, a furyl group, a thienyl group, an imidazolyl group, a thiazolyl group, a pyridyl group or a pyrimidinyl group each of which may be substituted by a group(s) selected from the group consisting of a halogeno group, a $C_1$-$C_4$ alkyl group, a halogeno-$C_1$-$C_4$ alkyl group, a $C_1$-$C_4$ alkoxy group and a halogeno-$C_1$-$C_4$ alkoxy group,

(18) a compound wherein $R^1$ is a hydrogen atom, a methyl group, an ethyl group, a propyl group, an isopropyl group or a tert-butyl group,

$R^2$ and $R^3$ are each independently a hydrogen atom or a methyl group,

Y is the formula (II):

[Formula 16]

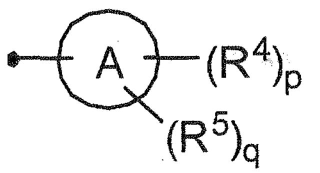 (II)

(wherein Ring A represents a phenyl group, a thienyl group, a thiazolyl group, a pyridyl group or a pyrimidinyl group, $R^4$ represents an ethyl group, a propyl group, an isopropyl group, a butyl group, a sec-butyl group, a tert-butyl group, a pentyl group, an isopentyl group, a neopentyl group, a tert-pentyl group, a 1-methylbutyl group, a 1-ethylpropyl group, a hexyl group, a 1-methylpentyl group, a 1-ethylbutyl group, a 1,1-dimethylbutyl group, a 2,2-dimethylbutyl group, a 1-ethyl-1-methylpropyl group, a 1-methylhexyl group, a 1,1-dimethylpentyl group, a 2,2-dimethylpentyl group, a 1,1-dimethylhexyl group, a 1,1,3,3-tetramethylbutyl group, a 1,1,4-trimethylpentyl group, a trifluoromethyl group, a 4,4,4-trifluorobutyl group, a 5,5,5-trifluoropentyl group, a 6,6,6-trifluorohexyl group, a 5,5,5-trifluoro-1-methylpentyl group, a 4,4,4-trifluoro-1,1-dimethylbutyl group, a 5,5,5-trifluoro-1,1-dimethylpentyl group, a 5,5,5-trifluoro-2,2-dimethylpentyl group, a propoxy group, a butoxy group, an isobutoxy group, a trifluoromethoxy group, a difluoromethoxy group, a trichloromethoxy group, a dichloromethoxy group, a 3-difluoromethoxypropyl group, a 3-difluoromethoxy-1-methylpropyl group, a 3-difluoromethoxy-1,1-dimethylpropyl group, a 4-difluoromethoxy-1,1-dimethylbutyl group, a 3-cyclopropylpropyl group, a 3-cyclopropyl-1-methylpropyl group, a 3-cyclopropyl-1,1-dimethylpropyl group, a 4-cyclopropyl-1,1-dimethylbutyl group, an isopropenyl group, a 1-methyl-1-propenyl group, a 1-methyl-1-butenyl group, a 1-methyl-1-pentenyl group or the formula (III):

[Formula 17]

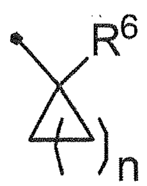

(III)

(wherein $R^6$ represents a methyl group, an ethyl group, a propyl group, an isopropyl group, a butyl group, an isobutyl group, a sec-butyl group, a tert-butyl group, a pentyl group, a hexyl group, a heptyl group, an octyl group, a nonyl group, a decyl group, a methoxy group, an ethoxy group, a propoxy group, an isopropoxy group, a benzyl group, a 1-phenylethyl group, a 2-phenylethyl group, a naphthalen-1-ylmethyl group, a naphthalen-2-ylmethyl group, an ethoxymethyl group, a propoxymethyl group, a butoxymethyl group, a 2-ethoxyethyl group, a 2-propoxyethyl group, a benzyloxymethyl group, a 2-benzyloxyethyl group, a 1-phenylethoxymethyl group, a 2-phenylethoxymethyl group, a 1-(1-phenylethoxy)ethyl group or a 2-(2-phenylethoxy)ethyl group, and n is an integer of 1 or 2.), p is an integer of 1 or 2, $R^5$ represents a fluoro group, a chloro group, an amino group, a methylamino group, a dimethylamino group or an ethylamino group, and q is an integer of 0 or 1.),

Z is a phenyl group, a furyl group, a thienyl group, an imidazolyl group, a thiazolyl group, a pyridyl group or a pyrimidinyl group each of which may be substituted by a group(s) selected from the group consisting of a fluoro group, a chloro group, a bromo group, a methyl group, an ethyl group, a propyl group, an isopropyl group, a tert-butyl group, a trifluoromethyl group, a difluoromethyl group, a trichloromethyl group, a dichloromethyl group, a 2,2,2-trifluoroethyl group, a 2,2,2-trichloroethyl group, a methoxy group, an ethoxy group, a propoxy group, an isopropoxy group, a trifluoromethoxy group, a difluoromethoxy group, a trichloromethoxy group and a dichloromethoxy group,

(19) a compound wherein $R^1$ is a hydrogen atom, a methyl group, an ethyl group or an isopropyl group,

$R^2$ and $R^3$ are both hydrogen atom,

Y is a 4-ethylphenyl group, a 3,4-diethylphenyl group, a 4-propylphenyl group, a 4-isopropylphenyl group, a 3-butylphenyl group, a 4-butylphenyl group, a 3-tert-butylphenyl group, a 4-tert-butylphenyl group, a 5-tert-butylthiophen-2-yl group, a 3-pentylphenyl group, a 4-pentylphenyl group, a 3-dimethylamino-4-pentylphenyl group, a 4-isopentylphenyl group, a 4-neopentylphenyl group, a 4-tert-pentylphenyl group, a 5-tert-pentylthiophen-2-yl group, a 4-(1-ethylpropyl)phenyl group, a 4-(1-methylbutyl)-phenyl group, a 3-hexylphenyl group, a 4-(1-ethylbutyl)phenyl group, a 4-(1-methylpentyl)phenyl group, a 5-(1-methylpentyl)thiophen-2-yl group, a 3-(1,1-dimethylbutyl)-phenyl group, a 4-(1,1-dimethylbutyl)phenyl group, a 2-fluoro-4-(1,1-dimethylbutyl)-phenyl group, a 3-fluoro-4-(1,1-dimethylbutyl)phenyl group, a 4-(2,2-dimethylbutyl)-phenyl group, a 5-(1,1-dimethylbutyl)thiophen-2-yl group, a 4-(1-ethyl-1-methylpropyl)phenyl group, a 3-(1,1-dimethylpentyl)phenyl group, a 4-(1,1-dimethylpentyl)-phenyl group, a 2-fluoro-4-(1,1-dimethylpentyl)phenyl group, a 3-fluoro-4-(1,1-dimethylpentyl)phenyl group, a 3-amino-4-(1,1-dimethylpentyl)phenyl group, a 3-methylamino-4-(1,1-dimethylpentyl)phenyl group, a 3-dimethylamino-4-(1,1-dimethylpentyl)-phenyl group, a 4-(1,1-dimethylpentyl)thiophen-2-yl group, a 5-(1,1-dimethylpentyl)-thiophen-2-yl group, a 5-(1,1-dimethylpentyl)thiazol-2-yl group, a 5-(1,1-dimethylpentyl)pyridin-2-yl group, a 6-(1,1-dimethylpentyl)pyridin-3-yl group, a 5-(1,1-dimethylpentyl)pyrimidin-2-yl group, a 2-(1,1-dimethylpentyl)pyrimidin-5-yl group, a 3-(2,2-dimethylpentyl)phenyl group, a 4-(2,2-dimethylpentyl)phenyl group, a 4-(1-methylhexyl)phenyl group, a 4-(1,1,3,3-tetramethylbutyl)phenyl group, a 4-(1,1,4-trimethylpentyl)phenyl group, a 4-(1,1-dimethylhexyl)phenyl group, a 4-trifluoromethylphenyl group, a 4-(4,4,4-trifluorobutyl)phenyl group, a 4-(5,5,5-trifluoropentyl)-phenyl group, a 4-(6,6,6-trifluorohexyl)phenyl group, a 4-(5,5,5-trifluoro-1-methylpentyl)phenyl group, a 4-(4,4,4-trifluoro-1,1-dimethylbutyl)phenyl group, a 4-(5,5,5-trifluoro-1,1-dimethylpentyl)phenyl group, a 4-(5,5,5-trifluoro-2,2-dimethylpentyl)-phenyl group, a 5-(5,5,5-trifluoro-1,1-dimethylpentyl)thiophen-2-yl group, a 4-propoxyphenyl group, a 4-butoxyphenyl group, a 4-isobutoxyphenyl group, a 4-difluoromethoxyphenyl group, a 4-trifluoromethoxyphenyl group, a 4-(3-difluoromethoxy-1-methylpropyl)phenyl group, a 4-(3-difluoromethoxy-1,1-dimethylpropyl)phenyl group, a 4-(3-cyclopropyl-1-methylpropyl)phenyl group, a 4-(3-cyclopropyl-1,1-dimethylpropyl)phenyl group, a 4-isopropenylphenyl group, a 4-(1-methyl-1-propenyl)phenyl group, a 4-(1-methyl-1-butenyl)phenyl group, a 4-(1-methyl-1-pentenyl)phenyl group, a 4-(1-methylcyclopropyl)phenyl group, a 4-(1-ethylcyclopropyl)phenyl group, a 4-(1-propylcyclopropyl)phenyl group, a 4-(1-isopropylcyclopropyl)phenyl group, a 3-(1-butylcyclopropyl)phenyl group, a 4-(1-butylcyclopropyl)phenyl group, a 2-fluoro-4-(1-butylcyclopropyl)phenyl group, a 3-fluoro-4-(1-butylcyclopropyl)phenyl group, a 3-dimethylamino-4-(1-butylcyclopropyl)phenyl group, a 5-(1-butylcyclopropyl)thiophen-2-yl group, a 4-(1-hexylcyclopropyl)phenyl group, a 4-(1-nonylcyclopropyl)phenyl group, a 4-(1-methoxycyclopropyl)phenyl

group, a 4-(1-ethoxycyclopropyl)phenyl group, a 4-(1-propoxycyclopropyl)phenyl group, a 4-(1-benzylcyclopropyl)phenyl group, a 4-{1-(1-phenylethyl)cyclopropyl}phenyl group, a 4-{1-(2-phenylethyl)cyclopropyl}phenyl group, a 4-(1-ethoxymethylcyclopropyl)phenyl group, a 4-(1-propoxymethylcyclopropyl)phenyl group, a 5-(1-propoxymethylcyclopropyl)thiophen-2-yl group, a 4-1-(2-ethoxyethyl)cyclopropylphenyl group, a 4-1-(2-propoxyethyl)cyclopropyl}phenyl group, a 4-(1-butoxymethylcyclopropyl)phenyl group, a 4-(1-benzyloxymethylcyclopropyl)phenyl group, a 4-{1-(2-benzyloxyethyl)cyclopropyl}phenyl group, a 4-1-(1-phenylethoxymethyl)cyclopropyl}phenyl group, a 4-{1-(2-phenylethoxymethyl)cyclopropyl}phenyl group, a 4-(1-ethylcyclobutyl)phenyl group, a 4-(1-butylcyclobutyl)phenyl group or 5-(1-butylcyclobutyl)thiophen-2-yl group,

Z is a phenyl group, a 2-fluorophenyl group, a 3-fluorophenyl group, a 4-fluorophenyl group, a 3,4-difluorophenyl group, a 3,5-difluorophenyl group, a 3,4,5-trifluorophenyl group, a 2-chlorophenyl group, a 3-chlorophenyl group, a 4-chlorophenyl group, a 2,6-dichlorophenyl group, a 4-chloro-3-fluorophenyl group, a 4-chloro-3,5-difluorophenyl group, a 4-bromophenyl group, a 4-methylphenyl group, a 3-fluoro-4-methylphenyl group, a 4-ethylphenyl group, a 4-ethyl-3-fluorophenyl group, a 4-propylphenyl group, a 4-isopropylphenyl group, a 4-tert-butylphenyl group, a 4-trifluoromethylphenyl group, a 3-fluoro-4-trifluoromethylphenyl group, a 4-difluoromethylphenyl group, a 4-trichloromethylphenyl group, a 4-dichloromethylphenyl group, a 4-(2,2,2-trifluoroethyl)phenyl group, a 4-(2,2,2-trichloroethyl)phenyl group, a 4-methoxyphenyl group, a 3-fluoro-4-methoxyphenyl group, a 4-ethoxyphenyl group, a 4-propoxyphenyl group, a 4-isopropoxyphenyl group, a 4-trifluoromethoxyphenyl group, a 4-difluoromethoxyphenyl group, a 4-difluoromethoxy-3-fluorophenyl group, a 4-trichloromethoxyphenyl group, a 4-dichloromethoxyphenyl group, a furan-2-yl group, a 5-methylfuran-2-yl group, a thiophen-2-yl group, a thiophen-3-yl group, a 5-chlorothiophen-2-yl group, a 1-methyl-1H-imidazol-4-yl group, a thiazol-2-yl group, a pyridin-2-yl group, a 5-fluoropyridin-2-yl group, a 5-chloropyridin-2-yl group, a 5-methylpyridin-2-yl group, a 5-ethylpyridin-2-yl group, a 5-trifluoromethylpyridin-2-yl group, a 5-methoxypyridin-2-yl group, a 5-difluoromethoxypyridin-2-yl group, a pyridin-3-yl group, a 6-fluoropyridin-3-yl group, a 6-chloropyridin-3-yl group, a 6-methylpyridin-3-yl group, a 6-ethylpyridin-3-yl group, a 6-trifluoromethylpyridin-3-yl group, a 6-methoxypyridin-3-yl group, a 6-difluoromethoxypyridin-3-yl group, a pyridin-4-yl group or a pyrimidin-2-yl group,

(20) a compound wherein $R^1$ is a hydrogen atom, a methyl group, an ethyl group or an isopropyl group, $R^2$ and $R^3$ are both hydrogen atom,

Y is a 3,4-diethylphenyl group, a 3-butylphenyl group, a 4-butylphenyl group, a 3-tert-butylphenyl group, a 4-tert-butylphenyl group, a 5-tert-butylthiophen-2-yl group, a 4-pentylphenyl group, a 3-dimethylamino-4-pentylphenyl group, a 4-isopentylphenyl group, a 4-neopentylphenyl group, a 4-tert-pentylphenyl group, a 5-tert-pentylthiophen-2-yl group, a 4-(1-ethylpropyl)phenyl group, a 4-(1-methylbutyl)phenyl group, a 3-hexylphenyl group, a 4-(1-methylpentyl)phenyl group, a 5-(1-methylpentyl)thiophen-2-yl group, a 3-(1,1-dimethylbutyl)phenyl group, a 4-(1,1-dimethylbutyl)phenyl group, a 2-fluoro-4-(1,1-dimethylbutyl)phenyl group, a 3-fluoro-4-(1,1-dimethylbutyl)-phenyl group, a 5-(1,1-dimethylbutyl)thiophen-2-yl group, a 4-(1-ethyl-1-methylpropyl)phenyl group, a 3-(1,1-dimethylpentyl)phenyl group, a 4-(1,1-dimethylpentyl)-phenyl group, a 2-fluoro-4-(1,1-dimethylpentyl)phenyl group, a 3-fluoro-4-(1,1-dimethylpentyl)phenyl group, a 3-dimethylamino-4-(1,1-dimethylpentyl)phenyl group, a 5-(1,1-dimethylpentyl)thiophen-2-yl group, a 4-(2,2-dimethylpentyl)phenyl group, a 4-(1,1,3,3-tetramethylbutyl)phenyl group, a 4-(1,1,4-trimethylpentyl)phenyl group, a 4-(1,1-dimethylhexyl)phenyl group, a 4-trifluoromethylphenyl group, a 4-(4,4,4-trifluoro-1,1-dimethylbutyl)phenyl group, a 4-(5,5,5-trifluoro-1,1-dimethylpentyl)phenyl group, a 5-(5,5,5-trifluoro-1,1-dimethylpentyl)thiophen-2-yl group, a 4-butoxyphenyl group, a 4-difluoromethoxyphenyl group, a 4-(3-difluoromethoxy-1,1-dimethylpropyl)phenyl group, a 4-(3-cyclopropyl-1,1-dimethylpropyl)phenyl group, a 4-(1-methyl-1-butenyl)-phenyl group, a 4-(1-methyl-1-pentenyl)phenyl group, a 4-(1-methylcyclopropyl)phenyl group, a 4-(1-ethylcyclopropyl)phenyl group, a 4-(1-propylcyclopropyl)phenyl group, a 4-(1-isopropylcyclopropyl)phenyl group, a 4-(1-butylcyclopropyl)phenyl group, a 2-fluoro-4-(1-butylcyclopropyl)phenyl group, a 3-fluoro-4-(1-butylcyclopropyl)phenyl group, a 5-(1-butylcyclopropyl)thiophen-2-yl group, a 4-(1-nonylcyclopropyl)phenyl group, a 4-(1-methoxycyclopropyl)phenyl group, a 4-(1-benzylcyclopropyl)phenyl group, a 4-{1-(2-phenylethyl)cyclopropyl}phenyl group, a 4-(1-propoxymethylcyclopropyl)phenyl group, a 5-(1-propoxymethylcyclopropyl)thiophen-2-yl group, a 4-(1-benzyloxymethylcyclopropyl)phenyl group, a 4-(1-ethylcyclobutyl)phenyl group or a 4-(1-butylcyclobutyl)phenyl group,

Z is a phenyl group, a 2-fluorophenyl group, a 3-fluorophenyl group, a 4-fluorophenyl group, a 3,4-difluorophenyl group, a 3,5-difluorophenyl group, a 2-chlorophenyl group, a 3-chlorophenyl group, a 4-chlorophenyl group, a 2,6-dichlorophenyl group, a 4-chloro-3-fluorophenyl group, a 4-methylphenyl group, a 3-fluoro-4-methylphenyl group, a 4-trifluoromethylphenyl group, a 3-fluoro-4-trifluoromethylphenyl group, a 4-methoxyphenyl group, a 3-fluoro-4-methoxyphenyl group, a 4-difluoromethoxyphenyl group, a 4-difluoromethoxy-3-fluorophenyl group, a furan-2-yl group, a 5-methylfuran-2-yl group, a thiophen-2-yl group, a thiophen-3-yl group, a 1-methyl-1H-imidazol-4-yl group, a thiazol-2-yl group, a pyridin-2-yl group, a 5-fluoropyridin-2-yl group, a 5-chloropyridin-2-yl group, a 5-methoxypyridin-2-yl group, a pyridin-3-yl group, a 6-fluoropyridin-3-yl group, a 6-chloropyridin-3-yl group, a 6-methoxypyridin-3-yl group or a pyridin-4-yl group, or,

(21) a compound wherein $R^1$ is a hydrogen atom, a methyl group, an ethyl group or an isopropyl group, $R^2$ and $R^3$ are both hydrogen atom,

Y is a 3,4-diethylphenyl group, a 3-butylphenyl group, a 3-tert-butylphenyl group, a 4-tert-butylphenyl group, a 4-pentylphenyl group, a 3-dimethylamino-4-pentylphenyl group, a 4-neopentylphenyl group, a 4-tert-pentylphenyl group, a 5-tert-pentylthiophen-2-yl group, a 4-(1-ethylpropyl)phenyl group, a 3-hexylphenyl group, a 4-(1-methylpentyl)phenyl group, a 4-(1,1-dimethylbutyl)phenyl group, a 4-(1-ethyl-1-methylpropyl)phenyl group, a 4-(1,1-dimethylpentyl)phenyl group, a 2-fluoro-4-(,1-dimethylpentyl)phenyl group, a 3-fluoro-4-(1,1-dimethylpentyl)phenyl group, a 5-(1,1-dimethylpentyl)thiophen-2-yl group, a 4-(2,2-dimethylpentyl)phenyl group, a 4-(1,1,3,3-tetramethylbutyl)phenyl group, a 4-(1,1,4-trimethylpentyl)phenyl group, a 4-(1,1-dimethylhexyl)phenyl group, a 4-trifluoromethylphenyl group, a 4-(5,5,5-trifluoro-1,1-dimethylpentyl)phenyl group, a 4-butoxyphenyl group, a 4-difluoromethoxyphenyl group, a 4-(3-difluoromethoxy-1,1-dimethylpropyl)phenyl group, a 4-(1-methyl-1-pentenyl)phenyl group, a 4-(1-methylcyclopropyl)phenyl group, a 4-(1-ethylcyclopropyl)phenyl group, a 4-(1-isopropylcyclopropyl)phenyl group, a 4-(1-butylcyclopropyl)phenyl group, a 4-(1-nonylcyclopropyl)phenyl group, a 4-(1-methoxycyclopropyl)phenyl group, a 4-(1-benzylcyclopropyl)phenyl group, a 4-(1-propoxymethylcyclopropyl)phenyl group, a 4-(1-benzyloxymethylcyclopropyl)phenyl group, a 4-(1-ethylcyclobutyl)phenyl group or a 4-(1-butylcyclobutyl)phenyl group,

Z is a phenyl group, a 2-fluorophenyl group, a 3-fluorophenyl group, a 4-fluorophenyl group, a 4-chlorophenyl group, a 4-methoxyphenyl group, a furan-2-yl group, a 5-methylfuran-2-yl group, a thiophen-2-yl group, a 1-methyl-1H-imidazol-4-yl group, a thiazol-2-yl group, a pyridin-2-yl group, a pyridin-3-yl group or a pyridin-4-yl group.

(22) As the aminopyridine compound, it can be preferably mentioned an aminopyridine compound wherein
{6-[(3,4-diethylbenzyl)(pyridin-2-ylsulfonyl)aminomethyl]pyridin-2-ylamino}acetic acid,
{6-[(3-butylbenzyl)(pyridin-2-ylsulfonyl)aminomethyl]pyridin-2-ylamino}acetic acid,
{6-[(3-tert-butylbenzyl)(pyridin-3-ylsulfonyl)aminomethyl]pyridin-2-ylamino}acetic acid,
{6-[(benzenesulfonyl)(4-tert-butylbenzyl)aminomethyl]pyridin-2-ylamino}acetic acid,
{6-[(4-tert-butylbenzyl)(2-fluorobenzenesulfonyl)aminomethyl]pyridin-2-ylamino}-acetic acid,
{6-[(4-tert-butylbenzyl)(3-fluorobenzenesulfonyl)aminomethyl]pyridin-2-ylamino}-acetic acid,
{6-[(4-tert-butylbenzyl)(4-fluorobenzenesulfonyl)aminomethyl]pyridin-2-ylamino}-acetic acid,
{6-[(4-tert-butylbenzyl)(4-chlorobenzenesulfonyl)aminomethyl]pyridin-2-ylamino}-acetic acid,
{6-[(4-tert-butylbenzyl)(4-methoxybenzenesulfonyl)aminomethyl]pyridin-2-ylamino}-acetic acid,
{6-[(4-tert-butylbenzyl)(pyridin-2-ylsulfonyl)aminomethyl]pyridin-2-ylamino}acetic acid,
{6-[(4-tert-butylbenzyl)(pyridin-3-ylsulfonyl)aminomethyl]pyridin-2-ylamino}acetic acid,
{6-[(4-pentylbenzyl)(pyridin-2-ylsulfonyl)aminomethyl]pyridin-2-ylamino}acetic acid, {6-[(3-dimethylamino-4-pentylbenzyl)(pyridin-2-ylsulfonyl)aminomethyl]pyridin-2-ylamino}acetic acid,
{6-[(4-neopentylbenzyl)(pyridin-2-ylsulfonyl)aminomethyl]pyridin-2-ylamino}acetic acid,
{6-[(4-tert-pentylbenzyl)(pyridin-2-ylsulfonyl)aminomethyl]pyridin-2-ylamino}acetic acid,
{6-[(5-tert-pentylthiophen-2-ylmethyl)(pyridin-2-ylsulfonyl)aminomethyl]pyridin-2-ylamino} acetic acid,
(6-{[4-(1-ethylpropyl)benzyl](pyridin-3-ylsulfonyl)aminomethyl}pyridin-2-ylamino)-acetic acid,
{6-[(3-hexylbenzyl)(pyridin-2-ylsulfonyl)aminomethyl]pyridin-2-ylamino}acetic acid,
(6- {[4-(1-methylpentyl)benzyl] (pyridin-2-ylsulfonyl)aminomethyl}pyridin-2-ylamino)-acetic acid,
(6-{[4-(,1-dimethylbutyl)benzyl](pyridin-2-ylsulfonyl)aminomethyl}pyridin-2-ylamino)acetic acid,
(6-{[4-(1-ethyl-1-methylpropyl)benzyl](pyridin-3-ylsulfonyl)aminomethyl}pyridin-2-ylamino)acetic acid,
(6- {(benzenesulfonyl)[4-(1,1-dimethylpentyl)benzyl]aminomethyl} pyridin-2-ylamino)-acetic acid,
(6- {(2-fluorobenzenesulfonyl)[4-(1,1-dimethylpentyl)benzyl]aminomethyl}pyridin-2-ylamino)acetic acid,
(6- {(3-fluorobenzenesulfonyl) [4-(1,1-dimethylpentyl)benzyl]aminomethyl}pyridin-2-ylamino)acetic acid,
(6-{(4-fluorobenzenesulfonyl)[4-(1,1-dimethylpentyl)benzyl]aminomethyl}pyridin-2-ylamino)acetic acid,
(6- {(4-chlorobenzenesulfonyl)[4-(1,1-dimethylpentyl)benzyl]aminomethyl}pyridin-2-ylamino)acetic acid,
(6-{(4-methoxybenzenesulfonyl)[4-(1,1-dimethylpentyl)benzyl]aminomethyl}pyridin-2-ylamino)acetic acid,
(6-{(furan-2-ylsulfonyl)[4-(1,1-dimethylpentyl)benzyl]aminomethyl}pyridin-2-ylamino)acetic acid,
(6-{[4-(,1-dimethylpentyl)benzyl](thiophen-2-ylsulfonyl)aminomethyl}pyridin-2-ylamino)acetic acid,
(6- {[4-(1,1-dimethylpentyl)benzyl](thiazol-2-ylsulfonyl)aminomethyl}pyridin-2-ylamino)acetic acid,
(6-{[4-(1,1-dimethylpentyl)benzyl](pyridin-2-ylsulfonyl)aminomethyl}pyridin-2-ylamino)acetic acid,
(6- {[4-(1,1-dimethylpentyl)benzyl](pyridin-3-ylsulfonyl)aminomethyl}pyridin-2-ylamino)acetic acid,
(6- {[4-(1,1-dimethylpentyl)benzyl](pyridin-4-ylsulfonyl)aminomethyl}pyridin-2-ylamino)acetic acid,
(6-{[2-fluoro-4-(,1-dimethylpentyl)benzyl](pyridin-2-ylsulfonyl)aminomethyl}-pyridin-2-ylamino)acetic acid,
(6- {[3-fluoro-4-(1,1-dimethylpentyl)benzyl](pyridin-2-ylsulfonyl)aminomethyl}-pyridin-2-ylamino)acetic acid,
(6-{(4-fluorobenzenesulfonyl)[5-(1,1-dimethylpentyl)thiophen-2-ylmethyl]aminomethyl} pyridin-2-ylamino)acetic acid,
(6-{[5-(1,1-dimethylpentyl)thiophen-2-ylmethyl](pyridin-2-ylsulfonyl)aminomethyl}-pyridin-2-ylamino)acetic acid,
(6-{[5-(1,1-dimethylpentyl)thiophen-2-ylmethyl](pyridin-3-ylsulfonyl)aminomethyl}-pyridin-2-ylamino)acetic acid,
(6-{[4-(2,2-dimethylpentyl)benzyl](pyridin-2-ylsulfonyl)aminomethyl}pyridin-2-ylamino)acetic acid,
(6-{[4-(1,1,3,3-tetramethylbutyl)benzyl](pyridin-2-ylsulfonyl)aminomethyl}pyridin-2-ylamino)acetic acid,
(6- {[4-(,1,4-trimethylpentyl)benzyl](pyridin-2-ylsulfonyl)aminomethyl}pyridin-2-ylamino)acetic acid,
(6- {[4-(1,1-dimethylhexyl)benzyl](pyridin-2-ylsulfonyl)aminomethyl}pyridin-2-ylamino)acetic acid,

{6-[(pyridin-3-ylsulfonyl)(4-trifluoromethylbenzyl)aminomethyl]pyridin-2-ylamino}-acetic acid,
(6-{[4-(5,5,5-trifluoro-1,1-dimethylpentyl)benzyl](pyridin-2-ylsulfonyl)aminomethyl}-pyridin-2-ylamino)acetic acid,
{6-[(4-butoxybenzyl)(pyridin-2-ylsulfonyl)aminomethyl]pyridin-2-ylaminoacetic acid,
{6-[(4-difluoromethoxybenzyl)(pyridin-3-ylsulfonyl)aminomethyl]pyridin-2-ylammo}-acetic acid,
(6-{[4-(3-difluoromethoxy-1,1-dimethylpropyl)benzyl](pyridin-2-ylsulfonyl)aminomethyl}pyridin-2-ylamino)acetic acid,
(6- {[4-(3-difluoromethoxy-1,1-dimethylpropyl)benzyl](pyridin-2-ylsulfonyl)aminomethyl}pyridin-2-ylamino)ethyl acetate,
(6- {[4-(1-methyl-1-pentenyl)benzyl](pyridin-2-ylsulfonyl)aminomethyl}pyridin-2-ylamino)acetic acid,
(6- {[4-(1-methylcyclopropyl)benzyl](pyridin-3-ylsulfonyl)aminomethyl}pyridin-2-ylamino)acetic acid,
(6- {[4-(1-ethylcyclopropyl)benzyl](4-fluorobenzenesulfonyl)aminomethyl}pyridin-2-ylamino)acetic acid,
(6- {[4-(1-ethylcyclopropyl)benzyl](pyridin-2-ylsulfonyl)aminomethyl}pyridin-2-ylamino)acetic acid,
(6- {[4-(1-ethylcyclopropyl)benzyl](pyridin-3-ylsulfonyl)aminomethyl}pyridin-2-ylamino)acetic acid,
(6-{[4-{1-isopropylcyclopropyl)benzyl](pyridin-3-ylsulfonyl)aminomethyl}pyridin-2-ylamino)acetic acid,
(6- {[4-(1-butylcyclopropyl)benzyl](1-methyl-1H-imidazol-4-ylsulfonyl)aminomethyl}-pyridin-2-ylamino)acetic acid,
(6- {[4-(1-butylcyclopropyl)benzyl](pyridin-2-ylsulfonyl)aminomethyl}pyridin-2-ylamino)acetic acid,
(6-{[4-(1-butylcyclopropyl)benzyl](pyridin-3-ylsulfonyl)aminomethyl}pyridin-2-ylamino)acetic acid,
(6- {[4-(1-butylcyclopropyl)benzyl](pyridin-4-ylsulfonyl)aminomethyl}pyridin-2-ylamino)acetic acid,
(6-{[4-(1-nonylcyclopropyl)benzyl](pyridin-3-ylsulfonyl)aminomethyl}pyridin-2-ylamino)acetic acid,
(6- {[4-(1-methoxycyclopropyl)benzyl](pyridin-2-ylsulfonyl)aminomethyl}pyridin-2-ylamino)acetic acid,
(6-{[4-(1-benzylcyclopropyl)benzyl](pyridin-2-ylsulfonyl)aminomethyl}pyridin-2-ylamino)acetic acid,
(6- {[4-(1-propoxymethylcyclopropyl)benzyl](pyridin-2-ylsulfonyl)aminomethyl}-pyridin-2-ylamino)acetic acid,
(6-{[4-(1-benzyloxymethylcyclopropyl)benzyl](pyridin-2-ylsulfonyl)aminomethyl}-pyridin-2-ylamino)acetic acid,
(6-{[4-(1-ethylcyclobutyl)benzyl](pyridin-3-ylsulfonyl)aminomethyl}pyridin-2-ylamino)acetic acid,
(6- {[4-(1-butylcyclobutyl)benzyl](pyridin-2-ylsulfonyl)aminomethyl}pyridin-2-ylamino)acetic acid, or
(6- {(5-methylfuran-2-ylsulfonyl)[4-(1,1-dimethylpentyl)benzyl]aminomethyl}pyridin-2-ylamino)acetic acid, etc.

Further, the present invention also provides:

(23) a medical composition containing as an active ingredient the above-mentioned compound represented by the formula (I), an aminopyridine compound according to any one of (1) to (22) or a pharmaceutically acceptable salt thereof,
(24) a medical composition according to (23) for the prevention or treatment of respiratory diseases, and
(25) a medical composition according to (23) for the prevention or treatment of glaucoma.

In the present invention, preferred compounds having the formula (I) may be specifically exemplified by the compounds of Table 1.

[Formula 18]

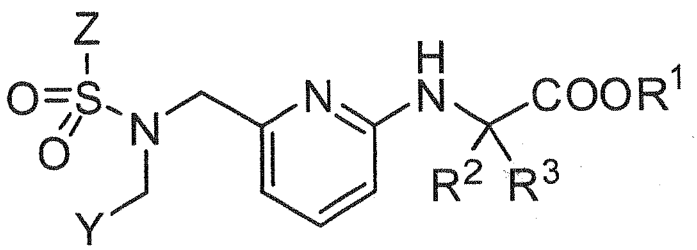

(I)

[Table 1]

| Compound No. | $R^1$ | $R^2$ | $R^3$ | Y | Z |
|---|---|---|---|---|---|
| 1 | H | H | H | 4-Et-Ph | 4-F-Ph |
| 2 | H | H | H | 4-Et-Ph | Py-2-yl |
| 3 | H | H | H | 4-Et-Ph | Py-3-yl |
| 4 | H | H | H | 3,4-diEt-Ph | Ph |

(continued)

| Compound No. | $R^1$ | $R^2$ | $R^3$ | Y | Z |
|---|---|---|---|---|---|
| 5 | H | H | H | 3,4-diEt-Ph | 2-F-Ph |
| 6 | H | H | H | 3,4-diEt-Ph | 3-F-Ph |
| 7 | H | H | H | 3,4-diEt-Ph | 4-F-Ph |
| 8 | H | H | H | 3,4-diEt-Ph | 3, 4-diF-Ph |
| 9 | H | H | H | 3,4-diEt-Ph | 3, 5-diF-Ph |
| 10 | H | H | H | 3,4-diEt-Ph | 4-Cl-Ph |
| 11 | H | H | H | 3,4-diEt-Ph | 4-Me-Ph |
| 12 | H | H | H | 3, 4-diEt-Ph | 4-$CF_3$-Ph |
| 13 | H | H | H | 3, 4-diEt-Ph | 4-0Me-Ph |
| 14 | H | H | H | 3,4-diEt-Ph | Fu-2-yl |
| 15 | H | H | H | 3,4-diEt-Ph | Th-2-yl |
| 16 | H | H | H | 3,4-diEt-Ph | Th-3-yl |
| 17 | H | H | H | 3,4-diEt-Ph | 1-Me-Im-4-yl |
| 18 | H | H | H | 3,4-diEt-Ph | Tz-2-yl |
| 19 | H | H | H | 3,4-diEt-Ph | Py-2-yl |
| 20 | Me | H | H | 3, 4-diEt-Ph | Py-2-yl |
| 21 | Et | H | H | 3,4-diEt-Ph | Py-2-yl |
| 22 | $Pr^1$ | H | H | 3,4-diEt-Ph | Py-2-yl |
| 23 | H | H | H | 3,4-diEt-Ph | 5-F-Py-2-yl |
| 24 | H | H | H | 3,4-diEt-Ph | Py-3-yl |
| 25 | H | H | H | 3,4-diEt-Ph | 6-F-Py-3-yl |
| 26 | H | H | H | 3,4-diEt-Ph | Py-4-yl |
| 27 | H | H | H | 4-Pr-Ph | 4-F-Ph |
| 28 | H | H | H | 4-Pr-Ph | Py-2-yl |
| 29 | H | H | H | 4-Pr-Ph | Py-3-yl |
| 30 | H | H | H | 4-$Pr^1$-Ph | 4-F-Ph |
| 31 | H | H | H | 4-$Pr^1$-Ph | Py-2-yl |
| 32 | H | H | H | 4-$Pr^1$-Ph | Py-3-yl |
| 33 | H | H | H | 3-Bu-Ph | Ph |
| 34 | H | H | H | 3-Bu-Ph | 2-F-Ph |
| 35 | H | H | H | 3-Bu-Ph | 3-F-Ph |
| 36 | H | H | H | 3-Bu-Ph | 4-F-Ph |
| 37 | H | H | H | 3-Bu-Ph | 3, 4-diF-Ph |
| 38 | H | H | H | 3-Bu-Ph | 3, 5-diF-Ph |
| 39 | H | H | H | 3-Bu-Ph | 4-Cl-Ph |
| 40 | H | H | H | 3-Bu-Ph | 4-Me-Ph |
| 41 | H | H | H | 3-Bu-Ph | 4-$CF_3$-Ph |
| 42 | H | H | H | 3-Bu-Ph | 4-0Me-Ph |
| 43 | H | H | H | 3-Bu-Ph | Fu-2-yl |
| 44 | H | H | H | 3-Bu-Ph | Th-2-yl |
| 45 | H | H | H | 3-Bu-Ph | Th-3-yl |
| 46 | H | H | H | 3-Bu-Ph | 1-Me-Im-4-yl |
| 47 | H | H | H | 3-Bu-Ph | Tz-2-yl |
| 48 | H | H | H | 3-Bu-Ph | Py-2-yl |
| 49 | Me | H | H | 3-Bu-Ph | Py-2-yl |
| 50 | Et | H | H | 3-Bu-Ph | Py-2-yl |
| 51 | $Pr^1$ | H | H | 3-Bu-Ph | Py-2-yl |
| 52 | H | H | H | 3-Bu-Ph | 5-F-Py-2-yl |
| 53 | H | H | H | 3-Bu-Ph | Py-3-yl |
| 54 | H | H | H | 3-Bu-Ph | 6-F-Py-3-yl |

(continued)

| Compound No. | $R^1$ | $R^2$ | $R^3$ | Y | Z |
|---|---|---|---|---|---|
| 55 | H | H | H | 3-Bu-Ph | Py-4-yl |
| 56 | H | H | H | 4-Bu-Ph | Ph |
| 57 | H | H | H | 4-Bu-Ph | 2-F-Ph |
| 58 | H | H | H | 4-Bu-Ph | 3-F-Ph |
| 59 | H | H | H | 4-Bu-Ph | 4-F-Ph |
| 60 | H | H | H | 4-Bu-Ph | 4-Cl-Ph |
| 61 | H | H | H | 4-Bu-Ph | 4-OMe-Ph |
| 62 | H | H | H | 4-Bu-Ph | Fu-2-yl |
| 63 | H | H | H | 4-Bu-Ph | Th-2-yl |
| 64 | H | H | H | 4-Bu-Ph | 1-Me-Im-4-yl |
| 65 | H | H | H | 4-Bu-Ph | Tz-2-yl |
| 66 | H | H | H | 4-Bu-Ph | Py-2-yl |
| 67 | H | H | H | 4-Bu-Ph | Py-3-yl |
| 68 | H | H | H | 4-Bu-Ph | Py-4-yl |
| 69 | H | H | H | 3-$Bu^t$-Ph | Ph |
| 70 | H | H | H | 3-$Bu^t$-Ph | 2-F-Ph |
| 71 | H | H | H | 3-$Bu^t$-Ph | 3-F-Ph |
| 72 | H | H | H | 3-$Bu^t$-Ph | 4-F-Ph |
| 73 | H | H | H | 3-$Bu^t$-Ph | 3, 4-diF-Ph |
| 74 | H | H | H | 3-$Bu^t$-Ph | 3, 5-diF-Ph |
| 75 | H | H | H | 3-$Bu^t$-Ph | 4-Cl-Ph |
| 76 | H | H | H | 3-$Bu^t$-Ph | 4-Me-Ph |
| 77 | H | H | H | 3-$Bu^t$-Ph | 4-$CF_3$-Ph |
| 78 | H | H | H | 3-$Bu^t$-Ph | 4-0Me-Ph |
| 79 | H | H | H | 3-$Bu^t$-Ph | Fu-2-yl |
| 80 | H | H | H | 3-$Bu^t$-Ph | Th-2-yl |
| 81 | H | H | H | 3-$Bu^t$-Ph | Th-3-yl |
| 82 | H | H | H | 3-$Bu^t$-Ph | 1-Me-Im-4-yl |
| 83 | H | H | H | 3-$Bu^t$-Ph | Tz-2-yl |
| 84 | H | H | H | 3-$Bu^t$-Ph | Py-2-yl |
| 85 | H | H | H | 3-$Bu^t$-Ph | 5-F-Py-2-yl |
| 86 | H | H | H | 3-$Bu^t$-Ph | Py-3-yl |
| 87 | Me | H | H | 3-$Bu^t$-Ph | Py-3-yl |
| 88 | Et | H | H | 3-$Bu^t$-Ph | Py-3-yl |
| 89 | $Pr^1$ | H | H | 3-$Bu^t$-Ph | Py-3-yl |
| 90 | H | H | H | 3-$Bu^t$-Ph | 6-F-Py-3-yl |
| 91 | H | H | H | 3-$Bu^t$-Ph | Py-4-yl |
| 92 | H | H | H | 4-$Bu^t$-Ph | Ph |
| 93 | Me | H | H | 4-$Bu^t$-Ph | Ph |
| 94 | Et | H | H | 4-$Bu^t$-Ph | Ph |
| 95 | $Pr^1$ | H | H | 4-$Bu^t$-Ph | Ph |
| 96 | H | H | H | 4-$Bu^t$-Ph | 2-F-Ph |
| 97 | Me | H | H | 4-$Bu^t$-Ph | 2-F-Ph |
| 98 | Et | H | H | 4-$Bu^t$-Ph | 2-F-Ph |
| 99 | $Pr^1$ | H | H | 4-$Bu^t$-Ph | 2-F-Ph |
| 100 | H | H | H | 4-$Bu^t$-Ph | 3-F-Ph |
| 101 | Me | H | H | 4-$Bu^t$-Ph | 3-F-Ph |
| 102 | Et | H | H | 4-$Bu^t$-Ph | 3-F-Ph |
| 103 | $Pr^1$ | H | H | 4-$Bu^t$-Ph | 3-F-Ph |
| 104 | H | H | H | 4-$Bu^t$-Ph | 4-F-Ph |

(continued)

| Compound No. | $R^1$ | $R^2$ | $R^3$ | Y | Z |
|---|---|---|---|---|---|
| 105 | Me | H | H | 4-$Bu^t$-Ph | 4-F-Ph |
| 106 | Et | H | H | 4-$Bu^t$-Ph | 4-F-Ph |
| 107 | $Pr^1$ | H | H | 4-$Bu^t$-Ph | 4-F-Ph |
| 108 | H | H | H | 4-$Bu^t$-Ph | 3, 4-diF-Ph |
| 109 | H | H | H | 4-$Bu^t$-Ph | 3, 5-diF-Ph |
| 110 | H | H | H | 4-$Bu^t$-Ph | 4-Cl-Ph |
| 111 | Me | H | H | 4-$Bu^t$-Ph | 4-Cl-Ph |
| 112 | Et | H | H | 4-$Bu^t$-Ph | 4-Cl-Ph |
| 113 | $Pr^1$ | H | H | 4-$Bu^t$-Ph | 4-Cl-Ph |
| 114 | H | H | H | 4-$Bu^t$-Ph | 4-Me-Ph |
| 115 | H | H | H | 4-$Bu^t$-Ph | 4-$CF_3$-Ph |
| 116 | H | H | H | 4-$Bu^t$-Ph | 4-OMe-Ph |
| 117 | Me | H | H | 4-$Bu^t$-Ph | 4-OMe-Ph |
| 118 | Et | H | H | 4-$Bu^t$-Ph | 4-OMe-Ph |
| 119 | $Pr^1$ | H | H | 4-$Bu^t$-Ph | 4-OMe-Ph |
| 120 | H | H | H | 4-$Bu^t$-Ph | Fu-2-yl |
| 121 | H | H | H | 4-$Bu^t$-Ph | Th-2-yl |
| 122 | H | H | H | 4-$Bu^t$-Ph | Th-3-yl |
| 123 | H | H | H | 4-$Bu^t$-Ph | 1-Me-Im-4-yl |
| 124 | H | H | H | 4-$Bu^t$-Ph | Tz-2-yl |
| 125 | H | H | H | 4-$Bu^t$-Ph | Py-2-yl |
| 126 | Me | H | H | 4-$Bu^t$-Ph | Py-2-yl |
| 127 | Et | H | H | 4-$Bu^t$-Ph | Py-2-yl |
| 128 | $Pr^1$ | H | H | 4-$Bu^t$-Ph | Py-2-yl |
| 129 | H | H | H | 4-$Bu^t$-Ph | 5-F-Py-2-yl |
| 130 | H | H | H | 4-$Bu^t$-Ph | Py-3-yl |
| 131 | Me | H | H | 4-$Bu^t$-Ph | Py-3-yl |
| 132 | Et | H | H | 4-$Bu^t$-Ph | Py-3-yl |
| 133 | $Pr^1$ | H | H | 4-$Bu^t$-Ph | Py-3-yl |
| 134 | H | H | H | 4-$Bu^t$-Ph | 6-F-Py-3-yl |
| 135 | H | H | H | 4-$Bu^t$-Ph | Py-4-yl |
| 136 | H | H | H | 5-$Bu^t$-Th-2-yl | Ph |
| 137 | H | H | H | 5-$Bu^t$-Th-2-yl | 2-F-Ph |
| 138 | H | H | H | 5-$Bu^t$-Th-2-yl | 3-F-Ph |
| 139 | H | H | H | 5-$Bu^t$-Th-2-yl | 4-F-Ph |
| 140 | H | H | H | 5-$Bu^t$-Th-2-yl | 4-Cl-Ph |
| 141 | H | H | H | 5-$Bu^t$-Th-20-yl | 4-OMe-Ph |
| 142 | H | H | H | 5-$Bu^t$-Th-2-yl | Fu-2-yl |
| 143 | H | H | H | 5-$Bu^t$-Th-2-yl | Th-2-yl |
| 144 | H | H | H | 5-$Bu^t$-Th-2-yl | 1-Me-Im-4-yl |
| 145 | H | H | H | 5-$Bu^t$-Th-2-yl | Tz-2-yl |
| 146 | H | H | H | 5-$Bu^t$-Th-2-yl | Py-2-yl |
| 147 | H | H | H | 5-$Bu^t$-Th-2-yl | Py-3-yl |
| 148 | H | H | H | 5-$Bu^t$-Th-2-yl | Py-4-yl |
| 149 | H | H | H | 3-Pn-Ph | 4-F-Ph |
| 150 | H | H | H | 3-Pn-Ph | Py-2-yl |
| 151 | H | H | H | 3-Pn-Ph | Py-3-yl |
| 152 | H | H | H | 4-Pn-Ph | Ph |
| 153 | H | H | H | 4-Pn-Ph | 2-F-Ph |
| 154 | H | H | H | 4-Pn-Ph | 3-F-Ph |

(continued)

| Compound No. | $R^1$ | $R^2$ | $R^3$ | Y | Z |
|---|---|---|---|---|---|
| 155 | H | H | H | 4-Pn-Ph | 4-F-Ph |
| 156 | H | H | H | 4-Pn-Ph | 3, 4-diF-Ph |
| 157 | H | H | H | 4-Pn-Ph | 3,5-diF-Ph |
| 158 | H | H | H | 4-Pn-Ph | 4-Cl-Ph |
| 159 | H | H | H | 4-Pn-Ph | 4-Me-Ph |
| 160 | H | H | H | 4-Pn-Ph | 4-$CF_3$-Ph |
| 161 | H | H | H | 4-Pn-Ph | 4-OMe-Ph |
| 162 | H | H | H | 4-Pn-Ph | Fu-2-yl |
| 163 | H | H | H | 4-Pn-Ph | Th-2-yl |
| 164 | H | H | H | 4-Pn-Ph | Th-3-yl |
| 165 | H | H | H | 4-Pn-Ph | 1-Me-Im-4-yl |
| 166 | H | H | H | 4-Pn-Ph | Tz-2-yl |
| 167 | H | H | H | 4-Pn-Ph | Py-2-yl |
| 168 | Me | H | H | 4-Pn-Ph | Py-2-yl |
| 169 | Et | H | H | 4-Pn-Ph | Py-2-yl |
| 170 | $Pr^1$ | H | H | 4-Pn-Ph | Py-2-yl |
| 171 | H | H | H | 4-Pn-Ph | 5-F-Py-2-yl |
| 172 | H | H | H | 4-Pn-Ph | Py-3-yl |
| 173 | H | H | H | 4-Pn-Ph | 6-F-Py-3-yl |
| 174 | H | H | H | 4-Pn-Ph | Py-4-yl |
| 175 | H | H | H | 3-$NMe_2$-4-Pn-Ph | Ph |
| 176 | H | H | H | 3-$NMe_2$-4-Pn-Ph | 2-F-Ph |
| 177 | H | H | H | 3-$NMe_2$-4-Pn-Ph | 3-F-Ph |
| 178 | H | H | H | 3-$NMe_2$-4-Pn-Ph | 4-F-Ph |
| 179 | H | H | H | 3-$NMe_2$-4-Pn-Ph | 3, 4-diF-Ph |
| 180 | H | H | H | 3-$NMe_2$-4-Pn-Ph | 3, 5-diF-Ph |
| 181 | H | H | H | 3-$NMe_2$-4-Pn-Ph | 4-Cl-Ph |
| 182 | H | H | H | 3-$NMe_2$-4-Pn-Ph | 4-Me-Ph |
| 183 | H | H | H | 3-$NMe_2$-4-Pn-Ph | 4-$CF_3$-Ph |
| 184 | H | H | H | 3-$NMe_2$-4-Pn-Ph | 4-OMe-Ph |
| 185 | H | H | H | 3-$NMe_2$-4-Pn-Ph | Fu-2-yl |
| 186 | H | H | H | 3-$NMe_2$-4-Pn-Ph | Th-2-yl |
| 187 | H | H | H | 3-$NMe_2$-4-Pn-Ph | Th-3-yl |
| 188 | H | H | H | 3-$NMe_2$-4-Pn-Ph | 1-Me-Im-4-yl |
| 189 | H | H | H | 3-$NMe_2$-4-Pn-Ph | Tz-2-yl |
| 190 | H | H | H | 3-$NMe_2$-4-Pn-Ph | Py-2-yl |
| 191 | Me | H | H | 3-$NMe_2$-4-Pn-Ph | Py-2-yl |
| 192 | Et | H | H | 3-$NMe_2$-4-Pn-Ph | Py-2-yl |
| 193 | $Pr^1$ | H | H | 3-$NMe_2$-4-Pn-Ph | Py-2-yl |
| 194 | H | H | H | 3-$NMe_2$-4-Pn-Ph | 5-F-Py-2-yl |
| 195 | H | H | H | 3-$NMe_2$-4-Pn-Ph | Py-3-yl |
| 196 | H | H | H | 3-$NMe_2$-4-Pn-Ph | 6-F-Py-3-yl |
| 197 | H | H | H | 3-$NMe_2$-4-Pn-Ph | Py-4-yl |
| 198 | H | H | H | 4-$Pn^1$-Ph | Ph |
| 199 | H | H | H | 4-$Pn^1$-Ph | 2-F-Ph |
| 200 | H | H | H | 4-$Pn^1$-Ph | 3-F-Ph |
| 201 | H | H | H | 4-$Pn^1$-Ph | 4-F-Ph |
| 202 | H | H | H | 4-$Pn^1$-Ph | 4-Cl-Ph |
| 203 | H | H | H | 4-$Pn^1$-Ph | 4-OMe-Ph |
| 204 | H | H | H | 4-$Pn^1$-Ph | Fu-2-yl |

(continued)

| Compound No. | $R^1$ | $R^2$ | $R^3$ | Y | Z |
|---|---|---|---|---|---|
| 205 | H | H | H | 4-$Pn^1$-Ph | Th-2-yl |
| 206 | H | H | H | 4-$Pn^1$-Ph | 1-Me-Im-4-yl |
| 207 | H | H | H | 4-$Pn^l$-Ph | Tz-2-yl |
| 208 | H | H | H | 4-$Pn^1$-Ph | Py-2-yl |
| 209 | H | H | H | 4-$Pn^1$-Ph | Py-3-yl |
| 210 | H | H | H | 4-$Pn^1$-Ph | Py-4-yl |
| 211 | H | H | H | 4-$Pn^{neo}$-Ph | Ph |
| 212 | H | H | H | 4-$Pn^{neo}$-Ph | 2-F-Ph |
| 213 | H | H | H | 4-$Pn^{neo}$-Ph | 3-F-Ph |
| 214 | H | H | H | 4-$Pn^{neo}$-Ph | 4-F-Ph |
| 215 | H | H | H | 4-$Pn^{neo}$-Ph | 3,4-diF-Ph |
| 216 | H | H | H | 4-$Pn^{neo}$-Ph | 3,5-diF-Ph |
| 217 | H | H | H | 4-$Pn^{neo}$-Ph | 4-Cl-Ph |
| 218 | H | H | H | 4-$Pn^{neo}$-Ph | 4-Me-Ph |
| 219 | H | H | H | 4-$Pn^{neo}$-Ph | 4-$CF_3$-Ph |
| 220 | H | H | H | 4-$Pn^{neo}$-Ph | 4-OMe-Ph |
| 221 | H | H | H | 4-$Pn^{neo}$-Ph | Fu-2-yl |
| 222 | H | H | H | 4-$Pn^{neo}$-Ph | Th-2-yl |
| 223 | H | H | H | 4-$Pn^{neo}$-Ph | Th-3-yl |
| 224 | H | H | H | 4-$Pn^{neo}$-Ph | 1-Me-Im-4-yl |
| 225 | H | H | H | 4-$Pn^{neo}$-Ph | Tz-2-yl |
| 226 | H | H | H | 4-$Pn^{neo}$-Ph | Py-2-yl |
| 227 | Me | H | H | 4-$Pn^{neo}$-Ph | Py-2-yl |
| 228 | Et | H | H | 4-$Pn^{neo}$-Ph | Py-2-yl |
| 229 | $Pr^1$ | H | H | 4-$Pn^{neo}$-Ph | Py-2-yl |
| 230 | H | H | H | 4-$Pn^{neo}$-Ph | 5-F-Py-2-yl |
| 231 | H | H | H | 4-$Pn^{neo}$-Ph | Py-3-yl |
| 232 | H | H | H | 4-$Pn^{neo}$-Ph | 6-F-Py-3-yl |
| 233 | H | H | H | 4-$Pn^{neo}$-Ph | Py-4-yl |
| 234 | H | H | H | 4-$Pn^t$-Ph | Ph |
| 235 | H | H | H | 4-$Pn^t$-Ph | 2-F-Ph |
| 236 | H | H | H | 4-$Pn^t$-Ph | 3-F-Ph |
| 237 | H | H | H | 4-$Pn^t$-Ph | 4-F-Ph |
| 238 | H | H | H | 4-$Pn^t$-Ph | 3,4-diF-Ph |
| 239 | H | H | H | 4-$Pn^t$-Ph | 3,5-diF-Ph |
| 240 | H | H | H | 4-$Pn^t$-Ph | 4-Cl-Ph |
| 241 | H | H | H | 4-$Pn^t$-Ph | 4-Me-Ph |
| 242 | H | H | H | 4-$Pn^t$-Ph | 4-$CF_3$-Ph |
| 243 | H | H | H | 4-$Pn^t$-Ph | 4-OMe-Ph |
| 244 | H | H | H | 4-$Pn^t$-Ph | Fu-2-yl |
| 245 | H | H | H | 4-$Pn^t$-Ph | Th-2-yl |
| 246 | H | H | H | 4-$Pn^t$-Ph | Th-3-yl |
| 247 | H | H | H | 4-$Pn^t$-Ph | 1-Me-Im-4-yl |
| 248 | H | H | H | 4-$Pn^t$-Ph | Tz-2-yl |
| 249 | H | H | H | 4-$Pn^t$-Ph | Py-2-yl |
| 250 | Me | H | H | 4-$Pn^t$-Ph | Py-2-yl |
| 251 | Et | H | H | 4-$Pn^t$-Ph | Py-2-yl |
| 252 | $Pr^l$ | H | H | 4-$Pn^t$-Ph | Py-2-yl |
| 253 | H | H | H | 4-$Pn^t$-Ph | 5-F-Py-2-yl |
| 254 | H | H | H | 4-$Pn^t$-Ph | Py-3-yl |

(continued)

| Compound No. | $R^1$ | $R^2$ | $R^3$ | Y | Z |
|---|---|---|---|---|---|
| 255 | H | H | H | 4-$Pn^t$-Ph | 6-F-Py-3-yl |
| 256 | H | H | H | 4-$Pn^t$-Ph | Py-4-yl |
| 257 | H | H | H | 5-$Pn^t$-Th-2-yl | Ph |
| 258 | H | H | H | 5-$Pn^t$-Th-2-yl | 2-F-Ph |
| 259 | H | H | H | 5-$Pn^t$-Th-2-yl | 3-F-Ph |
| 260 | H | H | H | 5-$Pn^t$-Th-2-yl | 4-F-Ph |
| 261 | H | H | H | 5-$Pn^t$-Th-2-yl | 3,4-diF-Ph |
| 262 | H | H | H | 5-$Pn^t$-Th-2-yl | 3,5-diF-Ph |
| 263 | H | H | H | 5-$Pn^t$-Th-2-yl | 4-Cl-Ph |
| 264 | H | H | H | 5-$Pn^t$-Th-2-yl | 4-Me-Ph |
| 265 | H | H | H | 5-$Pn^t$-Th-2-yl | 4-$CF_3$-Ph |
| 266 | H | H | H | 5-$Pn^t$-Th-2-yl | 4-OMe-Ph |
| 267 | H | H | H | 5-$Pn^t$-Th-2-yl | Fu-2-yl |
| 268 | H | H | H | 5-$Pn^t$-Th-2-yl | Th-2-yl |
| 269 | H | H | H | 5-$Pn^t$-Th-2-yl | Th-3-yl |
| 270 | H | H | H | 5-$Pn^t$-Th-2-yl | 1-Me-Im-4-yl |
| 271 | H | H | H | 5-$Pn^t$-Th-2-yl | Tz-2-yl |
| 272 | H | H | H | 5-$Pn^t$-Th-2-yl | Py-2-yl |
| 273 | Me | H | H | 5-$Pn^t$-Th-2-yl | Py-2-yl |
| 274 | Et | H | H | 5-$Pn^t$-Th-2-yl | Py-2-yl |
| 275 | $Pr^1$ | H | H | 5-$Pn^t$-Th-2-yl | Py-2-yl |
| 276 | H | H | H | 5-$Pn^t$-Th-2-yl | 5-F-Py-2-yl |
| 277 | H | H | H | 5-$Pn^t$-Th-2-yl | Py-3-yl |
| 278 | H | H | H | 5-$Pn^t$-Th-2-yl | 6-F-Py-3-yl |
| 279 | H | H | H | 5-$Pn^t$-Th-2-yl | Py-4-yl |
| 280 | H | H | H | 4-(1-EtPr)-Ph | Ph |
| 281 | H | H | H | 4-(1-EtPr)-Ph | 2-F-Ph |
| 282 | H | H | H | 4-(1-EtPr)-Ph | 3-F-Ph |
| 283 | H | H | H | 4-(1-EtPr)-Ph | 4-F-Ph |
| 284 | H | H | H | 4-(1-EtPr)-Ph | 3,4-diF-Ph |
| 285 | H | H | H | 4-(1-EtPr)-Ph | 3,5-diF-Ph |
| 286 | H | H | H | 4-(1-EtPr)-Ph | 4-Cl-Ph |
| 287 | H | H | H | 4-(1-EtPr)-Ph | 4-Me-Ph |
| 288 | H | H | H | 4-(1-EtPr)-Ph | 4-$CF_3$-Ph |
| 289 | H | H | H | 4-(1-EtPr)-Ph | 4-OMe-Ph |
| 290 | H | H | H | 4-(1-EtPr)-Ph | Fu-2-yl |
| 291 | H | H | H | 4-(1-EtPr)-Ph | Th-2-yl |
| 292 | H | H | H | 4-(1-EtPr)-Ph | Th-3-yl |
| 293 | H | H | H | 4-(1-EtPr)-Ph | 1-Me-Im-4-yl |
| 294 | H | H | H | 4-(1-EtPr)-Ph | Tz-2-yl |
| 295 | H | H | H | 4-(1-EtPr)-Ph | Py-2-yl |
| 296 | H | H | H | 4-(1-EtPr)-Ph | 5-F-Py-2-yl |
| 297 | H | H | H | 4-(1-EtPr)-Ph | Py-3-yl |
| 298 | Me | H | H | 4-(1-EtPr)-Ph | Py-3-yl |
| 299 | Et | H | H | 4-(1-EtPr)-Ph | Py-3-yl |
| 300 | $Pr^1$ | H | H | 4-(1-EtPr)-Ph | Py-3-yl |
| 301 | H | H | H | 4-(1-EtPr)-Ph | 6-F-Py-3-yl |
| 302 | H | H | H | 4-(1-EtPr)-Ph | Py-4-yl |
| 303 | H | H | H | 4-(1-MeBu)-Ph | Ph |
| 304 | H | H | H | 4-(1-MeBu)-Ph | 2-F-Ph |

(continued)

| Compound No. | $R^1$ | $R^2$ | $R^3$ | Y | Z |
|---|---|---|---|---|---|
| 305 | H | H | H | 4-(1-MeBu)-Ph | 3-F-Ph |
| 306 | H | H | H | 4-(1-MeBu)-Ph | 4-F-Ph |
| 307 | H | H | H | 4-(1-MeBu)-Ph | 4-Cl-Ph |
| 308 | H | H | H | 4-(1-MeBu)-Ph | 4-OMe-Ph |
| 309 | H | H | H | 4-(1-MeBu)-Ph | Fu-2-yl |
| 310 | H | H | H | 4-(1-MeBu)-Ph | Th-2-yl |
| 311 | H | H | H | 4-(1-MeBu)-Ph | 1-Me-Im-4-yl |
| 312 | H | H | H | 4-(1-MeBu)-Ph | Tz-2-yl |
| 313 | H | H | H | 4-(1-MeBu)-Ph | Py-2-yl |
| 314 | H | H | H | 4-(1-MeBu)-Ph | Py-3-yl |
| 315 | H | H | H | 4-(1-MeBu)-Ph | Py-4-yl |
| 316 | H | H | H | 4-(1-EtBu)-Ph | 4-F-Ph |
| 317 | H | H | H | 4-(1-EtBu)-Ph | Py-2-yl |
| 318 | H | H | H | 4-(1-EtBu)-Ph | Py-3-yl |
| 319 | H | H | H | 3-Hx-Ph | Ph |
| 320 | H | H | H | 3-Hx-Ph | 2-F-Ph |
| 321 | H | H | H | 3-Hx-Ph | 3-F-Ph |
| 322 | H | H | H | 3-Hx-Ph | 4-F-Ph |
| 323 | H | H | H | 3-Hx-Ph | 3,4-diF-Ph |
| 324 | H | H | H | 3-Hx-Ph | 3,5-diF-Ph |
| 325 | H | H | H | 3-Hx-Ph | 4-Cl-Ph |
| 326 | H | H | H | 3-Hx-Ph | 4-Me-Ph |
| 327 | H | H | H | 3-Hx-Ph | 4-$CF_3$-Ph |
| 328 | H | H | H | 3-Hx-Ph | 4-OMe-Ph |
| 329 | H | H | H | 3-Hx-Ph | Fu-2-yl |
| 330 | H | H | H | 3-Hx-Ph | Th-2-yl |
| 331 | H | H | H | 3-Hx-Ph | Th-3-yl |
| 332 | H | H | H | 3-Hx-Ph | 1-Me-Im-4-yl |
| 333 | H | H | H | 3-Hx-Ph | Tz-2-yl |
| 334 | H | H | H | 3-Hx-Ph | Py-2-yl |
| 335 | Me | H | H | 3-Hx-Ph | Py-2-yl |
| 336 | Et | H | H | 3-Hx-Ph | Py-2-yl |
| 337 | $Pr^1$ | H | H | 3-Hx-Ph | Py-2-yl |
| 338 | H | H | H | 3-Hx-Ph | 5-F-Py-2-yl |
| 339 | H | H | H | 3-Hx-Ph | Py-3-yl |
| 340 | H | H | H | 3-Hx-Ph | 6-F-Py-3-yl |
| 341 | H | H | H | 3-Hx-Ph | Py-4-yl |
| 342 | H | H | H | 4-(1-MePn)-Ph | Ph |
| 343 | H | H | H | 4-(1-MePn) -Ph | 2-F-Ph |
| 344 | H | H | H | 4-(1-MePn)-Ph | 3-F-Ph |
| 345 | H | H | H | 4-(1-MePn)-Ph | 4-F-Ph |
| 346 | H | H | H | 4-(1-MePn)-Ph | 3,4-diF-Ph |
| 347 | H | H | H | 4-(1-MePn)-Ph | 3,5-diF-Ph |
| 348 | H | H | H | 4-(1-MePn)-Ph | 4-Cl-Ph |
| 349 | H | H | H | 4-(1-MePn)-Ph | 4-Me-Ph |
| 350 | H | H | H | 4-(1-MePn)-Ph | 4-$CF_3$-Ph |
| 351 | H | H | H | 4-(1-MePn)-Ph | 4-OMe-Ph |
| 352 | H | H | H | 4-(1-MePn)-Ph | Fu-2-yl |
| 353 | H | H | H | 4-(1-MePn)-Ph | Th-2-yl |
| 354 | H | H | H | 4-(1-MePn)-Ph | Th-3-yl |

(continued)

| Compound No. | $R^1$ | $R^2$ | $R^3$ | Y | Z |
|---|---|---|---|---|---|
| 355 | H | H | H | 4-(1-MePn)-Ph | 1-Me-Im-4-yl |
| 356 | H | H | H | 4-(1-MePn)-Ph | Tz-2-yl |
| 357 | H | H | H | 4-(1-MePn)-Ph | Py-2-yl |
| 358 | Me | H | H | 4-(1-MePn)-Ph | Py-2-yl |
| 359 | Et | H | H | 4-(1-MePn)-Ph | Py-2-yl |
| 360 | $Pr^1$ | H | H | 4-(1-MePn)-Ph | Py-2-yl |
| 361 | H | H | H | 4-(1-MePn)-Ph | 5-F-Py-2-yl |
| 362 | H | H | H | 4-(1-MePn)-Ph | Py-3-yl |
| 363 | H | H | H | 4-(1-MePn)-Ph | 6-F-Py-3-yl |
| 364 | H | H | H | 4-(1-MePn)-Ph | Py-4-yl |
| 365 | H | H | H | 5-(1-MePn)-Th-2-yl | Ph |
| 366 | H | H | H | 5-(1-MePn)-Th-2-yl | 2-F-Ph |
| 367 | H | H | H | 5-(1-MePn)-Th-2-yl | 3-F-Ph |
| 368 | H | H | H | 5-(1-MePn)-Th-2-yl | 4-F-Ph |
| 369 | H | H | H | 5-(1-MePn)-Th-2-yl | 4-Cl-Ph |
| 370 | H | H | H | 5-(1-MePn)-Th-2-yl | 4-OMe-Ph |
| 371 | H | H | H | 5-(1-MePn)-Th-2-yl | Fu-2-yl |
| 372 | H | H | H | 5-(1-MePn)-Th-2-yl | Th-2-yl |
| 373 | H | H | H | 5-(1-MePn)-Th-2-yl | 1-Me-Im-4-yl |
| 374 | H | H | H | 5-(1-MePn)-Th-2-yl | Tz-2-yl |
| 375 | H | H | H | 5-(1-MePn)-Th-2-yl | Py-2-yl |
| 376 | H | H | H | 5-(1-MePn)-Th-2-yl | Py-3-yl |
| 377 | H | H | H | 5-(1-MePn)-Th-2-yl | Py-4-yl |
| 378 | H | H | H | 3-(1,1-diMeBu)-Ph | Ph |
| 379 | H | H | H | 3-(1, 1-diMeBu)-Ph | 2-F-Ph |
| 380 | H | H | H | 3-(1,1-diMeBu)-Ph | 3-F-Ph |
| 381 | H | H | H | 3-(1, 1-diMeBu)-Ph | 4-F-Ph |
| 382 | H | H | H | 3-(1, 1-diMeBu)-Ph | 4-Cl-Ph |
| 383 | H | H | H | 3-(1, 1-diMeBu)-Ph | 4-OMe-Ph |
| 384 | H | H | H | 3-(1, 1-diMeBu)-Ph | Fu-2-yl |
| 385 | H | H | H | 3-(1, 1-diMeBu)-Ph | Th-2-yl |
| 386 | H | H | H | 3-(1, 1-diMeBu)-Ph | 1-Me-Im-4-yl |
| 387 | H | H | H | 3-(1, 1-diMeBu)-Ph | Tz-2-yl |
| 388 | H | H | H | 3-(1, 1-diMeBu)-Ph | Py-2-yl |
| 389 | H | H | H | 3-(1, 1-diMeBu)-Ph | Py-3-yl |
| 390 | H | H | H | 3-(1, 1-diMeBu)-Ph | Py-4-yl |
| 391 | H | H | H | 4-(1, 1-diMeBu)-Ph | Ph |
| 392 | H | H | H | 4-(1, 1-diMeBu)-Ph | 2-F-Ph |
| 393 | H | H | H | 4-(1, 1-diMeBu)-Ph | 3-F-Ph |
| 394 | H | H | H | 4-(1, 1-diMeBu)-Ph | 4-F-Ph |
| 395 | Me | H | H | 4-(1, 1-diMeBu)-Ph | 4-F-Ph |
| 396 | Et | H | H | 4- (1, 1-diMeBu) -Ph | 4-F-Ph |
| 397 | $Pr^1$ | H | H | 4-(1, 1-diMeBu)-Ph | 4-F-Ph |
| 398 | H | H | H | 4-(1, 1-diMeBu)-Ph | 3,4-diF-Ph |
| 399 | H | H | H | 4-(1, 1-diMeBu)-Ph | 3,5-diF-Ph |
| 400 | H | H | H | 4-(1,1-diMeBu)-Ph | 4-Cl-Ph |
| 401 | H | H | H | 4- (1, 1-diMeBu)-Ph | 4-Me-Ph |
| 402 | H | H | H | 4-(1,1-diMeBu)-Ph | 4-$CF_3$-Ph |
| 403 | H | H | H | 4-(1,1-diMeBu)-Ph | 4-OMe-Ph |
| 404 | H | H | H | 4-(1, 1-diMeBu)-Ph | Fu-2-yl |

(continued)

| Compound No. | $R^1$ | $R^2$ | $R^3$ | Y | Z |
|---|---|---|---|---|---|
| 405 | H | H | H | 4-(1,1-diMeBu)-Ph | Th-2-yl |
| 406 | H | H | H | 4-(1,1-diMeBu)-Ph | Th-3-yl |
| 407 | H | H | H | 4-(1,1-diMeBu)-Ph | 1-Me-Im-4-yl |
| 408 | H | H | H | 4-(1,1-diMeBu)-Ph | Tz-2-yl |
| 409 | H | H | H | 4-(1,1-diMeBu)-Ph | Py-2-yl |
| 410 | H | Me | H | 4-(1,1-diMeBu)-Ph | Py-2-yl |
| 411 | H | Me | Me | 4-(1, 1-diMeBu)-Ph | Py-2-yl |
| 412 | Me | H | H | 4-(1, 1-diMeBu)-Ph | Py-2-yl |
| 413 | Et | H | H | 4-(1,1-diMeBu)-Ph | Py-2-yl |
| 414 | $Pr^i$ | H | H | 4-(1,1-diMeBu)-Ph | Py-2-yl |
| 415 | H | H | H | 4-(1,1-diMeBu)-Ph | 5-F-Py-2-yl |
| 416 | H | H | H | 4-(1, 1-diMeBu)-Ph | Py-3-yl |
| 417 | Me | H | H | 4-(1, 1-diMeBu)-Ph | Py-3-yl |
| 418 | Et | H | H | 4-(1,1-diMeBu)-Ph | Py-3-yl |
| 419 | $Pr^1$ | H | H | 4-(1,1-diMeBu)-Ph | Py-3-yl |
| 420 | H | H | H | 4-(1, 1-diMeBu)-Ph | 6-F-Py-3-yl |
| 421 | H | H | H | 4-(1,1-diMeBu)-Ph | Py-4-yl |
| 422 | H | H | H | 2-F-4-(1,1-diMeBu)-Ph | Ph |
| 423 | H | H | H | 2-F-4-(1, 1-diMeBu)-Ph | 2-F-Ph |
| 424 | H | H | H | 2-F-4-(1,1-diMeBu)-Ph | 3-F-Ph |
| 425 | H | H | H | 2-F-4-(1,1-diMeBu)-Ph | 4-F-Ph |
| 426 | H | H | H | 2-F-4-(1,1-diMeBu)-Ph | 4-Cl-Ph |
| 427 | H | H | H | 2-F-4-(1,1-diMeBu)-Ph | 4-OMe-Ph |
| 428 | H | H | H | 2-F-4-(1,1-diMeBu)-Ph | Fu-2-yl |
| 429 | H | H | H | 2-F-4-(1,1-diMeBu)-Ph | Th-2-yl |
| 430 | H | H | H | 2-F-4-(1,1-diMeBu)-Ph | 1-Me-Im-4-yl |
| 431 | H | H | H | 2-F-4-(1,1-diMeBu)-Ph | Tz-2-yl |
| 432 | H | H | H | 2-F-4-(1,1-diMeBu)-Ph | Py-2-yl |
| 433 | H | H | H | 2-F-4-(1, 1-diMeBu)-Ph | Py-3-yl |
| 434 | H | H | H | 2-F-4-(1,1-diMeBu)-Ph | Py-4-yl |
| 435 | H | H | H | 3-F-4-(1,1-diMeBu)-Ph | Ph |
| 436 | H | H | H | 3-F-4-(1,1-diMeBu)-Ph | 2-F-Ph |
| 437 | H | H | H | 3-F-4-(1,1-diMeBu)-Ph | 3-F-Ph |
| 438 | H | H | H | 3-F-4-(1,1-diMeBu)-Ph | 4-F-Ph |
| 439 | H | H | H | 3-F-4-(1,1-diMeBu)-Ph | 4-Cl-Ph |
| 440 | H | H | H | 3-F-4-(1,1-diMeBu)-Ph | 4-OMe-Ph |
| 441 | H | H | H | 3-F-4-(1,1-diMeBu)-Ph | Fu-2-yl |
| 442 | H | H | H | 3-F-4-(1,1-diMeBu)-Ph | Th-2-yl |
| 443 | H | H | H | 3-F-4-(1,1-diMeBu)-Ph | 1-Me-Im-4-yl |
| 444 | H | H | H | 3-F-4-(1,1-diMeBu)-Ph | Tz-2-yl |
| 445 | H | H | H | 3-F-4-(1,1-diMeBu)-Ph | Py-2-yl |
| 446 | H | H | H | 3-F-4-(1,1-diMeBu)-Ph | Py-3-yl |
| 447 | H | H | H | 3-F-4-(1,1-diMeBu)-Ph | Py-4-yl |
| 448 | H | H | H | 4- (2, 2-diMeBu) -Ph | 4-F-Ph |
| 449 | H | H | H | 4-(2,2-diMeBu)-Ph | Py-2-yl |
| 450 | H | H | H | 4-(2,2-diMeBu)-Ph | Py-3-yl |
| 451 | H | H | H | 5-(1,1-diMeBu)-Th-2-yl | Ph |
| 452 | H | H | H | 5-(1,1-diMeBu)-Th-2-yl | 2-F-Ph |
| 453 | H | H | H | 5-(1,1-diMeBu)-Th-2-yl | 3-F-Ph |
| 454 | H | H | H | 5-(1,1-diMeBu)-Th-2-yl | 4-F-Ph |

(continued)

| Compound No. | $R^1$ | $R^2$ | $R^3$ | Y | Z |
|---|---|---|---|---|---|
| 455 | H | H | H | 5-(1,1-diMeBu)-Th-2-yl | 4-Cl-Ph |
| 456 | H | H | H | 5-(1,1-diMeBu)-Th-2-yl | 4-OMe-Ph |
| 457 | H | H | H | 5-(1,1-diMeBu)-Th-2-yl | Fu-2-yl |
| 458 | H | H | H | 5-(1,1-diMeBu)-Th-2-yl | Th-2-yl |
| 459 | H | H | H | 5-(1,1-diMeBu)-Th-2-yl | 1-Me-Im-4-yl |
| 460 | H | H | H | 5-(1,1-diMeBu)-Th-2-yl | Tz-2-yl |
| 461 | H | H | H | 5-(1,1-diMeBu)-Th-2-yl | Py-2-yl |
| 462 | H | H | H | 5-(1,1-diMeBu)-Th-2-yl | Py-3-yl |
| 463 | H | H | H | 5-(1,1-diMeBu)-Th-2-yl | Py-4-yl |
| 464 | H | H | H | 4-(1-Et-1-MePr)-Ph | Ph |
| 465 | H | H | H | 4-(1-Et-1-MePr)-Ph | 2-F-Ph |
| 466 | H | H | H | 4-(1-Et-1-MePr)-Ph | 3-F-Ph |
| 467 | H | H | H | 4-(1-Et-1-MePr)-Ph | 4-F-Ph |
| 468 | H | H | H | 4-(1-Et-1-MePr)-Ph | 3,4-diF-Ph |
| 469 | H | H | H | 4-(1-Et-1-MePr)-Ph | 3,5-diF-Ph |
| 470 | H | H | H | 4-(1-Et-1-MePr)-Ph | 4-Cl-Ph |
| 471 | H | H | H | 4-(1-Et-1-MePr)-Ph | 4-Me-Ph |
| 472 | H | H | H | 4-(1-Et-1-MePr)-Ph | 4-$CF_3$-Ph |
| 473 | H | H | H | 4-(1-Et-1-MePr)-Ph | 4-OMe-Ph |
| 474 | H | H | H | 4-(1-Et-1-MePr)-Ph | Fu-2-yl |
| 475 | H | H | H | 4-(1-Et-1-MePr)-Ph | Th-2-yl |
| 476 | H | H | H | 4-(1-Et-1-MePr)-Ph | Th-3-yl |
| 477 | H | H | H | 4-(1-Et-1-MePr)-Ph | 1-Me-Im-4-yl |
| 478 | H | H | H | 4-(1-Et-1-MePr)-Ph | Tz-2-yl |
| 479 | H | H | H | 4-(1-Et-1-MePr)-Ph | Py-2-yl |
| 480 | H | H | H | 4-(1-Et-1-MePr)-Ph | 5-F-Py-2-yl |
| 481 | H | H | H | 4-(1-Et-1-MePr)-Ph | Py-3-yl |
| 482 | Me | H | H | 4-(1-Et-1-MePr)-Ph | Py-3-yl |
| 483 | Et | H | H | 4-(1-Et-1-MePr)-Ph | Py-3-yl |
| 484 | $Pr^1$ | H | H | 4-(1-Et-1-MePr)-Ph | Py-3-yl |
| 485 | H | H | H | 4-(1-Et-1-MePr)-Ph | 6-F-Py-3-yl |
| 486 | H | H | H | 4-(1-Et-1-MePr)-Ph | Py-4-yl |
| 487 | H | H | H | 3-(1,1-diMePn)-Ph | Ph |
| 488 | H | H | H | 3-(1, 1-diMePn)-Ph | 2-F-Ph |
| 489 | H | H | H | 3-(1, 1-diMePn)-Ph | 3-F-Ph |
| 490 | H | H | H | 3-(1, 1-diMePn)-Ph | 4-F-Ph |
| 491 | H | H | H | 3-(1,1-diMePn)-Ph | 4-Cl-Ph |
| 492 | H | H | H | 3-(1, 1-diMePn)-Ph | 4-OMe-Ph |
| 493 | H | H | H | 3-(1, 1-diMePn)-Ph | Fu-2-yl |
| 494 | H | H | H | 3-(1, 1-diMePn)-Ph | Th-2-yl |
| 495 | H | H | H | 3-(1,1-diMePn)-Ph | 1-Me-Im-4-yl |
| 496 | H | H | H | 3-(1,1-diMePn)-Ph | Tz-2-yl |
| 497 | H | H | H | 3-(1, 1-diMePn)-Ph | Py-2-yl |
| 498 | H | H | H | 3-(1, 1-diMePn)-Ph | Py-3-yl |
| 499 | H | H | H | 3-(1, 1-diMePn)-Ph | Py-4-yl |
| 500 | H | H | H | 4-(1,1-diMePn)-Ph | Ph |
| 501 | Me | H | H | 4-(1, 1-diMePn)-Ph | Ph |
| 502 | Et | H | H | 4-(1, 1-diMePn)-Ph | Ph |
| 503 | $Pr^1$ | H | H | 4-(1, 1-diMePn)-Ph | Ph |
| 504 | H | H | H | 4-(1,1-diMePn)-Ph | 2-F-Ph |

(continued)

| Compound No. | $R^1$ | $R^2$ | $R^3$ | Y | Z |
|---|---|---|---|---|---|
| 505 | Me | H | H | 4-(1, 1-diMePn)-Ph | 2-F-Ph |
| 506 | Et | H | H | 4-(1, 1-diMePn)-Ph | 2-F-Ph |
| 507 | $Pr^1$ | H | H | 4-(1, 1-diMePn)-Ph | 2-F-Ph |
| 508 | H | H | H | 4-(1,1-diMePn)-Ph | 3-F-Ph |
| 509 | Me | H | H | 4-(1,1-diMePn)-Ph | 3-F-Ph |
| 510 | Et | H | H | 4-(1, 1-diMePn)-Ph | 3-F-Ph |
| 511 | $Pr^1$ | H | H | 4-(1,1-diMePn)-Ph | 3-F-Ph |
| 512 | H | H | H | 4-(1,1-diMePn)-Ph | 4-F-Ph |
| 513 | Me | H | H | 4-(1,1-diMePn)-Ph | 4-F-Ph |
| 514 | Et | H | H | 4-(1, 1-diMePn)-Ph | 4-F-Ph |
| 515 | $Pr^1$ | H | H | 4-(1,1-diMePn)-Ph | 4-F-Ph |
| 516 | H | H | H | 4-(1,1-diMePn)-Ph | 3,4-diF-Ph |
| 517 | H | H | H | 4-(1, 1-diMePn)-Ph | 3,5-diF-Ph |
| 518 | H | H | H | 4-(1,1-diMePn)-Ph | 2-Cl-Ph |
| 519 | H | H | H | 4-(1,1-diMePn)-Ph | 3-Cl-Ph |
| 520 | H | H | H | 4-(1,1-diMePn)-Ph | 4-Cl-Ph |
| 521 | Me | H | H | 4-(1,1-diMePn)-Ph | 4-Cl-Ph |
| 522 | Et | H | H | 4-(1,1-diMePn)-Ph | 4-Cl-Ph |
| 523 | $Pr^1$ | H | H | 4-(1, 1-diMePn)-Ph | 4-Cl-Ph |
| 524 | H | H | H | 4-(1,1-diMePn)-Ph | 2,6-diCl-Ph |
| 525 | H | H | H | 4-(1, 1-diMePn)-Ph | 4-Cl-3-F-Ph |
| 526 | H | H | H | 4-(1,1-diMePn)-Ph | 4-Me-Ph |
| 527 | H | H | H | 4-(1,1-diMePn)-Ph | 3-F-4-Me-Ph |
| 528 | H | H | H | 4-(1,1-diMePn)-Ph | 4-$CF_3$-Ph |
| 529 | H | H | H | 4-(1,1-diMePn)-Ph | 3-F-4-$CF_3$-Ph |
| 530 | H | H | H | 4-(1,1-diMePn)-Ph | 4-OMe-Ph |
| 531 | Me | H | H | 4-(1, 1-diMePn)-Ph | 4-OMe-Ph |
| 532 | Et | H | H | 4-(1,1-diMePn)-Ph | 4-OMe-Ph |
| 533 | $Pr^1$ | H | H | 4-(1, 1-diMePn)-Ph | 4-OMe-Ph |
| 534 | H | H | H | 4-(1,1-diMePn)-Ph | 3-F-4-OMe-Ph |
| 535 | H | H | H | 4-(1, 1-diMePn)-Ph | 4-$OCHF_2$-Ph |
| 536 | H | H | H | 4-(1, 1-diMePn)-Ph | 4-$OCHF_2$-3-F-Ph |
| 537 | H | H | H | 4-(1, 1-diMePn)-Ph | Fu-2-yl |
| 538 | Me | H | H | 4-(1,1-diMePn)-Ph | Fu-2-yl |
| 539 | Et | H | H | 4-(1, 1-diMePn)-Ph | Fu-2-yl |
| 540 | $Pr^1$ | H | H | 4-(1,1-diMePn)-Ph | Fu-2-yl |
| 541 | H | H | H | 4-(1, 1-diMePn)-Ph | Th-2-yl |
| 542 | Me | H | H | 4-(1, 1-diMePn)-Ph | Th-2-yl |
| 543 | Et | H | H | 4-(1,1-diMePn)-Ph | Th-2-yl |
| 544 | $Pr^1$ | H | H | 4-(1, 1-diMePn)-Ph | Th-2-yl |
| 545 | H | H | H | 4-(1,1-diMePn)-Ph | Th-3-yl |
| 546 | H | H | H | 4-(1,1-diMePn)-Ph | 1-Me-Im-4-yl |
| 547 | H | H | H | 4-(1, 1-diMePn)-Ph | Tz-2-yl |
| 548 | Me | H | H | 4-(1,1-diMePn)-Ph | Tz-2-yl |
| 549 | Et | H | H | 4-(1, 1-diMePn)-Ph | Tz-2-yl |
| 550 | $Pr^1$ | H | H | 4-(1, 1-diMePn)-Ph | Tz-2-yl |
| 551 | H | H | H | 4-(1,1-diMePn)-Ph | Py-2-yl |
| 552 | H | Me | H | 4-(1, 1-diMePn)-Ph | Py-2-yl |
| 553 | H | Me | Me | 4-(1, 1-diMePn)-Ph | Py-2-yl |
| 554 | Me | H | H | 4-(1, 1-diMePn)-Ph | Py-2-yl |

(continued)

| Compound No. | $R^1$ | $R^2$ | $R^3$ | Y | Z |
|---|---|---|---|---|---|
| 555 | Et | H | H | 4-(1,1-diMePn)-Ph | Py-2-yl |
| 556 | $Pr^1$ | H | H | 4-(1, 1-diMePn)-Ph | Py-2-yl |
| 557 | H | H | H | 4-(1,1-diMePn)-Ph | 5-F-Py-2-yl |
| 558 | H | H | H | 4-(1,1-diMePn)-Ph | 5-Cl-Py-2-yl |
| 559 | H | H | H | 4-(1, 1-diMePn)-Ph | 5-OMe-Py-2-yl |
| 560 | H | H | H | 4- (1, 1-diMePn)-Ph | Py-3-yl |
| 561 | Me | H | H | 4- (1, 1-diMePn)-Ph | Py-3-yl |
| 562 | Et | H | H | 4-(1, 1-diMePn)-Ph | Py-3-yl |
| 563 | $Pr^1$ | H | H | 4-(1,1-diMePn)-Ph | Py-3-yl |
| 564 | H | H | H | 4-(1, 1-diMePn)-Ph | 6-F-Py-3-yl |
| 565 | H | H | H | 4-(1,1-diMePn)-Ph | 6-Cl-Py-3-yl |
| 566 | H | H | H | 4-(1,1-diMePn)-Ph | 6-OMe-Py-3-yl |
| 567 | H | H | H | 4- (1, 1-diMePn)-Ph | Py-4-yl |
| 568 | Me | H | H | 4-(1,1-diMePn)-Ph | Py-4-yl |
| 569 | Et | H | H | 4-(1,1-diMePn)-Ph | Py-4-yl |
| 570 | $Pr^1$ | H | H | 4-(1,1-diMePn)-Ph | Py-4-yl |
| 571 | H | H | H | 2-F-4-(1,1-diMePn)-Ph | Ph |
| 572 | H | H | H | 2-F-4-(1,1-diMePn)-Ph | 2-F-Ph |
| 573 | H | H | H | 2-F-4-(1, 1-diMePn)-Ph | 3-F-Ph |
| 574 | H | H | H | 2-F-4-(1,1-diMePn)-Ph | 4-F-Ph |
| 575 | H | H | H | 2-F-4-(1, 1-diMePn)-Ph | 3,4-diF-Ph |
| 576 | H | H | H | 2-F-4-(1, 1-diMePn)-Ph | 3,6-diF-Ph |
| 577 | H | H | H | 2-F-4-(1,1-diMePn)-Ph | 4-Cl-Ph |
| 578 | H | H | H | 2-F-4-(1,1-diMePn)-Ph | 4-Me-Ph |
| 579 | H | H | H | 2-F-4-(1,1-diMePn)-Ph | 4-$CF_3$-Ph |
| 580 | H | H | H | 2-F-4-(1,1-diMePn)-Ph | 4-OMe-Ph |
| 581 | H | H | H | 2-F-4-(1,1-diMePn)-Ph | Fu-2-yl |
| 582 | H | H | H | 2-F-4-(1,1-diMePn)-Ph | Th-2-yl |
| 583 | H | H | H | 2-F-4-(1,1-diMePn)-Ph | Th-3-yl |
| 584 | H | H | H | 2-F-4-(1,1-diMePn)-Ph | 1-Me-Im-4-yl |
| 585 | H | H | H | 2-F-4-(1,1-diMePn)-Ph | Tz-2-yl |
| 586 | H | H | H | 2-F-4-(1,1-diMePn)-Ph | Py-2-yl |
| 587 | Me | H | H | 2-F-4-(1,1-diMePn)-Ph | Py-2-yl |
| 588 | Et | H | H | 2-F-4-(1, 1-diMePn)-Ph | Py-2-yl |
| 589 | $Pr^1$ | H | H | 2-F-4-(1,1-diMePn)-Ph | Py-2-yl |
| 590 | H | H | H | 2-F-4-(1,1-diMePn)-Ph | 5-F-Py-2-yl |
| 591 | H | H | H | 2-F-4-(1,1-diMePn)-Ph | Py-3-yl |
| 592 | H | H | H | 2-F-4-(1,1-diMePn)-Ph | 6-F-Py-3-yl |
| 593 | H | H | H | 2-F-4-(1,1-diMePn)-Ph | Py-4-yl |
| 594 | H | H | H | 3-F-4-(1,1-diMePn)-Ph | Ph |
| 595 | H | H | H | 3-F-4-(1,1-diMePn)-Ph | 2-F-Ph |
| 596 | H | H | H | 3-F-4-(1,1-diMePn)-Ph | 3-F-Ph |
| 597 | H | H | H | 3-F-4-(1,1-diMePn)-Ph | 4-F-Ph |
| 598 | H | H | H | 3-F-4-(1,1-diMePn)-Ph | 3,4-diF-Ph |
| 599 | H | H | H | 3-F-4-(1, 1-diMePn)-Ph | 3,5-diF-Ph |
| 600 | H | H | H | 3-F-4-(1,1-diMePn)-Ph | 4-Cl-Ph |
| 601 | H | H | H | 3-F-4-(1,1-diMePn)-Ph | 4-Me-Ph |
| 602 | H | H | H | 3-F-4-(1,1-diMePn)-Ph | 4-$CF_3$-Ph |
| 603 | H | H | H | 3-F-4-(1,1-diMePn)-Ph | 4-OMe-Ph |
| 604 | H | H | H | 3-F-4-(1,1-diMePn)-Ph | Fu-2-yl |

(continued)

| Compound No. | $R^1$ | $R^2$ | $R^3$ | Y | Z |
|---|---|---|---|---|---|
| 605 | H | H | H | 3-F-4-(1,1-diMePn)-Ph | Th-2-yl |
| 606 | H | H | H | 3-F-4-(1,1-diMePn)-Ph | Th-3-yl |
| 607 | H | H | H | 3-F-4-(1, 1-diMePn)-Ph | 1-Me-Im-4-yl |
| 608 | H | H | H | 3-F-4-(1,1-diMePn)-Ph | Tz-2-yl |
| 609 | H | H | H | 3-F-4-(1,1-diMePn)-Ph | Py-2-yl |
| 610 | Me | H | H | 3-F-4-(1,1-diMePn)-Ph | Py-2-yl |
| 611 | Et | H | H | 3-F-4-(1,1-diMePn)-Ph | Py-2-yl |
| 612 | $Pr^1$ | H | H | 3-F-4-(1,1-diMePn)-Ph | Py-2-yl |
| 613 | H | H | H | 3-F-4-(1,1-diMePn)-Ph | 5-F-Py-2-yl |
| 614 | H | H | H | 3-F-4-(1,1-diMePn)-Ph | Py-3-yl |
| 615 | H | H | H | 3-F-4-(1,1-diMePn)-Ph | 6-F-Py-3-yl |
| 616 | H | H | H | 3-F-4-(1,1-diMePn)-Ph | Py-4-yl |
| 617 | H | H | H | 3-$NH_2$-4-(1,1-diMePn)-Ph | 4-F-Ph |
| 618 | H | H | H | 3-$NH_2$-4-(1, 1-diMePn)-Ph | Py-2-yl |
| 619 | H | H | H | 3-$NH_2$-4-(1,1-diMePn)-Ph | Py-3-yl |
| 620 | H | H | H | 3-NMe-4-(1,1-diMePn)-Ph | 4-F-Ph |
| 621 | H | H | H | 3-NMe-4-(1,1-diMePn)-Ph | Py-2-yl |
| 622 | H | H | H | 3-NMe-4-(1, 1-diMePn)-Ph | Py-3-yl |
| 623 | H | H | H | 3-$NMe_2$-4-(1,1-diMePn)-Ph | Ph |
| 624 | H | H | H | 3-$NMe_2$-4-(1,1-diMePn)-Ph | 2-F-Ph |
| 625 | H | H | H | 3-$NMe_2$-4-(1,1-diMePn)-Ph | 3-F-Ph |
| 626 | H | H | H | 3-$NMe_2$-4-(1,1-diMePn)-Ph | 4-F-Ph |
| 627 | H | H | H | 3-$NMe_2$-4-(1,1-diMePn)-Ph | 4-Cl-Ph |
| 628 | H | H | H | 3-$NMe_2$-4-(1,1-diMePn)-Ph | 4-OMe-Ph |
| 629 | H | H | H | 3-$NMe_2$-4-(1,1-diMePn)-Ph | Fu-2-yl |
| 630 | H | H | H | 3-$NMe_2$-4-(1,1-diMePn)-Ph | Th-2-yl |
| 631 | H | H | H | 3-$NMe_2$-4-(1,1-diMePn)-Ph | 1-Me-Im-4-yl |
| 632 | H | H | H | 3-$NMe_2$-4-(1,1-diMePn)-Ph | Tz-2-yl |
| 633 | H | H | H | 3-$NMe_2$-4-(1,1-diMePn)-Ph | Py-2-yl |
| 634 | H | H | H | 3-$NMe_2$-4-(1,1-diMePn)-Ph | Py-3-yl |
| 635 | H | H | H | 3-$NMe_2$-4-(1,1-diMePn)-Ph | Py-4-yl |
| 636 | H | H | H | 4-(1,1-diMePn)-Th-2-yl | 4-F-Ph |
| 637 | H | H | H | 4-(1,1-diMePn)-Th-2-yl | Py-2-yl |
| 638 | H | H | H | 4-(1,1-diMePn)-Th-2-yl | Py-3-yl |
| 639 | H | H | H | 5-(1,1-diMePn)-Th-2-yl | Ph |
| 640 | H | H | H | 5-(1,1-diMePn)-Th-2-yl | 2-F-Ph |
| 641 | H | H | H | 5-(1,1-diMePn)-Th-2-yl | 3-F-Ph |
| 642 | H | H | H | 5-(1,1-diMePn)-Th-2-yl | 4-F-Ph |
| 643 | Me | H | H | 5-(1,1-diMePn)-Th-2-yl | 4-F-Ph |
| 644 | Et | H | H | 5-(1, 1-diMePn)-Th-2-yl | 4-F-Ph |
| 645 | $Pr^1$ | H | H | 5-(1,1-diMePn)-Th-2-yl | 4-F-Ph |
| 646 | H | H | H | 5-(1, 1-diMePn)-Th-2-yl | 3,4-diF-Ph |
| 647 | H | H | H | 5-(1,1-diMePn)-Th-2-yl | 3,5-diF-Ph |
| 648 | H | H | H | 5- (1, 1-diMePn)-Th-2-yl | 2-Cl-Ph |
| 649 | H | H | H | 5-(1,1-diMePn)-Th-2-yl | 3-Cl-Ph |
| 650 | H | H | H | 5-(1,1-diMePn)-Th-2-yl | 4-Cl-Ph |
| 651 | H | H | H | 5-(1,1-diMePn)-Th-2-yl | 2,6-diCl-Ph |
| 652 | H | H | H | 5-(1,1-diMePn)-Th-2-yl | 4-Cl-3-F-Ph |
| 653 | H | H | H | 5-(1,1-diMePn)-Th-2-yl | 4-Me-Ph |
| 654 | H | H | H | 5-(1,1-diMePn)-Th-2-yl | 3-F-4-Me-Ph |

(continued)

| Compound No. | $R^1$ | $R^2$ | $R^3$ | Y | Z |
|---|---|---|---|---|---|
| 655 | H | H | H | 5-(1, 1-diMePn)-Th-2-yl | 4-$CF_3$-Ph |
| 656 | H | H | H | 5- (1, 1-diMePn)-Th-2-yl | 3-F-4-$CF_3$-Ph |
| 657 | H | H | H | 5-(1,1-diMePn)-Th-2-yl | 4-OMe-Ph |
| 658 | H | H | H | 5-(1,1-diMePn)-Th-2-yl | 3-F-4-OMe-Ph |
| 659 | H | H | H | 5-(1, 1-diMePn)-Th-2-yl | 4-$OCHF_2$-Ph |
| 660 | H | H | H | 5-(1,1-diMePn)-Th-2-yl | 4-$OCHF_2$-3-F-Ph |
| 661 | H | H | H | 5-(1,1-diMePn)-Th-2-yl | Fu-2-yl |
| 662 | H | H | H | 5-(1,1-diMePn)-Th-2-yl | Th-2-yl |
| 663 | H | H | H | 5-(1,1-diMePn)-Th-2-yl | Th-3-yl |
| 664 | H | H | H | 5-(1, 1-diMePn)-Th-2-yl | 1-Me-Im-4-yl |
| 665 | H | H | H | 5-(1, 1-diMePn)-Th-2-yl | Tz-2-yl |
| 666 | H | H | H | 5-(1,1-diMePn)-Th-2-yl | Py-2-yl |
| 667 | Me | H | H | 5-(1, 1-diMePn)-Th-2-yl | Py-2-yl |
| 668 | Et | H | H | 5-(1,1-diMePn)-Th-2-yl | Py-2-yl |
| 669 | $Pr^1$ | H | H | 5-(1, 1-diMePn)-Th-2-yl | Py-2-yl |
| 670 | H | H | H | 5-(1,1-diMePn)-Th-2-yl | 5-F-Py-2-yl |
| 671 | H | H | H | 5-(1,1-diMePn)-Th-2-yl | 5-Cl-Py-2-yl |
| 672 | H | H | H | 5-(1,1-diMePn)-Th-2-yl | 5-OMe-Py-2-yl |
| 673 | H | H | H | 5-(1, 1-diMePn)-Th-2-yl | Py-3-yl |
| 674 | Me | H | H | 5-(1, 1-diMePn)-Th-2-yl | Py-3-yl |
| 675 | Et | H | H | 5-(1,1-diMePn)-Th-2-yl | Py-3-yl |
| 676 | $Pr^1$ | H | H | 5-(1,1-diMePn)-Th-2-yl | Py-3-yl |
| 677 | H | H | H | 5-(1, 1-diMePn)-Th-2-yl | 6-F-Py-3-yl |
| 678 | H | H | H | 5-(1, 1-diMePn)-Th-2-yl | 6-Cl-Py-3-yl |
| 679 | H | H | H | 5-(1,1-diMePn)-Th-2-yl | 6-OMe-Py-3-yl |
| 680 | H | H | H | 5-(1,1-diMePn)-Th-2-yl | Py-4-yl |
| 681 | H | H | H | 5-(1,1-diMePn)-Tz-2-yl | 4-F-Ph |
| 682 | H | H | H | 5-(1,1-diMePn)-Tz-2-yl | Py-2-yl |
| 683 | H | H | H | 5-(1,1-diMePn)-Tz-2-yl | Py-3-yl |
| 684 | H | H | H | 5-(1, 1-diMePn)-Py-2-yl | 4-F-Ph |
| 685 | H | H | H | 5-(1,1-diMePn)-Py-2-yl | Py-2-yl |
| 686 | H | H | H | 5-(1,1-diMePn)-Py-2-yl | Py-3-yl |
| 687 | H | H | H | 6-(1,1-diMePn)-Py-3-yl | 4-F-Ph |
| 688 | H | H | H | 6-(1,1-diMePn)-Py-3-yl | Py-2-yl |
| 689 | H | H | H | 6-(1,1-diMePn)-Py-3-yl | Py-3-yl |
| 690 | H | H | H | 5-(1,1-diMePn)-Pm-2-yl | 4-F-Ph |
| 691 | H | H | H | 5-(1,1-diMePn)-Pm-2-yl | Py-2-yl |
| 692 | H | H | H | 5-(1,1-diMePn)-Pm-2-yl | Py-3-yl |
| 693 | H | H | H | 2-(1,1-diMePn)-Pm-5-yl | 4-F-Ph |
| 694 | H | H | H | 2-(1,1-diMePn)-Pm-5-yl | Py-2-yl |
| 695 | H | H | H | 2-(1,1-diMePn)-Pm-5-yl | Py-3-yl |
| 696 | H | H | H | 3-(2,2-diMePn)-Ph | 4-F-Ph |
| 697 | H | H | H | 3-(2,2-diMePn)-Ph | Py-2-yl |
| 698 | H | H | H | 3-(2,2-diMePn)-Ph | Py-3-yl |
| 699 | H | H | H | 4-(2,2-diMePn)-Ph | Ph |
| 700 | H | H | H | 4-(2,2-diMePn)-Ph | 2-F-Ph |
| 701 | H | H | H | 4-(2,2-diMePn)-Ph | 3-F-Ph |
| 702 | H | H | H | 4- (2, 2-diMePn) -Ph | 4-F-Ph |
| 703 | H | H | H | 4-(2,2-diMePn)-Ph | 3,4-diF-Ph |
| 704 | H | H | H | 4-(2,2-diMePn)-Ph | 3,5-diF-Ph |

(continued)

| Compound No. | $R^1$ | $R^2$ | $R^3$ | Y | Z |
|---|---|---|---|---|---|
| 705 | H | H | H | 4-(2,2-diMePn)-Ph | 4-Cl-Ph |
| 706 | H | H | H | 4- (2, 2-diMePn) -Ph | 4-Me-Ph |
| 707 | H | H | H | 4-(2,2-diMePn)-Ph | 4-$CF_3$-Ph |
| 708 | H | H | H | 4-(2,2-diMePn)-Ph | 4-OMe-Ph |
| 709 | H | H | H | 4-(2, 2-diMePn)-Ph | Fu-2-yl |
| 710 | H | H | H | 4-(2,2-diMePn)-Ph | Th-2-yl |
| 711 | H | H | H | 4-(2,2-diMePn)-Ph | Th-3-yl |
| 712 | H | H | H | 4-(2,2-diMePn)-Ph | 1-Me-Im-4-yl |
| 713 | H | H | H | 4-(2,2-diMePn)-Ph | Tz-2-yl |
| 714 | H | H | H | 4-(2, 2-diMePn)-Ph | Py-2-yl |
| 715 | Me | H | H | 4-(2, 2-diMePn)-Ph | Py-2-yl |
| 716 | Et | H | H | 4-(2, 2-diMePn)-Ph | Py-2-yl |
| 717 | $Pr^1$ | H | H | 4-(2,2-diMePn)-Ph | Py-2-yl |
| 718 | H | H | H | 4-(2,2-diMePn)-Ph | 5-F-Py-2-yl |
| 719 | H | H | H | 4-(2, 2-diMePn)-Ph | Py-3-yl |
| 720 | H | H | H | 4-(2,2-diMePn)-Ph | 6-F-Py-3-yl |
| 721 | H | H | H | 4-(2, 2-diMePn)-Ph | Py-4-yl |
| 722 | H | H | H | 4-(1-MeHx)-Ph | 4-F-Ph |
| 723 | H | H | H | 4-(1-MeHx)-Ph | Py-2-yl |
| 724 | H | H | H | 4-(1-MeHx)-Ph | Py-3-yl |
| 725 | H | H | H | 4-(1, 1, 3, 3-tetraMeBu)-Ph | Ph |
| 726 | H | H | H | 4-(1,1, 3, 3-tetraMeBu)-Ph | 2-F-Ph |
| 727 | H | H | H | 4-(1,1, 3, 3-tetraMeBu)-Ph | 3-F-Ph |
| 728 | H | H | H | 4-(1, 1, 3, 3-tetraMeBu)-Ph | 4-F-Ph |
| 729 | H | H | H | 4-(1,1,3,3-tetraMeBu)-Ph | 3,4-diF-Ph |
| 730 | H | H | H | 4-(1,1, 3, 3-tetraMeBu)-Ph | 3,5-diF-Ph |
| 731 | H | H | H | 4-(1,1,3, 3-tetraMeBu)-Ph | 4-Cl-Ph |
| 732 | H | H | H | 4-(1, 1, 3, 3-tetraMeBu)-Ph | 4-Me-Ph |
| 733 | H | H | H | 4-(1, 1,3,3-tetraMeBu)-Ph | 4-$CF_3$-Ph |
| 734 | H | H | H | 4-(1,1,3,3-tetraMeBu)-Ph | 4-OMe-Ph |
| 735 | H | H | H | 4-(1, 1, 3, 3-tetraMeBu)-Ph | Fu-2-yl |
| 736 | H | H | H | 4-(1,1,3, 3-tetraMeBu)-Ph | Th-2-yl |
| 737 | H | H | H | 4-(1,1,3,3-tetraMeBu)-Ph | Th-3-yl |
| 738 | H | H | H | 4-(1,1,3,3-tetraMeBu)-Ph | 1-Me-Im-4-yl |
| 739 | H | H | H | 4-(1,1,3,3-tetraMeBu)-Ph | Tz-2-yl |
| 740 | H | H | H | 4-(1,1,3,3-tetraMeBu)-Ph | Py-2-yl |
| 741 | Me | H | H | 4-(1,1,3, 3-tetraMeBu)-Ph | Py-2-yl |
| 742 | Et | H | H | 4-(1,1,3,3-tetraMeBu)-Ph | Py-2-yl |
| 743 | $Pr^1$ | H | H | 4-(1,1,3,3-tetraMeBu)-Ph | Py-2-yl |
| 744 | H | H | H | 4-(1,1,3,3-tetraMeBu)-Ph | 5-F-Py-2-yl |
| 745 | H | H | H | 4-(1,1,3,3-tetraMeBu)-Ph | Py-3-yl |
| 746 | H | H | H | 4-(1,1,3,3-tetraMeBu)-Ph | 6-F-Py-3-yl |
| 747 | H | H | H | 4-(1,1,3,3-tetraMeBu)-Ph | Py-4-yl |
| 748 | H | H | H | 4-(1,1,4-triMePn)-Ph | Ph |
| 749 | H | H | H | 4-(1,1,4-triMePn)-Ph | 2-F-Ph |
| 750 | H | H | H | 4-(1,1,4-triMePn)-Ph | 3-F-Ph |
| 751 | H | H | H | 4-(1,1,4-triMePn)-Ph | 4-F-Ph |
| 752 | H | H | H | 4-(1,1,4-triMePn)-Ph | 3,4-diF-Ph |
| 753 | H | H | H | 4-(1,1,4-triMePn)-Ph | 3,5-diF-Ph |
| 754 | H | H | H | 4-(1, 1, 4-triMePn)-Ph | 4-Cl-Ph |

(continued)

| Compound No. | $R^1$ | $R^2$ | $R^3$ | Y | Z |
|---|---|---|---|---|---|
| 755 | H | H | H | 4-(1,1,4-triMePn)-Ph | 4-Me-Ph |
| 756 | H | H | H | 4-(1, 1,4-triMePn)-Ph | 4-$CF_3$-Ph |
| 757 | H | H | H | 4-(1, 1, 4-triMePn)-Ph | 4-OMe-Ph |
| 758 | H | H | H | 4-(1,1,4-triMePn)-Ph | Fu-2-yl |
| 759 | H | H | H | 4-(1,1,4-triMePn)-Ph | Th-2-yl |
| 760 | H | H | H | 4-(1,1,4-triMePn)-Ph | Th-3-yl |
| 761 | H | H | H | 4-(1,1,4-triMePn)-Ph | 1-Me-Im-4-yl |
| 762 | H | H | H | 4-(1, 1, 4-triMePn)-Ph | Tz-2-yl |
| 763 | H | H | H | 4-(1,1,4-triMePn)-Ph | Py-2-yl |
| 764 | Me | H | H | 4-(1,1,4-triMePn)-Ph | Py-2-yl |
| 765 | Et | H | H | 4-(1,1,4-triMePn)-Ph | Py-2-yl |
| 766 | $Pr^1$ | H | H | 4-(1, 1, 4-triMePn)-Ph | Py-2-yl |
| 767 | H | H | H | 4-(1,1,4-triMePn)-Ph | 5-F-Py-2-yl |
| 768 | H | H | H | 4-(1,1,4-triMePn)-Ph | Py-3-yl |
| 769 | H | H | H | 4-(1,1, 4-triMePn)-Ph | 6-F-Py-3-yl |
| 770 | H | H | H | 4-(1, 1,4-triMePn)-Ph | Py-4-yl |
| 771 | H | H | H | 4-(1,1-diMeHx)-Ph | Ph |
| 772 | H | H | H | 4-(1,1-diMeHx)-Ph | 2-F-Ph |
| 773 | H | H | H | 4-(1, 1-diMeHx)-Ph | 3-F-Ph |
| 774 | H | H | H | 4-(1, 1-diMeHx)-Ph | 4-F-Ph |
| 775 | H | H | H | 4-(1,1-diMeHx)-Ph | 3,4-diF-Ph |
| 776 | H | H | H | 4-(1, 1-diMeHx)-Ph | 3,5-diF-Ph |
| 777 | H | H | H | 4-(1,1-diMeHx)-Ph | 4-Cl-Ph |
| 778 | H | H | H | 4-(1, 1-diMeHx)-Ph | 4-Me-Ph |
| 779 | H | H | H | 4-(1, 1-diMeHx)-Ph | 4-$CF_3$-Ph |
| 780 | H | H | H | 4-(1, 1-diMeHx)-Ph | 4-OMe-Ph |
| 781 | H | H | H | 4-(1, 1-diMeHx)-Ph | Fu-2-yl |
| 782 | H | H | H | 4-(1,1-diMeHx)-Ph | Th-2-yl |
| 783 | H | H | H | 4-(1,1-diMeHx)-Ph | Th-3-yl |
| 784 | H | H | H | 4-(1, 1-diMeHx)-Ph | 1-Me-Im-4-yl |
| 785 | H | H | H | 4-(1, 1-diMeHx)-Ph | Tz-2-yl |
| 786 | H | H | H | 4-(1,1-diMeHx)-Ph | Py-2-yl |
| 787 | Me | H | H | 4-(1, 1-diMeHx)-Ph | Py-2-yl |
| 788 | Et | H | H | 4-(1,1-diMeHx)-Ph | Py-2-yl |
| 789 | $Pr^1$ | H | H | 4-(1,2-diMeHx)-Ph | Py-2-yl |
| 790 | H | H | H | 4-(1, 1-diMeHx)-Ph | 5-F-Py-2-yl |
| 791 | H | H | H | 4-(1, 1-diMeHx)-Ph | Py-3-yl |
| 792 | H | H | H | 4-(1, 1-diMeHx)-Ph | 6-F-Py-3-yl |
| 793 | H | H | H | 4-(1, 1-diMeHx)-Ph | Py-4-yl |
| 794 | H | H | H | 4-$CF_3$-Ph | Ph |
| 795 | H | H | H | 4-$CF_3$-Ph | 2-F-Ph |
| 796 | H | H | H | 4-$CF_3$-Ph | 3-F-Ph |
| 797 | H | H | H | 4-$CF_3$-Ph | 4-F-Ph |
| 798 | H | H | H | 4-$CF_3$-Ph | 3,4-diF-Ph |
| 799 | H | H | H | 4-$CF_3$-Ph | 3,5-diF-Ph |
| 800 | H | H | H | 4-$CF_3$-Ph | 4-Cl-Ph |
| 801 | H | H | H | 4-$CF_3$-Ph | 4-Me-Ph |
| 802 | H | H | H | 4-$CF_3$-Ph | 4-$CF_3$-Ph |
| 803 | H | H | H | 4-$CF_3$-Ph | 4-OMe-Ph |
| 804 | H | H | H | 4-$CF_3$-Ph | Fu-2-yl |

(continued)

| Compound No. | $R^1$ | $R^2$ | $R^3$ | Y | Z |
|---|---|---|---|---|---|
| 805 | H | H | H | 4-$CF_3$-Ph | Th-2-yl |
| 806 | H | H | H | 4-$CF_3$-Ph | Th-3-yl |
| 807 | H | H | H | 4-$CF_3$-Ph | 1-Me-Im-4-yl |
| 808 | H | H | H | 4-$CF_3$-Ph | Tz-2-yl |
| 809 | H | H | H | 4-$CF_3$-Ph | Py-2-yl |
| 810 | H | H | H | 4-$CF_3$-Ph | 5-F-Py-2-yl |
| 811 | H | H | H | 4-$CF_3$-Ph | Py-3-yl |
| 812 | Me | H | H | 4-$CF_3$-Ph | Py-3-yl |
| 813 | Et | H | H | 4-$CF_3$-Ph | Py-3-yl |
| 814 | $Pr^1$ | H | H | 4-$CF_3$-Ph | Py-3-yl |
| 815 | H | H | H | 4-$CF_3$-Ph | 6-F-Py-3-yl |
| 816 | H | H | H | 4-$CF_3$-Ph | Py-4-yl |
| 817 | H | H | H | 4-(4, 4, 4-triFBu)-Ph | 4-F-Ph |
| 818 | H | H | H | 4-(4, 4, 4-triFBu)-Ph | Py-2-yl |
| 819 | H | H | H | 4-(4, 4, 4-triFBu)-Ph | Py-3-yl |
| 820 | H | H | H | 4-(5, 5, 5-triFPn)-Ph | 4-F-Ph |
| 821 | H | H | H | 4-(5, 5, 5-triFPn)-Ph | Py-2-yl |
| 822 | H | H | H | 4- (5, 5, 5-triFPn) -Ph | Py-3-yl |
| 823 | H | H | H | 4-(6, 6 ,6-triFHx)-Ph | 4-F-Ph |
| 824 | H | H | H | 4-(6, 6, 6-triFHx)-Ph | Py-2-yl |
| 825 | H | H | H | 4-(6, 6, 6-triFHx)-Ph | Py-3-yl |
| 826 | H | H | H | 4-(6, 5, 5-triF-1-MePn)-Ph | 4-F-Ph |
| 827 | H | H | H | 4-(5, 5, 5-triF-1-MePn)-Ph | Py-2-yl |
| 828 | H | H | H | 4-(5, 5, 5-triF-1-MePn)-Ph | Py-3-yl |
| 829 | H | H | H | 4-(4, 4, 4-triF-1,1-diMeBu)-Ph | Ph |
| 830 | H | H | H | 4-(4, 4, 4-triF-1,1-diMeBu)-Ph | 2-F-Ph |
| 831 | H | H | H | 4-(4, 4, 4-triF-1, 1-diMeBu)-Ph | 3-F-Ph |
| 832 | H | H | H | 4-(4, 4, 4-triF-1, 1-diMeBu)-Ph | 4-F-Ph |
| 833 | H | H | H | 4-(4, 4, 4-triF-1, 1-diMeBu)-Ph | 4-Cl-Ph |
| 834 | H | H | H | 4-(4, 4, 4-triF-1, 1-diMeBu)-Ph | 4-OMe-Ph |
| 835 | H | H | H | 4-(4, 4, 4-triF-1, 1-diMeBu)-Ph | Fu-2-yl |
| 836 | H | H | H | 4-(4, 4, 4-triF-1, 1-diMeBu)-Ph | Th-2-yl |
| 837 | H | H | H | 4-(4, 4, 4-triF-1, 1-diMeBu)-Ph | 1-Me-Im-4-yl |
| 838 | H | H | H | 4-(4, 4, 4-triF-1, 1-diMeBu)-Ph | Tz-2-yl |
| 839 | H | H | H | 4-(4, 4, 4-triF-1, 1-diMeBu)-Ph | Py-2-yl |
| 840 | H | H | H | 4-(4, 4, 4-triF-1,1-diMeBu)-Ph | Py-3-yl |
| 841 | H | H | H | 4-(4, 4, 4-triF-1, 1-diMeBu)-Ph | Py-4-yl |
| 842 | H | H | H | 4-(5, 5, 5-triF-1, 1-diMePn)-Ph | Ph |
| 843 | H | H | H | 4-(5, 5, 5-triF-1, 1-diMePn)-Ph | 2-F-Ph |
| 844 | H | H | H | 4-(5, 5, 5-triF-1,1-diMePn)-Ph | 3-F-Ph |
| 845 | H | H | H | 4-(5, 5, 5-triF-1, 1-diMePn)-Ph | 4-F-Ph |
| 846 | H | H | H | 4-(5, 5, 5-triF-1, 1-diMePn)-Ph | 3,4-diF-Ph |
| 847 | H | H | H | 4-(5, 5, 5-triF-1, 1-diMePn)-Ph | 3,5-diF-Ph |
| 848 | H | H | H | 4-(5, 5, 5-triF-1, 1-diMePn)-Ph | 4-Cl-Ph |
| 849 | H | H | H | 4-(5, 5, 5-triF-1, 1-diMePn)-Ph | 4-Me-Ph |
| 850 | H | H | H | 4-(5, 5, 5-triF-1, 1-diMePn)-Ph | 4-$CF_3$-Ph |
| 851 | H | H | H | 4-(5, 5, 5-triF-1, 1-diMePn)-Ph | 4-OMe-Ph |
| 852 | H | H | H | 4-(5, 5, 5-triF-1, 1-diMePn)-Ph | Fu-2-yl |
| 853 | H | H | H | 4-(5, 5, 5-triF-1, 1-diMePn)-Ph | Th-2-yl |
| 854 | H | H | H | 4-(5, 5, 5-triF-1, 1-diMePn)-Ph | Th-3-yl |

(continued)

| Compound No. | $R^1$ | $R^2$ | $R^3$ | Y | Z |
|---|---|---|---|---|---|
| 855 | H | H | H | 4-(5, 5, 5-triF-1, 1-diMePn)-Ph | 1-Me-Im-4-yl |
| 856 | H | H | H | 4-(5, 5, 5-triF-1, 1-diMePn)-Ph | Tz-2-yl |
| 857 | H | H | H | 4-(5, 5, 5-triF-1, 1-diMePn)-Ph | Py-2-yl |
| 858 | Me | H | H | 4-(5, 5, 5-triF-1, 1-diMePn)-Ph | Py-2-yl |
| 859 | Et | H | H | 4-(5, 5, 5-triF-1, 1-diMePn)-Ph | Py-2-yl |
| 860 | $Pr^1$ | H | H | 4-(5, 5, 5-triF-1, 1-diMePn)-Ph | Py-2-yl |
| 861 | H | H | H | 4-(5, 5, 5-triF-1, 1-diMePn)-Ph | 5-F-Py-2-yl |
| 862 | H | H | H | 4-(5, 5, 5-triF-1, 1-diMePn)-Ph | Py-3-yl |
| 863 | H | H | H | 4-(5, 5, 5-triF-1, 1-diMePn)-Ph | 6-F-Py-3-yl |
| 864 | H | H | H | 4-(5, 5, 5-triF-1, 1-diMePn)-Ph | Py-4-yl |
| 865 | H | H | H | 4-(5, 5, 5-triF-2,2-diMePn)-Ph | 4-F-Ph |
| 866 | H | H | H | 4-(5, 5, 5-triF-2, 2-diMePn)-Ph | Py-2-yl |
| 867 | H | H | H | 4-(5, 5, 5-triF-2, 2-diMePn)-Ph | Py-3-yl |
| 868 | H | H | H | 5-(5, 5, 5-triF-1, 1-diMePn)-Th-2-yl | Ph |
| 869 | H | H | H | 5-(5, 5, 5-triF-1, 1-diMePn)-Th-2-yl | 2-F-Ph |
| 870 | H | H | H | 5-(5, 5, 5-triF-1, 1-diMePn)-Th-2-yl | 3-F-Ph |
| 871 | H | H | H | 5-(5, 5, 5-triF-1, 1-diMePn)-Th-2-yl | 4-F-Ph |
| 872 | H | H | H | 5-(5, 5, 5-triF-1, 1-diMePn)-Th-2-yl | 4-Cl-Ph |
| 873 | H | H | H | 5-(5, 5, 5-triF-1, 1-diMePn)-Th-2-yl | 4-OMe-Ph |
| 874 | H | H | H | 5-(5, 5, 5-triF-1, 1-diMePn)-Th-2-yl | Fu-2-yl |
| 875 | H | H | H | 5-(5, 5, 5-triF-1,1-diMePn)-Th-2-yl | Th-2-yl |
| 876 | H | H | H | 5-(5, 5, 5-triF-1, 1-diMePn)-Th-2-yl | 1-Me-Im-4-yl |
| 877 | H | H | H | 5-(5, 5, 5-triF-1, 1-diMePn)-Th-2-yl | Tz-2-yl |
| 878 | H | H | H | 5-(5, 5, 5-triF-1,1-diMePn)-Th-2-yl | Py-2-yl |
| 879 | H | H | H | 5-(5, 5, 5-triF-1, 1-diMePn)-Th-2-yl | Py-3-yl |
| 880 | H | H | H | 5-(5, 5, 5-triF-1, 1-diMePn)-Th-2-yl | Py-4-yl |
| 881 | H | H | H | 4-OPr-Ph | 4-F-Ph |
| 882 | H | H | H | 4-OPr-Ph | Py-2-yl |
| 883 | H | H | H | 4-OPr-Ph | Py-3-yl |
| 884 | H | H | H | 4-OBu-Ph | Ph |
| 885 | H | H | H | 4-OBu-Ph | 2-F-Ph |
| 886 | H | H | H | 4-OBu-Ph | 3-F-Ph |
| 887 | H | H | H | 4-OBu-Ph | 4-F-Ph |
| 888 | H | H | H | 4-OBu-Ph | 3,4-diF-Ph |
| 889 | H | H | H | 4-OBu-Ph | 3,5-diF-Ph |
| 890 | H | H | H | 4-OBu-Ph | 4-Cl-Ph |
| 891 | H | H | H | 4-OBu-Ph | 4-Me-Ph |
| 892 | H | H | H | 4-OBu-Ph | 4-$CF_3$-Ph |
| 893 | H | H | H | 4-OBu-Ph | 4-OMe-Ph |
| 894 | H | H | H | 4-OBu-Ph | Fu-2-yl |
| 895 | H | H | H | 4-OBu-Ph | Th-2-yl |
| 896 | H | H | H | 4-OBu-Ph | Th-3-yl |
| 897 | H | H | H | 4-OBu-Ph | 1-Me-Im-4-yl |
| 898 | H | H | H | 4-OBu-Ph | Tz-2-yl |
| 899 | H | H | H | 4-OBu-Ph | Py-2-yl |
| 900 | Me | H | H | 4-OBu-Ph | Py-2-yl |
| 901 | Et | H | H | 4-OBu-Ph | Py-2-yl |
| 902 | $Pr^1$ | H | H | 4-OBu-Ph | Py-2-yl |
| 903 | H | H | H | 4-OBu-Ph | 5-F-Py-2-yl |
| 904 | H | H | H | 4-OBu-Ph | Py-3-yl |

(continued)

| Compound No. | $R^1$ | $R^2$ | $R^3$ | Y | Z |
|---|---|---|---|---|---|
| 905 | H | H | H | 4-OBu-Ph | 6-F-Py-3-yl |
| 906 | H | H | H | 4-OBu-Ph | Py-4-yl |
| 907 | H | H | H | 4-OBu$^1$-Ph | 4-F-Ph |
| 908 | H | H | H | 4-OBu$^1$-Ph | Py-2-yl |
| 909 | H | H | H | 4-OBu$^1$-Ph | Py-3-yl |
| 910 | H | H | H | 4-$OCHF_2$-Ph | Ph |
| 911 | H | H | H | 4-$OCHF_2$-Ph | 2-F-Ph |
| 912 | H | H | H | 4-$OCHF_2$-Ph | 3-F-Ph |
| 913 | H | H | H | 4-$OCHF_2$-Ph | 4-F-Ph |
| 914 | H | H | H | 4-$OCHF_2$-Ph | 3,4-diF-Ph |
| 915 | H | H | H | 4-$OCHF_2$-Ph | 3,5-diF-Ph |
| 916 | H | H | H | 4-$OCHF_2$-Ph | 4-Cl-Ph |
| 917 | H | H | H | 4-$OCHF_2$-Ph | 4-Me-Ph |
| 918 | H | H | H | 4-$OCHF_2$-Ph | 4-$CF_3$-Ph |
| 919 | H | H | H | 4-$OCHF_2$-Ph | 4-OMe-Ph |
| 920 | H | H | H | 4-$OCHF_2$-Ph | Fu-2-yl |
| 921 | H | H | H | 4-$OCHF_2$-Ph | Th-2-yl |
| 922 | H | H | H | 4-$OCHF_2$-Ph | Th-3-yl |
| 923 | H | H | H | 4-$OCHF_2$-Ph | 1-Me-Im-4-yl |
| 924 | H | H | H | 4-$OCHF_2$-Ph | Tz-2-yl |
| 925 | H | H | H | 4-$OCHF_2$-Ph | Py-2-yl |
| 926 | H | H | H | 4-$OCHF_2$-Ph | 5-F-Py-2-yl |
| 927 | H | H | H | 4-$OCHF_2$-Ph | Py-3-yl |
| 928 | Me | H | H | 4-$OCHF_2$-Ph | Py-3-yl |
| 929 | Et | H | H | 4-$OCHF_2$-Ph | Py-3-yl |
| 930 | Pr$^1$ | H | H | 4-$OCHF_2$-Ph | Py-3-yl |
| 931 | H | H | H | 4-$OCHF_2$-Ph | 6-F-Py-3-yl |
| 932 | H | H | H | 4-$OCHF_2$-Ph | Py-4-yl |
| 933 | H | H | H | 4-$OCF_3$-Ph | 4-F-Ph |
| 934 | H | H | H | 4-$OCF_3$-Ph | Py-2-yl |
| 935 | H | H | H | 4-$OCF_3$-Ph | Py-3-yl |
| 936 | H | H | H | 4-(3-$OCHF_2$-1-MePr)-Ph | 4-F-Ph |
| 937 | H | H | H | 4-(3-$OCHF_2$-1-MePr)-Ph | Py-2-yl |
| 938 | H | H | H | 4-(3-$OCHF_2$-1-MePr)-Ph | Py-3-yl |
| 939 | H | H | H | 4-(3-$OCHF_2$-1,1-diMePr)-Ph | Ph |
| 940 | H | H | H | 4-(3-$OCHF_2$-1,1-diMePr)-Ph | 2-F-Ph |
| 941 | H | H | H | 4-(3-$OCHF_2$-1,1-diMePr)-Ph | 3-F-Ph |
| 942 | H | H | H | 4-(3-$OCHF_2$-1,1-diMePr)-Ph | 4-F-Ph |
| 943 | H | H | H | 4-(3-$OCHF_2$-1,1-diMePr)-Ph | 3,4-diF-Ph |
| 944 | H | H | H | 4-(3-$OCHF_2$-1,1-diMePr)-Ph | 3,5-diF-Ph |
| 945 | H | H | H | 4-(3-$OCHF_2$-1,1-diMePr)-Ph | 4-Cl-Ph |
| 946 | H | H | H | 4-(3-$OCHF_2$-1,1-diMePr)-Ph | 4-Me-Ph |
| 947 | H | H | H | 4-(3-$OCHF_2$-1,1-diMePr)-Ph | 4-$CF_3$-Ph |
| 948 | H | H | H | 4-(3-$OCHF_2$-1,1-diMePr)-Ph | 4-OMe-Ph |
| 949 | H | H | H | 4-(3-$OCHF_2$-1,1-diMePr)-Ph | Fu-2-yl |
| 950 | H | H | H | 4-(3-$OCHF_2$-1,1-diMePr)-Ph | Th-2-yl |
| 951 | H | H | H | 4-(3-$OCHF_2$-1,1-diMePr)-Ph | Th-3-yl |
| 952 | H | H | H | 4-(3-$OCHF_2$-1,1-diMePr)-Ph | 1-Me-Im-4-yl |
| 953 | H | H | H | 4-(3-$OCHF_2$-1,1-diMePr)-Ph | Tz-2-yl |
| 954 | H | H | H | 4-(3-$OCHF_2$-1,1-diMePr)-Ph | Py-2-yl |

(continued)

| Compound No. | $R^1$ | $R^2$ | $R^3$ | Y | Z |
|---|---|---|---|---|---|
| 955 | Me | H | H | 4-(3-$OCHF_2$-1,1-diMePr)-Ph | Py-2-yl |
| 956 | Et | H | H | 4-(3-$OCHF_2$-1,1-diMePr)-Ph | Py-2-yl |
| 957 | $Pr^1$ | H | H | 4-(3-$OCHF_2$-1,1-diMePr)-Ph | Py-2-yl |
| 958 | H | H | H | 4-(3-$OCHF_2$-1,1-diMePr)-Ph | 5-F-Py-2-yl |
| 959 | H | H | H | 4-(3-$OCHF_2$-1,1-diMePr)-Ph | Py-3-yl |
| 960 | H | H | H | 4-(3-$OCHF_2$-1,1-diMeP)-Ph | 6-F-Py-3-yl |
| 961 | H | H | H | 4-(3-$OCHF_2$-1,1-diMePr)-Ph | Py-4-yl |
| 962 | H | H | H | 4-(3-$Pr^c$-1-MePr)-Ph | 4-F-Ph |
| 963 | H | H | H | 4-(3-$Pr^c$-1-MePr)-Ph | Py-2-yl |
| 964 | H | H | H | 4-(3-$Pr^c$-1-MePr)-Ph | Py-3-yl |
| 965 | H | H | H | 4-(3-$Pr^c$-1,1-diMePr)-Ph | Ph |
| 966 | H | H | H | 4-(3-$Pr^c$-1,1-diMePr)-Ph | 2-F-Ph |
| 967 | H | H | H | 4-(3-$Pr^c$-1,1-diMePr)-Ph | 3-F-Ph |
| 968 | H | H | H | 4-(3-$Pr^c$-1,1-diMePr)-Ph | 4-F-Ph |
| 969 | H | H | H | 4-(3-$Pr^c$-1,1-diMePr)-Ph | 4-Cl-Ph |
| 970 | H | H | H | 4-(3-$Pr^c$-1,1-diMePr)-Ph | 4-OMe-Ph |
| 971 | H | H | H | 4-(3-$Pr^c$-1,1-diMePr)-Ph | Fu-2-yl |
| 972 | H | H | H | 4-(3-$Pr^c$-1, 1-diMePr)-Ph | Th-2-yl |
| 973 | H | H | H | 4-(3-$Pr^c$-1,1-diMePr)-Ph | 1-Me-Im-4-yl |
| 974 | H | H | H | 4-(3-$Pr^c$-1, 1-diMePr)-Ph | Tz-2-yl |
| 975 | H | H | H | 4-(3-$Pr^c$-1, 1-diMePr)-Ph | Py-2-yl |
| 976 | H | H | H | 4-(3-$Pr^c$-1,1-diMePr)-Ph | Py-3-yl |
| 977 | H | H | H | 4-(3-$Pr^c$-1,1-diMePr)-Ph | Py-4-yl |
| 978 | H | H | H | 4-$Pre^1$-Ph | 4-F-Ph |
| 979 | H | H | H | 4-$Pre^1$-Ph | Py-2-yl |
| 980 | H | H | H | 4-$Pre^1$-Ph | Py-3-yl |
| 981 | H | H | H | 4-(1-Me-1-Pre)-Ph | 4-F-Ph |
| 982 | H | H | H | 4-(1-Me-1-Pre)-Ph | Py-2-yl |
| 983 | H | H | H | 4-(1-Me-1-Pre)-Ph | Py-3-yl |
| 984 | H | H | H | 4-(1-Me-1-Bue)-Ph | Ph |
| 985 | H | H | H | 4-(1-Me-1-Bue)-Ph | 2-F-Ph |
| 986 | H | H | H | 4-(1-Me-1-Bue)-Ph | 3-F-Ph |
| 987 | H | H | H | 4-(1-Me-1-Bue)-Ph | 4-F-Ph |
| 988 | H | H | H | 4-(1-Me-1-Bue)-Ph | 4-Cl-Ph |
| 989 | H | H | H | 4-(1-Me-1-Bue)-Ph | 4-OMe-Ph |
| 990 | H | H | H | 4-(1-Me-1-Bue)-Ph | Fu-2-yl |
| 991 | H | H | H | 4-(1-Me-1-Bue)-Ph | Th-2-yl |
| 992 | H | H | H | 4-(1-Me-1-Bue)-Ph | 1-Me-Im-4-yl |
| 993 | H | H | H | 4-(1-Me-1-Bue)-Ph | Tz-2-yl |
| 994 | H | H | H | 4-(1-Me-1-Bue)-Ph | Py-2-yl |
| 995 | H | H | H | 4-(1-Me-1-Bue)-Ph | Py-3-yl |
| 996 | H | H | H | 4-(1-Me-1-Bue)-Ph | Py-4-yl |
| 997 | H | H | H | 4-(1-Me-1-Pne)-Ph | Ph |
| 998 | H | H | H | 4-(1-Me-1-Pne)-Ph | 2-F-Ph |
| 999 | H | H | H | 4-(1-Me-1-Pne)-Ph | 3-F-Ph |
| 1000 | H | H | H | 4-(1-Me-1-Pne)-Ph | 4-F-Ph |
| 1001 | H | H | H | 4-(1-Me-1-Pne)-Ph | 3,4-diF-Ph |
| 1002 | H | H | H | 4-(1-Me-1-Pne)-Ph | 3,5-diF-Ph |
| 1003 | H | H | H | 4-(1-Me-1-Pne)-Ph | 4-Cl-Ph |
| 1004 | H | H | H | 4-(1-Me-1-Pne)-Ph | 4-Me-Ph |

(continued)

| Compound No. | $R^1$ | $R^2$ | $R^3$ | Y | Z |
|---|---|---|---|---|---|
| 1005 | H | H | H | 4-(1-Me-1-Pne)-Ph | 4-$CF_3$-Ph |
| 1006 | H | H | H | 4-(1-Me-1-Pne)-Ph | 4-OMe-Ph |
| 1007 | H | H | H | 4-(1-Me-1-Pne)-Ph | Fu-2-yl |
| 1008 | H | H | H | 4-(1-Me-1-Pne)-Ph | Th-2-yl |
| 1009 | H | H | H | 4-(1-Me-1-Pne)-Ph | Th-3-yl |
| 1010 | H | H | H | 4-(1-Me-1-Pne)-Ph | 1-Me-Im-4-yl |
| 1011 | H | H | H | 4-(1-Me-1-Pne)-Ph | Tz-2-yl |
| 1012 | H | H | H | 4-(1-Me-1-Pne)-Ph | Py-2-yl |
| 1013 | Me | H | H | 4-(1-Me-1-Pne)-Ph | Py-2-yl |
| 1014 | Et | H | H | 4-(1-Me-1-Pne)-Ph | Py-2-yl |
| 1015 | $Pr^1$ | H | H | 4-(1-Me-1-Pne)-Ph | Py-2-yl |
| 1016 | H | H | H | 4-(1-Me-1-Pne)-Ph | 5-F-Py-2-yl |
| 1017 | H | H | H | 4-(1-Me-1-Pne)-Ph | Py-3-yl |
| 1018 | H | H | H | 4-(1-Me-1-Pne)-Ph | 6-F-Py-3-yl |
| 1019 | H | H | H | 4-(1-Me-1-Pne)-Ph | Py-4-yl |
| 1020 | H | H | H | 4- (1-$MePr^c$) -Ph | Ph |
| 1021 | H | H | H | 4-(1-$MePr^c$)-Ph | 2-F-Ph |
| 1022 | H | H | H | 4-(1-$MePr^c$)-Ph | 3-F-Ph |
| 1023 | H | H | H | 4-(1-$MePr^c$)-Ph | 4-F-Ph |
| 1024 | H | H | H | 4-(1-$MePr^c$)-Ph | 3,4-diF-Ph |
| 1025 | H | H | H | 4-(1-$MePr^c$)-Ph | 3,5-diF-Ph |
| 1026 | H | H | H | 4-(1-$MePr^c$)-Ph | 4-Cl-Ph |
| 1027 | H | H | H | 4-(1-$MePr^c$)-Ph | 4-Me-Ph |
| 1028 | H | H | H | 4-(1-$MePr^c$)-Ph | 4-$CF_3$-Ph |
| 1029 | H | H | H | 4-(1-$MePr^c$)-Ph | 4-OMe-Ph |
| 1030 | H | H | H | 4-(1-$MePr^c$)-Ph | Fu-2-yl |
| 1031 | H | H | H | 4-(1-$MePr^c$)-Ph | Th-2-yl |
| 1032 | H | H | H | 4-(1-$MePr^c$)-Ph | Th-3-yl |
| 1033 | H | H | H | 4-(1-$MePr^c$)-Ph | 1-Me-Im-4-yl |
| 1034 | H | H | H | 4-(1-$MePr^c$)-Ph | Tz-2-yl |
| 1035 | H | H | H | 4-(1-$MePr^c$)-Ph | Py-2-yl |
| 1036 | H | H | H | 4-(1-$MePr^c$)-Ph | 5-F-Py-2-yl |
| 1037 | H | H | H | 4-(1-$MePr^c$)-Ph | Py-3-yl |
| 1038 | Me | H | H | 4-(1-$MePr^c$)-Ph | Py-3-yl |
| 1039 | Et | H | H | 4-(1-$MePr^c$)-Ph | Py-3-yl |
| 1040 | $Pr^1$ | H | H | 4-(1-$MePr^c$)-Ph | Py-3-yl |
| 1041 | H | H | H | 4-(1-$MePr^c$)-Ph | 6-F-Py-3-yl |
| 1042 | H | H | H | 4-(1-$MePr^c$)-Ph | Py-4-yl |
| 1043 | H | H | H | 4-(1-$EtPr^c$)-Ph | Ph |
| 1044 | H | H | H | 4-(1-$EtPr^c$)-Ph | 2-F-Ph |
| 1045 | H | H | H | 4-(1-$EtPr^c$)-Ph | 3-F-Ph |
| 1046 | H | H | H | 4-(1-$EtPr^c$)-Ph | 4-F-Ph |
| 1047 | Me | H | H | 4-(1-$EtPr^c$)-Ph | 4-F-Ph |
| 1048 | Et | H | H | 4-(1-$EtPr^c$)-Ph | 4-F-Ph |
| 1049 | $Pr^1$ | H | H | 4- (1-$EtPr^c$) -Ph | 4-F-Ph |
| 1050 | H | H | H | 4-(1-$EtPr^c$)-Ph | 3,4-diF-Ph |
| 1051 | H | H | H | 4-(1-$EtPr^c$)-Ph | 3,5-diF-Ph |
| 1052 | H | H | H | 4-(1-$EtPr^c$)-Ph | 4-Cl-Ph |
| 1053 | H | H | H | 4- (1-$EtPr^c$) -Ph | 4-Me-Ph |
| 1054 | H | H | H | 4-(1-$EtPr^c$)-Ph | 4-$CF_3$-Ph |

(continued)

| Compound No. | $R^1$ | $R^2$ | $R^3$ | Y | Z |
|---|---|---|---|---|---|
| 1055 | H | H | H | 4-(1-Et$Pr^c$)-Ph | 4-OMe-Ph |
| 1056 | H | H | H | 4-(1-Et$Pr^c$)-Ph | Fu-2-yl |
| 1057 | H | H | H | 4-(1-Et$Pr^c$)-Ph | Th-2-yl |
| 1058 | H | H | H | 4-(1-Et$Pr^c$)-Ph | Th-3-yl |
| 1059 | H | H | H | 4-(1-Et$Pr^c$)-Ph | 1-Me-Im-4-yl |
| 1060 | H | H | H | 4-(1-Et$Pr^c$)-Ph | Tz-2-yl |
| 1061 | H | H | H | 4-(1-Et$Pr^c$)-Ph | Py-2-yl |
| 1062 | Me | H | H | 4-(1-Et$Pr^c$)-Ph | Py-2-yl |
| 1063 | Et | H | H | 4-(1-Et$Pr^c$)-Ph | Py-2-yl |
| 1064 | $Pr^1$ | H | H | 4- (1-Et$Pr^c$) -Ph | Py-2-yl |
| 1065 | H | H | H | 4-(1-Et$Pr^c$)-Ph | 5-F-Py-2-yl |
| 1066 | H | H | H | 4-(1-Et$Pr^c$)-Ph | Py-3-yl |
| 1067 | Me | H | H | 4-(1-Et$Pr^c$)-Ph | Py-3-yl |
| 1068 | Et | H | H | 4-(1-Et$Pr^c$)-Ph | Py-3-yl |
| 1069 | $Pr^1$ | H | H | 4-(1-Et$Pr^c$)-Ph | Py-3-yl |
| 1070 | H | H | H | 4-(1-Et$Pr^c$)-Ph | 6-F-Py-3-yl |
| 1071 | H | H | H | 4- (1-Et$Pr^c$) -Ph | Py-4-yl |
| 1072 | H | H | H | 4- (1-Pr$Pr^c$) -Ph | Ph |
| 1073 | H | H | H | 4- (1-Pr$Pr^c$) -Ph | 2-F-Ph |
| 1074 | H | H | H | 4-(1-Pr$Pr^c$)-Ph | 3-F-Ph |
| 1075 | H | H | H | 4- (1-Pr$Pr^c$) -Ph | 4-F-Ph |
| 1076 | H | H | H | 4-(1-Pr$Pr^c$)-Ph | 4-Cl-Ph |
| 1077 | H | H | H | 4- (1-Pr$Pr^c$) -Ph | 4-OMe-Ph |
| 1078 | H | H | H | 4-(1-Pr$Pr^c$)-Ph | Fu-2-yl |
| 1079 | H | H | H | 4-(1-Pr$Pr^c$)-Ph | Th-2-yl |
| 1080 | H | H | H | 4-(1-Pr$Pr^c$)-Ph | 1-Me-Im-4-yl |
| 1081 | H | H | H | 4-(1-Pr$Pr^c$)-Ph | Tz-2-yl |
| 1082 | H | H | H | 4-(1-Pr$Pr^c$)-Ph | Py-2-yl |
| 1083 | H | H | H | 4-(1-Pr$Pr^c$)-Ph | Py-3-yl |
| 1084 | H | H | H | 4-(1-Pr$Pr^c$)-Ph | Py-4-yl |
| 1085 | H | H | H | 4- (1-$Pr^1Pr^c$) -Ph | Ph |
| 1086 | H | H | H | 4-(1-$Pr^1Pr^c$)-Ph | 2-F-Ph |
| 1087 | H | H | H | 4- (1-$Pr^1Pr^c$) -Ph | 3-F-Ph |
| 1088 | H | H | H | 4-(1-$Pr^1Pr^c$)-Ph | 4-F-Ph |
| 1089 | H | H | H | 4-(1-$Pr^1Pr^c$)-Ph | 3,4-diF-Ph |
| 1090 | H | H | H | 4-(1-$Pr^1Pr^c$)-Ph | 3,5-diF-Ph |
| 1091 | H | H | H | 4-(1-$Pr^1Pr^c$)-Ph | 4-Cl-Ph |
| 1092 | H | H | H | 4- (1-$Pr^1Pr^c$) -Ph | 4-Me-Ph |
| 1093 | H | H | H | 4-(1-$Pr^1Pr^c$)-Ph | 4-$CF_3$-Ph |
| 1094 | H | H | H | 4- (1-$Pr^1Pr^c$) -Ph | 4-OMe-Ph |
| 1095 | H | H | H | 4-(1-$Pr^1Pr^c$)-Ph | Fu-2-yl |
| 1096 | H | H | H | 4-(1-$Pr^1Pr^c$)-Ph | Th-2-yl |
| 1097 | H | H | H | 4-(1-$Pr^1Pr^c$)-Ph | Th-3-yl |
| 1098 | H | H | H | 4-(1-$Pr^1Pr^c$)-Ph | 1-Me-Im-4-yl |
| 1099 | H | H | H | 4-(1-$Pr^1Pr^c$)-Ph | Tz-2-yl |
| 1100 | H | H | H | 4-(1-$Pr^1Pr^c$)-Ph | Py-2-yl |
| 1101 | H | H | H | 4-(1-$Pr^1Pr^c$)-Ph | 5-F-Py-2-yl |
| 1102 | H | H | H | 4-(1-$Pr^1Pr^c$)-Ph | Py-3-yl |
| 1103 | Me | H | H | 4-(1-$Pr^1Pr^c$)-Ph | Py-3-yl |
| 1104 | Et | H | H | 4-(1-$Pr^1Pr^c$)-Ph | Py-3-yl |

(continued)

| Compound No. | $R^1$ | $R^2$ | $R^3$ | Y | Z |
|---|---|---|---|---|---|
| 1105 | $Pr^1$ | H | H | 4-(1-$Pr^1Pr^c$)-Ph | Py-3-yl |
| 1106 | H | H | H | 4-(1-$Pr^1Pr^c$)-Ph | 6-F-Py-3-yl |
| 1107 | H | H | H | 4-(1-$Pr^1Pr^c$)-Ph | Py-4-yl |
| 1108 | H | H | H | 3-(1-Bu$Pr^c$) -Ph | 4-F-Ph |
| 1109 | H | H | H | 3-(1-Bu$Pr^c$)-Ph | Py-2-yl |
| 1110 | H | H | H | 3-(1-Bu$Pr^c$)-Ph | Py-3-yl |
| 1111 | H | H | H | 4-(1-Bu$Pr^c$)-Ph | Ph |
| 1112 | H | H | H | 4-(1-Bu$Pr^c$) -Ph | 2-F-Ph |
| 1113 | H | H | H | 4-(1-Bu$Pr^c$)-Ph | 3-F-Ph |
| 1114 | H | H | H | 4-(1-Bu$Pr^c$)-Ph | 4-F-Ph |
| 1115 | H | Me | H | 4-(1-Bu$Pr^c$)-Ph | 4-F-Ph |
| 1116 | H | Me | Me | 4-(1-Bu$Pr^c$)-Ph | 4-F-Ph |
| 1117 | Me | H | H | 4-(1-Bu$Pr^c$)-Ph | 4-F-Ph |
| 1118 | Et | H | H | 4-(1-Bu$Pr^c$)-Ph | 4-F-Ph |
| 1119 | $Pr^1$ | H | H | 4-(1-Bu$Pr^c$)-Ph | 4-F-Ph |
| 1120 | H | H | H | 4-(1-Bu$Pr^c$)-Ph | 3, 4-diF-Ph |
| 1121 | H | H | H | 4-(1-Bu$Pr^c$)-Ph | 3, 5-diF-Ph |
| 1122 | H | H | H | 4-(1-Bu$Pr^c$)-Ph | 2-Cl-Ph |
| 1123 | H | H | H | 4-(1-Bu$Pr^c$)-Ph | 3-Cl-Ph |
| 1124 | H | H | H | 4-(1-Bu$Pr^c$)-Ph | 4-Cl-Ph |
| 1125 | H | H | H | 4-(1-Bu$Pr^c$)-Ph | 2,6-diCl-Ph |
| 1126 | H | H | H | 4-(1-Bu$Pr^c$)-Ph | 4-Cl-3-F-Ph |
| 1127 | H | H | H | 4-(1-Bu$Pr^c$)-Ph | 4-Me-Ph |
| 1128 | H | H | H | 4-(1-Bu$Pr^c$)-Ph | 3-F-4-Me-Ph |
| 1129 | H | H | H | 4-(1-Bu$Pr^c$)-Ph | 4-$CF_3$-Ph |
| 1130 | H | H | H | 4-(1-Bu$Pr^c$)-Ph | 3-F-4-$CF_3$-Ph |
| 1131 | H | H | H | 4-(1-Bu$Pr^c$)-Ph | 4-OMe-Ph |
| 1132 | H | H | H | 4-(1-Bu$Pr^c$)-Ph | 3-F-4-OMe-Ph |
| 1133 | H | H | H | 4-(1-Bu$Pr^c$)-Ph | 4-$OCHF_2$-Ph |
| 1134 | H | H | H | 4-(1-Bu$Pr^c$)-Ph | 4-$OCHF_2$-3-F-Ph |
| 1135 | H | H | H | 4-(1-Bu$Pr^c$)-Ph | Fu-2-yl |
| 1136 | H | H | H | 4-(1-Bu$Pr^c$)-Ph | Th-2-yl |
| 1137 | H | H | H | 4-(1-Bu$Pr^c$)-Ph | Th-3-yl |
| 1138 | H | H | H | 4-(1-Bu$Pr^c$)-Ph | 1-Me-Im-4-yl |
| 1139 | Me | H | H | 4-(1-Bu$Pr^c$)-Ph | 1-Me-Im-4-yl |
| 1140 | Et | H | H | 4-(1-Bu$Pr^c$)-Ph | 1-Me-Im-4-yl |
| 1141 | $Pr^1$ | H | H | 4-(1-Bu$Pr^c$)-Ph | 1-Me-Im-4-yl |
| 1142 | H | H | H | 4-(1-Bu$Pr^c$)-Ph | Tz-2-yl |
| 1143 | H | H | H | 4-(1-Bu$Pr^c$)-Ph | Py-2-yl |
| 1144 | H | Me | H | 4-(1-Bu$Pr^c$)-Ph | Py-2-yl |
| 1145 | H | Me | Me | 4-(1-Bu$Pr^c$)-Ph | Py-2-yl |
| 1146 | Me | H | H | 4-(1-Bu$Pr^c$)-Ph | Py-2-yl |
| 1147 | Et | H | H | 4-(1-Bu$Pr^c$)-Ph | Py-2-yl |
| 1148 | $Pr^1$ | H | H | 4-(1-Bu$Pr^c$)-Ph | Py-2-yl |
| 1149 | H | H | H | 4-(1-Bu$Pr^c$)-Ph | 5-F-Py-2-yl |
| 1150 | H | H | H | 4-(1-Bu$Pr^c$)-Ph | 5-Cl-Py-2-yl |
| 1151 | H | H | H | 4-(1-Bu$Pr^c$)-Ph | 5-OMe-Py-2-yl |
| 1152 | H | H | H | 4-(1-Bu$Pr^c$)-Ph | Py-3-yl |
| 1153 | H | Me | H | 4-(1-Bu$Pr^c$)-Ph | Py-3-yl |
| 1154 | H | Me | Me | 4-(1-Bu$Pr^c$)-Ph | Py-3-yl |

(continued)

| Compound No. | $R^1$ | $R^2$ | $R^3$ | Y | Z |
|---|---|---|---|---|---|
| 1155 | Me | H | H | 4-(1-BuPr$^c$)-Ph | Py-3-yl |
| 1156 | Et | H | H | 4-(1-BuPr$^c$)-Ph | Py-3-yl |
| 1157 | $Pr^1$ | H | H | 4-(1-BuPr$^c$)-Ph | Py-3-yl |
| 1158 | H | H | H | 4-(1-BuPr$^c$)-Ph | 6-F-Py-3-yl |
| 1159 | H | H | H | 4-(1-BuPr$^c$)-Ph | 6-Cl-Py-3-yl |
| 1160 | H | H | H | 4-(1-BuPr$^c$)-Ph | 6-OMe-Py-3-yl |
| 1161 | H | H | H | 4-(1-BuPr$^c$)-Ph | Py-4-yl |
| 1162 | Me | H | H | 4-(1-BuPr$^c$)-Ph | Py-4-yl |
| 1163 | Et | H | H | 4-(1-BuPr$^c$)-Ph | Py-4-yl |
| 1164 | $Pr^1$ | H | H | 4-(1-BuPr$^c$)-Ph | Py-4-yl |
| 1165 | H | H | H | 2-F-4-(1-BuPr$^c$)-Ph | Ph |
| 1166 | H | H | H | 2-F-4-(1-BuPr$^c$)-Ph | 2-F-Ph |
| 1167 | H | H | H | 2-F-4-(1-BuPr$^c$)-Ph | 3-F-Ph |
| 1168 | H | H | H | 2-F-4-(1-BuPr$^c$)-Ph | 4-F-Ph |
| 1169 | H | H | H | 2-F-4-(1-BuPr$^c$)-Ph | 4-Cl-Ph |
| 1170 | H | H | H | 2-F-4-(1-BuPr$^c$)-Ph | 4-OMe-Ph |
| 1171 | H | H | H | 2-F-4-(1-BuPr$^c$)-Ph | Fu-2-yl |
| 1172 | H | H | H | 2-F-4-(1-BuPr$^c$)-Ph | Th-2-yl |
| 1173 | H | H | H | 2-F-4-(1-BuPr$^c$)-Ph | 1-Me-Im-4-yl |
| 1174 | H | H | H | 2-F-4-(1-BuPr$^c$)-Ph | Tz-2-yl |
| 1175 | H | H | H | 2-F-4-(1-BuPr$^c$)-Ph | Py-2-yl |
| 1176 | H | H | H | 2-F-4-(1-BuPr$^c$)-Ph | Py-3-yl |
| 1177 | H | H | H | 2-F-4-(1-BuPr$^c$)-Ph | Py-4-yl |
| 1178 | H | H | H | 3-F-4-(1-BuPr$^c$)-Ph | Ph |
| 1179 | H | H | H | 3-F-4-(1-BuPr$^c$)-Ph | 2-F-Ph |
| 1180 | H | H | H | 3-F-4-(1-BuPr$^c$)-Ph | 3-F-Ph |
| 1181 | H | H | H | 3-F-4-(1-BuPr$^c$)-Ph | 4-F-Ph |
| 1182 | H | H | H | 3-F-4-(1-BuPr$^c$)-Ph | 4-Cl-Ph |
| 1183 | H | H | H | 3-F-4-(1-BuPr$^c$)-Ph | 4-OMe-Ph |
| 1184 | H | H | H | 3-F-4-(1-BuPr$^c$)-Ph | Fu-2-yl |
| 1185 | H | H | H | 3-F-4-(1-BuPr$^c$)-Ph | Th-2-yl |
| 1186 | H | H | H | 3-F-4-(1-BuPr$^c$)-Ph | 1-Me-Im-4-yl |
| 1187 | H | H | H | 3-F-4-(1-BuPr$^c$)-Ph | Tz-2-yl |
| 1188 | H | H | H | 3-F-4-(1-BuPr$^c$)-Ph | Py-2-yl |
| 1189 | H | H | H | 3-F-4-(1-BuPr$^c$)-Ph | Py-3-yl |
| 1190 | H | H | H | 3-F-4-(1-BuPr$^c$)-Ph | Py-4-yl |
| 1191 | H | H | H | 3-$NMe_2$-4-(1-BuPr$^c$)-Ph | 4-F-Ph |
| 1192 | H | H | H | 3-$NMe_2$-4-(1-BuPr$^c$)-Ph | Py-2-yl |
| 1193 | H | H | H | 3-$NMe_2$-4-(1-BuPr$^c$)-Ph | Py-3-yl |
| 1194 | H | H | H | 5-(1-BuPr$^c$)-Th-2-yl | Ph |
| 1195 | H | H | H | 5-(1-BuPr$^c$)-Th-2-yl | 2-F-Ph |
| 1196 | H | H | H | 5-(1-BuPr$^c$)-Th-2-yl | 3-F-Ph |
| 1197 | H | H | H | 5-(1-BuPr$^c$)-Th-2-yl | 4-F-Ph |
| 1198 | H | H | H | 5-(1-BuPr$^c$)-Th-2-yl | 4-Cl-Ph |
| 1199 | H | H | H | 5-(1-BuPr$^c$)-Th-2-yl | 4-OMe-Ph |
| 1200 | H | H | H | 5-(1-BuPr$^c$)-Th-2-yl | Fu-2-yl |
| 1201 | H | H | H | 5-(1-BuPr$^c$)-Th-2-yl | Th-2-yl |
| 1202 | H | H | H | 5-(1-BuPr$^c$)-Th-2-yl | 1-Me-Im-4-yl |
| 1203 | H | H | H | 5-(1-BuPr$^c$)-Th-2-yl | Tz-2-yl |
| 1204 | H | H | H | 5-(1-BuPr$^c$)-Th-2-yl | Py-2-yl |

(continued)

| Compound No. | $R^1$ | $R^2$ | $R^3$ | Y | Z |
|---|---|---|---|---|---|
| 1205 | H | H | H | 5-(1-BuPr$^c$)-Th-2-yl | Py-3-yl |
| 1206 | H | H | H | 5-(1-BuPr$^c$)-Th-2-yl | Py-4-yl |
| 1207 | H | H | H | 4-(1-HxPr$^c$)-Ph | 4-F-Ph |
| 1208 | H | H | H | 4-(1-HxPr$^c$)-Ph | Py-2-yl |
| 1209 | H | H | H | 4-(1-HxPr$^c$)-Ph | Py-3-yl |
| 1210 | H | H | H | 4-(1-NnPr$^c$)-Ph | Ph |
| 1211 | H | H | H | 4-(1-NnPr$^c$)-Ph | 2-F-Ph |
| 1212 | H | H | H | 4-(1-NnPr$^c$)-Ph | 3-F-Ph |
| 1213 | H | H | H | 4-(1-NnPr$^c$)-Ph | 4-F-Ph |
| 1214 | H | H | H | 4-(1-NnPr$^c$)-Ph | 3, 4-diF-Ph |
| 1215 | H | H | H | 4-(1-NnPr$^c$)-Ph | 3, 5-diF-Ph |
| 1216 | H | H | H | 4-(1-NnPr$^c$)-Ph | 4-Cl-Ph |
| 1217 | H | H | H | 4- (1-NnPr$^c$) -Ph | 4-Me-Ph |
| 1218 | H | H | H | 4-(1-NnPr$^c$)-Ph | 4-$CF_3$-Ph |
| 1219 | H | H | H | 4- (1-NnPr$^c$) -Ph | 4-OMe-Ph |
| 1220 | H | H | H | 4-(1-NnPr$^c$)-Ph | Fu-2-yl |
| 1221 | H | H | H | 4-(1-NnPr$^c$)-Ph | Th-2-yl |
| 1222 | H | H | H | 4-(1-NnPr$^c$)-Ph | Th-3-yl |
| 1223 | H | H | H | 4-(1-NnPr$^c$)-Ph | 1-Me-Im-4-yl |
| 1224 | H | H | H | 4-(1-NnPr$^c$)-Ph | Tz-2-yl |
| 1225 | H | H | H | 4-(1-NnPr$^c$)-Ph | Py-2-yl |
| 1226 | H | H | H | 4-(1-NnPr$^c$)-Ph | 5-F-Py-2-yl |
| 1227 | H | H | H | 4-(1-NnPr$^c$)-Ph | Py-3-yl |
| 1228 | Me | H | H | 4-(1-NnPr$^c$)-Ph | Py-3-yl |
| 1229 | Et | H | H | 4-(1-NnPr$^c$)-Ph | Py-3-yl |
| 1230 | Pr$^1$ | H | H | 4-(1-NnPr$^c$)-Ph | Py-3-yl |
| 1231 | H | H | H | 4-(1-NnPr$^c$)-Ph | 6-F-Py-3-yl |
| 1232 | H | H | H | 4-(1-NnPr$^c$)-Ph | Py-4-yl |
| 1233 | H | H | H | 4-(1-OMePr$^c$)-Ph | Ph |
| 1234 | H | H | H | 4-(1-OMePr$^c$)-Ph | 2-F-Ph |
| 1235 | H | H | H | 4-(1-OMePr$^c$)-Ph | 3-F-Ph |
| 1236 | H | H | H | 4-(1-OMePr$^c$)-Ph | 4-F-Ph |
| 1237 | H | H | H | 4-(1-OMePr$^c$)-Ph | 3, 4-diF-Ph |
| 1238 | H | H | H | 4-(1-OMePr$^c$)-Ph | 3, 5-diF-Ph |
| 1239 | H | H | H | 4-(1-OMePr$^c$)-Ph | 4-Cl-Ph |
| 1240 | H | H | H | 4-(1-OMePr$^c$)-Ph | 4-Me-Ph |
| 1241 | H | H | H | 4-(1-OMePr$^c$)-Ph | 4-$CF_3$-Ph |
| 1242 | H | H | H | 4-(1-OMePr$^c$)-Ph | 4-OMe-Ph |
| 1243 | H | H | H | 4-(1-OMePr$^c$)-Ph | Fu-2-yl |
| 1244 | H | H | H | 4-(1-OMePr$^c$)-Ph | Th-2-yl |
| 1245 | H | H | H | 4-(1-OMePr$^c$)-Ph | Th-3-yl |
| 1246 | H | H | H | 4-(1-OMePr$^c$)-Ph | 1-Me-Im-4-yl |
| 1247 | H | H | H | 4-(1-OMePr$^c$)-Ph | Tz-2-yl |
| 1248 | H | H | H | 4-(1-OMePr$^c$)-Ph | Py-2-yl |
| 1249 | Me | H | H | 4-(1-OMePr$^c$)-Ph | Py-2-yl |
| 1250 | Et | H | H | 4-(1-OMePr$^c$)-Ph | Py-2-yl |
| 1251 | Pr$^1$ | H | H | 4-(1-OMePr$^c$)-Ph | Py-2-yl |
| 1252 | H | H | H | 4-(1-OMePr$^c$)-Ph | 5-F-Py-2-yl |
| 1253 | H | H | H | 4-(1-OMePr$^c$)-Ph | Py-3-yl |
| 1254 | H | H | H | 4-(1-OMePr$^c$)-Ph | 6-F-Py-3-yl |

(continued)

| Compound No. | $R^1$ | $R^2$ | $R^3$ | Y | Z |
|---|---|---|---|---|---|
| 1255 | H | H | H | 4-(1-OMePr$^c$)-Ph | Py-4-yl |
| 1256 | H | H | H | 4-(1-OEtPr$^c$)-Ph | 4-F-Ph |
| 1257 | H | H | H | 4-(1-OEtPr$^c$)-Ph | Py-2-yl |
| 1258 | H | H | H | 4-(1-OEtPr$^c$)-Ph | Py-3-yl |
| 1259 | H | H | H | 4-(1-OPrPr$^c$)-Ph | 4-F-Ph |
| 1260 | H | H | H | 4-(1-OPrPr$^c$)-Ph | Py-2-yl |
| 1261 | H | H | H | 4-(1-OPrPr$^c$)-Ph | Py-3-yl |
| 1262 | H | H | H | 4-(1-BnPr$^c$)-Ph | Ph |
| 1263 | H | H | H | 4-(1-BnPr$^c$)-Ph | 2-F-Ph |
| 1264 | H | H | H | 4-(1-BnPr$^c$)-Ph | 3-F-Ph |
| 1265 | H | H | H | 4-(1-BnPr$^c$)-Ph | 4-F-Ph |
| 1266 | H | H | H | 4-(1-BnPr$^c$)-Ph | 3, 4-diF-Ph |
| 1267 | H | H | H | 4-(1-BnPr$^c$)-Ph | 3, 5-diF-Ph |
| 1268 | H | H | H | 4-(1-BnPr$^c$)-Ph | 4-Cl-Ph |
| 1269 | H | H | H | 4-(1-BnPr$^c$)-Ph | 4-Me-Ph |
| 1270 | H | H | H | 4-(1-BnPr$^c$)-Ph | 4-$CF_3$-Ph |
| 1271 | H | H | H | 4-(1-BnPr$^c$)-Ph | 4-OMe-Ph |
| 1272 | H | H | H | 4-(1-BnPr$^c$)-Ph | Fu-2-yl |
| 1273 | H | H | H | 4-(1-BnPr$^c$)-Ph | Th-2-yl |
| 1274 | H | H | H | 4-(1-BnPr$^c$)-Ph | Th-3-yl |
| 1275 | H | H | H | 4-(1-BnPr$^c$)-Ph | 1-Me-Im-4-yl |
| 1276 | H | H | H | 4-(7-BnPr$^c$)-Ph | Tz-2-yl |
| 1277 | H | H | H | 4-(1-BnPr$^c$)-Ph | Py-2-yl |
| 1278 | Me | H | H | 4-(1-BnPr$^c$)-Ph | Py-2-yl |
| 1279 | Et | H | H | 4-(1-BnPr$^c$)-Ph | Py-2-yl |
| 1280 | $Pr^1$ | H | H | 4-(1-BnPr$^c$)-Ph | Py-2-yl |
| 1281 | H | H | H | 4-(1-BnPr$^c$)-Ph | 5-F-Py-2-yl |
| 1282 | H | H | H | 4-(1-BnPr$^c$)-Ph | Py-3-yl |
| 1283 | H | H | H | 4-(1-BnPr$^c$)-Ph | 6-F-Py-3-yl |
| 1284 | H | H | H | 4-(1-BnPr$^c$)-Ph | Py-4-yl |
| 1285 | H | H | H | 4-(1-CHPhMePr$^c$)-Ph | 4-F-Ph |
| 1286 | H | H | H | 4-(1-CHPhMePr$^c$)-Ph | Py-2-yl |
| 1287 | H | H | H | 4-(1-CHPhMePr$^c$)-Ph | Py-3-yl |
| 1288 | H | H | H | 4-(1-$CH_2CH_2$PhPr$^c$)-Ph | Ph |
| 1289 | H | H | H | 4-(1-$CH_2CH_2$PhPr$^c$)-Ph | 2-F-Ph |
| 1290 | H | H | H | 4-(1-$CH_2CH_2$PhPr$^c$)-Ph | 3-F-Ph |
| 1291 | H | H | H | 4-(1-$CH_2CH_2$PhPr$^c$)-Ph | 4-F-Ph |
| 1292 | H | H | H | 4-(1-$CH_2CH_2$PhPr$^c$)-Ph | 4-Cl-Ph |
| 1293 | H | H | H | 4-(1-$CH_2H_2$PhPr$^c$)-Ph | 4-OMe-Ph |
| 1294 | H | H | H | 4-(1-$CH_2CH_2$PhPr$^c$)-Ph | Fu-2-yl |
| 1295 | H | H | H | 4-(1-$CH_2CH_2$PhPr$^c$)-Ph | Th-2-yl |
| 1296 | H | H | H | 4-(1-$CH_2CH_2$PhPr$^c$)-Ph | 1-Me-Im-4-yl |
| 1297 | H | H | H | 4-(1-$CH_2CH_2$PhPr$^c$)-Ph | Tz-2-yl |
| 1298 | H | H | H | 4-(1-$CH_2CH_2$PhPr$^c$)-Ph | Py-2-yl |
| 1299 | H | H | H | 4-(1-$CH_2CH_2$PhPr$^c$)-Ph | Py-3-yl |
| 1300 | H | H | H | 4-(1-$CH_2CH_2$PhPr$^c$)-Ph | Py-4-yl |
| 1301 | H | H | H | 4-(1-$CH_2$OEtPr$^c$)-Ph | 4-F-Ph |
| 1302 | H | H | H | 4-(1-$CH_2$OEtPr$^c$)-Ph | Py-2-yl |
| 1303 | H | H | H | 4-(1-$CH_2$OEtPr$^c$)-Ph | Py-3-yl |
| 1304 | H | H | H | 4-(1-$CH_2$OPrPr$^c$)-Ph | Ph |

(continued)

| Compound No. | $R^1$ | $R^2$ | $R^3$ | Y | Z |
|---|---|---|---|---|---|
| 1305 | H | H | H | 4-(1-$CH_2$OPrPr$^c$)-Ph | 2-F-Ph |
| 1306 | H | H | H | 4-(1-$CH_2$OPrPr$^c$)-Ph | 3-F-Ph |
| 1307 | H | H | H | 4-(1-$CH_2$OPrPr$^c$)-Ph | 4-F-Ph |
| 1308 | H | H | H | 4-(1-$CH_2$OPrPr$^c$)-Ph | 3, 4-diF-Ph |
| 1309 | H | H | H | 4-(1-$CH_2$OPrPr$^c$)-Ph | 3, 5-diF-Ph |
| 1310 | H | H | H | 4-(1-$CH_2$OPrPr$^c$)-Ph | 4-Cl-Ph |
| 1311 | H | H | H | 4-(1-$CH_2$OPrPr$^c$)-Ph | 4-Me-Ph |
| 1312 | H | H | H | 4-(1-$CH_2$OPrPr$^c$)-Ph | 4-$CF_3$-Ph |
| 1313 | H | H | H | 4-(1-$CH_2$OPrPr$^c$)-Ph | 4-OMe-Ph |
| 1314 | H | H | H | 4-(1-$CH_2$OPrPr$^c$)-Ph | Fu-2-yl |
| 1315 | H | H | H | 4-(1-$CH_2$OPrPr$^c$)-Ph | Th-2-yl |
| 1316 | H | H | H | 4-(1-$CH_2$OPrPr$^c$)-Ph | Th-3-yl |
| 1317 | H | H | H | 4-(1-$CH_2$OPrPr$^c$)-Ph | 1-Me-Im-4-yl |
| 1318 | H | H | H | 4-(1-$CH_2$OPrPr$^c$)-Ph | Tz-2-yl |
| 1319 | H | H | H | 4-(1-$CH_2$OPrPr$^c$)-Ph | Py-2-yl |
| 1320 | Me | H | H | 4-(1-$CH_2$OPrPr$^c$)-Ph | Py-2-yl |
| 1321 | Et | H | H | 4-(1-$CH_2$OPrPr$^c$)-Ph | Py-2-yl |
| 1322 | Pr$^1$ | H | H | 4-(1-$CH_2$OPrPr$^c$)-Ph | Py-2-yl |
| 1323 | H | H | H | 4-(1-$CH_2$OPrPr$^c$)-Ph | 5-F-Py-2-yl |
| 1324 | H | H | H | 4-(1-$CH_2$OPrPr$^c$)-Ph | Py-3-yl |
| 1325 | H | H | H | 4-(1-$CH_2$OPrPr$^c$)-Ph | 6-F-Py-3-yl |
| 1326 | H | H | H | 4-(1-$CH_2$OPrPr$^c$)-Ph | Py-4-yl |
| 1327 | H | H | H | 5-(1-$CH_2$OPrPr$^c$)-Th-2-yl | Ph |
| 1328 | H | H | H | 5-(1-$CH_2$OPrPr$^c$)-Th-2-yl | 2-F-Ph |
| 1329 | H | H | H | 5-(1-$CH_2$OPrPr$^c$)-Th-2-yl | 3-F-Ph |
| 1330 | H | H | H | 5-(1-$CH_2$OPrPr$^c$)-Th-2-yl | 4-F-Ph |
| 1331 | H | H | H | 5-(1-$CH_2$OPrPr$^c$)-Th-2-yl | 4-Cl-Ph |
| 1332 | H | H | H | 5-(1-$CH_2$OPrPr$^c$)-Th-2-yl | 4-OMe-Ph |
| 1333 | H | H | H | 5-(1-$CH_2$OPrPr$^c$)-Th-2-yl | Fu-2-yl |
| 1334 | H | H | H | 5-(1-$CH_2$OPrPr$^c$)-Th-2-yl | Th-2-yl |
| 1335 | H | H | H | 5-(1-$CH_2$OPrPr$^c$)-Th-2-yl | 1-Me-Im-4-yl |
| 1336 | H | H | H | 5-(1-$CH_2$OPrPr$^c$)-Th-2-yl | Tz-2-yl |
| 1337 | H | H | H | 5-(1-$CH_2$OPrPr$^c$)-Th-2-yl | Py-2-yl |
| 1338 | H | H | H | 5-(1-$CH_2$OPrPr$^c$)-Th-2-yl | Py-3-yl |
| 1339 | H | H | H | 5-(1-$CH_2$OPrPr$^c$)-Th-2-yl | Py-4-yl |
| 1340 | H | H | H | 4-(1-$CH_2CH_2$OEtPr$^c$)-Ph | 4-F-Ph |
| 1341 | H | H | H | 4-(1-$CH_2CH_2$OEtPr$^c$)-Ph | Py-2-yl |
| 1342 | H | H | H | 4-(1-$CH_2CH_2$OEtPr$^c$)-Ph | Py-3-yl |
| 1343 | H | H | H | 4-(1-$CH_2CH_2$OPrPr$^c$)-Ph | 4-F-Ph |
| 1344 | H | H | H | 4-(1-$CH_2CH_2$OPrPr$^c$)-Ph | Py-2-yl |
| 1345 | H | H | H | 4-(1-$CH_2CH_2$OPrPr$^c$)-Ph | Py-3-yl |
| 1346 | H | H | H | 4-(1-$CH_2$OBuPr$^c$)-Ph | 4-F-Ph |
| 1347 | H | H | H | 4-(1-$CH_2$OBuPr$^c$)-Ph | Py-2-yl |
| 1348 | H | H | H | 4-(1-$CH_2$OBuPr$^c$)-Ph | Py-3-yl |
| 1349 | H | H | H | 4-(1-$CH_2$OBnPr$^c$)-Ph | Ph |
| 1350 | H | H | H | 4-(1-$CH_2$OBnPr$^c$)-Ph | 2-F-Ph |
| 1351 | H | H | H | 4-(1-$CH_2$OBnPr$^c$)-Ph | 3-F-Ph |
| 1352 | H | H | H | 4-(1-$CH_2$OBnPr$^c$)-Ph | 4-F-Ph |
| 1353 | H | H | H | 4-(1-$CH_2$OBnPr$^c$)-Ph | 3, 4-diF-Ph |
| 1354 | H | H | H | 4-(1-$CH_2$OBnPr$^c$)-Ph | 3, 5-diF-Ph |

(continued)

| Compound No. | $R^1$ | $R^2$ | $R^3$ | Y | Z |
|---|---|---|---|---|---|
| 1355 | H | H | H | 4-(1-$CH_2$OBnPr$^c$)-Ph | 4-Cl-Ph |
| 1356 | H | H | H | 4-(1-$CH_2$OBnPr$^c$)-Ph | 4-Me-Ph |
| 1357 | H | H | H | 4-(1-$CH_2$OBnPr$^c$)-Ph | 4-$CF_3$-Ph |
| 1358 | H | H | H | 4-(1-$CH_2$OBnPr$^c$)-Ph | 4-OMe-Ph |
| 1359 | H | H | H | 4-(1-$CH_2$OBnPr$^c$)-Ph | Fu-2-yl |
| 1360 | H | H | H | 4-(1-$CH_2$OBnPr$^c$)-Ph | Th-2-yl |
| 1361 | H | H | H | 4-(1-$CH_2$OBnPr$^c$)-Ph | Th-3-yl |
| 1362 | H | H | H | 4- (1-$CH_2$OBnPr$^c$) -Ph | 1-Me-Im-4-yl |
| 1363 | H | H | H | 4-(1-$CH_2$OBnPr$^c$)-Ph | Tz-2-yl |
| 1364 | H | H | H | 4-(1-$CH_2$OBnPr$^c$)-Ph | Py-2-yl |
| 1365 | Me | H | H | 4-(1-$CH_2$OBnPr$^c$)-Ph | Py-2-yl |
| 1366 | Et | H | H | 4-(1-$CH_2$OBnPr$^c$)-Ph | Py-2-yl |
| 1367 | $Pr^1$ | H | H | 4-(1-$CH_2$OBnPr$^c$)-Ph | Py-2-yl |
| 1368 | H | H | H | 4-(1-$CH_2$OBnPr$^c$)-Ph | 5-F-Py-2-yl |
| 1369 | H | H | H | 4-(1-$CH_2$OBnPr$^c$)-Ph | Py-3-yl |
| 1370 | H | H | H | 4-(1-$CH_2$OBnPr$^c$)-Ph | 6-F-Py-3-yl |
| 1371 | H | H | H | 4-(1-$CH_2$OBnPr$^c$)-Ph | Py-4-yl |
| 1372 | H | H | H | 4-(1-$CH_2CH_2$OBnPr$^c$)-Ph | 4-F-Ph |
| 1373 | H | H | H | 4-(1-$CH_2CH_2$OBnPr$^c$)-Ph | Py-2-yl |
| 1374 | H | H | H | 4-(1-$CH_2CH_2$OBnPr$^c$)-Ph | Py-3-yl |
| 1375 | H | H | H | 4-(1-$CH_2$OCHPhMePr$^c$)-Ph | 4-F-Ph |
| 1376 | H | H | H | 4-(1-$CH_2$OCHPhMePr$^c$)-Ph | Py-2-yl |
| 1377 | H | H | H | 4-(1-$CH_2$OCHPhMePr$^c$)-Ph | Py-3-yl |
| 1378 | H | H | H | 4-(1-$CH_2OCH_2CH_2$PhPr$^c$)-Ph | 4-F-Ph |
| 1379 | H | H | H | 4-(1-$CH_2OCH_2CH_2$PhPr$^c$)-Ph | Py-2-yl |
| 1380 | H | H | H | 4-(1-$CH_2OCH_2CH_2$PhPr$^c$)-Ph | Py-3-yl |
| 1381 | H | H | H | 4-(1-EtBu$^c$)-Ph | Ph |
| 1382 | H | H | H | 4-(1-EtBu$^c$)-Ph | 2-F-Ph |
| 1383 | H | H | H | 4-(1-EtBu$^c$)-Ph | 3-F-Ph |
| 1384 | H | H | H | 4-(1-EtBu$^c$)-Ph | 4-F-Ph |
| 1385 | H | H | H | 4-(1-EtBu$^c$)-Ph | 3, 4-diF-Ph |
| 1386 | H | H | H | 4-(1-EtBu$^c$)-Ph | 3, 5-diF-Ph |
| 1387 | H | H | H | 4-(1-EtBu$^c$)-Ph | 4-Cl-Ph |
| 1388 | H | H | H | 4-(1-EtBu$^c$)-Ph | 4-Me-Ph |
| 1389 | H | H | H | 4-(1-EtBu$^c$)-Ph | 4-$CF_3$-Ph |
| 1390 | H | H | H | 4-(1-EtBu$^c$)-Ph | 4-OMe-Ph |
| 1391 | H | H | H | 4-(1-EtBu$^c$)-Ph | Fu-2-yl |
| 1392 | H | H | H | 4-(1-EtBu$^c$)-Ph | Th-2-yl |
| 1393 | H | H | H | 4-(1-EtBu$^c$)-Ph | Th-3-yl |
| 1394 | H | H | H | 4-(1-EtBu$^c$)-Ph | 1-Me-Im-4-yl |
| 1395 | H | H | H | 4-(1-EtBu$^c$)-Ph | Tz-2-yl |
| 1396 | H | H | H | 4-(1-EtBu$^c$)-Ph | Py-2-yl |
| 1397 | H | H | H | 4-(1-EtBu$^c$)-Ph | 5-F-Py-2-yl |
| 1398 | H | H | H | 4-(1-EtBu$^c$)-Ph | Py-3-yl |
| 1399 | Me | H | H | 4-(1-EtBu$^c$)-Ph | Py-3-yl |
| 1400 | Et | H | H | 4-(1-EtBu$^c$)-Ph | Py-3-yl |
| 1401 | $Pr^1$ | H | H | 4-(1-EtBu$^c$)-Ph | Py-3-yl |
| 1402 | H | H | H | 4-(1-EtBu$^c$)-Ph | 6-F-Py-3-yl |
| 1403 | H | H | H | 4-(1-EtBu$^c$)-Ph | Py-4-yl |
| 1404 | H | H | H | 4-(1-BuBu$^c$)-Ph | Ph |

(continued)

| Compound No. | $R^1$ | $R^2$ | $R^3$ | Y | Z |
|---|---|---|---|---|---|
| 1405 | H | H | H | 4-(1-BuBu$^c$)-Ph | 2-F-Ph |
| 1406 | H | H | H | 4-(1-BuBu$^c$)-Ph | 3-F-Ph |
| 1407 | H | H | H | 4-(1-BuBu$^c$)-Ph | 4-F-Ph |
| 1408 | H | H | H | 4-(1-BuBu$^c$)-Ph | 3, 4-diF-Ph |
| 1409 | H | H | H | 4-(1-BuBu$^c$)-Ph | 3, 5-diF-Ph |
| 1410 | H | H | H | 4-(1-BuBu$^c$)-Ph | 4-Cl-Ph |
| 1411 | H | H | H | 4-(1-BuBu$^c$)-Ph | 4-Me-Ph |
| 1412 | H | H | H | 4-(1-BuBu$^c$)-Ph | 4-$CF_3$-Ph |
| 1413 | H | H | H | 4-(1-BuBu$^c$)-Ph | 4-OMe-Ph |
| 1414 | H | H | H | 4-(1-BuBu$^c$)-Ph | Fu-2-yl |
| 1415 | H | H | H | 4-(1-BuBu$^c$)-Ph | Th-2-yl |
| 1416 | H | H | H | 4-(1-BuBu$^c$)-Ph | Th-3-yl |
| 1417 | H | H | H | 4-(1-BuBu$^c$)-Ph | 1-Me-Im-4-yl |
| 1418 | H | H | H | 4-(1-BuBu$^c$)-Ph | Tz-2-yl |
| 1419 | H | H | H | 4-(1-BuBu$^c$)-Ph | Py-2-yl |
| 1420 | Me | H | H | 4-(1-BuBu$^c$)-Ph | Py-2-yl |
| 1421 | Et | H | H | 4-(1-BuBu$^c$)-Ph | Py-2-yl |
| 1422 | Pr$^1$ | H | H | 4-(1-BuBu$^c$)-Ph | Py-2-yl |
| 1423 | H | H | H | 4-(1-BuBu$^c$)-Ph | 5-F-Py-2-yl |
| 1424 | H | H | H | 4-(1-BuBu$^c$)-Ph | Py-3-yl |
| 1425 | H | H | H | 4-(1-BuBu$^c$)-Ph | 6-F-Py-3-yl |
| 1426 | H | H | H | 4-(1-BuBu$^c$)-Ph | Py-4-yl |
| 1427 | H | H | H | 5-(1-BuBu$^c$)-Th-2-yl | 4-F-Ph |
| 1428 | H | H | H | 5-(1-BuBu$^c$)-Th-2-yl | Py-2-yl |
| 1429 | H | H | H | 5-(1-BuBu$^c$)-Th-2-yl | Py-3-yl |
| 1430 | H | H | H | 3,4-diEt-Ph | 5-Me-Fu-2-yl |
| 1431 | H | H | H | 3-Bu-Ph | 5-Me-Fu-2-yl |
| 1432 | H | H | H | 3-Bu$^t$-Ph | 5-Me-Fu-2-yl |
| 1433 | H | H | H | 4-Bu$^t$-Ph | 5-Me-Fu-2-yl |
| 1434 | H | H | H | 4-Pn-Ph | 5-Me-Fu-2-yl |
| 1435 | H | H | H | 3-$NMe_2$-4-Pn-Ph | 5-Me-Fu-2-yl |
| 1436 | H | H | H | 4-Pn$^{neo}$-Ph | 5-Me-Fu-2-yl |
| 1437 | H | H | H | 4-Pn$^t$-Ph | 5-Me-Fu-2-yl |
| 1438 | H | H | H | 5-Pn$^t$-Th-2-yl | 5-Me-Fu-2-yl |
| 1439 | H | H | H | 4-(1-EtPr)-Ph | 5-Me-Fu-2-yl |
| 1440 | H | H | H | 3-Hx-Ph | 5-Me-Fu-2-yl |
| 1441 | H | H | H | 4-(1-MePn)-Ph | 5-Me-Fu-2-yl |
| 1442 | H | H | H | 4-(1,1-diMeBu)-Ph | 5-Me-Fu-2-yl |
| 1443 | H | H | H | 4-(1-Et-1-MePr)-Ph | 5-Me-Fu-2-yl |
| 1444 | H | H | H | 4-(1,1-diMePn)-Ph | 5-Me-Fu-2-yl |
| 1445 | H | H | H | 2-F-4-(1,1-diMePn)-Ph | 5-Me-Fu-2-yl |
| 1446 | H | H | H | 3-F-4-(1,1-diMePn)-Ph | 5-Me-Fu-2-yl |
| 1447 | H | H | H | 5-(1,1-diMePn)-Th-2-yl | 5-Me-Fu-2-yl |
| 1448 | H | H | H | 4-(2,2-diMePn)-Ph | 5-Me-Fu-2-yl |
| 1449 | H | H | H | 4-(1,1,3,3-tetraMeBu)-Ph | 5-Me-Fu-2-yl |
| 1450 | H | H | H | 4-(1,1,4-triMePn)-Ph | 5-Me-Fu-2-yl |
| 1451 | H | H | H | 4-(1,1-diMeHx)-Ph | 5-Me-Fu-2-yl |
| 1452 | H | H | H | 4-$CF_3$-Ph | 5-Me-Fu-2-yl |
| 1453 | H | H | H | 4-(5,5,5-triF-1,1-diMePn)-Ph | 5-Me-Fu-2-yl |
| 1454 | H | H | H | 4-OBu-Ph | 5-Me-Fu-2-yl |

(continued)

| Compound No. | $R^1$ | $R^2$ | $R^3$ | Y | Z |
|---|---|---|---|---|---|
| 1455 | H | H | H | 4-$OCHF_2$-Ph | 5-Me-Fu-2-yl |
| 1456 | H | H | H | 4-(3-$OCHF_2$-1,1-diMePr)-Ph | 5-Me-Fu-2-yl |
| 1457 | H | H | H | 4-(1-Me-1-Pne)-Ph | 5-Me-Fu-2-yl |
| 1458 | H | H | H | 4-(1-MePr$^c$)-Ph | 5-Me-Fu-2-yl |
| 1459 | H | H | H | 4-(1-EtPr$^c$)-Ph | 5-Me-Fu-2-yl |
| 1460 | H | H | H | 4-(1-Pr$^1$Pr$^c$)-Ph | 5-Me-Fu-2-yl |
| 1461 | H | H | H | 4-(1-BuPr$^c$)-Ph | 5-Me-Fu-2-yl |
| 1462 | H | H | H | 4-(1-NnPr$^c$)-Ph | 5-Me-Fu-2-yl |
| 1463 | H | H | H | 4-(1-OMePr$^c$)-Ph | 5-Me-Fu-2-yl |
| 1464 | H | H | H | 4-(1-BnPr$^c$)-Ph | 5-Me-Fu-2-yl |
| 1465 | H | H | H | 4-(1-$CH_2$OPrPr$^c$)-Ph | 5-Me-Fu-2-yl |
| 1466 | H | H | H | 4-(1-$CH_2$OBnPr$^c$)-Ph | 5-Me-Fu-2-yl |
| 1467 | H | H | H | 4-(1-EtBu$^c$)-Ph | 5-Me-Fu-2-yl |
| 1468 | H | H | H | 4-(1-BuBu$^c$)-Ph | 5-Me-Fu-2-yl |

Incidentally, the abbreviations in the above-mentioned table represent the following groups.
H: a hydrogen atom,
Me: methyl group,
Et: ethyl group,
Pr$^i$: isopropyl group,
4-Et-Ph: 4-ethylphenyl group,
3,4-diEt-Ph: 3,4-diethylphenyl group,
4-Pr-Ph: 4-propylphenyl group,
4-Pr$^i$-Ph: 4-isopropylphenyl group,
3-Bu-Ph: 3-butylphenyl group,
4-Bu-Ph: 4-butylphenyl group,
3-Bu$^t$-Ph: 3-tert-butylphenyl group,
4-Bu$^t$-Ph: 4-tert-butylphenyl group,
5-Bu$^t$-Th-2-yl: 5-tert-butylthiophen-2-yl group,
3-Pn-Ph: 3-pentylphenyl group,
4-Pn-Ph: 4-pentylphenyl group,
3-$NMe_2$-4-Pn-Ph: 3-dimethylamino-4-pentylphenyl group,
4-Pn$^i$-Ph: 4-isopentylphenyl group,
4-Pn$^{neo}$-Ph: 4-neopentylphenyl group,
4-Pn$^t$-Ph: 4-tert-pentylphenyl group,
5-Pn$^t$-Th-2-yl: 5-tert-pentylthiophen-2-yl group,
4-(1-EtPr)-Ph: 4-(1-ethylpropyl)phenyl group,
4-(1-EtBu)-Ph: 4-(1-ethylbutyl)phenyl group,
3-Hx-Ph: 3-hexylphenyl group,
4-(1-MePn)-Ph: 4-(1-methylpentyl)phenyl group,
5-(1-MePn)-Th-2-yl: 5-(1-methylpentyl)thiophen-2-yl group,
3-(1,1-diMeBu)-Ph: 3-(1,1-dimethylbutyl)phenyl group,
4-(1,1-diMeBu)-Ph: 4-(1,1-dimethylbutyl)phenyl group,
2-F-4-(1,1-diMeBu)-Ph: 2-fluoro-4-(1,1-dimethylbutyl)phenyl group,
3-F-4-(1,1-diMeBu)-Ph: 3-fluoro-4-(1,1-dimethylbutyl)phenyl group,
4-(2,2-diMeBu)-Ph: 4-(2,2-dimethylbutyl)phenyl group,
5-(1,1-diMeBu)-Th-2-yl: 5-(1,1-dimethylbutyl)thiophen-2-yl group,
4-(1-Et-1-MePr)-Ph: 4-(1-ethyl-1-methylpropyl)phenyl group,
3-(1,1-diMePn)-Ph: 3-(1,1-dimethylpentyl)phenyl group,
4-(1,1-diMePn)-Ph: 4-(1,1-dimethylpentyl)phenyl group,
2-F-4-(1,1-diMePn)-Ph: 2-fluoro-4-(1,1-dimethylpentyl)phenyl group,
3-F-4-(1,1-diMePn)-Ph: 3-fluoro-4-(1,1-dimethylpentyl)phenyl group,
3-$NH_2$-4-(1,1-diMePn)-Ph: 3-amino-4-(1,1-dimethylpentyl)phenyl group,

3-NHMe-4-(1,1-diMePn)-Ph: 3-methylamino-4-(1,1-dimethylpentyl)phenyl group,
$3-NMe_2$-4-(1,1-diMePn)-Ph: 3-dimethylamino-4-(1,1-dimethylpentyl)phenyl group,
4-(1,1-diMePn)-Th-2-yl: 4-(1,1-dimethylpentyl)thiophen-2-yl group,
5-(1,1-diMePn)-Th-2-yl: 5-(1,1-dimethylpentyl)thiophen-2-yl group,
5-(1,1-diMePn)-Tz-2-yl: 5-(1,1-dimethylpentyl)thiazol-2-yl group,
5-(1,1-diMePn)-Py-2-yl: 5-(1,1-dimethylpentyl)pyridin-2-yl group,
6-(1,1-diMePn)-Py-3-yl: 6-(1,1-dimethylpentyl)pyridin-3-yl group,
5-(1,1-diMePn)-Pm-2-yl: 5-(1,1-dimethylpentyl)pyrimidin-2-yl group,
2-(1,1-diMePn)-Pm-5-yl: 2-(1,1-dimethylpentyl)pyrimidin-5-yl group,
3-(2,2-diMePn)-Ph: 3-(2,2-dimethylpentyl)phenyl group,
4-(2,2-diMePn)-Ph: 4-(2,2-dimethylpentyl)phenyl group,
4-(1-MeHx)-Ph: 4-(1-methylhexyl)phenyl group,
4-(1,1,3,3-tetraMeBu)-Ph: 4-(1,1,3,3-tetramethylbutyl)phenyl group,
4-(1,1,4-triMePn)-Ph: 4-(1,1,4-trimethylpentyl)phenyl group,
4-(1,1-diMeHx)-Ph: 4-(1,1-dimethylhexyl)phenyl group,
$4-CF_3$-Ph: 4-trifluoromethylphenyl group,
4-(4,4,4-triFBu)-Ph: 4-(4,4,4-trifluorobutyl)phenyl group,
4-(5,5,5-triFPn)-Ph: 4-(5,5,5-trifluoropentyl)phenyl group,
4-(6,6,6-triFHx)-Ph: 4-(6,6,6-trifluorohexyl)phenyl group,
4-(5,5,5-triF-1-MePn)-Ph: 4-(5,5,5-trifluoro-1-methylpentyl)phenyl group,
4-(4,4,4-triF-1,1-diMeBu)-Ph: 4-(4,4,4-trifluoro-1,1-dimethylbutyl)phenyl group,
4-(5,5,5-triF-1,1-diMePn)-Ph: 4-(5,5,5-trifluoro-1,1-dimethylpentyl)phenyl group,
4-(5,5,5-triF-2,2-diMePn)-Ph: 4-(5,5,5-trifluoro-2,2-dimethylpentyl)phenyl group,
5-(5,5,5-triF-1,1-diMePn)-Th-2-yl: 5-(5,5,5-trifluoro-1,1-dimethylpentyl)thiophen-2-yl group,
4-OPr-Ph: 4-propoxyphenyl group,
4-OBu-Ph: 4-butoxyphenyl group,
$4-OBu^i$-Ph: 4-isobutoxyphenyl group,
$4-OCHF_2$-Ph: 4-difluoromethoxyphenyl group,
$4-OCF_3$-Ph: 4-trifluoromethoxyphenyl group,
$4-(3-OCHF_2$-1-MePr)-Ph: 4-(3-difluoromethoxy-1-methylpropyl)phenyl group,
$4-(3-OCHF_2$-1,1-diMePr)-Ph: 4-(3-difluoromethoxy-1,1-dimethylpropyl)phenyl group,
$4-(3-Pr^c$-1-MePr)-Ph: 4-(3-cyclopropyl-1-methylpropyl)phenyl group,
$4-(3-Pr^c$-1,1-diMePr)-Ph: 4-(3-cyclopropyl-1,1-dimethylpropyl)phenyl group,
$4-Pre^i$-Ph: 4-isopropenylphenyl group,
4-(1-Me-1-Pre)-Ph: 4-(1-methyl-1-propenyl)phenyl group,
4-(1-Me-1-Bue)-Ph: 4-(1-methyl-1-butenyl)phenyl group,
4-(1-Me-1-Pne)-Ph: 4-(1-methyl-1-pentenyl)phenyl group,
$4-(1-MePr^c)$-Ph: 4-(1-methylcyclopropyl)phenyl group,
$4-(1-EtPr^c)$-Ph: 4-(1-ethylcyclopropyl)phenyl group,
$4-(1-PrPr^c)$-Ph: 4-(1-propylcyclopropyl)phenyl group,
$4-(1-Pr^iPr^c)$-Ph: 4-(1-isopropylcyclopropyl)phenyl group,
$3-(1-BuPr^c)$-Ph: 3-(1-butylcyclopropyl)phenyl group,
$4-(1-BuPr^c)$-Ph: 4-(1-butylcyclopropyl)phenyl group,
$2-F-4-(1-BuPr^c)$-Ph: 2-fluoro-4-(1-butylcyclopropyl)phenyl group,
$3-F-4-(1-BuPr^c)$-Ph: 3-fluoro-4-(1-butylcyclopropyl)phenyl group,
$3-NMe_2-4-(1-BnPr^c)$-Ph: 3-dimethylamino-4-(1-butylcyclopropyl)phenyl group,
$5-(1-BuPr^c)$-Th-2-yl: 5-(1-butylcyclopropyl)thiophen-2-yl group,
$4-(1-HxPr^c)$-Ph: 4-(1-hexylcyclopropyl)phenyl group,
$4-(1-NnPr^c)$-Ph: 4-(1-nonylcyclopropyl)phenyl group,
$4-(1-OMePr^c)$-Ph: 4-(1-methoxycyclopropyl)phenyl group,
$4-(1-OEtPr^c)$-Ph: 4-(1-ethoxycyclopropyl)phenyl group,
$4-(1-OPrPr^c)$-Ph: 4-(1-propoxycyclopropyl)phenyl group,
$4-(1-BnPr^c)$-Ph: 4-(1-benzylcyclopropyl)phenyl group,
$4-(1-CHPhMePr^c)$-Ph: 4-{1-(1-phenylethyl)cyclopropyl}phenyl group,
$4-(1-CH_2CH_2PhPr^c)$-Ph: 4-{1-(2-phenylethyl)cyclopropyl}phenyl group,
$4-(1-CH_2OEtPr^c)$-Ph: 4-(1-ethoxymethylcyclopropyl)phenyl group,
$4-(1-CH_2OPrPr^c)$-Ph: 4-(1-propoxymethylcyclopropyl)phenyl group,
$5-(1-CH_2OPrPr^c)$-Th-2-yl: 5-(1-propoxymethylcyclopropyl)thiophen-2-yl group,

4-(1-$CH_2CH_2OEtPr^c$)-Ph: 4-{1-(2-ethoxyethyl)cyclopropyl}phenyl group,
4-(1-$CH_2CH_2OPrPr^c$)-Ph: 4-{1-(2-propoxyethyl)cyclopropyl}phenyl group,
4-(1-$CH_2OBuPr^c$)-Ph: 4-(1-butoxymethylcyclopropyl)phenyl group,
4-(1-$CH_2OBnPr^c$)-Ph: 4-(1-benzyloxymethylcyclopropyl)phenyl group,
4-(-$CH_2CH_2OBnPr^c$)-Ph: 4-{1-(2-benzyloxyethyl)cyclopropyl}phenyl group,
4-(1-$CH_2OCHPhMePr^c$)-Ph: 4-{1-(1-phenylethoxymethyl)cyclopropyl}phenyl group,
4-(1-$CH_2OCH_2CH_2PhPr^c$)-Ph: 4-{1-(2-phenylethoxymethyl)cyclopropyl}phenyl group,
4-(1-$EtBu^c$)-Ph: 4-(1-ethylcyclobutyl)phenyl group,
4-(1-$BuBu^c$)-Ph: 4-(1-butylcyclobutyl)phenyl group,
5-(1-$BuBu^c$)-Th-2-yl: 5-(1-butylcyclobutyl)thiophen-2-yl group,
Ph: phenyl group, 2-F-Ph: 2-fluorophenyl group,
3-F-Ph: 3-fluorophenyl group,
4-F-Ph: 4-fluorophenyl group,
3,4-diF-Ph: 3,4-difluorophenyl group,
3,5-diF-Ph: 3,5-difluorophenyl group,
2-Cl-Ph: 2-chlorophenyl group,
3-Cl-Ph: 3-chlorophenyl group,
4-Cl-Ph: 4-chlorophenyl group,
2,6-diCl-Ph: 2,6-dichlorophenyl group,
4-Cl-3-F-Ph: 4-chloro-3-fluorophenyl group,
4-Me-Ph: 4-methylphenyl group,
3-F-4-Me-Ph: 3-fluoro-4-methylphenyl group,
4-$CF_3$-Ph: 4-trifluoromethylphenyl group,
3-F-4-$CF_3$-Ph: 3-fluoro-4-trifluoromethylphenyl group,
4-OMe-Ph: 4-methoxyphenyl group,
3-F-4-OMe-Ph: 3-fluoro-4-methoxyphenyl group,
4-$OCHF_2$-Ph: 4-difluoromethoxyphenyl group,
4-$OCHF_2$-3-F-Ph: 4-difluoromethoxy-3-fluorophenyl group,
Fu-2-yl: furan-2-yl group,
Th-2-yl: thiophen-2-yl group,
Th-3-yl: thiophen-3-yl group,
1-Me-Im-4-yl: 1-methyl-1H-imidazol-4-yl group,
Tz-2-yl: thiazol-2-yl group,
Py-2-yl: pyridin-2-yl group,
5-F-Py-2-yl: 5-fluoropyridin-2-yl group,
5-Cl-Py-2-yl: 5-chloropyridin-2-yl group,
5-OMe-Py-2-yl: 5-methoxypyridin-2-yl group,
Py-3-yl: pyridin-3-yl group,
6-F-Py-3-yl: 6-fluoropyridin-3-yl group,
6-Cl-Py-3-yl: 6-chloropyridin-3-yl group,
6-OMe-Py-3-yl: 6-methoxypyridin-3-yl group,
Py-4-yl: pyridin-4-yl group, or
5-Me-Fu-2-yl: 5-methylfuran-2-yl group.

In the above-mentioned Table, further preferred are compounds of Compound No. 2, 3, 4, 7, 19, 24, 28, 29, 31, 33, 36, 48, 53, 59, 66, 67, 84, 86, 92, 96, 100, 104, 110, 116, 125, 130, 135, 136, 139, 146, 147, 150, 155, 167, 172, 175, 178, 190, 195, 197, 208, 209, 211, 214, 226, 231, 234, 237, 245, 248, 249, 254, 256, 257, 260, 268, 271, 272,277,279,280,283,295,297,302,306,313,314,319,322,334,339,342, 345, 357, 362, 364, 365, 368, 375, 376, 381, 388, 391, 394, 404, 405, 408, 409, 416, 421, 425, 432, 433, 434, 438, 445, 446, 447, 449, 451, 454, 458, 460, 461, 462, 463, 464, 467, 479, 481, 486, 490, 497, 498, 500, 504, 508, 512, 516; 517, 518, 519, 520, 524, 525, 526, 527, 528, 529, 530, 534; 535, 536, 537, 541, 545, 546, 547, 551, 552; 553, 554, 555, 556, 557, 558, 559, 560, 564, 565, 566, 567, 571, 574, 586, 591, 593, 594, 597, 609, 614, 616, 620, 626, 633, 634, 637, 639, 641, 642,646, 650, 653, 657, 659, 661, 662,665, 666, 667, 668, 669, 670, 673, 677, 680, 682,683, 685, 686, 688, 689, 691, 692,694, 695, 697, 698, 699, 702, 714, 719, 721, 724, 725, 728, 740, 745, 747, 748, 751, 763, 768, 770, 771, 774, 781, 782, 785, 786, 791, 793, 797, 809, 811, 818, 821, 824, 827, 829, 832, 839, 840, 842, 845, 857, 862, 864, 866, 867, 868, 871, 878, 879, 882, 884, 887, 899, 904, 908, 910, 913, 925, 927, 934, 937, 938, 939, 942, 954, 956, 959, 961, 963, 965, 968, 975, 976, 977, 979, 982, 984, 987, 994, 995, 997, 1000, 1012, 1017, 1019, 1020, 1023, 1035, 1037, 1042, 1043, 1046, 1061, 1066, 1071, 1072, 1075, 1082, 1083, 1084, 1085, 1088, 1100, 1102, 1109, 1111, 1113, 1114, 1121, 1123, 1128, 1130, 1132, 1134, 1135, 1136, 1138, 1142, 1143, 1144, 1145, 1146, 1147, 1148, 1149, 1152, 1158, 1161, 1165, 1168, 1175, 1176, 1178, 1181, 1188,

1189, 1192, 1194, 1197, 1204, 1205, 1208, 1210, 1213, 1225, 1227, 1232, 1233, 1236, 1248, 1253, 1256, 1257, 1258, 1260, 1262, 1265, 1277, 1282, 1284, 1286, 1291, 1298, 1299, 1302, 1303, 1304, 1307, 1314, 1315, 1318, 1319, 1324, 1326, 1327, 1330, 1337, 1338, 1341, 1344, 1347, 1349, 1352, 1364, 1369, 1373, 1376, 1379, 1381, 1384, 1391, 1392, 1395, 1396, 1398, 1403, 1404, 1407, 1414, 1415, 1418, 1419, 1424, 1426, 1428, 1429, 1433, 1437, 1438, 1441, 1442, 1444, 1447, 1457, 1461, 1465 or 1468,

further more preferred are compounds of Compound No. 2, 7, 19, 24, 28, 31, 48, 53, 66, 67, 84, 86, 92, 96, 100, 104, 110, 116, 125, 130, 139, 146, 147, 150, 167, 172, 178, 190, 195, 208, 209, 226, 231, 234, 237, 249, 254, 260, 272,277, 283, 295, 297, 306, 313, 314, 322, 334, 339, 342, 345, 357, 362, 368, 375, 388, 391, 394, 405, 408, 409, 416, 421, 432, 433, 445, 446, 447, 449, 461, 462, 467, 479, 481, 497, 500, 504, 508, 512, 520, 530, 537, 541, 547, 551, 554, 555, 556, 560, 567, 574, 586, 591, 597, 609, 614, 620, 633, 634, 637, 639, 641, 642,662,665, 666, 667, 668, 669, 673, 680, 682,685, 688, 691, 694, 697, 702, 714, 719, 724, 728, 740, 745, 751, 763, 768, 770, 771, 774, 786, 791, 793, 809, 811, 818, 821, 824, 827, 832, 839, 845, 857, 862, 864, 866, 871, 878, 879, 882, 887, 899, 904, 908, 913, 925, 927, 934, 937, 938, 942, 954, 956, 959, 963, 968, 975, 976, 979, 982, 987, 994, 995, 1000, 1012, 1017, 1023, 1035, 1037, 1042, 1043, 1046, 1061, 1066, 1071, 1075, 1082, 1083, 1088, 1100, 1102, 1109, 1111, 1113, 1114, 1136, 1138, 1143, 1146, 1147, 1148, 1152, 1161, 1175, 1176, 1188, 1189, 1192, 1197, 1204, 1205, 1208, 1213, 1225, 1227, 1236, 1248, 1253, 1257, 1260, 1265, 1277, 1282, 1286, 1298, 1302, 1303, 1307, 1319, 1324, 1326, 1330, 1337, 1338, 1341, 1344, 1347, 1352, 1364, 1369, 13733 1376, 1379, 1384, 1396, 1398, 1403, 1407, 1419, 1424, 1426, 1428, 1429, 1433, 1437, 1438, 1442, 1444 or 1461,

particularly preferred are compounds of Compound No. 19, 48, 66, 84, 86, 92, 96, 100, 104, 110, 116, 125, 130, 146, 167, 190, 195, 208, 226, 237, 249, 254, 260, 272,277, 295, 297, 313, 334, 345, 357, 362, 375, 388, 394, 409, 416, 421, 432, 445, 461, 479, 481, 497, 500, 504, 508, 512, 520, 530, 537, 541, 547, 551, 554, 555, 556, 560, 567, 586, 591, 609, 614, 633, 642,666, 667, 668, 669, 673, 680, 714, 719, 740, 763, 774, 786, 791, 793, 811, 839, 845, 857, 862, 878, 899, 927, 954, 956, 959, 975, 994, 995, 1012, 1017, 1035, 1037, 1046, 1061, 1066, 1071, 1082, 1100, 1102, 1114, 1138, 1143, 1146, 1147, 1148, 1152, 1161, 1175, 1188, 1204, 1227, 1248, 1277, 1319, 1324, 1337, 1364, 1396, 1398, 1407, 1419, 1424, 1428, 1442, 1444 or 1461, and

most preferred are compounds of

Compound No. 19: {6-[(3,4-diethylbenzyl)(pyridin-2-ylsulfonyl)aminomethyl]pyridin-2-ylamino}acetic acid,
Compound No. 48: {6-[(3-butylbenzyl)(pyridin-2-ylsulfonyl)aminomethyl]pyridin-2-ylamino} acetic acid,
Compound No. 86: {6-[(3-tert-butylbenzyl)(pyridin-3-ylsulfonyl)aminomethyl]pyridin-2-ylamino}acetic acid,
Compound No. 92: {6-[(benzenesulfonyl)(4-tert-butylbenzyl)aminomethyl]pyridin-2-ylamino}acetic acid,
Compound No. 96: {6-[(4-tert-butylbenzyl)(2-fluorobenzenesulfonyl)aminomethyl]-pyridin-2-ylamino}acetic acid,
Compound No. 100: {6-[(4-tert-butylbenzyl)(3-fluorobenzenesulfonyl)aminomethyl]-pyridin-2-ylamino}acetic acid,
Compound No. 104: {6-[(4-tert-butylbenzyl)(4-fluorobenzenesulfonyl)aminomethyl]-pyridin-2-ylamino}acetic acid,
Compound No. 110: {6-[(4-tert-butylbenzyl)(4-chlorobenzenesulfonyl)aminomethyl]-pyridin-2-ylamino}acetic acid,
Compound No. 116: {6-[(4-tert-butylbenzyl)(4-methoxybenzenesulfonyl)aminomethyl]pyridin-2-ylamino}acetic acid,
Compound No. 125: {6-[(4-tert-butylbenzyl)(pyridin-2-ylsulfonyl)aminomethyl]-pyridin-2-ylamino} acetic acid,
Compound No. 130: {6-[(4-tert-butylbenzyl)(pyridin-3-ylsulfonyl)aminomethyl]-pyridin-2-ylamino}acetic acid,
Compound No. 167: {6-[(4-pentylbenzyl)(pyridin-2-ylsulfonyl)aminomethyl]pyridin-2-ylamino} acetic acid,
Compound No. 190: {6-[(3-dimethylamino-4-pentylbenzyl)(pyridin-2-ylsulfonyl)-aminomethyl]pyridin-2-ylamino} acetic acid,
Compound No. 226: {6-[(4-neopentylbenzyl)(pyridin-2-ylsulfonyl)aminomethyl]-pyridin-2-ylamino} acetic acid,
Compound No. 249: {6-[(4-tert-pentylbenzyl)(pyridin-2-ylsulfonyl)aminomethyl]-pyridin-2-ylamino} acetic acid,
Compound No. 272: {6-[(5-tert-pentylthiophen-2-ylmethyl)(pyridin-2-ylsulfonyl)-aminomethyl]pyridin-2-ylamino}acetic acid,
Compound No. 297: (6-{[4-(1-ethylpropyl)benzyl](pyridin-3-ylsulfonyl)aminomethyl}pyridin-2-ylamino)acetic acid,
Compound No. 334: {6-[(3-hexylbenzyl)(pyridin-2-ylsulfonyl)aminomethyl]pyridin-2-ylamino}acetic acid,
Compound No. 357: (6-{[4-(1-methylpentyl)benzyl](pyridin-2-ylsulfonyl)aminomethyl}pyridin-2-ylamino)acetic acid,
Compound No. 409: (6-{[4-(1,1-dimethylbutyl)benzyl](pyridin-2-ylsulfonyl)aminomethyl}pyridin-2-ylamino)acetic acid,
Compound No. 481: (6-{[4-(1-ethyl-1-methylpropyl)benzyl](pyridin-3-ylsulfonyl)-aminomethyl}pyridin-2-ylamino)acetic acid,
Compound No. 500: (6-{(benzenesulfonyl)[4-(1,1-dimethylpentyl)benzyl]aminomethyl}pyridin-2-ylamino)acetic acid,
Compound No. 504: (6-{(2-fluorobenzenesulfonyl)[4-(1,1-dimethylpentyl)benzyl]-aminomethyl}pyridin-2-ylamino)acetic acid,
Compound No. 508: (6-{(3-fluorobenzenesulfonyl)[4-(1,1-dimethylpentyl)benzyl]-aminomethyl}pyridin-2-ylamino)acetic acid,
Compound No. 512: (6-{(4-fluorobenzenesulfonyl)[4-(1,1-dimethylpentyl)benzyl]-aminomethyl}pyridin-2-ylamino)acetic acid,
Compound No. 520: (6-{(4-chlorobenzenesulfonyl)[4-(1,1-dimethylpentyl)benzyl]-aminomethyl}pyridin-2-ylamino)acetic

acid,

Compound No. 530: (6-{(4-methoxybenzenesulfonyl)[4-(1,1-dimethylpentyl)benzyl]-aminomethyl}pyridin-2-ylamino)acetic acid,

Compound No. 537: (6-{(furan-2-ylsulfonyl)[4-(1,1-dimethylpentyl)benzyl]aminomethyl}pyridin-2-ylamino)acetic acid,

Compound No. 541: (6-{[4-(1,1-dimethylpentyl)benzyl](thiophen-2-ylsulfonyl)aminomethyl}pyridin-2-ylamino)acetic acid,

Compound No. 547: (6-{[4-(1,1-dimethylpentyl)benzyl](thiazol-2-ylsulfonyl)aminomethyl}pyridin-2-ylamino)acetic acid,

Compound No. 551: (6-{[4-(1,1-dimethylpentyl)benzyl](pyridin-2-ylsulfonyl)aminomethyl}pyridin-2-ylamino)acetic acid,

Compound No. 560: (6-{[4-(1,1-dimethylpentyl)benzyl](pyridin-3-ylsulxfonyl)aminomethyl}pyridin-2-ylamino)acetic acid,

Compound No. 567: (6-{[4-(1,1-dimethylpentyl)benzyl](pyridin-4-ylsulfonyl)aminomethyl}pyridin-2-ylamino)acetic acid,

Compound No. 586: (6-{[2-fluoro-4-(1,1-dimethylpentyl)benzyl](pyridin-2-ylsulfonyl)-aminomethyl}pyridin-2-ylamino)acetic acid,

Compound No. 609: (6-{[3-fluoro-4-(1,1-dimethylpentyl)benzyl](pyridin-2-ylsulfonyl)-aminomethyl}pyridin-2-ylamino)acetic acid,

Compound No. 642: (6-{(4-fluorobenzenesulfonyl)[5-(1,1-dimethylpentyl)thiophen-2-ylmethyl]aminomethyl}pyridin-2-ylamino)acetic acid,

Compound No. 666: (6-{[5-(1,1-dimethylpentyl)thiophen-2-ylmethyl](pyridin-2-yl-sulfonyl)aminomethyl}pyridin-2-ylamino)acetic acid,

Compound No. 673: (6-{[5-(1,1-dimethylpentyl)thiophen-2-ylmethyl](pyridin-3-yl-sulfonyl)aminomethyl}pyridin-2-ylamino)acetic acid,

Compound No. 714: (6-{[4-(2,2-dimethylpentyl)benzyl](pyridin-2-ylsulfonyl)aminomethyl}pyridin-2-ylamino)acetic acid,

Compound No. 740: (6-{[4-(1,1,3,3-tetramethylbutyl)benzyl](pyridin-2-ylsulfonyl)-aminomethyl}pyridin-2-ylamino)acetic acid,

Compound No. 763: (6-{[4-(1,1,4-trimethylpentyl)benzyl](pyridin-2-ylsulfonyl)aminomethyl}pyridin-2-ylamino)acetic acid,

CompoundNo. 786: (6-{[4-(1,1-dimethylhexyl)benzyl](pyridin-2-ylsulfonyl)aminomethyl}pyridin-2-ylamino)acetic acid,

Compound No. 811: {6-[(pyridin-3-ylsulfonyl)(4-trifluoromethylbenzyl)aminomethyl]-pyridin-2-ylamino} acetic acid,

Compound No. 857: (6-{[4-(5,5,5-trifluoro-1,1-dimethylpentyl)benzyl](pyridin-2-yl-sulfonyl)aminomethyl}pyridin-2-ylamino)acetic acid,

Compound No. 899: {6-[(4-butoxybenzyl)(pyridin-2-ylsulfonyl)aminomethyl]pyridin-2-ylamino}acetic acid,

Compound No. 927: {6-[(4-difluoromethoxybenzyl)(pyridin-3-ylsulfonyl)aminomethyl]pyridin-2-ylamino}acetic acid,

Compound No. 954: (6-{[4-(3-difluoromethoxy-1,1-dimethylpropyl)benzyl](pyridin-2-ylsulfonyl)aminomethyl}pyridin-2-ylamino)acetic acid,

Compound No. 956: (6-{[4-(3-difluoromethoxy-1,1-dimethylpropyl)benzyl](pyridin-2-ylsulfonyl)aminomethyl}pyridin-2-ylamino)ethyl acetate,

Compound No. 1012: (6-{[4-(1-methyl-1-pentenyl)benzyl](pyridin-2-ylsulfonyl)aminomethyl}pyridin-2-ylamino)acetic acid,

Compound No. 1037: (6-{[4-(1-methylcyclopropyl)benzyl](pyridin-3-ylsulfonyl)aminomethyl}pyridin-2-ylamino)acetic acid,

Compound No. 1046: (6-{[4-(1-ethylcyclopropyl)benzyl](4-fluorobenzenesulfonyl)-aminomethyl}pyridin-2-ylamino)acetic acid,

Compound No. 1061: (6-{[4-(1-ethylcyclopropyl)benzyl](pyridin-2-ylsulfonyl)aminomethyl}pyridin-2-ylamino)acetic acid,

Compound No. 1066: (6-{[4-{1-ethylcyclopropyl)benzyl](pyridin-3-ylsulfonyl)aminomethyl}pyridin-2-ylamino)acetic acid,

Compound No. 1102: (6-{[4-(1-isopropylcyclopropyl)benzyl](pyridin-3-ylsulfonyl)-aminomethyl}pyridin-2-ylamino)acetic acid,

Compound No. 1138: (6-{[4-(1-butylcyclopropyl)benzyl](1-methyl-1H-imidazol-4-yl-sulfonyl)aminomethyl}pyridin-2-ylamino)acetic acid,

Compound No. 1143: (6-{[4-(1-butylcyclopropyl)benzyl](pyridin-2-ylsulfonyl)aminomethyl}pyridin-2-ylamino)acetic acid,

Compound No. 1152: (6-{[4-(1-butylcyclopropyl)benzyl](pyridin-3-ylsulfonyl)aminomethyl}pyridin-2-ylamino)acetic acid,

Compound No. 1161: (6-1{[4-(1-butylcyclopropyl)benzyl](pyridin-4-ylsulfonyl)aminomethyl}pyridin-2-ylamino)acetic acid,

Compound No. 1227: (6-{[4-(1-nonylcyclopropyl)benzyl](pyridin-3-ylsulfonyl)aminomethyl}pyridin-2-ylamino)acetic acid,

Compound No. 1248: (6-{[4-(1-methoxycyclopropyl)benzyl](pyridin-2-ylsulfonyl)-aminomethyl}pyridin-2-ylamino)acetic acid,

Compound No. 1277: (6-{[4-(1-benzylcyclopropyl)benzyl](pyridin-2-ylsulfonyl)aminomethyl}pyridin-2-ylamino)acetic acid,

Compound No. 1319: (6-{[4-(1-propoxymethylcyclopropyl)benzyl](pyridin-2-yl-sulfonyl)aminomethyl}pyridin-2-ylamino)acetic acid,

Compound No. 1364(6-{[4-(1-benzyloxymethylcyclopropyl)benzyl](pyridin-2-yl-sulfonyl)aminomethyl}pyridin-2-ylamino)acetic acid,
Compound No. 1398: (6-{[4-(1-ethylcyclobutyl)benzyl](pyridin-3-ylsulfonyl)aminomethyl}pyridin-2-ylamino)acetic acid,
Compound No. 1419: (6-{[4-(1-butylcyclobutyl)benzyl](pyridin-2-ylsulfonyl)aminomethyl}pyridin-2-ylamino)acetic acid, or
Compound No. 1444: (6-{(5-methylfuran-2-ylsulfonyl)[4-(1,1-dimethylpentyl)benzyl]-aminomethyl}pyridin-2-ylamino)acetic acid.

The compound represented by the formula (I) of the present invention can be prepared according to the following methods. Incidentally, specific preparation methods of the respective compounds of the present invention are explained in detail in the following mentioned Examples.

[Preparation method 1]

"Preparation method 1" is a method for preparing Compound (Ia) of the present invention wherein $R^1$ in the formula (I) is a hydrogen atom and Compound (Ib) of the present invention wherein $R^1$ in the formula (I) is a $C_1$-$C_6$ alkyl group.

[Formula 19]

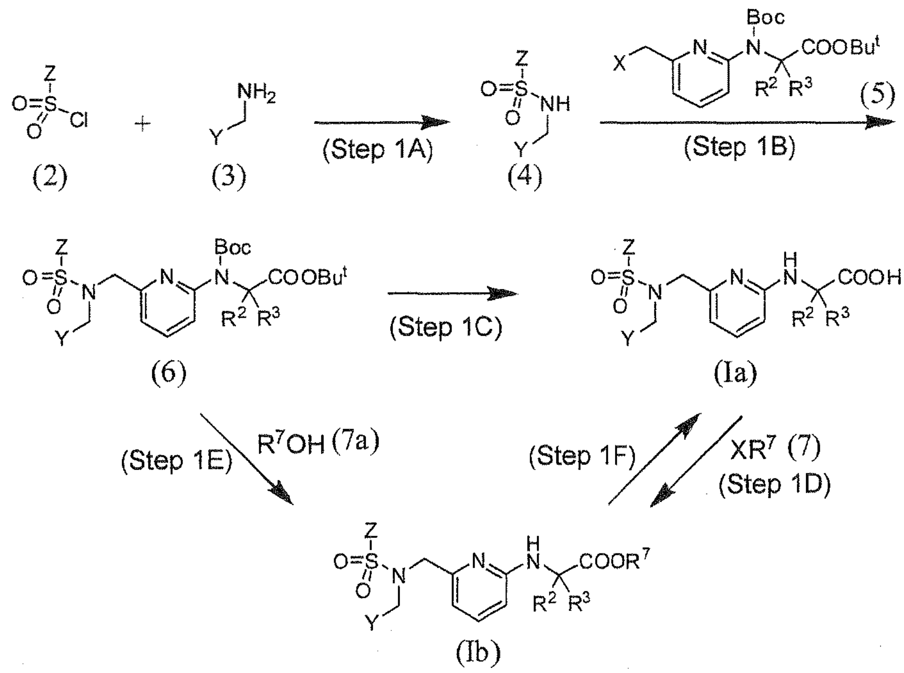


[wherein $R^2$, $R^3$, Y and Z have the same meanings as defined above, $R^7$ represents a $C_1$-$C_6$ alkyl group having the same meanings as mentioned above, X represents a hydroxy group, a chlorine atom, a bromine atom, an iodine atom, a methanesulfonyloxy group, a benzenesulfonyloxy group, a p-toluenesulfonyloxy group or a trifluoromethanesulfonyloxy group, Boc represents a tert-butoxycarbonyl group, and $Bu^t$ represents a tert-butyl group.]

"Step 1A" is a step for preparing Compound (4) by reacting Compound (2) and Compound (3) in the presence or in the absence of a base (preferably in the persence of) in an inert solvent.
Compound (2) and Compound (3) are known, or can be prepared according to the known method from the known compound(s).
The inert solvent to be used is not particularly limited so long as it does not inhibit the reaction and dissolves the starting materials with a certain extent, and there may be mentioned, for example, aromatic hydrocarbons such as benzene, toluene and xylene, etc.; halogenated aliphatic hydrocarbons such as methylene chloride, chloroform and 1,2-dichloroethane, etc.; ethers such as 1,4-dioxane, tetrahydrofuran, diethyl ether and 1,2-dimethoxyethane, etc.; amides such as N,N-dimethylformamide, N,N-dimethylacetamide and N-methylpyrrolidone, etc.; nitriles such as acetonitrile and propionitrile, etc.; or a mixed solvent of optional combination thereof, etc., preferably methylene chloride, 1,2-dichloroethane, N,N-dimethylformamide, acetonitrile or a mixed solvent thereof.

As the base to be used, there may be mentioned, for example, organic bases such as triethylamine and diisopropylethylamine, etc.; or inorganic bases such as sodium hydrogen carbonate, potassium hydrogen carbonate, sodium carbonate and potassium carbonate, etc., preferably triethylamine or diisopropylethylamine. An amount of the base to be used is generally 0.9 to 20-fold mol amount, preferably 1 to 10-fold mol amount based on 1 mol of Compound (2).
An amount of Compound (3) to be used is generally 0.7 to 5-fold mol amount, preferably 0.8 to 1.5-fold mol amount based on 1 mol of Compound (2).
The reaction temperature may vary depending on a kind or amount(s), etc., of the starting materials, solvent(s), etc., and is generally -20°C to 100°C, preferably -5°C to 50°C.
The reaction time may vary depending on a reaction temperature, etc., and is generally 1 minute to 36 hours, preferably 1 hour to 18 hours.

"Step 1B" is a step of preparing Compound (6) by reacting Compound (4) and Compound (5).
Compound (5) can be prepared by the following mentioned "Preparation method 6" or "Preparation method 7".

In "Step 1B", when the group X of Compound (5) is a hydroxy group, it is the so-called Mitsunobu reaction, and the reaction can be carried out in the precense of a phosphine compound and an azo compound in an inert solvent.
The inert solvent to be used is not particularly limited so long as it does not inhibit the reaction and dissolves the starting materials with a certain extent, and there may be mentioned, for example, aromatic hydrocarbons such as benzene, toluene and xylene, etc.; ethers such as diethyl ether, tetrahydrofuran, 1,4-dioxane and 1,2-dimethoxyethane, etc.; amides such as N,N-dimethylformamide, N,N-dimethylacetamide and N-methylpyrrolidone, etc.; nitriles such as acetonitrile and propionitrile, etc.; esters such as methyl acetate, ethyl acetate and isopropyl acetate, etc.; or a mixed solvent of optional combination thereof, etc., preferably tetrahydrofuran, N,N-dimethylformamide, acetonitrile or a mixed solvent thereof.
The phosphine compound to be used may be mentioned, for example, trimethylphosphine, triethylphosphine, tri-n-butylphosphine or triphenylphosphine, etc., preferably tri-n-butylphosphine or triphenylphosphine. An amount of the phosphine compound to be used is generally 0.9 to 10-fold mol amount, preferably 1 to 5-fold mol amount based on 1 mol of Compound (4).
The azo compound to be used may be mentioned, for example, diethylazodicarboxylate (DEAD), diisopropylazodicarboxylate (DIAD), N,N,N',N'-tetraisopropylazodicarboxamide (TIPA), 1,1'-(azodicarbonyl)dipiperidine (ADDP), N,N,N',N'-tetramethylazodicarboxamide (TMAD) or 1,6-dimethyl-1,5,7-hexahydro-1,4,6,7-tetrazocin-2,5-dione (DHTD), etc., preferably diethylazodicarboxylate (DEAD) or N,N,N',N'-tetramethylazodicarboxamide (TMAD). An amount of the azo compound to be used is generally 0.9 to 10-fold mol amount, preferably 1 to 5-fold mol amount based on 1 mol of Compound (4).
An amount of Compound (5) to be used is generally 0.8 to 2-fold mol amount, preferably 0.9 to 1.5-fold mol amount based on 1 mol of Compound (4).
The reaction temperature may vary depending on a kind or amount(s), etc., of the starting materials, solvent(s), etc., and is generally -20°C to 100°C, preferably -5°C to 50°C.
The reaction time may vary depending on a reaction temperature, etc., and is generally 30 minutes to 48 hours, preferably 1 hour to 24 hours.

In "Step 1B", when the group X of Compound (5) is a chlorine atom, a bromine atom, an iodine atom, a methanesulfonyloxy group, a benzenesulfonyloxy group, a p-toluenesulfonyloxy group or a trifluoromethanesulfonyloxy group, the reaction can be carried out in the precense of a base in an inert solvent.
The inert solvent to be used is not particularly limited so long as it does not inhibit the reaction and dissolves the starting materials with a certain extent, and there may be mentioned, for example, ethers such as tetrahydrofuran, 1,4-dioxane and 1,2-dimethoxyethane, etc.; halogenated aliphatic hydrocarbons such as methylene chloride, chloroform and 1,2-dichloroethane, etc.; nitriles such as acetonitrile and propionitrile, etc.; esters such as methyl formate, ethyl formate, methyl acetate and ethyl acetate, etc.; aromatic hydrocarbons such as benzene and toluene, etc.; amides such as N,N-dimethylformamide, N,N-dimethylacetamide and N-methylpyrrolidone, etc.; sulfoxides such as dimethylsulfoxide, etc.; or a mixed solvent of optional combination thereof, etc., preferably tetrahydrofuran, N,N-dimethylformamide, methylene chloride or 1,2-dichloroethane.
As the base to be used, there may be mentioned, for example, alkali metal hydrides such as sodium hydride and potassium hydride, etc.; alkali metal amides such as lithium amide, sodium amide, lithium diisopropylamide and lithium bistrimethylsilyl amide, etc.; alkali metal alkoxides such as sodium methoxide, sodium ethoxide, sodium tert-butoxide and potassium tert-butoxide, etc.; alkali metal carbonates such as sodium carbonate and potassium carbonate, etc.; and amines such as triethylamine, tributylamine, diisopropylethylamine, pyridine, picoline, 2,6-lutidine or 4-dimethylaminopyridine, etc., preferably sodium hydride, potassium carbonate, triethylamine or diisopropylethylamine. However, when the inert solvent to be used is esters, nitriles or halogenated aliphatic hydrocarbons, the base is preferably triethylamine or diisopropylethylamine. An amount of the base to be used is generally 1 to 5-fold mol amount, preferably 1 to 2.5-fold mol amount based on 1 mol of Compound (4).
An amount of Compound (5) to be used is generally 0.5 to 3-fold mol amount, preferably 0.5 to 1.5-fold mol amount

based on 1 mol of Compound (4).
The reaction temperature may vary depending on a kind or amount(s), etc., of the starting materials, solvent(s), etc., and is generally -80°C to 100°C, preferably 0°C to 80°C.
The reaction time may vary depending on a reaction temperature, etc., and is generally 10 minutes to 48 hours, preferably 1 hour to 24 hours.

"Step 1C" is a step of preparing Compound (Ia) of the present invention by simultaneously removing the Boc group and the $Bu^t$ group of Compound (6). This step can be carried out by referring to published material (see T.W.Greene & P.G.M. Wuts, Protective Groups in Organic Synthesis 4th Ed., John Wiley & Sons, Inc., pp.582 and 725), for example, it can be carried out by treating Compound (6) with an acid in an inert solvent, but it is not limited by these.
The inert solvent to be used is not particularly limited so long as it does not inhibit the reaction and dissolves the starting materials with a certain extent, and there may be mentioned, for example, ethers such as tetrahydrofuran, 1,4-dioxane and dimethoxyethane, etc.; halogenated aliphatic hydrocarbons such as methylene chloride, chloroform and 1,2-dichloroethane, etc.; water; or a mixed solvent of optional combination thereof, etc., preferably tetrahydrofuran, 1,4-dioxane, methylene chloride, water or a mixed solvent thereof.
The acid to be used may be mentioned, for example, hydrogen chloride, hydrochloric acid, hydrobromic acid, hydroiodic acid, sulfuric acid, phosphoric acid, p-toluenesulfonic acid, methanesulfonic acid or trifluoroacetic acid, etc., preferably hydrogen chloride, hydrochloric acid or trifluoroacetic acid. An amount of the acid to be used is generally 1 to 200-fold mol amount, preferably 5 to 100-fold mol amount based on 1 mol of Compound (6), and it may be used markedly excessively as a solvent.
To promote the reaction anisole compounds such as anisole or thioanisole, etc., may be added. An amount of the anisole compound to be used is generally 1 to 200-fold mol amount, preferably 5 to 100-fold mol amount based on 1 mol of Compound (6).
The reaction temperature may vary depending on a kind or amount(s), etc., of the starting materials, solvent(s), etc., and is generally 0°C to 150°C, preferably 5°C to 100°C.
The reaction time may vary depending on a reaction temperature, etc., and is generally 10 minutes to 48 hours, preferably 1 hour to 24 hours.

"Step 1D" is a step for preparing Compound (Ib) of the present invention by reacting Compound (Ia) and Compound (7).
Compound (7) are known, or can be prepared according to the known method from the known compound(s).

In "Step 1D", when the group X of Compound (7) is a hydroxy group, it can be carried out by referring to a published article (see T.W.Greene & P.G.M.Wuts, Protective Groups in Organic Synthesis 4th Ed., John Wiley & Sons, Inc., p.538). For example, it can be carried out by reacting with Compound (7) in the presence of an acid, or after activating the carboxy group of Compound (Ia), but the reaction is not limited by these methods.

In "Step 1D", when the group X of Compound (7) is a hydroxy group, and the present step is carried out in the presence of an acid, the reaction is carried out in an inert solvent or in the absence of a solvent.
The inert solvent to be used is not particularly limited so long as it does not inhibit the reaction and dissolves the starting materials with a certain extent, and there may be mentioned, for example, aromatic hydrocarbons such as benzene, toluene and xylene, etc.; ethers such as tetrahydrofuran, 1,4-dioxane and dimethoxyethane, etc.; halogenated aliphatic hydrocarbons such as methylene chloride, chloroform and 1,2-dichloroethane, etc.; or a mixed solvent of optional combination thereof, etc., preferably 1,4-dioxane, methylene chloride, 1,2-dichloroethane or a mixed solvent thereof.
The acid to be used may be mentioned, for example, hydrogen chloride, sulfuric acid, methanesulfonic acid, p-toluenesulfonic acid or trifluoroacetic acid, etc., preferably hydrogen chloride, sulfuric acid or p-toluenesulfonic acid. An amount of the acid to be used is generally 1 to 200-fold mol amount, preferably 5 to 100-fold mol amount based on 1 mol of Compound (Ia).
An amount of Compound (7) to be used is generally 1 to 100-fold mol amount, preferably 1 to 5-fold mol amount based on 1 mol of Compound (Ia), and it may be used markedly excessively as a solvent.
The reaction temperature may vary depending on a kind or amount(s), etc., of the starting materials, solvent(s), etc., and is generally -20°C to 150°C, preferably -5°C to 100°C.
The reaction time may vary depending on a reaction temperature, etc., and is generally 1 minute to 72 hours, preferably 1 hour to 48 hours.

In "Step 1D", when the group X of Compound (7) is a hydroxy group, and the present step is carried out by activating the carboxy group of Compound (Ia), it is carried out by changing the carboxy group to "an active material of the carboxy group" such as an acid chloride, a mixed acid anhydride and an imidazolide, etc., by using an activating agent in an inert solvent or in the absence of a solvent, and then, reacting it with Compound (7) in the presence or in the absence of a base (preferably in the persence of). The "active material of the carboxy group" obtained by the reaction can be used for the reaction with Compound (7) without isolation.
The inert solvent to be used is not particularly limited so long as it does not inhibit the reaction and dissolves the starting materials with a certain extent, and there may be mentioned, for example, aromatic hydrocarbons such as benzene,

toluene and xylene, etc.; halogenated aliphatic hydrocarbons such as methylene chloride, chloroform and 1,2-dichloroethane, etc.; ethers such as tetrahydrofuran, 1,2-dimethoxyethane and 1,4-dioxane, etc.; nitriles such as acetonitrile and propionitrile, etc.; amides such as N,N-dimethylformamide, N,N-dimethylacetamide and N-methylpyrrolidone, etc.; or a mixed solvent of optional combination thereof, etc., preferably methylene chloride, tetrahydrofuran or acetonitrile.
The activating agent of the carboxy group may be mentioned, for example, chlorides such as thionyl chloride, oxalyl chloride, phosphorus oxychloride and phosphorus pentachloride, etc.; 1-ethyl-3-(3-dimethylaminopropyl)carbodiimide (hereinafter abbreviated to as EDC); carbonyldiimidazolide (hereinafter abbreviated to as CDI); or chloroformates such as chloromethyl formate and chloroethyl formate, etc., preferably thionyl chloride, EDC or CDI. An amount of the activating agent to be used is generally 1 to 5-fold mol amount, preferably 1 to 2-fold mol amount based on 1 mol of Compound (Ia).
As the base to be used, there may be mentioned, for example, organic bases such as triethylamine, diisopropylethylamine and N,N-dimethylaminopyridine, etc.; or inorganic bases such as sodium hydrogen carbonate, potassium hydrogen carbonate, sodium carbonate and potassium carbonate, etc., preferably triethylamine, diisopropylethylamine or N,N-dimethylaminopyridine. An amount of the base to be used is generally 1 to 100-fold mol amount, preferably 1 to 10-fold mol amount based on 1 mol of Compound (Ia).
An amount of Compound (7) to be used is generally 1 to 100-fold mol amount, preferably 1 to 5-fold mol amount based on 1 mol of Compound (Ia).
The reaction temperature may vary depending on a kind or amount(s), etc., of the starting materials, solvent(s), etc., and is generally -20°C to 150°C, preferably -5°C to 100°C.
The reaction time may vary depending on a reaction temperature, etc., and is generally 1 minute to 24 hours, preferably 1 hour to 12 hours.

In "Step 1D", when the group X of Compound (7) is a chlorine atom, a bromine atom, an iodine atom, a methanesulfonyloxy group, a benzenesulfonyloxy group, a p-toluenesulfonyloxy group or a trifluoromethanesulfonyloxy group, it is carried out in the presence of a base in an inert solvent.
The inert solvent to be used is not particularly limited so long as it does not inhibit the reaction and dissolves the starting materials with a certain extent, and there may be mentioned, for example, aromatic hydrocarbons such as benzene, toluene and xylene, etc.; halogenated aliphatic hydrocarbons such as methylene chloride, chloroform and 1,2-dichloroethane, etc.; ethers such as diethyl ether, tetrahydrofuran, 1,4-dioxane and 1,2-dimethoxyethane, etc.; ketones such as acetone, methyl ethyl ketone, methyl isobutyl ketone and methyltert-butyl ketone, etc.; amides such as N,N-dimethylformamide, N,N-dimethylacetamide and N-methylpyrrolidone, etc.; nitriles such as acetonitrile and propionitrile, etc.; or a mixed solvent of optional combination thereof, etc., preferably methylene chloride, 1,2-dichloroethane, acetone, N,N-dimethylformamide, acetonitrile or a mixed solvent thereof.
As the base to be used, there may be mentioned, for example, organic bases such as triethylamine, diisopropylethylamine, pyridine, N,N-dimethylaminopyridine and picoline, etc.; or inorganic bases such as sodium hydrogen carbonate, potassium hydrogen carbonate, sodium carbonate and potassium carbonate, etc., preferably triethylamine, diisopropylethylamine or potassium carbonate. An amount of the base to be used is generally 1 to 100-fold mol amount, preferably 1 to 10-fold mol amount based on 1 mol of Compound (Ia).
An amount of Compound (7) to be used is generally 0.9 to 10-fold mol amount, preferably 1 to 2-fold mol amount based on 1 mol of Compound (Ia).
The reaction temperature may vary depending on a kind or amount(s), etc., of the starting materials, solvent(s), etc., and is generally -20°C to 100°C, preferably -5°C to 60°C.
The reaction time may vary depending on a reaction temperature, etc., and is generally 1 minute to 24 hours, preferably 1 hour to 12 hours.

"Step 1E" is a step of preparing Compound (Ib) by reacting Compound (6) and Compound (7a) in the presence of an acid, in an inert solvent or in the absence of a solvent.
Compound (7a) are known, or can be prepared according to the known method from the known compound(s).
The inert solvent to be used is not particularly limited so long as it does not inhibit the reaction and dissolves the starting materials with a certain extent, and there may be mentioned, for example, aromatic hydrocarbons such as benzene, toluene and xylene, etc.; halogenated aliphatic hydrocarbons such as methylene chloride, chloroform and 1,2-dichloroethane, etc.; ethers such as tetrahydrofuran, 1,4-dioxane and 1,2-dimethoxyethane, etc.; or a mixed solvent of optional combination thereof, etc., preferably methylene chloride, 1,2-dichloroethane, 1,4-dioxaneor a mixed solvent thereof.
An amount of Compound (7a) to be used is generally 1 to 1000-fold mol amount, preferably 10 to 100-fold mol amount based on 1 mol of Compound (6), and it may be used markedly excessively as a solvent.
The acid to be used may be mentioned, for example, hydrogen chloride, hydrochloric acid, sulfuric acid, methanesulfonic acid, p-toluenesulfonic acid or trifluoroacetic acid, etc., preferably hydrogen chloride, sulfuric acid, or p-toluenesulfonic acid. An amount of the acid to be used is generally 1 to 200-fold mol amount, preferably 1 to 100-fold mol amount based on 1 mol of Compound (6).
The reaction temperature may vary depending on a kind or amount(s), etc., of the starting materials, solvent(s), etc., and is generally -20°C to 150°C, preferably 0°C to 100°C.

The reaction time may vary depending on a reaction temperature, etc., and is generally 10 minutes to 48 hours, preferably 1 hour to 24 hours.

"Step 1F" is a step of preparing Compound (Ia) by hydrolyzing Compound (Ib). The present step is carried out under the acidic conditions or under the basic conditions.

When "Step 1F" is carried out under the acidic conditions, it can be carried out by treating Compound (Ib) with an acid in the presence of water in an orgnic solvent.

The solvent to be used is not particularly limited so long as it does not inhibit the reaction and dissolves the starting materials with a certain extent, and there may be mentioned,, for example, alcohols such as methanol, ethanol, propanol and isopropanol, etc.; ethers such as tetrahydrofuran, 1,4-dioxane and 1,2-dimethoxyethane, etc.; acetic acid; or a mixed solvent of optional combination thereof, etc., preferably methanol, ethanol, tetrahydrofuran, acetic acidor a mixed solvent thereof.

An amount of water to be used is generally 10 to 1000-fold mol amount based on 1 mol of Compound (Ib), and it may be used markedly excessively as a solvent.

The acid to be used may be mentioned, for example, inorganic acids such as hydrochloric acid, hydrobromic acid, hydroiodic acid, sulfuric acid and phosphoric acid, etc.; or sulfonic acids such as methanesulfonic acid, benzenesulfonic acid and p-toluenesulfonic acid, etc., preferably hydrochloric acid, hydrobromic acid or sulfuric acid. An amount of the acid to be used is generally 1 to 1000-fold mol amount, preferably 10 to 100-fold mol amount based on 1 mol of Compound (Ib).

The reaction temperature may vary depending on a kind or amount(s), etc., of the starting materials, solvent(s), etc., and is generally -5°C to 150°C, preferably 0°C to 100°C.

The reaction time may vary depending on a reaction temperature, etc., and is generally 10 minutes to 72 hours, preferably 30 minutes to 48 hours.

When "Step 1F" is carried out under the basic conditions, it can be carried out by treating Compound (Ib) with a base in the presence of water in an orgnic solvent.

The solvent to be used is not particularly limited so long as it does not inhibit the reaction and dissolves the starting materials with a certain extent, and there may be mentioned, for example, alcohols such as methanol, ethanol, propanol and isopropanol, etc.; ethers such as tetrahydrofuran, 1,4-dioxane and 1,2-dimethoxyethane, etc.; or a mixed solvent of optional combination thereof, etc., preferably methanol, ethanol, tetrahydrofuranor a mixed solvent thereof.

An amount of water to be used is generally 10 to 1000-fold mol amount based on 1 mol of Compound (Ib), and it may be used markedly excessively as a solvent.

As the base to be used, there may be mentioned, for example, alkali metal hydroxides such as lithium hydroxide, sodium hydroxide and potassium hydroxide, etc.; or alkali metal carbonates such as sodium carbonate and potassium carbonate, etc., preferably lithium hydroxide, sodium hydroxide or potassium hydroxide. An amount of the base to be used is generally 0.9 to 10-fold mol amount, preferably 1 to 5-fold mol amount based on 1 mol of Compound (Ib).

The reaction temperature may vary depending on a kind or amount(s), etc., of the starting materials, solvent(s), etc., and is generally 0°C to 150°C, preferably 0°C to 80°C.

The reaction time may vary depending on a reaction temperature, etc., and is generally 10 minutes to 72 hours, preferably 30 minutes to 48 hours.

[Preparation method 2]

"Preparation method 2" is another method for preparing the above-mentioned Compound (6).

[Formula 20]

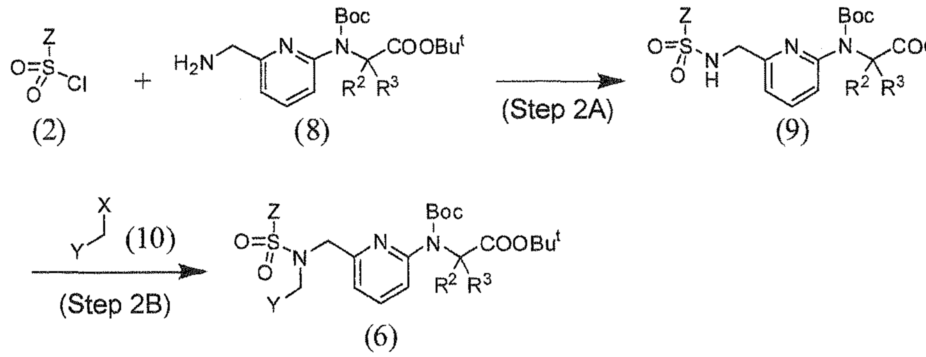


(2) (8) (9) (10) (6)

[wherein $R^2$, $R^3$, X, Y and Z have the same meanings as mentioned above.]

"Step 2A" is a step of preparing Compound (9) by reacting Compound (2) and Compound (8) in the presence or in the absence of a base (preferably in the persence of) in an inert solvent. The present step can be carried out in accordance with the above-mentioned "Step 1A" except for using Compound (8) in place of Compound (3). Compound (8) can be prepared by the following mentioned "Preparation method 8".

"Step 2B" is a step of preparing Compound (6) by reacting Compound (9) and Compound (10). The present step can be carried out in accordance with the above-mentioned "Step 1B" except for using Compound (9) in place of Compound (4), and Compound (10) in place of Compound (5), respectively. Compound (10) are known, or can be prepared according to the known method from the known compound(s).

[Preparation method 3]

"Preparation method 3" is another method for preparing Compound (Ib) of the present invention.

[Formula 21]

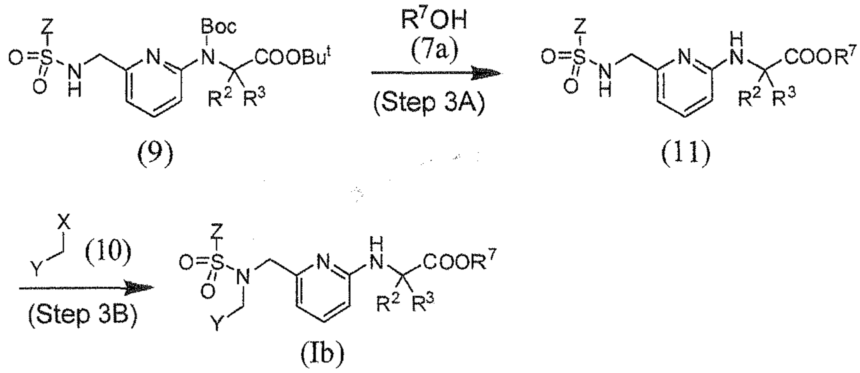


(9) (11) (10) (Ib)

[wherein $R^2$, $R^3$, $R^7$, X, Y and Z have the same meanings as mentioned above.]

"Step 3A" is a step of preparing Compound (11) by reacting Compound (9) and Compound (7a) in the presence of an acid in an inert solvent or in the absence of a solvent. The present step is carried out in accordance with the above-mentioned "Step 1E" except for using Compound (9) in place of Compound (6).

"Step 3B" is a step of preparing Compound (Ib) by reacting Compound (11) and Compound (10). The present step is carried out in accordance with the above-mentioned "Step 1B" except for using Compound (11) in place of Compound (4), and using Compound (10) in place of Compound (5), respectively.

[Preparation method 4]

"Preparation method 4" is another method for preparing the above-mentioned Compound (6).

[Formula 22]

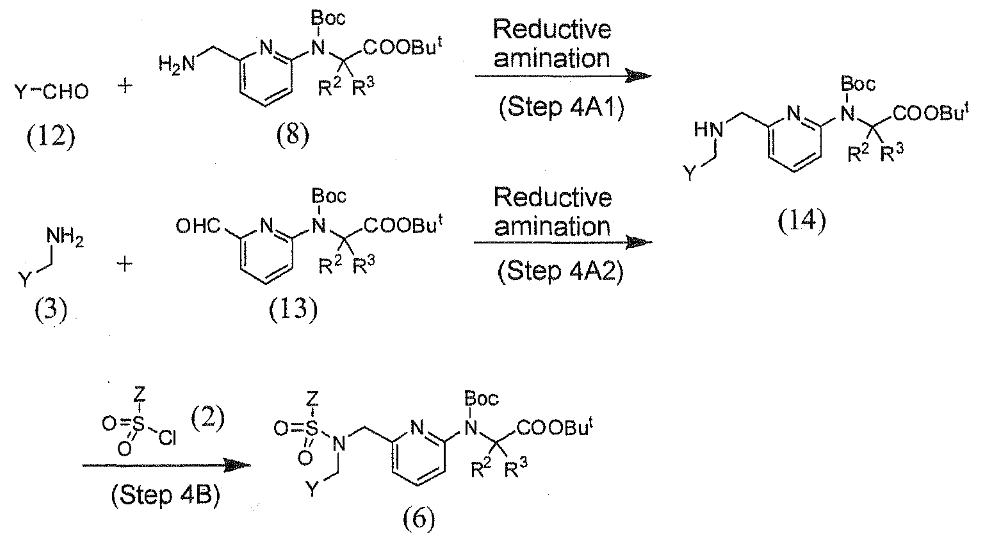


(12) (8) (3) (13) (14) (2) (6)

[wherein $R^2$, $R^3$, Y and Z have the same meanings as mentioned above.]

"Step 4A1" is a step of preparing Compound (14) by reacting Compound (12) and Compound (8) in the presence of or in the absence of a dehydrating agent in an inert solvent to prepare an imine material, then reducing the same by using a boron hydride compound.

Compound (12) are known, or can be prepared according to the known method from the known compound(s).

The inert solvent to be used is not particularly limited so long as it does not inhibit the reaction and dissolves the starting materials with a certain extent, and there may be mentioned, for example, halogenated aliphatic saturated hydrocarbons such as methylene chloride, chloroform and 1,2-dichloroethane, etc.; aromatic hydrocarbons such as benzene and toluene, etc.; or alcohols such as methanol, ethanol and propanol, etc., preferably methylene chloride, 1,2-dichloroethane, methanol or ethanol.

The dehydrating agent to be used may be mentioned, for example, Molecular Sieve or anhydrous magnesium sulfate, etc. An amount of the dehydrating agent to be used is generally 100 g to 2000 g, preferably 500 g to 1000 g based on 1 mol of Compound (8).

An amount of Compound (12) to be used is generally 0.3 to 10-fold mol amount, preferably 0.4 to 1.5-fold mol amount based on 1 mol of Compound (8). When Compound (8) is an acid addition salt (for example, hydrochloride or hydrobromide, etc.), a base may be added. In such a case, as the base to be used, there may be mentioned, for example, triethylamine or diisopropylethylamine, etc. An amount of the base to be used is generally 1 to 10-fold mol amount, preferably 1 to 3-fold mol amount based on 1 mol of Compound (8).

The reaction temperature may vary depending on a kind or amount(s), etc., of the starting materials, solvent(s), etc., and is generally -20°C to 100°C, preferably 0°C to 50°C.

The reaction time may vary depending on a reaction temperature, etc., and is generally 10 minutes to 24 hours, preferably 1 hour to 12 hours.

The obtained imine material is isolated or without isolation and subsequently reduced by using a boron hydride compound. The boron hydride compound to be used may be mentioned, for example, potassium borohydride, potassium cyanoborohydride or triacetoxy potassium borohydride, etc., preferably potassium borohydride or triacetoxy potassium borohydride. An amount of the boron hydride compound to be used is generally 1 to 10-fold mol amount, preferably 1 to 3-fold mol amount based on 1 mol of Compound (8).

When the obtained imine material is isolated, the inert solvent to be used in the reduction is not particularly limited so long as it does not inhibit the reaction and dissolves the starting materials with a certain extent, and there may be mentioned, for example, halogenated aliphatic hydrocarbons such as methylene chloride, chloroform and 1,2-dichloroethane, etc.; aromatic hydrocarbons such as benzene and toluene, etc.; or alcohols such as methanol, ethanol and propanol, etc., preferably methylene chloride, 1,2-dichloroethane, methanol or ethanol.

The reaction temperature may vary depending on a kind or amount(s), etc., of the starting materials, solvent(s), etc., and is generally -20°C to 100°C, preferably 0°C to 50°C.

The reaction time may vary depending on a reaction temperature, etc., and is generally 10 minutes to 12 hours, preferably 1 hour to 6 hours.

"Step 4A2" is a step of preparing Compound (14) by reacting Compound (3) and Compound (13) in the presence of or in the absence of a dehydrating agent in an inert solvent to prepare an imine material, then reducing the same by using a boron hydride compound. The present step is carried out in accordance with the above-mentioned "Step 4A1" except for using Compound (13) in place of Compound (12), and using Compound (3) in place of Compound (8), respectively.
Compound (13) can be prepared by the following mentioned "Step 8A".
"Step 4B" is a step of preparing Compound (6) by reacting Compound (14) and Compound (2) in the presence or in the absence of a base (preferably in the persence of) in an inert solvent.
The present step is carried out in accordance with the above-mentioned "Step 1A" except for using Compound (14) in place of Compound Compound (3).

[Preparation method 5]

"Preparation method 5" is another method for preparing the above-mentioned Compound (6).
[Formula 23]

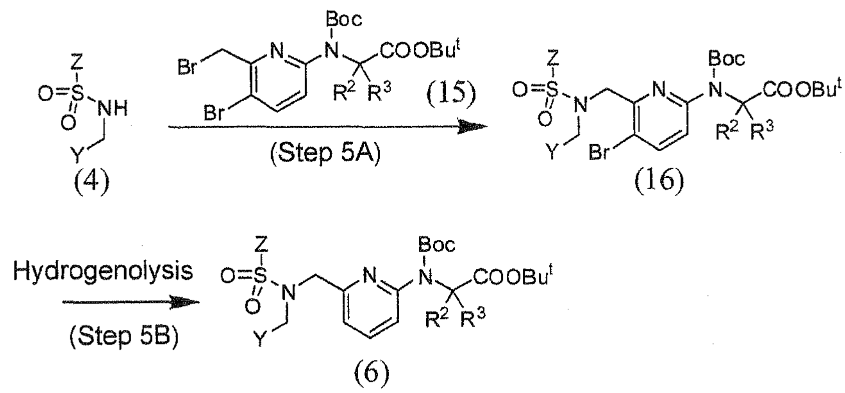


[wherein $R^2$, $R^3$, Y and Z have the same meanings as mentioned above.]
"Step 5A" is a step of preparing Compound (16) by reacting Compound (4) and Compound (15). The present step is carried out in accordance with the above-mentioned "Step 1B" where X is a bromine atom except for using Compound (15) in place of Compound (5).
Compound (15) can be prepared by the following mentioned "Preparation method 9".
"Step 5B" is a step of preparing Compound (6) by reacting Compound (16) with hydrogen in the presence or in the absence of a base (preferably in the persence of) a catalyst in an inert solvent.
The inert solvent to be used is not particularly limited so long as it does not inhibit the reaction and dissolves the starting materials with a certain extent, and there may be mentioned, for example, alcohols such as methanol, ethanol, propanol and isopropanol, etc.; ethers such as tetrahydrofuran, 1,4-dioxane and 1,2-dimethoxyethane, etc.; halogenated aliphatic hydrocarbons such as methylene chloride, chloroform and 1,2-dichloroethane, etc.; esters such as methyl formate, ethyl formate, methyl acetate and ethyl acetate, etc.; aromatic hydrocarbons such as benzene and toluene, etc.; water; or a mixed solvent of optional combination thereof, etc., preferably methanol or ethanol.
As the base to be used, there may be mentioned, for example, organic bases such as triethylamine, diisopropylethylamine, pyridine, N,N-dimethylaminopyridine, picoline and 2,6-lutidine, etc.; or inorganic bases such as sodium hydrogen carbonate, potassium hydrogen carbonate, sodium carbonate and potassium carbonate, etc., preferably triethylamine or diisopropylethylamine. An amount of the base to be used is generally 1 to 100-fold mol amount, preferably 1 to 10-fold mol amount based on 1 mol of Compound (16).
The catalyst to be used may be mentioned, for example, palladium-active carbon, platinum-active carbon, platinum black, rhodium-active carbon or Raney nickel, etc., preferably palladium-active carbon, platinum black or Raney nickel. An amount of the catalyst to be used is generally 0.0005 to 1-fold mol amount, preferably 0.01 to 0.3-fold mol amount based on 1 mol of Compound (16).
A hydrogen partial pressure is generally I atm to 10 atm, preferably 1 atm to 5 atm.
The reaction temperature may vary depending on a kind or amount(s), etc., of the starting materials, solvent(s), etc., and is generally 0°C to 100°C, preferably 15°C to 80°C.
The reaction time may vary depending on a reaction temperature, etc., and is generally 15 minutes to 72 hours, preferably

30 minutes to 24 hours.

[Preparation method 6]

"Preparation method 6" is a general method of preparing the above-mentioned Compound (5). In the pesent preparation method, Compound (5a) which is a compound wherein X is a hydroxy group in Compound (5), then, Compound (5b) which is a compound wherein X is a chlorine atom, a bromine atom, an iodine atom, a methanesulfonyloxy group, a benzenesulfonyloxy group, a p-toluenesulfonyloxy group or a trifluoromethanesulfonyloxy group in Compound (5) are successively prepared.

[Formula 24]

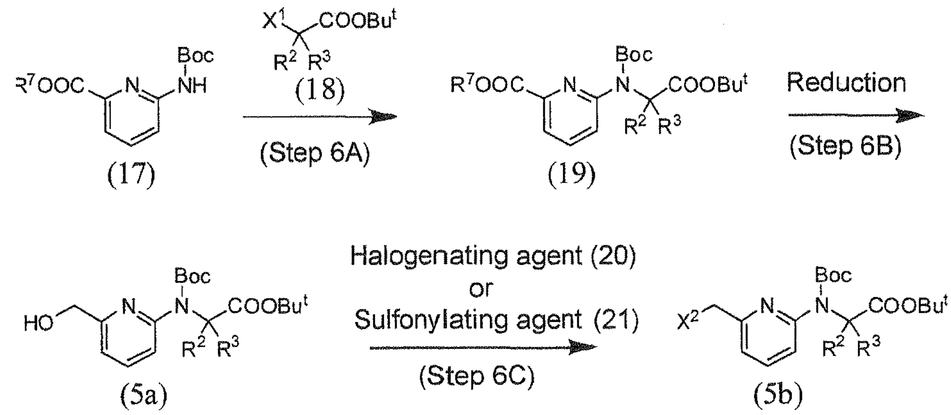


(17) (19) (5a) (5b)

[wherein $R^2$, $R^3$ and $R^7$ have the same meanings as defined above, $X^1$ represents a chlorine atom, a bromine atom or an iodine atom, $X^2$ represents a chlorine atom, a bromine atom, an iodine atom, a methanesulfonyloxy group, a benzenesulfonyloxy group, a p-toluenesulfonyloxy group or a trifluoromethanesulfonyloxy group.]

"Step 6A" is a step of preparing Compound (19) by reacting Compound (17) and Compound (18) in the presence of a base in an inert solvent. The present step is carried out in accordance with the above-mentioned "Step 1B" except for using Compound (17) in place of Compound (4), and using Compound (18) in place of Compound Compound (5), respectively.

Compound (17) and Compound (18) are known, or can be prepared according to the known method from the known compound(s).

"Step 6B" is a step of preparing Compound (5a) by reducing Compound (19) using potassium borohydride in the presence of or in the absence of (preferably in the persence of) calcium chloride in an inert solvent.

The inert solvent to be used is not particularly limited so long as it does not inhibit the reaction and dissolves the starting materials with a certain extent, and there may be mentioned, for example, alcohols such as methanol, ethanol, propanol, isopropanol, butanol, isobutanol, sec-butanol and tert-butanol, etc.; ethers such as tetrahydrofuran, 1,4-dioxane, 1,2-dimethoxyethane, diethylene glycol dimethyl ether, triethylene glycol dimethyl ether and tetraethylene glycol dimethyl ether, etc.; nitriles such as acetonitrile and propionitrile, etc.; amides such as N,N-dimethylformamide, N,N-dimethylacetamide and N-methylpyrrolidone, etc.; sulfoxides such as dimethylsulfoxide, etc.; or a mixed solvent of optional combination thereof, etc., preferably methanol, ethanol, tetrahydrofuran, triethylene glycol dimethyl ether, tetraethylene glycol dimethyl etheror a mixed solvent thereof.

An amount of the calcium chloride to be used is generally 0.5 to 10-fold mol amount, preferably 1 to 3-fold mol amount based on 1 mol of Compound (19).

An amount of the potassium borohydride to be used is generally 0.5 to 10-fold mol amount, preferably 1 to 3-fold mol amount based on 1 mol of Compound (19).

The reaction temperature may vary depending on a kind or amount(s), etc., of the starting materials, solvent(s), etc., and is generally -20°C to 100°C, preferably 0°C to 50°C.

The reaction time may vary depending on a reaction temperature, etc., and is generally 1 minute to 12 hours, preferably 15 minutes to 6 hours.

"Step 6C" is a step of preparing Compound (5b) by reacting Compound (5a) and a halogenating agent (20) or a sulfonylating agent (21). In the present step, when the halogenating agent (20) is used, a compound wherein $X^2$ is a

chlorine atom, a bromine atom or an iodine atom in the formula (5b) can be prepared, and when the sulfonylating agent (21) is used, a compound wherein $X^2$ is a methanesulfonyloxy group, a benzenesulfonyloxy group, a p-toluenesulfonyloxy group or a trifluoromethanesulfonyloxy group in the formula (5b) can be prepared.

In "Step 6C", when a halogenating agent (20) is used, it is necessary to select reaction conditions depending on the kind of the halogenating agent (20).

The halogenating agent (20) to be used may be mentioned, for example, thionyl chloride, oxalyl chloride, phosphorus oxychloride, phosphorus pentachloride, thionyl bromide, N-chlorosuccinimide (hereinafter abbreviated to as NCS), N-bromosuccinimide (hereinafter abbreviated to as NBS), N-iodosuccinimide (hereinafter abbreviated to as NIS), carbon tetrachloride, carbon tetrabromide or iodine, etc.

When thionyl chloride, oxalyl chloride, phosphorus oxychloride, phosphorus pentachloride or thionyl bromide is used as the halogenating agent (20), the reaction is carried out in an inert solvent.

The inert solvent to be used is not particularly limited so long as it does not inhibit the reaction and dissolves the starting materials with a certain extent, and there may be mentioned, for example, aromatic hydrocarbons such as benzene, toluene and xylene, etc.; halogenated aliphatic hydrocarbons such as methylene chloride, chloroform and 1,2-dichloroethane, etc.; ethers such as tetrahydrofuran, 1,2-dimethoxyethane and 1,4-dioxane, etc.; nitriles such as acetonitrile and propionitrile, etc.; amides such as N,N-dimethylformamide, N,N-dimethylacetamide and N-methylpyrrolidone, etc.; or a mixed solvent of optional combination thereof, etc., preferably toluene, methylene chloride, tetrahydrofuran or acetonitrile.

An amount of the halogenating agent (20) to be used is generally 0.9 to 10-fold mol amount, preferably 1 to 2-fold mol amount based on 1 mol of Compound (5a).

It may be added, for example, a base such as triethylamine, diisopropylethylamine, imidazole, pyridine and N,N-dimethylaminopyridine, etc., to promote the reaction. An amount of the base to be used is generally 1 to 10-fold mol amount, preferably 1 to 2-fold mol amount based on 1 mol of Compound (5a).

The reaction temperature may vary depending on a kind or amount(s), etc., of the starting materials, solvent(s), etc., and is generally -20°C to 150°C, preferably 0°C to 50°C.

The reaction time may vary depending on a reaction temperature, etc., and is generally 1 minute to 24 hours, preferably 1 hour to 12 hours.

When NCS, NBS, NIS, carbon tetrachloride, carbon tetrabromide or iodine is used as the halogenating agent (20), the reaction is carried out in the presence of a phosphine compound in an inert solvent.

The inert solvent to be used is not particularly limited so long as it does not inhibit the reaction and dissolves the starting materials with a certain extent, and there may be mentioned, for example, ethers such as tetrahydrofuran, 1,2-dimethoxyethane and 1,4-dioxane, etc.; nitriles such as acetonitrile and propionitrile, etc.; amides such as N,N-dimethylformamide, N,N-dimethylacetamide and N-methylpyrrolidone, etc.; or a mixed solvent of optional combination thereof, etc., preferably tetrahydrofuran or acetonitrile.

The phosphine compound to be used may be mentioned, for example, trimethylphosphine, triethylphosphine, tri-n-butylphosphine or triphenylphosphine, etc, preferablytriphenylphosphine. An amount of the phosphine compound to be used is generally 0.9 to 10-fold mol amount, preferably 1 to 2-fold mol amount based on 1 mol of Compound (5a).

An amount of the halogenating agent (20) to be used is generally 0.9 to 10-fold mol amount, preferably 1 to 2-fold mol amount based on 1 mol of Compound (5a).

When iodine is used as the halogenating agent, for example, a base such as imidazole, etc., may be added to promote the reaction. An amount of the base to be used is generally 1 to 10-fold mol amount, preferably 1 to 2-fold mol amount based on 1 mol of Compound (5a).

The reaction temperature may vary depending on a kind or amount(s), etc., of the starting materials, solvent(s), etc., and is generally 0°C to 100°C, preferably 0°C to 50°C.

The reaction time may vary depending on a reaction temperature, etc., and is generally 1 minute to 24 hours, preferably 1 hour to 12 hours.

In "Step 6C", when the sulfonylating agent (21) is used, the reaction is carried out in the presence of a base in an inert solvent.

The inert solvent to be used is not particularly limited so long as it does not inhibit the reaction and dissolves the starting materials with a certain extent, and there may be mentioned, for example, aromatic hydrocarbons such as benzene, toluene and xylene, etc.; halogenated aliphatic hydrocarbons such as methylene chloride, chloroform and 1,2-dichloroethane, etc.; ethers such as tetrahydrofuran, 1,2-dimethoxyethane, and 1,4-dioxane, etc.; nitriles such as acetonitrile and propionitrile, etc.; amides such as N,N-dimethylformamide, N,N-dimethylacetamide and N-methylpyrrolidone, etc.; or a mixed solvent of optional combination thereof, etc., preferably toluene, methylene chloride, tetrahydrofuran or acetonitrile.

The base to be used may be mentioned, for example, organic bases such as triethylamine, diisopropylethylamine, pyridine and N,N-dimethylaminopyridine, etc., preferably triethylamine, diisopropylethylamine or pyridine. An amount of the base to be used is generally 0.9 to 10-fold mol amount, preferably 1 to 1.5-fold mol amount based on 1 mol of

Compound (5a).
The sulfonylating agent (21) to be used may be mentioned methanesulfonyl chloride, benzenesulfonyl chloride, p-toluenesulfonyl chloride or trifluoromethanesulfonic acid anhydride, etc. An amount of the sulfonylating agent (21) to be used is generally 0.9 to 10-fold mol amount, preferably 1 to 2-fold mol amount based on 1 mol of Compound (5a).
The reaction temperature may vary depending on a kind or amount(s), etc., of the starting materials, solvent(s), etc., and is generally -20°C to 130°C, preferably -20°C to 50°C.
The reaction time may vary depending on a reaction temperature, etc., and is generally 1 minute to 24 hours, preferably 1 hour to 12 hours.

[Preparation method 7]

"Preparation method 7" is another method for preparing the above-mentioned Compound (5a).
[Formula 25]

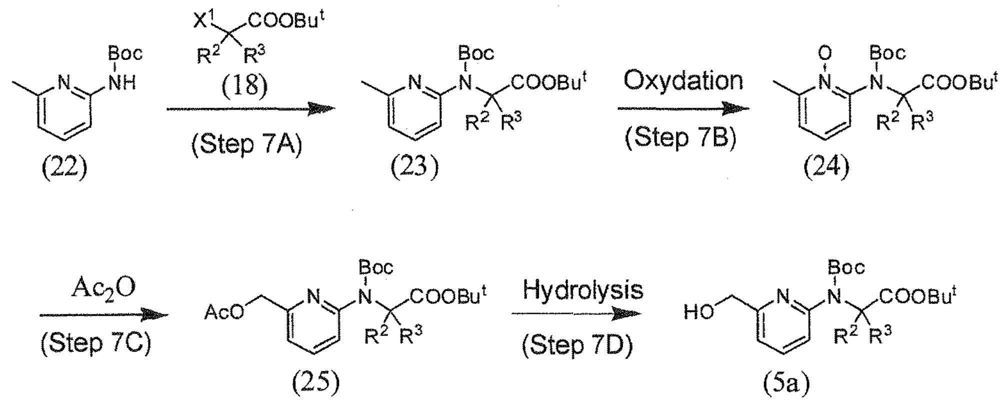


(22) (23) (24) (25) (5a)

[wherein $R^2$, $R^3$ and $X^1$ have the same meanings as defined above, and Ac represents an acetyl group.]
"Step 7A" is a step of preparing Compound (23) by reacting Compound (22) and Compound (18) in the presence of a base in an inert solvent. The present step is carried out in accordance with the above-mentioned "Step 6A",except for using Compound (22) in place of Compound (17).
"Step 7B" is a step of preparing Compound (24) by oxidating Compound (23) using an oxidizing agent in an inert solvent.
The inert solvent to be used is not particularly limited so long as it does not inhibit the reaction and dissolves the starting materials with a certain extent, and there may be mentioned, for example, halogenated aliphatic hydrocarbons such as methylene chloride, chloroform and 1,2-dichloroethane, etc., preferablymethylene chloride.
The oxidizing agent to be used may be mentioned, for example, an oxidizing agent such as m-chloroperbenzoic acid and hydrogen peroxide, etc., preferably m-chloroperbenzoic acid. An amount of the oxidizing agent to be used is generally 1 to 10-fold mol amount, preferably 1 to 3-fold mol amount based on 1 mol of Compound (23).
The reaction temperature may vary depending on a kind or amount(s), etc., of the starting materials, solvent(s), etc., and is generally 0°C to 100°C, preferably 10°C to 50°C.
The reaction time may vary depending on a reaction temperature, etc., and is generally 30 minutes to 24 hours, preferably 1 hour to 6 hours.
"Step 7C" is a step of preparing Compound (25) by rearrangement reaction of Compound (24) in acetic acid anhydride.
An amount of the acetic acid anhydride to be used is generally 1 to 100-fold mol amount, preferably 5 to 30-fold mol amount based on 1 mol of Compound (24), and it may be used markedly excessively as a solvent.
The reaction temperature may vary depending on a kind or amount(s), etc., of the starting materials, solvent(s), etc., and is generally 0°C to 150°C, preferably 50°C to 120°C.
The reaction time may vary depending on a reaction temperature, etc., and is generally 30 minutes to 24 hours, preferably 1 hour to 12 hours.
"Step 7D" is a step of preparing Compound (5a) by treating Compound (25) with a base in the presence of water in a solven.
The solvent to be used is not particularly limited so long as it does not inhibit the reaction and dissolves the starting materials with a certain extent, and there may be mentioned, for example, alcohols such as methanol, ethanol, propanol

and isopropanol, etc.; ethers such as tetrahydrofuran, 1,4-dioxane and 1,2-dimethoxyethane, etc.; or a mixed solvent of optional combination thereof, etc., preferably methanol, ethanol, tetrahydrofuranor a mixed solvent thereof.
An amount of water to be used is generally 10 to 1000-fold mol amount based on 1 mol of Compound (25), and it may be used markedly excessively as a solvent.
As the base to be used, there may be mentioned, for example, alkali metal hydroxides such as lithium hydroxide, sodium hydroxide and potassium hydroxide, etc.; or alkali metal carbonates such as sodium carbonate and potassium carbonate, etc., preferably lithium hydroxide, sodium hydroxide or potassium hydroxide. An amount of the base to be used is generally 0.9 to 10-fold mol amount, preferably 1 to 5-fold mol amount based on 1 mol of Compound (25).
The reaction temperature may vary depending on a kind or amount(s), etc., of the starting materials, solvent(s), etc., and is generally -20°C to 150°C, preferably 0°C to 80°C.
The reaction time may vary depending on a reaction temperature, etc., and is generally 15 minutes to 72 hours, preferably 30 minutes to 48 hours.

[Preparation method 8]

"Preparation method 8" is a step of preparing the above-mentioned Compound (8) and Compound (13).
[Formula 26]

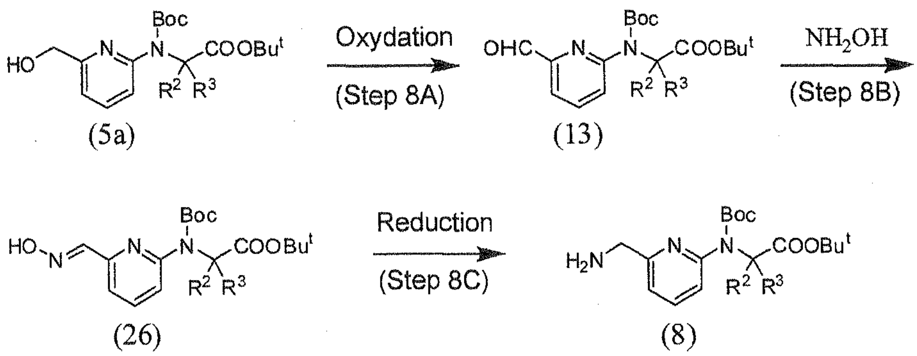

(5a) (13) (26) (8)

[wherein $R^2$ and $R^3$ have the same meanings as mentioned above.]
"Step 8A" is a step of preparing Compound (13) by oxidizing Compound (5a) using an oxidizing agent in an inert solvent. The oxidizing agent in the present step may be mentioned, for example, manganese dioxide, pyridinium chlorochromate (PCC), pyridinium dichromate (PDC) or 1,1,1,1-tris(acetoxy)-1,1-dihydro-1,2-benziodoxol-3-(1H)-one (hereinafter abbreviated to as Dess-Martin reagent), or the so-called TEMPO oxidizing agent in which sodium hypochlorite and 2,2,6,6-tetramethylpiperidine 1-oxyl (hereinafter abbreviated to as TEMPO) are used in combination, etc., and it is necessary to select reaction conditions depending on the kinds of the oxidizing agent to be used.
When manganese dioxide, pyridinium chlorochromate (PCC), pyridinium dichromate (PDC) or Dess-Martin reagent is used as the oxidizing agent, the reaction is carried out in an inert solvent.
The inert solvent to be used is not particularly limited so long as it does not inhibit the reaction and dissolves the starting materials with a certain extent, and there may be mentioned, for example, halogenated aliphatic hydrocarbons such as methylene chloride, chloroform and 1,2-dichloroethane, etc.; nitriles such as acetonitrile, etc.; or esters such as methyl acetate, ethyl acetate and isopropyl acetate, etc., preferably methylene chloride.
An amount of the oxidizing agent to be used may vary depending on the kinds of the oxidizing agent, but it is generally 0.9 to 100-fold mol amount, preferably 1 to 20-fold mol amount based on 1 mol of Compound (5a).
The reaction temperature may vary depending on a kind or amount(s), etc., of the starting materials, solvent(s), etc., and is generally 0°C to 150°C, preferably 0°C to 100°C.
The reaction time may vary depending on a reaction temperature, etc., and is generally 30 minutes to 24 hours, preferably 1 hour to 12 hours.
When sodium hypochlorite and TEMPO are used as the oxidizing agent, and the so-called TEMPO oxidation is carried out, the reaction is carried out in the presence of potassium bromide in an inert solvent.
The inert solvent to be used is not particularly limited so long as it does not inhibit the reaction and dissolves the starting materials with a certain extent, and there may be mentioned, for example, halogenated aliphatic hydrocarbons such as methylene chloride, chloroform and 1,2-dichloroethane, etc.; water; or a mixed solvent of optional combination thereof, etc., preferably a mixed solvent of methylene chloride and water.

An amount of the sodium hypochlorite to be used is generally 0.8 to 3-fold mol amount, preferably 0.9 to 1.5-fold mol amount based on 1 mol of Compound (5a). Incidentally, the sodium hypochlorite may be added as an aqueous solution wherein a pH of which is adjuted to 8 to 10 with sodium hydrogen carbonate.
An amount of TEMPO to be used is generally 0.001 to 0.1-fold mol amount, preferably 0.005 to 0.05-fold mol amount based on 1 mol of Compound (5a).
An amount of the potassium bromide to be used is generally 0.01 to 1-fold mol amount, preferably 0.05 to 0.2-fold mol amount based on 1 mol of Compound (5a).
The reaction temperature may vary depending on a kind or amount(s), etc., of the starting materials, solvent(s), etc., and is generally -30°C to 30°C, preferably -15°C to 15°C.
The reaction time may vary depending on a reaction temperature, etc., and is generally 10 minutes to 12 hours, preferably 30 minutes to 6 hours.

"Step 8B" is a step of preparing Compound (26) by reacting Compound (13) with hydroxylamine in an inert solvent.
The inert solvent to be used is not particularly limited so long as it does not inhibit the reaction and dissolves the starting materials with a certain extent, and there may be mentioned, for example, alcohols such as methanol, ethanol and isopropanol, etc.; halogenated aliphatic hydrocarbons such as methylene chloride, chloroform and 1,2-dichloroethane, etc.; nitriles such as acetonitrile, etc.; or esters such as methyl acetate, ethyl acetate and isopropyl acetate, etc., preferably methanol.
An amount of the hydroxylamine to be used is generally 1 to 5-fold mol amount, preferably 1 to 2-fold mol amount based on 1 mol of Compound (13).
A base, for example, triethylamine, diisopropylethylamine or pyridine, etc., may be added to promote the reaction. An amount of the base to be used is generally 0.5 to 20-fold mol amount, preferably 1 to 10-fold mol amount based on 1 mol of Compound (13).
The reaction temperature may vary depending on a kind or amount(s), etc., of the starting materials, solvent(s), etc., and is generally 0°C to 100°C, preferably 0°C to 60°C.
The reaction time may vary depending on a reaction temperature, etc., and is generally 30 minutes to 24 hours, preferably 1 hour to 12 hours.

"Step 8C" is a step of preparing Compound (8) by reducing Compound (26) under hydrogen atmosphere in the presence of a catalyst in an inert solvent.
The inert solvent to be used is not particularly limited so long as it does not inhibit the reaction and dissolves the starting materials with a certain extent, and there may be mentioned, for example, alcohols such as methanol, ethanol, propanol and isopropanol, etc.; ethers such as tetrahydrofuran, 1,4-dioxane and dimethoxyethane, etc.; halogenated aliphatic hydrocarbons such as methylene chloride, chloroform and 1,2-dichloroethane, etc.; esters such as methyl formate, ethyl formate, methyl acetate and ethyl acetate, etc.; aromatic hydrocarbons such as benzene and toluene, etc.; water; or a mixed solvent of optional combination thereof, etc., preferably methanol or ethanol.
The catalyst to be used may be mentioned, for example, palladium-active carbon, platinum-active carbon, platinum black, platinum oxide, rhodium-active carbon or Raney nickel, etc., preferably palladium-active carbon, platinum black, platinum oxide or Raney nickel. An amount of the catalyst to be used is generally 0.0005 to 1-fold mol amount, preferably 0.01 to 0.3-fold mol amount based on 1 mol of Compound (26).
A hydrogen partial pressure is generally 1 atm to 10 atm, preferably 1 atm to 5 atm.
An acid, for example, hydrogen chloride, hydrochloric acid, hydrobromic acid, hydroiodic acid, sulfuric acid, phosphoric acid, p-toluenesulfonic acid, methanesulfonic acid or trifluoroacetic acid, etc. (preferably hydrochloric acid or acetic acid) may be added to promote the reaction. An amount of the acid to be used is generally 1 to 200-fold mol amount, preferably 5 to 100-fold mol amount based on 1 mol of Compound (26).
The reaction temperature may vary depending on a kind or amount(s), etc., of the starting materials, solvent(s), etc., and is generally 0°C to 100°C, preferably 20°C to 80°C.
The reaction time may vary depending on a reaction temperature, etc., and is generally 15 minutes to 72 hours, preferably 30 minutes to 48 hours.

[Preparation method 9]

"Preparation method 9" is a step of preparing the above-mentioned Compound (15).
[Formula 27]

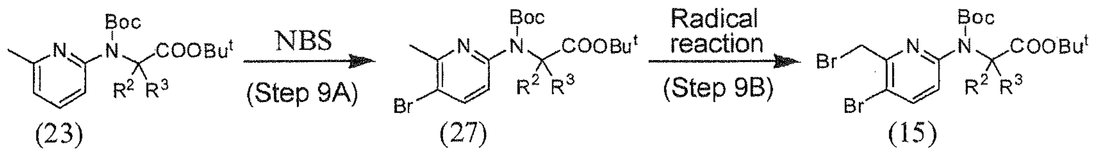


(23) (27) (15)

[wherein $R^2$ and $R^3$ have the same meanings as mentioned above.]

"Step 9A" is a step of preparing Compound (27) by treating Compound (23) with NBS in an inert solvent.

The inert solvent to be used is not particularly limited so long as it does not inhibit the reaction and dissolves the starting materials with a certain extent, and there may be mentioned, for example, aromatic hydrocarbons such as benzene, or chlorobenzene, etc.; halogenated aliphatic hydrocarbons such as methylene chloride, chloroform and 1,2-dichloroethane, etc.; ethers such as tetrahydrofuran, 1,2-dimethoxyethane and 1,4-dioxane, etc.; nitriles such as acetonitrile and propionitrile, etc.; amides such as N,N-dimethylformamide, N,N-dimethylacetamide and N-methylpyrrolidone, etc.; or a mixed solvent of optional combination thereof, etc., preferably acetonitrile.

An amount of NBS to be used is generally 0.9 to 5-fold mol amount, preferably 1 to 2-fold mol amount based on I mol of Compound (23).

The reaction temperature may vary depending on a kind or amount(s), etc., of the starting materials, solvent(s), etc., and is generally -20°C to 100°C, preferably 0°C to 60°C.

The reaction time may vary depending on a reaction temperature, etc., and is generally 10 minutes to 24 hours, preferably 1 hour to 12 hours.

"Step 9B" is a step of preparing Compound (15) by treating Compound (27) with NBS in the presence of a radical initiator or under photoirradiation in an inert solvent.

The inert solvent to be used is not particularly limited so long as it does not inhibit the reaction and dissolves the starting materials with a certain extent, and there may be mentioned, for example, halogenated aliphatic hydrocarbons such as methylene chloride, chloroform and 1,2-dichloroethane, etc.; or aromatic hydrocarbons such as benzene, chlorobenzene and dichlorobenzene, etc., preferably 1,2-dichloroethane or chlorobenzene.

An amount of NBS to be used is generally 0.9 to 5-fold mol amount, preferably 1 to 3-fold mol amount based on 1 mol of Compound (27).

The radical initiator to be used may be mentioned, for example, azobisisobutyronitrile, 2,2'-azobis(2,4-dimethylvaleronitrile), 2,2'-azobis(2-methylbutyronitrile) or benzoyl peroxide, etc. An amount of the radical initiator to be used is generally 0.001 to 1-fold mol amount, preferably 0.01 to 0.5-fold mol amount based on 1 mol of Compound (31).

The reaction temperature may vary depending on a kind or amount(s), etc., of the starting materials, solvent(s), etc., and is generally 0°C to 150°C, preferably 30°C to 100°C.

The reaction time may vary depending on a reaction temperature, etc., and is generally 10 minutes to 12 hours, preferably 15 minutes to 6 hours.

When the reaction is carried out by generating radicals under photoirradiation, the reaction is carried out in accordance with the case where a radical initiator is used except for irradiating a light using a mercury lamp as a light source.

[Preparation method 10]

"Preparation method 10" is another method for preparing Compound (4).

[Formula 28]

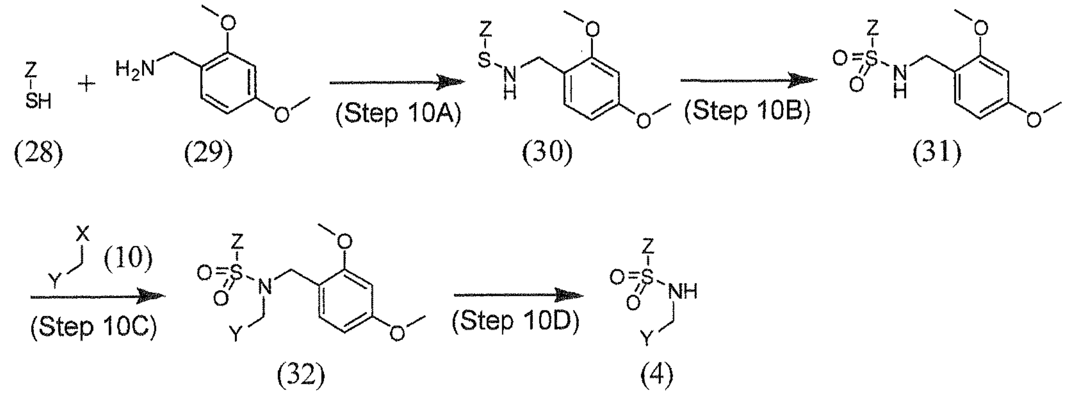


(28) (29) (30) (31) (32) (4)

[wherein X, Y and Z have the same meanings as mentioned above.]

"Step 10A" is a step of preparing Compound (30) by reacting Compound (28) and Compound (29) in the presence of trichloroisocyanuric acid and quaternary ammonium chloride in an inert solvent. For example, when trichloroisocyanuric acid and quaternary ammonium chloride are mixed in an inert solvent, then, reacting with Compound (28), and then, reacting with Compound (29) in the presence or in the absence of a base.

Compound (28) are known, or can be prepared according to the known method from the known compound(s).

The inert solvent to be used is not particularly limited so long as it does not inhibit the reaction and dissolves the starting materials with a certain extent, and there may be mentioned, for example, alcohols such as methanol, ethanol, propanol and isopropanol, etc.; ethers such as tetrahydrofuran, 1,4-dioxane and 1,2-dimethoxyethane, etc.; halogenated aliphatic hydrocarbons such as methylene chloride, chloroform and 1,2-dichloroethane, etc.; water; or a mixed solvent of optional combination thereof, etc., preferably methylene chloride, methanol, water or a mixed solvent of optional combination thereof.

An amount of Compound (29) to be used is generally 1 to 20-fold mol amount, preferably 1 to 10-fold mol amount based on 1 mol of Compound (28).

An amount of the trichloroisocyanuric acid to be used is generally 0.1 to 10-fold mol amount, preferably 0.3 to 3-fold mol amount based on 1 mol of Compound (28).

The quaternary ammonium chloride to be used may be mentioned, for example, tetramethyl ammonium chloride, tetraethyl ammonium chloride, tetrabutyl ammonium chloride, benzyltrimethyl ammonium chloride or benzyldimethylphenyl ammonium chloride, etc., preferably benzyltrimethyl ammonium chloride. An amount of the quaternary ammonium chloride to be used is generally 0.5 to 10-fold mol amount, preferably 1 to 5-fold mol amount based on 1 mol of Compound (28).

As the base to be used, there may be mentioned, for example, organic bases such as triethylamine, diisopropylethylamine, pyridine, N,N-dimethylaminopyridine, picoline and 2,6-lutidine, etc.; or inorganic bases such as sodium hydrogen carbonate, potassium hydrogen carbonate, sodium carbonate and potassium carbonate, etc., preferably triethylamine or diisopropylethylamine. An amount of the base to be used is generally 1 to 100-fold mol amount, preferably 1 to 10-fold mol amount based on 1 mol of Compound (28).

The reaction temperature may vary depending on a kind or amount(s), etc., of the starting materials, solvent(s), etc., and is generally -78°C to 100°C, preferably -78°C to 30°C.

The reaction time may vary depending on a reaction temperature, etc., and is generally 15 minutes to 72 hours, preferably 30 minutes to 24 hours.

"Step 10B" is a step of preparing Compound (31) by oxidizing Compound (30) using an oxidizing agent in an inert solvent.

The inert solvent to be used is not particularly limited so long as it does not inhibit the reaction and dissolves the starting materials with a certain extent, and there may be mentioned, for example, alcohols such as methanol, ethanol, propanol and isopropanol, etc.; ketones such as acetone and methyl ethyl ketone, etc.; halogenated aliphatic hydrocarbons such as methylene chloride, chloroform and 1,2-dichloroethane, etc.; carboxylic acids such as acetic acid and formic acid, etc.; water; or a mixed solvent of optional combination thereof, etc., preferably acetone, water or a mixed solvent of optional combination thereof.

The oxidizing agent to be used may be mentioned, for example, potassium permanganate, chromium(VI) oxide or potassium dichromate, etc., preferably potassium permanganate. An amount of the oxidizing agent to be used is generally 0.5 to 10-fold mol amount, preferably 1 to 3-fold mol amount based on 1 mol of Compound (30).

The reaction temperature may vary depending on a kind or amount(s), etc., of the starting materials, solvent(s), etc., and is generally -50°C to 100°C, preferably -20°C to 50°C.

The reaction time may vary depending on a reaction temperature, etc., and is generally 30 minutes to 72 hours, preferably 1 hour to 24 hours.

"Step 10C" is a step of preparing Compound (32) by reacting Compound (31) and Compound (10). The present step is carried out in accordance with the above-mentioned "Step 1B" except for using Compound (31) in place of Compound (4), and using Compound (10) in place of Compound (5), respectively.

"Step 10D" is a step of preparing Compound (4) by removing a 2,4-dimethoxybenzyl group from Compound (32). The present step is carried out in accordance with the published article (see T.W.Greene & P.G.M.Wuts, Protective Groups in Organic Synthesis 4th Ed., John Wiley & Sons, Inc., p.917), and can be carried out, for example, by treating Compound (32) with an acid in an inert solvent, but this is not limited by the method.

The inert solvent to be used is not particularly limited so long as it does not inhibit the reaction and dissolves the starting materials with a certain extent, and there may be mentioned, for example, halogenated aliphatic hydrocarbons such as methylene chloride, chloroform and 1,2-dichloroethane, etc.; ethers such as diethyl ether, tetrahydrofuran, 1,4-dioxane and 1,2-dimethoxyethane, etc.; water; or a mixed solvent of optional combination thereof, etc., preferably methylene chloride.

The acid to be used may be mentioned, for example, hydrogen chloride, hydrochloric acid, hydrobromic acid, hydroiodic acid, sulfuric acid, phosphoric acid, p-toluenesulfonic acid, methanesulfonic acid or trifluoroacetic acid, etc., preferably trifluoroacetic acid or p-toluenesulfonic acid. An amount of the acid to be used is generally 1 to 200-fold mol amount, preferably 5 to 100-fold mol amount based on 1 mol of Compound (32), and it may be used markedly excessively as a solvent.

The reaction temperature may vary depending on a kind or amount(s), etc., of the starting materials, solvent(s), etc., and is generally -78°C to 100°C, preferably -78°C to 50°C.

The reaction time may vary depending on a reaction temperature, etc., and is generally 30 minutes to 72 hours, preferably 1 hour to 24 hours.

The substituent(s) on the substituent Y and/or the substituent Z may be previously introduced before starting the preparation, and a desired substituent may be introduced before preparing a basic skeleton of the compound according to the present invention by the above-mentioned Preparation method using a synthetic method(s) conventionally used in organic chemical reactions in combination, if necessary. Further, after preparing the basic skeleton according to the above-mentioned Preparation method, a desired substituent(s) may be introduced into the basic skeleton.

The preparation method of the synthetic intermediates of the compound according to the present invention will be explained in the following Reference Examples in detail.

The objective compounds formed in each of the respective reactions can be obtained from a reaction mixture in accordance with the conventional methods. For example, after suitably neutralizing the reaction mixture, or removing insolubles by filtration in the case such insolubles are present, an organic solvent such as ethyl acetate that is not miscible with water is added followed by rinsing with water, separating the organic layer containing the objective compound, drying with a drying agent such as anhydrous magnesium sulfate, etc., and distilling off the solvent to obtain the objective compound.

The resulting objective compound can be separated and purified as necessary by suitably combining the conventional methods, examples of which include recrystallization; reprecipitation; or a method commonly used to separate and purify ordinary organic compounds (such as adsorption column chromatography using a carrier such as silica gel, alumina, etc.; ion exchange chromatography; or normal or reverse phase column chromatography using silica gel or alkylated silica gel (preferably, high-performance liquid chromatography)).

Although the compound represented by the formula (I) of the present invention can be converted into a pharmaceutically acceptable salt in accordance with ordinary methods as necessary, it can be also separated directly from the reaction mixture as a salt.

In the case of using the compound represented by the formula (I) or a pharmaceutically acceptable salt thereof of the present invention as a medicine, the compound or a pharmaceutically acceptable salt thereof, per se can be administered (as a bulk powder), or can be administered orally or parenterally (such as intravenous administration, intramuscular administration, intraperitoneal administration, transcutaneous administration, transtracheal administration, intracutaneous administration and subcutaneous administration) in a form such as a tablet, capsule, powder, syrup, granule, fine particles, pill, suspension, emulsion, transdermal preparation, suppository, ointment, lotion, inhalant and injection, which is prepared by mixing with a suitable pharmaceutically acceptable vehicle or diluent and the like.

These preparations are prepared by commonly known methods using additives such as vehicles, lubricants, binders, disintegrators, emulsifiers, stabilizers, corrigents or diluents and the like.

Examples of vehicles include organic vehicles and inorganic vehicles. Examples of organic vehicles include sugar derivatives such as lactose, sucrose, glucose, mannitol and sorbitol; starch derivatives such as cornstarch, potato starch, α-starch and dextrin; cellulose derivatives such as crystalline cellulose; gum Arabic; dextran; and pullulan. Examples of inorganic vehicles include light silicic acid anhydride; and sulfates such as calcium sulfate.

Examples of lubricants include stearic acid; stearic acid metal salts such as calcium stearate and magnesium

stearate; talc; colloidal silica; waxes such as beeswax and spermaceti; boric acid; adipic acid; sulfates such as sodium sulfate; glycol; fumaric acid; sodium benzoate; D,L-leucine; sodium lauryl sulfate; silicic acids such as silicic acid anhydride and silicic acid hydrate; and the above-mentioned starch derivatives listed as examples of the vehicles.

Examples of binders include hydroxypropyl cellulose, hydroxypropyl methylcellulose, polyvinyl pyrrolidone, Macrogol and the above-mentioned compounds listed as examples of the vehicles.

Examples of disintegrators include cellulose derivatives such as low substitution-degree hydroxypropyl cellulose, carboxymethyl cellulose, calcium carboxymethyl cellulose and internally-crosslinked calcium carboxymethyl cellulose; crosslinked polyvinyl pyrrolidone; and chemically modified starch or cellulose derivatives such as carboxymethyl starch and sodium carboxymethyl starch.

Examples of emulsifiers include colloidal clays such as bentonite and bee gum; anionic surfactants such as sodium lauryl sulfate; cationic surfactants such as benzalkonium chloride; and nonionic surfactants such as polyoxyethylene alkyl ethers, polyoxyethylene sorbitan fatty acid esters and sucrose fatty acid esters.

Examples of stabilizers include para-hydroxybenzoic acid esters such as methylparaben and propylparaben; alcohols such as chlorobutanol, benzyl alcohol and phenylethyl alcohol; benzalkonium chloride; phenols such as phenol and cresol; thimerosal; acetic anhydride; and sorbic acid.

Examples of corrigents include sweeteners such as sodium saccharin and aspartame; sour flavorings such as citric acid, malic acid and tartaric acid; and flavorings such as menthol, lemon extract and orange extract.

Examples of diluents include compounds ordinarily used as diluents, such as lactose, mannitol, glucose, sucrose, calcium sulfate, hydroxypropyl cellulose, microcrystalline cellulose, water, ethanol, polyethylene glycol, propylene glycol, glycerol, starch, polyvinyl pyrrolidone and mixtures thereof.

When the effective ingredient of a medical composition of the present invention is used as a medicine, in addition to the above-mentioned administration forms, ophthalmic solutions can be mentioned, and it is particularly preferred for glaucoma treatment. The ophthalmic solution can be prepared by the method known to the art, and as additives, etc., an isotonic agent, a buffer, a pH adjuster, a solibilizing agent, a thickening agent, a stabilizer, a preservative (antiseptic), etc., may be optionally formulated. Also, by adding a pH adjuster, thickening agent, dispersant, etc., the medical component is dispersed, whereby stable ophthalmic solutions can be obtained.

As the isotonic agent, there may be mentioned, for example, glycerin, propylene glycol, sodium chloride, potassium chloride, sorbitol, mannitol, etc.

As the buffer, there may be mentioned, for example, phosphoric acid, phosphate, citric acid, acetic acid, ε-aminocaproic acid, etc.

As the pH adjuster, there may be mentioned, for example, hydrochloric acid, citric acid, phosphoric acid, acetic acid, sodium hydroxide, potassium hydroxide, boric acid, borax, sodium carbonate, sodium hydrogen carbonate, etc.

As the solibilizing agent, there may be mentioned, for example, Polysorbate 80, polyoxyethylene hardened castor oil 60, Macrogol 4000, etc.

As the thickening agent and dispersant, there may be mentioned, for example, cellulose series polymers such as hydroxypropylmethyl cellulose, hydroxypropyl cellulose, etc., polyvinyl alcohol, polyvinyl pyrrolidone, etc., and as the stabilizer, there may be mentioned, for example, edetic acid, disodium edetate, etc.

As the preservative (antiseptic), there may be mentioned, for example, general purpose sorbic acid, potassium sorbate, benzalkonium chloride, benzetonium chloride, methyl paraoxybenzoate, propyl paraoxybenzoate, chlorobutanol, etc., and these preservatives may be used in combination.

Ophthalmic solutions containing the effective ingredient of the medical composition according to the present invention is desirably set a pH to 4.0 to 8.5.

A dosage of the effective ingredient in a medical composition of the present invention may vary depending on a symptom, age, administration method, etc., and, for example, in the case of oral administration, it can be administered with a lower limit of 0.001 mg/Kg (preferably 0.01 mg/Kg) and an upper limit of 100 mg/Kg (preferably 10 mg/Kg) per each administration, and in the case of parenteral administration, it can be administered with a lower limit of 0.0001 mg/Kg (preferably 0.0005 mg/Kg) and an upper limit of 10 mg/Kg (preferably 5 mg/Kg) per each administration, with 1 to 6 times per day to an adult person depending on the symptoms. In the case of ophthalmic solutions, it may be eye-dropped with a concentration of preferably 0.000001 to 1% (w/v), more preferably 0.00001 to 0.1% (w/v) with one to several drops per each time once to several times (for example, 1 to 8 times) per day.

However, the administration dose may vary depending on the various conditions, so that there is a case where a less dosage than the above-mentioned administration dose may be sufficient in some cases, and there is a case where it is necessary to administer with a larger amount than the above range.

EXAMPLES

In the following, the present invention will be explained in detail by referring to Examples, Reference examples, and Test examples, but the scope of the present invention is not limited by these. Incidentally, the Rf values in Examples

are values measured by using thin-layer chromatography (available from Merck, TLC plate silica gel $60F_{254}$ (Trade name)), and the description in the parentheses represent an eluent (volume ratio).

[Example 1]

(6-{[5-(1,1-Dimethylpentyl)thiophen-2-ylmethyl](pyridin-3-ylsulfonyl)aminomethyl}-pyridin-2-ylamino)acetic acid (Exemplary compound No. 673)

1-(a): tert-Butyl [tert-butoxycarbonyl(6-{[5-(1,1-dimethylpentyl)thiophen-2-ylmethyl]-(pyridin-3-ylsulfonyl)aminomethyl}pyridin-2-yl)amino]acetate

To 4.0 ml of a tetrahydrofuran solution containing 422 mg (0.880 mmol) of tert-butyl (tert-butoxycarbonyl{6-[(pyridin-3-ylsulfonyl)aminomethyl]pyridin-2-yl}-amino)acetate obtained in the same manner as in Reference example 1-(f), Reference example 4-(c) were added 1.27 g (6.00 mmol) of [5-(1,1-dimethylpentyl)thiophen-2-yl]methanol, 395 μl (1.60mol) of tri-n-butylphosphine and 276 mg (1.60 mmol) of N,N,N',N'-tetramethylazodicarboxamide, and the mixture was stirred at room temperature for 16 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; toluene:ethyl acetate=5:1→4:1 (V/V)), and the fractions containing the objective material were concentrated under reduced pressure to obtain 485 mg of the title compound as colorless oily product. (Yield: 90%) Mass Spectrum (FAB, m/z): 673 ($M^+$+1).
$^1$H-NMR Spectrum ($CDCl_3$, δ ppm): 8.95 (dd, J=2.4, 0.7 Hz, 1H), 8.70 (dd, J=4.9, 1.6 Hz, 1H), 7.88 (ddd, J=8.1, 2.4, 1.6 Hz, 1H), 7.76 (d, J=8.4 Hz, 1H), 7.58 (dd, J=8.4, 7.3 Hz, 1H), 7.30 (ddd, J=8.1, 4.9, 0.7 Hz, 1H), 7.00 (d, J=7.3 Hz, 1H), 6.66 (d, J=3.6 Hz, 1H), 6.51 (d, J=3.6 Hz, 1H), 4.65 (s, 2H), 4.47 (s, 2H), 4.46 (s, 2H), 1.54-1.48 (m, 11H), 1.43 (s, 9H), 1.31-1.19 (m, 2H), 1.25 (s, 6H), 1.14-1.04 (m, 2H), 0.86 (t, J=7.2 Hz, 3H).

1-(b): (6-{[5-(1-1-Dimethylpentyl)thiophen-2-ylmethyl](pyridin-3-ylsulfonyl)aminomethyl}pyridin-2-ylamino)acetic acid

To 476 mg (0.707 mmol) of tert-butyl [tert-butoxycarbonyl(6-{[5-(1,1-dimethylpentyl)thiophen-2-ylmethyl](pyridin-3-ylsulfonyl)aminomethyl}pyridin-2-yl)-amino]acetate obtained in Example 1-(a) was added a mixed solution comprising 2.7 ml (35 mmol) of trifluoroacetic acid and 10 ml of methylene chloride, and the mixture was stirred at room temperature for 16 hours. After completion of the reaction, the reaction mixture was concentrated under reduced pressure. To the residue was added 20 ml of water, the mixture was adjusted to pH 4.5 with 1N aqueous sodium hydroxide solution and 1N hydrochloric acid, and extracted with ethyl acetate. The organic layer was washed with a saturated aqueous sodium chloride solution, dried over anhydrous magnesium sulfate and concentrated under reduced pressure. The residue was applied to silica gel column chromatography (eluent; methylene chloride:methanol=9:1 (V/V)), and the fractions containing the objective material were concentrated under reduced pressure to obtain 340 mg of the title compound as pale yellow foam. (Yield: 93%) Mass Spectrum (FAB, m/z): 517 ($M^+$+1).
$^1$H-NMR Spectrum (DMSO-$d_6$, δ ppm): 12.37 (brs, 0.6H), 8.83 (dd, J=2.4, 0.7 Hz, 1H), 8.72 (dd, J=4.8, 1.7 Hz, 1H), 8.03 (ddd, J=8.1, 2.4, 1.7 Hz, 1H), 7.48 (ddd, J=8.1, 4.8, 0.7 Hz, 1H), 7.28 (dd, J=8.4, 7.2 Hz, 1H), 6.78 (t, J=5.9 Hz, 1H), 6.76 (d, J=3.5 Hz, 1H), 6.61 (d, J=3.5 Hz, 1H), 6.40 (d, J=8.4 Hz, 1H), 6.39 (d, J=7.2 Hz, 1H), 4.73 (s, 2H), 4.22 (s, 2H), 3.75 (d, J=5.9 Hz, 2H), 1.51-1.48 (m, 2H), 1.25-1.18 (m, 2H), 1.23 (s, 6H), 1.10-1.03 (m, 2H), 0.83 (t, J=7.3 Hz, 3H).
Rf value: 0.50 (n-butanol:acetic acid:water=10:1:1).

[Example 2]

(6-{[5-(1,1-Dimethylpentyl)thiophen-2-ylmethyl](pyridin-2-ylsulfonyl)aminomethyl}-pyridin-2-ylamino)acetic acid (Exemplary compound No. 666)

2-(a): tert-Butyl [tert-butoxycarbonyl(6-{[5-(1,1-dimethylpentyl)thiophen-2-ylmethyl]-(pyridin-2-ylsulfonyl)aminomethyl} pyridin-2-yl)amino]acetate

To 30 ml of a tetrahydrofuran solution containing 3.16 g (6.60 mmol) of tert-butyl (tert-butoxycarbonyl{6-[(pyridin-2-ylsulfonyl)aminomethyl]pyridin-2-yl}amino)-acetate obtained in the same manner as in Reference example 2 were added 1.27 g (6.00 mmol) of [5-(1,1-dimethylpentyl)thiophen-2-yl]methanol obtained in Reference example 4-(c), 2.96 ml (12.0mol) of tri-n-butylphosphine and N,N,N',N'-tetramethyl-azodicarboxamide 2.07 g (12.0 mmol), and the mixture was stirred at room temperature for 16 hours. After completion of the reaction, water was added to the reaction mixture and the mixture was extracted with ethyl acetate. The organic layer was washed with a saturated aqueous sodium

chloride solution, dried over anhydrous magnesium sulfate and concentrated under reduced pressure. The residue was applied to silica gel column chromatography (eluent; toluene:ethyl acetate=9:1→7:1 (V/V)), and the fractions containing the objective material were concentrated under reduced pressure to obtain 3.50 g of the title compound as white foam. (Yield: 87%)
Mass Spectrum (FAB, m/z): 673 ($M^+$+1).
$^1$H-NMR Spectrum ($CDCl_3$, δ ppm): 8.59 (ddd, J=4.6, 1.7, 1.1 Hz, 1H), 7.84 (ddd, J=7.7, 1.3, 1.1 Hz, 1H), 7.77 (ddd, J=7.7, 7.5, 1.7 Hz, 1H), 7.69 (d, J=8.4 Hz, 1H), 7.52 (dd, J=8.4, 7.4 Hz, 1H), 7.37 (ddd, J=7.5, 4.6, 1.3 Hz, 1H), 7.03 (d, J=7.4 Hz, 1H), 6.62 (d, J=3.6 Hz, 1H), 6.45 (d, J=3.6 Hz, 1H), 4.75 (s, 2H), 4.59 (s, 2H), 4.52 (s, 2H), 1.52-1.46 (m, 11H), 1.43 (s, 9H), 1.27-1.18 (m, 2H), 1.23 (s, 6H), 1.12-1.02 (m, 2H), 0.86 (t, J=7.2 Hz, 3H).

2-(b): (6-{[5-(1,1-Dimethylpentyl)thiophen-2-ylmethyl](pyridin-2-ylsulfonyl)aminomethyl}pyridin-2-ylamino)acetic acid

To 3.40 g (5.05 mmol) oftert-butyl [tert-butoxycarbonyl(6-{[5-(1,1-dimethylpentyl)thiophen-2-ylmethyl](pyridin-2-ylsulfonyl)aminomethyl}pyridin-2-yl) amino] - acetate obtained in Example 2-(a) was added a mixed solution comprising 20 ml (260 mmol) of trifluoroacetic acid and 60 ml of methylene chloride, and the mixture was stirred at room temperature for 8 hours. After completion of the reaction, the reaction mixture was concentrated under reduced pressure. To the residue was added 50 ml of water, the mixture was adjusted to pH 4.5 with 1N aqueous sodium hydroxide solution and 1N hydrochloric acid, and extracted with methylene chloride. The organic layer was washed with a saturated aqueous sodium chloride solution, dried over anhydrous magnesium sulfate and concentrated under reduced pressure to obtain 2.38 g of the title compound as pale yellowish white foam. (Yield: 91 %)
Mass Spectrum (FAB, m/z): 517 ($M^+$+1).
$^1$H-NMR Spectrum (DMSO-$d_6$, δ ppm): 12.39 (brs, 0.6H), 8.62 (ddd, J=4.8, 1.8, 0.9 Hz, 1H), 7.97 (ddd, J=7.7, 7.7, 1.8 Hz, 1H), 7.81 (ddd, J=7.7, 1.1, 0.9 Hz, 1H), 7.58 (ddd, J=7.7, 4.8, 1.1 Hz, 1H), 7.25 (dd, J=8.4, 7.2 Hz, 1H), 6.77 (t, J=6.0 Hz, 1H), 6.71 (d, J=3.5 Hz, 1H), 6.56 (d, J=3.5 Hz, 1H), 6.38 (d, J=8.4 Hz, 1H), 6.36 (d, J=7.2 Hz, 1H), 4.75 (s, 2H), 4.29 (s, 2H), 3.85 (d, J=6.0 Hz, 2H), 1.49-1.45 (m, 2H), 1.25-1.18 (m, 2H), 1.21 (s, 6H), 1.08-1.01 (m, 2H), 0.83 (t, J=7.3 Hz, 3H).
Rfvalue: 0.48 (n-butanol:acetic acid:water=10:1:1).

[Example 3]

(6-{(4-Fluorobenzenesulfonyl)[5-(1,1-dimethylpentyl)thiophen-2-ylmethyl]aminomethyl}pyridin-2-ylamino)acetic acid (Exemplary compound No. 642)

3-(a): tert-Butyl [tert-butoxycarbonyl(6-{(4-fluorobenzenesulfonyl)[5-(1,1-dimethylpentyl)thiophen-2-ylmethyl]aminomethyl}pyridin-2-yl)amino]acetate

To 4.0 ml of a tetrahydrofuran solution containing 436 mg (0.880 mmol) of tert-butyl (tert-butoxycarbonyl{6-[(4-fluorobenzenesulfonyl)aminomethyl]pyridin-2-yl}amino)acetate obtained in Reference example 3 were added 170 mg (0.800 mmol) of [5-(1,1-dimethylpentyl)thiophen-2-yl]methanol obtained in Reference example 4-(c), 395 μl (1.60mol) oftri-n-butylphosphine and 276 mg (1.60 mol) of N,N,N',N'-tetra-methylazodicarboxamide, and the mixture was stirred at room temperature for 16 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; toluene:ethyl acetate=8:1 (V/V)), and the fractions containing the objective material were concentrated under reduced pressure to obtain 489 mg of the title compound as colorless oily product. (Yield: 89%)
Mass Spectrum (FAB, m/z): 690 ($M^+$+1).
$^1$H-NMR Spectrum ($CDCl_3$, δ ppm): 7.75-7.67 (m, 3H), 7.56 (dd, J=8.3, 7.3 Hz, 1H), 7.11-7.03 (m, 2H), 7.00 (d, J=7.3 Hz, 1H), 6.62 (d, J=3.4 Hz, 1H), 6.50 (d, J=3.4 Hz, 1H), 4.60 (s, 2H), 4.48 (s, 2H), 4.41 (s, 2H), 1.53-1.48 (m, 11H), 1.43 (s, 9H), 1.28-1.18 (m, 2H), 1.25 (s, 6H), 1.14-1.04 (m, 2H), 0.85 (t, J=7.2 Hz, 3H).

3-(b): (6-{(4-Fluorobenzenesulfonyl)[5-(1,1-dimethylpentyl)thiophen-2-ylmethyl]-aminomethyl}pyridin-2-ylamino)acetic acid

To 482 mg (0.698 mmol) of tert-butyl [tert-butoxycarbonyl(6-{(4-fluorobenzenesulfonyl)[5-(1,1-dimethylpentyl) thiophen-2-ylmethyl]aminomethyl}pyridin-2-yl)amino]acetate obtained in Example 3-(a) was added a mixed solution comprising 1.6 ml (21 mmol) of trifluoroacetic acid and 8 ml of methylene chloride, and the mixture was stirred at room temperature for 16 hours. After completion of the reaction, the reaction mixture was concentrated under reduced pressure. To the residue was added 20 ml of water, the mixture was adjusted to pH 4.5 with 1N aqueous sodium hydroxide solution and 1N hydrochloric acid, and extracted with ethyl acetate. The organic layer was washed with a saturated aqueous sodium chloride solution, dried over anhydrous magnesium sulfate and concentrated under reduced pressure. The residue was applied to silica gel column chromatography (eluent; methylene chloride:methanol= 15:1→10:1 (V/V)), and the fractions containing the objective material were concentrated under reduced pressure to obtain 224 mg of the title compound as white solid. (Yield: 60%) Mass Spectrum (FAB, m/z): 534 ($M^+$+1).
$^1$H-NMR Spectrum (DMSO-$d_6$, δ ppm): 12.39 (brs, 0.3H), 7.77-7.73 (m, 2H), 7.31-7.26 (m, 3H), 6.80 (t, J=5.8 Hz, 1H), 6.73 (d, J=3.5 Hz, 1H), 6.60 (d, J=3.5 Hz, 1H), 6.41 (d, J=7.9 Hz, 1H), 6.37 (d, J=7.0 Hz, 1H), 4.67 (s, 2H), 4.18 (s, 2H), 3.80 (d, J=5.8 Hz, 2H), 1.51-1.47 (m, 2H), 1.25-1.17 (m, 2H), 1.23 (s, 6H), 1.10-1.02 (m, 2H), 0.82 (t, J=7.3 Hz, 3H).
Rf value: 0.66 (n-butanol:acetic acid:water=10:1:1).

[Example 4]

{6-[(5-tert-Pentylthiophen-2-ylmethyl)(pyridin-2-ylsulfonyl)aminomethyl]pyridin-2-ylamino}acetic acid (Exemplary compound No. 272)

4-(a): tert-Butyl(tert-butoxycarbonyl{6-[(5-tert-pentylthiophen-2-ylmethyl)(pyridin-2-ylsulfonyl)aminomethyl]pyridin-2-yl} amino)acetate

To 4.8 ml of a tetrahydrofuran solution containing 200 mg (0.418 mmol) of tert-butyl (tert-butoxycarbonyl{6-[(pyridin-2-ylsulfonyl)aminomethyl]pyridin-2-yl}-amino)acetate obtained in the same manner as in Reference example 2 were added 77 mg (0.42 mmol) of [(5-tert-pentylthiophen-2-yl)methanol obtained in Reference example 5, 198 μl (0.803 mmol) of tri-n-butylphosphine and 113 mg (0.656 mmol) of N,N,N',N'-tetramethylazodicarboxamide, and the mixture was stirred at room temperature for 20 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=7:3 (V/V)), and the fractions containing the objective material were concentrated under reduced pressure to obtain 201 mg of the title compound as pale yellow oil. (Yield: 74%) Mass Spectrum (FAB, m/z): 645 ($M^+$+1).
$^1$H-NMR Spectrum ($CDCl_3$, δ ppm): 8.59 (ddd, J=4.7, 1.7, 1.1 Hz, 1H), 7.87-7.74 (m, 2H), 7.69 (d, J=8.3 Hz, 1H), 7.52 (dd, J=8.3, 7.4 Hz, 1H), 7.38 (ddd, J=7.4, 4.7, 1.4 Hz, 1H), 7.04 (d, J=7.4 Hz, 1H), 6.62 (d, J=3.7 Hz, 1H), 6.46 (d, J=3.7 Hz, 1H), 4.75 (s, 2H), 4.59 (s, 2H), 4.53 (s, 2H), 1.58-1.49 (m, 2H), 1.52 (s, 9H), 1.43 (s, 9H), 1.22 (s, 6H), 0.71 (t, J=7.4 Hz, 3H).

4-(b): {6-[(5-tert-Pentylthiophen-2-ylmethyl)(pyridin-2-ylsulfonyl)aminomethyl]-pyridin-2-ylamino}acetic acid

To 1.7 ml of a methylene chloride solution containing 191 mg (0.296 mmol) of tert-butyl (tert-butoxycarbonyl {6-[(5-tert-pentylthiophen-2-ylmethyl)(pyridin-2-yl-sulfonyl)aminomethyl]pyridin-2-yl}amino)acetate obtained in Example 4-(a) was added 1.4 ml (5.6 mmol) of 4N hydrogen chloride/1,4-dioxane solution, and the mixture was stirred at room temperature for 19 hours. After completion of the reaction, the reaction mixture was concentrated under reduced pressure. Water was added to the residue, and the mixture was adjusted to pH 4.5 with 1N aqueous sodium hydroxide solution and 1N hydrochloric acid. Precipitated solid was collected by filtration, washed with water and dried at 50°C under reduced pressure to obtain 123 mg of the title compound as white solid. (Yield: 85%)
Mass Spectrum (FAB, m/z): 489 ($M^+$+1).
$^1$H-NMR Spectrum (DMSO-$d_6$, δ ppm): 8.62 (ddd, J=4.7, 1.8, 0.9 Hz, 1H), 7.97 (ddd, J=7.7, 7.8, 1.8 Hz, 1H), 7.81 (ddd, J=7.8, 1.0, 0.9 Hz, 1H), 7.58 (ddd, J=7.7, 4.7, 1.0 Hz, 1H), 7.25 (dd, J=8.2, 7.1 Hz, 1H), 6.75 (t, J=5.6 Hz, 0.9H), 6.72 (d, J=3.5 Hz, 1H), 6.57 (d, J=3.5 Hz, 1H), 6.38 (d, J=8.2 Hz, 1H), 6.36 (d, J=7.1 Hz, 1H), 4.75 (s, 2H), 4.29 (s, 2H), 3.83 (d, J=5.6 Hz, 2H), 1.51 (q, J=7.4 Hz, 2H), 1.20 (s, 6H), 0.67 (t, J=7.4 Hz, 3H). Rf value: 0.45 (n-butanol:acetic acid:water=10:1:1).

[Example 5]

(6-{[4-(1-Methylcyclopropyl)benzyl](pyridin-3-ylsulfonyl)aminomethyl}pyridin-2-ylamino)acetic acid hydrochloride (Exemplary compound No. 1037)

5-(a): tert-Butyl[tert-butoxycarbonyl(6-{[4-(1-methylcyclopropyl)benzyl](pyridin-3-yl-sulfonyl)aminomethyl}pyridin-2-yl)amino]acetate

To 12.8 ml of a tetrahydrofuran solution containing 465 mg (1.54 mmol) of N-[4-(1-methylcyclopropyl)benzyl]pyridin-3-ylsulfonamide obtained in Reference example 6-(c) were added 520 mg (1.54 mmol) of tert-butyl [tert-butoxycarbonyl(6-hydroxymethylpyridin-2-yl)amino]acetate obtained in Reference example 1-(b), 0.57 ml (2.3 mmol) of tri-n-butylphosphine and 398 mg (2.31 mmol) of N,N,N',N'-tetramethyl-azodicarboxamide, and the mixture was stirred at room temperature for 3 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The obtained residue was applied to silica gel column chromatography (eluent; n-hexane: ethyl acetate=1:1 (V/V)), and the fractions containing the objective material were concentrated under reduced pressure to obtain 868 mg of the title compound as white foam. (Yield: 91%)
Mass Spectrum (FAB, m/z): 622 ($M^+$).
$^1$H-NMR Spectrum ($CDCl_3$, δ ppm): 8.93 (dd, J=2.3, 0.8 Hz, 1H), 8.69 (dd, J=4.8, 1.6 Hz, 1H), 7.84 (ddd, J=8.0, 2.3, 1.6 Hz, 1H), 7.71 (d, J=8.3 Hz, 1H), 7.51 (dd, J=8.3, 7.4 Hz, 1H), 7.29 (ddd, J=8.0, 4.8, 0.8 Hz, 1H), 7.15-7.09 (m, 4H), 6.85 (d, J=7.4 Hz, 1H), 4.53 (s, 2H), 4.38 (s, 2H), 4.37 (s, 2H), 1.53 (s, 9H), 1.43 (s, 9H), 1.37 (s, 3H), 0.87-0.68 (m, 4H).

5-(b): (6-{[4-(1-Methylcyclopropyl)benzyl](pyridin-3-ylsulfonyl)aminomethyl}pyridin-2-ylamino)acetic acid hydrochloride

To 7.7 ml of a methylene chloride solution containing 800 mg (1.28 mmol) of tert-butyl [tert-butoxycarbonyl(6-{[4-(1-methylcyclopropyl)benzyl](pyridin-3-yl-sulfonyl)aminomethyl}pyridin-2-yl)amino]acetate obtained in Example 5-(a) was added 6.1 ml (24 mmol) of 4N hydrogen chloride/1,4-dioxane solution, and the mixture was stirred at room temperature for 23 hours. After completion of the reaction, the reaction mixture was concentrated under reduced pressure, diisopropyl ether was added to the obtained residue and ultrasonication treatment was carried out. The precipitated solid was collected by filtration and dried under reduced pressure to obtain 694 mg of the title compound as white solid substantially quantitatively.
Mass Spectrum (FAB, m/z): 467 ($M^+$+1).
$^1$H-NMR Spectrum ($CD_3OD$, δ ppm): 9.36 (d, J=1.9 Hz, 1H), 9.06 (dd, J=5.4, 1.4 Hz, 1H), 8.82 (ddd, J=8.2, 1.9, 1.4 Hz, 1H), 8.10 (ddd, J=8.2, 5.4, 0.6 Hz, 1H), 7.73 (dd, J=8.9, 7.3 Hz, 1H), 7.17-7.13 (m, 2H), 7.09-7.05 (m, 2H), 6.85 (d, J=8.9 Hz, 1H), 6.68 (d, J=7.3 Hz, 1H), 4.64 (s, 2H), 4.51 (s, 2H), 4.16 (s, 2H), 1.32 (s, 3H), 0.77-0.69 (m, 4H).
Rf value: 0.45 (n-butanol:acetic acid:water=10:1:1).

[Example 6]

(6-{[4-(1-Ethylcyclopropyl)benzyl](pyridin-2-ylsulfonyl)aminomethyl}pyridin-2-ylamino)acetic acid (Exemplary compound No. 1061)

6-(a): tert-Butyl [tert-butoxycarbonyl(6-{[4-(1-ethylcyclopropyl)benzyl](pyridin-2-ylsulfonyl)aminomethyl}pyridin-2-yl)amino]acetate

To 7.2 ml of a tetrahydrofuran solution containing 300 mg (0.627 mmol) of tert-butyl (tert-butoxycarbonyl{6-[(pyridin-2-ylsulfonyl)aminomethyl]pyridin-2-yl}-amino)acetate obtained in the same manner as in Reference example 2 were added 110 mg (0.624 mmol) of 4-(1-ethylcyclopropyl)benzyl alcohol obtained in Reference example 7, 0.30 ml (1.2 mmol) of tri-n-butylphosphine and 171 mg (0.993 mmol) of N,N,N',N'-tetramethylazodicarboxamide, and the mixture was stirred at room temperature for 17 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The obtained residue was applied to silica gel column chromatography (eluent; n-hexane:ethyl acetate=7:3 (V/V)), and the fractions containing the objective material were concentrated under reduced pressure to obtain 393 mg of the title compound as white foam. (Yield: 98%) Mass Spectrum (FAB, m/z): 637 ($M^+$+1).
$^1$H-NMR Spectrum ($CDCl_3$, δ ppm): 8.58 (ddd, J=4.7, 1.7, 1.0 Hz, 1H), 7.81 (ddd, J=7.8, 1.1, 1.0 Hz, 1H), 7.76 (ddd, J=7.8, 7.6, 1.7 Hz, 1H), 7.64 (d, J=8.4 Hz, 1H), 7.44 (dd, J=8.4, 7.4 Hz, 1H), 7.37 (ddd, J=7.6, 4.7, 1.1 Hz, 1H), 7.13-7.08 (m, 4H), 6.90 (d, J=7.4 Hz, 1H), 4.64 (s, 2H), 4.49 (s, 2H), 4.46 (s, 2H), 1.55-1.49 (m, 11H), 1.43 (s, 9H), 0.79 (t, J=7.3

Hz, 3H), 0.72-0.60 (m, 4H).

6-(b): (6-{[4-(1-Ethylcyclopropyl)benzyl](pyridin-2-ylsulfonyl)aminomethyl}pyridin-2-ylamino)acetic acid

To 3.5 ml of a methylene chloride solution containing 380 mg (0.597 mmol) of tert-butyl [tert-butoxycarbonyl(6-{[4-(1-ethylcyclopropyl)benzyl](pyridin-2-ylsulfon-yl)aminomethyl}pyridin-2-yl)amino]acetate obtained in Example 6-(a) was added 2.9 ml (12 mmol) of 4N hydrogen chloride/1,4-dioxane solution, and the mixture was stirred at room temperature for 16 hours. After completion of the reaction, the reaction mixture was adjusted to pH 11.8 by adding 1N aqueous sodium hydroxide solution, and then, the mixture was adjusted again to pH 4.5 with 1N hydrochloric acid. Precipitated solid was collected by filtration, washed with water and dried under reduced pressure to obtain 218 mg of the title compound as white solid. (Yield: 76%)
Mass Spectrum (FAB, m/z): 481 ($M^{+}$+1).
$^1$H-NMR Spectrum (DMSO-$d_6$, $\delta$ ppm): 8.61 (ddd, J=4.7, 1.7, 0.8 Hz, 1H), 7.93 (ddd, J=7.7, 7.7, 1.7 Hz, 1H), 7.77 (ddd, J=7.7, 1.0, 0.8 Hz, 1H), 7.55 (ddd, J=7.7, 4.7, 1.0 Hz, 1H), 7.18 (dd, J=8.3, 7.2 Hz, 1H), 7.13 (s, 4H), 6.72 (t, J=5.5 Hz, 0.9H), 6.33 (d, J=8.3 Hz, 1H), 6.26 (d, J=7.2 Hz, 1H), 4.63 (s, 2H), 4.21 (s, 2H), 3.81 (d, J=5.5 Hz, 2H), 1.53 (q, J=7.3 Hz, 2H), 0.76 (t, J=7.3 Hz, 3H), 0.70-0.63 (m, 4H).
Rf value: 0.44 (n-butanol:acetic acid:water=10: 1: 1).

[Example 7]

(6-{[4-(1-Ethylcyclopropyl)benzyl](pyridin-3-ylsulfonyl)aminomethyl}pyridin-2-ylamino)acetic acid (Exemplary compound No. 1066)

7-(a): tert-Butyl [tert-butoxycarbonyl(6-{[4-(1-ethylcyclopropyl)benzyl](pyridin-3-yl sulfonyl)aminomethyl}pyridin-2-yl)amino]acetate

To 72 ml of a tetrahydrofuran solution containing 3.50 g (7.31 mmol) of tert-butyl (tert-butoxycarbonyl{6-[(pyridin-3-ylsulfonyl)aminomethyl]pyridin-2-yl}amino)-acetate obtained in the same manner as in Reference example 1-(f) were added 1.42 g (8.06 mmol) of 4-(1-ethyleyclopropyl)benzyl alcohol obtained in Reference example 7, 2.7 ml (11 mmol) of tri-n-butylphosphine and 1.89 g (11.0 mmol) of N,N,N',N'-tetra-methylazodicarboxamide, and the mixture was stirred at room temperature for 2 hours. After completion of the reaction, the reaction mixture was distilled under reduced pressure, water was added to the residue, and the mixture was extracted with ethyl acetate. The organic layer was washed with a saturated aqueous sodium chloride solution, dried over anhydrous sodium sulfate and concentrated under reduced pressure. The obtained residue was applied to silica gel column chromatography (eluent; n-hexane:ethyl acetate=10:1→1:1 (V/V)), and the fractions containing the objective material were concentrated under reduced pressure to obtain 4.52 g of the title compound as pale yellow oil. (Yield: 97%)
Mass Spectrum (FAB, m/z): 637 ($M^{+}$+1).
$^1$H-NMR Spectrum ($CDCl_3$, $\delta$ ppm): 8.93 (dd, J=2.4, 0.7 Hz, 1H), 8.69 (dd, J=4.9, 1.6 Hz, 1H), 7.84 (ddd, J=8.1, 2.4, 1.6 Hz, 1H), 7.70 (d, J=8.4 Hz, 1H), 7.50 (dd, J=8.4, 7.4 Hz, 1H), 7.29 (ddd, J=8.1, 4.9, 0.7 Hz, 1H), 7.20-7.14 (m, 2H), 7.13-7.08 (m, 2H), 6.86 (d, J=7.4 Hz, 1H), 4.53 (s, 2H), 4.38 (s, 4H), 1.59-1.50 (m, 11H), 1.42 (s, 9H), 0.81 (t, J=7.4 Hz, 3H), 0.75-0.61 (m, 4H).

7-(b): (6-{[4-(1-Ethylcyclopropyl)benzyl](pyridin-3-ylsulfonyl)aminomethyl}piridin-2-ylamino)acetic acid

To 14 ml of a tetrahydrofuran solution containing 4.41 g (6.93 mmol) of tert-butyl [tert-butoxycarbonyl(6-{[4-(1-ethylcyclopropyl)benzyl](pyridin-3-ylsulfonyl)-aminomethyl}pyridin-2-yl)amino]acetate obtained in Example 7-(a) were added 14 ml of water and 6.0 ml of concentrated hydrochloric acid, and the mixture was stirred at 65°C for 3 hours. To the mixture was further added 1.2 ml of concentrated hydrochloric acid, and the mixture was stirred at 65°C for 2 hours. After completion of the reaction, the reaction mixture was adjusted to pH 4.5 by adding 6N aqueous sodium hydroxide solution, and then, tetrahydrofuran was distilled off under reduced pressure. Precipitated solid was collected by filtration, and recrystallized from 30 ml of acetone to obtain 2.47 g of the title compound as white solid. (Yield: 74%)
Mass Spectrum (FAB, m/z): 481 ($M^{+}$+1).
$^1$H-NMR Spectrum (DMSO-$d_6$, $\delta$ ppm): 12.37 (brs, 0.9H), 8.79 (dd, J=2.4, 0.8 Hz, 1H), 8.70 (dd, J=4.8, 1.5 Hz, 1H), 7.98 (ddd, J=8.0, 2.4, 1.5 Hz, 1H), 7.45 (ddd, J=8.0, 4.8, 0.8 Hz, 1H), 7.23 (dd, J=8.2, 7.1 Hz, 1H), 7.21-7.16 (m, 4H), 6.75 (t, J=5.6 Hz, 0.9H), 6.36 (d, J=8.2 Hz, 1H), 6.30 (d, J=7.1 Hz, 1H), 4.61 (s, 2H), 4.16 (s, 2H), 3.70 (d, J=5.6 Hz, 2H), 1.55 (q, J=7.3 Hz, 2H), 0.77 (t, J=7.3 Hz, 3H), 0.73-0.64 (m, 4H).
Rf value: 0.48 (n-butanol:acetic acid:water=10:1:1).

[Example 8]

(6-{[4-(1-Isopropylcyclopropyl)benzyl](pyridin-3-ylsulfonyl)aminomethyl}pyridin-2-ylamino)acetic acid (Exemplary compound No. 1102)

8-(a): tert-Butyl [tert-butoxycarbonyl(6-{[4-(1-isopropylcyclopropyl)benzyl](pyridin-3-ylsulfonyl)aminomethyl}pyridin-2-yl)amino]acetate

To 5.8 ml of a N,N-dimethylformamide solution containing 722 mg (1.51 mmol) of tert-butyl (tert-butoxycarbonyl {6-[(pyridin-3-ylsulfonyl)aminomethyl]-pyridin-2-yl}amino)acetate obtained in the same manner as in Reference example 1-(f) was added under ice-cooling 105 mg (2.41 mmol) of sodium hydride (mineral oil 55% dispersed product). After the mixture was once raised to room temperature, the mixture was again ice-cooled and 1.7 ml of a N,N-dimethylformamide solution containing 318 mg (1.26 mmol) of 4-(1-isopropylcyclopropyl)benzyl bromide obtained in Reference example 8-(c) was added to the mixture. After the mixture was raised to room temperature, and the mixture was stirred for 3 hours. After completion of the reaction, a saturated aqueous ammonium chloride solution was added to the reaction mixture, and the mixture was extracted with ethyl acetate. The organic layer was washed with a saturated aqueous sodium chloride solution, dried over anhydrous magnesium sulfate and concentrated under reduced pressure. The obtained residue was applied to silica gel column chromatography (eluent; n-hexane:ethyl acetate=1:0$\rightarrow$6:4 (V/V)), and the fractions containing the objective material were concentrated under reduced pressure to obtain 434 mg of the title compound as pale yellow foam. (Yield: 53%)
Mass Spectrum (CI, m/z): 651 ($M^+$+1).
$^1$H-NMR Spectrum ($CDCl_3$, $\delta$ ppm): 8.94 (dd, J=2.3, 0.9 Hz, 1H), 8.69 (dd, J=4.9, 1.7 Hz, 1H), 7.84 (ddd, J=8.1, 2.3, 1.7 Hz, 1H), 7.70 (d, J=8.1 Hz, 1H), 7.50 (dd, J=8.1, 7.3 Hz, 1H), 7.29 (ddd, J=8.1, 4.9, 0.9 Hz, 1H), 7.19-7.14 (m, 2H), 7.12-7.06 (m, 2H), 6.86 (d, J=7.3 Hz, 1H), 4.53 (s, 2H), 4.39 (s, 4H), 1.52 (s, 9H), 1.42 (s, 9H), 1.23-1.08 (m, 1H), 0.83 (d, J=6.8 Hz, 6H), 0.68-0.58 (m, 4H).

8-(b): (6-{[4-(1-Isopropylcyclopropyl)benzyl](pyridin-3-ylsulfonyl)aminomethyl}-pyridin-2-ylamino)acetic acid

To 1.3 ml of a tetrahydrofuran solution containing 421 mg (0.647 mmol) of tert-butyl [tert-butoxycarbonyl(6-{[4-(1-isopropylcyclopropyl)benzyl](pyridin-3-yl-sulfonyl)aminomethyl}pyridin-2-yl)amino]acetate obtained in Example 8-(a) were added 1.3 ml of water and 0.54 ml of concentrated hydrochloric acid, and the mixture was stirred at 65°C for 3 hours. After completion of the reaction, water was added to the reaction mixture, and the mixture was adjusted to pH 10.7 with 1N aqueous sodium hydroxide solution, and then, adjusted again to pH 4.5 with 1N hydrochloric acid. The mixture was extracted with ethyl acetate, the organic layer was dried over anhydrous magnesium sulfate and concentrated under reduced pressure. To the obtained reisude were added tert-butyl methyl ether and diisopropyl ether, and ultrasonication treatment was carried out. Precipitated solid was collected by filtration, and dried under reduced pressure to obtain 261 mg of the title compound as white solid. (Yield: 82%)
Mass Spectrum (FAB, m/z): 495 ($M^+$+1).
$^1$H-NMR Spectrum ($CDCl_3$, $\delta$ ppm): 9.06 (dd, J=2.4, 0.7 Hz, 1H), 8.73 (dd, J=4.9, 1.6 Hz, 1H), 7.96 (ddd, J=8.1, 2.4, 1.6 Hz, 1H), 7.37-7.28 (m, 2H), 7.18 (s, 4H), 6.51 (d, J=7.3 Hz, 1H), 6.29 (d, J=8.1 Hz, 1H), 4.60 (s, 2H), 4.36 (s, 2H), 3.88 (s, 2H), 1.23-1.08 (m, 1H), 0.83 (d, J=6.8 Hz, 6H), 0.69-0.58 (m, 4H).
Rf value: 0.49 (n-butanol:acetic acid:water=10:1:1).

[Example 9]

(6-{[4-{1-Butylcyclopropyl)benzyl](pyridin-2-ylsulfonyl)aminomethyl}pyridin-2-ylamino)acetic acid hydrochloride (Exemplary compound No. 1143)

9-(a): tert-Butyl [tert-butoxycarbonyl(6-{[4-(1-butylcyclopropyl)benzyl](pyridin-2-yl-sulfonyl)aminomethyl}pyridin-2-yl)amino] acetate

To 4.8 ml of a tetrahydrofuran solution containing 200 mg (0.418 mmol) of tert-butyl (tert-butoxycarbonyl{6-[(pyridin-2-ylsulfonyl)aminomethyl]pyridin-2-yl}-amino)acetate obtained in the same manner as in Reference example 2 were added 86 mg (0.42 mmol) of 4-(1-butylcyclopropyl)benzyl alcohol obtained in Reference example 9-(d), 0.20 ml (0.80 mmol) of tri-n-butylphosphine and 114 mg (0.662 mmol) of N,N,N',N'-tetramethylazodicarboxamide, and the mixture was stirred at room temperature for 6 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The obtained

residue was applied to silica gel column chromatography (eluent; n-hexane:ethyl acetate=7:3 (V/V)), and the fractions containing the objective material were concentrated under reduced pressure to obtain 235 mg of the title compound as colorless oily product.
(Yield: 84%)
Mass Spectrum (FAB, m/z): 665 ($M^+$+1).
$^1$H-NMR Spectrum ($CDCl_3$, δ ppm): 8.58 (ddd, J=4.7, 1.7, 1.0 Hz, 1H), 7.81 (ddd, J=7.8, 1.2, 1.0 Hz, 1H), 7.75 (ddd, J=7.8, 7.6, 1.7 Hz, 1H), 7.63 (d, J=8.3 Hz, 1H), 7.43 (dd, J=8.3, 7.5 Hz, 1H), 7.37 (ddd, J=7.6, 4.7, 1.2 Hz, 1H), 7.11 (s, 4H), 6.90 (d, J=7.5 Hz, 1H), 4.64 (s, 2H), 4.49 (s, 2H), 4.46 (s, 2H), 1.54-1.47 (m, 2H), 1.52 (s, 9H), 1.43 (s, 9H), 1.28-1.14 (m, 4H), 0.83 (t, J=7.2 Hz, 3H), 0.72-0.60 (m, 4H).

9-(b): (6-{[4-(1-Butylcyclopropyl)benzyl](pyridin-2-ylsulfonyl)aminomethyl}pyridin-2-ylamino)acetic acid hydrochloride

To 2.1 ml of a methylene chloride solution containing 230 mg (0.346 mmol) of tert-butyl [tert-butoxycarbonyl(6-{[4-(-butylcyclopropyl)benzyl](pyridin-2-ylsulfon-yl)aminomethyl}pyridin-2-yl)amino]acetate obtained in Example 9-(a) was added 1.7 ml (6.8 mmol) of 4N hydrogen chloride/1,4-dioxane solution, and the mixture was stirred at room temperature for 15 hours. After completion of the reaction, the reaction mixture was concentrated under reduced pressure, diisopropyl ether was added to the obtained residue, and ultrasonication treatment was carried out. After removing the solvent under reduced pressure, the residue was dried under reduced pressure to obtain 197 mg of the title compound as white foam substantially quantitatively.
Mass Spectrum (FAB, m/z): 509 ($M^+$+1).
$^1$H-NMR Spectrum ($CD_3OD$, δ ppm): 8.73 (ddd, J=4.8, 1.6, 1.0 Hz, 1H), 8.08 (ddd, J=7.7, 7.7, 1.6 Hz, 1H), 8.04 (ddd, J=7.7, 1.1, 1.0 Hz, 1H), 7.70-7.65 (m, 2H), 7.14-7.08 (m, 4H), 6.81 (d, J=9.0 Hz, 1H), 6.63 (d, J=7.3 Hz, 1H), 4.72 (s, 2H), 4.48 (s, 2H), 4.15 (s, 2H), 1.53-1.46 (m, 2H), 1.29-1.20 (m, 2H), 1.19-1.12 (m, 2H), 0.83 (t, J=7.3 Hz, 3H), 0.65-0.62 (m, 4H).
Rf value: 0.48 (n-butanol:acetic acid:water=10:1:1).

[Example 10]

(6-{[4-(1-Butylcyclopropyl)benzyl](pyridin-3-ylsulfonyl)aminomethyl}pyridin-2-ylamino)acetic acid hydrochloride (Exemplary compound No. 1152)

10-(a): tert-Butyl [tert-butoxycarbonyl(6-{[4-(1-butylcyclopropyl)benzyl](pyridin-3-yl-sulfonyl)aminomethyl }pyridin-2-yl) amino acetate

To 4.8 ml of a tetrahydrofuran solution containing 200 mg (0.418 mmol) of tert-butyl (tert-butoxycarbonyl{6-[(pyridin-3-ylsulfonyl)aminomethyl]pyridin-2-yl}-amino)acetate obtained in the same manner as in Reference example 1-(f) were added 86 mg (0.42 mmol) of 4-(1-butylcyclopropyl)benzyl alcohol obtained in Reference example 9-(d), 0.20 ml (0.80 mmol) of tri-n-butylphosphine and 114 mg (0.662 mmol) of N,N,N',N'-tetramethylazodicarboxamide, and the mixture was stirred at room temperature for 14 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The obtained residue was applied to silica gel column chromatography (eluent; n-hexane:ethyl acetate=1:2 (V/V)), and the fractions containing the objective material were concentrated under reduced pressure to obtain 253 mg of the title compound as colorless oily product.
(Yield: 91 %)
Mass Spectrum (FAB, m/z): 665 ($M^+$+1).
$^1$H-NMR Spectrum ($CDCl_3$, δ ppm): 8.93 (dd, J=2.3, 0.7 Hz, 1H), 8.69 (dd, J=4.8, 1.7 Hz, 1H), 7.84 (ddd, J=8.0, 2.3, 1.7 Hz, 1H), 7.70 (d, J=8.4 Hz, 1H), 7.50 (dd, J=8.4, 7.4 Hz, 1H), 7.29 (ddd, J=8.0, 4.8, 0.7 Hz, 1H), 7.19-7.15 (m, 2H), 7.12-7.09 (m, 2H), 6.85 (d, J=7.4 Hz, 1H), 4.53 (s, 2H), 4.38 (s, 4H), 1.53-1.49 (m, 2H), 1.53 (s, 9H), 1.43 (s, 9H), 1.27-1.15 (m, 4H), 0.83 (t, J=7.1 Hz, 3H), 0.75-0.62 (m, 4H).

10-(b): (6-{[4-(1-Butylcyclopropyl)benzyl](pyridin-3-ylsulfonyl)aminomethyl}pyridin-2-ylamino)acetic acid hydrochloride

To 2.1 ml of a methylene chloride solution containing 235 mg (0.353 mmol) of tert-butyl [tert-butoxycarbonyl(6-{[4-(1-butylcyclopropyl)benzyl](pyridin-3-ylsulfon-yl)aminomethyl}pyridin-2-yl)amino]acetate obtained in Example 10-(a) was added 2.6 ml (10 mmol) of 4N hydrogen chloride/1,4-dioxane solution, and the mixture was stirred at room temperature for 15 hours, and further stirred at 40°C for 3 hours. After completion of the reaction, the reaction mixture was concentrated under reduced pressure, methylene chloride was added to the obtained residue, and ultrasonication

treatment was carried out. Precipitated solid was collected by filtration, and dried under reduced pressure to obtain 171 mg of the title compound as white solid. (Yield: 83%) Mass Spectrum (FAB, m/z): 509 ($M^+$+1).
$^1$H-NMR Spectrum ($CD_3OD$, δ ppm): 9.32 (dd, J=2.2, 0.6 Hz, 1H), 9.03 (dd, J=5.3, 1.5 Hz, 1H), 8.75 (ddd, J=8.2,2.2, 1.5 Hz, 1H), 8.03 (ddd, J=8.2, 5.3, 0.6 Hz, 1H), 7.70 (dd, J=9.0, 7.5 Hz, 1H), 7.18-7.15 (m, 2H), 7.14-7.11 (m, 2H), 6.84 (d, J=9.0 Hz, 1H), 6.63 (d, J=7.5 Hz, 1H), 4.63 (s, 2H), 4.52 (s, 2H), 4.17 (s, 2H), 1.53-1.48 (m, 2H), 1.29-1.20 (m, 2H), 1.19-1.11 (m, 2H), 0.83 (t, J=7.3 Hz, 3H), 0.66-0.63 (m, 4H).
Rf value: 0.52 (n-butanol:acetic acid:water=10:1:1).

[Example 11]

(6-{[4-(1-Nonylcyclopropyl)benzyl]pyridin-3-ylsulfonyl)aminomethyl}pyridin-2-ylamino)acetic acid hydrochloride (Exemplary compound No. 1227)

11-(a): tert-Butyl [tert-butoxycarbonyl(6-{[4-(1-nonylcyclopropyl)benzyl](pyridin-3-ylsulfonyl)aminomethyl}pyridin-2-yl)amino]acetate

To 1.8 ml of a tetrahydrofuran solution containing 177 mg (0.370 mmol) of tert-butyl (tert-butoxycarbonyl{6-[(pyridin-3-ylsulfonyl)aminomethyl]pyridin-2-yl}-amino)acetate obtained in the same manner as in Reference example 1-(f) were added 100 mg (0.364 mmol) of 4-(1-nonylcyclopropyl)benzyl alcohol obtained in Reference example 10-(d), 0.14 ml (0.56 mmol) of tri-n-butylphosphine and 99.7 mg (0.579 mmol) of N,N,N',N'-tetramethylazodicarboxamide, and the mixture was stirred at room temperature for 1 hour. After completion of the reaction, the reaction mixture was concentrated under reduced pressure, water was added to the residue, and the mixture was extracted with ethyl acetate. The organic layer was washed with a saturated aqueous sodium chloride solution, dried over anhydrous sodium sulfate and concentrated under reduced pressure. The obtained residue was applied to silica gel column chromatography (eluent; n-hexane:ethyl acetate=1:0→1:1 (V/V)), and the fractions containing the objective material were concentrated under reduced pressure to obtain 244 mg of the title compound as pale yellow oil. (Yield: 91 %)
Mass Spectrum (FAB, m/z): 735 ($M^+$+1).
$^1$H-NMR Spectrum ($CDCl_3$, δ ppm): 8.93 (d, J=2.3 Hz, 1H), 8.69 (dd, J=5.0, 1.7 Hz, 1H), 7.83 (ddd, J=8.1, 2.3, 1.7 Hz, 1H), 7.70 (d, J=8.4 Hz, 1H), 7.50 (dd, J=8.4, 7.4 Hz, 1H), 7.28 (dd, J=8.1, 5.0 Hz, 1H), 7.19-7.14 (m, 2H), 7.13-7.08 (m, 2H), 6.85 (d, J=7.4 Hz, 1H), 4.53 (s, 2H), 4.38 (s, 4H), 1.57-1.46 (m, 11H), 1.43 (s, 9H), 1.31-1.17 (m, 14H), 0.86 (t, J=7.1 Hz, 3H), 0.74-0.62 (m, 4H).

11-(b): (6-{[4-(1-Nonylcyclopropyl)benzyl](pyridin-3-ylsulfonyl)aminomethyl}pyridin-2-ylamino)acetic acid hydrochloride

To 3.3 ml of a methylene chloride solution containing 240 mg (0.327 mmol) of tert-butyl [tert-butoxycarbonyl(6-{[4-(1-nonylcyclopropyl)benzyl](pyridin-3-ylsulfonyl)aminomethyl}pyridin-2-yl)amino]acetate obtained in Example 11-(a) was added 3.3 ml (13 mmol) of 4N hydrogen chloride/1,4-dioxane solution, and the mixture was stirred at room temperature for 19 hours. To the mixture was further added 3.3 ml (13 mmol) of 4N hydrogen chloride/1,4-dioxane solution, and the mixture was stirred at room temperature for 5 hours. After completion of the reaction, the reaction mixture was concentrated under reduced pressure, and acetone was added to the obtained residue. After removing the solvent under reduced pressure, the residue was dried under reduced pressure to obtain 197 mg of the title compound as white solid substantially quantitatively.
Mass Spectrum (FAB, m/z): 579 ($M^+$+1).
$^1$H-NMR Spectrum ($CD_3OD$, δ ppm): 9.18 (dd, J=2.2, 0.6 Hz, 1H), 8.94 (dd, J=5.1, 1.5 Hz, 1H), 8.52 (ddd, J=8.1, 2.2, 1.5 Hz, 1H), 7.84 (ddd, J=8.1, 5.1, 0.6 Hz, 1H), 7.68 (dd, J=8.8, 7.4 Hz, 1H), 7.16-7.10 (m, 4H), 6.83 (d, J=8.8 Hz, 1H), 6.60 (d, J=7.4 Hz, 1H), 4.56 (s, 2H), 4.47 (s, 2H), 4.16 (s, 2H), 1.53-1.46 (m, 2H), 1.34-1.11 (m, 14H), 0.88 (t, J=7.1 Hz, 3H), 0.67-0.62 (m, 4H).
Rf value: 0.56 (n-butanol:acetic acid:water=10:1:1).

[Example 12]

(6-{[4-1-Methoxycyclopropyl)benzyl](pyridin-2-ylsulfonyl)aminomethyl}pyridin-2-ylamino)acetic acid (Exemplary compound No. 1248)

12-(a): tert-But [tert-butoxycarbonyl(6-{[4-(1-methoxycyclopropyl)benzyl](pyridin-2-ylsulfonyl)aminomethyl}pyridin-2-yl)amino acetate

To 6.9 ml of a tetrahydrofuran solution containing 286 mg (0.598 mmol) of tert-butyl (tert-butoxycarbonyl{6-[(pyridin-2-ylsulfonyl)aminomethyl]pyridin-2-yl}-amino)acetate obtained in the same manner as in Reference example 2 were added 107 mg (0.600 mmol) of 4-(1-methoxycyclopropyl)benzyl alcohol obtained in Reference example 11, 0.28 ml (1.1 mmol) of tri-n-butylphosphine and 163 mg (0.947 mmol) of N,N,N',N'-tetramethylazodicarboxamide, and the mixture was stirred at room temperature for 17 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The obtained residue was applied to silica gel column chromatography (eluent; n-hexane:ethyl acetate=3:2 (V/V)), and the fractions containing the objective material were concentrated under reduced pressure to obtain 361 mg of the title compound as colorless oily product.
(Yield: 94%)
Mass Spectrum (FAB, m/z): 639 ($M^+$+1).
$^1$H-NMR Spectrum ($CDCl_3$, δ ppm): 8.59 (ddd, J=4.7, 1.7, 1.0 Hz, 1H), 7.82 (ddd, J=7.7, 1.2, 1.0 Hz, 1H), 7.77 (ddd, J=7.7, 7.6, 1.7 Hz, 1H), 7.64 (d, J=8.2 Hz, 1H), 7.43 (dd, J=8.2, 7.5 Hz, 1H), 7.38 (ddd, J=7.6, 4.7, 1.2 Hz, 1H), 7.21-7.18 (m, 2H), 7.17 - 7.13 (m, 2H), 6.89 (d, J=7.5 Hz, 1H), 4.68 (s, 2H), 4.48 (s, 2H), 4.45 (s, 2H), 3.19 (s, 3H), 1.52 (s, 9H), 1.43 (s, 9H), 1.21-1.10 (m, 2H), 0.98-0.85 (m, 2H).

12-(b): <u>(6-{[4-(1-Methoxycyclopropyl)benzyl](pyridin-2-ylsulfonyl)aminomethyl}-pyridin-2-ylamino)acetic acid</u>

To 3.2 ml of a methanol solution containing 336 mg (0.526 mmol) of tert-butyl [tert-butoxycarbonyl(6-{[4-(1-methoxycyclopropyl)benzyl](pyridin-2-ylsulfonyl)-aminomethyl}pyridin-2-yl)amino]acetate obtained in Example 12-(a) was added 2.6 ml (10 mmol) of 4N hydrogen chloride/1,4-dioxane solution, and the mixture was stirred at room temperature for 18 hours. After completion of the reaction, the reaction mixture was concentrated under reduced pressure, 2 ml of methanol and 2.1 ml of 1N aqueous sodium hydroxide solution were added to the obtained residue, and the mixture was stirred at room temperature for 6 hours. Then, water was added to the mixture to make the whole amount 30 ml, and the resulting mixture was adjusted to pH 4.4 with 1N hydrochloric acid. Precipitated solid was collected by filtration, washed with water and dried under reduced pressure to obtain 180 mg of the title compound as white solid.
(Yield: 71 %)
Mass Spectrum (FAB, m/z): 483 ($M^+$+1).
$^1$H-NMR Spectrum (DMSO-$d_6$, δ ppm): 12.38 (brs, 0.4H), 8.63-8.60 (m, 1H), 7.94 (ddd, J=7.7, 7.7, 1.7 Hz, 1H), 7.80-7.76 (m, 1H), 7.56 (ddd, J=7.7, 4.6, 1.1 Hz, 1H), 7.22-7.13 (m, 5H), 6.74 (t, J=5.8 Hz, 0.9H), 6.34 (d, J=8.3 Hz, 1H), 6.27 (d, J=7.2 Hz, 1H), 4.66 (s, 2H), 4.22 (s, 2H), 3.82 (d, J=5.8 Hz, 2H), 3.12 (s, 3H), 1.17-1.05 (m, 2H), 0.97-0.85 (m, 2H).
Rf value: 0.33 (n-butanol:acetic acid:water=10:1:1).

[Example 13]

<u>(6-{[4-(1-Ethylcyclobutyl)benzyl](pyridin-3-ylsulfonyl)aminomethyl}pyridin-2-ylamino)acetic acid</u> (Exemplary compound No. 1398)

13-(a): <u>tert-Butyl [tert-butoxycarbonyl(6-{[4-(-ethylcyclobutyl)benzyl](pyridin-3-yl-sulfonyl)aminomethyl} pyridin-2-yl)amino]</u> acetate

To 0.91 ml of a tetrahydrofuran solution containing 91.4 mg (0.191 mmol) of tert-butyl (tert-butoxycarbonyl{6-[(pyridin-3-ylsulfonyl)aminomethyl]pyridin-2-yl}-amino)acetate obtained in the same manner as in Reference example 1-(f) were added 39.9 mg (0.210 mmol) of 4-(1-ethylcyclobutyl)benzyl alcohol obtained in Reference example 12-(d), 72 μl (0.29 mmol) of tri-n-butylphosphine and 49.4 mg (0.287 mmol) of N,N,N',N'-tetramethylazodicarboxamide, and the mixture was stirred at room temperature for 16 hours. After completion of the reaction, water was added to the reaction mixture and the mixture was extracted with ethyl acetate. The organic layer was dried over anhydrous magnesium sulfate and concentrated under reduced pressure. The obtained residue was applied to silica gel column chromatography (eluent; n-hexane:ethyl acetate=1:0→3:1 (V/V)), and the fractions containing the objective material were concentrated under reduced pressure to obtain 121 mg of the title compound as white foam. (Yield: 97%)
Mass Spectrum (CI, m/z): 651 ($M^+$+1).
$^1$H-NMR Spectrum ($CDCl_3$, δ ppm): 8.95 (dd, J=2.4, 0.8 Hz, 1H), 8.69 (dd, J=4.9, 1.7 Hz, 1H), 7.85 (ddd, J=8.0, 2.4, 1.7 Hz, 1H), 7.71 (d, J=8.2 Hz, 1H), 7.51 (dd, J=8.2, 7.2 Hz, 1H), 7.30 (ddd, J=8.0, 4.9, 0.8 Hz, 1H), 7.15-7.09 (m, 2H), 7.01-6.95 (m, 2H), 6.86 (d, J=7.2 Hz, 1H), 4.53 (s, 2H), 4.40 (s, 2H), 4.39 (s, 2H), 2.33-2.19 (m, 2H), 2.14-1.96 (m, 3H), 1.86-1.70 (m, 1H), 1.75 (q, J=7.3 Hz, 2H), 1.52 (s, 9H), 1.43 (s, 9H), 0.60 (t, J=7.3 Hz, 3H).

13-(b): 6-{[4-(1 -Ethylcyclobutyl)benzyl](pyridin-3-ylsulfonyl)aminomethyl}pyridin-2-ylamino)acetic acid

To 0.32 ml of a tetrahydrofuran solution containing 102 mg (0.157 mmol) of tert-butyl [tert-butoxycarbonyl(6-{[4-(1-ethylcyclobutyl)benzyl](pyridin-3-ylsulfonyl)-aminomethyl}pyridin-2-yl)amino]acetate obtained in Example 13-(a) were added 0.32 ml of water and 0.13 ml of concentrated hydrochloric acid, and the mixture was stirred at 65°C for 2 hours. After completion of the reaction, water was added to the reaction mixture, and the mixture was adjusted to pH 11.3 with 1N aqueous sodium hydroxide solution, and then, adjusted again to pH 4.3 with 1N hydrochloric acid. The mixture was extracted with ethyl acetate, and the organic layer was dried over anhydrous magnesium sulfate and concentrated under reduced pressure. To the obtained residue were added tert-butyl methyl ether and diisopropyl ether, and ultrasonication treatment was carried out. Precipitated solid was collected by filtration, and dried under reduced pressure to obtain 54.2 mg of the title compound as white solid. (Yield: 70%)
Mass Spectrum (FAB, m/z): 495 ($M^+$+1).
$^1$H-NMR Spectrum ($CDCl_3$, δ ppm): 9.06 (s, 1H), 8.74 (s, 1H), 7.96 (d, J=8.3 Hz, 1H), 7.39-7.17 (m, 4H), 7.04-6.96 (m, 2H), 6.47 (d, J=7.1 Hz, 1H), 6.27 (d, J=8.3 Hz, 1H), 4.62 (s, 2H), 4.35 (s, 2H), 3.85 (s, 2H), 2.34-2.19 (m, 2H), 2.14-1.96 (m, 3H), 1.86-1.69 (m, 1H), 1.75 (q, J=7.3 Hz, 2H), 0.60 (t, J=7.3 Hz, 3H).
Rf value: 0.49 (n-butanol:acetic acid:water=10:1:1).

[Example 14]

(6-{[4-(1-Ethylcyclopropyl)benzyl](4-fluorobenzenesulfonyl)aminomethyl}pyridin-2-ylamino)acetic acid (Exemplary compound No. 1046)

14-(a): tert-Butyl [tert-butoxycarbonyl(6-{[4-(1-ethylcyclopropyl)benzyl](4-fluorobenzenesulfonyl)aminomethyl}pyridin-2-yl)amino]acetate

To 25 ml of a tetrahydrofuran solution containing 1.23 g (2.48 mmol) of tert-butyl (tert-butoxycarbonyl{6-[(4-fluorobenzenesulfonyl)aminomethyl]pyridin-2-y}-amino)acetate obtained in Reference example 3 were added 435 mg (2.47 mmol) of 4-(1-ethylcyclopropyl)benzyl alcohol obtained in Reference example 7, 920 μl (3.73 mmol) of tri-n-butylphosphine and 641 mg (3.72 mmol) of N,N,N',N'-tetramethyl-azodicarboxamide, and the mixture was stirred at room temperature for 3 hours. After completion of the reaction, the reaction mixture was concentrated under reduced pressure, water was added to the residue, and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=5:1→4:1 (V/V)), and the fractions containing the objective material were concentrated under reduced pressure to obtain 1.32 g of the title compound. (Yield: 82%)
Mass Spectrum (CI, m/z): 654 ($M^+$+1).
$^1$H-NMR Spectrum ($CDCl_3$, δ ppm): 7.72-7.65 (m, 3H), 7.49 (dd, J=8.3, 7.5 Hz, 1H), 7.18-7.03 (m, 6H), 6.85 (dd, J=7.5, 0.5 Hz, 1H), 4.46 (s, 2H), 4.41 (s, 2H), 4.34 (s, 2H), 1.58-1.50 (m, 2H), 1.52 (s, 9H), 1.42 (s, 9H), 0.80 (t, J=7.3 Hz, 3H), 0.74-0.68 (m, 2H), 0.67-0.61 (m, 2H).

14-(b): (6-{[4-(1-Ethylcyclopropyl)benzyl](4-fluorobenzenesulfonyl)aminomethyl}-pyridin-2-ylamino)acetic acid

To 12 ml of a tetrahydrofuran solution containing 1.31 g (2.00 mmol) of tert-butyl [tert-butoxycarbonyl(6-{[4-(1-ethylcyclopropyl)benzyl](4-fluorobenzenesulfonyl)aminomethyl}pyridin-2-yl)amino]acetate obtained in Example 14-(a) were added 6 ml of water and 3.4 ml of concentrated hydrochloric acid, and the mixture was stirred at 65°C for 11 hours. After completion of the reaction, 30 ml of water was added to the reaction mixture, and the mixture was adjusted to pH 4.5 with 2N aqueous sodium hydroxide solution and extracted with ethyl acetate. The organic layer was washed with a saturated aqueous sodium chloride solution, dried over anhydrous magnesium sulfate and concentrated under reduced pressure. To the residue was added 30 ml of a mixed solvent (ethyl acetate:diisopropyl ether=1:1 (V/V)), and precipitated solid was collected by filtration and dried at 60°C under reduced pressure to obtain 896 mg of the title compound as white solid. (Yield: 90%)
Mass Spectrum (FAB, m/z): 498 ($M^+$+1).
$^1$H-NMR Spectrum (DMSO-$d_6$, δ ppm): 7.75-7.67 (m, 2H), 7.30-7.12 (m, 7H), 6.78 (t, J=5.5 Hz, 1H), 6.37 (d, J=8.3 Hz, 1H), 6.28 (d, J=7.1 Hz, 1H), 4.54 (s, 2H), 4.12 (s, 2H), 3.75 (d, J=5.5 Hz, 2H), 1.55 (q, J=7.3 Hz, 2H), 0.77 (t, J=7.3 Hz, 3H), 0.74-0.61 (m, 4H).
Rf value: 0.70 (n-butanol:acetic acid:water=10:1:1).

[Example 15]

(6-{[4-(1-Butylcyclopropyl)benzyl](1-methyl-1H-imidazol-4-ylsulfonyl)aminomethyl}-pyridin-2-ylamino)acetic acid (Exemplary compound No. 1138)

15-(a): tert-Butyl [tert-butoxycarbonl(6-{[4-(1-butylcyclopropyl)benzyl](1-methyl-1H-imidazol-4-ylsulfonyl)aminomethyl}pyridin-2-yl)amino]acetate

To 6 ml of a methylene chloride solution containing 307 mg (0.586 mmol) of tert-butyl [tert-butoxycarbonyl(6-{[4-(1-butylcyclopropyl)benzyl]aminomethyl}pyridin-2-yl)amino]acetate obtained in Reference example 13 were added uncer ice-cooling 163 $\mu$l (1.17 mmol) of triethylamine and 127 mg (0.703 mmol) of 1-methyl-1H-imidazol-4-ylsulfonyl chloride, and the mixture was stirred at the same temperature for 2.5 hours and at room temperature for 6 hours. After completion of the reaction, water was added to the reaction mixture, and the mixture was extracted with methylene chloride. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=3:7$\rightarrow$1:3 (V/V)), and the fractions containing the objective material were concentrated under reduced pressure to obtain 293 mg of the title compound as colorless oily product. (Yield: 75%)
Mass Spectrum (CI, m/z): 668 ($M^+$+1).
$^1$H-NMR Spectrum ($CDCl_3$, $\delta$ ppm): 7.65 (d, J=8.2 Hz, 1H), 7.46 (dd, J=8.2, 7.4 Hz, 1H), 7.38 (d, J=1.2 Hz, 1H), 7.18 (d, J=1.2 Hz, 1H), 7.15 (s, 4H), 6.96 (d, J=7.4 Hz, 1H), 4.58 (s, 2H), 4.50 (s, 2H), 4.38 (s, 2H), 3.67 (s, 3H), 1.55-1.47 (m, 2H), 1.52 (s, 9H), 1.43 (s, 9H), 1.26-1.15 (m, 4H), 0.83 (t, J=6.8 Hz, 3H), 0.74-0.68 (m, 2H), 0.66-0.59 (m, 2H).

15-(b): (6-{[4-(1-Butylcyclopropyl)benzyl](1-methyl-1H-imidazol-4-ylsulfonyl)aminomethyl}pyridin-2-ylamino)acetic acid

To 1.4 ml of a tetrahydrofuran solution containing 290 mg (0.434 mmol) of tert-butyl [tert-butoxycarbonyl(6-{[4-(1-butylcyclopropyl)benzyl](1-methyl-1H-imidazol-4-ylsulfonyl)aminomethyl}pyridin-2-yl)amino]acetate obtained in Example 15-(a) were added 1.0 ml of water and 0.37 ml of concentrated hydrochloric acid, and the mixture was stirred at 60°C for 3 hours. After completion of the reaction, the reaction mixture was concentrated under reduced pressure, the residue was adjusted to pH 4.8 with 1N aqueous sodium hydroxide solution and 2N hydrochloric acid, and the mixture was extracted with ethyl acetate. The organic layer was washed with a saturated aqueous sodium chloride solution, dried over anhydrous magnesium sulfate and concentrated under reduced pressure to obtain 191 mg of the title compound as white foam. (Yield: 86%)
Mass Spectrum (FAB, m/z): 512 ($M^+$+1).
$^1$H-NMR Spectrum (DMSO-$d_6$, $\delta$ ppm): 7.74 (d, J=1.0 Hz, 1H), 7.60 (d, J=1.0 Hz, 1H), 7.22 (dd, J=8.1, 7.4 Hz, 1H), 7.14 (s, 4H), 6.80-6.68 (m, 1H), 6.36 (d, J=8.1 Hz, 1H), 6.33 (d, J=7.4 Hz, 1H), 4.43 (s, 2H), 4.09 (s, 2H), 3.86 (d, J=4.9 Hz, 2H), 3.66 (s, 3H), 1.56-1.46 (m, 2H), 1.28-1.07 (m, 4H), 0.79 (t, J=7.0 Hz, 3H), 0.73-0.59 (m, 4H). Rf value: 0.14 (n-butanol:acetic acid:water=10:1:1).

[Example 16]

(6-{[4-(1-Butylcyclopropyl)benzyl](pyridm-4-ylsulfonyl)aminomethyl}pyridin-2-yl-amino)acetic acid (Exemplary compound No. 1161)

16-(a): tert-Butyl[tert-butoxycarbonyl(6-{[4-(1-butylcyclopropyl)benzyl](pyridin-4-ylsulfonyl)aminomethyl}pyridin-2-yl)amino]acetate

To 2.6 ml of a tetrahydrofuran solution containing 94.2 mg (0.273 mmol) of N-[4-(1-butylcyclopropyl)benzyl]pyridin-4-ylsulfonamide obtained in Reference example 14-(d) were added 90.2 mg (2.67 mmol) of tert-butyl [tert-butoxycarbonyl(6-hydroxy-methylpyridin-2-yl)amino]acetate obtained in Reference example 1-(b), 99 $\mu$l (0.40 mmol) of tri-n-butylphosphine and 71.7 mg (0.416 mmol) of N,N,N',N'-tetramethyl-azodicarboxamide, and the mixture was stirred at room temperature for 15 hours. After completion of the reaction, the reaction mixture was concentrated under reduced pressure, water was added to the residue, and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane: ethyl acetate=2: 1 $\rightarrow$ 1:1 1 (V/V)), and the fractions containing the obj ective material were concentrated under reduced pressure to obtain 172 mg of the title compound as pale yellow oil. (Yield: 95%)
Mass Spectrum (CI, m/z): 665 ($M^+$+1).
$^1$H-NMR Spectrum ($CDCl_3$, $\delta$ ppm): 8.70 (dd, J=4.4, 1.7 Hz, 2H), 7.73 (d, J=8.6 Hz, 1H), 7.50 (dd, J=8.6, 7.5 Hz, 1H),

7.47 (dd, J=4.4, 1.7 Hz, 2H), 7.21-7.05 (m, 4H), 6.83 (d, J=7.5 Hz, 1H), 4.52 (s, 2H), 4.38 (s, 2H), 4.33 (s, 2H), 1.54-1.46 (m, 2H), 1.53 (s, 9H), 1.43 (s, 9H), 1.26-1.16 (m, 4H), 0.83 (t, J=6.8 Hz, 3H), 0.75-0.70 (m, 2H), 0.68-0.62 (m, 2H).

16-(b): (6-{[4-(1-Butylcyclopropyl)benzyl](pyridin-4-ylsulfonyl)aminomethyl}pyridin-2-ylamino)acetic acid

To 0.71 ml of a tetrahydrofuran solution containing 168 mg (0.253 mmol) of tert-butyl [tert-butoxycarbonyl(6-{[4-(1-butylcyclopropyl)benzyl](pyridin-4-ylsulfon-yl)aminomethyl}pyridin-2-yl)amino]acetate obtained in Example 16-(a) were added 0.49 ml of water and 0.21 ml of concentrated hydrochloric acid, and the mixture was stirred at 60°C for 5 hours. After completion of the reaction, 1 ml of water was added to the reaction mixture, and the mixture was concentrated under reduced pressure. The residue was adjusted to pH 4.2 with 1N aqueous sodium hydroxide solution and 1N hydrochloric acid, and extracted with ethyl acetate. The organic layer was washed with a saturated aqueous sodium chloride solution, dried over anhydrous magnesium sulfate and concentrated under reduced pressure. To the residue were added 2.4 ml of acetone and 9.6 ml of diisopropyl ether, precipitated solid was collected by filtration, and dried at 50°C under reduced pressure to obtain 107 mg of the title compound as white solid. (Yield: 83%)
Mass Spectrum (FAB, m/z): 509 ($M^+$+1).
$^1$H-NMR Spectrum (DMSO-$d_6$, δ ppm): 8.66 (dd, J=4.4, 1.7 Hz, 2H), 7.53 (dd, J=4.4, 1.7 Hz, 2H), 7.23-7.16 (m, 5H), 6.81-6.75 (m, 0.8H), 6.36 (dd, J=8.3, 0.5 Hz, 1H), 6.28 (dd, J=7.2, 0.5 Hz, 1H), 4.63 (s, 2H), 4.16 (s, 2H), 3.70 (d, J=5.7 Hz, 2H), 1.55-1.50 (m, 2H), 1.26-1.12 (m, 4H), 0.80 (t, J=7.2 Hz, 3H), 0.73-0.63 (m, 4H).
Rf value: 0.55 (n-butanol:acetic acid:water=10:1:1).

[Example 17]

(6-{[4-(1-Butylcyclobutyl)benzyl](pyridin-2-ylsulfonyl)aminomethyl}pyridin-2-ylamino)acetic acid (Exemplary compound No. 1419)

17-(a): tert-Butyl[tert-butoxycarbonyl(6-{[4-(1-butylcyclobutyl)benzyl]{pyridin-2-yl-sulfonyl)aminomethyl}pyridin-2-yl)amino]acetate

To 5 ml of a tetrahydrofuran solution containing 427 mg (0.892 mmol) of tert-butyl (tert-butoxycarbonyl {6-[(pyridin-2-ylsulfonyl)aminomethyl]pyridin-2-yl}amino)-acetate obtained in the same manner as in Reference example 2 were added 0.15 g (0.69 mmol) of 4-(1-butylcyclobutyl)benzyl alcohol obtained in Reference example 15-(d), 343 μl (1.37 mmol) of tri-n-butylphosphine and 237 mg (1.37 mmol) of N,N,N',N'-tetramethylazodicarboxamide, and the mixture was stirred at room temperature for 3 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=4:1→2:1 (V/V)), and the fractions containing the objective material were concentrated under reduced pressure to obtain 0.29 g of the title compound as yellow oil. (Yield: 62%)
Mass Spectrum (CI, m/z): 679 ($M^+$+1).
$^1$H-NMR Spectrum ($CDCl_3$, δ ppm): 8.58 (ddd, J=4.6, 1.7, 1.1 Hz, 1H), 7.82 (ddd, J=7.8, 1.3, 1.1 Hz, 1H), 7.76 (ddd, J=7.8, 7.5, 1.7 Hz, 1H), 7.64 (d, J=8.3 Hz, 1H), 7.44 (dd, J=8.3, 7.6 Hz, 1H), 7.37 (ddd, J=7.5, 4.6, 1.3 Hz, 1H), 7.15-7.09 (m, 2H), 6.96-6.89 (m, 3H), 4.64 (s, 2H), 4.50 (s, 2H), 4.47 (s, 2H), 2.32-2.18 (m, 2H), 2.13-1.98 (m, 2H), 1.84-1.34 (m, 4H), 1.52 (s, 9H), 1.43 (s, 9H), 1.28-1.12 (m, 2H), 1.04-0.87 (m, 2H), 0.81 (t, J=7.3 Hz, 3H).

17-(b): (6-{[4-(1-Butylcyclobutyl)benzyl](pyridin-2-ylsulfonyl)aminomethyl}pyridin-2-ylamino)acetic acid

To 4 ml of a methylene chloride solution containing 0.28 g (0.41 mmol) of tert-butyl [tert-butoxycarbonyl(6-{[4-(1-butylcyclobutyl)benzyl](pyridin-2-ylsulfonyl)-aminomethyl}pyridin-2-yl)amino]acetate obtained in Example 17-(a) was added 4.0 ml (54 mmol) of trifluoroacetic acid, and the mixture was stirred at room temperature for 8 hours. After completion of the reaction, the reaction mixture was concentrated under reduced pressure. Water was added to the residue, the mixture was adjusted to pH 4.5 with 1N aqueous sodium hydroxide solution and 1N hydrochloric acid, and extracted with ethyl acetate. The organic layer was washed with a saturated aqueous sodium chloride solution, dried over anhydrous magnesium sulfate and concentrated under reduced pressure. The residue was applied to silica gel column chromatography (eluent; ethyl acetate:methanol=19:1 (V/V)), and the fractions containing the objective material were concentrated under reduced pressure to obtain 0.13 g of the title compound as white foam. (Yield: 61 %)
Mass Spectrum (FAB, m/z): 523 ($M^+$+1).
$^1$H-NMR Spectrum (DMSO-$d_6$, δ ppm): 12.41 (brs, 0.2H), 8.64-8.59 (m, 1H), 7.94 (ddd, J=7.8, 7.8, 1.6 Hz, 1H), 7.80-7.75 (m, 1H), 7.59-7.53 (m, 1H), 7.21-7.11 (m, 3H), 7.00-6.94 (m, 2H), 6.74 (t, J=5.8 Hz, 1H), 6.33 (d, J=8.3 Hz, 1H), 6.26

(d, J=7.3 Hz, 1H), 4.64 (s, 2H), 4.22 (s, 2H), 3.82 (d, J=5.8 Hz, 2H), 2.29-2.12 (m, 2H), 2.12-1.94 (m, 2H), 1.84-1.63 (m, 3H), 1.46-1.29 (m, 1H), 1.25-1.08 (m, 2H), 0.95-0.81 (m, 2H), 0.78 (t, J=7.3 Hz, 3H).
Rf value: 0.48 (n-butanol:acetic acid:water=10:1:1).

[Example 1.8]

(6-{[4-(1-Propoxymethylcyclopropyl)benzyl](pyridin-2-ylsulfonyl)aminomethyl}-pyridin-2-ylamino)acetic acid (Exemplary compound No. 1319)

18-(a): tert-Butyl [tert-butoxycarbonyl(6-{[4-(1-propoxymethylcyclopropyl)benzyl]-(pyridin-2-ylsulfonyl)aminomethyl}pyridin-2-yl)amino acetate

To 2.6 ml of a tetrahydrofuran solution containing 250 mg (0.523 mmol) of tert-butyl (tert-butoxycarbonyl{6-[(pyridin-2-ylsulfonyl)aminomethyl]pyridin-2-yl}-amino)acetate obtained in the same manner as in Reference example 2 were added 126 mg (0.573 mmol) of 4-(1-propoxymethylcyclopropyl)benzyl alcohol obtained in Reference example 16-(c), 0.20 ml (0.80 mmol) of tri-n-butylphosphine and 135 mg (0.784 mmol) of N,N,N',N'-tetramethylazodicarboxamide, and the mixture was stirred at room temperature for 3 hours. After completion of the reaction, the reaction mixture was concentrated under reduced pressure, water was added to the residue, and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=10:1→1:1 (V/V)), and the fractions containing the objective material were concentrated under reduced pressure to obtain 332 mg of the title compound as pale yellow oil. (Yield: 93%)
Mass Spectrum (CI, m/z): 681 ($M^+$+1).
$^1$H-NMR Spectrum ($CDCl_3$, $\delta$ ppm): 8.58 (ddd, J=4.6, 1.6, 1.0 Hz, 1H), 7.82-7.71 (m, 2H), 7.64 (d, J=8.2 Hz, 1H), 7.44 (dd, J=8.2, 7.4 Hz, 1H), 7.39-7.33 (m, 1H), 7.20-7.10 (m, 4H), 6.88 (d, J=7.4 Hz, 1H), 4.66 (s, 2H), 4.46 (s, 2H), 4.45 (s, 2H), 3.49 (s, 2H), 3.35 (t, J=6.6 Hz, 2H), 1.60-1.48 (m, 2H), 1.52 (s, 9H), 1.43 (s, 9H), 0.88-0.78 (m, 4H), 0.86 (t, J=7.3 Hz, 3H).

18-(b): (6-{[4-(1-Propoxymethylcyclopropyl)benzyl](pyridin-2-ylsulfonyl)aminomethyl}pyridin-2-ylamino)acetic acid

To 1.4 ml of a tetrahydrofuran solution containing 328 mg (0.482 mmol) of tert-butyl [tert-butoxycarbonyl(6-{[4-(1-propoxymethylcyclopropyl)benzyl](pyridin-2-ylsulfonyl)aminomethyl}pyridin-2-yl)amino]acetate obtained in Example 18-(a) were added 1 ml of water and 0.42 ml of concentrated hydrochloric acid, and the mixture was stirred at 60°C for 3 hours. After completion of the reaction, the reaction mixture was concentrated under reduced pressure, the residue was adjusted to pH 4.2 with 1N aqueous sodium hydroxide solution and 1N hydrochloric acid, and extracted with ethyl acetate. The organic layer was washed with a saturated aqueous sodium chloride solution, dried over dried over anhydrous sodium sulfate and concentrated under reduced pressure. The residue was applied to silica gel column chromatography (eluent; acetonitrile :water:acetic acid=50:1:1→20:1:1 (V/V)), and the fractions containing the objective material were concentrated under reduced pressure to obtain 222 mg of the title compound as white foam. (Yield: 88%)
Mass Spectrum (FAB, m/z): 525 ($M^+$+1).
$^1$H-NMR Spectrum ($CDCl_3$, $\delta$ ppm): 8.71-8.68 (m, 1H), 7.94-7.90 (m, 1H), 7.85 (ddd, J=7.7, 7.7, 1.7 Hz, 1H), 7.46 (ddd, J=7.7, 4.7, 1.1 Hz, 1H), 7.37 (dd, J=8.3, 7.5 Hz, 1H), 7.13-7.06 (m, 4H), 6.72 (d, J=7.5 Hz, 1H), 6.30 (d, J=8.3 Hz, 1H), 4.60 (s, 2H), 4.54 (s, 2H), 3.86 (s, 2H), 3.49 (s, 2H), 3.37 (t, J=6.7 Hz, 2H), 1.58-1.49 (m, 2H), 0.86 (t, J=7.4 Hz, 3H), 0.86-0.76 (m, 4H).
Rf value: 0.42 (n-butanol:acetic acid:water=10:1:1).

[Example 19]

(6-{[4-(1-Benzylcyclopropyl)benzyl](pyridin-2-ylsulfonyl)aminomethyl}pyridin-2-ylamino)acetic acid (Exemplary compound No. 1277)

19-(a): tert-Butyl [(6-{[4-(1-benzylcyclopropyl)benzyl](pyridin-2-ylsulfonyl)aminomethyl}pyridin-2-yl)tert-butoxycarbonylamino]acetate

To 1 ml of a tetrahydrofuran solution containing 80.9 mg (0.169 mmol) of tert-butyl (tert-butoxycarbonyl {6-[(pyridin-2-ylsulfonyl)aminomethyl]pyridin-2-yl}amino)-acetate obtained in the same manner as in Reference example 2 were added 40.2 mg (0.169 mmol) of 4-(1-benzylcyclopropyl)benzyl alcohol obtained in Reference example 17-(d), 106 μl (0.424 mmol) of tri-n-butylphosphine and 72.8 mg (0.423 mmol) of N,N,N',N'-tetramethylazodicarboxamide, and the

mixture was stirred at room temperature for 16 hours. After completion of the reaction, the reaction mixture was concentrated under reduced pressure, water was added to the residue, and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=4:1→7:3 (V/V)), and the fractions containing the objective material were concentrated under reduced pressure to obtain 112 mg of the title compound as colorless oily product. (Yield: 95%)
Mass Spectrum (CI, m/z): 699 ($M^+$+1).
$^1$H-NMR Spectrum ($CDCl_3$, δ ppm): 8.57-8.53 (m, 1H), 7.79 (ddd, J=7.6, 1.3 1.1 Hz, 1H), 7.73 (ddd, J=7.6, 7.4, 1.6 Hz, 1H), 7.63 (d, J=8.2 Hz, 1H), 7.41 (dd, J=8.2, 7.4 Hz, 1H), 7.35 (ddd, J=7.4, 4.7, 1.3 Hz, 1H), 7.21-7.13 (m, 3H), 7.06-6.94 (m, 6H), 6.86 (d, J=7.4 Hz, 1H), 4.61 (s, 2H), 4.44 (s, 4H), 2.89 (s, 2H), 1.52 (s, 9H), 1.42 (s, 9H), 0.83-0.79 (m, 4H).

19-(b): <u>(6-{[4-(1-Benzylcyclopropyl)benzyl](pyridin-2-ylsulfonyl)aminomethyl}-pyridin-2-ylamino)acetic acid</u>

To 0.56 ml of a tetrahydrofuran solution containing 116 mg (0.166 mmol) of tert-butyl [(6-{[4-(1-benzylcyclopropyl)benzyl](pyridin-2-ylsulfonyl)aminomethyl}-pyridin-2-yl)tert-butoxycarbonylamino]acetate obtained in the same manner as in Example 19-(a) were added 0.4 ml of water and 0.14 ml of concentrated hydrochloric acid, and the mixture was stirred at 60°C for 5 hours. After completion of the reaction, the reaction mixture was concentrated under reduced pressure, and the residue was adjusted to pH 4.2 with 0.2N aqueous sodium hydroxide solution and 0.2N hydrochloric acid. Precipitated solid was collected by filtration, and dried at 50°C under reduced pressure to obtain 74.8 mg of the title compound as white solid. (Yield: 83%)
Mass Spectrum (FAB, m/z): 543 ($M^+$+1).
$^1$H-NMR Spectrum (DMSO-$d_6$, δ ppm): 12.38 (brs, 0.2H), 8.59 (ddd, J=4.7, 1.7, 0.9, Hz, 1H), 7.91 (ddd, J=7.8, 7.7, 1.7 Hz, 1H), 7.75 (ddd, J=7.8, 1.0, 0.9 Hz, 1H), 7.54 (ddd, J=7.7, 4.7, 1.0 Hz, 1H), 7.22-7.09 (m, 4H), 7.08-6.99 (m, 6H), 6.73 (t, J=5.9 Hz, 1H), 6.33 (d, J=8.1 Hz, 1H), 6.24 (d, J=7.1 Hz, 1H), 4.60 (s, 2H), 4.17 (s, 2H), 3.80 (d, J=5.9 Hz, 2H), 2.92 (s, 2H), 0.89-0.76 (m, 4H).
Rf value: 0.48 (n-butanol:acetic acid:water=10:1:1).

[Example 20]

<u>(6-{[4-(1-Benzyloxymethylcyclopropyl)benzyl](pyridin-2-ylsulfonyl)aminomethyl}-pyridin-2-ylamino)acetic acid</u> (Exemplary compound No. 1364)

20-(a):
<u>tert-Butyl[(6-{[4-(1-benzyloxymethylcyclopropyl)benzyl](pyridin-2-ylsulfon-yl)aminomethyl}pyridin-2-yl)tert-butoxycarbonylamino]acetate</u>

To 2 ml of a tetrahydrofuran solution containing 121 mg (0.253 mmol) of tert-butyl (tert-butoxycarbonyl {6-[(pyridin-2-ylsulfonyl)aminomethyl]pyridin-2-yl}amino)-acetate obtained in the same manner as in Reference example 2 were added 75 mg (0.28 mmol) of 4-(1-benzyloxymethylcyclopropyl)benzyl alcohol obtained in Reference example 18-(c), 0.18 ml (0.72 mmol) of tri-n-butylphosphine and 125 mg (0.726 mmol) of N,N,N',N'-tetramethylazodicarboxamide, and the mixture was stirred at room temperature for 5 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=9:1→1:1 (V/V)), and the fractions containing the objective material were concentrated under reduced pressure to obtain 136 mg of the title compound as yellow oil. (Yield: 74%)
Mass Spectrum (CI, m/z): 729 ($M^+$+1).
$^1$H-NMR Spectrum ($CDCl_3$, δ ppm): 8.56 (ddd, J=4.8, 1.6, 1.0 Hz, 1H), 7.79 (ddd, J=7.7, 1.2, 1.0 Hz, 1H), 7.73 (ddd, J=7.7, 7.6, 1.6 Hz, 1H), 7.64 (d, J=8.3 Hz, 1H), 7.43 (dd, J=8.3, 7.3 Hz, 1H), 7.38-7.10 (m, 10H), 6.88 (d, J=7.3 Hz, 1H), 4.67 (s, 2H), 4.48 (s, 2H), 4.47 (s, 2H), 4.45 (s, 2H), 3.53 (s, 2H), 1.52 (s, 9H), 1.42 (s, 9H), 0.87-0.82 (m, 4H).

20-(b): <u>(6-{[4-(1-Benzyloxymethylcyclopropyl)benzyl)(pyridin-2-ylsulfonyl)aminomethyl}pyridin-2-ylamino)acetic acid</u>

To 2 ml of a tetrahydrofuran solution containing 90 mg (0.12 mmol) of tert-butyl [(6- {[4-(1-benzyloxymethylcyclopropyl)benzyl](pyridin-2-ylsulfonyl)aminomethyl}pyridin-2-yl)tert-butoxycarbonylamino] acetate obtained in Example 20-(a) were added 2 ml of water and 0.21 ml of concentrated hydrochloric acid, and the mixture was stirred at 60°C for 9 hours. Then, 2 ml of acetone was added to the mixture, and the resulting mixture was stirred at 60°C for 38 hours. After completion of the reaction, the reaction mixture was adjusted to pH 4.4 with 1N aqueous sodium hydroxide solution

and 1N hydrochloric acid. After removing the supernatant, the residue was applied to silica gel column chromatography (eluent; ethyl acetate:methanol=9:1 (V/V)), and the fractions containing the objective material were concentrated under reduced pressure to obtain 16 mg of the title compound as white foam. (Yield: 23%) Mass Spectrum (FAB, m/z): 573 ($M^+$+1).
$^1$H-NMR Spectrum (DMSO-$d_6$, δ ppm): 8.60 (ddd, J=4.7, 1.7, 0.9 Hz, 1H), 7.92 (ddd, J=7.8, 7.7, 1.7 Hz, 1H), 7.76 (ddd, J=7.8, 1.1, 0.9 Hz, 1H), 7.54 (ddd, J=7.7, 4.7, 1.1 Hz, 1H), 7.37-7.10 (m, 10H), 6.72 (t, J=5.6 Hz, 0.8H), 6.33 (d, J=8.1 Hz, 1H), 6.26 (d, J=7.1 Hz, 1H), 4.66 (s, 2H), 4.46 (s, 2H), 4.19 (s, 2H), 3.80 (d, J=5.6 Hz, 2H), 3.54 (s, 2H), 0.89-0.77 (m, 4H).
Rf value: 0.46 (n-butanol:acetic acid:water=10:1:1).

[Example 21]

{6-[(Benzenesulfonyl)(4-tert-butylbenzyl)aminomethyl]-pyridin-2-ylamino}acetic acid hydrochloride (Exemplary compound No. 92)

21-(a): tert-Butyl({6-[{benzenesulfonyl)(4-tert-butylbenzyl)aminomethyl]pyridin-2-yl}tert-butoxycarbonylamino)acetate

To 4.0 ml of a tetrahydrofuran solution containing 149 mg (0.491 mmol) of N-(4-tert-butylbenzyl)benzenesulfonamide obtained in Reference example 19 were added 165 mg (0.488 mmol) of tert-butyl [tert-butoxycarbonyl(6-hydroxymethylpyridin-2-yl)-amino]acetate obtained in Reference example 1-(b), 0.18 ml (0.72 mmol) of tri-n-butylphosphine and 127 mg (0.738 mmol) of N,N,N',N'-tetramethylazodicarboxamide, and the mixture was stirred at room temperature for 2.5 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The obtained residue was applied to silica gel column chromatography (eluent; n-hexane:ethyl acetate=4:1 (V/V)), and the fractions containing the objective material were concentrated under reduced pressure to obtain 247 mg of the title compound as white foam. (Yield: 81%)
Mass Spectrum (FAB, m/z): 624 ($M^+$+1).
$^1$H-NMR Spectrum ($CDCl_3$, δ ppm): 7.74-7.71 (m, 2H), 7.67 (d, J=8.2 Hz, 1H), 7.54-7.50 (m, 1H), 7.47 (dd, J=8.2, 7.4 Hz, 1H), 7.45-7.40 (m, 2H), 7.25-7.21 (m, 2H), 7.09-7.05 (m, 2H), 6.85 (d, J=7.4 Hz, 1H), 4.47 (s, 2H), 4.37 (s, 2H), 4.35 (s, 2H), 1.52 (s, 9H), 1.42 (s, 9H), 1.27 (s, 9H).

21-(b): {6-[(Benzenesulfonyl)(4-tert-butylbenzyl)aminomethyl]pyridin-2-ylamino}-acetic acid hydrochloride

To 2.3 ml of a methylene chloride solution containing 239 mg (0.383 mmol) of tert-butyl ({6-[(benzenesulfonyl)(4-tert-butylbenzyl)aminomethyl]pyridin-2-yl)tert-butoxycarbonylamino)acetate obtained in Example 21-(a) was added 1.8 ml (7.2 mmol) of 4N hydrogen chloride/1,4-dioxane solution, and the mixture was stirred at room temperature for 16 hours. After completion of the reaction, the reaction mixture was concentrated under reduced pressure, diethyl ether was added to the obtained residue and ultrasonication treatment was carried out. The precipitated solid was collected by filtration and dried under reduced pressure to obtain 161 mg of the title compound as white solid. (Yield: 83%)
Mass Spectrum (FAB, m/z): 468 ($M^+$+1).
$^1$H-NMR Spectrum ($CD_3OD$, δ ppm): 7.98-7.93 (m, 2H), 7.77-7.72 (m, 1H), 7.70-7.61 (m, 3H), 7.24-7.20 (m, 2H), 7.14-7.10 (m, 2H), 6.79 (d, J=8.8 Hz, 1H), 6.52 (d, J=7.2 Hz, 1H), 4.42 (s, 2H), 4.38 (s, 2H), 4.13 (s, 2H), 1.22 (s, 9H).
Rf value: 0.58 {n-butanol:acetic acid:water=10:1:1).

[Example 22]

{6-[(3-tert-Butylbenzyl)(pyridin-3-ylsulfonyl)aminomethyl]pyridin-2-ylamino} acetic acid hydrochloride (Exemplary compound No. 86)

22-(a): tert-Butyl (tert-butoxycarbonyl{6-[(3-tert-butylbenzyl)(pyridin-3-ylsulfonyl)-aminomethylpyridin-2-yl}amino)acetate

To 5.1 ml of a tetrahydrofuran solution containing 191 mg (0.627 mmol) of N-(3-tert-butylbenzyl)pyridin-3-ylsulfonamide obtained in Reference example 20 were added 213 mg (0.629 mmol) of tert-butyl [tert-butoxycarbonyl(6-hydroxymethyl-pyridin-2-yl)amino]acetate obtained in Reference example 1-(b), 0.23 ml (0.92 mmol) of tri-n-butylphosphine and 163 mg (0.947 mmol) of N,N,N',N'-tetramethylazodicarb-oxamide, and the mixture was stirred at room temperature for 4 hours. After completion of the reaction, water was added to the reaction mixture and the mixture was extracted with ethyl acetate. The organic layer was washed with a saturated aqueous sodium chloride solution, dried over anhydrous

magnesium sulfate and concentrated under reduced pressure. The obtained residue was applied to silica gel column chromatography (eluent; n-hexane:ethyl acetate=3:2 (V/V)), and the fractions containing the objective material were concentrated under reduced pressure to obtain 289 mg of the title compound as colorless oily product. (Yield: 74%)
Mass Spectrum (FAB, m/z): 625 ($M^{+}$+1).
$^{1}$H-NMR Spectrum ($CDCl_3$, $\delta$ ppm): 8.95 (dd, J=2.3, 0.8 Hz, 1H), 8.71 (dd, J=4.8, 1.6 Hz, 1H), 7.88 (ddd, J=8.0, 2.3, 1.6 Hz, 1H), 7.71 (d, J=8.4 Hz, 1H), 7.52 (dd, J=8.4, 7.5 Hz, 1H), 7.31 (ddd, J=8.0, 4.8, 0.8 Hz, 1H), 7.29-7.26 (m, 1H), 7.22-7.17 (m, 2H), 7.03-7.00 (m, 1H), 6.88 (d, J=7.5 Hz, 1H), 4.56 (s, 2H), 4.39 (s, 2H), 4.38 (s, 2H), 1.52 (s, 9H), 1.42 (s, 9H), 1.25 (s, 9H).

22-(b): <u>{6-[(3-tert-Butylbenzyl)(pyridin-3-ylsulfonyl)aminomethyl]pyridin-2-ylamino}-acetic acid hydrochloride</u>

To 1.7 ml of a methylene chloride solution containing 177 mg (0.283 mmol) of tert-butyl (tert-butoxycarbonyl{6-[(3-tert-butylbenzyl)(pyridin-3-ylsulfonyl)aminomethyl]pyridin-2-yl}amino)acetate obtained in Example 22-(a) was added 1.3 ml (5.2 mmol) of 4N hydrogen chloride/1,4-dioxane solution, and the mixture was stirred at room temperature for 20.5 hours. After completion of the reaction, the reaction mixture was concentrated under reduced pressure, methylene chloride was added to the obtained reisude and ultrasonication treatment was carried out. The precipitated solid was collected by filtration and dried under reduced pressure to obtain 137 mg of the title compound as white solid. (Yield: 89%)
Mass Spectrum (FAB, m/z): 469 ($M^{+}$+1).
$^{1}$H-NMR Spectrum ($CD_3OD$, $\delta$ ppm): 9.28 (d, J=2.1 Hz, 1H), 9.02 (dd, J=5.3, 1.4 Hz, 1H), 8.72 (ddd, J=8.2,2.1, 1.4 Hz, 1H), 8.01 (dd, J=8.2, 5.3 Hz, 1H), 7.72 (dd, J=8.9, 7.4 Hz, 1H), 7.27-7.24 (m, 2H), 7.21-7.16 (m, 1H), 7.14-7.10 (m, 1H), 6.86 (d, J=8.9 Hz, 1H), 6.61 (d, J=7.4 Hz, 1H), 4.64 (s, 2H), 4.54 (s, 2H), 4.17 (s, 2H), 1.22 (s, 9H).
Rf value: 0.46 (n-butanol:acetic acid:water=10:1:1).

[Example 23]

<u>{6-[(4-tert-Butylbenzyl)(pyridin-2-ylsulfonyl)aminomethyl]pyridin-2-ylamino}acetic acid hydrochloride</u> (Exemplary compound No. 125)

23-(a): <u>tert-Butyl (tert-butoxycarbonyl{6-[(4-tert-butylbenzyl)(pyridin-2-ylsulfonyl)-aminomethyl]pyridin-2-yl}amino acetate</u>

To 5.4 ml of a tetrahydrofuran solution containing 200 mg (0.657 mmol) of N-(4-tert-butylbenzyl)pyridin-2-ylsulfonamide obtained in Reference example 21 were added 222 mg (0.656 mmol) of tert-butyl [tert-butoxycarbonyl(6-hydroxymethyl-pyridin-2-yl)amino]acetate obtained in Reference example 1-(b), 0.24 ml (0.96 mmol) of tri-n-butylphosphine and 171 mg (0.993 mmol) of N,N,N',N'-tetramethylazodi-carboxamide, and the mixture was stirred at room temperature for 4 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The obtained residue was applied to silica gel column chromatography (eluent; n-hexane:ethyl acetate=1:1 (V/V)), and the fractions containing the objective material were concentrated under reduced pressure to obtain 311 mg of the title compound as white foam. (Yield: 76%)
Mass Spectrum (FAB, m/z): 625 ($M^{+}$+1).
$^{1}$H-NMR Spectrum ($CDCl_3$, $\delta$ ppm): 8.60-8.57 (m, 1H), 7.83-7.79 (m, 1H), 7.75 (ddd, J=7.7, 7.5, 1.8 Hz, 1H), 7.64 (d, J=8.3 Hz, 1H), 7.44 (dd, J=8.3, 7.3 Hz, 1H), 7.37 (ddd, J=7.5, 4.4, 1.5 Hz, 1H), 7.24-7.19 (m, 2H), 7.15-7.11 (m, 2H), 6.90 (d, J=7.3 Hz, 1H), 4.64 (s, 2H), 4.49 (s, 2H), 4.47 (s, 2H), 1.52 (s, 9H), 1.43 (s, 9H), 1.27 (s, 9H).

23-(b): <u>{6-[(4-tert-Butylbenzyl)(pyridin-2-ylsulfonyl)aminomethyl]pyridin-2-ylamino}-acetic acid hydrochloride</u>

To 2.9 ml of a methylene chloride solution containing 302 mg (0.484 mmol) of tert-butyl (tert-butoxycarbonyl{6-[(4-tert-butylbenzyl)(pyridin-2-ylsulfonyl)aminomethyl]pyridin-2-yl}amino)acetate obtained in Example 23-(a) was added 2.4 ml (9.6 mmol) of 4N hydrogen chloride/1,4-dioxane solution, and the mixture was stirred at room temperature for 16 hours. After completion of the reaction, the reaction mixture was concentrated under reduced pressure, diisopropyl ether was added to the obtained residue and ultrasonication treatment was carried out. The precipitated solid was collected by filtration and dried under reduced pressure to obtain 198 mg of the title compound as white solid. (Yield: 76%)
Mass Spectrum (FAB, m/z): 469 ($M^{+}$+1).
$^{1}$H-NMR Spectrum ($CD_3OD$, $\delta$ ppm): 8.76-8.71 (m, 1H), 8.08 (ddd, J=7.6, 7.6, 1.8 Hz, 1H), 8.05-8.02 (m, 1H), 7.70-7.64 (m, 2H), 7.25-7.19 (m, 2H), 7.15-7.11 (m, 2H), 6.80 (d, J=9.3 Hz, 1H), 6.62 (d, J=7.3 Hz, 1H), 4.71 (s, 2H), 4.48 (s, 2H), 4.14 (s, 2H), 1.23 (s, 9H).
Rf value: 0.40 (n-butanol:acetic acid:water=10:1:1).

[Example 24]

(6-{[4-(1-Methyl-1-pentenyl)benzyl](pyridin-2-ylsulfonyl)aminomethyl}pyridin-2-yl-amino)acetic acid (Exemplary compound No. 1012)

24-(a): tert-Butyl ({6-[(4-bromobenzyl)(pyridin-2-ylsulfonyl)aminomethyl]pyridin-2-yl}tert-butoxycarbonylamino)acetate

To 15 ml of a tetrahydrofuran solution containing 654 mg (2.00 mmol) of N-(4-bromobenzyl)pyridin-2-ylsulfonamide obtained in Reference example 22 were added 735 mg (2.01 mmol) of tert-butyl [tert-butoxycarbonyl(6-hydroxymethylpyridin-2-yl)-amino]acetate obtained in Reference example 1-(b), 1.1 ml (4.4 mmol) of tri-n-butylphosphine and 563 mg (3.27 mmol) of N,N,N',N'-tetramethylazodicarboxamide, and the mixture was stirred at room temperature for 13 hours. After completion of the reaction, a saturated aqueous sodium chloride solution was added to the reaction mixture, and the mixture was extracted with ethyl acetate. The organic layer was washed with a saturated aqueous sodium chloride solution, dried over anhydrous magnesium sulfate and concentrated under reduced pressure. The obtained residue was applied to silica gel column chromatography (eluent; n-hexane:ethyl acetate= 1:0→1:1 (V/V)), and the fractions containing the objective material were concentrated under reduced pressure to obtain 1.39 g of the title compound as white foam substantially quantitatively.
Mass Spectrum (FAB, m/z): 647, 649 ($M^+$+1).
$^1$H-NMR Spectrum ($CDCl_3$, δ ppm): 8.59 (ddd, J=4.7, 1.6, 0.9 Hz, 1H), 7.81 (ddd, J=7.7, 1.6, 0.9 Hz, 1H), 7.78 (ddd, J=7.7, 7.3, 1.6 Hz, 1H), 7.65 (d, J=7.9 Hz, 1H), 7.44 (dd, J=7.9, 7.4 Hz, 1H), 7.40 (ddd, J=7.3, 4.7, 1.6 Hz, 1H), 7.37-7.33 (m, 2H), 7.14-7.11 (m, 2H), 6.87 (d, J=7.4 Hz, 1H), 4.66 (s, 2H), 4.44 (s, 2H), 4.43 (s, 2H), 1.52 (s, 9H), 1.42 (s, 9H).

24-(b): tert-Butyl ({6-[(4-acetylbenzyl)(pyridin-2-ylsulfonyl)aminomethyl]pyridin-2-yl}tert-butoxycarbonylamino)acetate

To 5.0 ml of a N,N-dimethylformamide solution containing 1.25 g (1.93 mmol) of tert-butyl ({6-[(4-bromobenzyl)(pyridin-2-ylsulfonyl)aminomethyl]pyridin-2-yl}tert-butoxycarbonylamino)acetate obtained in Example 24-(a) were added 21.7 mg (0.0562 mmol) of 1,3-bis(diphenylphosphino)propane, 11.9 mg (0.0526 mmol) of palladium acetate, 1.00 g (10.0 mmol) of butyl vinyl ether and 0.70 ml (4.1 mmol) of diisopropylethylamine, and after degassing, the mixture was stirred at 100°C for 3 hours. To the mixture were further added 62.2 mg (0.151 mmol) of 1,3-bis(diphenylphosphino)-propane, 30.9 mg (0.138 mmol) of palladium acetate and 1.00 g (10.0 mmol) of butyl vinyl ether, and after degassing, the mixture was stirred at 120°C for 17 hours. After completion of the reaction, water was added to the reaction mixture and the mixture was extracted with toluene. The organic layer was washed with a 10% aqueous potassium hydrogen sulfate solution, 5.0 ml of acetic acid was added thereto, and the mixture was stirred at room temperature for 4 hours. The mixture was washed with a saturated aqueous sodium hydrogen carbonate solution and a saturated aqueous sodium chloride solution, dried over anhydrous magnesium sulfate and concentrated under reduced pressure. The obtained residue was applied to silica gel column chromatography (eluent; n-hexane:ethyl acetate=19:1→2:3 (V/V)), and the fractions containing the objective material were concentrated under reduced pressure to obtain 600 mg of the title compound as orange foam. (Yield: 51%)
Mass Spectrum (FAB, m/z): 611 ($M^+$+1).
$^1$H-NMR Spectrum ($CDCl_3$, δ ppm): 8.60 (ddd, J=4.7, 1.7, 0.9 Hz, 1H), 7.85-7.81 (m, 3H), 7.78 (ddd, J=7.7, 7.5, 1.7 Hz, 1H), 7.64 (d, J=8.1 Hz, 1H), 7.43 (dd, J=8.1, 7.3 Hz, 2H), 7.40 (ddd, J=7.5, 4.7, 1.3 Hz, 1H), 7.37-7.34 (m, 2H), 6.87 (d, J=7.3 Hz, 1H), 4.78 (s, 2H), 4.46 (s, 2H), 4.42 (s, 2H), 2.58 (s, 3H), 1.52 (s, 9H), 1.42 (s, 9H).

24-(c): tert-Butyl [tert-butoxycarbonyl(6-{[4-(1-methyl-1-pentenyl)benzyl](pyridin-2-ylsulfonyl)aminomethyl}pyridin-2-yl)amino]acetate

To 2.0 ml of a tetrahydrofuran solution containing 202 mg (0.506 mmol) of butyltriphenylphosphonium bromide was added 0.26 ml (0.42 mmol) of 1.6M n-butyl lithium/n-hexane solution at -78°C, and the mixture was stirred at room temperature for 1 hour. Then, to the mixture was added dropwise 1.0 ml of a tetrahydrofuran solution containing 206 mg (0.337 mmol) of tert-butyl ({6-[(4-acetylbenzyl)(pyridin-2-ylsulfon-yl)aminomethyl]pyridin-2-yl}tert-butoxycarbonylamino)acetate obtained in Example 24-(b) at room temperature, and the mixture was refluxed for 16 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The obtained residue was applied to silica gel column chromatography (eluent; n-hexane:ethyl acetate=97:3→2:3 (V/V)), and the fractions containing the objective material were concentrated under reduced pressure to obtain 8.9 mg of the title compound as white oil. (Yield: 4%)
Mass Spectrum (FAB, m/z): 651 ($M^+$+1).
$^1$H-NMR Spectrum ($CDCl_3$, δ ppm): 8.60-8.58 (m, 1H), 7.84-7.74 (m, 2H), 7.68-7.60 (m, 1H), 7.45, 7.44 (each dd, J=8.4,

7.5 Hz, total 1H), 7.37 (ddd, J=7.5, 4.6, 1.3 Hz, 1H), 7.25-7.00 (m, 4H), 6.92-6.89 (m, 1H), 5.76-5.71, 5.45-5.41 (each m, total 1H), 4.67 (s, 2H), 4.51, 4.47 (each s, total 2H), 4.46, 4.45 (each s, total 2H), 2.19-2.13, 1.91-1.86 (each m, total 2H), 1.99-1.96 (m, 3H), 1.52 (s, 9H), 1.51-1.44, 1.38-1.30 (each m, total 2H), 1.43 (s, 9H), 0.96, 0.85 (each t, J=7.3 Hz, total 3H).

24-(d): (6-{[4-(1-Methyl-1-pentenyl)benzyl](pyridin-2-ylsulfonyl)aminomethyl}-pyridin-2-ylamino)acetic acid

To 0.2 ml of a methylene chloride solution containing 8.9 mg (0.014 mmol) of tert-butyl [tert-butoxycarbonyl(6-{[4-(1-methyl-1-pentenyl)benzyl](pyridin-2-ylsulfon-yl)aminomethyl}pyridin-2-yl)amino]acetate obtained in Example 24-(c) were added 10.3 mg (0.0829 mmol) of thioanisole and 0.060 ml (0.81 mmol) of trifluoroacetic acid, and the mixture was stirred at room temperature for 6.5 hours. After completion of the reaction, 1N aqueous sodium hydroxide solution was added to the reaction mixture, and the mixture was extracted with diethyl ether. The aqueous layer was adjusted to pH 4.7 with diluted hydrochloric acid, and extracted with ethyl acetate. The organic layer was washed with a saturated aqueous sodium chloride solution, dried over anhydrous magnesium sulfate and concentrated under reduced pressure to obtain 7.0 mg of the title compound as pale brown solid substantially quantitatively.
Mass Spectrum (FAB, m/z): 495 ($M^+$+1).
$^1$H-NMR Spectrum ($CD_3OD$, $\delta$ ppm): 8.50-8.48 (m, 1H), 7.81-7.76 (m, 1H), 7.71-7.67 (m, 1H), 7.43-7.39 (m, 1H), 7.19-6.94 (m, 5H), 6.32 (d, J=7.0 Hz, 1H), 6.27-6.23 (m, 1H), 5.68-5.64, 5.38-5.33 (each m, total 1H), 4.66, 4.64 (each s, total 2H), 4.26, 4.23 (each s, total 2H), 3.80, 3.79 (each s, total 2H), 2.09, 1.80 (each q, J=7.4 Hz, total 2H), 1.91-1.87 (m, 3H), 1.44-1.35, 1.29-1.21 (each m, total 2H), 0.88, 0.75 (each t, J=7.4 Hz, total 3H).
Rf value: 0.44 (n-butanol:acetic acid:water=10:1:1).

[Example 25]

{6-[(Pyridin-3-ylsulfonyl)(4-trifluoromethylbenzyl)aminomethyl]pyridin-2-ylamino}-acetic acid hydrochloride (Exemplary compound No. 811)

25-(a): tert-Butyl (tert-butoxycarbonyl{6-[(pyridin-3-ylsulfonyl)(4-trifluoromethylbenzyl)aminomethyl]pyridin-2-yl}amino)acetate

To 1.5 ml of a tetrahydrofuran solution containing 62.6 mg (0.131 mmol) of tert-butyl (tert-butoxycarbonyl {6-[(pyridin-3-ylsulfonyl)aminomethyl]pyridin-2-yl}-amino)acetate obtained in the same manner as in Reference example 1-(f) were added 26.3 mg (0.149 mmol) of 4-trifluoromethylbenzyl alcohol, 0.065 ml (0.26 mmol) of tri-n-butylphosphine and 37.5 mg (0.218 mmol) of N,N,N',N'-tetramethylazodicarb-oxamide, and the mixture was stirred at room temperature for 16 hours. After completion of the reaction, a saturated aqueous sodium chloride solution was added to the reaction mixture, and the mixture was extracted with ethyl acetate. The organic layer was dried over anhydrous magnesium sulfate and concentrated under reduced pressure. The obtained residue was applied to silica gel column chromatography (eluent; n-hexane:ethyl acetate=9:1→1:1 (V/V)), and the fractions containing the objective material were concentrated under reduced pressure to obtain 67.8 mg of the title compound as pale yellow oil. (Yield: 81 %)
Mass Spectrum (FAB, m/z): 637 ($M^+$+1).
$^1$H-NMR Spectrum ($CDCl_3$, $\delta$ ppm): 8.94 (dd, J=2.3, 0.7 Hz, 1H), 8.72 (dd, J=4.9, 1.7 Hz, 1H), 7.86 (ddd, J=8.0, 2.3, 1.7 Hz, 1H), 7.71 (d, J=8.3 Hz, 1H), 7.54 (d, J=8.2 Hz, 2H), 7.50 (dd, J=8.3, 7.3 Hz, 1H), 7.38 (d, J=8.2 Hz, 2H), 7.32 (ddd, J=8.0, 4.9, 0.7 Hz, 1H), 6.82 (d, J=7.3 Hz, 1H), 4.64 (s, 2H), 4.38 (s, 2H), 4.32 (s, 2H), 1.53 (s, 9H), 1.41 (s, 9H).

25-(b): {6-[(Pyridin-3-ylsulfonyl)(4-trifluoromethylbenzyl)aminomethyl]pyridin-2-ylamino}acetic acid hydrochloride

To 0.25 ml of a methylene chloride solution containing 65.2 mg (0.102 mmol) of tert-butyl (tert-butoxycarbonyl {6-[(pyridin-3-ylsulfonyl)(4-trifluoromethylbenzyl)-aminomethyl]pyridin-2-yl}amino)acetate obtained in Example 25-(a) was added 1.0 ml (4.0 mmol) of 4N hydrogen chloride/1,4-dioxane solution, and the mixture was stirred at room temperature for 2 hours. After completion of the reaction, the reaction mixture was concentrated under reduced pressure, acetone and diisopropyl ether were added to the obtained residue and ultrasonication treatment was carried out. The precipitated solid was collected by filtration and dried under reduced pressure to obtain 42.8 mg of the title compound as pale yellow solid. (Yield: 76%)
Mass Spectrum (FAB, m/z): 481 ($M^+$+1).
$^1$H-NMR Spectrum ($CD_3OD$, $\delta$ ppm): 9.16 (d, J=2.2 Hz, 1H), 8.92 (dd, J=5.0, 1.5 Hz, 1H), 8.46 (ddd, J=8.1, 2.2, 1.5 Hz, 1H), 7.78 (ddd, J=8.1, 5.0, 0.6 Hz, 1H), 7.70 (dd, J=9.0, 7.4 Hz, 1H), 7.52 (d, J=8.2 Hz, 2H), 7.44 (d, J=8.2 Hz, 2H), 6.85 (d, J=9.0 Hz, 1H), 6.66 (d, J=7.4 Hz, 1H), 4.59 (s, 2H), 4.59 (s, 2H), 4.13 (s, 2H).
Rfvalue: 0.45 (n-butanol:acetic acid:water=10:1:1).

[Example 26]

<u>(6-{[4-(1-Ethyl-1-methylpropyl)benzyl)(pyridin-3-ylsulfonyl)aminomethyl}pyridin-2-ylamino)acetic acid</u> (Exemplary compound No. 481)

26-(a): <u>tert-Butyl [tert-butoxycarbonyl(6-{[4-(1-ethyl-1-methylpropyl)benzyl)(pyridin-3-ylsulfonyl)aminomethyl}pyridin-2-yl)amino]acetate</u>

To 3.8 ml of a tetrahydrofuran solution containing 375 mg (0.784 mmol) of tert-butyl (tert-butoxycarbonyl{6-[(pyridin-3-ylsulfonyl)aminomethyl]pyridin-2-yl}-amino)acetate obtained in the same manner as in Reference example 1-(f) were added 137 mg (0.712 mmol) of 4-(1-ethyl-1-methylpropyl)benzyl alcohol obtained in Reference example 23-(d), 0.27 ml (1.1 mmol) of tri-n-butylphosphine and 184 mg (1.07 mmol) of N,N,N',N'-tetramethylazodicarboxamide, and the mixture was stirred at room temperature for 16 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The obtained residue was applied to silica gel column chromatography (eluent; n-hexane:ethyl acetate=1:0→7:3 (V/V)), and the fractions containing the objective material were concentrated under reduced pressure to obtain 424 mg of the title compound as white foam. (Yield: 91 %)
Mass Spectrum (FAB, m/z): 653 ($M^+$+1).
$^1$H-NMR Spectrum ($CDCl_3$, δ ppm): 8.95 (dd, J=2.3, 0.9 Hz, 1H), 8.69 (dd, J=4.9, 1.7 Hz, 1H), 7.85 (ddd, J=8.0, 2.3, 1.7 Hz, 1H), 7.70 (d, J=8.2 Hz, 1H), 7.50 (dd, J=8.2, 7.4 Hz, 1H), 7.29 (ddd, J=8.0, 4.9, 0.9 Hz, 1H), 7.19-7.10 (m, 4H), 6.86 (d, J=7.4 Hz, 1H), 4.53 (s, 2H), 4.40 (s, 2H), 4.39 (s, 2H), 1.69 (dq, J=14.2, 7.3 Hz, 2H), 1.60-1.45 (m, 11H), 1.42 (s, 9H), 1.20 (s, 3H), 0.63 (t, J=7.3 Hz, 6H).

26-(b): <u>(6-{[4-(1-Ethyl-1-methylpropyl)benzyl](pyridin-3-ylsulfonyl)aminomethyl}-pyridin-2-ylamino)acetic acid</u>

To 1.3 ml of a tetrahydrofuran solution containing 420 mg (0.643 mmol) of tert-butyl [tert-butoxycarbonyl(6-{[4-(1-ethyl-1-methylpropyl)benzyl](pyridin-3-yl-sulfonyl)aminomethyl}pyridin-2-yl)amino]acetate obtained in Example 26-(a) were added 1.3 ml of water and 0.54 ml of concentrated hydrochloric acid, and the mixture was stirred at 65°C for 6.5 hours. After completion of the reaction, water, and then, 1N aqueous sodium hydroxide solution were added to the reaction mixture to adjust pH to 4.4, and the mixture was extracted with ethyl acetate. The organic layer was dried over anhydrous magnesium sulfate and concentrated under reduced pressure. To the obtained residue were added ethyl acetate and diisopropyl ether, and the mixture was stirred at 50°C for 1 hour. The precipitated solid was collected by filtration and dried under reduced pressure to obtain 258 mg of the title compound as white solid. (Yield: 81%)
Mass Spectrum (FAB, m/z): 497 ($M^+$+1).
$^1$H-NMR Spectrum ($CDCl_3$, δ ppm): 9.07 (d, J=2.0 Hz, 1H), 8.73 (dd, J=5.0, 1.6 Hz, 1H), 7.97 (ddd, J=7.9, 2.0, 1.6 Hz, 1H), 7.37-7.15 (m, 6H), 6.50 (d, J=7.1 Hz, 1H), 6.30 (d, J=8.3 Hz, 1H), 4.61 (s, 2H), 4.35 (s, 2H), 3.89 (s, 2H), 1.70 (dq, J=14.4, 7.5 Hz, 2H), 1.52 (dq, J=14.4, 7.5 Hz, 2H), 1.21 (s, 3H), 0.63 (t, J=7.5 Hz, 6H).
Rf value: 0.47 (n-butanol:acetic acid:water=10:1:1).

[Example 27]

<u>(6-{[4-(1-Ethylpropyl)benzyl](pyridin-3-ylsulfonyl)aminomethyl}pyridin-2-ylamino)-acetic acid</u> (Exemplary compound No. 297)

27-(a): <u>tert-Butyl [tert-butoxycarbonyl(6-{[4-(1-ethylpropyl)benzyl](pyridin-3-ylsulfon-yl)aminomethyl}pyridin-2-yl)amino]acetate</u>

To 17 ml of a tetrahydrofuran solution containing 840 mg (1.76 mmol) of tert-butyl (tert-butoxycarbonyl {6-[(pyridin-3-ylsulfonyl)aminomethyl]pyridin-2-yl} amino)-acetate obtained in the same manner as in Reference example 1-(f) were added 347 mg (1.95 mmol) of 1-ethylpropylbenzyl alcohol obtained in Reference example 24-(b), 0.66 ml (2.6 mmol) of tri-n-butylphosphine and 455 mg (2.64 mmol) of N,N,N',N'-tetramethylazodicarboxamide, and the mixture was stirred at room temperature for 4 hours. After completion of the reaction, the reaction mixture was concentrated under reduced pressure, water was added to the residue, and the mixture was extracted with ethyl acetate. The organic layer was washed with a saturated aqueous sodium chloride solution, dried over anhydrous sodium sulfate and concentrated under reduced pressure. The obtained residue was applied to silica gel column chromatography (eluent; n-hexane:ethyl acetate=1:0→1:1 (V/V)), and the fractions containing the objective material were concentrated under reduced pressure to obtain 1.10 g of the title compound as colorless oily product. (Yield: 98%)

Mass Spectrum (FAB, m/z): 639 ($M^+$+1).
$^1$H-NMR Spectrum ($CDCl_3$, δ ppm): 8.95 (dd, J=2.3, 0.9 Hz, 1H), 8.69 (dd, J=4.9, 1.5 Hz, 1H), 7.85 (ddd, J=8.1, 2.3, 1.5 Hz, 1H), 7.70 (d, J=8.4 Hz, 1H), 7.50 (dd, J=8.4, 7.1 Hz, 1H), 7.29 (ddd, J=8.1, 4.9, 0.9 Hz, 1H), 7.15-7.09 (m, 2H), 7.05-6.99 (m, 2H), 6.86 (d, J=7.1 Hz, 1H), 4.53 (s, 2H), 4.40 (s, 2H), 4.39 (s, 2H), 2.33-2.20 (m, 1H), 1.75-1.35 (m, 4H), 1.52 (s, 9H), 1.42 (s, 9H), 0.73 (t, J=7.3 Hz, 6H).

27-(b): <u>(6-{[4-(1-Ethylpropyl)benzyl)(pyridin-3-ylsulfonyl)aminomethyl}pyridin-2-ylamino)acetic acid</u>

To 3.4 ml of a tetrahydrofuran solution containing 1.09 g (1.71 mmol) of tert-butyl [tert-butoxycarbonyl(6-{[4-(1-ethylpropyl)benzyl](pyridin-3-ylsulfonyl)aminomethyl}pyridin-2-yl)amino]acetate obtained in Example 27-(a) were added 3.4 ml of water and 1.5 ml of concentrated hydrochloric acid, and the mixture was stirred at 65°C for 2 hours. To the mixture was further added 0.3 ml of concentrated hydrochloric acid, and the mixture was stirred at 65°C for 2 hours, and then, at 70°C for 2 hours. After completion of the reaction, 6N aqueous sodium hydroxide solution was added to the reaction mixture to adjust pH to 4.6, and the mixture was extracted with methylene chloride. The organic layer was dried over anhydrous sodium sulfate, and concentrated under reduced pressure. To the obtained residue were added ethyl acetate and diisopropyl ether, and the mixture was stirred at 50°C for 30 minutes, and then, at room temperature for 16 hours. The precipitated solid was collected by filtration and dried under reduced pressure to obtain 691 mg of the title compound as white solid. (Yield: 84%)
Mass Spectrum (FAB, m/z): 483 ($M^+$+1).
$^1$H-NMR Spectrum ($CDCl_3$, δ ppm): 9.07 (d, J=1.8 Hz, 1H), 8.74 (dd, J=5.0, 1.7 Hz, 1H), 7.96 (ddd, J=7.9, 1.8, 1.7 Hz, 1H), 7.34 (dd, J=7.9, 5.0 Hz, 1H), 7.31 (dd, J=8.3, 7.3 Hz, 1H), 7.24-7.20 (m, 2H), 7.08-7.03 (m, 2H), 6.49 (d, J=7.3 Hz, 1H), 6.29 (d, J=8.3 Hz, 1H), 4.61 (s, 2H), 4.36 (s, 2H), 3.88 (s, 2H), 2.32-2.24 (m, 1H), 1.71-1.61 (m, 2H), 1.56-1.44 (m, 2H), 0.74 (t, J=7.3 Hz, 6H).
Rf value: 0.46 (n-butanol:acetic acid:water=10:1:1).

[Example 28]

<u>{6-[(4-tert-Pentylbenzyl)(pyridin-2-ylsulfonyl)aminomethyl]pyridin-2-ylamino}acetic acid hydrochloride</u> (Exemplary compound No. 249)

28-(a): <u>tert-Butyl (tert-butoxycarbonyl{6-[(4-tert-pentylbenzyl)(pyridin-2-ylsulfonyl)-aminomethyl]pyridin-2-yl}amino)acetate</u>

To 5.0 ml of a tetrahydrofuran solution containing 308 mg (0.644 mmol) of tert-butyl (tert-butoxycarbonyl{6-[(pyridin-2-ylsulfonyl)aminomethyl]pyridin-2-yl}-amino)acetate obtained in the same manner as in Reference example 2 were added 113 mg (0.634 mmol) of 4-tert-pentylbenzyl alcohol (see WO2002/059077A), 0.35 ml (1.4 mmol) of tri-n-butylphosphine and 179 mg (1.04 mmol) of N,N,N',N'-tetramethylazo-dicarboxamide, and the mixture was stirred at room temperature for 15 hours. After completion of the reaction, a saturated aqueous sodium chloride solution was added to the reaction mixture, and the mixture was extracted with ethyl acetate. The organic layer was washed with a saturated aqueous sodium chloride solution, dried over anhydrous magnesium sulfate and concentrated under reduced pressure. The obtained residue was applied to silica gel column chromatography (eluent; n-hexane:ethyl acetate=1:0→3:2 (V/V)), and the fractions containing the objective material were concentrated under reduced pressure to obtain 430 mg of the title compound as colorless oily product substantially quantitatively.
Mass Spectrum (FAB, m/z): 639 ($M^+$+1).
$^1$H-NMR Spectrum ($CDCl_3$, δ ppm): 8.58 (ddd, J=4.8, 1.8, 1.0 Hz, 1H), 7.81 (ddd, J=7.8, 1.1, 1.0 Hz, 1H), 7.76 (ddd, J=7.8, 7.6, 1.8 Hz, 1H), 7.64 (d, J=8.0 Hz, 1H), 7.44 (dd, J=8.0, 7.4 Hz, 1H), 7.37 (ddd, J=7.6, 4.8, 1.1 Hz, 1H), 7.17-7.11 (m, 4H), 6.91 (d, J=7.4 Hz, 1H), 4.64 (s, 2H), 4.50 (s, 2H), 4.47 (s, 2H), 1.59 (q, J=7.4 Hz, 2H), 1.52 (s, 9H), 1.43 (s, 9H), 1.23 (s, 6H), 0.63 (t, J=7.4 Hz, 3H).

28-(b): <u>{6-[(4-tert-Pentylbenzyl)(pyridin-2-ylsulfonyl)aminomethyl]pyridin-2-ylamino}acetic acid hydrochloride</u>

To 1.8 ml of a methylene chloride solution containing 423 mg (0.662 mmol) of tert-butyl (tert-butoxycarbonyl{6-[(4-tert-pentylbenzyl)(pyridin-2-ylsulfonyl)aminomethyl]pyridin-2-yl}amino)acetate obtained in Example 28-(a) was added 5.3 ml (21 mmol) of 4N hydrogen chloride/1,4-dioxane solution, and the mixture was stirred at room temperature for 20.5 hours. After completion of the reaction, the reaction mixture was concentrated under reduced pressure to obtain 342 mg of the title compound as white solid substantially quantitatively.
Mass Spectrum (FAB, m/z): 483 ($M^+$+1).
$^1$H-NMR Spectrum ($CD_3OD$, δ ppm): 8.74 (ddd, J=4.7, 1.6, 0.9 Hz, 1H), 8.10 (ddd, J=7.7, 7.6, 1.6 Hz, 1H), 8.06 (ddd,

J=7.7, 1.3, 0.9 Hz, 1H), 7.71-7.66 (m, 2H), 7.18-7.12 (m, 4H), 6.82 (d, J=9.0 Hz, 1H), 6.64 (d, J=7.2 Hz, 1H), 4.74 (s, 2H), 4.48 (s, 2H), 4.15 (s, 2H), 1.58 (q, J=7.4 Hz, 2H), 1.19 (s, 6H), 0.60 (t, J=7.4 Hz, 3H).
Rf value: 0.43 (n-butanol:acetic acid:water=10:1:1).

[Example 29]

{6-[(4-Neopentylbenzyl)(pyridin-2-ylsulfonyl)aminomethyl]pyridin-2-ylamino}acetic acid hydrochloride (Exemplary compound No. 226)

29-(a): tert-Butyl (tert-butoxycarbonyl{6-[(4-neopentylbenzyl)(pyridin-2-ylsulfonyl)-aminomethyl]pyridin-2-yl}amino)acetate

To 4.8 ml of a tetrahydrofuran solution containing 200 mg (0.418 mmol) of tert-butyl (tert-butoxycarbonyl {6-[(pyridin-2-ylsulfonyl)aminomethyl]pyridin-2-yl}-amino)acetate obtained in the same manner as in Reference example 2 were added 74.7 mg (0.419 mmol) of 4-neopentylbenzyl alcohol (see W02002/059077A), 0.20 ml (0.80 mmol) of tri-n-butylphosphine and 114 mg (0.662 mmol) of N,N,N',N'-tetramethylazo-dicarboxamide, and the mixture was stirred at room temperature for 23 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The obtained residue was applied to silica gel column chromatography (eluent; n-hexane:ethyl acetate=7:3 (V/V)), and the fractions containing the objective material were concentrated under reduced pressure to obtain 261 mg of the title compound as white foam. (Yield: 98%)
Mass Spectrum (FAB, m/z): 639 ($M^+$+1).
$^1$H-NMR Spectrum ($CDCl_3$, $\delta$ ppm): 8.59 (ddd, J=4.7, 1.7, 1.0 Hz, 1H), 7.82 (ddd, J=7.8, 1.1, 1.0 Hz, 1H), 7.77 (ddd, J=7.8, 7.6, 1.7 Hz, 1H), 7.64 (d, J=8.3 Hz, 1H), 7.44 (dd, J=8.3, 7.6 Hz, 1H), 7.37 (ddd, J=7.6, 4.7, 1.1 Hz, 1H), 7.12-7.08 (m, 2H), 6.98-6.94 (m, 2H), 6.91 (dd, J=7.4, 0.6 Hz, 1H), 4.65 (s, 2H), 4.49 (s, 2H), 4.46 (s, 2H), 2.42 (s, 2H), 1.52 (s, 9H), 1.43 (s, 9H), 0.85 (s, 9H).

29-(b): {6-[(4-Neopentylbenzyl)(pyridin-2-ylsulfonyl)aminomethyl]pyridin-2-ylamino}acetic acid hydrochloride

To 2.3 ml of a methylene chloride solution containing 247 mg (0.387 mmol) of tert-butyl (tert-butoxycarbonyl{6-[(4-neopentylbenzyl)(pyridin-2-ylsulfonyl)aminomethyl]pyridin-2-yl}amino)acetate obtained in Example 29-(a) was added 1.9 ml (7.6 mmol) of 4N hydrogen chloride/1,4-dioxane solution, and the mixture was stirred at room temperature for 17 hours. After completion of the reaction, the reaction mixture was concentrated under reduced pressure, diisopropyl ether was added to the obtained residue and ultrasonication treatment was carried out. The precipitated solid was collected by filtration and dried under reduced pressure to obtain 183 mg of the title compound as white solid. (Yield: 91 %)
Mass Spectrum (FAB, m/z): 483 ($M^+$+1).
$^1$H-NMR Spectrum ($CD_3OD$, $\delta$ ppm): 8.74 (ddd, J=4.7, 1.7, 1.0 Hz, 1H), 8.09 (ddd, J=7.7, 7.6, 1.7 Hz, 1H), 8.05 (ddd, J=7.7, 1.1, 1.0 Hz, 1H), 7.71-7.64 (m, 2H), 7.15-7.10 (m, 2H), 6.99-6.94 (m, 2H), 6.80 (d, J=8.8 Hz, 1H), 6.66 (d, J=7.2 Hz, 1H), 4.72 (s, 2H), 4.49 (s, 2H), 4.12 (s, 2H), 2.40 (s, 2H), 0.83 (s, 9H).
Rf value: 0.43 (n-butanol:acetic acid:water=10:1:1).

[Example 30]

{6-[(4-tert-Butylbenzyl)(4-fluorobenzenesulfonyl)aminomethyl]pyridin-2-ylamino}-acetic acid hydrochloride (Exemplary compound No. 104)

30-(a): tert-Butyl (tert-butoxycarbonyl{6-[(4-tert-butylbenzyl)(4-fluorobenzenesulfonyl)aminomethyl]pyridin-2-yl}amino)acetate

To 1.1 ml of a methylene chloride solution containing 132 mg (0.273 mmol) of tert-butyl (tert-butoxycarbonyl{6-[(4-tert-butylbenzyl)aminomethyl]pyridin-2-yl}-amino)acetate obtained in Reference example 25 were added 64.0 mg (0.329 mmol) of 4-fluorobenzenesulfonyl chloride and 0.076 ml (0.55 mmol) of triethylamine, and the mixture was stirred at room temperature for 6 hours. After completion of the reaction, water was added to the reaction mixture, and the mixture was extracted with methylene chloride. The organic layer was dried over anhydrous sodium sulfate, and concentrated under reduced pressure. The obtained residue was applied to silica gel column chromatography (eluent; n-hexane:ethyl acetate=1:0$\rightarrow$2:1 (V/V)), and the fractions containing the objective material were concentrated under reduced pressure to obtain 163 mg of the title compound as white foam. (Yield: 93%)
Mass Spectrum (FAB, m/z): 642 ($M^+$+1).

$^1$H-NMR Spectrum ($CDCl_3$, δ ppm): 7.72-7.66 (m, 3H), 7.49 (dd, J=8.3, 7.5 Hz, 1H), 7.27-7.24 (m, 2H), 7.12-7.04 (m, 4H), 6.86 (dd, J=7.5, 0.6 Hz, 1H), 4.47 (s, 2H), 4.42 (s, 2H), 4.35 (s, 2H), 1.52 (s, 9H), 1.42 (s, 9H), 1.28 (s, 9H).

30-(b): {6-[(4-tert-Butylbenzyl)(4-fluorobenzenesulfonyl)aminomethyl]pyridin-2-ylamino}acetic acid hydrochloride

To 2.5 ml of a methylene chloride solution containing 161 mg (0.251 mmol) of tert-butyl (tert-butoxycarbonyl{6-[(4-tert-butylbenzyl)(4-fluorobenzenesulfonyl)aminomethyl]pyridin-2-yl}amino)acetate obtained in Example 30-(a) was added 1.3 ml (5.2 mmol) of 4N hydrogen chloride/1,4-dioxane solution, and the mixture was stirred at room temperature for 16 hours. After completion of the reaction, the reaction mixture was concentrated under reduced pressure, acetone was added to the obtained residue and ultrasonication treatment was carried out. After removing the solvent under reduced pressure, the residue was dried under reduced pressure to obtain 137 mg of the title compound as white solid substantially quantitatively.
Mass Spectrum (FAB, m/z): 486 ($M^+$+1).
$^1$H-NMR Spectrum ($CD_3OD$, δ ppm): 8.02-7.96 (m, 2H), 7.64 (dd, J=8.8, 7.3 Hz, 1H), 7.42-7.35 (m, 2H), 7.25-7.21 (m, 2H), 7.15-7.11 (m, 2H), 6.78 (d, J=8.8 Hz, 1H), 6.53 (dd, J=7.3, 0.6 Hz, 1H), 4.42 (s, 2H), 4.40 (s, 2H), 4.12 (s, 2H), 1.23 (s, 9H).
Rf value: 0.67 (n-butanol:acetic acid:water=10:1:1).

[Example 31]

{6-[(4-tert-Butylbenzyl)(3-fluorobenzenesulfonyl)aminomethyl]pyridin-2-ylamino}-acetic acid hydrochloride (Exemplary compound No. 100)

31-(a): tert-Buty(tert-butoxycarbonyl{6-[(4-tert-butylbenzyl)(3-fluorobenzenesulfonyl)aminomethyl]pyridin-2-yl}amino)acetate

To 3.0 ml of a methylene chloride solution containing 130 mg (0.269 mmol) of tert-butyl (tert-butoxycarbonyl{6-[(4-tert-butylbenzyl)aminomethyl]pyridin-2-yl}-amino)acetate obtained in Reference example 25 were added 54.9 mg (0.282 mmol) of 3-fluorobenzenesulfonyl chloride and 0.12 ml (0.86 mmol) of triethylamine, and the mixture was stirred at room temperature for 16 hours. After completion of the reaction, the reaction mixture was applied to silica gel column chromatography (eluent; n-hexane: ethyl acetate=4:1 (V/V)), and the fractions containing the objective material were concentrated under reduced pressure to obtain 128 mg of the title compound as colorless oily product. (Yield: 74%)
$^1$H-NMR Spectrum ($CDCl_3$, δ ppm): 7.69 (d, J=8.4 Hz, 1H), 7.53-7.45 (m, 2H), 7.41-7.36 (m, 2H), 7.27-7.24 (m, 2H), 7.22-7.17 (m, 1H), 7.12-7.08 (m, 2H), 6.86 (d, J=7.5 Hz, 1H), 4.49 (s, 2H), 4.40 (s, 2H), 4.36 (s, 2H), 1.52 (s, 9H), 1.42 (s, 9H), 1.28 (s, 9H).

31-(b): {6-[(4-tert-Butylbenzyl)(3-fluorobenzenesulfonyl)aminomethyl]pyridin-2-ylamino}acetic acid hydrochloride

To 1.1 ml of a methylene chloride solution containing 114 mg (0.178 mmol) of tert-butyl (tert-butoxycarbonyl{6-[(4-tert-butylbenzyl)(3-fluorobenzenesulfonyl)aminomethyl]pyridin-2-yl}amino)acetate obtained in Example 31-(a) was added 0.89 ml (3.6 mmol) of 4N hydrogen chloride/1,4-dioxane solution, and the mixture was stirred at room temperature for 16 hours. After completion of the reaction, the reaction mixture was concentrated under reduced pressure, acetone and diisopropyl ether were added to the obtained residue and ultrasonication treatment was carried out. The precipitated solid was collected by filtration and dried under reduced pressure to obtain 68.0 mg of the title compound as white solid. (Yield: 73%)
Mass Spectrum (FAB, m/z): 486 ($M^+$+1).
$^1$H-NMR Spectrum ($CD_3OD$, δ ppm): 7.79-7.74 (m, 1H), 7.72-7.66 (m, 3H), 7.64 (dd, J=8.8, 7.4 Hz, 1H), 7.52-7.47 (m, 1H), 7.25-7.21 (m, 2H), 7.15-7.12 (m, 2H), 6.78 (d, J=8.8 Hz, 1H), 6.54 (dd, J=7.4, 0.7 Hz, 1H), 4.45 (s, 2H), 4.42 (s, 2H), 4.12 (s, 2H), 1.23 (s, 9H).
Rf value: 0.67 (n-butanol:acetic acid:water=10:1:1).

[Example 32]

<u>{6-[(4-tert-Butylbenzyl)(2-fluorobenzenesulfonyl)aminomethyl]pyridin-2-ylamino}-acetic acid hydrochloride</u> (Exemplary compound No. 96)

32-(a): <u>tert-Butyl (tert-butoxycarbonyl{6-[(4-tert-butylbenzyl)(2-fluorobenzenesulfonyl)aminomethyl]pyridin-2-yl}amino)acetate</u>

To 3.0 ml of a methylene chloride solution containing 130 mg (0.269 mmol) of tert-butyl (tert-butoxycarbonyl{6-[(4-tert-butylbenzyl)aminomethyl]pyridin-2-yl}-amino)acetate obtained in Reference example 25 were added 54.9 mg (0.282 mmol) of 2-fluorobenzenesulfonyl chloride and 0.12 ml (0.86 mmol) of triethylamine, and the mixture was stirred at room temperature for 16 hours. After completion of the reaction, the reaction mixture was applied to silica gel column chromatography (eluent; n-hexane:ethyl acetate=4:1 (V/V)), and the fractions containing the objective material were concentrated under reduced pressure to obtain 128 mg of the title compound as white foam. (Yield: 74%)
$^1$H-NMR Spectrum ($CDCl_3$, $\delta$ ppm): 7.81 (ddd, J=7.7, 7.0, 1.7 Hz, 1H), 7.67 (d, J=8.1 Hz, 1H), 7.51-7.43 (m, 2H), 7.24-7.20 (m, 2H), 7.16 (ddd, J=7.7, 7.7, 1.1 Hz, 1H), 7.10 (ddd, J=10.2, 8.3, 1.1 Hz, 1H), 7.08-7.04 (m, 2H), 6.82 (d, J=7.3 Hz, 1H), 4.57 (s, 2H), 4.48 (s, 2H), 4.45 (s, 2H), 1.52 (s, 9H), 1.44 (s, 9H), 1.27 (s, 9H).

32-(b): <u>{6-[(4-tert-Butylbenzyl)(2-fluorobenzenesulfonyl)aminomethyl]pyridin-2-ylamino}acetic acid hydrochloride</u>

To 1.1 ml of a methylene chloride solution containing 121 mg (0.189 mmol) of tert-butyl (tert-butoxycarbonyl{6-[(4-tert-butylbenzyl)(2-fluorobenzenesulfonyl)aminomethyl]pyridin-2-yl}amino)acetate obtained in Example 32-(a) was added 0.94 ml (3.8 mmol) of 4N hydrogen chloride/1,4-dioxane solution, and the mixture was stirred at room temperature for 16 hours. After completion of the reaction, the reaction mixture was concentrated under reduced pressure, acetone and diisopropyl ether were added to the obtained residue and ultrasonication treatment was carried out. The precipitated solid was collected by filtration and dried under reduced pressure to obtain 82.0 mg of the title compound as white solid. (Yield: 83%)
Mass Spectrum (FAB, m/z): 486 ($M^+$+1).
$^1$H-NMR Spectrum ($CD_3OD$, $\delta$ ppm): 7.97 (ddd, J=7.9, 7.2, 1.8 Hz, 1H), 7.77-7.72 (m, 1H), 7.67 (dd, J=9.0, 7.3 Hz, 1H), 7.43-7.37 (m, 2H), 7.24-7.20 (m, 2H), 7.13-7.09 (m, 2H), 6.81 (d, J=9.0 Hz, 1H), 6.61 (d, J=7.3 Hz, 1H), 4.58 (s, 2H), 4.47 (s, 2H), 4.14 (s, 2H), 1.23 (s, 9H).
Rf value: 0.63 (n-butanol:acetic acid:water=10:1:1).

[Example 33]

<u>{6-[(4-tert-Butylbenzyl)(4-chlorobenzenesulfonyl)aminomethyl]pyridin-2-ylamino}-acetic acid hydrochloride</u> (Exemplary compound No. 110)

33-(a): <u>tert-Butyl (tert-butoxycarbonyl{6-[(4-tert-butylbenzyl)(4-chlorobenzenesulfonyl)aminomethyl]pyridin-2-yl}amino)acetate</u>

To 0.8 ml of a methylene chloride solution containing 100 mg (0.207 mmol) of tert-butyl (tert-butoxycarbonyl{6-[(4-tert-butylbenzyl)aminomethyl]pyridin-2-yl}-amino)acetate obtained in Reference example 25 were added 53.2 mg (0.252 mmol) of 4-chlorobenzenesulfonyl chloride and 0.058 ml (0.42 mmol) of triethylamine, and the mixture was stirred at room temperature for 7 hours. After completion of the reaction, water was added to the reaction mixture, and the mixture was extracted with methylene chloride. The organic layer was dried over anhydrous sodium sulfate, and concentrated under reduced pressure. The obtained residue was applied to silica gel column chromatography (eluent; n-hexane:ethyl acetate=1:0$\rightarrow$2:1 (V/V)), and the fractions containing the objective material were concentrated under reduced pressure to obtain 131 mg of the title compound as white foam. (Yield: 96%)
Mass Spectrum (FAB, m/z): 658 ($M^+$+1).
$^1$H-NMR Spectrum ($CDCl_3$, $\delta$ ppm): 7.72 (d, J=8.1 Hz, 1H), 7.63-7.59 (m, 2H), 7.50 (dd, J=8.1, 7.4 Hz, 1H), 7.39-7.35 (m, 2H), 7.27-7.23 (m, 2H), 7.11-7.07 (m, 2H), 6.86 (dd, J=7.4, 0.6 Hz, 1H), 4.46 (s, 2H), 4.41 (s, 2H), 4.36 (s, 2H), 1.53 (s, 9H), 1.42 (s, 9H), 1.28 (s, 9H).

33-(b): <u>{6-[(4-tert-Butylbenzyl)(4-chlorobenzenesulfonyl)aminomethyl]pyridin-2-ylamino}acetic acid hydrochloride</u>

To 2.0 ml of a methylene chloride solution containing 129 mg (0.196 mmol) of tert-butyl (tert-butoxycarbonyl{6-[(4-tert-butylbenzyl)(4-chlorobenzenesulfonyl)aminomethyl]pyridin-2-yl}amino)acetate obtained in Example 33-(a)

was added 0.98 ml (3.9 mmol) of 4N hydrogen chloride/1,4-dioxane solution, and the mixture was stirred at room temperature for 20 hours. After completion of the reaction, the reaction mixture was concentrated under reduced pressure, diisopropyl ether was added to the obtained residue and ultrasonication treatment was carried out. After removing the solvent under reduced pressure, the residue was dried under reduced pressure to obtain 113 mg of the title compound as white solid substantially quantitatively.
Mass Spectrum (FAB, m/z): 503 ($M^+$+1).
$^1$H-NMR Spectrum ($CD_3OD$, δ ppm): 7.92-7.88 (m, 2H), 7.68-7.61 (m, 3H), 7.25-7.21 (m, 2H), 7.15-7.11 (m, 2H), 6.78 (d, J=9.0 Hz, 1H), 6.54 (dd, J=7.3, 0.6 Hz, 1H), 4.43 (s, 2H), 4.40 (s, 2H), 4.11 (s, 2H), 1.23 (s, 9H).
Rf value: 0.72 (n-butanol:acetic acid:water=10:1:1).

[Example 34]

{6-[(4-tert-Butylbenzyl)(4-methoxybenzenesulfonyl)aminomethyl]pyridin-2-ylamino}-acetic acid hydrochloride (Exemplary compound No. 116)

34-(a): tert-Butyl (tert-butoxycarbonyl{6-[(4-tert-butylbenzyl)(4-methoxybenzenesulfonyl)aminomethyl)pyridin-2-yl}amino)acetate

To 3.0 ml of a methylene chloride solution containing 123 mg (0.254 mmol) of tert-butyl (tert-butoxycarbonyl{6-[(4-tert-butylbenzyl)aminomethyl]pyridin-2-yl}-amino)acetate obtained in Reference example 25 were added 54.4 mg (0.263 mmol) of 4-methoxybenzenesulfonyl chloride and 0.10 ml (0.71 mmol) of triethylamine, and the mixture was stirred at room temperature for 2.5 hours. After completion of the reaction, the reaction mixture was concentrated under reduced pressure. The obtained residue was applied to silica gel column chromatography (eluent; n-hexane:ethyl acetate=1:0→4:1 (V/V)), and the fractions containing the objective material were concentrated under reduced pressure to obtain 153 mg of the title compound as white foam. (Yield: 92%)
Mass Spectrum (FAB, m/z): 654 ($M^+$+1).
$^1$H-NMR Spectrum ($CDCl_3$, δ ppm): 7.68-7.64 (m, 3H), 7.48 (dd, J=8.4, 7.5 Hz, 1H), 7.25-7.21 (m, 2H), 7.09-7.06 (m, 2H), 6.91-6.86 (m, 3H), 4.43 (s, 2H), 4.42 (s, 2H), 4.32 (s, 2H), 3.86 (s, 3H), 1.52 (s, 9H), 1.42 (s, 9H), 1.27 (s, 9H).

34-(b): {6-[(4-tert-Butylbenzyl)(4-methoxybenzenesulfonyl)aminomethyl]pyridin-2-ylamino}acetic acid hydrochloride

To 0.6 ml of a methylene chloride solution containing 149 mg (0.228 mmol) of tert-butyl (tert-butoxycarbonyl{6-[(4-tert-butylbenzyl)(4-methoxybenzenesulfonyl)-aminomethyl]pyridin-2-yl}amino)acetate obtained in Example 34-(a) was added 2.3 ml (9.2 mmol) of 4N hydrogen chloride/1,4-dioxane solution, and the mixture was stirred at room temperature for 13 hours. After completion of the reaction, the reaction mixture was concentrated under reduced pressure, diisopropyl ether was added to the obtained residue and ultrasonication treatment was carried out. The precipitated solid was collected by filtration and dried under reduced pressure to obtain 119 mg of the title compound as white solid. (Yield: 98%)
Mass Spectrum (FAB, m/z): 498 ($M^+$+1).
$^1$H-NMR Spectrum ($CD_3OD$, δ ppm): 7.86-7.81 (m, 2H), 7.58 (dd, J=8.9, 7.2 Hz, 1H), 7.25-7.20 (m, 2H), 7.16-7.09 (m, 4H), 6.72 (d, J=8.9 Hz, 1H), 6.49 (d, J=7.2 Hz, 1H), 4.37 (s, 2H), 4.35 (s, 2H), 4.09 (s, 2H), 3.90 (s, 3H), 1.23 (s, 9H).
Rf value: 0.55 (n-butanol:acetic acid:water=10:1:1).

[Example 35]

{6-[(4-tert-Butylbenzyl)(pyridin-3-ylsulfonyl)aminomethyl]pyridin-2-ylamino}acetic acid hydrochloride (Exemplary compound No. 130)

35-(a): tert-Butyl ({5-bromo-6-[(4-tert-butylbenzyl)(pyridin-3-ylsulfonyl)aminomethyl]pyridin-2-yl}tert-butoxycarbonylamino)acetate

To 0.2 ml of a N,N-dimethylformamide solution containing 11.9 mg (0.0391 mmol) of N-(4-tert-butylbenzyl)pyridin-3-ylsulfonamide obtained in Reference example 27 were added 27.1 mg (pure content: 0.040 mmol) of tert-butyl [(5-bromo-6-bromomethylpyridin-2-yl)tert-butoxycarbonylamino] acetate obtained in the same manner as in Reference example 26-(c) and 11.1 mg (0.0803 mmol) of potassium carbonate, and the mixture was stirred at room temperature for 3.5 hours. After completion of the reaction, water was added to the reaction mixture and the mixture was extracted with ethyl acetate. The organic layer was dried over anhydrous sodium sulfate, and concentrated under reduced pressure. The obtained residue was applied to silica gel column chromatography (eluent; n-hexane: ethyl acetate=1:0→1:1 (V/V)),

and the fractions containing the objective material were concentrated under reduced pressure to obtain 23.4 mg of the title compound as pale yellow foam. (Yield: 85%)
Mass Spectrum (CI, m/z): 703, 705 ($M^+$+1).
$^1$H-NMR Spectrum ($CDCl_3$, δ ppm): 8.99 (dd, J=2.4, 0.9 Hz, 1H), 8.71 (dd, J=4.9, 1.7 Hz, 1H), 7.93 (ddd, J=8.1, 2.4, 1.7 Hz, 1H), 7.70 (d, J=8.9 Hz, 1H), 7.62 (d, J=8.9 Hz, 1H), 7.32 (ddd, J=8.1, 4.9, 0.9 Hz, 1H), 7.28-7.22 (m, 2H), 7.06-7.00 (m, 2H), 4.57 (s, 4H), 4.42 (s, 2H), 1.53 (s, 9H), 1.47 (s, 9H), 1.28 (s, 9H).

35-(b): tert-Butyl (tert-butoxycarbonyl{6-[(4-tert-butylbenzyl)(pyridin-3-ylsulfonyl)-aminomethyl]pyridin-2-yl}amino)acetate

To 0.6 ml of an ethanol solution containing 21.1 mg (0.0300 mmol) of tert-butyl ({5-bromo-6-[(4-tert-butylbenzyl)(pyridin-3-ylsulfonyl)aminomethyl]pyridin-2-yl}tert-butoxycarbonylamino)acetate obtained in Example 35-(a) were added 4.0 mg of 10% palladium-active carbon (50% hydrate) and 0.021 ml (0.15 mmol) of triethylamine, and under hydrogen atmosphere at 1 atm, the mixture was stirred at room temperature for 1 hour. After completion of the reaction, insoluble material was filtered off and the filtrate was concentrated under reduced pressure. The obtained residue was applied to reverse phase C18 column chromatography (eluent; water: acetonitrile=1:0→0:1 (V/V)), and the fractions containing the objective material were concentrated under reduced pressure to obtain 5.8 mg of the title compound as white foam. (Yield: 31%)
Mass Spectrum (FAB, m/z): 625 ($M^+$+1).
$^1$H-NMR Spectrum ($CDCl_3$, δ ppm): 8.94 (dd, J=2.2, 0.6 Hz, 1H), 8.69 (dd, J=4.9, 1.7 Hz, 1H), 7.84 (ddd, J=8.1, 2.2, 1.7 Hz, 1H), 7.71 (d, J=8.3 Hz, 1H), 7.51 (dd, J=8.3, 7.5 Hz, 1H), 7.31-7.25 (m, 3H), 7.15-7.11 (m, 2H), 6.86 (d, J=7.5 Hz, 1H), 4.53 (s, 2H), 4.39 (s, 2H), 4.39 (s, 2H), 1.53 (s, 9H), 1.43 (s, 9H), 1.29 (s, 9H).

35-(c): {6-[(4-tert-Butylbenzyl)(pyridin-3-ylsulfonyl)aminomethyl]pyridin-2-ylamino}-acetic acid hydrochloride

To 0.5 ml of a methylene chloride solution containing 39.4 mg (0.0631 mmol) of tert-butyl (tert-butoxycarbonyl {6-[(4-tert-butylbenzyl)(pyridin-3-ylsulfonyl)aminomethyl]pyridin-2-yl}amino)acetate obtained in the same manner as in Example 35-(b) was added 0.32 ml (1.3 mmol) of 4N hydrogen chloride/1,4-dioxane solution, and the mixture was stirred at room temperature for 16 hours. After completion of the reaction, the reaction mixture was concentrated under reduced pressure, acetone was added to the obtained residue and ultrasonication treatment was carried out. After removing the solvent under reduced pressure, the residue was dried under reduced pressure to obtain 35.0 mg of the title compound as white solid substantially quantitatively.
Mass Spectrum (FAB, m/z): 469 ($M^+$+1).
$^1$H-NMR Spectrum ($CD_3OD$, δ ppm): 9.12 (dd, J=2.4, 0.8 Hz, 1H), 8.90 (dd, J=5.0, 1.5 Hz, 1H), 8.41 (ddd, J=8.1, 2.4, 1.5 Hz, 1H), 7.75 (ddd, J=8.1, 5.0, 0.8 Hz, 1H), 7.69 (dd, J=9.0, 7.3 Hz, 1H), 7.25-7.21 (m, 2H), 7.16-7.12 (m, 2H), 6.83 (d, J=9.0 Hz, 1H), 6.61-6.56 (m, 1H), 4.53 (s, 2H), 4.45 (s, 2H), 4.15 (s, 2H), 1.23 (s, 9H).
Rf value: 0.45 (n-butanol:acetic acid:water=10:1:1).

[Example 36]

{6-[(4-Difluoromethoxybenzyl)(pyridin-3-ylsulfonyl)aminomethyl]pyridin-2-ylamino}-acetic acid hydrochloride (Exemplary compound No. 927)

36-(a): tert-Butyl ({5-bromo-6-[(4-difluoromethoxybenzyl)(pyridin-3-ylsulfonyl)aminomethyl]pyridin-2-yl}tert-butoxycarbonylamino)acetate

To 1.7 ml of a N,N-dimethylformamide solution containing 126 mg (0.401 mmol) of N-(4-difluoromethoxybenzyl)pyridin-3-ylsulfonamide obtained in Reference example 28 were added 193 mg (0.402 mmol) of tert-butyl [(5-bromo-6-bromomethylpyridin-2-yl)tert-butoxycarbonylamino]acetate obtained in Reference example 26-(c) and 110 mg (0.796 mmol) of potassium carbonate, and the mixture was stirred at room temperature for 4 hours. After completion of the reaction, water was added to the reaction mixture and the mixture was extracted with toluene. The organic layer was washed with a saturated aqueous sodium chloride solution, dried over anhydrous sodium sulfate and concentrated under reduced pressure. The obtained residue was applied to silica gel column chromatography (eluent; n-hexane:ethyl acetate=4:1→2:3 (V/V)), and the fractions containing the objective material were concentrated under reduced pressure to obtain 174 mg of the title compound as pale yellow foam. (Yield: 61%)
Mass Spectrum (FAB, m/z): 713, 715 ($M^+$+1).
$^1$H-NMR Spectrum ($CDCl_3$, δ ppm): 9.00 (dd, J=2.4, 0.8 Hz, 1H), 8.74 (dd, J=4.8, 1.7 Hz, 1H), 7.96 (ddd, J=8.1, 2.4, 1.7 Hz, 1H), 7.66 (d, J=8.8 Hz, 1H), 7.60 (d, J=8.8 Hz, 1H), 7.35 (ddd, J=8.1, 4.8, 0.8 Hz, 1H), 7.17-7.13 (m, 2H), 7.01-6.97

(m, 2H), 6.48 (t, J=73.8 Hz, 1H), 4.55 (s, 4H), 4.35 (s, 2H), 1.53 (s, 9H), 1.45 (s, 9H).

36-(b): tert-Butyl (tert-butoxycarbonyl{6-[(4-difluoromethoxybenzyl)(pyridin-3-yl-sulfonyl)aminomethyl]pyridin-2-yl}amino)acetate

To 2.4 ml of an ethanol solution containing 169 mg (0.237 mmol) of tert-butyl ({5-bromo-6-[(4-difluoromethoxybenzyl)(pyridin-3-ylsulfonyl)aminomethyl]pyridin-2-yl}tert-butoxycarbonylamino)acetate obtained in Example 36-(a) were added 24 mg of 10% palladium-active carbon (50% hydrate) and 0.17 ml (1.2 mmol) of triethylamine, and under hydrogen atmosphere at 1 atm, the mixture was stirred at room temperature for 2 hours. After completion of the reaction, insoluble material was filtered off and the filtrate was concentrated under reduced pressure. The obtained residue was applied to silica gel column chromatography (eluent; n-hexane:ethyl acetate=3:2→2:3 (V/V)), and the fractions containing the objective material were concentrated under reduced pressure to obtain 103 mg of the title compound as pale yellow foam. (Yield: 68%)
Mass Spectrum (FAB, m/z): 635 ($M^{+}$+1).
$^{1}$H-NMR Spectrum ($CDCl_3$, $\delta$ ppm): 8.94 (dd, J=2.4, 0.8 Hz, 1H), 8.72 (dd, J=4.8, 1.6 Hz, 1H), 7.87 (ddd, J=8.0, 2.4, 1.6 Hz, 1H), 7.70 (d, J=8.4 Hz, 1H), 7.50 (dd, J=8.4, 7.3 Hz, 1H), 7.32 (ddd, J=8.0, 4.8, 0.8 Hz, 1H), 7.25-7.21 (m, 2H), 7.03-6.99 (m, 2H), 6.84 (dd, J=7.3, 0.6 Hz, 1H), 6.49 (t, J=73.8 Hz, 1H), 4.55 (s, 2H), 4.37 (s, 2H), 4.34 (s, 2H), 1.53 (s, 9H), 1.42 (s, 9H).

36-(c): {6-[(4-Difluoromethoxybenzyl)(pyridin-3-ylsulfonyl)aminomethyl]pyridin-2-ylamino}acetic acid hydrochloride

To 1.5 ml of a methylene chloride solution containing 94.5 mg (0.149 mmol) of tert-butyl (tert-butoxycarbonyl{6-[(4-difluoromethoxybenzyl)(pyridin-3-ylsulfonyl)-aminomethyl]pyridin-2-yl}amino)acetate obtained in Example 36-(b) was added 0.74 ml (3.0 mmol) of 4N hydrogen chloride/1,4-dioxane solution, and the mixture was stirred at room temperature for 16 hours. After completion of the reaction, the reaction mixture was concentrated under reduced pressure, acetone was added to the obtained residue and ultrasonication treatment was carried out. After removing the solvent under reduced pressure, the residue was dried under reduced pressure to obtain 83.0 mg of the title compound as white solid substantially quantitatively.
Mass Spectrum (FAB, m/z): 479 ($M^{+}$+1).
$^{1}$H-NMR Spectrum ($CD_3OD$, $\delta$ ppm): 9.23 (dd, J=2.2, 0.7 Hz, 1H), 8.98 (dd, J=5.3, 1.5 Hz, 1H), 8.61 (ddd, J=8.2,2.2, 1.5 Hz, 1H), 7.92 (ddd, J=8.2, 5.3, 0.7 Hz, 1H), 7.73 (dd, J=9.0, 7.2 Hz, 1H), 7.31-7.25 (m, 2H), 7.01-6.95 (m, 2H), 6.86 (d, J=9.0 Hz, 1H), 6.77 (t, J=73.8 Hz, 1H), 6.67 (d, J=7.2 Hz, 1H), 4.61 (s, 2H), 4.52 (s, 2H), 4.17 (s, 2H).
Rf value: 0.42 (n-butanol:acetic acid:water=10:1:1).

[Example 37]

(6-{[4-(1,1-Dimethylpentyl)benzyl](pyridin-2-ylsulfonyl)aminomethyl}pyridin-2-ylamino)acetic acid (Exemplary compound No. 551)

37-(a): tert-Butyl [tert-butoxycarbonyl(6-{[4-(1,1-dimethylpentyl)benzyl](pyridin-2-yl-sulfonyl)aminomethyl}pyridin-2-yl)amino]acetate

To 2.5 ml of a tetrahydrofuran solution containing 253 mg (0.529 mmol) of tert-butyl (tert-butoxycarbonyl{6-[(pyridin-2-ylsulfonyl)aminomethyl]pyridin-2-yl}-amino)acetate obtained in the same manner as in Reference example 2 were added 109 mg (0.528 mmol) of 4-(1,1-dimethylpentyl)benzyl alcohol obtained in Reference example 29-(b), 260 μl (1.04 mmol) of tri-n-butylphosphine and 182 mg (1.06 mmol) of N,N,N',N'-tetramethylazodicarboxamide, and the mixture was stirred at room temperature for 16 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; toluene: ethyl acetate=5:1→4:1 (V/V)), and the fractions containing the objective material were concentrated under reduced pressure to obtain 312 mg of the title compound as colorless oily product. (Yield: 89%) Mass Spectrum (FAB, m/z): 667 ($M^{+}$+1).
$^{1}$H-NMR Spectrum ($CDCl_3$, $\delta$ ppm): 8.58 (ddd, J=4.7, 1.7, 0.9 Hz, 1H), 7.83-7.79 (m, 1H), 7.76 (ddd, J=7.6, 7.4, 1.7 Hz, 1H), 7.64 (d, J=8.4 Hz, 1H), 7.44 (dd, J=8.4, 7.4 Hz, 1H), 7.37 (ddd, J=7.4, 4.7, 1.5 Hz, 1H), 7.19-7.09 (m, 4H), 6.90 (d, J=7.4 Hz, 1H), 4.64 (s, 2H), 4.50 (s, 2H), 4.47 (s, 2H), 1.58-1.49 (m, 2H), 1.52 (s, 9H), 1.43 (s, 9H), 1.29-1.14 (m, 2H), 1.23 (s, 6H), 1.04-0.91 (m, 2H), 0.83 (t, J=7.3 Hz, 3H).

37-(b): (6-{[4-(1,1-Dimethylpentyl)benzyl](pyridin-2-ylsulfonyl)aminomethyl}pyridin-2-ylamino}acetic acid

To 1 ml of a tetrahydrofuran solution containing 284 mg (0.426 mmol) of tert-butyl [tert-butoxycarbonyl(6-{[4-(1,1-dimethylpentyl)benzyl](pyridin-2-ylsulfonyl)-aminomethyl}pyridin-2-yl)amino]acetate obtained in Example 37-(a) were added 0.36 ml of water and 0.54 ml of concentrated hydrochloric acid, and the mixture was stirred at 70°C for 6 hours. After completion of the reaction, the reaction mixture was concentrated under reduced pressure, and 10 ml of water was added to the residue. The mixture was adjusted to pH 4.4 with 2N aqueous sodium hydroxide solution, and extracted with tert-butyl methyl ether. The organic layer was washed with a saturated aqueous sodium chloride solution, dried over anhydrous magnesium sulfate and concentrated under reduced pressure to obtain 174 mg of the title compound as white foam. (Yield: 80%)
Mass Spectrum (FAB, m/z): 511 ($M^+$+1).
$^1$H-NMR Spectrum (DMSO-$d_6$, $\delta$ ppm): 8.61 (ddd, J=4.7, 1.8, 0.9 Hz, 1H), 7.93 (ddd, J=7.8, 7.7, 1.8 Hz, 1H), 7.77 (ddd, J=7.8, 1.1, 0.9 Hz, 1H), 7.55 (ddd, J=7.7, 4.7, 1.1 Hz, 1H), 7.24-7.11 (m, 4H), 7.18 (dd, J=8.2, 7.1 Hz, 1H), 6.72 (t, J=5.8 Hz, 0.8H), 6.33 (d, J=8.2 Hz, 1H), 6.26 (d, J=7.1 Hz, 1H), 4.64 (s, 2H), 4.22 (s, 2H), 3.81 (d, J=5.8 Hz, 2H), 1.59-1.49 (m, 2H), 1.26-1.10 (m, 2H), 1.21 (s, 6H), 1.01-0.86 (m, 2H), 0.79 (t, J=7.2 Hz, 3H).
Rf value: 0.49 (n-butanol:acetic acid:water=10:1:1).

[Example 38]

(6-{[4-(1,1,3,3-Tetramethylbutyl)benzyl](pyridin-2-ylsulfonyl)aminomethyl}pyridin-2-ylamino)acetic acid (Exemplary compound No. 740)

38-(a): tert-butyl [tert-butoxycarbonyl(6-{[4-(1,1,3,3-tetramethylbutyl)benzyl](pyridin-2-ylsulfonyl)aminomethyl}pyridin-2-yl)amino]acetate

To 3.5 ml of a tetrahydrofuran solution containing 360 mg (0.752 mmol) of tert-butyl (tert-butoxycarbonyl{6-[(pyridin-2-ylsulfonyl)aminomethyl]pyridin-2-yl}-amino)acetate obtained in the same manner as in Reference example 2 were added 164 mg (0.744 mmol) of 4-(1,1,3,3-tetramethylbutyl)benzyl alcohol obtained in Reference example 30, 275 μl (1.10 mmol) of tri-n-butylphosphine and 192 mg (1.12 mmol) of N,N,N',N'-tetramethylazodicarboxamide, and the mixture was stirred at room temperature for 16 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; toluene: ethyl acetate=9:1 (V/V)), and the fractions containing the objective material were concentrated under reduced pressure to obtain 347 mg of the title compound as white foam. (Yield: 68%)
Mass Spectrum (FAB, m/z): 681 ($M^+$+1).
$^1$H-NMR Spectrum ($CDCl_3$, $\delta$ ppm): 8.58 (ddd, J=4.7, 1.8, 1.0 Hz, 1H), 7.82 (ddd, J=7.8, 1.1, 1.0 Hz, 1H), 7.76 (ddd, J=7.8, 7.6, 1.8 Hz, 1H), 7.64 (d, J=7.9 Hz, 1H), 7.44 (dd, J=7.9, 7.4 Hz, 1H), 7.37 (ddd, J=7.6, 4.7, 1.1 Hz, 1H), 7.23-7.19 (m, 2H), 7.13-7.09 (m, 2H), 6.90 (d, J=7.4 Hz, 1H), 4.65 (s, 2H), 4.47 (s, 2H), 4.47 (s, 2H), 1.69 (s, 2H), 1.52 (s, 9H), 1.43 (s, 9H), 1.31 (s, 6H), 0.67 (s, 9H).

38-(b): (6-{[4(1,1,3,3-Tetramethylbutyl)benzyl](pyridin-2-ylsulfonyl)aminomethyl}-pyridin-2-ylamino)acetic acid

To 1.0 ml of a tetrahydrofuran solution containing 341 mg (0.501 mmol) of tert-butyl [tert-butoxycarbonyl(6-{[4-(1,1,3,3-tetramethylbutyl)benzyl](pyridin-2-ylsulfonyl)aminomethyl}pyridin-2-yl)amino]acetate obtained in Example 38-(a) were added 0.60 ml of water and 0.42 ml of concentrated hydrochloric acid, and the mixture was stirred at 65°C for 3.5 hours. After completion of the reaction, 15 ml of water was added to the reaction mixture. The mixture was adjusted to pH 4.4 with 2N aqueous sodium hydroxide solution, and extracted with ethyl acetate. The organic layer was washed with a saturated aqueous sodium chloride solution, dried over anhydrous magnesium sulfate and concentrated under reduced pressure to obtain 228 mg of the title compound as white foam. (Yield: 87%)
Mass Spectrum (FAB, m/z): 525 ($M^+$+1).
$^1$H-NMR Spectrum (DMSO-$d_6$, $\delta$ ppm): 12.41 (brs, 0.8H), 8.62 (ddd, J=4.7, 1.8, 0.9 Hz, 1H), 7.94 (ddd, J=7.8, 7.7, 1.8 Hz, 1H), 7.79 (ddd, J=7.8, 1.1, 0.9 Hz, 1H), 7.57 (ddd, J=7.7, 4.7, 1.1 Hz, 1H), 7.29-7.25 (m, 2H), 7.18 (dd, J=8.4, 7.2 Hz, 1H), 7.14-7.11 (m, 2H), 6.72 (t, J=5.6 Hz, 0.8H), 6.33 (dd, J=8.4, 0.6 Hz, 1H), 6.26 (dd, J=7.2, 0.6 Hz, 1H), 4.63 (s, 2H), 4.19 (s, 2H), 3.82 (d, J=5.6 Hz, 2H), 1.69 (s, 2H), 1.29 (s, 6H), 0.66 (s, 9H).
Rf value: 0.50 (n-butanol:acetic acid:water=10:1:1).

[Example 39]

{6-[(4-Pentylbenzyl)(pyridin-2-ylsulfonyl)aminomethyl]pyridin-2-ylamino}acetic acid (Exemplary compound No. 167)

39-(a): tert-Butyl (tert-butoxycarbonyl{6-[(4-pentylbenzyl)(pyridin-2-ylsulfonyl)aminomethyl]pyridin-2-yl}amino)acetate

To 4.0 ml of a tetrahydrofuran solution containing 363 mg (0.759 mmol) of tert-butyl (tert-butoxycarbonyl{6-[(pyridin-2-ylsulfonyl)aminomethyl]pyridin-2-yl}-amino)acetate obtained in the same manner as in Reference example 2 were added 134 mg (0.752 mmol) of 4-pentylbenzyl alcohol (see Journal of the American Chemical Society, 119, 2119 (1997)), 278 μl (1.11 mmol) of tri-n-butylphosphine and 194 mg (1.13 mmol) of N,N,N',N'-tetramethylazodicarboxamide, and the mixture was stirred at room temperature for 2 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; toluene:ethyl acetate=5:1 (V/V)), and the fractions containing the objective material were concentrated under reduced pressure to obtain 416 mg of the title compound as colorless oily product.
(Yield: 87%)
Mass Spectrum (FAB, m/z): 639 ($M^+$+1).
$^1$H-NMR Spectrum ($CDCl_3$, δ ppm): 8.59 (ddd, J=4.6, 1.6, 0.9 Hz, 1H), 7.81 (ddd, J=7.7, 1.6, 0.9 Hz, 1H), 7.76 (ddd, J=7.7, 7.4, 1.6 Hz, 1H), 7.64 (d, J=8.3 Hz, 1H), 7.44 (dd, J=8.3, 7.5 Hz, 1H), 7.37 (ddd, J=7.4, 4.6, 1.6 Hz, 1H), 7.15-7.08 (m, 2H), 7.05-6.99 (m, 2H), 6.89 (d, J=7.5 Hz, 1H), 4.65 (s, 2H), 4.47 (s, 2H), 4.45 (s, 2H), 2.53 (t, J=7.7 Hz, 2H), 1.64-1.23 (m, 6H), 1.52 (s, 9H), 1.43 (s, 9H), 0.89 (t, J=7.0 Hz, 3H).

39-(b): {6-[(4-Pentylbenzyl)(pyridin-2-ylsulfonyl)aminomethyl]pyridin-2-ylamino}-acetic acid

To 407 mg (0.637 mmol) of tert-butyl (tert-butoxycarbonyl{6-[(4-pentylbenzyl)(pyridin-2-ylsulfonyl)aminomethyl]pyridin-2-yl}amino)acetate obtained in obtained in Example 39-(a) was added 10 ml of 30vol.% trifluoroacetic acid/methylene chloride solution, and the mixture was stirred at room temperature for 5 hours. After completion of the reaction, the reaction mixture was concentrated under reduced pressure, 10 ml of water was added to the mixture, and the mixture was adjusted to pH 4.3 with 1N aqueous sodium hydroxide solution and 1N hydrochloric acid, and extracted with tert-butyl methyl ether. The organic layer was washed with a saturated aqueous sodium chloride solution, dried over anhydrous magnesium sulfate and concentrated under reduced pressure to obtain 276 mg of the title compound as pale yellowish white foam. (Yield: 90%)
Mass Spectrum (FAB, m/z): 483 ($M^+$+1).
$^1$H-NMR Spectrum (DMSO-$d_6$, δ ppm): 12.39 (brs, 0.3H), 8.62 (ddd, J=4.7, 1.8, 0.9 Hz, 1H), 7.94 (ddd, J=7.8, 7.7, 1.8 Hz, 1H), 7.78 (ddd, J=7.8, 1.0, 0.9 Hz, 1H), 7.56 (ddd, J=7.7, 4.7, 1.0 Hz, 1H), 7.19 (dd, J=8.2, 7.1 Hz, 1H), 7.14-7.11 (m, 2H), 7.10-7.06 (m, 2H), 6.72 (t, J=5.8 Hz, 0.9H), 6.33 (d, J=8.2 Hz, 1H), 6.26 (d, J=7.1 Hz, 1H), 4.64 (s, 2H), 4.20 (s, 2H), 3.81 (d, J=5.8 Hz, 2H), 2.59-2.49 (m, 2H), 1.57-1.49 (m, 2H), 1.35-1.20 (m, 4H), 0.86 (t, J=7.2 Hz, 3H).
Rf value: 0.48 (n-butanol:acetic acid:water=10:1:1).

[Example 40]

{6-{[4-(1-Methylpentyl)benzyl](pyridin-2-ylsulfonyl)aminomethyl}pyridin-2-ylamino)-acetic acid (Exemplary compound No. 357)

40-(a): tert-Butyl [tert-butoxycarbonyl(6-{[4-(1-methylpentyl)benzyl](pyridin-2-yl-sulfonyl)aminomethyl}pyridin-2-yl)amino]acetate

To 4.0 ml of a tetrahydrofuran solution containing 377 mg (0.788 mmol) of tert-butyl (tert-butoxycarbonyl {6-[(pyridin-2-ylsulfonyl)aminomethyl)pyridin-2-yl}-amino)acetate obtained in the same manner as in Reference example 2 were added 144 mg (0.749 mmol) of 4-(1-methylpentyl)benzyl alcohol obtained in Reference example 31-(f), 278 μl (1.11 mmol) of tri-n-butylphosphine and 194 mg (1.13 mmol) of N,N,N',N'-tetramethylazodicarboxamide, and the mixture was stirred at room temperature for 16 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; toluene:ethyl acetate=6:1(V/V)), and the fractions containing the objective material were concentrated under reduced pressure to obtain 461 mg of the title compound as colorless oily product. (Yield: 94%)

Mass Spectrum (FAB, m/z): 653 ($M^{+}$+1).
$^1$H-NMR Spectrum ($CDCl_3$, δ ppm): 8.58 (ddd, J=4.7, 1.7, 0.9 Hz, 1H), 7.81 (ddd, J=7.7, 1.4, 0.9 Hz, 1H), 7.75 (ddd, J=7.7, 7.5, 1.7 Hz, 1H), 7.64 (d, J=8.3 Hz, 1H), 7.44 (dd, J=8.3, 7.3 Hz, 1H), 7.36 (ddd, J=7.5, 4.7, 1.4 Hz, 1H), 7.15-7.08 (m, 2H), 7.04-6.97 (m, 2H), 6.90 (d, J=7.3 Hz, 1H), 4.64 (s, 2H), 4.50 (s, 2H), 4.47 (s, 2H), 2.69-2.51 (m, 1H), 1.58-1.02 (m, 6H), 1.52 (s, 9H), 1.43 (s, 9H), 1.17 (d, J=6.8 Hz, 3H), 0.85 (t, J=7.2 Hz, 3H).

40-(b): (6-{[4-(1-Methylpentyl)benzyl](pyridin-2-ylsulfonyl)aminomethyl)pyridin-2-ylamino)acetic acid

To 457 mg (0.700 mmol) of tert-butyl [tert-butoxycarbonyl(6-{[4-(1-methylpentyl)benzyl] (pyridin-2-ylsulfonyl)aminomethyl}pyridin-2-yl)amino]acetate obtained in Example 40-(a) was added 10 ml of 30vol.% trifluoroacetic acid/methylene chloride solution, and the mixture was stirred at room temperature for 17 hours. After completion of the reaction, the reaction mixture was concentrated under reduced pressure, 10 ml of water was added to the mixture, and the mixture was adjusted to pH 4.4 with 1N aqueous sodium hydroxide solution and 1N hydrochloric acid, and extracted with tert-butyl methyl ether. The organic layer was washed with a saturated aqueous sodium chloride solution, dried over anhydrous magnesium sulfate and concentrated under reduced pressure to obtain 334 mg of the title compound as white foam. (Yield: 96%) Mass Spectrum (FAB, m/z): 497 ($M^{+}$+1).
$^1$H-NMR Spectrum (DMSO-$d_6$, δ ppm): 12.39 (brs, 0.8H), 8.61 (ddd, J=4.6, 1.8, 0.9 Hz, 1H), 7.93 (ddd, J=7.8, 7.6, 1.8 Hz, 1H), 7.77 (ddd, J=7.8, 1.0, 0.9 Hz, 1H), 7.56 (ddd, J=7.6, 4.6, 1.0 Hz, 1H), 7.18 (dd, J=8.2, 7.2 Hz, 1H), 7.16-7.12 (m, 2H), 7.10-7.05 (m, 2H), 6.74 (t, J=5.8 Hz, 0.9H), 6.33 (d, J=8.2 Hz, 1H), 6.26 (d, J=7.2 Hz, 1H), 4.64 (s, 2H), 4.22 (s, 2H), 3.82 (d, J=5.8 Hz, 2H), 2.65-2.56 (m, 1H), 1.53-1.45 (m, 2H), 1.32-0.98 (m, 4H), 1.14 (d, J=7.0 Hz, 3H), 0.82 (t, J=7.3 Hz, 3H).
Rf value: 0.49 (n-butanol:acetic acid:water=10:1:1).

[Example 41]

{6-[(3-Butylbenzyl)(pyridin-2-ylsulfonyl)aminomethyl]pyridin-2-ylamino}acetic acid (Exemplary compound No. 48)

41-(a): tert-Butyl (tert-butoxycarbon{6-[(3-butylbenzyl)(pyridin-2-ylsulfonyl)aminomethyl]pyridin-2-yl}amino)acetate

To 4.8 ml of a tetrahydrofuran solution containing 200 mg (0.418 mmol) of tert-butyl (tert-butoxycarbonyl{6-[(pyridin-2-ylsulfonyl)aminomethyl]pyridin-2-yl}-amino)acetate obtained in the same manner as in Reference example 2 were added 68.6 mg (0.418 mmol) of 3-butylbenzyl alcohol obtained in Reference example 32-(b), 198 μl (0.793 mmol) of tri-n-butylphosphine and 113 mg (0.656 mmol) of N,N,N',N'-tetramethylazodicarboxamide, and the mixture was stirred at room temperature for 16 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=3:2 (V/V), and the fractions containing the objective material were concentrated under reduced pressure to obtain 232 mg of the title compound as colorless oily product. (Yield: 89%)
Mass Spectrum (FAB, m/z): 625 ($M^{+}$+1).
$^1$H-NMR Spectrum ($CDCl_3$, δ ppm): 8.60 (ddd, J=4.7, 1.7, 0.9 Hz, 1H), 7.82 (ddd, J=7.7, 1.5, 0.9 Hz, 1H), 7.77 (ddd, J=7.7, 7.4, 1.7 Hz, 1H), 7.63 (d, J=8.3 Hz, 1H), 7.45 (dd, J=8.3, 7.4 Hz, 1H), 7.38 (ddd, J=7.4, 4.7, 1.5 Hz, 1H), 7.15-7.08 (m, 2H), 7.04-6.98 (m, 2H), 6.92 (d, J=7.4 Hz, 1H), 4.66 (s, 2H), 4.48 (s, 2H), 4.44 (s, 2H), 2.50 (t, J=7.7 Hz, 2H), 1.58-1.20 (m, 4H), 1.52 (s, 9H), 1.42 (s, 9H), 0.92 (t, J=7.2 Hz, 3H).

41-(b): {6-[(3-Butylbenzyl)(pyridin-2-ylsulfonyl)aminomethyl]pyridin-2-ylamino}-acetic acid

To 2.07 ml of a methylene chloride solution containing 222 mg (0.355 mmol) of tert-butyl (tert-butoxycarbonyl{6-[(3-butylbenzyl)(pyridin-2-ylsulfonyl)aminomethyl]pyridin-2-yl}amino)acetate obtained in Example 41-(a) was added 1.72 ml (6.9 mmol) of 4N hydrogen chloride/1,4-dioxane solution, and the mixture was stirred at room temperature for 16 hours. After completion of the reaction, the reaction mixture was concentrated under reduced pressure, and water was added to the residue. The mixture was adjusted to pH 4.5 with 1N aqueous sodium hydroxide solution and 1N hydrochloric acid, and the precipitated solid was collected by filtration and dried at 45°C under reduced pressure to obtain 91.7 mg of the title compound as white solid. (Yield: 55%)
Mass Spectrum (FAB, m/z): 469 ($M^{+}$+1).
$^1$H-NMR Spectrum (DMSO-$d_6$, δ ppm): 8.64 (ddd, J=4.5, 1.8, 0.9 Hz, 1H), 7.96 (ddd, J=7.8, 7.7, 1.8 Hz, 1H), 7.81 (ddd, J=7.8, 1.1, 0.9 Hz, 1H), 7.59 (ddd, J=7.7, 4.5, 1.1 Hz, 1H), 7.20 (dd, J=8.4, 7.2 Hz, 1H), 7.18-7.14 (m, 1H), 7.07-7.01 (m, 2H), 6.96-6.94 (m, 1H), 6.68 (t, J=5.4 Hz, 0.8H), 6.35 (d, J=8.4 Hz, 1H), 6.30 (d, J=7.2 Hz, 1H), 4.63 (s, 2H), 4.22

(s, 2H), 3.81 (d, J=5.4 Hz, 2H), 2.47 (t, J=7.7 Hz, 2H), 1.50-1.42 (m, 2H), 1.31-1.23 (m, 2H), 0.89 (t, J=7.3 Hz, 3H).
Rf value: 0.48 (n-butanol:acetic acid:water=10:1:1).

[Example 42]

{6-[(3-Hexylbenzyl)(pyridin-2-ylsulfonyl)aminomethyl]pyridin-2-ylamino}acetic acid (Exemplary compound No. 334)

42-(a): tert-Butyl (tert-butoxycarbonyl{6-[(3-hexylbenzyl)(pyridin-2-ylsulfonyl)aminomethyl]pyridin-2-yl}amino)acetate

To 4.8 ml of a tetrahydrofuran solution containing 200 mg (0.418 mmol) of tert-butyl (tert-butoxycarbonyl{6-[(pyridin-2-ylsulfonyl)aminomethyl]pyridin-2-yl}-amino)acetate obtained in the same manner as in Reference example 2 were added 80.4 mg (0.418 mmol) of 3-hexylbenzyl alcohol (see Synlett, 671 (1998)), 198 μl (0.793 mmol) of tri-n-butylphosphine and 113 mg (0.656 mmol) of N,N,N',N'-tetramethylazo-dicarboxamide, and the mixture was stirred at room temperature for 48 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=7:3 (V/V), and the fractions containing the objective material were concentrated under reduced pressure to obtain 260 mg of the title compound as colorless oily product. (Yield: 95%)
Mass Spectrum (FAB, m/z): 653 ($M^+$+1).
$^1$H-NMR Spectrum ($CDCl_3$, δ ppm): 8.60 (ddd, J=4.8, 1.6, 0.9 Hz, 1H), 7.84-7.79 (m, 1H), 7.76 (ddd, J=7.6, 7.4, 1.6 Hz, 1H), 7.64 (d, J=8.3 Hz, 1H), 7.45 (dd, J=8.3, 7.4 Hz, 1H), 7.38 (ddd, J=7.4, 4.8, 1.6 Hz, 1H), 7.15-7.08 (m, 1H), 7.03-6.98 (m, 3H), 6.91 (d, J=7.4 Hz, 1H), 4.66 (s, 2H), 4.48 (s, 2H), 4.44 (s, 2H), 2.49 (t, J=7.8 Hz, 2H), 1.52 (s, 9H), 1.42 (s, 9H), 1.35-1.20 (m, 8H), 0.89 (t, J=6.7 Hz, 3H).

42-(b): {6-[(3-Hexylbenzyl)(pyridin-2-ylsulfonyl)aminomethyl]pyridin-2-ylamino}-acetic acid

To 2.2 ml of a methylene chloride solution containing 247 mg (0.378 mmol) of tert-butyl (tert-butoxycarbonyl{6-[(3-hexylbenzyl)(pyridin-2-ylsulfonyl)aminomethyl]-pyridin-2-yl}amino)acetate obtained in Example 42-(a) was added 1.82 ml (7.3 mmol) of 4N hydrogen chloride/1,4-dioxane solution, and the mixture was stirred at room temperature for 24 hours. After completion of the reaction, the reaction mixture was concentrated under reduced pressure, and water was added to the residue. The mixture was adjusted to pH 4.5 with 1N aqueous sodium hydroxide solution and 1N hydrochloric acid, and extracted with ethyl acetate. The organic layer was washed with a saturated aqueous sodium chloride solution, dried over anhydrous magnesium sulfate and concentrated under reduced pressure to obtain 151 mg of the title compound as white solid. (Yield: 80%)
Mass Spectrum (FAB, m/z): 497 ($M^+$+1).
$^1$H-NMR Spectrum (DMSO-$d_6$, δ ppm): 8.64 (ddd, J=4.7, 1.7, 0.9 Hz, 1H), 7.96 (ddd, J=7.8, 7.7, 1.7 Hz, 1H), 7.81 (ddd, J=7.8, 1.1, 0.9 Hz, 1H), 7.58 (ddd, J=7.7, 4.7, 1.1 Hz, 1H), 7.20 (dd, J=8.3, 7.1 Hz, 1H), 7.18-7.14 (m, 1H), 7.07-7.00 (m, 2H), 6.96-6.94 (m, 1H), 6.72 (t, J=5.6 Hz, 0.9H), 6.35 (d, J=8.3 Hz, 1H), 6.30 (d, J=7.1 Hz, 1H), 4.64 (s, 2H), 4.21 (s, 2H), 3.83 (d, J=5.6 Hz, 2H), 2.47 (t, J=7.5 Hz, 2H), 1.52-1.43 (m, 2H), 1.30-1.22 (m, 6H), 0.86 (t, J=6.8 Hz, 3H).
Rf value: 0.51 (n-butanol:acetic acid:water=10:1:1).

[Example 43]

(6-{[4-(1,1-Dimethylpentyl)benzyl](pyridin-4-ylsulfonyl)aminomethyl}pyridin-2-ylamino)acetic acid (Exemplary compound No. 567)

43-(a): tert-Butyl [tert-butoxycarbonyl(6-{[4-(1,1-dimethylpentyl)benzyl](pyridin-4-yl-sulfonyl)aminomethyl}pyridin-2-yl)amino]acetate

To 4 ml of a tetrahydrofuran solution containing 272 mg (0.785 mmol) of N-[4-(1,1-dimethylpentyl)benzyl]pyridin-4-ylsulfonamide obtained in Reference example 33-(b) were added 292 mg (0.863 mmol) of tert-butyl [tert-butoxycarbonyl(6-hydroxy-methylpyridin-2-yl)amino]acetate obtained in Reference example 1-(b), 0.29 ml (1.2 mmol) of tri-n-butylphosphine and 204 mg (1.18 mmol) of N,N,N',N'-tetramethylazo-dicarboxamide, and the mixture was stirred at room temperature for 2 hours. After completion of the reaction, the reaction mixture was concentrated under reduced pressure, water was added to the residue, and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=4:1→2:1 (V/V)), and the fractions containing the objective material were concentrated under reduced pressure to

obtain 535 mg of the title compound as colorless oily product substantially quantitatively.
Mass Spectrum (CI, m/z): 667 ($M^+$+1).
$^1$H-NMR Spectrum ($CDC1_3$, δ ppm): 8.70 (dd, J=4.4, 1.7, Hz, 2H), 7.73 (d, J=8.5 Hz, 1H), 7.54-7.47 (m, 1H), 7.48 (dd, J=4.4, 1.7 Hz, 2H), 7.25-7.18 (m, 2H), 7.14-7.07 (m, 2H), 6.84 (d, J=7.1 Hz, 1H), 4.52 (s, 2H), 4.39 (s, 2H), 4.35 (s, 2H), 1.61-1.48 (m, 2H), 1.53 (s, 9H), 1.43 (s, 9H), 1.26-1.14 (m, 2H), 1.25 (s, 6H), 1.06-0.93 (m, 2H), 0.82 (t, J=7.2 Hz, 3H).

43-(b): (6-{[4-(1,1-Dimethylpentyl)benzyl](pyridin-4-ylsulfonyl)aminomethyl}pyridin-2-ylamino)acetic acid

To 8 ml of a methylene chloride solution containing 532 mg (0.798 mmol) of tert-butyl [tert-butoxycarbonyl(6-{[4-(1,1-dimethylpentyl)benzyl](pyridin-4-ylsulfon-yl)aminomethyl}pyridin-2-yl)amino]acetate obtained in Example 43-(a) was added 4.0 ml (54 mmol) of trifluoroacetic acid, and the mixture was stirred at room temperature for 9 hours. After completion of the reaction, the reaction mixture was concentrated under reduced pressure, and 2 ml of water was added to the residue. The mixture was adjusted to pH 4.2 with a saturated aqueous sodium hydrogen carbonate solution and 1N hydrochloric acid, and extracted with ethyl acetate. The organic layer was washed with a saturated aqueous sodium chloride solution, dried over anhydrous sodium sulfate and concentrated under reduced pressure. The residue was applied to reverse phase C18 column chromatography (eluent; water:acetonitrile=7:3→3:7 (V/V)), and the fractions containing the objective material were concentrated under reduced pressure. To the residue was added 1.8 ml of diisopropyl ether, and precipitated solid was collected by filtration, and dried at 50°C under reduced pressure to obtain 345 mg of the title compound as white solid. (Yield: 85%)
Mass Spectrum (FAB, m/z): 511 ($M^+$+1).
$^1$H-NMR Spectrum (DMSO-$d_6$, δ ppm): 12.41 (brs, 0.2H), 8.67 (dd, J=4.4, 1.5 Hz, 2H), 7.54 (dd, J=4.4, 1.5 Hz, 2H), 7.29-7.25 (m, 2H), 7.22-7.17 (m, 2H), 7.21 (dd, J=8.3, 7.2 Hz, 1H), 6.78 (t, J=5.9 Hz, 0.9H), 6.37 (d, J=8.3 Hz, 1H), 6.29 (d, J=7.2 Hz, 1H), 4.63 (s, 2H), 4.16 (s, 2H), 3.72 (d, J=5.9 Hz, 2H), 1.58-1.52 (m, 2H), 1.23 (s, 6H), 1.22-1.14 (m, 2H), 0.99-0.91 (m, 2H), 0.79 (t, J=7.3 Hz, 3H).
Rf value: 0.52 (n-butanol:acetic acid:water=10:1:1).

[Example 44]

{6-[(3,4-Diethylbenzyl)(pyridin-2-ylsulfonyl)aminomethyl]pyridin-2-ylamino}acetic acid (Exemplary compound No. 19)

44-(a): tert-Butyl (tert-butoxycarbonyl{6-[(3,4-diethylbenzyl)(pyridin-2-ylsulfonyl)-aminomethyl]pyridin-2-yl} amino)acetate

To 3.7 ml of a tetrahydrofuran solution containing 386 mg (0.807 mmol) of tert-butyl (tert-butoxycarbonyl{6-[(pyridin-2-ylsulfonyl)aminomethyl]pyridin-2-yl}-amino)acetate obtained in the same manner as in Reference example 2 were added 120 mg (0.731 mmol) of 3,4-diethylbenzyl alcohol (see W02004/037811A), 275μl (1.10 mmol) of tri-n-butylphosphine and 189 mg (1.10 mmol) of N,N,N',N'-tetramethyl-azodicarboxamide, and the mixture was stirred at room temperature for 3 hours. After completion of the reaction, the reaction mixture was concentrated under reduced pressure, water was added to the residue, and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=4:1 (V/V)), and the fractions containing the objective material were concentrated under reduced pressure to obtain 392 mg of the title compound as pale yellow oil. (Yield: 86%)
Mass Spectrum (CI, m/z): 625 ($M^+$+1).
$^1$H-NMR Spectrum ($CDCl_3$, δ ppm): 8.62-8.58 (m, 1H), 7.84-7.79 (m, 1H), 7.76 (ddd, J=7.6, 7.4, 1.7 Hz, 1H), 7.64 (d, J=8.2 Hz, 1H), 7.45 (dd, J=8.2, 7.3 Hz, 1H), 7.37 (ddd, J=7.4, 4.8, 1.5 Hz, 1H), 7.03-6.90 (m, 4H), 4.63 (s, 2H), 4.49 (s, 2H), 4.46 (s, 2H), 2.58 (q, J=7.5 Hz, 2H), 2.54 (q, J=7.5 Hz, 2H), 1.52 (s, 9H), 1.42 (s, 9H), 1.17 (t, J=7.5 Hz, 3H), 1.13 (t, J=7.5 Hz, 3H).

44-(b): {6-[(3,4-Diethylbenzyl)(pyridin-2-ylsulfonyl)aminomethyl]pyridin-2-ylamino}-acetic acid

To 6 ml of a methylene chloride solution containing 390 mg (0.624 mmol) of tert-butyl (tert-butoxycarbonyl{6-[(3,4-diethylbenzyl)(pyridin-2-ylsulfonyl)aminomethyl]pyridin-2-yl}amino)acetate obtained in Example 44-(a) was added 3.0 ml (40 mmol) of trifluoroacetic acid, and the mixture was stirred at room temperature for 5 hours. After completion of the reaction, the reaction mixture was concentrated under reduced pressure, and water was added to the residue. The mixture was adjusted to pH 4.2 with a saturated aqueous sodium hydrogen carbonate solution and 1N hydrochloric acid, and extracted with ethyl acetate. The organic layer was washed with a saturated aqueous sodium chloride solution, dried over anhydrous sodium sulfate and concentrated under reduced pressure. The residue was

applied to reverse phase C 18 column chromatography (eluent; water: acetonitrile =3:2→1:1 (V/V)), and the fractions containing the objective material were concentrated under reduced pressure to obtain 272 mg the title compound as white foam. (Yield: 93%)
Mass Spectrum (FAB, m/z): 469 ($M^+$+1).
$^1$H-NMR Spectrum ($CDCl_3$, δ ppm): 8.72-8.69 (m, 1H), 7.95-7.91 (m, 1H), 7.85 (ddd, J=7.7, 7.7, 1.5 Hz, 1 H), 7.46 (ddd, J=7.7, 4.7, 1.0 Hz, 1H), 7.40 (dd, J=8.3, 7.4 Hz, 1H), 7.00-6.92 (m, 3H), 6.76 (d, J=7.4 Hz, 1H), 6.29 (d, J=8.3 Hz, 1H), 4.61 (s, 2H), 4.57 (s, 2H), 3.86 (s, 2H), 2.56-2.46 (m, 4H), 1.12 (t, J=7.5 Hz, 3H), 1.09 (t, J=7.5 Hz, 3H).
Rf value: 0.40(n-butanol:acetic acid:water=10:1:1).

[Example 45]

{6-[(4-Butoxybenzyl)(pyridin-2-ylsulfonyl)aminomethyl]pyridin-2-ylamino}acetic acid (Exemplary compound No. 899)

45-(a): Ethyl {6-[(4-Butoxybenzyl)(pyridin-2-ylsylfonyl)aminomethyl]pyridin-2-ylamino} acetate

To 2.5 ml of a tetrahydrofuran solution containing 158 mg (0.451 mmol) of ({6-[(pyridin-2-ylsulfonyl)aminomethyl]pyridin-2-yl} amino)ethyl acetate obtained in Reference example 34 were added 89.2 mg (0.495 mmol) of 4-butoxybenzyl alcohol, 169 μl (0.677 mmol) of tri-n-butylphosphine and 116 mg (0.674 mmol) of N,N,N',N'-tetramethylazodicarboxamide, and the mixture was stirred at room temperature for 3 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=9:1→1:4 (V/V)), and the fractions containing the objective material were concentrated under reduced pressure to obtain 212 mg of the title compound as colorless oily product. (Yield: 92%)
Mass Spectrum (FAB, m/z): 513 ($M^+$+1).
$^1$H-NMR Spectrum ($CDCl_3$, δ ppm): 8.60 (ddd, J=4.8, 1.7, 1.0 Hz, 1H), 7.81 (ddd, J=7.8, 1.3, 1.0 Hz, 1H), 7.74 (ddd, J=7.8, 7.6, 1.7 Hz, 1H), 7.37 (ddd, J=7.6, 4.8, 1.3 Hz, 1H), 7.23 (dd, J=8.3, 7.2 Hz, 1H), 7.20-7.16 (m, 2H), 6.79-6.73 (m, 2H), 6.49 (d, J=7.2 Hz, 1H), 6.22 (d, J=8.3 Hz, 1H), 4.71-4.65 (m, 1H), 4.68 (s, 2H), 4.36 (s, 2H), 4.23 (q, J=7.1 Hz, 2H), 3.96 (d, J=5.6 Hz, 2H), 3.92 (t, J=6.5 Hz, 2H), 1.80-1.69 (m, 2H), 1.54-1.42 (m, 2H), 1.29 (t, J=7.1 Hz, 3H), 0.97 (t, J=7.4 Hz, 3H).

45-(b): {6-[(4-Butoxybenzyl)(pyridin-2-ylsulfonyl)aminomethyl]pyridin-2-ylamino}-acetic acid

To 4 ml of an ethanol solution containing 207 mg (0.404 mmol) of ethyl {6-[(4-butoxybenzyl)(pyridin-2ylsulfonyl)aminomethyl]pyridin-2-ylamino}acetate obtained in Example 45-(a) was added 1.6 ml (1.6 mmol) of 1N aqueous sodium hydroxide solution, and the mixture was stirred at room temperature for 4 hours. After completion of the reaction, 10 ml was added to the reaction mixture, and the mixture was adjusted to pH 4.2 with 1N hydrochloric acid and extracted with ethyl acetate. The organic layer was washed with a saturated aqueous sodium chloride solution, dried over anhydrous magnesium sulfate and concentrated under reduced pressure to obtain 151 mg of the title compound as white solid. (Yield: 77%)
Mass Spectrum (FAB, m/z): 485 ($M^+$+1).
$^1$H-NMR Spectrum (DMSO-$d_6$, δ ppm): 8.62 (ddd, J=4.7, 1.7, 0.9 Hz, 1H), 7.95 (ddd, J=7.8, 7.7, 1.7 Hz, 1H), 7.78 (ddd, J=7.8, 1.0, 0.9 Hz, 1H), 7.57 (ddd, J=7.7, 4.7, 1.0 Hz, 1H), 7.20 (dd, J=8.2, 7.2 Hz, 1H), 7.15-7.09 (m, 2H), 6.84-6.77 (m, 2H), 6.71 (t, J=5.5 Hz, 0.8H), 6.34 (d, J=8.2 Hz, 1H), 6.27 (d, J=7.2 Hz, 1H), 4.59 (s, 2H), 4.18 (s, 2H), 3.92 (t, J=6.5 Hz, 2H), 3.82 (d, J=5.5 Hz, 2H), 1.73-1.61 (m, 2H), 1.49-1.35 (m, 2H), 0.93 (t, J=7.3 Hz, 3H).
Rf value: 0.41 (n-butanol:acetic acid:water=10:1:1).

[Example 46]

(6-{[4-(1,1-Dimethylpentyl)benzyl](pyridin-3-ylsulfonyl)aminomethyl}pyridin-2-ylamino)acetic acid (Exemplary compound No. 560)

46-(a): tert-Butyl [tert-butoxycarbonyl(6-{[4-(1,1-dimethylpentyl)benzyl](pyridin-3-yl-sulfonyl)aminomethyl}pyridin-2-yl)amino]acetate

To 4 ml of a tetrahydrofuran solution containing 422 mg (0.882 mmol) of tert-butyl (tert-butoxycarbonyl{6-[(pyridin-3-ylsulfonyl)aminomethyl]pyridin-2-yl}amino)-acetate obtained in the same manner as in Reference example 1-(f) were added 165 mg (0.800 mmol) of 4-(1,1-dimethylpentyl)benzyl alcohol obtained in the same manner as in Reference

example 29-(b), 395 μl (1.58 mmol) of tri-n-butylphosphine and 276 mg (1.60 mmol) of N,N,N',N'-tetramethylazodicarboxamide, and the mixture was stirred at room temperature for 16 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; toluene:ethyl acetate=7:1→ 6:1 (V/V)), and the fractions containing the objective material were concentrated under reduced pressure to obtain 488 mg of the title compound as colorless oily product.
(Yield: 91%)
Mass Spectrum (FAB, m/z): 667 ($M^+$+1).
$^1$H-NMR Spectrum ($CDCl_3$, δ ppm): 8.94 (dd, J=2.4, 0.9 Hz, 1H), 8.69 (dd, J=4.9, 1.6 Hz, 1H), 7.84 (ddd, J=8:1, 2.4, 1.6 Hz, 1H), 7.70 (d, J=8.4 Hz, 1H), 7.50 (dd, J=8.4, 7.6 Hz, 1H), 7.29 (ddd, J=8.1, 4.9, 0.9 Hz, 1H), 7.24-7.17 (m, 2H), 7.16-7.10 (m, 2H), 6.86 (d, J=7.6 Hz, 1H), 4.53 (s, 2H), 4.40 (s, 2H), 4.39 (s, 2H), 1.60-1.48 (m, 2H), 1.52 (s, 9H), 1.43 (s, 9H), 1.27-1.14 (m, 2H), 1.25 (s, 6H), 1.06-0.93 (m, 2H), 0.82 (t, J=7.3 Hz, 3H).

46-(b): <u>(6-{[4-(1,1-Dimethylpentyl)benzyl](pyridin-3-ylsulfonyl)aminomethyl}pyridin-2-ylamino)acetic acid</u>

To 480 mg (0.720 mmol) of tert-butyl [tert-butoxycarbonyl(6-{[4-(1,1-dimethylpentyl)benzyl](pyridin-3-ylsulfonyl)aminomethyl}pyridin-2-yl)amino]acetate obtained in Example 46-(a) were added 4.5 ml (18 mmol) of 4N hydrogen chloride/1,4-dioxane solution and 4.5 ml of water, and the mixture was stirred at 70°C for 6 hours. After completion of the reaction, the reaction mixture was concentrated under reduced pressure, and 20 ml of water was added to the residue. The mixture was adjusted to pH 4.5 with 1N aqueous sodium hydroxide solution and 1N hydrochloric acid, and extracted with tert-butyl methyl ether. The organic layer was washed with a saturated aqueous sodium chloride solution, dried over anhydrous magnesium sulfate and concentrated under reduced pressure. The residue was applied to silica gel column chromatography (eluent; methylene chloride :methanol=19:1→9:1 (V/V)), and the fractions containing the objective material were concentrated under reduced pressure to obtain 330 mg of the title compound as white foam.
(Yield: 90%)
Mass Spectrum (FAB, m/z): 511 ($M^+$+1).
$^1$H-NMR Spectrum (DMSO-$d_6$, δ ppm): 12.39 (brs, 0.8H), 8.80 (dd, J=2.3, 0.7 Hz, 1H), 8.70 (dd, J=4.9, 1.7 Hz, 1H), 7.98 (ddd, J=8.1, 2.3, 1.7 Hz, 1H), 7.45 (ddd, J=8.1, 4.9, 0.7 Hz, 1H), 7.28-7.25 (m, 2H), 7.23 (dd, J=8.4, 7.2 Hz, 1H), 7.21-7.17 (m, 2H), 6.74 (t, J=5.7 Hz, 1H), 6.36 (d, J=8.4 Hz, 1H), 6.30 (d, J=7.2 Hz, 1H), 4.62 (s, 2H), 4.17 (s, 2H), 3.71 (d, J=5.7 Hz, 2H), 1.59-1.52 (m, 2H), 1.23 (s, 6H), 1.23-1.13 (m, 2H), 0.99-0.91 (m, 2H), 0.79 (t, J=7.3 Hz, 3H).
Rf value: 0.49 (n-butanol:acetic acid:water=10:1:1).

[Example 47]

<u>(6-{(4-Fluorobenzenesulfonyl)[4-(1,1-dimethylpentyl)benzyl]aminomethyl}pyridin-2-ylamino)acetic acid</u> (Exemplary compound No. 512)

47-(a): <u>tert-Butyl [tert-butoxycarbonyl(6-{(4-fluorobenzenesulfonyl)[4-(1,1-dimethylpentyl)benzyl]aminomethyl}pyridin-2-yl)amino]acetate</u>

To 4.0 ml of a tetrahydrofuran solution containing tert-butyl (tert-butoxycarbonyl {6-[(4-fluorobenzenesulfonyl)aminomethyl]pyridin-2-yl}amino)acetate obtained in Reference example 3 436 mg (0.880 mmol) were added 165 mg (0.800 mmol) of 4-(1,1-dimethylpentyl)benzyl alcohol obtained in the same manner as in Reference example 29-(b), 395 μl (1.58 mmol) of tri-n-butylphosphine and 276 mg (1.60 mmol) of N,N,N',N'-tetramethylazodicarboxamide, and the mixture was stirred at room temperature for 16 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; toluene: ethyl acetate=15:1 (van)), and the fractions containing the objective material were concentrated under reduced pressure to obtain 489 mg of the title compound as colorless oily product.
(Yield: 89%)
Mass Spectrum (FAB, m/z): 684 ($M^+$+1).
$^1$H-NMR Spectrum ($CDCl_3$, δ ppm): 7.72-7.65 (m, 3H), 7.49 (dd, J=8.3, 7.4 Hz, 1H), 7.22-7.17 (m, 2H), 7.12-7.03 (m, 4H), 6.85 (d, J=7.4 Hz, 1H), 4.47 (s, 2H), 4.42 (s, 2H), 4.35 (s, 2H), 1.60-1.48 (m, 2H), 1.52 (s, 9H), 1.42 (s, 9H), 1.28-1.14 (m, 2H), 1.25 (s, 6H), 1.05-0.92 (m, 2H), 0.82 (t, J=7.3 Hz, 3H).

47-(b): (6-{(4-Fluorobenzenesulfonyl)[4-(1,1-dimethylpentyl)benzyl]aminomethyl}-pyridin-2-ylamino)acetic acid

To 479 mg (0.700 mmol) of tert-butyl [tert-butoxycarbonyl(6-{(4-fluorobenzenesulfonyl)[4-(1,1-dimethylpentyl)benzyl]aminomethyl}pyridin-2-yl)amino]-acetate obtained in Example 47-(a) was added 4.4 ml (18 mmol) of 4N hydrogen chloride/1,4-dioxane solution, and the mixture was stirred at room temperature for 16 hours. Then, 4.0 ml of water was added to the reaction mixture, and the mixture was stirred at 70°C for 3 hours. After completion of the reaction, the reaction mixture was concentrated under reduced pressure, 20 ml of water was added to the residue. The mixtue was adjusted to pH 4.4 with 1N aqueous sodium hydroxide solution and 1N hydrochloric acid, and extracted with tert-butyl methyl ether. The organic layer was washed with a saturated aqueous sodium chloride solution, dried over anhydrous magnesium sulfate and concentrated under reduced pressure. The residue was applied to silica gel column chromatography (eluent; methylene chloride:methanol=19:1→9:1 (V/V)), and the fractions containing the objective material were concentrated under reduced pressure. To the residue was added 5 ml of tert-butyl methyl ether, and the mixture was stirred at room temperature for 2 hours. Precipitated solid was collected by filtration, and dried at 50°C under reduced pressure to obtain 286 mg of the title compound as white solid. (Yield: 77%)
Mass Spectrum (FAB, m/z): 528 ($M^+$+1).
$^1$H-NMR Spectrum (DMSO-$d_6$, δ ppm): 12.39 (brs, 0.5H), 7.74-7.69 (m, 2H), 7.28-7.23 (m, 4H), 7.22 (dd, J=8.3, 7.2 Hz, 1H), 7.18-7.14 (m, 2H), 6.76 (t, J=5.7 Hz, 1H), 6.37 (d, J=8.3 Hz, 1H), 6.28 (d, J=7.2 Hz, 1H), 4.55 (s, 2H), 4.13 (s, 2H), 3.77 (d, J=5.7 Hz, 2H), 1.58-1.51 (m, 2H), 1.23 (s, 6H), 1.23-1.13 (m, 2H), 0.99-0.90 (m, 2H), 0.79 (t, J=7.3 Hz, 3H).
Rf value: 0.72 (n-butanol:acetic acid:water=10:1:1).

[Example 48]

(6-{[4-(1,1-Dimethylpentyl)benzyl](thiophen-2-ylsulfonyl)aminomethyl}pyridin-2-ylamino)acetic acid (Exemplary compound No. 541)

48-(a): tert-Butyl [tert-butoxycarbonyl(6-{[4-(1,1-dimethylpentyl)benzyl](thiophen-2-ylsulfonyl)aminomethyl}pyridin-2-yl)amino]acetate

To 5 ml of a tetrahydrofuran solution containing 0.20 g (0.41 mmol) of tert-butyl (tert-butoxycarbonyl{6-[(thiophen-2-ylsulfonyl)aminomethyl]pyridin-2-yl}-amino)acetate obtained in Reference example 35 were added 86 mg (0.42 mmol) of 4-(1,1-dimethylpentyl)benzyl alcohol obtained in the same manner as in Reference example 29-(b), 135 μl (0.540 mmol) of tri-n-butylphosphine and 86 mg (0.50 mmol) of N,N,N',N'-tetramethylazodicarboxamide, and the mixture was stirred at room temperature for 3 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=4:1 (V/V)), and the fractions containing the objective material were concentrated under reduced pressure to obtain 0.18 g of the title compound as colorless oily product. (Yield: 65%)
Mass Spectrum (FAB, m/z): 672 ($M^+$+1).
$^1$H-NMR Spectrum (DMSO-$d_6$, δ ppm): 7.96 (dd, J=5.0, 1.3 Hz, 1H), 7.64-7.54 (m, 3H), 7.29-7.15 (m, 3H), 7.10-7.04 (m, 2H), 6.90-6.85 (m, 1H), 4.41 (s, 2H), 4.33 (s, 2H), 4.29 (s, 2H), 1.60-1.47 (m, 2H), 1.45 (s, 9H), 1.37 (s, 9H), 1.22-1.11 (m, 2H), 1.19 (s, 6H), 1.00-0.83 (m, 2H), 0.78 (t, J=7.3 Hz, 3H).

48-(b): (6-{[4-(1,1-Dimethylpentyl)benzyl](thiophen-2-ylsulfonyl)aminomethyl}-pyridin-2-ylamino)acetic acid

To 4 ml of a methylene chloride solution containing 0.18 g (0.27 mmol) of tert-butyl [tert-butoxycarbonyl(6-{[4-(1,1-dimethylpentyl)benzyl](thiophen-2-ylsulfonyl)-aminomethyl}pyridin-2-yl)amino]acetate obtained in Example 48-(a) was added 4.0 ml (54 mmol) of trifluoroacetic acid, and the mixture was stirred at room temperature for 4 hours. After completion of the reaction, the reaction mixture was concentrated under reduced pressure. The residue was applied to silica gel column chromatography (eluent; ethyl acetate:methanol=19:1 (V/V)), and the fractions containing the objective material were concentrated under reduced pressure to obtain 93.3 mg of the title compound as white foam. (Yield: 67%)
Mass Spectrum (FAB, m/z): 516 ($M^+$+1).
$^1$H-NMR Spectrum (DMSO-$d_6$, δ ppm): 7.96 (d, J=4.4 Hz, 1H), 7.63-7.54 (m, 1H), 7.44-7.32 (m, 1H), 7.25-7.11 (m, 5H), 6.54 (d, J=8.8 Hz, 1H), 6.39 (d, J=7.1 Hz, 1H), 4.46 (s, 2H), 4.23 (s, 2H), 3.93 (s, 2H), 1.58-1.48 (m, 2H), 1.26-1.10 (m, 2H), 1.21 (s, 6H), 1.00-0.86 (m, 2H), 0.78 (t, J=7.2 Hz, 3H).
Rf value: 0.64(n-butanol:acetic acid:water=10:1:1).

[Example 49]

(6-{(4-Chlorobenzenesulfonyl)[4-(1,1-dimethylpentyl)benzyl]aminomethyl}pyridin-2-ylamino)acetic acid (Exemplary compound No. 520)

49-(a): tert-Butyl [tert-butoxycarbonyl(6-1{(4-chlorobenzenesulfonyl)[4-(1,1-dimethylpentyl)benzyl]aminomethyl}pyridin-2-yl)amino]acetate

To 3 ml of a methylene chloride solution containing 0.12 g (0.23 mmol) of tert-butyl [tert-butoxycarbonyl(6-{[4-(1,1-dimethylpentyl)benzyl]aminomethyl}pyridin-2-yl)amino]acetate obtained in Reference example 36 were added 58 mg (0.27 mmol) of 4-chlorobenzenesulfonyl chloride and 65 μl (0.47 mmol) of triethylamine, and the mixture was stirred at room temperature for 5 hours. After completion of the reaction, water was added to the reaction mixture, and the mixture was extracted with methylene chloride. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=4:1→1:1 (V/V)), and the fractions containing the objective material were concentrated under reduced pressure to obtain 0.10 g of the title compound as colorless oily product. (Yield: 62%)
Mass Spectrum (CI, m/z): 700 ($M^+$+1).
$^1$H-NMR Spectrum (DMSO-$d_6$, δ ppm): 7.76-7.70 (m, 2H), 7.64-7.53 (m, 4H), 7.21-7.14 (m, 2H), 7.08-7.03 (m, 2H), 6.91-6.85 (m, 1H), 4.42 (s, 2H), 4.32 (s, 2H), 4.31 (s, 2H), 1.56-1.47 (m, 2H), 1.45 (s, 9H), 1.37 (s, 9H), 1.23-1.10 (m, 2H), 1.19 (s, 6H), 0.98-0.84 (m, 2H), 0.78 (t, J=7.3 Hz, 3H).

49-(b): (6-{(4-Chlorobenzenesulfonyl)[4-(1,1-dimethylpentyl)benzyl]aminomethyl}-pyridin-2-ylamino)acetic acid

To 4 ml of a methylene chloride solution containing 0.10 g (0.14 mmol) of tert-butyl [tert-butoxycarbonyl(6-{(4-chlorobenzenesulfonyl)[4-(1,1-dimethylpentyl)-benzyl]aminomethyl}pyridin-2-yl)amino]acetate obtained in Example 49-(a) was added 4.0 ml (54 mmol) of trifluoroacetic acid, and the mixture was stirred at room temperature for 6 hours. After completion of the reaction, the reaction mixture was concentrated under reduced pressure, and water was added to the residue. The mixture was adjusted to pH 4.8 with 1N aqueous sodium hydroxide solution and 1N hydrochloric acid, and extracted with ethyl acetate. The organic layer was washed with a saturated aqueous sodium chloride solution, dried over anhydrous magnesium sulfate and concentrated under reduced pressure. The residue was applied to silica gel column chromatography (eluent; ethyl acetate:methanol=19:1 (V/V)), and the fractions containing the objective material were concentrated under reduced pressure to obtain 23.6 mg of the title compound as white solid. (Yield: 31 %)
Mass Spectrum (FAB, m/z): 544 ($M^+$+1).
$^1$H-NMR Spectrum (DMSO-$d_6$, δ ppm): 7.67-7.61 (m, 2H), 7.52-7.46 (m, 2H), 7.28-7.12 (m, 4H), 7.22 (dd, J=8.3, 7.0 Hz, 1H), 6.79 (t, J=5.6 Hz, 1H), 6.38 (d, J=8.3 Hz, 1H), 6.28 (d, J=7.0 Hz, 1H), 4.55 (s, 2H), 4.13 (s, 2H), 3.76 (d, J=5.6 Hz, 2H), 1.62-1.46 (m, 2H), 1.26-1.11 (m, 2H), 1.23 (s, 6H), 1.02-0.88 (m, 2H), 0.79 (t, J=7.3 Hz, 3H).
Rf value: 0.76 (n-butanol:acetic acid:water=10:1:1).

[Example 50]

6-{(4-Methoxybenzenesulfonyl)[4-(1,1-dimethylpentyl)benzyl]aminomethyl}pyridin-2-ylamino)acetic acid (Exemplary compound No. 530)

50-(a): tert-Butyl [tert-butoxycarbonyl(6-{(4-methoxybenzenesulfonyl)[4-(1,1-dimethylpentyl)benzyl]aminomethyl}pyridin-2-yl)amino]acetate

To 3 ml of a methylene chloride solution containing 0.16 g (0.30 mmol) of tert-butyl [tert-butoxycarbonyl(6-{[4-(1,1-dimethylpentyl)benzyl]aminomethyl}pyridin-2-yl)amino]acetate obtained in Reference example 36 were added 74 mg (0.36 mmol) of 4-methoxybenzenesulfonyl chloride and 87 μl (0.62 mmol) of triethylamine, and the mixture was stirred at room temperature for 5 hours. After completion of the reaction, water was added to the reaction mixture, and the mixture was extracted with methylene chloride. The organic layer was washed with a saturated aqueous sodium chloride solution, dried over anhydrous magnesium sulfate and concentrated under reduced pressure. The residue was applied to silica gel column chromatography (eluent; n-hexane: ethyl acetate=4:1→1:1 (V/V)), and the fractions containing the obj ective material were concentrated under reduced pressure to obtain 0.13 g of the title compound as colorless oily product. (Yield: 62%)
Mass Spectrum (CI, m/z): 696 ($M^+$+1).
$^1$H-NMR Spectrum (DMSO-$d_6$, δ ppm): 7.72-7.65 (m, 2H), 7.62-7.53 (m, 2H), 7.20-7.13 (m, 2H), 7.08-7.01 (m, 4H),

6.89-6.82 (m, 1H), 4.36 (s, 2H), 4.31 (s, 2H), 4.24 (s, 2H), 3.84 (s, 3H), 1.56-1.47 (m, 2H), 1.45 (s, 9H), 1.37 (s, 9H), 1.23-1.09 (m, 2H), 1.19 (s, 6H), 0.97-0.84 (m, 2H), 0.77 (t, J=7.3 Hz, 3H).

50-(b): (6-{(4-Methoxybenzenesulfonyl)[4-(1,1-dimethylpentyl)benzyl]aminomethyl}-pyridin-2-ylamino)acetic acid

To 4 ml of a methylene chloride solution containing 0.13 g (0.19 mmol) of tert-butyl [tert-butoxycarbonyl(6-{(4-methoxybenzenesulfonyl)[4-(1,1-dimethylpentyl)-benzyl]aminomethyl}pyridin-2-yl)amino]acetate obtained in Example 50-(a) was added 4.0 ml (54 mmol) of trifluoroacetic acid, and the mixture was stirred at room temperature for 6 hours. After completion of the reaction, the reaction mixture was concentrated under reduced pressure, and water was added to the residue. The mixture was adjusted to pH 5.3 with 1N aqueous sodium hydroxide solution and 1N hydrochloric acid, and extracted with ethyl acetate. The organic layer was washed with a saturated aqueous sodium chloride solution, dried over anhydrous magnesium sulfate and concentrated under reduced pressure. The residue was applied to silica gel column chromatography (eluent; ethyl acetate:methanol=19:1 (V/V)), and the fractions containing the objective material were concentrated under reduced pressure to obtain 93.6 mg of the title compound as white foam. (Yield: 91 %)
Mass Spectrum (FAB, m/z): 540 ($M^{+}$+1).
$^{1}$H-NMR Spectrum (DMSO-$d_6$, $\delta$ ppm): 7.68 (d, J=8.5 Hz, 2H), 7.45-7.29 (m, 1H), 7.26-6.97 (m, 6H), 6.51 (d, J=6.1 Hz, 1H), 6.35 (d, J=6.6 Hz, 1H), 4.41 (s, 2H), 4.18 (s, 2H), 3.88 (s, 2H), 3.84 (s, 3H), 1.59-1.46 (m, 2H), 1.28-1.09 (m, 2H), 1.21 (s, 6H), 1.02-0.85 (m, 2H), 0.78 (t, J=7.3 Hz, 3H).
Rf value: 0.62 (n-butanol: acetic acid:water=10:1:1).

[Example 51]

(6-{[4-(1,1-Dimethylbutyl)benzyl](pyridin-2-ylsulfonyl)aminomethyl}pyridin-2-ylamino)acetic acid (Exemplary compound No. 409)

51-(a): tert-Butyl [tert-butoxycarbonyl(6-{[4-1,1-dimethylbutyl)benzyl](pyridin-2-ylsulfonyl)aminomethyl}pyridin-2-yl)amino]acetate

To 6 ml of an acetonitrile solution containing 402 mg (0.840 mmol) of tert-butyl (tert-butoxycarbonyl {6-[(pyridin-2-ylsulfonyl)aminomethyl]pyridin-2-yl}amino)-acetate obtained in the same manner as in Reference example 2 were added 280 mg (1.10 mmol) of 4-(1,1-dimethylbutyl)benzyl bromide obtained in Reference example 37 and 151 mg (1.09 mmol) of potassium carbonate, and the mixture was stirred at 80°C for 22 hours. After completion of the reaction, a saturated aqueous sodium chloride solution was added to the reaction mixture, and the mixture was extracted with ethyl acetate. The organic layer was dried over anhydrous magnesium sulfate and concentrated under reduced pressure. The residue was applied to silica gel column chromatography (eluent; n-hexane:ethyl acetate=19:1→1: (V/V)), and the fractions containing the objective material were concentrated under reduced pressure to obtain 3 77 mg of the title compound as colorless oily product. (Yield: 69%)
Mass Spectrum (CI, m/z): 653 ($M^{+}$+1).
$^{1}$H-NMR Spectrum ($CDCl_3$, $\delta$ ppm): 8.58 (ddd, J=4.7, 1.6, 1.1 Hz, 1H), 7.82 (ddd, J=7.7, 1.4, 1.1 Hz, 1H), 7.76 (ddd, J=7.7, 7.4, 1.6 Hz, 1H), 7.64 (d, J=8.4 Hz, 1H), 7.44 (dd, J=8.4, 7.4 Hz, 1H), 7.37 (ddd, J=7.4, 4.7, 1.4 Hz, 1H), 7.18-7.09 (m, 4H), 6.91 (d, J=7.4 Hz, 1H), 4.64 (s, 2H), 4.50 (s, 2H), 4.47 (s, 2H), 1.55-1.46 (m, 2H), 1.52 (s, 9H), 1.43 (s, 9H), 1.23 (s, 6H), 1.08-0.93 (m, 2H), 0.81 (t, J=7.1 Hz, 3H).

51-(b): (6-{[4-(1,1-Dimethylbutyl)benzyl](pyridin-2-ylsulfonyl)aminomethyl}pyridin-2-ylamino)acetic acid

To 6 ml of a methylene chloride solution containing 369 mg (0.565 mmol) of tert-butyl [tert-butoxycarbonyl(6-{[4-(1,1-dimethylbutyl)benzyl](pyridin-2-ylsulfonyl)-aminomethyl}pyridin-2-yl)amino]acetate obtained in Example 51-(a) was added 3.0 ml (40 mmol) of trifluoroacetic acid, and the mixture was stirred at room temperature for 19 hours. After completion of the reaction, the reaction mixture was concentrated under reduced pressure, and water was added to the residue. The mixture was adjusted to pH 4.5 with 1N aqueous sodium hydroxide solution and 1N hydrochloric acid, and extracted with ethyl acetate. The organic layer was washed with water, dried over anhydrous sodium sulfate and concentrated under reduced pressure to obtain 266 mg of the title compound as pale brown foam. (Yield: 95%)
Mass Spectrum (FAB, m/z): 497 ($M^{+}$+1).
$^{1}$H-NMR Spectrum (DMSO-$d_6$, $\delta$ ppm): 12.40 (brs, 1H), 8.61 (ddd, J=4.6, 1.7, 0.9 Hz, 1H), 7.93 (ddd, J=7.7, 7.7, 1.7 Hz, 1H), 7.77 (ddd, J=7.7, 1.0, 0.9 Hz, 1H), 7.56 (ddd, J=7.7, 4.6, 1.0 Hz, 1H), 7.23-7.12 (m, 5H), 6.75 (brs, 1H), 6.34 (d, J=8.1 Hz, 1H), 6.27 (d, J=7.0 Hz, 1H), 4.63 (s, 2H), 4.22 (s, 2H), 3.83 (d, J=4.8 Hz, 2H), 1.54-1.48 (m, 2H), 1.21 (s, 6H), 1.00-0.91 (m, 2H), 0.78 (t, J=7.2 Hz, 3H).

Rf value: 0.46 (n-butanol:acetic acid:water=10:1:1).

[Example 52]

(6-{[4-(1,1-Dimethylpentyl)benzyl](thiazol-2-ylsulfonyl)aminomethyl}pyridin-2-ylamino)acetic acid (Exemplary compound No. 547)

52-(a): tert-Butyl [tert-butoxycarbonyl(6-{[4-(1,1-dimethylpentyl)benzyl](thiazol-2-ylsulfonyl)aminomethyl}pyridin-2-yl)amino]acetate

To 3 ml of a methylene chloride solution containing 0.13 g (0.25 mmol) of tert-butyl [tert-butoxycarbonyl(6-{[4-(1,-dimethylpentyl)benzyl]aminomethyl}pyridin-2-yl)amino]acetate obtained in Reference example 36 were added 91 mg (0.50 mmol) of thiazol-2-ylsulfonyl chloride (see Bioorganic and Medicinal Chemistry, 14, 6628 (2006)) and 96 μl (0.69 mmol) of triethylamine, and the mixture was stirred at room temperature for 5 hours. After completion of the reaction, water was added to the reaction mixture, and the mixture was extracted with methylene chloride. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate= 9:1→2:1 (V/V)), and the fractions containing the objective material were concentrated under reduced pressure to obtain 96.3 mg of the title compound as colorless oily product. (Yield: 57%)
Mass Spectrum (CI, m/z): 673 ($M^+$+1).
$^1$H-NMR Spectrum ($CDCl_3$, δ ppm): 7.86 (d, J=3.2 Hz, 1H), 7.69 (d, J=8.4 Hz, 1H), 7.49 (d, J=3.2 Hz, 1H), 7.48 (dd, J=8.4, 7.6 Hz, 1H), 7.21-7.11 (m, 4H), 6.89 (d, J=7.6 Hz, 1H), 4.66 (s, 2H), 4.50 (s, 2H), 4.49 (s, 2H), 1.59-1.49 (m, 2H), 1.52 (s, 9H), 1.43 (s, 9H), 1.29-1.14 (m, 2H), 1.24 (s, 6H), 1.05-0.92 (m, 2H), 0.83 (t, J=7.3 Hz, 3H).

52-(b):(6-{[4-(1,1-Dimethylpentyl)benzyl](thiazol-2-ylsulifonyl)aminomethyl}pyridin-2-ylamino)acetic acid

To 2 ml of a methylene chloride solution containing 90 mg (0.13 mmol) of tert-butyl [tert-butoxycarbonyl(6-{[4-(1,1-dimethylpentyl)benzyl](thiazol-2-ylsulfonyl)-aminomethyl}pyridin-2-yl)amino]acetate obtained in Example 52-(a) was added 2.0 ml (27 mmol) of trifluoroacetic acid, and the mixture was stirred at room temperature for 8 hours. After completion of the reaction, the reaction mixture was concentrated under reduced pressure, and water was added to the residue. The mixture was adjusted to pH 4.3 with 1N aqueous sodium hydroxide solution and 1N hydrochloric acid, and extracted with ethyl acetate. The organic layer was washed with a saturated aqueous sodium chloride solution, dried over anhydrous magnesium sulfate and concentrated under reduced pressure. The residue was applied to silica gel column chromatography (eluent; ethyl acetate:methanol=19:1 (V/V)), and the fractions containing the objective material were concentrated under reduced pressure to obtain 65.3 mg of the title compound as a pale yellow solid. (Yield: 97%)
Mass Spectrum (FAB, m/z): 517 ($M^+$+1).
$^1$H-NMR Spectrum ($CDCl_3$, δ ppm): 7.95 (s, 1H), 7.57 (s, 1H), 7.43-6.73 (m, 6H), 6.59 (d, J=7.1 Hz, 1H), 6.29 (d, J=8.5 Hz, 1H), 4.63 (s, 2H), 4.59 (s, 2H), 3.89 (s, 2H), 1.57-1.46 (m, 2H), 1.29-1.10 (m, 2H), 1.20 (s, 6H), 1.04-0.88 (m, 2H), 0.81 (t, J=6.8 Hz, 3H).
Rf value: 0.62 (n-butanol:acetic acid:water=10:1:1).

[Example 53]

(6-{[4-(5,5,5-Trifluoro-1,1-dimethylpenyl)benzyl](pyridin-2-ylsulfonyl)aminomethyl}-pyridin-2-ylamino)acetic acid (Exemplary compound No. 857)

53-(a): tert-Butyl [tert-butoxycarbonyl(6-{[4-(5,5,5-trifluoro-1,1-dimethylpentyl)-benzyl](pyridin-2-ylsulfonyl)aminomethyl}pyridin-2-yl)amino]acetate

To 3.5 ml of a tetrahydrofuran solution containing 363 mg (0.759 mmol) of tert-butyl (tert-butoxycarbonyl{6-[(pyridin-2-ylsulfonyl)aminomethyl]pyridin-2-yl}-amino)acetate obtained in the same manner as in Reference example 2 were added 195 mg (0.749 mmol) of 4-(5,5,5-trifluoro-1,1-dimethylpentyl)benzyl alcohol obtained in Reference example 38-(c), 278 μl (1.11 mmol) of tri-n-butylphosphine and 194 mg (1.13 mmol) of N,N,N',N'-tetramethylazodicarboxamide, and the mixture was stirred at room temperature for 16 hours. After completion of the reaction, water was added to the reaction mixture, and the mixture was extracted with ethyl acetate. The organic layer was washed with a saturated aqueous sodium chloride solution, dried over anhydrous magnesium sulfate and concentrated under reduced pressure. The

residue was applied to silica gel column chromatography (eluent; toluene:ethyl acetate=9:1 (V/V)), and the fractions containing the objective material were concentrated under reduced pressure to obtain 469 mg of the title compound as white foam. (Yield: 87%) Mass Spectrum (FAB, m/z): 721 ($M^+$+1).

$^1$H-NMR Spectrum ($CDCl_3$, δ ppm): 8.61-8.55 (m, 1H), 7.82 (ddd, J=7.7, 1.2, 1.1 Hz, 1H), 7.76 (ddd, J=7.7, 7.6, 1.8 Hz, 1H), 7.63 (d, J=7.7 Hz, 1H), 7.43 (dd, J=7.7, 7.3 Hz, 1H), 7.37 (ddd, J=7.6, 4.7, 1.2 Hz, 1H), 7.20-7.10 (m, 4H), 6.89 (d, J=7.3 Hz, 1H), 4.66 (s, 2H), 4.49 (s, 2H), 4.47 (s, 2H), 2.03-1.88 (m, 2H), 1.66-1.57 (m, 2H), 1.52 (s, 9H), 1.45 (s, 9H), 1.33-1.20 (m, 2H), 1.26 (s, 6H).

53-(b): <u>(6-{[4-(5,5,5-Trifluoro-1,1-dimethylpentyl)benzyl](pyridin-2-ylsulfonyl)aminomethyl}pyridin-2-ylamino)acetic acid</u>

To 1.0 ml of a tetrahydrofuran solution containing 462 mg (0.641 mmol) of tert-butyl [tert-butoxycarbonyl(6-{[4-(5,5,5-trifluoro-1,1-dimethylpentyl)benzyl]-(pyridin-2-ylsulfonyl)aminomethyl}pyridin-2-yl)amino]acetate obtained in Example 53-(a) were added 0.60 ml of water and 0.54 ml of concentrated hydrochloric acid, and the mixture was stirred at 65°C for 6 hours. After completion of the reaction, the reaction mixture was concentrated under reduced pressure, and 20 ml of water was added. The mixture was adjusted to pH 4.4 with 2N aqueous sodium hydroxide solution, and extracted with ethyl acetate. The organic layer was washed with a saturated aqueous sodium chloride solution, dried over anhydrous magnesium sulfate and concentrated under reduced pressure to obtain 361 mg of the title compound as white foam substantially quantitatively.
Mass Spectrum (FAB, m/z): 565 ($M^+$+1).
$^1$H-NMR Spectrum (DMSO-$d_6$, δ ppm): 12.40 (brs, 0.6H), 8.61 (ddd, J=4.7, 1.7, 0.9 Hz, 1H), 7.93 (ddd, J=7.8, 7.7, 1.7 Hz, 1H), 7.78 (ddd, J=7.8, 1.0, 0.9 Hz, 1H), 7.56 (ddd, J=7.7, 4.7, 1.0 Hz, 1H), 7.25-7.20 (m, 2H), 7.18 (dd, J=8.4, 7.0 Hz, 1H), 7.18-7.14 (m, 2H), 6.72 (t, J=5.5 Hz, 1H), 6.33 (d, J=8.4 Hz, 1H), 6.26 (d, J=7.0 Hz, 1H), 4.64 (s, 2H), 4.22 (s, 2H), 3.82 (d, J=5.5 Hz, 2H), 2.23-2.08 (m, 2H), 1.67-1.58 (m, 2H), 1.23 (s, 6H), 1.21-1.13 (m, 2H).
Rf value: 0.47 (n-butanol:acetic acid:water=10:1:1).

[Example 54]

<u>(6-{[3-Fluoro-4-(1,1-dimethylpentyl)benzyl](pyridin-2-ylsulfonyl)aminomethyl}-pyridin-2-ylamino)acetic acid</u> (Exemplary compound No. 609)

54-(a): <u>tert-Butyl [tert-butoxycarbonyl(6-{[3-fluoro-4-(1,1-dimethylpentyl)benzyl]-(pyridin-2-ylsulfonyl)aminomethyl}pyridin-2-yl)amino]acetate</u>

To 4.8 ml of a tetrahydrofuran solution containing 200 mg (0.418 mmol) of tert-butyl (tert-butoxycarbonyl {6-[(pyridin-2-ylsulfonyl)aminomethyl]pyridin-2-yl}-amino)acetate obtained in the same manner as in Reference example 2 were added 93.8 mg (0.418 mmol) of 3-fluoro-4-(1,1-dimethylpentyl)benzyl alcohol obtained in Reference example 39-(b), 198 μl (0.793 mmol) of tri-n-butylphosphine and 113 mg (0.656 mmol) of N,N,N',N'-tetramethylazodicarboxamide, and the mixture was stirred at room temperature for 16 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=7:3 (V/V)), and the fractions containing the objective material were concentrated under reduced pressure to obtain 245 mg of the title compound as colorless oily product.
(Yield: 86%)
Mass Spectrum (FAB, m/z): 685 ($M^+$+1).
$^1$H-NMR Spectrum ($CDCl_3$, δ ppm): 8.58 (ddd, J=4.7, 1.6, 1.1 Hz, 1H), 7.82 (ddd, J=7.7, 1.4, 1.1 Hz, 1H), 7.77 (ddd, J=7.7, 7.4, 1.6 Hz, 1H), 7.65 (d, J=8.4 Hz, 1H), 7.45 (dd, J=8.4, 7.3 Hz, 1H), 7.38 (ddd, J=7.4, 4.7, 1.4 Hz, 1H), 7.06 (dd, J=8.2, 8.2 Hz, 1H), 6.94-6.87 (m, 2H), 6.85 (dd, J=13.4, 1.7 Hz, 1H), 4.64 (s, 2H), 4.50 (s, 2H), 4.46 (s, 2H), 1.72-1.62 (m, 2H), 1.52 (s, 9H), 1.45 (s, 9H), 1.32-1.15 (m, 2H), 1.29 (s, 6H), 1.08-0.87 (m, 2H), 0.83 (t, J=7.3 Hz, 3H).

54-(b): <u>(6-{[3-Fluoro-4-(1,1-dimethylpentyl)benzyl](pyridin-2-ylsulfonyl)aminomethyl}pyridin-2-ylamino)acetic_acid</u>

To 2.0 ml of a methylene chloride solution containing 235 mg (0.343 mmol) of tert-butyl [tert-butoxycarbonyl(6-{[3-fluoro-4-(1,1-dimethylpentyl)benzyl](pyridin-2-ylsulfonyl)aminomethyl}pyridin-2-yl)amino]acetate obtained in Example 54-(a) was added 1.7 ml (6.8 mmol) of 4N hydrogen chloride/1,4-dioxane solution, and the mixture was stirred at room temperature for 13 hours. After completion of the reaction, the reaction mixture was concentrated under reduced pressure, and water was added to the residue. The mixture was adjusted to pH 4.5 with 1N aqueous sodium hydroxide solution and 1N hydrochloric acid. Precipitated solid was collected by filtration, and dried at 50°C under reduced pressure

to obtain 104 mg of the title compound as white solid. (Yield: 57%)
Mass Spectrum (FAB, m/z): 529 ($M^{+}$+1).
$^{1}$H-NMR Spectrum (DMSO-$d_6$, δ ppm): 8.62 (ddd, J=4.7, 1.8, 0.9 Hz, 1H), 7.95 (ddd, J=7.8, 7.7, 1.8 Hz, 1H), 7.79 (ddd, J=7.8, 1.1, 0.9 Hz, 1H), 7.57 (ddd, J=7.7, 4.7, 1.1 Hz, 1H), 7.18 (dd, J=8.3, 7.2 Hz, 1H), 7.15 (dd, J=8.3, 8.2 Hz, 1H), 7.00 (dd, J=8.2, 1.7 Hz, 1H), 6.89 (dd, J=14.0, 1.7 Hz, 1H), 6.74 (t, J=5.8 Hz, 1H), 6.33 (d, J=8.3 Hz, 1H), 6.26 (d, J=7.2 Hz, 1H), 4.65 (s, 2H), 4.25 (s, 2H), 3.81 (d, J=5.8 Hz, 2H), 1.67-1.61 (m, 2H), 1.23 (s, 6H), 1.25-1.15 (m, 2H), 0.98-0.88 (m, 2H), 0.80 (t, J=7.3 Hz, 3H).
Rf value: 0.50 (n-butanol:acetic acid:water=10:1:1).

[Example 55]

(6-{[2-Fluoro-4-(1,1-dimethylpentyl)benzyl](pyridin-2-ylsulfonyl)aminomethyl}-pyridin-2-ylamino)acetic acid (Exemplary compound No. 586)

55-(a): tert-Butyl [tert-butoxycarbonyl(6-{[2-fluoro-4-(1,1-dimethylpentyl)benzyl]-(pyridin-2-ylsulfonyl)aminomethyl}pyridin-2-yl)amino]acetate

To 5.3 ml of a tetrahydrofuran solution containing 222 mg (0.464 mmol) of tert-butyl (tert-butoxycarbonyl{6-[(pyridin-2-ylsulfonyl)aminomethyl]pyridin-2-yl}-amino)acetate obtained in the same manner as in Reference example 2 were added 104 mg (0.464 mmol) of 2-fluoro-4-(1,1-dimethylpentyl)benzyl alcohol obtained in Reference example 40-(d), 220 μl (0.881 mmol) of tri-n-butylphosphine and 125 mg (0.726 mmol) of N,N,N',N-tetramethylazodicarboxamide, and the mixture was stirred at room temperature for 16 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=3:2 (V/V)), and the fractions containing the objective material were concentrated under reduced pressure to obtain 75 mg of the title compound as colorless oily product.
(Yield: 24%)
Mass Spectrum (FAB, m/z): 685 ($M^{+}$+1).
$^{1}$H-NMR Spectrum ($CDCl_3$, δ ppm): 8.58 (ddd, J=4.8, 1.6, 1.1 Hz, 1H), 7.80 (ddd, J=7.7, 1.4, 1.1 Hz, 1H), 7.75 (ddd, J=7.7, 7.5, 1.6 Hz, 1H), 7.63 (d, J=8.3 Hz, 1H), 7.44 (dd, J=8.3, 7.3 Hz, 1H), 7.36 (ddd, J=7.5, 4.8, 1.4 Hz, 1H), 7.27 (dd, J=8.2, 8.2 Hz, 1H), 6.99-6.92 (m, 2H), 6.80 (dd, J=12.3, 1.8 Hz, 1H), 4.71 (s, 2H), 4.56 (s, 2H), 4.49 (s, 2H), 1.55-1.48 (m, 2H), 1.52 (s, 9H), 1.43 (s, 9H), 1.26-1.14 (m, 2H), 1.21 (s, 6H), 1.03-0.91 (m, 2H), 0.83 (t, J=7.3 Hz, 3H).

55-(b): (6-{[2-Fluoro-4-(1,1-dimethylpentyl)benzyl](pyridin-2-ylsulfonyl)aminomethyl}pyridin-2-ylamino)acetic acid

To 0.63 ml of a methylene chloride solution containing 74 mg (0.11 mmol) of tert-butyl [tert-butoxycarbonyl(6-{[2-fluoro-4-(1,1-dimethylpentyl)benzyl](pyridin-2-ylsulfonyl)aminomethyl}pyridin-2-yl)amino]acetate obtained in Example 55-(a) was added 0.53 ml (2.1 mmol) of 4N hydrogen chloride/1,4-dioxane solution, and the mixture was stirred at room temperature for 16 hours. After completion of the reaction, the reaction mixture was concentrated under reduced pressure, and water was added to the residue. The mixture was adjusted to pH 4.4 with 1N aqueous sodium hydroxide solution and 1N hydrochloric acid. Precipitated solid was collected by filtration, and dried at 50°C under reduced pressure to obtain 43.3 mg of the title compound as white solid. (Yield: 74%)
Mass Spectrum (FAB, m/z): 529 ($M^{+}$+1).
$^{1}$H-NMR Spectrum (DMSO-$d_6$, δ ppm): 8.60 (ddd, J=4.7, 1.7, 0.9 Hz, 1H), 7.93 (ddd, J=7.8, 7.7, 1.7 Hz, 1H), 7.76 (ddd, J=7.8, 1.0, 0.9 Hz, 1H), 7.56 (ddd, J=7.7, 4.7, 1.0 Hz, 1H), 7.24 (dd, J=8.3, 8.2 Hz, 1H), 7.21 (dd, J=8.3, 7.2 Hz, 1H), 7.02 (dd, J=8.2, 1.8 Hz, 1H), 6.94 (dd, J=12.4, 1.8 Hz, 1H), 6.71-6.63 (m, 1H), 6.33 (d, J=8.3 Hz, 1H), 6.33 (d, J=7.2 Hz, 1H), 4.67 (s, 2H), 4.32 (s, 2H), 3.82 (d, J=5.3 Hz, 2H), 1.55-1.48 (m, 2H), 1.25-1.12 (m, 2H), 1.19 (s, 6H), 0.96-0.88 (m, 2H), 0.80 (t, J=7.4 Hz, 3H).
Rf value: 0.50 (n-butanol:acetic acid:water=10:1:1).

[Example 56]

{6-[(3-Dimethylamino-4-pentylbenzyl)(pyridin-2-ylsulfonyl)aminomethyl]pyridin-2-ylamino}acetic acid (Exemplary compound No. 190)

56-(a): tert-Butyl (tert-butoxycarbonyl{6-[(3-dimethylamino-4-pentylbenzyl)(pyridin-2-ylsulfonyl)aminomethyl]pyridin-2-yl}amino)acetate

To 13 ml of a tetrahydrofuran solution containing 597 mg (1.25 mmol) of tert-butyl (tert-butoxycarbonyl{6-[(pyridin-2-ylsulfonyl)aminomethyl]pyridin-2-yl}amino)-acetate obtained in the same manner as in Reference example 2 were added 277 mg (1.25 mmol) of 3-dimethylamino-4-pentylbenzyl alcohol obtained in Reference example 41-(d), 720 μl (2.88 mmol) of tri-n-butylphosphine and 324 mg (1.88 mmol) of N,N,N',N'-tetramethylazodicarboxamide, and the mixture was stirred at room temperature for 26 hours. After completion of the reaction, a saturated aqueous sodium chloride solution was added to the reaction mixture, and the mixture was extracted with ethyl acetate. The organic layer was dried over anhydrous magnesium sulfate and concentrated under reduced pressure. The residue was applied to silica gel column chromatography (eluent; n-hexane: ethyl acetate=9:1→1:1 (V/V)), and the fractions containing the objective material were concentrated under reduced pressure to obtain 662 mg of the title compound as colorless oily product. (Yield: 78%)
Mass Spectrum (CI, m/z): 682 ($M^+$+1).
$^1$H-NMR Spectrum ($CDCl_3$, δ ppm): 8.61 (ddd, J=4.8, 1.7, 1.0 Hz, 1H), 7.82 (ddd, J=7.7, 1.3, 1.0 Hz, 1H), 7.77 (ddd, J=7.7, 7.4, 1.7 Hz, 1H), 7.64 (d, J=8.4 Hz, 1H), 7.46 (dd, J=8.4, 7.7 Hz, 1H), 7.38 (ddd, J=7.4, 4.8, 1.3 Hz, 1H), 7.02 (d, J=7.7 Hz, 1H), 6.96-6.89 (m, 2H), 6.81 (dd, J=7.8, 1.7 Hz, 1H), 4.62 (s, 2H), 4.49 (s, 2H), 4.45 (s, 2H), 2.63-2.56 (m, 2H), 2.57 (s, 6H), 1.64-1.49 (m, 2H), 1.52 (s, 9H), 1.42 (s, 9H), 1.39-1.30 (m, 4H), 0.90 (t, J=6.8 Hz, 3H).

56-(b): {6-[(3-Dimethylamino-4-pentylbenzyl)(pyridin-2-ylsulfonyl)aminomethyl]-pyridin-2-ylamino}acetic acid

To 10 ml of a methylene chloride solution containing 656 mg (0.962 mmol) of tert-butyl (tert-butoxycarbonyl{6-[(3-dimethylamino-4-pentylbenzyl)(pyridin-2-yl-sulfonyl)aminomethyl]pyridin-2-yl} amino)acetate obtained in Example 56-(a) was added 10 ml (0.13 mol) of trifluoroacetic acid, and the mixture was stirred at room temperature for 5 hours. After completion of the reaction, the reaction mixture was concentrated under reduced pressure, and water was added to the residue. The mixture was adjusted to pH 5.0 with 2N aqueous sodium hydroxide solution and 1N hydrochloric acid, and extracted with ethyl acetate. The organic layer was washed with water, dried over anhydrous magnesium sulfate and concentrated under reduced pressure to obtain 473 mg of the title compound as pale brown foam. (Yield: 93%) Mass Spectrum (FAB, m/z): 526 ($M^+$+1).
$^1$H-NMR Spectrum (DMSO-$d_6$, δ ppm): 12.42 (brs, 0.8H), 8.63 (ddd, J=4.6, 1.7, 0.9 Hz, 1H), 7.95 (ddd, J=7.9, 7.7, 1.7 Hz, 1H), 7.80 (ddd, J=7.9, 1.1, 0.9 Hz, 1H), 7.56 (ddd, J=7.7, 4.6, 1.1 Hz, 1H), 7. 19 (dd, J=8.4, 7.3 Hz, 1H), 7.02 (d, J=7.7 Hz, 1H), 6.84 (d, J=1.7 Hz, 1H), 6.80 (dd, J=7.7, 1.7 Hz, 1H), 6.70 (t, J=5.6 Hz, 1H), 6.33 (d, J=8.4 Hz, 1H), 6.30 (d, J=7.3 Hz, 1H), 4.57 (s, 2H), 4.21 (s, 2H), 3.82 (d, J=5.6 Hz, 2H), 2.56-2.51 (m, 2H), 2.48 (s, 6H), 1.55-1.47 (m, 2H), 1.33-1.20 (m, 4H), 0.85 (t, J=7.0 Hz, 3H).
Rf value: 0.18 (n-butanol:acetic acid:water=10:1:1).

[Example 57]

(6-{[4-(2,2-Dimethypentyl)benzyl](pyridin-2-ylsulfonyl)aminomethyl}pyridin-2-ylamino)acetic acid (Exemplary compound No. 714)

57-(a): tert-Butyl [tert-butoxycarbonyl(6-{[4-(2,2-dimethylpentyl)benzyl](pyridin-2-yl-sulfonyl)aminomethyl}pyridin-2-yl)amino]acetate

To 4.4 ml of a tetrahydrofuran solution containing 460 mg (0.961 mmol) of tert-butyl (tert-butoxycarbonyl{6-[(pyridin-2-ylsulfonyl)aminomethyl]pyridin-2-yl}-amino)acetate obtained in the same manner as in Reference example 2 were added 181 mg (0.877 mmol) of 4-(2,2-dimethylpentyl)benzyl alcohol obtained in Reference example 42-(d), 0.33 ml (1.3 mmol) of tri-n-butylphosphine and 226 mg (1.31 mmol) of N,N,N',N'-tetramethylazodicarboxamide, and the mixture was stirred at room temperature for 4 hours. After completion of the reaction, the reaction mixture was concentrated under reduced pressure, water was added to the residue, and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=4:1→7:3 (V/V)), and the fractions containing the objective material were concentrated under reduced pressure

to obtain 539 mg of the title compound as colorless oily product. (Yield: 92%)
Mass Spectrum (CI, m/z): 667 ($M^+$+1).
$^1$H-NMR Spectrum ($CDCl_3$, δ ppm): 8.59 (ddd, J=4.8, 1.7, 1.0 Hz, 1H), 7.82 (ddd, J=7.8, 1.3, 1.0 Hz, 1H), 7.76 (ddd, J=7.8, 7.5, 1.7 Hz, 1H), 7.64 (d, J=8.5 Hz, 1H), 7.44 (dd, J=8.5, 7.5 Hz, 1H), 7.37 (ddd, J=7.5, 4.8, 1.3 Hz, 1H), 7.13-7.06 (m, 2H), 6.97-6.92 (m, 2H), 6.91 (d, J=7.5 Hz, 1H), 4.65 (s, 2H), 4.49 (s, 2H), 4.46 (s, 2H), 2.42 (s, 2H), 1.52 (s, 9H), 1.43 (s, 9H), 1.39-1.25 (m, 2H), 1.17-1.08 (m, 2H), 0.90 (t, J=7.2 Hz, 3H), 0.79 (s, 6H).

57-(b): <u>(6-{[4-(2,2-Dimethylpentyl)benzyl](pyridin-2-ylsulfonyl)aminomethyl}pyridin-2-ylamino)acetic acid</u>

To 8 ml of a methylene chloride solution containing 535 mg (0.802 mmol) of tert-butyl [tert-butoxycarbonyl(6-{[4-(2,2-dimethylpentyl)benzyl](pyridin-2-ylsulfon-yl)aminomethyl}pyridin-2-yl)amino]acetate obtained in Example 57-(a) was added 8.0 ml (0. 11 mol) of trifluoroacetic acid, and the mixture was stirred at room temperature for 3 hours. After completion of the reaction, the reaction mixture was concentrated under reduced pressure, and 10 ml of water was added to the residue. The mixture was adjusted to pH 4.4 with a saturated aqueous sodium hydrogen carbonate solution and 1N hydrochloric acid, and extracted with ethyl acetate. The organic layer was washed with a saturated aqueous sodium chloride solution, dried over anhydrous magnesium sulfate and concentrated under reduced pressure to obtain 416 mg of the title compound as white foam substantially quantitatively.
Mass Spectrum (FAB, m/z): 511 ($M^+$+1).
$^1$H-NMR Spectrum (DMSO-$d_6$, δ ppm): 12.39 (brs, 0.6H), 8.62 (ddd, J=4.6, 1.8, 0.8 Hz, 1H), 7.95 (ddd, J=7.7, 7.7, 1.8 Hz, 1H), 7.80 (m, 1H), 7.57 (ddd, J=7.7, 4.6, 0.9 Hz, 1H), 7.18 (m, 1H), 7.14-7.10 (m, 2H), 7.01-6.97 (m, 2H), 6.74 (brs, 0.8H), 6.34 (d, J=8.4 Hz, 1H), 6.27 (d, J=7.0 Hz, 1H), 4.63 (s, 2H), 4.23 (s, 2H), 3.82 (d, J=2.6 Hz, 2H), 2.41 (s, 2H), 1.36-1.25 (m, 2H), 1.13-1.06 (m, 2H), 0.87 (t, J=7.3 Hz, 3H), 0.77 (s, 6H).
Rf value: 0.48 (n-butanol:acetic acid:water=10:1:1).

[Example 58]

<u>(6-{[4-(1,1-Dimethylhexyl)benzyl](pyridin-2-ylsulfonyl)aminomethyl}pyridin-2-ylamino)acetic acid</u> (Exemplary compound No. 786)

58-(a): <u>tert-Butyl [tert-butoxycarbonyl(6-{[4-(1,1-dimethylhexyl)benzyl(pyridin-2-ylsulfonyl)aminomethyl}pyridin-2-yl)amino]acetate</u>

To 5 ml of an acetonitrile solution containing 358 mg (0.748 mmol) of tert-butyl (tert-butoxycarbonyl{6-[(pyridin-2-ylsulfonyl)aminomethyl]pyridin-2-yl}amino)-acetate obtained in the same manner as in Reference example 2 were added 210 mg (0.741 mmol) of 4-(1,1-dimethylhexyl)benzyl bromide obtained in Reference example 43 and 142 mg (1.03 mmol) of potassium carbonate, and the mixture was stirred at 80°C for 20 hours. After completion of the reaction, a saturated aqueous sodium chloride solution was added to the reaction mixture, and the mixture was extracted with ethyl acetate. The organic layer was dried over anhydrous magnesium sulfate and concentrated under reduced pressure. The residue was applied to silica gel column chromatography (eluent; n-hexane:ethyl acetate=19:1→1: (V/V)), and the fractions containing the objective material were concentrated under reduced pressure to obtain 268 mg of the title compound as pale yellow oil. (Yield: 53%)
Mass Spectrum (FAB, m/z): 681 ($M^+$+1).
$^1$H-NMR Spectrum ($CDCl_3$, δ ppm): 8.58 (ddd, J=4.7, 1.6, 1.0 Hz, 1H), 7.81 (ddd, J=7.7, 1.3, 1.0 Hz, 1H), 7.75 (ddd, J=7.7, 7.4, 1.6 Hz, 1H), 7.64 (d, J=8.4 Hz, 1H), 7.44 (dd, J=8.4, 7.3 Hz, 1H), 7.36 (ddd, J=7.4, 4.7, 1.3 Hz, 1H), 7.18-7.10 (m, 4H), 6.90 (d, J=7.3 Hz, 1H), 4.64 (s, 2H), 4.50 (s, 2H), 4.47 (s, 2H), 1.57-1.50 (m, 2H), 1.52 (s, 9H), 1.43 (s, 9H), 1.28-1.11 (m, 4H), 1.23 (s, 6H), 1.07-0.94 (m, 2H), 0.83 (t, J=7.0 Hz, 3H).

58-(b): <u>(6-{[4-(1,1-Dimethylhexyl)benzyl](pyridin-2-ylsulfonyl)aminomethyl}pyridin-2-ylamino)acetic acid</u>

To 4 ml of a methylene chloride solution containing 263 mg (0.386 mmol) of tert-butyl [tert-butoxycarbonyl(6-{[4-(1,1-dimethylhexyl)benzyl](pyridin-2-ylsulfonyl)-aminomethyl}pyridin-2-yl)amino]acetate obtained in Example 58-(a) was added 2.0 ml (27 mmol) of trifluoroacetic acid, and the mixture was stirred at room temperature for 9 hours. After completion of the reaction, the reaction mixture was concentrated under reduced pressure, water was added to the residue. The mixture was adjusted to pH 4.5 5 with 1N aqueous sodium hydroxide solution and 1N hydrochloric acid, and extracted with ethyl acetate. The organic layer was dried over anhydrous sodium sulfate, and concentrated under reduced pressure to obtain 170 mg of the title compound as pale brown foam. (Yield: 84%)
Mass Spectrum (FAB, m/z): 525 ($M^+$+1).
$^1$H-NMR Spectrum (DMSO-$d_6$, δ ppm): 12.39 (brs, 0.3H), 8.61 (ddd, J=4.8, 1.7, 0.8 Hz, 1H), 7.93 (ddd, J=7.9, 7.7, 1.7

Hz, 1H), 7.77 (ddd, J=7.9, 1.0, 0.8 Hz, 1H), 7.55 (ddd, J=7.7, 4.8, 1.0 Hz, 1H), 7.22-7.19 (m, 2H), 7.18 (dd, J=8.2, 7.1 Hz, 1H), 7.17-7.13 (m, 2H), 6.73 (t, J=5.8 Hz, 1H), 6.33 (d, J=8.2 Hz, 1H), 6.26 (d, J=7.1 Hz, 1H), 4.64 (s, 2H), 4.21 (s, 2H), 3.82 (d, J=5.8 Hz, 2H), 1.55-1.50 (m, 2H), 1.25-1.10 (m, 4H), 1.21 (s, 6H), 1.01-0.91 (m, 2H), 0.79 (t, J=7.2 Hz, 3H).
Rf value: 0.51 (n-butanol:acetic acid:water=10:1:1).

[Example 59]

(6-{[4-(1,1,4-Trimethylpentyl)benzyl](pyridin-2-ylsulfonyl)aminomethyl}pyridin-2-ylamino)acetic acid (Exemplary compound No. 763)

59-(a): tert-Butyl [tert-butoxycarbonyl(6{[4-(1,1,4-trimethylpentyl)benzyl](pyridin-2-ylsulfonyl)aminomethyl}pyridin-2-yl)amino]acetate

To 12 ml of a tetrahydrofuran solution containing 555 mg (1.16 mmol) of tert-butyl (tert-butoxycarbonyl {6-[(pyridin-2-ylsulfonyl)aminomethyl]pyridin-2-yl} amino)-acetate obtained in the same manner as in Reference example 2 were added 255 mg (1.16 mmol) of 4-(1,1,4-trimethylpentyl)benzyl alcohol obtained in Reference example 44-(b), 724 μl (2.90 mmol) of tri-n-butylphosphine and 300 mg (1.74 mmol) of N,N,N',N'-tetramethylazodicarboxamide, and the mixture was stirred at room temperature for 4 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=9:1→4: (V/V)), and the fractions containing the objective material were concentrated under reduced pressure to obtain 764 mg of the title compound as colorless oily product. (Yield: 97%) Mass Spectrum (CI, m/z): 681 ($M^+$+1).
$^1$H-NMR Spectrum ($CDCl_3$, δ ppm): 8.58 (ddd, J=4.8, 1.6, 1.1 Hz, 1H), 7.81 (ddd, J=7.7, 1.3, 1.1 Hz, 1H), 7.75 (ddd, J=7.7, 7.4, 1.6 Hz, 1H), 7.64 (d, J=8.3 Hz, 1H), 7.44 (dd, J=8.3, 7.5 Hz, 1H), 7.37 (ddd, J=7.4, 4.8, 1.3 Hz, 1H), 7.24-7.10 (m, 4H), 6.90 (d, J=7.5 Hz, 1H), 4.65 (s, 2H), 4.50 (s, 2H), 4.47 (s, 2H), 1.59-1.49 (m, 2H), 1.52 (s, 9H), 1.43 (s, 9H), 1.46-1.30 (m, 1H), 1.23 (s, 6H), 0.94-0.84 (m, 2H), 0.81 (d, J=6.6 Hz, 6H).

59-(b): (6-{[4-(1,1,4-Trimethylpentyl)benzyl](pyridin-2-ylsulfonyl)aminomethyl}-pyridin-2-ylamino)acetic acid

To 6 ml of a methylene chloride solution containing 407 mg (0.598 mmol) of tert-butyl [tert-butoxycarbonyl(6-{[4-(1,1,4-trimethylpentyl)benzyl](pyridin-2-ylsulfon-yl)aminomethyl}pyridin-2-yl)amino]acetate obtained in Example 59-(a) was added 6.0 ml (81 mmol) of trifluoroacetic acid, and the mixture was stirred at room temperature for 15 hours. After completion of the reaction, the reaction mixture was concentrated under reduced pressure, and 6 ml of water was added to the residue. The mixture was adjusted to pH 4.5 with 2N aqueous sodium hydroxide solution and 2N hydrochloric acid, and extracted with diethyl ether. The organic layer was washed with a saturated aqueous sodium chloride solution, dried over anhydrous magnesium sulfate and concentrated under reduced pressure to obtain 264 mg of the title compound as white foam. (Yield: 84%)
Mass Spectrum (FAB, m/z): 525 ($M^+$+1).
$^1$H-NMR Spectrum (DMSO-$d_6$, δ ppm): 12.39 (brs, 0.7H), 8.61 (ddd, J=4.7, 1.7, 0.9 Hz, 1H), 7.93 (ddd, J=7.8, 7.7, 1.7 Hz, 1H), 7.77 (ddd, J=7.8, 1.1, 0.9 Hz, 1H), 7.56 (ddd, J=7.7, 4.7, 1.1 Hz, 1H), 7.26-7.09 (m, 5H), 6.74 (t, J=5.7 Hz, 1H), 6.33 (d, J=8.3 Hz, 1H), 6.26 (d, J=7.1 Hz, 1H), 4.64 (s, 2H), 4.22 (s, 2H), 3.83 (d, J=5.7 Hz, 2H), 1.62-1.48 (m, 2H), 1.42-1.31 (m, 1H), 1.21 (s, 6H), 0.92-0.80 (m, 2H), 0.78 (d, J=6.6 Hz, 6H).
Rf value: 0.50 (n-butanol:acetic acid:water=10:1:1).

[Example 60]

(6-{(3-Fluorobenzenesulfonyl)[4-(1,1-dimethylpentyl)benzyl]aminomethyl}pyridin-2-ylamino)acetic acid (Exemplary compound No. 508)

60-(a): tert-Butyl [tert-butoxycarbonyl(6-{(3-fluorobenzenesulfonyl)[4-(1,1-dimethylpentyl)benzyl]aminomethyl}pyridin-2-yl)amino]acetate

To 3 ml of a methylene chloride solution containing 0.13 g (0.25 mmol) of tert-butyl [tert-butoxycarbonyl(6-{[4-(1,1-dimethylpentyl)benzyl]aminomethyl}pyridin-2-yl)amino]acetate obtained in the same manner as in Reference example 36 were added 96 mg (0.49 mmol) of 3-fluorobenzenesulfonyl chloride and 96 μl (0.69 mmol) of triethylamine, and the mixture was stirred at room temperature for 6 hours. After completion of the reaction, water was added to the reaction mixture, and the mixture was extracted with methylene chloride. The organic layer was washed with a

saturated aqueous sodium chloride solution, dried over anhydrous magnesium sulfate and concentrated under reduced pressure. The residue was applied to silica gel column chromatography (eluent; n-hexane:ethyl acetate=9:1→2:1 (V/V)), and the fractions containing the objective material were concentrated under reduced pressure to obtain 0.11 g of the title compound as colorless oily product. (Yield: 64%)
Mass Spectrum (CI, m/z): 684 ($M^+$+1).
$^1$H-NMR Spectrum ($CDCl_3$, $\delta$ ppm): 7.69 (d, J=8.5 Hz, 1H), 7.54-7.44 (m, 2H), 7.43-7.33 (m, 2H), 7.24-7.14 (m, 3H), 7.14-7.05 (m, 2H), 6.86 (d, J=7.3 Hz, 1H), 4.49 (s, 2H), 4.40 (s, 2H), 4.37 (s, 2H), 1.59-1.51 (m, 2H), 1.52 (s, 9H), 1.42 (s, 9H), 1.32-1.11 (m, 2H), 1.25 (s, 6H), 1.06-0.92 (m, 2H), 0.82 (t, J=7.3 Hz, 3H).

60-(b): <u>(6-{(3-Fluorobenzenesulfonyl)[4-(1,1-dimethylpentyl)benzyl]aminomethyl}-pyridin-2-ylamino)acetic acid</u>

To 2 ml of a methylene chloride solution containing 0.11 g (0.16 mmol) of tert-butyl [tert-butoxycarbonyl(6-{(3-fluorobenzenesulfonyl)[4-(1,1-dimethylpentyl)-benzyl]aminomethyl}pyridin-2-yl)amino]acetate obtained in Example 60-(a) was added 2.0 ml (27 mmol) of trifluoroacetic acid, and the mixture was stirred at room temperature for 8 hours. After completion of the reaction, the reaction mixture was concentrated under reduced pressure, and water was added to the residue. The mixture was adjusted to pH 4.8 with 1N aqueous sodium hydroxide solution and 1N hydrochloric acid, and extracted with ethyl acetate. The organic layer was washed with a saturated aqueous sodium chloride solution, dried over anhydrous magnesium sulfate and concentrated under reduced pressure. The residue was applied to silica gel column chromatography (eluent; ethyl acetate:methanol=19:1 (V/V)), and the fractions containing the objective material were concentrated under reduced pressure to obtain 61.3 mg of the title compound as white solid. (Yield: 72%)
Mass Spectrum (FAB, m/z): 528 ($M^+$+1).
$^1$H-NMR Spectrum (DMSO-$d_6$, $\delta$ ppm): 7.57-7.34 (m, 4H), 7.28-7.22 (m, 2H), 7.23 (dd, J=8.3, 7.3 Hz, 1H), 7.20-7.13 (m, 2H), 6.76 (t, J=5.7 Hz, 0.9H), 6.37 (d, J=8.3 Hz, 1H), 6.30 (d, J=7.3 Hz, 1H), 4.58 (s, 2H), 4.14 (s, 2H), 3.74 (d, J=5.7 Hz, 2H), 1.60-1.49 (m, 2H), 1.29-1.08 (m, 2H), 1.23 (s, 6H), 1.02-0.88 (m, 2H), 0.79 (t, J=7.3 Hz, 3H).
Rf value: 0.72 (n-butanol:acetic acid:water=10:1:1).

[Example 61]

<u>(6-{(2-Fluorobenzenesulfonyl)[4-(1,1-dimethylpentyl)benzyl]aminomethyl}pyridin-2-ylamino)acetic acid</u> (Exemplary compound No. 504)

61-(a): <u>tert-Butyl [tert-butoxycarbonyl(6-{(2-fluorobenzenesulfonyl)[4-(1,1-dimethylpentyl)benzyl]aminomethyl}pyridin-2-yl)amino]acetate</u>

To 3 ml of a methylene chloride solution containing 0.13 g (0.25 mmol) of tert-butyl [tert-butoxycarbonyl(6-{[4-(1,1-dimethylpentyl)benzyl]aminomethyl}pyridin-2-yl)amino]acetate obtained in the same manner as in Reference example 36 were added 96 mg (0.49 mmol) of 2-fluorobenzenesulfonyl chloride and 96 $\mu$l (0.69 mmol) of triethylamine, and the mixture was stirred at room temperature for 6 hours. After completion of the reaction, water was added to the reaction mixture, and the mixture was extracted with methylene chloride. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=9:1→2:1 (V/V)), and the fractions containing the objective material were concentrated under reduced pressure to obtain 0.12 g of the title compound as colorless oily product. (Yield: 70%)
Mass Spectrum (CI, m/z): 684 ($M^+$+1).
$^1$H-NMR Spectrum ($CDCl_3$, $\delta$ ppm): 7.85-7.79 (m, 1H), 7.67 (d, J=8.8 Hz, 1H), 7.53-7.30 (m, 3H), 7.21-7.00 (m, 5H), 6.82 (d, J=7.6 Hz, 1H), 4.57 (s, 2H), 4.49 (s, 2H), 4.45 (s, 2H), 1.57-1.50 (m, 2H), 1.52 (s, 9H), 1.44 (s, 9H), 1.30-1.13 (m, 2H), 1.23 (s, 6H), 1.05-0.94 (m, 2H), 0.82 (t, J=7.3 Hz, 3H).

61-(b): <u>(6-{(2-Fluorobenzenesulfonyl)[4-(1-1-dimethylpentyl)benzyl]aminomethyl}-pyridin-2-ylamino)acetic acid</u>

To 2 ml of a methylene chloride solution containing 0.12 g (0.18 mmol) of tert-butyl [tert-butoxycarbonyl(6-{(2-fluorobenzenesulfonyl)[4-(1,1-dimethylpentyl)-benzyl]aminomethyl}pyridin-2-yl)amino]acetate obtained in Example 61-(a) was added 2.0 ml (27 mmol) of trifluoroacetic acid, and the mixture was stirred at room temperature for 8 hours. After completion of the reaction, the reaction mixture was concentrated under reduced pressure, and water was added to the residue. The mixture was adjusted to pH 4.8 with 1N aqueous sodium hydroxide solution and 1N hydrochloric acid, and extracted with ethyl acetate. The organic layer was washed with a saturated aqueous sodium chloride solution, dried over anhydrous magnesium sulfate and concentrated under reduced pressure. The residue was applied to silica gel column chromatography (eluent; ethyl acetate:methanol=19:1 (V/V)), and the fractions containing the objective

material were concentrated under reduced pressure to obtain 69.3 mg of the title compound as white solid. (Yield: 73%)
Mass Spectrum (FAB, m/z): 528 ($M^+$+1).
$^1$H-NMR Spectrum (DMSO-$d_6$, δ ppm): 12.41 (brs, 0.4H), 7.71 (ddd, J=7.6, 7.5, 1.6 Hz, 1H), 7.65-7.60 (m, 1H), 7.32 (ddd, J=10.7, 8.3, 1.0 Hz, 1H), 7.28-7.21 (m, 3H), 7.18 (dd, J=8.3, 7.2 Hz, 1H), 7.15-7.10 (m, 2H), 6.74 (t, J=6.0 Hz, 1H), 6.35 (d, J=8.3 Hz, 1H), 6.20 (d, J=7.2 Hz, 1H), 4.61 (s, 2H), 4.19 (s, 2H), 3.82 (d, J=6.0 Hz, 2H), 1.57-1.51 (m, 2H), 1.22 (s, 6H), 1.22-1.14 (m, 2H), 0.99-0.90 (m, 2H), 0.79 (t, J=7.3 Hz, 3H).
Rf value: 0.70 (n-butanol:acetic acid:water=10:1:1).

[Example 62]

6-{(Benzenesulfonyl)[4-1,1-dimethylpentyl)benzyl]aminomethyl}pyridin-2-ylamino)-acetic acid (Exemplary compound No. 500)

62-(a): tert-Butyl [(6-{(benzenesulfonyl)[4-(1,1-dimethylpentyl)benzyl]aminomethyl}-pyridin-2-yl)tert-butoxycarbonylamino]acetate

To 3 ml of a methylene chloride solution containing 0.15 g (0.29 mmol) of tert-butyl [tert-butoxycarbonyl(6-{[4-(1,1-dimethylpentyl)benzyl]aminomethyl}pyridin-2-yl)amino]acetate obtained in the same manner as in Reference example 36 were added 100 mg (0.566 mmol) of benzenesulfonyl chloride and 111 μl (0.796 mmol) of triethylamine, and the mixture was stirred at room temperature for 12 hours. After completion of the reaction, water was added to the reaction mixture, and the mixture was extracted with methylene chloride. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=9:1→2:1 (V/V)), and the fractions containing the objective material were concentrated under reduced pressure to obtain 0.14 g of the title compound as yellow oil. (Yield: 72%)
Mass Spectrum (CI, m/z): 666 ($M^+$+1).
$^1$H-NMR Spectrum ($CDCl_3$, δ ppm): 7.75-7.69 (m, 2H), 7.66 (d, J=8.3 Hz, 1H), 7.57-7.38 (m, 4H), 7.20-7.14 (m, 2H), 7.10-7.04 (m, 2H), 6.85 (d, J=7.6 Hz, 1H), 4.47 (s, 2H), 4.37 (s, 2H), 4.36 (s, 2H), 1.60-1.47 (m, 2H), 1.52 (s, 9H), 1.42 (s, 9H), 1.29-1.14 (m, 2H), 1.24 (s, 6H), 1.04-0.92 (m, 2H), 0.82 (t, J=7.3 Hz, 3H).

62-(b): (6-{(Benzenesulfonyl)[4-(1,1-dimethylpentyl)benzyl]aminomethyl}pyridin-2-ylamino)acetic acid

To 4 ml of a methylene chloride solution containing 0.13 g (0.20 mmol) of tert-butyl [(6-{(benzenesulfonyl)[4-(1,1-dimethylpentyl)benzyl]aminomethyl}pyridin-2-yl)tert-butoxycarbonylamino]acetate obtained in Example 62-(a) was added 4.0 ml (54 mmol) of trifluoroacetic acid, and the mixture was stirred at room temperature for 36 hours. After completion of the reaction, the reaction mixture was concentrated under reduced pressure, and water was added to the residue. The mixture was adjusted to pH 4.2 with 1N aqueous sodium hydroxide solution and 1N hydrochloric acid, and extracted with ethyl acetate. The organic layer was washed with a saturated aqueous sodium chloride solution, dried over anhydrous magnesium sulfate and concentrated under reduced pressure. The residue was applied to silica gel column chromatography (eluent; ethyl acetate:methanol=19:1 (V/V)), and the fractions containing the objective material were concentrated under reduced pressure to obtain 74.8 mg of the title compound as white solid. (Yield: 73%)
Mass Spectrum (FAB, m/z): 510 ($M^+$+1).
$^1$H-NMR Spectrum (DMSO-$d_6$, δ ppm): 12.40 (brs, 0.4H), 7.70 (d, J=7.5 Hz, 2H), 7.62-7.56 (m, 1H), 7.49 (t, J=7.5 Hz, 2H), 7.26-7.19 (m, 3H), 7.15-7.11 (m, 2H), 6.77 (brs, 0.7H), 6.38 (d, J=7.0 Hz, 1H), 6.27 (d, J=7.3 Hz, 1H), 4.50 (s, 2H), 4.13 (s, 2H), 3.79 (s, 2H), 1.57-1.51 (m, 2H), 1.24-1.13 (m, 2H), 1.22 (s, 6H), 0.99-0.89 (m, 2H), 0.79 (t, J=7.3 Hz, 3H).
Rf value: 0.67 (n-butanol:acetic acid:water=10:1:1).

[Example 63]

(6-{(Furan-2-ylsulfonyl)[4-(1,1-dimethylpentyl)benzyl]aminomethyl}pyridin-2-ylamino)acetic acid (Exemplary compound No. 537)

63-(a): tert-Butyl [tert-butoxycarbonyl(6-{(furan-2-ylsulfonyl)[4-(1,1-dimethylpentyl)-benzyl]aminomethyl}pyridin-2-yl)amino]acetate

To 0.5 ml of a methylene chloride solution containing 208 mg (0.396 mmol) of tert-butyl [tert-butoxycarbonyl(6-{[4-(1,1-dimethylpentyl)benzyl]aminomethyl}pyridin-2-yl)amino]acetate obtained in the same manner as in Reference example 36 were added 132 mg (0.792 mmol) of furan-2-ylsulfonyl chloride and 166 μl (1.19 mmol) of triethylamine,

and the mixture was stirred at room temperature for 4 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=19:1→17:3 (V/V)), and the fractions containing the objective material were concentrated under reduced pressure to obtain 232 mg of the title compound as colorless oily product. (Yield: 89%)
Mass Spectrum (CI, m/z): 656 ($M^+$+1).
$^1$H-NMR Spectrum ($CDCl_3$, δ ppm): 7.71 (d, J=8.4 Hz, 1H), 7.51 (dd, J=8.4, 7.4 Hz, 1H), 7.44 (dd, J=1.8, 0.8 Hz, 1H), 7.24-7.17 (m, 2H), 7.14-7.08 (m, 2H), 6.89 (d, J=7.4 Hz, 2H), 6.86 (dd, J=3.4, 0.8 Hz, 1H), 6.39 (dd, J=3.4, 1.8 Hz, 1H), 4.54 (s, 4H), 4.53 (s, 4H), 4.39 (s, 2H), 1.60-1.50 (m, 2H), 1.52 (s, 9H), 1.43 (s, 9H), 1.30-1.12 (m, 2H), 1.25 (s, 6H), 1.06-0.93 (m, 2H), 0.82 (t, J=7.2 Hz, 3H).

63-(b): <u>(6-{(Furan-2-ylsulfonyl)[4-(1,1-dimethylpentyl)benzyl]aminomethyl}pyridin-2-ylamino)acetic acid</u>

To 0.90 ml of a methylene chloride solution containing 57.3 mg (0.0873 mmol) of tert-butyl [tert-butoxycarbonyl(6-{(furan-2-ylsulfonyl)[4-(1,1-dimethylpentyl)-benzyl]aminomethyl}pyridin-2-yl)amino]acetate obtained in Example 63-(a) was added 1.74 ml (23 mmol) of trifluoroacetic acid, and the mixture was stirred at room temperature for 18.5 hours. After completion of the reaction, the reaction mixture was concentrated under reduced pressure, and water was added to the residue. The mixture was adjusted to pH 4.5 with 2N aqueous sodium hydroxide solution and 2N hydrochloric acid, and extracted with ethyl acetate. The organic layer was washed with a saturated aqueous sodium chloride solution, dried over anhydrous magnesium sulfate and concentrated under reduced pressure to obtain 26.7 mg of the title compound as white solid. (Yield: 61%)
Mass Spectrum (FAB, m/z): 500 ($M^+$+1).
$^1$H-NMR Spectrum (DMSO-$d_6$, δ ppm): 7.87 (dd, J=1.8, 0.7 Hz, 1H), 7.29-7.20 (m, 3H), 7.18-7.12 (m, 2H), 7.01 (dd, J=3.5, 0.7 Hz, 1H), 6.59 (dd, J=3.5, 1.8 Hz, 1H), 6.46 (brs, 0.5H), 6.37 (d, J=8.3 Hz, 1H), 6.26 (d, J=7.3 Hz, 1H), 4.54 (s, 2H), 4.14 (s, 2H), 3.76-3.68 (m, 2H), 1.60-1.50 (m, 2H), 1.26-1.10 (m, 2H), 1.22 (s, 6H), 1.01-0.87 (m, 2H), 0.78 (t, J=7.3 Hz, 3H).
Rf value: 0.67 (n-butanol:acetic acid:water=10:1:1).

[Example 64]

<u>(6- {[4-(3-Difluoromethoxy-1,1-dimethylpropyl)benzyl](pyridin-2-ylsulfonyl)aminomethyl}pyridin-2-ylamino)acetic acid</u> (Exemplary compound No. 954)

64-(a): <u>Ethyl (6-{[4-(3-difluoromethoxy-1,1-dimethylpropyl)benzyl](pyridin-2-yl-sulfonyl)aminomethyl}pyridin-2-ylamino)acetate</u> (Exemplary compound No. 956)

To 2 ml of an acetonitrile solution containing 100 mg (0.285 mmol) of ethyl {6-[(pyridin-2-ylsulfonyl)aminomethyl]pyridin-2-ylamino}acetate obtained in Reference example 34 were added 140 mg (0.456 mmol) of 4-(3-difluoromethoxy-1,1-dimethylpropyl)benzyl bromide obtained in Reference example 45-(b) and 47.3 mg (0.342 mmol) of potassium carbonate, and the mixture was stirred at 80°C for 7 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane: ethyl acetate=3:2→1:1 (V/V)), and the fractions containing the objective material were concentrated under reduced pressure to obtain 78 mg of the title compound as pale yellow oil. (Yield: 47%)
Mass Spectrum (FAB, m/z): 577 ($M^+$+1).
$^1$H-NMR Spectrum ($CDCl_3$, δ ppm): 8.60 (ddd, J=4.7, 1.7, 1.0 Hz, 1H), 7.82 (ddd, J=7.8, 1.2, 1.0 Hz, 1H), 7.75 (ddd, J=7.8, 7.6, 1.7 Hz, 1H), 7.37 (ddd, J=7.6, 4.7, 1.2 Hz, 1H), 7.25-7.16 (m, 5H), 6.50 (d, J=7.3 Hz, 1H), 6.22 (d, J=8.3 Hz, 1H), 6.10 (t, J=75.2 Hz, 1H), 4.75-4.66 (m, 1H), 4.71 (s, 2H), 4.40 (s, 2H), 4.23 (q, J=7.1 Hz, 2H), 3.97 (d, J=5.4 Hz, 2H), 3.60 (t, J=7.6 Hz, 2H), 1.98 (t, J=7.6 Hz, 2H), 1.31 (s, 6H), 1.26 (t, J=7.1 Hz, 3H).
Rf value: 0.28 (n-hexane:ethyl acetate=1:1).

64-(b): <u>(6-{[4-(3-Difluoromethoxy-1,1-dimethylpropyl)benzyl](pyridin-2-ylsulfonyl)-aminomethyl}pyridin-2-ylamino)acetic acid</u>

To 0.6 ml of an ethanol solution containing 68 mg (0.12 mmol) of ethyl (6-{[4-(3-difluoromethoxy-1,1-dimethylpropyl)benzyl](pyridin-2-ylsulfonyl)aminomethyl}-pyridin-2-ylamino)acetate obtained in Example 64-(a) was added 0.59

ml (0.59 mmol) of 1N aqueous sodium hydroxide solution, and the mixture was stirred at room temperature for 2 hours. After completion of the reaction, the reaction mixture was adjusted to pH 4.5 with 1N hydrochloric acid, and extracted with ethyl acetate. The organic layer was washed with water, dried over anhydrous sodium sulfate and concentrated under reduced pressure to obtain 62 mg of the title compound as white foam. (Yield: 94%) Mass Spectrum (FAB, m/z): 549 ($M^+$+1).

$^1$H-NMR Spectrum (DMSO-$d_6$, δ ppm): 12.40 (brs, 0.4H), 8.61 (ddd, J=4.5, 1.8, 0.9 Hz, 1H), 7.93 (ddd, J=7.8, 7.7, 1.8 Hz, 1H), 7.77 (ddd, J=7.8, 1.1, 0.9 Hz, 1H), 7.56 (ddd, J=7.7, 4.5, 1.1 Hz, 1H), 7.27-7.13 (m, 5H), 6.74 (t, J=5.9 Hz, 1H), 6.56 (t, J=76.3 Hz, 1H), 6.33 (d, J=8.1 Hz, 1H), 6.26 (d, J=7.0 Hz, 1H), 4.65 (s, 2H), 4.22 (s, 2H), 3.82 (d, J=5.9 Hz, 2H), 3.56 (t, J=7.5 Hz, 2H), 1.93 (t, J=7.5 Hz, 2H), 1.27 (s, 6H).

Rf value: 0.47 (n-butanol:acetic acid:water=10:1:1).

[Example 65]

<u>(6-{(5-Methylfuran-2-ylsulfonyl)[4-(1,1-dimethylpentyl)benzyl]aminomethyl}pyridin-2-ylamino)acetic acid</u> (Exemplary compound No. 1444)

65-(a): <u>tert-Butyl [tert-butoxycarbonyl(6-{(5-methylfuran-2-ylsulfonyl)[4-(1,1-dimethylpentyl)benzyl]aminomethyl}pyridin-2-yl)amino]acetate</u>

To 5 ml of a methylene chloride solution containing 0.20 g (0.38 mmol) of tert-butyl [tert-butoxycarbonyl(6-{[4-(1,1-dimethylpentyl)benzyl]aminomethyl}pyridin-2-yl)amino]acetate obtained in the same manner as in Reference example 36 were added 0.21 g (1.2 mmol) of 5-methylfuran-2-ylsulfonyl chloride (see Bioorganic and Medicinal Chemistry, 14, 7121 (2006)) and 197 μl (1.41 mmol) of triethylamine, and the mixture was stirred at room temperature for 8 hours. After completion of the reaction, water was added to the reaction mixture, and the mixture was extracted with methylene chloride. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=9:1→2:1 (V/V)), and the fractions containing the objective material were concentrated under reduced pressure to obtain 154 mg of the title compound as yellow oil. (Yield: 60%)

Mass Spectrum (CI, m/z): 670 ($M^+$+1).

$^1$H-NMR Spectrum ($CDCl_3$, δ ppm): 7.72 (d, J=8.1 Hz, 1H), 7.52 (dd, J=8.1, 7.4 Hz, 1H), 7.24-7.18 (m, 2H), 7.16-7.10 (m, 2H), 6.94 (d, J=7.4 Hz, 1H), 6.79 (d, J=3.3 Hz, 1H), 6.00 (dd, J=3.3, 0.7 Hz, 1H), 4.54 (s, 2H), 4.51 (s, 2H), 4.38 (s, 2H), 2.27 (d, J=0.7 Hz, 3H), 1.60-1.53 (m, 2H), 1.52 (s, 9H), 1.42 (s, 9H), 1.25 (s, 6H), 1.24-1.12 (m, 2H), 1.06-0.94 (m, 2H), 0.82 (t, J=7.3 Hz, 3H).

65-(b): <u>(6-{(5-Methylfuran-2-ylsulfonyl)[4-(1,1-dimethylpentyl)benzyl]aminomethyl}-pyridin-2-ylamino)acetic acid</u>

To 0.5 ml of a methylene chloride solution containing 27.1 mg (0.0405 mmol) of tert-butyl [tert-butoxycarbonyl(6-{(5-methylfuran-2-ylsulfonyl)[4-(1,1-dimethylpentyl)benzyl]aminomethyl}pyridin-2-yl)amino]acetate obtained in Example 65-(a) was added 0.50 ml (6.7 mmol) of trifluoroacetic acid, and the mixture was stirred at room temperature for 6 hours. After completion of the reaction, the reaction mixture was concentrated under reduced pressure, and water was added to the residue. The mixture was adjusted to pH 4.2 with 1N aqueous sodium hydroxide solution and 1N hydrochloric acid, and extracted with ethyl acetate. The organic layer was washed with a saturated aqueous sodium chloride solution, dried over anhydrous magnesium sulfate and concentrated under reduced pressure. The residue was applied to silica gel column chromatography (eluent; ethyl acetate:methanol=19:1 (V/V)), and the fractions containing the objective material were concentrated under reduced pressure to obtain 14.8 mg of the title compound as white solid. (Yield: 71 %)

Mass Spectrum (FAB, m/z): 514 ($M^+$+1).

$^1$H-NMR Spectrum (DMSO-$d_6$, δ ppm): 12.41 (brs, 1H), 7.31-7.25 (m, 3H), 7.20-7.14 (m, 2H), 6.89 (d, J=2.9 Hz, 1H), 6.83 (t, J=5.7 Hz, 0.9H), 6.43 (d, J=8.1 Hz, 1H), 6.32 (d, J=7.3 Hz, 1H), 6.19 (d, J=2.9 Hz, 1H), 4.55 (s, 2H), 4.12 (s, 2H), 3.88 (d, J=5.7 Hz, 2H), 2.24 (s, 3H), 1.59-1.52 (m, 2H), 1.23 (s, 6H), 1.22-1.13 (m, 2H), 1.00-0.91 (m, 2H), 0.79 (t, J=7.3 Hz, 3H).

Rf value: 0.70 (n-butanol:acetic acid:water=10:1:1).

The compounds used in Examples were synthesized as follows.

[Reference example 1]

tert-Butyl (tert-butoxycarbonyl{6-[(pyridin-3-ylsulfonyl)aminomethyl]pyridin-2-yl}-amino)acetate

1-(a): tert-Butyl [tert-butoxycarbonyl(6-ethoxycarbonylpyridin-2-yl)amino]acetate

To 362 ml of a N,N-dimethylformamide solution containing 15.7 g (0.360 mol) of sodium hydride (mineral oil 55% dispersed product) was added dropwise 300 ml of a N,N-dimethylformamide solution containing 81.2 g (0.305 mol) of ethyl 6-tert-butoxy-carbonylaminopyridin-2-carboxylate (see WO2006/074884A) over 20 minutes under argon atmosphere and under ice-cooling, and the mixture was stirred at room temperature for 1 hour. Then, 54.0 ml (0.366 mol) of tert-butyl bromoacetate was added dropwise to the mixtuer over 10 minutes under ice-cooling, and the mixture was further stirred at room temperature for 1 hour. After completion of the reaction, to the reaction mixture was added an aqueous solution in which 1.77 g (33.0 mmol) of ammonium chloride had been dissolved in 300 ml of water, and the mixture was extracted with toluene. The organic layer was washed with a saturated aqueous sodium chloride solution, dried over anhydrous magnesium sulfate and concentrated under reduced pressure. The obtained residue was applied to silica gel column chromatography (eluent; n-hexane:ethyl acetate=9:1→4:1 (V/V)), and the fractions containing the objective material were concentrated under reduced pressure to obtain 108 g of the title compound as pale yellow oil. (Yield: 93%)
Mass Spectrum (CI, m/z): 381 ($M^+$+1).
$^1$H-NMR Spectrum ($CDCl_3$, $\delta$ ppm): 8.04 (d, J=7.8 Hz, 1H), 7.81 (dd, J=7.6, 1.5 Hz, 1H), 7.76 (dd, J=7.8, 7.6 Hz, 1H), 4.67 (s, 2H), 4.40 (q, J=7.1 Hz, 2H), 1.52 (s, 9H), 1.45 (s, 9H), 1.40 (t, J=7.1 Hz, 3H).

1-(b): tert-Butyl [tert-butoxycarbonyl(6-hydroxymethylpyridin-2-yl)amino]acetate

To 195 ml of an ethanol solution containing 98.8 g (0.260 mol) of tert-butyl [tert-butoxycarbonyl(6-ethoxycarbonylpyridin-2-yl)amino]acetate obtained in Reference example 1-(a) was added dropwise 195 ml of an ethanol solution containing 34.6 g (0.312 mol) of calcium chloride over 20 minutes under ice-cooling. After completion of the dropwise addition, 105 ml (0.315 mol) of 3M potassium borohydride/tetraethylene glycol dimethyl ether solution was added dropwise to the mixture at 35°C or lower over 20 minutes, and the mixture was further stirred at room temperature for 15 minutes. After completion of the reaction, the reaction mixture was added dropwise to 195 ml of aqueous solution containing 17.8 ml of acetic acid under ice-cooling over 10 minutes, and the mixture was stirred at room temperature for 1 hour. Then, 315 ml of water was added to the mixture, and the resulting mixture was extracted with toluene. The organic layer was washed successively with a saturated aqueous sodium hydrogen carbonate solution, water, and then, saturated aqueous sodium chloride solution, and concentrated under reduced pressure. The obtained residue was applied to silica gel column chromatography (eluent; n-hexane:ethyl acetate=4:1→3:2 (V/V)), and the fractions containing the objective material were concentrated under reduced pressure to obtain 81.1 g of the title compound as pale yellow oil. (Yield: 92%)
Mass Spectrum (CI, m/z): 339 ($M^+$+1).
$^1$H-NMR Spectrum ($CDCl_3$, $\delta$ ppm): 7.74 (d, J=8.2 Hz, 1H), 7.63 (dd, J=8.2, 7.4 Hz, 1H), 6.93-6.98 (m, 1H), 4.68-4.65 (m, 2H), 4.54 (s, 2H), 3.39 (t, J=5.3 Hz, 1H), 1.54 (s, 9H), 1.46 (s, 9H).

1-(c): tert-Butyl [tert-butoxycarbonyl(6-formylpyridin-2-yl)amino]acetate

To 130 ml of a methylene chloride solution containing 12.9 g (30.4 mmol) of Dess-martin reagent was added dropwise 50 ml of a methylene chloride solution containing 10.0 g (29.6 mmol) of tert-butyl [tert-butoxycarbonyl(6-hydroxymethyl-pyridin-2-yl)amino] acetate obtained in Reference example 1-(b) over 20 minutes under argon atmosphere and under ice-cooling. After completion of the dropwise addition, the mixture was stirred at room temperature for 2 hours. After completion of the reaction, to the reaction mixture was added 305 ml of 0.1% aqueous sodium thiosulfate solution, and the mixture was extracted with methylene chloride. The organic layer was washed successively with a 0.5N aqueous sodium hydroxide solution, then, with a saturated aqueous sodium chloride solution, dried over anhydrous magnesium sulfate and concentrated under reduced pressure to obtain 9.61 g of the title compound as pale yellow oil substantially quantitatively.
Mass Spectrum (EI, m/z): 336 ($M^+$).
$^1$H-NMR Spectrum (DMSO-$d_6$, $\delta$ ppm): 9.82 (s, 1H), 8.11-7.99 (m, 2H), 7.68 (dd, J=6.6, 1.5 Hz, 1H), 4.58 (s, 2H), 1.48 (s, 9H), 1.42 (s, 9H).

1-(d): tert-Butyl [tert-butoxycarbonyl(6-hydroxyiminomethylpyridin-2-yl)amino]acetate

To 29 ml of a methanol solution containing 2.88 g (8.56 mmol) oftert-butyl [tert-butoxycarbonyl(6-formylpyridin-2-yl)amino]acetate obtained in Reference example 1-(c) were added 0.650 g (9.35 mmol) of hydroxyl ammonium chloride and 3.5 ml (43 mmol) of pyridine, and the mixture was stirred at room temperature for 1 hour. After completion of the reaction, the reaction mixture was concentrated under reduced pressure. Ethyl acetate was added to the obtained residue, and the mixture was washed successively with 5% aqueous potassium hydrogen sulfate solution, a saturated aqueous sodium hydrogen carbonate solution and a saturated aqueous sodium chloride solution, dried over anhydrous magnesium sulfate and concentrated under reduced pressure. The obtained residue was applied to silica gel column chromatography (eluent; n-hexane:ethyl acetate=3:2 (V/V)), and the fractions containing the objective material were concentrated under reduced pressure to obtain 2.76 g of the title compound as colorless oily product. (Yield: 92%)
Mass Spectrum (EI, m/z): 351 ($M^+$).
$^1$H-NMR Spectrum ($CDCl_3$, $\delta$ ppm): 8.06 (s, 1H), 7.91 (s, 1H), 7.85 (d, J=8.2 Hz, 1H), 7.65 (dd, J=8.2, 7.6 Hz, 1H), 7.47 (dd, J=7.6, 0.7 Hz, 1H), 4.59 (s, 2H), 1.53 (s, 9H), 1.45 (s, 9H).

1-(e): tert-Butyl [(6-aminomethylpyridin-2-yl)tert-butoxycarbonylamino]acetate

To 49 ml of an ethanol solution containing 2.75 g (7.83 mmol) of tert-butyl [tert-butoxycarbonyl(6-hydroxyiminomethylpyridin-2-yl)amino]acetate obtained in Reference example 1-(d) was added 0.98 g of 10% palladium-active carbon (50% hydrate), and under hydrogen atmosphere at 1 atm, the mixture was stirred at room temperature for 1 hour. After completion of the reaction, insoluble materials were filtered off, and the filtrate was concentrated under reduced pressure to obtain 2.48 g of the title compound as colorless oily product. (Yield: 94%)
Mass Spectrum (CI, m/z): 338 ($M^+$+1).
$^1$H-NMR Spectrum ($CDCl_3$, $\delta$ ppm): 7.68 (d, J=8.3 Hz, 1H), 7.58 (dd, J=8.3, 7.4 Hz, 1H), 6.91 (d, J=7.4 Hz, 1H), 4.57 (s, 2H), 3.85 (s, 2H), 1.53 (s, 9H), 1.46 (s, 9H).

1-(f): tert-Butyl (tert-butoxycarbonyl{6-[(pyridin-3-ylsulfonyl)aminomethyl]pyridin-2-yl}amino)acetate

To 14 ml of a methylene chloride solution containing 0.640 g (3.60 mmol) of 3-pyridylsulfonyl chloride were added 1.20 g (3.56 mmol) of tert-butyl [(6-amino-methylpyridin-2-yl)tert-butoxycarbonylamino]acetate obtained in Reference example 1-(e) and 2.24 ml (16.2 mmol) of triethylamine, and the mixture was stirred at room temperature for 1 hour. After completion of the reaction, to the reaction mixture was added a 5% aqueous potassium hydrogen sulfate solution, and the mixture was extracted with chloroform. The organic layer was washed successively with a saturated aqueous sodium hydrogen carbonate solution, and then, with a saturated aqueous sodium chloride solution, dried over anhydrous magnesium sulfate and concentrated under reduced pressure. The obtained residue was applied to silica gel column chromatography (eluent; n-hexane: ethyl acetate=1:1→1:2 (V/V)), and the fractions containing the objective material were concentrated under reduced pressure to obtain 1.45 g of the title compound as colorless oily product. (Yield: 85%)
Mass Spectrum (CI, m/z): 479 ($M^+$+1).
$^1$H-NMR Spectrum ($CDCl_3$, $\delta$ ppm): 9.06 (d, J=2.2 Hz, 1H), 8.71 (dd, J=4.6, 1.5 Hz, 1H), 8.13-8.08 (m, 1H), 7.68 (d, J=8.2 Hz, 1H), 7.52 (dd, J=8.2, 7.4 Hz, 1H), 7.38-7.32 (m, 1H), 6.77 (d, J=7.4 Hz, 1H), 5.80 (t, J=5.1 Hz, 1H), 4.40 (s, 2H), 4.24 (d, J=5.1 Hz, 2H), 1.53 (s, 9H), 1.46 (s, 9H).

[Reference example 2]

tert-Butyl (tert-butoxycarbonyl{6-[(pyridin-2-ylsulfonyl)aminomethyl]pyridin-2-yl}-amino)acetate

Reaction and post-treatment were carried out in the similar manner as in Reference example 1-(f) except for using 1.20 g (3.56 mmol) of tert-butyl [(6-amino-methylpyridin-2-yl)tert-butoxycarbonylamino]acetate obtained in Reference example 1-(e), and using 640 mg (3.60 mmol) of 2-pyridylsulfonyl chloride in place of 3-pyridylsulfonyl chloride to obtain 1.46 g of the title compound as white solid. (Yield: 86%) Mass Spectrum (APCI, m/z): 479 ($M^+$+1).
$^1$H-NMR Spectrum ($CDCl_3$, $\delta$ ppm): 8.56 (ddd, J=4.7, 1.7, 0.9 Hz, 1H), 7.97 (ddd, J=7.8, 1.1, 0.9 Hz, 1H), 7.84 (ddd, J=7.8, 7.7, 1.7 Hz, 1H), 7.68 (d, J=8.4 Hz, 1H), 7.52 (dd, J=8.4, 7.4 Hz, 1H), 7.40 (ddd, J=7.7, 4.7, 1.1 Hz, 1H), 6.84 (dd, J=7.4, 0.5 Hz, 1H), 5.86 (t, J=5.6 Hz, 1H), 4.48 (s, 2H), 4.36 (d, J=5.6 Hz, 2H), 1.53 (s, 9H), 1.45 (s, 9H).

[Reference example 3]

tert-Butyl (tert-butoxycarbonyl{6-[(4-fluorobenzenesulfonyl)aminomethyl]pyridin-2-yl}amino)acetate

Reaction and post-treatment were carried out in the similar manner as in Reference example 1-(f) except for using 7.00 g (20.7 mmol) of tert-butyl [(6-amino-methylpyridin-2-yl)tert-butoxycarbonylamino]acetate obtained in the same manner as in Reference example 1-(e), and using 4.00 g (20.6 mmol) of 4-fluorobenzenesulfonyl chloride in place of 3-pyridylsulfonyl chloride to obtain 4.91 g of the title compound as white solid. (Yield: 48%)
Mass Spectrum (FAB, m/z): 496 ($M^{+}$+1).
$^{1}$H-NMR Spectrum ($CDCl_3$, $\delta$ ppm): 7.90-7.81 (m, 2H), 7.69 (d, J=8.3 Hz, 1H), 7.52 (dd, J=8.3, 7.4 Hz, 1H), 7.14-7.05 (m, 2H), 6.76 (dd, J=7.4, 0.6 Hz, 1H), 5.60 (t, J=5.3 Hz, 0.9H), 4.42 (s, 2H), 4.18 (d, J=5.3 Hz, 2H), 1.53 (s, 9H), 1.46 (s, 9H).

[Reference example 4]

[5-(1,1-Dimethylpentyl)thiophen-2-yl]methanol

4-(a): 2-Bromo-5-(1,1-dimethylpentyl)thiophene

To 90 ml of a methylene chloride solution containing 3.45 ml (31.5 mmol) of titanium tetrachloride was added dropwise 15.7 ml (31.4 mmol) of 2.0M dimethylzinc/- toluene solution at -50°C over 20 minutes, and the mixture was stirred at the same temperature for 50 minutes. Then, 15 ml of a methylene chloride solution containing 3.60 g (14.6 mmol) of 2-bromo-5-pentanoylthiophene (see Tetrahedron, 57, 5757 (2001)) was added dropwise to the mixture at -50°C over 30 minutes. After completion of the dropwise addition, the temperature of the mixture was gradually raised to room temperature. After completion of the reaction, the reaction mixture was poured into ice-water and the liquids were separated. The organic layer was washed successively with diluted hydrochloric acid, water, and a saturated aqueous sodium chloride solution, dried over anhydrous magnesium sulfate and concentrated under reduced pressure. The residue was applied to silica gel column chromatography (eluent; n-hexane), and the fractions containing the objective material were concentrated under reduced pressure to obtain 3.53 g of the title compound as colorless oily product.
(Yield: 93%)
Mass Spectrum (EI, m/z): 260, 262 ($M^{+}$).
$^{1}$H-NMR Spectrum ($CDCl_3$, $\delta$ ppm): 6.84 (d, J=3.8 Hz, 1H), 6.53 (d, J=3.8 Hz, 1H), 1.30-1.09 (m, 12H), 0.86 (t, J=7.1 Hz, 3H).

4-(b): 5-(1,1-Dimethylpentyl)thiophen-2-carboaldehyde

To 50 ml of a tetrahydrofuran solution containing 3.81 g (14.6 mmol) of 2-bromo-5-(1,1-dimethylpentyl)thiophene obtained in the same manner as in Reference example 4-(a) was added dropwise 11.0 ml (17.5 mmol) of 1.59M n-butyl lithium/n-hexane solution at -70°C over 15 minutes, and the mixture was stirred at the same temperature for 45 minutes. Then, 3.4 ml (44 mmol) of N,N-dimethylformamide was added dropwise to the mixture at -70°C over 10 minutes. After completion of the dropwise addition, the temperature of the mixture was gradually raised to room temperature. After completion of the reaction, a saturated aqueous ammonium chloride solution and water were added to the mixture, and the resulting mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane: ethyl acetate=9:1 (V/V)), and the fractions containing the objective material were concentrated under reduced pressure to obtain 2.91 g of the title compound as pale yellow oil. (Yield: 95%)
Mass Spectrum (CI, m/z): 211 ($M^{+}$+1).
$^{1}$H-NMR Spectrum ($CDCl_3$, $\delta$ ppm): 9.83 (s, 1H), 7.62 (t, J=4.3 Hz, 1H), 6.93 (t, J=4.3 Hz, 1H), 1.67-1.61 (m, 2H), 1.38 (s, 6H), 1.32-1.08 (m, 4H), 0.85 (t, J=7.1 Hz, 3H).

4-(c): [5-(1,1-Dimethylpentyl)thiophen-2-yl]methanol

To 25 ml of an ethanol solution containing 2.90 g (13.8 mmol) of 5-(1,1-dimethylpentyl)thiophen-2-carboaldehyde obtained in Reference example 4-(b) was added dropwise 261 mg (6.90 mmol) of potassium borohydride at room temperature over 5 minutes, and the mixture was stirred at the same temperature for 2 hours. After completion of the reaction, water and diluted hydrochloric acid were added to the reaction mixture, and the resulting mixture was extracted with ethyl acetate. The organic layer was washed with a saturated aqueous sodium chloride solution, dried over anhydrous sodium sulfate and concentrated under reduced pressure. The obtained residue was applied to silica gel column chromatography (eluent; n-hexane:ethyl acetate=4:1 (V/V)), and the fractions containing the objective material were concentrated under reduced pressure to obtain 2.90 g of the title compound as pale yellow oil.
(Yield: 99%)
Mass Spectrum (EI, m/z): 212 ($M^+$).
$^1$H-NMR Spectrum ($CDCl_3$, δ ppm): 6.81 (d, J=3.4 Hz, 1H), 6.64 (d, J=3.4 Hz, 1H), 4.76 (d, J=5.6 Hz, 2H), 1.69-1.55 (m, 2H), 1.34-1.10 (m, 10H), 0.86 (t, J=7.1 Hz, 3H).

[Reference example 5]

(5-tert-Pentylthiophen-2-yl)methanol

Reaction and post-treatment were carried out in the similar manner as in Reference example 4-(c) except for using 1.35 g (7.41 mmol) of 5-tert-pentylthiophen-2-carboaldehyde (see WO2004/069792A) in place of 5-(1,1-dimethylpentyl)thiophen-2-carboaldehyde, and using 140 mg (3.70 mmol) of potassium borohydride to obtain 1.30 g of the title compound as colorless oily product. (Yield: 95%)
Mass Spectrum (EI, m/z): 184 ($M^+$).
$^1$H-NMR Spectrum ($CDCl_3$, δ ppm): 6.81 (dt, J=3.4, 0.7 Hz, 1H), 6.64 (d, J=3.4 Hz, 1H), 4.76 (dd, J=5.9, 0.7 Hz, 2H), 1.64 (q, J=7.5 Hz, 2H), 1.32 (s, 6H), 0.79 (t, J=7.5 Hz, 3H).

[Reference example 6]

N-[4-(1-Methylcyclopropyl)benzyl]pyridin-3-ylsulfonamide

6-(a): 4-(1-Methylcyclopropyl)benzaldehyde oxime

To 20.5 ml of a methanol solution containing 1.00 g (6.24 mmol) of 4-(1-methylcyclopropyl)benzaldehyde (see WO2006/013048A) were added 2.54 ml (31.4 mmol) of pyridine and 480 mg (6.91 mmol) of hydroxylamine hydrochloride, and the mixture was stirred at room temperature for 1 hour. After completion of the reaction, the reaction mixture was concentrated under reduced pressure, and the residue was dissolved in ethyl acetate. This solution was washed successively with 1N hydrochloric acid, and a saturated aqueous sodium chloride solution, dried over anhydrous magnesium sulfate and concentrated under reduced pressure. The obtained residue was applied to silica gel column chromatography (eluent; n-hexane:ethyl acetate=4:1 (V/V)), and the fractions containing the objective material were concentrated under reduced pressure to obtain 960 mg the title compound as white solid. (Yield: 88%) Mass Spectrum (CI, m/z): 176 ($M^+$+1).
$^1$H-NMR Spectrum ($CDCl_3$, δ ppm): 8.11 (s, 1H), 7.51-7.46 (m, 2H), 7.28-7.22 (m, 2H), 1.42 (s, 3H), 0.96-0.70 (m, 4H).

6-(b): 4-(1-Methylcyclopropyl)benzylamine

To 50 ml of an ethanol solution containing 900 mg (5.14 mmol) of 4-(1-methylcyclopropyl)benzaldehyde oxime obtained in Reference example 6-(a) were added 18.5 ml (323 mmol) of acetic acid and 183 mg of platinum oxide, and under hydrogen atmosphere at 1 atm, the mixture was stirred at room temperature for 1.5 hours. After completion of the reaction, insoluble material was filtered off from the reaction mixture and the filtrate was concentrated under reduced pressure. Water was added to the residue, and the mixture was adjusted to pH 11 with 1N aqueous sodium hydroxide solution, and extracted with diethyl ether. The organic layer was washed with a saturated aqueous sodium chloride solution, dried over anhydrous magnesium sulfate and concentrated under reduced pressure to obtain 774 mg of the title compound as colorless oily product. (Yield: 93%)
Mass Spectrum (CI, m/z): 162 ($M^+$+1).
$^1$H-NMR Spectrum ($CDCl_3$, δ ppm): 7.30-7.15 (m, 4H), 3.83 (s, 2H), 1.40 (s, 3H), 0.91-0.65 (m, 4H).

6-(c): N-[4-(1-methycyclopropyl)benzyl]pyridin-3-ylsulfonamide

To 10 ml of a methylene chloride solution containing 495 mg (3.07 mmol) of 4-(1-methylcyclopropyl)benzylamine obtained in Reference example 6-(b) were added 465 mg (4.60 mmol) of triethylamine, then, 600 mg (3.38 mmol) of 3-pyridylsulfonyl chloride, and the mixture was stirred at room temperature for 2 hours. After completion of the reaction, the reaction mixture was concentrated under reduced pressure. The obtained residue was applied to silica gel column chromatography (eluent; n-hexane:ethyl acetate=2:3 (V/V)), and the fractions containing the objective material were concentrated under reduced pressure to obtain 814 mg of the title compound as a pale yellow solid. (Yield: 88%)
Mass Spectrum (CI, m/z): 303 ($M^+$+1).
$^1$H-NMR Spectrum ($CDCl_3$, δ ppm): 9.05 (d, J=2.1 Hz, 1H), 8.77 (dd, J=4.9, 1.7 Hz, 1H), 8.07 (ddd, J=8.1, 2.1, 1.7 Hz,

1H), 7.40 (ddd, J=8.1, 4.9, 0.6 Hz, 1H), 7.17-7.12 (m, 2H), 7.11-7.05 (m, 2H), 4.88 (t, J=5.8 Hz, 0.9H), 4.18 (d, J=5.8 Hz, 2H), 1.36 (s, 3H), 0.83-0.68 (m, 4H).

[Reference example 7]

4-(1-Ethylcyclopropyl)benzyl alcohol

To 55 ml of an ethanol solution containing 7.64 g (43.8 mmol) of 4-(1-ethylcyclopropyl)benzaldehyde (see WO2006/013048A was added 828 mg (21.9 mmol) of potassium borohydride at room temperature, and the mixture was stirred at the same temperature for 16 hours. After completion of the reaction, water was added to the reaction mixture and the mixture was extracted with ethyl acetate. The organic layer was washed with a saturated aqueous sodium chloride solution, dried over anhydrous sodium sulfate and concentrated under reduced pressure. The obtained residue was applied to silica gel column chromatography (eluent; n-hexane:ethyl acetate=1:0→5:1 (V/V)), and the fractions containing the objective material were concentrated under reduced pressure to obtain 7.43 g of the title compound as pale yellow oil. (Yield: 96%) Mass Spectrum (EI, m/z): 176 ($M^+$).
$^1$H-NMR Spectrum ($CDCl_3$, $\delta$ ppm): 7.32-7.24 (m, 4H), 4.66 (d, J=5.6 Hz, 2H), 1.59 (t, J=5.6 Hz, 1H), 1.58 (q, J=7.3 Hz, 2H), 0.84 (t, J=7.3 Hz, 3H), 0.80-0.61 (m, 4H).

[Reference example 8]

4-(1-Isopropylcyclopropyl)benzyl bromide

8-(a): 1-Methyl-4-(3-methylbutan-1-en-2-yl)benzene

To 100 ml of a tetrahydrofuran solution containing 19.8 g (55.4 mmol) of methyltriphenylphosphonium bromide were added dropwise 32.5 ml (52 mmol) of 1.6M n-butyl lithium/n-hexane solution at -70°C over 20 minutes. After completion of the dropwise addition, the temperature of the mixture was gradually raised to room temperature over 1.5 hours. Then, 18 ml of a tetrahydrofuran solution containing 6.00 g (37.0 mmol) of 2-methyl-1-p-tollylpropan-1-one (see WO2005/100292A was added dropwise to the mixture over 25 minutes, and the mixture was refluxed for 2 hours. After completion of the reaction, a saturated aqueous sodium hydrogen carbonate solution was added to the reaction mixture, and the resulting mixture was extracted with diethyl ether. The organic layer was washed with a saturated aqueous sodium chloride solution, dried over anhydrous magnesium sulfate and concentrated under reduced pressure to obtain 6.61 g (pure content: 5.93 g) of the title compound as pale yellow oil substantially quantitatively.
Mass Spectrum (EI, m/z): 160 ($M^+$).
$^1$H-NMR Spectrum ($CDCl_3$, $\delta$ ppm): 7.27-7.22 (m, 2H), 7.16-7.09 (m, 2H), 5.12 (dd, J=1.3, 0.5 Hz, 1H), 4.99 (dd, J=1.3, 1.3 Hz, 1H), 2.90-2.74 (m, 1H), 2.34 (s, 3H), 1.09 (d, J=6.6 Hz, 6H).

8-(b): 1-(1-Isopropylcyclopropyl)-4-methylbenzene

To 70.5 ml of a methylene chloride solution containing 74 ml (74.0 mmol) of 1.0M diethylzinc/hexane solution was added dropwise 5.50 ml (74.0 mmol) of trifluoroacetic acid at 0°C over 40 minutes, and the mixture was stirred at the same temperature for 40 minutes. Then, 5.96 ml (74.0 mmol) of diiodomethane was added dropwise to the mixture over 15 minutes, and the mixture was stirred at the same temperature for 30 minutes. To the mixture was further added dropwise 27.5 ml of a methylene chloride solution containing 6.61 g (pure content: 5.93 g, 37.0 mmol) of 1-methyl-4-(3-methylbutan-1-en-2-yl)benzene obtained in Reference example 8-(a) over 25 minutes, and the temperature of the mixture was raised to room temperature over 3.5 hours and stirred. After completion of the reaction, a saturated aqueous ammonium chloride solution was added to the reaction mixture and the liquids were separated. The organic layer was successively washed with a saturated aqueous ammonium chloride solution, water, and a saturated aqueous sodium chloride solution, dried over anhydrous magnesium sulfate and concentrated under reduced pressure. The obtained crude material was applied to distillation under reduced pressure (64 to 65°C/120Pa) to obtain 4.31 g of the title compound as colorless oily product. (Yield: 67%)
Mass Spectrum (EI, m/z): 174 ($M^+$).
$^1$H-NMR Spectrum ($CDCl_3$, $\delta$ ppm): 7.21-7.14 (m, 2H), 7.10-7.03 (m, 2H), 2.32 (s, 3H), 1.23-1.05 (m, 1H), 0.86 (d, J=6.8 Hz, 6H), 0.71-0.56 (m, 4H).

8-(c): 4-(1-Isopropylcyclopropyl)benzyl bromide

To 57.5 ml of a 1,2-dichloroethane solution containing 1.00 g (5.74 mmol) of 1-(1-isopropylcyclopropyl)-4-methylbenzene obtained in Reference example 8-(b) was added 1.12 g (6.31 mmol) of NBS, and the mixture was gently refluxed for 2.5 hours under photo-irradiation. During the reaction, 407 mg (2.29 mmol) of NBS was additionally added. After completion of the reaction, the reaction mixture was concentrated under reduced pressure. The obtained residue was applied to silica gel column chromatography (eluent; n-hexane), and the fractions containing the objective material were concentrated under reduced pressure to obtain 461 mg of the title compound as pale yellow oil. (Yield: 32%)
Mass Spectrum (CI, m/z): 253, 255 ($M^+$+1).
$^1$H-NMR Spectrum ($CDCl_3$, $\delta$ ppm): 7.32-7.21 (m, 4H), 4.49 (s, 2H), 1.26-1.11 (m, 1H), 0.86 (d, J=6.8 Hz, 6H), 0.72-0.60 (m, 4H).

[Reference example 9]

4-(1-Butylcyclopropyl)benzyl alcohol

9-(a): 1-Bromo-4-(hexan-1-en-2-yl)benzene

Reaction was carried out in the same manner as in Reference example 8-(a) except for using 10.2 g (42.3 mmol) of 1-(4-bromophenyl)pentan-1-one in place of 2-methyl-1-p-tollylpropan-1-one, and using 22.7 g (63.5 mmol) of methyltriphenyl phosphonium bromide. After completion of the reaction, a saturated aqueous sodium hydrogen carbonate solution was added to the reaction mixture, and the mixture was extracted with diethyl ether. The organic layer was washed with a saturated aqueous sodium chloride solution, dried over anhydrous magnesium sulfate and concentrated under reduced pressure. The obtained residue was applied to silica gel column chromatography (eluent; n-hexane), and the fractions containing the objective material were concentrated under reduced pressure to obtain 10.1 g of the title compound as colorless oily product quantitatively.
Mass Spectrum (CI, m/z): 239, 241 ($M^+$+1).
$^1$H-NMR Spectrum ($CDCl_3$, $\delta$ ppm): 7.47-7.40 (m, 2H), 7.30-7.23 (m, 2H), 5.24 (d, J=1.0 Hz, 1H), 5.08-5.05 (m, 1H), 2.46 (t, J=7.0 Hz, 2H), 1.47-1.24 (m, 4H), 0.89 (t, J=7.2 Hz, 3H).

9-(b): 1-Bromo-4-(1-butylcyclopropyl)benzene

Reaction was carried out in the similar manner as in Reference example 8-(b) except for using 10.0 g (41.8 mmol) of 1-bromo-4-(hexan-1-en-2-yl)benzene obtained in Reference example 9-(a) in place of 1-methyl-4-(3-methylbutan-1-en-2-yl)benzene, and using 80 ml (88 mmol) of 1.1M diethylzinc/hexane solution, 7.7 ml (0.10 mol) of trifluoroacetic acid and 8.3 ml (0.10 mol) of diiodomethane. After completion of the reaction, a saturated aqueous ammonium chloride solution was added to the reaction mixture and the liquids were separated. The organic layer was washed with a saturated aqueous sodium chloride solution, dried over anhydrous magnesium sulfate and concentrated under reduced pressure. The obtained residue was applied to silica gel column chromatography (eluent; n-hexane), and the fractions containing the objective material were concentrated under reduced pressure to obtain 7.85 g of the title compound as colorless oily product. (Yield: 74%)
Mass Spectrum (CI, m/z): 253, 255 ($M^+$+1).
$^1$H-NMR Spectrum ($CDCl_3$, $\delta$ ppm): 7.42-7.34 (m, 2H), 7.19-7.12 (m, 2H), 1.56-1.46 (m, 2H), 1.31-1.13 (m, 4H), 0.82 (t, J=6.8 Hz, 3H), 0.77-0.62 (m, 4H).

9-(c): 4-(1-Butylcyclopropyl)benzaldehyde

To 88 ml of a tetrahydrofuran solution containing 7.84 g (31.0 mmol) of 1-bromo-4-(1-butylcyclopropyl)benzene obtained in Reference example 9-(b) was added dropwise 19.3 ml (31 mmol) of 1.6M n-butyl lithium/n-hexane solution at -70°C over 30 minutes, and the mixture was stirred at the same temperature for 30 minutes. Then, 6.8 g (93 mmol) of N,N-dimethylformamide was added dropwise to the mixture over 10 minutes, the mixture was stirred at the same temperature for 10 minutes, and then, the temperature of the mixture was gradually raised to room temperature over 1.5 hours. After completion of the reaction, a saturated aqueous ammonium chloride solution was added to the reaction mixture, and the resulting mixture was extracted with diethyl ether. The organic layer was washed with a saturated aqueous sodium chloride solution, dried over anhydrous magnesium sulfate and concentrated under reduced pressure. The obtained residue was applied to silica gel column chromatography (eluent; n-hexane:ethyl acetate=9:1 (V/V)), and the fractions containing the objective material were concentrated under reduced pressure to obtain 5.57 g of the title compound as colorless oily product. (Yield: 89%)
Mass Spectrum (CI, m/z): 203 ($M^+$+1).
$^1$H-NMR Spectrum ($CDCl_3$, $\delta$ ppm): 9.97 (s, 1H), 7.82-7.77 (m, 2H), 7.46-7.40 (m, 2H), 1.64-1.58 (m, 2H), 1.30-1.18 (m, 4H), 0.88-0.73 (m, 7H).

9-(d): 4-(1-Butylcyclopropyl)benzyl alcohol

Reaction and post-treatment were carried out in the similar manner as in Reference example 7 except for using 2.02 g (9.99 mmol) of 4-(1-butylcyclopropyl)-benzaldehyde obtained in Reference example 9-(c) in place of 4-(1-ethylcyclopropyl)-benzaldehyde, and using 189 mg (5.00 mmol) of potassium borohydride to obtain 1.30 g of the title compound as colorless oily product. (Yield: 64%)
Mass Spectrum (EI, m/z): 204 ($M^+$).
$^1$H-NMR Spectrum ($CDCl_3$, $\delta$ ppm): 7.33-7.23 (m, 4H), 4.65 (d, J=5.9 Hz, 2H), 1.61 (t, J=5.9 Hz, 1H), 1.57-1.49 (m, 2H), 1.30-1.18 (m, 4H), 0.82 (t, J=7.0 Hz, 3H), 0.80-0.61 (m, 4H).

[Reference example 10]

4-(1-Nonylcyclopropyl)benzyl alcohol

10-(a): 1-[1-(4-Bromophenyl)cyclopropyl]nonan-1-one

To 23 ml of a tetrahydrofuran solution containing 2.50 g (11.3 mmol) of 1-(4-bromophenyl)cyclopropanecarbonitrile (see US6369261B1) was added dropwise 6.8 ml (14 mmol) of 2.0M octyl magnesium chloride/tetrahydrofuran solution at room temperature over 10 minutes, and then, the mixture was refluxed for 5.5 hours. After completion of the reaction, the reaction mixture was poured into a saturated aqueous ammonium chloride solution, diluted sulfuric acid was added to the mixture, and the resulting mixture was extracted with toluene. The organic layer was washed with a saturated aqueous sodium chloride solution, dried over anhydrous sodium sulfate and concentrated under reduced pressure. The obtained residue was applied to silica gel column chromatography (eluent; n-hexane:ethyl acetate=1:0$\rightarrow$10:1 (V/V)), and the fractions containing the objective material were concentrated under reduced pressure to obtain 2.15 g of the title compound as colorless oily product. (Yield: 56%)
Mass Spectrum (CI, m/z): 337, 339 ($M^+$+1).
$^1$H-NMR Spectrum ($CDCl_3$, $\delta$ ppm): 7.50-7.45 (m, 2H), 7.26-7.20 (m, 2H), 2.24 (t, J=7.1 Hz, 2H), 1.58 (q, J=3.5 Hz, 2H), 1.50-1.37 (m, 2H), 1.31-1.05 (m, 10H), 1.10 (q, J=3.5 Hz, 2H), 0.86 (t, J=7.1 Hz, 3H).

10-(b): 1-Bromo-4-(1-nonylcyclopropyl)benzene

To 11.5 ml of a diethylene glycol solution containing 1.94 g (5.76 mmol) of 1-[1-(4-bromophenyl)cyclopropyl]nonan-1-one obtained in Reference example 10-(a) were added 878 mg (17.5 mmol) of hydrazine monohydrate and 973 mg (17.3 mmol) of potassium hydroxide, and the mixture was stirred at 200°C for 5 hours. After completion of the reaction, water and a saturated aqueous sodium chloride solution were added to the reaction mixture, and the resulting mixture was extracted with diethyl ether. The organic layer was washed with a saturated aqueous sodium chloride solution, dried over anhydrous sodium sulfate and concentrated under reduced pressure. The obtained residue was applied to silica gel column chromatography (eluent; n-hexane), and the fractions containing the objective material were concentrated under reduced pressure to obtain 1.34 g of the title compound as colorless oily product. (Yield: 72%)
Mass Spectrum (CI, m/z): 323, 325 ($M^+$+1).
$^1$H-NMR Spectrum ($CDCl_3$, $\delta$ ppm): 7.41-7.35 (m, 2H), 7.18-7.12 (m, 2H), 1.56-1.45 (m, 2H), 1.34-1.15 (m, 14H), 0.87 (t, J=6.8 Hz, 3H), 0.76-0.62 (m, 4H).

10-(c): 4-(1-Nonylcyclopropyl)benzaldehyde

Reaction and post-treatment were carried out in the similar manner as in Reference example 9-(c) except for using 324 mg (1.00 mmol) of 1-bromo-4-(1-nonylcyclopropyl)benzene obtained in Reference example 10-(b) in place of 1-bromo-4-(1-butylcyclopropyl)benzene, and using 0.75 ml (1.2 mmol) of 1.6M n-butyl lithium/n-hexane solution and 0.25 ml (3.2 mmol) of N,N-dimethylformamide to obtain 251 mg of the title compound as colorless oily product. (Yield: 92%)
Mass Spectrum (CI, m/z): 273 ($M^+$+1).
$^1$H-NMR Spectrum ($CDCl_3$, $\delta$ ppm): 9.97 (s, 1H), 7.82-7.76 (m, 2H), 7.45-7.40 (m, 2H), 1.65-1.53 (m, 2H), 1.33-1.14 (m, 14H), 0.90-0.73 (m, 7H).

10-(d): 4-(1-Nonylcyclopropyl)benzyl alcohol

Reaction and post-treatment were carried out in the similar manner as in Reference example 7 except for using 246 mg (0.903 mmol) of 4-(1-nonylcyclopropyl)-benzaldehyde obtained in Reference example 10-(c) in place of 4-(1-ethylcyclopropyl)-benzaldehyde, and using 17.5 mg (0.463 mmol) of potassium borohydride to obtain 240 mg of the title

compound as pale yellow oil. (Yield: 97%)
Mass Spectrum (EI, m/z): 274 ($M^+$).
$^1$H-NMR Spectrum ($CDCl_3$, δ ppm): 7.31-7.25 (m, 4H), 4.66 (d, J=6.1 Hz, 2H), 1.58-1.49 (m, 3H), 1.34-1.14 (m, 14H), 0.86 (t, J=6.7 Hz, 3H), 0.80-0.61 (m, 4H).

[Reference example 11]

4-(1-Methoxycyclopropyl)benzyl alcohol

Reaction and post-treatment were carried out in the similar manner as in Reference example 7 except for using 1.17 g (6.64 mmol) of 4-(1-methoxycyclopropyl)benzaldehyde (see WO2006/013048A) in place of 4-(1-ethylcyclopropyl)-benzaldehyde, and using 125 mg (3.30 mmol) of potassium borohydride to obtain 1.03 g of the title compound as colorless oily product. (Yield: 87%)
Mass Spectrum (EI, m/z): 178 ($M^+$).
$^1$H-NMR Spectrum ($CDCl_3$, δ ppm): 7.37-7.28 (m, 4H), 4.68 (d, J=5.6 Hz, 2H), 3.23 (s, 3H), 1.77 (t, J=5.6 Hz, 0.9H), 1.27-1.11 (m, 2H), 1.04-0.87 (m, 2H).

[Reference example 12]

4-(1-Ethylcyclobutyl)benzyl alcohol

12-(a): 1-[1-(4-Bromophenyl)cyclobutyl]ethan-1-one

Reaction and post-treatment were carried out in the similar manner as in Reference example 10-(a) except for using 4.45 g (18.8 mmol) of 1-(4-bromophenyl)-cyclobutanecarbonitrile (see WO2008/018827A) in place of 1-(4-bromophenyl)cyclopropanecarbonitrile, and using 48.0 ml (48 mmol) of 1.0M methylmagnesium bromide/tetrahydrofuran solution in place of 2.0M octylmagnesium chloride/tetrahydrofuran solution, respectively, to obtain 4.69 g of the title compound as pale yellow oil. (Yield: 98%)
Mass Spectrum (CI, m/z): 253, 255 ($M^+$+1).
$^1$H-NMR Spectrum ($CDCl_3$, δ ppm): 7.51-7.44 (m, 2H), 7.15-7.08 (m, 2H), 2.80-2.68 (m, 2H), 2.44-2.31 (m, 2H), 2.00-1.78 (m, 2H), 1.93 (s, 3H).

12-(b): 1-Bromo-4-1-ethylcyclobutyl)benzene

Reaction and post-treatment were carried out in the similar manner as in Reference example 10-(b) except for using 503 mg (1.99 mmol) of 1-[1-(4-bromophenyl)cyclobutyl]ethan-1-one obtained in Reference example 12-(a) in place of 1-[1-(4-bromophenyl)cyclopropyl]nonan-1-one, and using 0.29 ml (6.0 mmol) of hydrazine monohydrate and 334 mg (5.95 mmol) of potassium hydroxide to obtain 397 mg of the title compound as colorless oily product. (Yield: 83%)
Mass Spectrum (EI, m/z): 238, 240 ($M^+$).
$^1$H-NMR Spectrum ($CDCl_3$, δ ppm): 7.43-7.36 (m, 2H), 7.00-6.93 (m, 2H), 2.34-2.20 (m, 2H), 2.15-1.94 (m, 3H), 1.90-1.71 (m, 1H), 1.76 (q, J=7.4 Hz, 2H), 0.63 (t, J=7.4 Hz, 3H).

12-(c): 4-(1-Ethylcyclobutyl)benzaldehyde

Reaction and post-treatment were carried out in the similar manner as in Reference example 9-(c) except for using 258 mg (1.08 mmol) of 1-bromo-4-(1-ethylcyclobutyl)benzene obtained in Reference example 12-(b) in place of 1-bromo-4-(1-butylcyclopropyl)benzene, and using 0.82 ml (1.3 mmol) of 1.6M n-butyl lithium/n-hexane solution and 0.26 ml (3.4 mmol) of N,N-dimethylformamide to obtain 56 mg of the title compound as pale yellow oil. (Yield: 28%)
Mass Spectrum (CI, m/z): 189 ($M^+$+1).
$^1$H-NMR Spectrum ($CDCl_3$, δ ppm): 9.98 (s, 1H), 7.84-7.78 (m, 2H), 7.28-7.22 (m, 2H), 2.42-2.27 (m, 2H), 2.22-1.99 (m, 3H), 1.90-1.77 (m, 1H), 1.84 (q, J=7.4 Hz, 2H), 0.65 (t, J=7.4 Hz, 3H).

12-(d): 4-(1-Ethylcyclobutyl)benzyl alcohol

Reaction and post-treatment were carried out in the similar manner as in Reference example 7 except for using 43 mg (0.23 mmol) of 4-(1-ethylcyclobutyl)-benzaldehyde obtained in Reference example 12-(c) in place of 4-(1-ethylcyclopropyl)-benzaldehyde, and using 4.6 mg (0.12 mmol) of potassium borohydride to obtain 44 mg of the title compound as pale yellow oil quantitatively.

Mass Spectrum (EI, m/z): 190 ($M^+$).
$^1$H-NMR Spectrum ($CDCl_3$, δ ppm): 7.33-7.27 (m, 2H), 7.13-7.07 (m, 2H), 4.66 (s, 2H), 2.38-2.24 (m, 2H), 2.17-1.97 (m, 3H), 1.87-1.76 (m, 1H), 1.79 (q, J=7.3 Hz, 2H), 0.64 (t, J=7.3 Hz, 3H).

[Reference example 13]

tert-Butyl [tert-butoxycarbonyl(6-{[4-(1-butylcyclopropyl)benzyl]aminomethyl}-pyridin-2-yl)amino]acetate

To 5 ml of a 1,2-dichloroethane solution containing 202 mg (0.999 mmol) of 4-(1-butylcyclopropyl)benzaldehyde obtained in Reference example 9-(c) was added 321 mg (0.951 mmol) of tert-butyl [(6-aminomethylpyridin-2-yl)tert-butoxycarbonylamino]-acetate obtained in the same manner as in Reference example 1-(e), and the mixture was stirred at room temperature for 30 minutes. Then, 318 mg (1.50 mmol) of triacetoxy potassium borohydride was added by dividing into several portions to the mixture at room temperature, and the mixture was stirred at the same temperature for 7 hours. After completion of the reaction, a saturated aqueous sodium hydrogen carbonate solution was added to the reaction mixture, and the mixture was extracted with ethyl acetate. The organic layer was dried over anhydrous magnesium sulfate and concentrated under reduced pressure. The residue was applied to silica gel column chromatography (eluent; n-hexane:ethyl acetate=1:1→1:3 (V/V)), and the fractions containing the objective material were concentrated under reduced pressure to obtain 314 mg of the title compound as colorless oily product. (Yield: 63%)
Mass Spectrum (CI, m/z): 524 ($M^+$+1).
$^1$H-NMR Spectrum ($CDCl_3$, δ ppm): 7.68 (d, J=8.2 Hz, 1H), 7.58 (dd, J=8.2, 7.6 Hz, 1H), 7.31-7.19 (m, 4H), 6.97 (d, J=7.6 Hz, 1H), 4.56 (s, 2H), 3.81 (s, 2H), 3.76 (s, 2H), 1.56-1.45 (m, 2H), 1.52 (s, 9H), 1.41 (s, 9H), 1.29-1.18 (m, 4H), 0.86-0.78 (m, 3H), 0.78-0.72 (m, 2H), 0.66-0.61 (m, 2H).

[Reference example 14]

N-[4-(1-butylcyclopropyl)benzyl]pyridin-4-ylsulfonamide

14-(a): N-(2,4-dimethoxybenzyl)-S-(pyridin-4-yl)thiohydroxylamine

To 18 ml of a methylene chloride solution containing 2.00 g (10.8 mmol) of benzyltrimethyl ammonium chloride were added 836 mg (3.60 mmol) of trichloroisocyanuric acid and 90 μl of methanol, and the mixture was stirred at room temperature for 30 minutes. This solution was added dropwise to 36 ml of a methylene chloride solution containing 1.00 g (9.00 mmol) of 4-mercaptopyridine at -70°C over 4 minutes, and the mixture was stirred at the same temperature for 1 hour. Then, 4.75 ml (31.6 mmol) of 2,4-dimethoxybenzylamine was added dropwise to the mixture at -70 over 10 minutes, then, 1.25 ml (8.97 mmol) of triethylamine was added to the same, and the temperature of the mixture was raised to room temperature over 4 hours. After completion of the reaction, water was added to the reaction mixture under ice-cooling and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=1:1→0:1 (V/V)), and the fractions containing the objective material were concentrated under reduced pressure to obtain 1.62 g of the title compound as yellowish brown oil. (Yield: 65%)
Mass Spectrum (CI, m/z): 277 ($M^+$+1).
$^1$H-NMR Spectrum ($CDCl_3$, δ ppm): 8.40 (dd, J=4.6, 1.5 Hz, 2H), 7.23 (dd, J=4.6, 1.5 Hz, 2H), 7.14 (d, J=8.2 Hz, 1H), 6.48 (d, J=2.3 Hz, 1H), 6.45 (dd, J=8.2,2.3 Hz, 1H), 4.03 (d, J=6.1 Hz, 2H), 3.86 (s, 3H), 3.82 (s, 3H), 3.22 (t, J=6.1 Hz, 0.9H).

14-(b): N-(2,4-dimethoxybenzyl)pyridin-4-ylsulfonamide

To 120 ml of an acetone solution containing 1.61 g (5,83 mmol) of N-(2,4-dimethoxybenzyl)-S-(pyridin-4-yl)thiohydroxylamine obtained in Reference example 14-(a) was added dropwise 58 ml (12 mmol) of 0.200 mol/l aqueous potassium permanganate solution under ice-cooling over 30 minutes, and the mixture was stirred at room temperature for 14 hours. After completion of the reaction, the reaction mixture was filtered through Celite, the filtrate was concentrated under reduced pressure, and extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane: ethyl acetate=1:1→0:1 (V/V)), and the fractions containing the objective material were concentrated under reduced pressure to obtain 387 mg of the title compound as yellow oil. (Yield: 22%)
Mass Spectrum (CI, m/z): 309 ($M^+$+1).

$^1$H-NMR Spectrum ($CDCl_3$, δ ppm): 8.69 (dd, J=4.4, 1.6 Hz, 2H), 7.53 (dd, J=4.4, 1.6 Hz, 2H), 6.95 (d, J=8.2 Hz, 1H), 6.31 (dd, J=8.2,2.4 Hz, 1H), 6.26 (d, J=2.4 Hz, 1H), 5.18 (t, J=6.3 Hz, 0.8H), 4.17 (d, J=6.3 Hz, 2H), 3.76 (s, 3H), 3.69 (s, 3H).

14-(c): N-[4-(1-butylcyclopropyl)benzyl]-N-(2,4-dimethoxybenzyl)pyridin-4-yl-sulfonamide

To 3 ml of a tetrahydrofuran solution containing 181 mg (0.587 mmol) of N-(2,4-dimethoxybenzyl)pyridin-4-yl-sulfonamide obtained in Reference example 14-(b) were added 134 mg (0.656 mmol) of 1-butylcyclopropylbenzyl alcohol obtained in Reference example 9-(d), 0.22 ml (0.88 mmol) of tri-n-butylphosphine and 152 mg (0.883 mmol) of N,N,N',N'-tetramethylazodicarboxamide, and the mixture was stirred at room temperature for 3 hours. After completion of the reaction, the reaction mixture was concentrated under reduced pressure, water was added to the residue, and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=10:1→1:1(V/V), and the fractions containing the objective material were concentrated under reduced pressure to obtain 275 mg of the title compound as yellow oil. (Yield: 95%)
Mass Spectrum (CI, m/z): 495 ($M^+$+1).
$^1$H-NMR Spectrum ($CDCl_3$, δ ppm): 8.66 (dd, J=4.5, 1.6 Hz, 2H), 7.41 (dd, J=4.5, 1.6 Hz, 2H), 7.21-7.15 (m, 2H), 7.14-7.04 (m, 3H), 6.38 (dd, J=8.4, 2.3 Hz, 1H), 6.21 (d, J=2.3 Hz, 1H), 4.42 (s, 2H), 4.34 (s, 2H), 3.78 (s, 3H), 3.54 (s, 3H), 1.57-1.47 (m, 2H), 1.30-1.18 (m, 4H), 0.84 (t, J=6.8 Hz, 3H), 0.76-0.70 (m, 2H), 0.69-0.61 (m, 2H).

14-(d): N-[4-(1-butylcyclopropyl)benzyl]pyridin-4-ylsulfonamide

To 2.2 ml of a methylene chloride solution containing 216 mg (0.437 mmol) of N-[4-(1-butylcyclopropyl)benzyl]-N-(2,4-dimethoxybenzyl)pyridin-4-ylsulfonamide obtained in Reference example 14-(c) was added dropwise 2.2 ml (30 mmol) of trifluoroacetic acid under ice-cooling over 15 minutes, and the mixture was stirred at room temperature for 24 hours. After completion of the reaction, the reaction mixture was concentrated under reduced pressure, and water was added to the residue. The mixture was made basic with a saturated aqueous sodium hydrogen carbonate solution, and extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=1:1→1:10 (V/V)), then, purified by HPLC preparative (column; SunFireC18 (available from Waters), eluent; water:acetonitrile =11:9(V/V)), and the fractions containing the objective material were concentrated under reduced pressure to obtain 96.6 mg of the title compound as white solid. (Yield: 64%)
Mass Spectrum (CI, m/z): 345 ($M^+$+1).
$^1$H-NMR Spectrum ($CDCl_3$, δ ppm): 8.80 (dd, J=4.4, 1.7 Hz, 2H), 7.66 (dd, J=4.4, 1.7 Hz, 2H), 7.23-7.17 (m, 2H), 7.11-7.04 (m, 2H), 4.78 (t, J=5.9 Hz, 0.8H), 4.19 (d, J=5.9 Hz, 2H), 1.58-1.47 (m, 2H), 1.29-1.18 (m, 4H), 0.82 (t, J=6.8 Hz, 3H), 0.75-0.70 (m, 2H), 0.68-0.62 (m, 2H).

[Reference example 15]

4-(1-Butylcyclobutyl)benzyl alcohol

15-(a): 1-[1-(4-Bromophenyl)cyclobutyl]butan-1-one

To 30 ml of a tetrahydrofuran solution containing 4.0 g (17 mmol) of 1-(4-bromophenyl)cyclobutanecarbonitrile was added 38 ml (76 mmol) of 2M propylmagnesium bromide/tetrahydrofuran solution, and the mixture was refluxed for 11 hours. After completion of the reaction, the reaction mixture was poured into 200 ml of 1N hydrochloric acid, and extracted with ethyl acetate. The organic layer was washed successively with a saturated aqueous sodium hydrogen carbonate solution and a saturated aqueous sodium chloride solution in this order, dried over anhydrous magnesium sulfate and concentrated under reduced pressure. The residue was applied to silica gel column chromatography (eluent; n-hexane:ethyl acetate=19:1 (V/V), and the fractions containing the objective material were concentrated under reduced pressure to obtain 3.78 g of the title compound as yellow oil. (Yield: 79%)
Mass Spectrum (CI, m/z): 281, 283 ($M^+$+1).
$^1$H-NMR Spectrum ($CDCl_3$, δ ppm): 7.50-7.44 (m, 2H), 7.14-7.08 (m, 2H), 2.81-2.66 (m, 2H), 2.44-2.29 (m, 2H), 2.18 (t, J=7.4 Hz, 2H), 1.96-1.78 (m, 2H), 1.53-1.37 (m, 2H), 0.74 (t, J=7.4 Hz, 3H).

15-(b): 1 Bromo-4-(1-butylcyclobutyl)benzene

To 30 ml of an ethylene glycol solution containing 3.70 g (13.2 mmol) of 1-[1-(4-bromophenyl)cyclobutyl]butan-1-one obtained in Reference example 15-(a) were added 1.92 ml (39.2 mmol) of hydrazine monohydrate and 2.22 g (39.6 mmol) of potassium hydroxide, and the mixture was stirred at 200°C for 6 hours. After completion of the reaction, water was added to the reaction mixture, and the resulting mixture was extracted with diisopropyl ether. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=19:1 (V/V)), and the fractions containing the objective material were concentrated under reduced pressure to obtain 1.58 g of the title compound as colorless oily product. (Yield: 45%)
Mass Spectrum (EI, m/z): 266, 268 ($M^+$).
$^1$H-NMR Spectrum ($CDCl_3$, $\delta$ ppm): 7.42-7.36 (m, 2H), 7.00-6.94 (m, 2H), 2.34-2.20 (m, 2H), 2.15-1.98 (m, 3H), 1.86-1.76 (m, 1H), 1.76-1.68 (m, 2H), 1.28-1.12 (m, 2H), 1.02-0.89 (m, 2H), 0.81 (t, J=7.3 Hz, 3H).

15-(c): 4-(1-Butylcyclobutyl)benzaldehyde

Reaction and post-treatment were carried out in the similar manner as in Reference example 9-(c) except for using 0.90 g (3.4 mmol) of 1-bromo-4-(1-butylcyclobutyl)benzene obtained in Reference example 15-(b) in place of 1-bromo-4-(1-butylcyclopropyl)benzene, and using 3.1 ml (5.0 mmol) of 1.6M n-butyl lithium/n-hexane solution and 0.80 ml (10 mmol) of N,N-dimethylformamide to obtain 0.42 g of the title compound as pale yellow oil. (Yield: 57%)
Mass Spectrum (CI, m/z): 217 ($M^+$+1).
$^1$H-NMR Spectrum ($CDCl_3$, $\delta$ ppm): 9.98 (s, 1H), 7.85-7.77 (m, 2H), 7.29-7.22 (m, 2H), 2.45-2.26 (m, 2H), 2.23-1.99 (m, 3H), 1.91-1.73 (m, 3H), 1.27-1.13 (m, 2H), 1.04-0.88 (m, 2H), 0.80 (t, J=7.2 Hz, 3H).

15-(d): 4-(1-Butylcyclobutyl)benzyl alcohol

Reaction and post-treatment were carried out in the similar manner as in Reference example 7 except for using 0.30 g (1.4 mmol) of 4-(1-butylcyclobutyl)-benzaldehyde obtained in Reference example 15-(c) in place of 4-(1-ethylcyclopropyl)-benzaldehyde, and using 105 mg (2.77 mmol) of potassium borohydride to obtain 0.19 g of the title compound as colorless oily product. (Yield: 62%)
Mass Spectrum (EI, m/z): 218 ($M^+$).
$^1$H-NMR Spectrum ($CDCl_3$, $\delta$ ppm): 7.33-7.27 (m, 2H), 7.14-7.07 (m, 2H), 4.67 (d, J=5.5 Hz, 2H), 2.42-2.22 (m, 2H), 2.18-1.95 (m, 3H), 1.90-1.69 (m, 3H), 1.58 (t, J=5.5 Hz, 1H), 1.28-1.12 (m, 2H), 1.05-0.88 (m, 2H), 0.81 (t, J=7.3 Hz, 3H).

[Reference example 16]

4-(1-Propoxymethylcyclopropyl)benzyl alcohol

16-(a): 1-Bromo-4-(1-propoxymethylcyclopropyl)benzene

To 4.8 ml of a N,N-dimethylformamide solution containing 550 mg (2.42 mmol) of [1-(4-bromophenyl)cyclopropyl]methanol (see WO2010/047982A) was added dropwise 141 mg (3.2 mmol) of sodium hydride (mineral oil 55% dispersed product) by dividing into several portions at room temperature, and the mixture was stirred at the same temperature for 30 minutes. Then, 0.29 ml (3.2 mmol) of 1-bromopropane was added dropwise to the mixture at room temperature, and the mixture was stirred at the same temperature for 16 hours. After completion of the reaction, water was added to the reaction mixture, and the resulting mixture was extracted with n-hexane. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate= 10:1→1:1 (V/V)), and the fractions containing the obj ective material were concentrated under reduced pressure to obtain 282 mg of the title compound as pale yellow oil.
(Yield: 43%)
Mass Spectrum (EI, m/z): 268, 270 ($M^+$).
$^1$H-NMR Spectrum ($CDCl_3$, $\delta$ ppm): 7.42-7.36 (m, 2H), 7.24-7.17 (m, 2H), 3.48 (s, 2H), 3.34 (t, J=6.6 Hz, 2H), 1.60-1.46 (m, 2H), 0.91-0.81 (m, 7H).

16-(b): 4-(1-Propoxymethylcyclopropyl)benzaldehyde

Reaction and post-treatment were carried out in the similar manner as in Reference example 9-(c) except for

using 280 mg (1.04 mmol) of 1-bromo-4-(1-propoxymethylcyclopropyl)benzene obtained in 16-(a) in place of 1-bromo-4-(1-butylcyclopropyl)benzene, and using 0.78 ml (1.2 mmol) of 1.6M n-butyl lithium/n-hexane solution and 0.25 ml (3.2 mmol) of N,N-dimethylformamide to obtain 205 mg of the title compound as pale yellow oil. (Yield: 90%)
Mass Spectrum (CI, m/z): 219 ($M^+$+1).
$^1$H-NMR Spectrum ($CDCl_3$, δ ppm): 9.97 (s, 1H), 7.83-7.76 (m, 2H), 7.49-7.43 (m, 2H), 3.57 (s, 2H), 3.38 (t, J=6.7 Hz, 2H), 1.63-1.49 (m, 2H), 1.01-0.96 (m, 4H), 0.87 (t, J=7.3 Hz, 3H).

16-(c): <u>4-(1-Propoxymethylcyclopropyl)benzyl alcohol</u>

Reaction and post-treatment were carried out in the similar manner as in Reference example 7 except for using 201 mg (0.921 mmol) of 4-(1-propoxymethylcyclopropyl)benzaldehyde obtained in Reference example 16-(b) in place of 4-(1-ethylcyclopropyl)benzaldehyde, and using 21.5 mg (0.568 mmol) of potassium borohydride to obtain 130 mg of the title compound as colorless oily product. (Yield: 64%) Mass Spectrum (EI, m/z): 220 ($M^+$).
$^1$H-NMR Spectrum ($CDCl_3$, δ ppm): 7.36-7.31 (m, 2H), 7.31-7.25 (m, 2H), 4.66 (d, J=5.9 Hz, 2H), 3.52 (s, 2H), 3.36 (t, J=6.7 Hz, 2H), 1.61-1.47 (m, 2H), 0.90-0.82 (m, 4H), 0.86 (t, J=7.4 Hz, 3H).

[Reference example 17]

<u>4-(1-Benzylcyclopropyl)benzyl alcohol</u>

17-(a): [1-(4-Bromophenyl)cyclopropyl](phenyl)methanone

To 8 ml of a tetrahydrofuran solution containing 444 mg (2·00 mmol) of 1-(4-bromophenyl)cyclopropanecarbonitrile (see US2007/66820A) was added dropwise 1.2 ml (2.4 mmol) of 2M phenylmagnesium chloride/tetrahydrofuran solution under ice-cooling over 10 minutes. After completion of the dropwise addition, the mixture was stirred at the same temperature for 20 minutes, then, at room temperature for 30 minutes, and further refluxed for 5.5 hours. After completion of the reaction, the reaction mixture was poured into 32 ml of 2N hydrochloric acid, and extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=19:1 (V/V)), and the fractions containing the objective material were concentrated under reduced pressure to obtain 378 mg of the title compound as yellowish white solid. (Yield: 63%)
Mass Spectrum (EI, m/z): 300, 302 ($M^+$).
$^1$H-NMR Spectrum ($CDCl_3$, δ ppm): 7.76-7.70 (m, 2H), 7.45-7.26 (m, 5H), 7.10-7.03 (m, 2H), 1.71-1.65 (m, 2H), 1.36-1.29 (m, 2H).

17-(b): <u>1-Bromo-4-(1-benzylcyclopropyl)benzene</u>

To 2.1 ml of a diethylene glycol solution containing 316 mg (1.05 mmol) of [1-(4-bromophenyl)cyclopropyl](phenyl)methanone obtained in Reference example 17-(a) were added 158 mg (3.16 mmol) of hydrazine monohydrate and 177 mg (3.15 mmol) of potassium hydroxide, and the mixture was stirred at 200°C for 5 hours. After completion of the reaction, water was added to the reaction mixture, and the resulting mixture was extracted with diethyl ether. The organic layer was washed with a saturated aqueous sodium chloride solution, dried over anhydrous magnesium sulfate and concentrated under reduced pressure. The residue was applied to silica gel column chromatography (eluent; n-hexane), and the fractions containing the objective material were concentrated under reduced pressure to obtain 99.2 mg of the title compound as colorless oily product. (Yield: 33%)
Mass Spectrum (EI, m/z): 286, 288 ($M^+$).
$^1$H-NMR Spectrum ($CDCl_3$, δ ppm): 7.33-7.27 (m, 2H), 7.23-7.11 (m, 3H), 7.03-6.94 (m, 4H), 2.88 (s, 2H), 0.89-0.82 (m, 4H).

17-(c): <u>4-(1-Benzylcyclopropyl)benzaldehyde</u>

Reaction and post-treatment were carried out in the similar manner as in Reference example 9-(c) except for using 95.1 mg (0.331 mmol) of 1-bromo-4-(1-benzylcyclopropyl)benzene obtained in 17-(b) in place of 1-bromo-4-(1-butylcyclopropyl)benzene, and using 0.24 ml (0.40 mmol) of 1.65M n-butyl lithium/n-hexane solution and 77 μl (0.99 mmol) of N,N-dimethylformamide to obtain 68.7 mg of the title compound as colorless oily product. (Yield: 88%)
Mass Spectrum (CI, m/z): 237 ($M^+$+1).
$^1$H-NMR Spectrum ($CDCl_3$, δ ppm): 9.93 (s, 1H), 7.75-7.67 (m, 2H), 7.32-7.25 (m, 2H), 7.23-7.1 (m, 3H), 7.02-6.96 (m, 2H), 2.98 (s, 2H), 0.99-0.94 (m, 4H).

17-(d): 4-(1-Benzylcyclopropyl)benzyl alcohol

Reaction and post-treatment were carried out in the similar manner as in Reference example 7 except for using 63.4 mg (0.268 mmol) of 4-(1-benzylcyclopropyl)benzaldehyde obtained in Reference example 17-(c) in place of 4-(1-ethylcyclopropyl)benzaldehyde, and using 5.0 mg (0.13 mmol) of potassium borohydride to obtain 41.6 mg of the title compound as colorless oily product. (Yield: 65%)
Mass Spectrum (EI, m/z): 238 ($M^+$).
$^1$H-NMR Spectrum ($CDCl_3$, $\delta$ ppm): 7.23-7.12 (m, 7H), 7.03-6.97 (m, 2H), 4.63 (d, J=5.9 Hz, 2H), 2.93 (s, 2H), 0.86 (s, 4H).

[Reference example 18]

4-(1-Benzyloxymethylcyclopropyl)benzyl alcohol

18-(a): 1-Bromo-4-(1-benzyloxymethylcyclopropyl)benzene

To 3 ml of a N,N-dimethylformamide solution containing 50 mg (1.1 mmol) of sodium hydride (mineral oil 55% dispersed product) was added 200 mg (0.88 mmol) of [1-(4-bromophenyl)cyclopropyl]methanol (see WO2010/047982A) under ice-cooling, and the mixture was stirred at room temperature for 30 minutes. Then, 136 μl (1.15 mmol) of benzyl bromide was added to the mixture under ice-cooling, and the resulting mixture was stirred at room temperature for 3 hours. After completion of the reaction, the reaction mixture was poured into water and extracted with n-hexane. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=9:1 (V/V)), and the fractions containing the objective material were concentrated under reduced pressure to obtain 196 mg of the title compound as colorless oily product.
(Yield: 70%)
Mass Spectrum (EI, m/z): 316, 318 ($M^+$).
$^1$H-NMR Spectrum ($CDCl_3$, $\delta$ ppm): 7.43-7.18 (m, 9H), 4.48 (s, 2H), 3.52 (s, 2H), 0.93-0.83 (m, 4H).

18-(b): 4-(1-Benzyloxymethylcyclopropyl)benzaldehyde

Reaction and post-treatment were carried out in the similar manner as in Reference example 9-(c) except for using 0.19 g (0.60 mmol) of 1-bromo-4-(1-benzyloxymethylcyclopropyl)benzene obtained in 18-(a) in place of 1-bromo-4-(1-butylcyclopropyl)benzene, and using 0.47 ml (0.75 mmol) of 1.6M n-butyl lithium/n-hexane solution and 0.15 ml (1.9 mmol) of N,N-dimethylformamide to obtain 0.10 g of the title compound as colorless oily product. (Yield: 63%)
Mass Spectrum (CI, m/z): 267 ($M^++1$).
$^1$H-NMR Spectrum ($CDCl_3$, $\delta$ ppm): 9.98 (s, 1H), 7.83-7.77 (m, 2H), 7.49-7.44 (m, 2H), 7.36-7.21 (m, 5H), 4.51 (s, 2H), 3.60 (s, 2H), 1.01-0.96 (m, 4H).

18-(c): 4-(1-Benzyloxymethylcyclopropyl)benzyl alcohol

Reaction and post-treatment were carried out in the similar manner as in Reference example 7 except for using 0.10 g (0.38 mmol) of 4-(1-benzyloxymethylcyclopropyl)benzaldehyde obtained in Reference example 18-(b) in place of 4-(1-ethylcyclopropyl)benzaldehyde, and using 11 mg (0.29 mmol) of potassium borohydride to obtain 76.3 mg of the title compound as colorless oily product. (Yield: 75%)
Mass Spectrum (EI, m/z): 268 ($M^+$).
$^1$H-NMR Spectrum ($CDCl_3$, $\delta$ ppm): 7.39-7.20 (m, 9H), 4.67 (d, J=5.9 Hz, 2H), 4.49 (s, 2H), 3.56 (s, 2H), 1.57 (t, J=5.9 Hz, 1H), 0.93-0.83 (m, 4H).

[Reference example 19]

N-(4-tert-butylbenzyl)benzenesulfonamide

To 3.3 ml of a methylene chloride solution containing 166 mg (1.02 mmol) of 4-tert-butylbenzylamine were added 103 mg (1.26 mmol) of triethylamine and 200 mg (1.13 mmol) of benzenesulfonyl chloride, and the mixture was stirred at room temperature for 1 hour. After completion of the reaction, the reaction mixture was concentrated under reduced pressure. The obtained residue was applied to silica gel column chromatography (eluent; n-hexane:ethyl acetate=4:1→3:2 (V/V)), and the fractions containing the objective material were concentrated under reduced pressure to obtain 317 mg of the title compound as white solid. (Yield: 92%)

Mass Spectrum (CI, m/z): 304 ($M^{+}$+1).
$^{1}$H-NMR Spectrum ($CDCl_3$, δ ppm): 7.90-7.84 (m, 2H), 7.62-7.46 (m, 3H), 7.32-7.26 (m, 2H), 7.14-7.08 (m, 2H), 4.64 (t, J=6.0 Hz, 0.9H), 4.12 (d, J=6.0 Hz, 2H), 1.28 (s, 9H).

[Reference example 20]

N-(3-tert-butylbenzyl)pyridin-3-ylsulfonamide

To 2.5 ml of a methylene chloride solution containing 137 mg (0.845 mmol) of 3-tert-butylbenzylamine (see WO2003/080578A) were added 116 mg (1.15 mmol) of triethylamine and 150 mg (0.845 mmol) of 3-pyridylsulfonyl chloride, and the mixture was stirred at room temperature for 2 hours. After completion of the reaction, the reaction mixture was applied to silica gel column chromatography (eluent; n-hexane: ethyl acetate=2:3 (V/V)), and the fractions containing the objective material were concentrated under reduced pressure to obtain 207 mg of the title compound as pale yellow oil substantially quantitatively.
Mass Spectrum (CI, m/z): 305 ($M^{+}$+1).
$^{1}$H-NMR Spectrum ($CDCl_3$, δ ppm): 9.04 (dd, J=2.3, 0.9 Hz, 1H), 8.76 (dd, J=4.9, 1.6 Hz, 1H), 8.08 (ddd, J=8.1, 2.3, 1.6 Hz, 1H), 7.40 (ddd, J=8.1, 4.9, 0.9 Hz, 1H), 7.31-7.26 (m, 1H), 7.24-7.15 (m, 2H), 7.02-6.97 (m, 1H), 5.13 (t, J=5.8 Hz, 1H), 4.22 (d, J=5.8 Hz, 2H), 1.26 (s, 9H).

[Reference example 21]

N-(4-tert-butylbenzyl)pyridin-2-ylsulfonamide

To 5.0 ml of a methylene chloride solution containing 292 mg (1.79 mmol) of 4-tert-butylbenzylamine were added 241 mg (2.38 mmol) of triethylamine and 312 mg (1.76 mmol) of 2-pyridylsulfonyl chloride, and the mixture was stirred at room temperature for 1 hour. After completion of the reaction, the reaction mixture was applied to silica gel column chromatography (eluent; n-hexane:ethyl acetate=1:1 (V/V)), and the fractions containing the objective material were concentrated under reduced pressure to obtain 477 mg of the title compound as white solid. (Yield: 89%)
Mass Spectrum (CI, m/z): 305 ($M^{+}$+1).
$^{1}$H-NMR Spectrum ($CDCl_3$, δ ppm): 8.64 (ddd, J=4.6, 1.7, 1.0 Hz, 1H), 7.97 (ddd, J=7.8, 1.2, 1.0 Hz, 1H), 7.86 (ddd, J=7.8, 7.6, 1.7 Hz, 1H), 7.45 (ddd, J=7.6, 4.6, 1.2 Hz, 1H), 7.31-7.25 (m, 2H), 7.19-7.13 (m, 2H), 5.31 (t, J=6.0 Hz, 0.9H), 4.23 (d, J=6.0 Hz, 2H), 1.28 (s, 9H).

[Reference example 22]

N-(4-bromobenzyl)pyridin-2-ylsulfonamide

To 20 ml of a methylene chloride solution containing 1.13 g (5.08 mmol) of 4-bromobenzylamine hydrochloride was added 0.904 g (5.09 mmol) of 2-pyridylsulfonyl chloride, then, under ice-cooling, 1.8 ml (13 mmol) of triethylamine was added dropwise to the mixture, and the resulting mixture was stirred at room temperature for 1 hour. After completion of the reaction, the reaction mixture was concentrated under reduced pressure. Water was added to the residue, and the mixture was extracted with ethyl acetate. The organic layer was washed with water and then with a saturated aqueous sodium chloride solution, dried over anhydrous magnesium sulfate, and concentrated under reduced pressure. The obtained residue was applied to silica gel column chromatography (n-hexane:ethyl acetate=2:3 (V/V)), and the fractions containing the objective material were concentrated under reduced pressure to obtain 1.51 g of the title compound as white solid. (Yield: 91%)
Mass Spectrum (CI, m/z): 327 ($M^{+}$+1).
$^{1}$H-NMR Spectrum (DMSO-$d_6$, δ ppm): 8.70 (ddd, J=4.7, 1.6, 0.9 Hz, 1H), 8.46 (s, 0.8H), 8.04 (ddd, J=7.7, 7.6, 1.6 Hz, 1H), 7.91-7.87 (m, 1H), 7.64 (ddd, J=7.6, 4.7, 1.0 Hz, 1H), 7.49-7.43 (m, 2H), 7.23-7.18 (m, 2H), 4.14 (s, 2H).

[Reference example 23]

4-(1-Ethyl-1-methylpropyl)benzyl alcohol

23-(a): 1-Bromo-4-(1-ethyl-1-formylpropyl)benzene

To 35 ml of a toluene solution containing 1.18 g (4.68 mmol) of 1-bromo-4-(1-cyano-1-ethylpropyl)benzene (see WO2005/035503A) was added dropwise 9.4 ml (9.4 mmol) of 1.0M diisobutyl aluminum hydride/toluene solution

at -60°C over 6 minutes, and the mixture was stirred at the same temperature for 40 minutes. Then, the temperature of the mixture was gradually raised to room temperature over 3 hours. After completion of the reaction, 0.3 ml of methanol was added to the reaction mixture, and the resulting mixture was poured into a saturated aqueous ammonium chloride solution. Then, 5% aqueous sulfuric acid solution was added to the mixture and the liquids were separated. The organic layer was washed successively with a 5% aqueous sulfuric acid solution and a saturated aqueous sodium chloride solution, dried over anhydrous magnesium sulfate and concentrated under reduced pressure. The obtained residue was applied to silica gel column chromatography (eluent; n-hexane:ethyl acetate=1:0→ 19:1 (V/V)), and the fractions containing the objective material were concentrated under reduced pressure to obtain 724 mg (purity: 50%; pure content: 362 mg) of the title compound as colorless oily product. (Yield: 30%)
Mass Spectrum (CI, m/z): 255, 257 ($M^{+}$+1).
$^{1}$H-NMR Spectrum ($CDCl_3$, δ ppm): 9.46 (s, 1H), 7.55-7.47 (m, 2H), 7.14-7.07 (m, 2H), 2.11-1.80 (m, 4H), 0.75 (t, J=7.4 Hz, 6H).

23-(b): 1-Bromo-4-(1-ethyl-1-methylpropyl)benzene

To 5.7 ml of a diethylene glycol solution containing 724 mg (pure content: 1.42 mmol) of 1-bromo-4-(1-ethyl-1-formylpropyl)benzene obtained in Reference example 23-(a) were added 0.40 ml (8.3 mmol) of hydrazine monohydrate and 470 mg (pure content: 7.1 mmol) of potassium hydroxide, and the mixture was stirred at 200°C for 1.5 hours. After completion of the reaction, to the reaction mixture were added water and a saturated aqueous sodium chloride solution, and extracted with diethyl ether. The organic layer was washed with a saturated aqueous sodium chloride solution, dried over anhydrous magnesium sulfate and concentrated under reduced pressure. The obtained residue was applied to silica gel column chromatography (eluent; n-hexane), and the fractions containing the objective material were concentrated under reduced pressure to obtain 361 mg of the title compound as colorless oily product substantially quantitatively.
Mass Spectrum (EI, m/z): 240, 242 ($M^{+}$).
$^{1}$H-NMR Spectrum ($CDCl_3$, δ ppm): 7.44-7.37 (m, 2H), 7.17-7.11 (m, 2H), 1.70 (dq, J=14.3, 7.3 Hz, 2H), 1.53 (dq, J=14.3, 7.3 Hz, 2H), 1.21 (s, 3H), 0.65 (t, J=7.3 Hz, 6H).

23-(c): 4-(1-Ethyl-1-methylpropyl)benzaldehyde

To 4.2 ml of a tetrahydrofuran solution containing 421 mg (1.75 mmol) of 1-bromo-4-(1-ethyl-1-methylpropyl)benzene obtained in the same manner as in Reference example 23-(b) was added dropwise 1.35 ml (2.2 mmol) of 1.6M n-butyl lithium/n-hexane solution at -60°C over 8 minutes, and the mixture was stirred at the same temperature for 1 hour. Then, 0.41 ml (5.3 mmol) of N,N-dimethylformamide was added dropwise to the mixture over 5 minutes, and the temperature of the mixture was gradually raised to room temperature over 15 hours. After completion of the reaction, to the reaction mixture were added a saturated aqueous ammonium chloride solution and water, and extracted with diethyl ether. The organic layer was washed with a saturated aqueous sodium chloride solution, dried over anhydrous magnesium sulfate and concentrated under reduced pressure. The obtained residue was applied to silica gel column chromatography (eluent; n-hexane:ethyl acetate=1:0→93:7 (V/V)), and the fractions containing the objective material were concentrated under reduced pressure to obtain 151 mg of the title compound as yellow oil. (Yield: 45%)
Mass Spectrum (CI, m/z): 191 ($M^{+}$+1).
$^{1}$H-NMR Spectrum ($CDCl_3$, δ ppm): 9.99 (s, 1H), 7.85-7.79 (m, 2H), 7.48-7.42 (m, 2H), 1.78 (dq, J=14.3, 7.3 Hz, 2H), 1.60 (dq, J=14.3, 7.3 Hz, 2H), 1.29 (s, 3H), 0.66 (t, J=7.3 Hz, 6H).

23-(d): 4-(1-Ethyl-1-methylpropyl)benzyl alcohol

To 0.8 ml of an ethanol solution containing 150 mg (0.788 mmol) of 4-(1-ethyl-1-methylpropyl)benzaldehyde obtained in Reference example 23-(c) was added 14.9 mg (0.394 mmol) of potassium borohydride, and the mixture was stirred at room temperature for 3 hours. After completion of the reaction, to the reaction mixture were added water and a saturated aqueous sodium chloride solution, and the resulting mixture was extracted with ethyl acetate. The organic layer was washed with a saturated aqueous sodium chloride solution, dried over anhydrous magnesium sulfate and concentrated under reduced pressure. The obtained residue was applied to silica gel column chromatography (eluent; n-hexane:ethyl acetate=1:0→9:1 (V/V)), and the fractions containing the objective material were concentrated under reduced pressure to obtain 138 mg of the title compound as colorless oily product. (Yield: 91%)
Mass Spectrum (EI, m/z): 192 ($M^{+}$).
$^{1}$H-NMR Spectrum ($CDCl_3$, δ ppm): 7.33-7.24 (m, 4H), 4.67 (d, J=5.9 Hz, 2H), 1.73 (dq, J=14.4, 7.4 Hz, 2H), 1.63-1.49 (m, 3H), 1.24 (s, 3H), 0.66 (t, J=7.4 Hz, 6H).

[Reference example 24]

4-(1-Ethylpropyl)benzyl alcohol

24-(a): 4-(1-Ethylpropyl)benzaldehyde

To 23 ml of a tetrahydrofuran solution containing 2.63 g (11.6 mmol) of 1-bromo-4-(1-ethylpropyl)benzene (see WO2008/033455A) was added dropwise 7.6 ml (12 mmol) of 1.6M n-butyl lithium/n-hexane solution at -78°C over 7 minutes, and the mixture was stirred at the same temperature for 30 minutes. Then, 2.7 ml (35 mmol) of N,N-dimethylformamide was added dropwise to the mixture at -78°C over 5 minutes, and the mixture was stirred under ice-cooling for 16 hours. After completion of the reaction, to the reaction mixture were added a saturated aqueous ammonium chloride solution and water, and the resulting mixture was extracted with diethyl ether. The organic layer was washed with a saturated aqueous sodium chloride solution, dried over anhydrous sodium sulfate and concentrated under reduced pressure. The obtained residue was applied to silica gel column chromatography (eluent; n-hexane:ethyl acetate=1:0→10:1 (V/V)), and the fractions containing the objective material were concentrated under reduced pressure to obtain 1.44 g of the title compound as pale yellow oil. (Yield: 70%)
Mass Spectrum (EI, m/z): 176 ($M^+$).
$^1$H-NMR Spectrum ($CDCl_3$, $\delta$ ppm): 9.98 (s, 1H), 7.85-7.78 (m, 2H), 7.34-7.28 (m, 2H), 2.49-2.36 (m, 1H), 1.82-1.49 (m, 4H), 0.77 (t, J=7.3 Hz, 6H).

24-(b): 4-(1-Ethylpropyl)benzyl alcohol

To 8.0 ml of an ethanol solution containing 1.43 g (8.11 mmol) of 4-(1-ethylpropyl)benzaldehyde obtained in Reference example 24-(a) was added 155 mg (4.10 mmol) of potassium borohydride under ice-cooling, and the mixture was stirred at room temperature for 3 hours. After completion of the reaction, to the reaction mixture were added water and saturated aqueous sodium chloride solution, and the resulting mixture was extracted with ethyl acetate. The organic layer was washed with a saturated aqueous sodium chloride solution, dried over anhydrous sodium sulfate and concentrated under reduced pressure. The obtained residue was applied to silica gel column chromatography (eluent; n-hexane:ethyl acetate=1:0→5:1 (V/V)), and the fractions containing the objective material were concentrated under reduced pressure to obtain 1.24 g of the title compound as colorless oily product. (Yield: 86%)
Mass Spectrum (EI, m/z): 178 ($M^+$).
$^1$H-NMR Spectrum ($CDCl_3$, $\delta$ ppm): 7.32-7.26 (m, 2H), 7.17-7.10 (m, 2H), 4.66 (d, J=5.9 Hz, 2H), 2.38-2.25 (m, 1H), 1.77-1.45 (m, 5H), 0.76 (t, J=7.3 Hz, 6H).

[Reference example 25]

tert-Butyl (tert-butoxycarbonyl{6-[(4-tert-butylbenzyl)aminomethyl]pyridin-2-yl}-amino)acetate

To 50 ml of a methylene chloride solution containing 1.51 g (9.25 mmol) of 4-tert-butylbenzylamine were added 1.50 g (4.46 mmol) of tert-butyl [tert-butoxycarbonyl(6-formylpyridin-2-yl)amino]acetate obtained in Reference example 1-(c) and 5.20 g of Molecular Sieve (4A), and the mixture was stirred at room temperature for 1 hour. Then, 2.56 g (12.1 mmol) of triacetoxy potassium borohydride was added to the mixture by dividing into several portions at room temperature, and the mixture was stirred at the same temperature for 2 hours. After completion of the reaction, to the reaction mixture was added a saturated aqueous sodium hydrogen carbonate solution and ethyl acetate, and insoluble materials were filtered off. The organic layer of the filtrate was washed with a saturated aqueous sodium chloride solution, dried over anhydrous sodium sulfate and concentrated under reduced pressure. The obtained residue was applied to silica gel column chromatography (eluent; n-hexane:ethyl acetate:triethylamine=6:4:0.1 (V/V/V)), and the fractions containing the objective material were concentrated under reduced pressure to obtain 2.23 g of the title compound as colorless oily productsubstantially quantitatively.
$^1$H-NMR Spectrum ($CDCl_3$ $\delta$ ppm): 7.68 (d, J=8.3 Hz, 1H), 7.58 (dd, J=8.3, 7.3 Hz, 1H), 7.38-7.25 (m, 4H), 6.97 (dd, J=7.3, 0.7 Hz, 1H), 4.56 (s, 2H), 3.81 (s, 2H), 3.78 (s, 2H), 1.52 (s, 9H), 1.41 (s, 9H), 1.31 (s, 9H).

[Reference example 26]

tert-Butyl [(5-bromo-6-bromomethylpyridin-2-yl)tert-butoxycarbonylamino]acetate

26-(a): tert-Butyl [tert-butoxycarbonyl(6-methylpyridin-2-yl)amino]acetate

To 12 ml of a N,N-dimethylformamide solution containing 723 mg (3.47 mmol) of 2-(tert-butoxycarbonylamino)-6-methylpyridine was added 0.18 g (4.2 mmol) of sodium hydride (mineral oil 55% dispersed product) under ice-cooling by dividing into several portions. The mixture was stirred at room temperature for 30 minutes, then 0.62 ml (4.2 mmol) of tert-butyl bromoacetate was added dropwise to the mixture under ice-cooling, and the resulting mixture was stirred at room temperature for 2 hours. After completion of the reaction, water was added to the reaction mixture and the resulting mixture was extracted with ethyl acetate. The organic layer was washed with a saturated aqueous sodium chloride solution, dried over anhydrous sodium sulfate and concentrated under reduced pressure. The obtained residue was applied to silica gel column chromatography (eluent; n-hexane:ethyl acetate=10:1→5:1 (V/V)), and the fractions containing the objective material were concentrated under reduced pressure to obtain 1.14 g of the title compound as colorless oily product substantially quantitatively. Mass Spectrum (EI, m/z): 322 ($M^+$).
$^1$H-NMR Spectrum ($CDCl_3$, $\delta$ ppm): 7.58 (d, J=8.2 Hz, 1H), 7.51 (dd, J=8.2, 7.1 Hz, 1H), 6.86-6.81 (m, 1H), 4.56 (s, 2H), 2.43 (s, 3H), 1.51 (s, 9H), 1.45 (s, 9H).

26-(b): tert-Butyl [(5-bromo-6-methylpyridin-2-yl)tert-butoxycarbonylamino]acetate

To 3.0 ml of an acetonitrile solution containing 477 mg (1.48 mmol) of tert-butyl [tert-butoxycarbonyl(6-methylpyridin-2-yl)amino] acetate obtained in Reference example 26-(a) was added 398 mg (2.24 mmol) of NBS, and the mixture was stirred at 40°C for 3 hours. After completion of the reaction, the reaction mixture was concentrated under reduced pressure. The obtained residue was applied to silica gel column chromatography (eluent; n-hexane:ethyl acetate=20:1→5:1 (V/V)), and the fractions containing the objective material were concentrated under reduced pressure to obtain 565 mg of the title compound as white solid. (Yield: 95%)
Mass Spectrum (EI, m/z): 400, 402 ($M^+$).
$^1$H-NMR Spectrum ($CDCl_3$, $\delta$ ppm): 7.69 (d, J=8.9 Hz, 1H), 7.56 (d, J=8.9 Hz, 1H), 4.54 (s, 2H), 2.52 (s, 3H), 1.52 (s, 9H), 1.45 (s, 9H).

26-(c): tert-Butyl [(5-bromo-6-bromomethylpyridin-2-yl)tert-butoxycarbonylamino]-acetate

To 4.7 ml of a 1,2-dichloroethane solution containing 560 mg (1.40 mmol) of tert-butyl [(5-bromo-6-methylpyridin-2-yl)tert-butoxycarbonylamino]acetate obtained in Reference example 26-(b) were added 373 mg (2.10 mmol) of NBS and 10 mg (0.052 mmol) of 2,2'-azobis(2-methylbutyronitrile), and the mixture was stirred at 90°C for 1 hour. After completion of the reaction, the reaction mixture was concentrated under reduced pressure. The obtained residue was applied to silica gel column chromatography (eluent; n-hexane: ethyl acetate=20:1→10:1 (V/V)), and the fractions containing the objective material were concentrated under reduced pressure to obtain 355 mg of a mixture containing the title compound as pale yellow oil. (Yield: 38%)
Mass Spectrum (EI, m/z): 480 ($M^+$).
$^1$H-NMR Spectrum ($CDCl_3$, $\delta$ ppm): 7.75 (s, 2H), 4.58 (s, 2H), 4.56 (s, 2H), 1.52 (s, 9H), 1.47 (s, 9H).

[Reference example 27]

N-(4-tert-butylbenzyl)pyridin-3-ylsulfonamide

To 30 ml of a methylene chloride solution containing 1.56 g (8.78 mmol) of 3-pyridylsulfonyl chloride were added 1.75 ml (9.64 mmol) of 4-tert-butylbenzylamine and 2.45 ml (17.6 mmol) of triethylamine, and the mixture was stirred at room temperature for 16 hours. After completion of the reaction, water was added to the reaction mixture, and the resulting mixture was extracted with methylene chloride and with ethyl acetate. The organic layer was dried over anhydrous magnesium sulfate and concentrated under reduced pressure. The obtained residue was applied to silica gel column chromatography (eluent; n-hexane:ethyl acetate=2:1→1:2 (V/V)), and the fractions containing the objective material were concentrated under reduced pressure to obtain 2.44 g of the title compound as white solid. (Yield: 91 %)
Mass Spectrum (CI, m/z): 305 ($M^+$+1).
$^1$H-NMR Spectrum ($CDCl_3$, $\delta$ ppm): 9.06 (dd, J=2.3, 0.7 Hz, 1H), 8.77 (dd, J=4.9, 1.7 Hz, 1H), 8.07 (ddd, J=8.0, 2.3, 1.7 Hz, 1H), 7.40 (ddd, J=8.0, 4.9, 0.7 Hz, 1H), 7.32-7.26 (m, 2H), 7.14-7.07 (m, 2H), 4.85 (t, J=6.0 Hz, 0.9H), 4.19 (d, J=6.0 Hz, 2H), 1.28 (s, 9H).

[Reference example 28]

N-(4-difluoromethoxybenzyl)pyridin-3-ylsulfonamide

To 4.0 ml of a methylene chloride solution containing 200 mg (1.16 mmol) of 4-difluoromethoxybenzylamine were added 226 mg (1.27 mmol) of 3-pyridylsulfonyl chloride and 175 mg (1.73 mmol) of triethylamine, and the mixture was stirred at room temperature for 2 hours. After completion of the reaction, the reaction mixture was applied to silica gel column chromatography (eluent; n-hexane:ethyl acetate=1:1 (V/V)), and the fractions containing the objective material were concentrated under reduced pressure to obtain 333 mg of the title compound as pale yellow solid. (Yield: 91%)
Mass Spectrum (CI, m/z): 315 ($M^+$+1).
$^1$H-NMR Spectrum ($CDCl_3$, $\delta$ ppm): 9.04 (dd, J=2.3, 0.8 Hz, 1H), 8.78 (dd, J=4.9, 1.6 Hz, 1H), 8.07 (ddd, J=8.1, 2.3, 1.6 Hz, 1H), 7.42 (ddd, J=8.1, 4.9, 0.8 Hz, 1H), 7.24-7.17 (m, 2H), 7.06-7.00 (m, 2H), 6.48 (t, J=73.6 Hz, 1H), 5.07 (brs, 0.8H), 4.21 (s, 2H).

[Reference example 29]

4-(1,1-Dimethylpentyl)benzyl alcohol

29-(a): 4-(1,1-Dimethylpentyl)benzaldehyde

To 2 ml of a tetrahydrofuran solution containing 230 mg (0.901 mmol) of 1-bromo-4-(1,1-dimethylpentyl)benzene (see Synthesis, 4, 547 (2005)) was added dropwise 0.68 ml (1.1 mmol) of 1.6M n-butyl lithium/n-hexane solution at -70°C over 2 minutes, and the mixture was stirred at the same temperature for 30 minutes. Then, 0.21 ml (2.7 mmol) of N,N-dimethylformamide was added dropwise to the mixture at -70°C over 2 minutes, and the temperature of the mixture was gradually raised to room temperature over 2 hours. After completion of the reaction, to the reaction mixture were added a saturated aqueous ammonium chloride solution and water, and the resulting mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=30:1 (V/V)), and the fractions containing the objective material were concentrated under reduced pressure to obtain 122 mg of the title compound as pale yellow oil. (Yield: 66%)
Mass Spectrum (CI, m/z): 205 ($M^+$+1).
$^1$H-NMR Spectrum ($CDCl_3$, $\delta$ ppm): 9.99 (s, 1H), 8.01-7.82 (m, 2H), 7.82-7.63 (m, 2H), 2.20-2.10 (m, 2H), 1.33 (s, 6H), 1.35-1.21 (m, 2H), 1.10-0.95 (m, 2H), 0.82 (t, J=7.2 Hz, 3H).

29-(b): 4-(1,1-Dimethylpentyl)benzyl alcohol

Reaction and post-treatment were carried out in the similar manner as in Reference example 23-(d) except for using 118 mg (0.578 mmol) of 4-(1,1-dimethylpentyl)benzaldehyde obtained in Reference example 29-(a) in place of 4-(1-ethyl-1-methylpropyl)benzaldehyde, and using 10.9 mg (0.288 mmol) of potassium borohydride to obtain 113 mg of the title compound as colorless oily product. (Yield: 95%)
Mass Spectrum (EI, m/z): 206 ($M^+$).
$^1$H-NMR Spectrum ($CDCl_3$, $\delta$ ppm): 7.36-7.28 (m, 4H), 4.67 (s, 2H), 1.63-1.53 (m, 2H), 1.29 (s, 6H), 1.20 (t, J=7.2 Hz, 2H), 1.09-0.96 (m, 2H), 0.82 (t, J=7.3 Hz, 3H).

[Reference example 30]

4-(1,1,3,3-Tetramethylbutyl)benzyl alcohol

Reaction and post-treatment were carried out in the similar manner as in Reference example 23-(d) except for using 179 mg (0.820 mmol) of 4-(1,1,3,3-tetramethylbutyl)benzaldehyde (see JP54046735A) in place of 4-(1-ethyl-1-methylpropyl)benzaldehyde, and using 15.5 mg (0.410 mmol) of potassium borohydride to obtain 169 mg of the title compound as pale yellow oil. (Yield: 94%)
Mass Spectrum (EI, m/z): 220 ($M^+$).
$^1$H-NMR Spectrum ($CDCl_3$, $\delta$ ppm): 7.39-7.36 (m, 2H), 7.29-7.26 (m, 2H), 4.67 (s, 2H), 1.74 (s, 2H), 1.37 (s, 6H), 0.71 (s, 9H).

[Reference example 31]

4-(1-Methylpentyl)benzyl alcohol

31-(a): 4-Hydroxymethyl-N-methoxy-N-methylbenzamide

To 63 ml of a tetrahydrofuran solution containing 3.08 g (31.6 mmol) of N,O-dimethylhydroxylamine hydrochloride, 4.83 ml (34.7 mmol) of triethylamine and 4.43 g (23.1 mmol) of EDC was added dropwise 17 ml of a tetrahydrofuran solution containing 3.19 g (21.0 mmol) of 4-hydroxymethylbenzoic acid under ice-cooling, the mixture was stirred at the same temperature for 15 minutes, and the temperature of the mixture was gradually raised to room temperature. After completion of the reaction, insoluble material was filtered off and the filtrate was concentrated under reduced pressure. To the residue was added 100 ml of water, and the mixture was adjusted to pH 7.5 with 1N hydrochloric acid and extracted with ethyl acetate. The organic layer was washed with a saturated aqueous sodium chloride solution, dried over anhydrous magnesium sulfate and concentrated under reduced pressure. The residue was applied to silica gel column chromatography (eluent; toluene:ethyl acetate=1:4 (V/V)), and the fractions containing the objective material were concentrated under reduced pressure to obtain 1.72 g of the title compound as pale yellow oil. (Yield: 42%)
Mass Spectrum (CI, m/z): 196 ($M^+$+1).
$^1$H-NMR Spectrum ($CDCl_3$, δ ppm): 7.71-7.65 (m, 2H), 7.43-7.37 (m, 2H), 4.75 (d, J=5.6 Hz, 2H), 3.55 (s, 3H), 3.36 (s, 3H).

31-(b): N-methoxy-4-(4-methoxybenzyloxymethyl)-N-methylbenzamide

To 5.0 ml of a N,N-dimethylformamide solution containing 1.38 g (7.00 mmol) of 4-hydroxymethyl-N-methoxy-N-methylbenzamide obtained in Reference example 31-(a) was added 336 mg (7.7 mmol) of sodium hydride (mineral oil 55% dispersed product) under ice-cooling, and the mixture was stirred at the same temperature for 30 minutes. Then, 1.10 ml (7.63 mmol) of 4-methoxybenzyl bromide was added dropwise to the mixture under ice-cooling, and the temperature of the mixture was gradually raised to room temperature. After completion of the reaction, to the reaction mixture was added a saturated aqueous ammonium chloride solution, and the resulting mixture was extracted with toluene. The organic layer was washed with a saturated aqueous sodium chloride solution, dried over anhydrous magnesium sulfate and concentrated under reduced pressure. The residue was applied to silica gel column chromatography (eluent; n-hexane: ethyl acetate=2:1→1:1 (V/V)), and the fractions containing the objective material were concentrated under reduced pressure to obtain 1.70 g of the title compound as colorless oily product. (Yield: 77%)
Mass Spectrum (CI, m/z): 316 ($M^+$+1).
$^1$H-NMR Spectrum ($CDCl_3$, δ ppm): 7.71-7.65 (m, 2H), 7.43-7.37 (m, 2H), 7.32-7.26 (m, 2H), 6.93-6.86 (m, 2H), 4.56 (s, 2H), 4.51 (s, 2H), 3.81 (s, 3H), 3.55 (s, 3H), 3.36 (s, 3H).

31-(c): 1-[4-(4-Methoxybenzyloxymethyl)phenyl]pentan-1-one

To 1.0 ml of a tetrahydrofuran solution containing 152 mg (6.25 mmol) of magnesium was added dropwise 5.0 ml of a tetrahydrofuran solution containing 700 μl (6.54 mmol) of 1-bromobutane to prepare a Grignard reagent. To 10 ml of a tetrahydrofuran solution containing 1.58 g (5.01 mmol) of N-methoxy-4-(4-methoxybenzyl-oxymethyl)-N-methylbenzamide obtained in Reference example 31-(b) was added dropwise 6.6 ml of the prepared Grignard reagent under ice-cooling, and the temperature of the mixture was gradually raised to room temperature. After completion of the reaction, to the reaction mixture were added a saturated aqueous ammonium chloride solution and water, and the resulting mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane: ethyl acetate=2:1→ 0:1 (V/V)), and the fractions containing the objective material were concentrated under reduced pressure to obtain 1.40 g of the title compound as colorless oily product. (Yield: 89%)
Mass Spectrum (CI, m/z): 313 ($M^+$+1).
$^1$H-NMR Spectrum ($CDCl_3$, δ ppm): 7.98-7.91 (m, 2H), 7.48-7.41 (m, 2H), 7.33-7.27 (m, 2H), 6.94-6.86 (m, 2H), 4.58 (s, 2H), 4.52 (s, 2H), 3.81 (s, 3H), 2.96 (t, J=7.4 Hz, 2H), 1.79-1.65 (m, 2H), 1.49-1.34 (m, 2H), 0.95 (t, J=7.3 Hz, 3H).

31-(d): 1-(Hexan-1-en-2-yl)-4-(4-methoxybenzyloxymethyl)benzene

To 16.6 ml of a tetrahydrofuran solution containing 2.19 g (purity: 98%:6.0 mmol) of methyltriphenylphosphonium bromide was added dropwise 3.17 ml (5.04 mmol) of 1.59M n-butyl lithium/n-hexane solution at -70°C, and the temperature of the mixture was gradually raised to room temperature. Then, to the mixture was added dropwise 4.8 ml of a tetrahydrofuran solution containing 1.25 g (4.00 mmol) of 1-[4-(4-methoxybenzyloxymethyl)phenyl]pentan-1-one obtained in Reference example 31-(c) at room temperature, and the mixture was stirred at the same temperature for 3 hours. After completion of the reaction, to the reaction mixture were added a saturated aqueous ammonium chloride solution and water, and the resulting mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=8:1→6:1 (V/V)), and the fractions containing the objective material were concentrated under reduced pressure to obtain 1.14 g of the title compound as pale yellow oil. (Yield: 92%)
Mass Spectrum (CI, m/z): 311 ($M^{+}$+1).
$^{1}$H-NMR Spectrum ($CDCl_3$, δ ppm): 7.42-7.36 (m, 2H), 7.33-7.27 (m, 4H), 6.92-6.86 (m, 2H), 5.26-5.24 (m, 1H), 5.05-5.03 (m, 1H), 4.52 (s, 2H), 4.50 (s, 2H), 3.81 (s, 3H), 2.53-2.46 (m, 2H), 1.49-1.25 (m, 4H), 0.89 (t, J=7.2 Hz, 3H).

31-(e): 1-(4-methoxybenzyloxymethyl)-4-(1-methylpentyl)benzene

To 5.5 ml of an ethanol solution containing 497 mg (1.60 mmol) of 1-(hexan-1-en-2-yl)-4-(4-methoxybenzyloxymethyl)benzene obtained in Reference example 31-(d) was added 49.8 mg (0.219 mmol) of platinum oxide, and under hydrogen atmosphere at 1 atm, the mixture was stirred at room temperature for 6 hours. After completion of the reaction, insoluble material was filtered off and the filtrate was concentrated under reduced pressure. The residue was applied to silica gel column chromatography (eluent; n-hexane:ethyl acetate=9:1→4:1 (V/V)), and the fractions containing the objective material were concentrated under reduced pressure to obtain 432 mg of the title compound as colorless oily product. (Yield: 86%)
Mass Spectrum (EI, m/z): 312 ($M^{+}$).
$^{1}$H-NMR Spectrum ($CDCl_3$, δ ppm): 7.32-7.24 (m, 4H), 7.18-7.13 (m, 2H), 6.92-6.85 (m, 2H), 4.49 (s, 4H), 3.81 (s, 3H), 2.74-2.59 (m, 1H), 1.63-1.48 (m, 2H), 1.36-1.07 (m, 4H), 1.22 (d, J=6.8 Hz, 3H), 0.84 (t, J=7.0 Hz, 3H).

31-(f): 4-(1-Methylpentyl)benzyl alcohol

To 6.5 ml of a methylene chloride solution containing 427 mg (1.37 mmol) of 1-(4-methoxybenzyloxymethyl)-4-(1-methylpentyl)benzene obtained in Reference example 31-(e) were added 467 mg (0.206 mmol) of 2,3-dichloro-5,6-dicyano-p-benzoquinone (DDQ) and 650 μl of water, and the mixture was stirred at room temperature for 16 hours. After completion of the reaction, to the reaction mixture were added a saturated aqueous sodium hydrogen carbonate solution and water, and the resulting mixture was extracted with tert-butyl methyl ether. The organic layer was washed with a saturated aqueous sodium chloride solution, dried over anhydrous magnesium sulfate and concentrated under reduced pressure. The residue was applied to silica gel column chromatography (eluent; toluene:ethyl acetate=6:1 (V/V)), and the fractions containing the objective material were concentrated under reduced pressure to obtain 211 mg of the title compound as pale yellow oil. (Yield: 80%)
Mass Spectrum (EI, m/z): 192 ($M^{+}$).
$^{1}$H-NMR Spectrum ($CDCl_3$, δ ppm): 7.33-7.26 (m, 2H), 7.21-7.15 (m, 2H), 4.66 (d, J=5.9 Hz, 2H), 2.75-2.59 (m, 1H), 1.61-1.51 (m, 2H), 1.35-1.07 (m, 4H), 1.22 (d, J=7.0 Hz, 3H), 0.85 (t, J=7.0 Hz, 3H).

[Reference example 32]

3-Butylbenzyl alcohol

32-(a): Ethyl_3-butylbenzoate

To 2.0 ml of a tetrahydrofuran solution containing 20.0 mg (0.0891 mmol) of palladium acetate and 84.0 mg (0.204 mmol) of 2-dicyclohexylphosphino-2',6'-dimethoxybiphenyl were added 20 ml of a toluene solution containing 1.88 g (6.81 mmol) of ethyl 3-iodobenzoate, as well as 2.16 g (21.2 mmol) of butylboric acid and 6.76 g (31.8 mmol) of potassium phosphate. After degassing under reduced pressure, atmosphere was replaced with nitrogen, and the mixture was refluxed for 2 hours. After completion of the reaction, water was added to the reaction mixture and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=19:1 (V/V)), and the fractions containing the objective material were concentrated under reduced pressure to obtain 1.27 g of the title compound as colorless oily product. (Yield: 90%)
Mass Spectrum (CI, m/z): 207 ($M^{+}$+1).
$^{1}$H-NMR Spectrum ($CDCl_3$, δ ppm): 7.88-7.81 (m, 2H), 7.40-7.31 (m, 2H), 4.37 (q, J=7.1 Hz, 2H), 2.66 (t, J=7.7 Hz, 2H), 1.69-1.56 (m, 2H), 1.41-1.28 (m, 2H), 1.40 (t, J=7.1 Hz, 3H), 0.93 (t, J=7.3 Hz, 3H).

32-(b): 3-Butylbenzyl alcohol

To 19 ml of a tetrahydrofuran solution containing 1.26 g (6.11 mmol) of ethyl 3-butylbenzoate obtained in Reference example 32-(a) was added dropwise 4.9 ml (4.9 mmol) of 1.0M lithium aluminum hydride/tetrahydrofuran solution at room temperature over 15 minutes, and the mixture was stirred at the same temperature for 16 hours. After completion of the reaction, to the reaction mixture were successively added 185 μl of water, 185 μl of 4N aqueous sodium hydroxide solution, and 555 μl of water. The precipitated insoluble materials were filtered off, the filtrate was dried over anhydrous magnesium sulfate and concentrated under reduced pressure to obtain 809 mg of the title compound as colorless oily product. (Yield: 81%)
Mass Spectrum (EI, m/z): 164 ($M^+$).
$^1$H-NMR Spectrum ($CDCl_3$, δ ppm): 7.31-7.24 (m, 2H), 7.21-7.09 (m, 2H), 4.67 (d, J=6.1 Hz, 2H), 2.62 (t, J=7.8 Hz, 2H), 1.67-1.53 (m, 2H), 1.46-1.28 (m, 2H), 0.93 (t, J=7.3 Hz, 3H).

[Reference example 33]

N-[4-(1,1-dimethylpentyl)benzyl]pyridin-4-ylsulfonamide

33-(a): N-(2,4-dimethoxybenzyl)-N-[4-(1,1-dimethylpentyl)benzyl]pyridin-4-ylsulfon-amide

To 5 ml of a tetrahydrofuran solution containing 362 mg (1.17 mmol) of N-(2,4-dimethoxybenzyl)pyridin-4-ylsulfonamide obtained in the same manner as in Reference example 14-(b) were added 202 mg (0.979 mmol) of 4-(1,1-dimethylpentyl)-benzyl alcohol obtained in the same manner as in Reference example 29-(b), 365 μl (1.46 mmol) of tri-n-butylphosphine and 253 mg (1.47 mmol) of N,N,N',N'-tetra-methylazodicarboxamide, and the mixture was stirred at room temperature for 2 hours. After completion of the reaction, the reaction mixture was concentrated under reduced pressure. Water was added to the residue, and the resulting mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane: ethyl acetate=4:1→2:1 (V/V), and the fractions containing the objective material were concentrated under reduced pressure to obtain 456 mg of the title compound as yellow oil. (Yield: 94%)
Mass Spectrum (CI, m/z): 497 ($M^+$+1).
$^1$H-NMR Spectrum ($CDCl_3$, δ ppm): 8.66 (dd, J=4.5, 1.5 Hz, 2H), 7.41 (dd, J=4.5, 1.5 Hz, 2H), 7.25-7.19 (m, 2H), 7.16-7.05 (m, 3H), 6.38 (dd, J=8.4, 2.3 Hz, 1H), 6.22 (d, J=2.3 Hz, 1H), 4.43 (s, 2H), 4.35 (s, 2H), 3.78 (s, 3H), 3.54 (s, 3H), 1.62-1.51 (m, 2H), 1.30-1.16 (m, 2H), 1.26 (s, 6H), 1.09-0.95 (m, 2H), 0.83 (t, J=6.8 Hz, 3H).

33-(b): N-[4-(1,1-dimethylpentyl)benzyl]pyridin-4-ylsulfonamide

To 4.5 ml of a methylene chloride solution containing 452 mg (0.910 mmol) of N-(2,4-dimethoxybenzyl)-N-[4-(1,1-dimethylpentyl)benzyl]pyridin-4-ylsulfonamide obtained in 33-(a) was added 2.8 ml (38 mmol) of trifluoroacetic acid, and the mixture was stirred at room temperature for 3 hours. After completion of the reaction, the reaction mixture was concentrated under reduced pressure. Water was added to the residue, and the resulting mixture was made basic with a saturated aqueous sodium hydrogen carbonate solution and extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=2:1→1:1 (V/V)), and the fractions containing the objective material were concentrated under reduced pressure. To the residue were added 0.58 ml of diisopropyl ether and 0.87 ml of heptane, the precipitated solid was collected by filtration and dried under reduced pressure to obtain 273 mg of the title compound as white solid. (Yield: 87%)
Mass Spectrum (CI, m/z): 347 ($M^+$+1).
$^1$H-NMR Spectrum ($CDCl_3$, δ ppm): 8.81 (dd, J=4.5, 1.6 Hz, 2H), 7.67 (dd, J=4.5, 1.6 Hz, 2H), 7.28-7.20 (m, 2H), 7.13-7.05 (m, 2H), 4.77 (t, J=5.9 Hz, 1H), 4.20 (d, J=5.9 Hz, 2H), 1.61-1.50 (m, 2H), 1.29-1.13 (m, 2H), 1.25 (s, 6H), 1.06-0.92 (m, 2H), 0.82 (t, J=7.2 Hz, 3H).

[Reference example 34]

Ethyl {6-[(pyridin-2-ylsulfonyl)aminomethyl]pyridin-2-ylamino}acetate

To 3.59 g (7.50 mmol) of tert-butyl (tert-butoxycarbonyl{6-[(pyridin-2-yl-sulfonyl)aminomethyl]pyridin-2-yl}amino)acetate obtained in the same manner as in Reference example 2 was added 37.5 ml of 2N hydrogen chloride/ethanol solution, and the mixture was refluxed for 3 hours. After completion of the reaction, water was added to the reaction

mixture. The resulting mixture was neutralized with 1N aqueous sodium hydroxide solution and extracted with ethyl acetate. The organic layer was washed with a saturated aqueous sodium chloride solution, dried over anhydrous magnesium sulfate and concentrated under reduced pressure to obtain 2.17 g of the title compound as brown oil. (Yield: 83%)
Mass Spectrum (CI, m/z): 351 ($M^{+}$+1).
$^{1}$H-NMR Spectrum ($CDCl_3$, δ ppm): 8.62 (ddd, J=4.8, 1.7, 0.9 Hz, 1H), 7.97 (ddd, J=7.8, 1.1, 0.9 Hz, 1H), 7.84 (ddd, J=7.8, 7.7, 1.7 Hz, 1H), 7.41 (ddd, J=7.7, 4.8, 1.1 Hz, 1H), 7.29 (dd, J=8.2, 7.2 Hz, 1H), 6.44 (d, J=7.2 Hz, 1H), 6.29 (d, J=8.2 Hz, 1H), 6.04 (brs, 0.8H), 4.92 (t, J=5.2 Hz, 1H), 4.30-4.21 (m, 4H), 4.07 (d, J=5.2 Hz, 2H), 1.31 (t, J=7.2 Hz, 3H).

[Reference example 35]

tert-Butyl (tert-butoxycarbonyl{6-[(thiophen-2-ylsulfonyl)aminomethyl]pyridin-2-yl}-amino)acetate

Reaction and post-treatment were carried out in the similar manner as in Reference example 1-(f) except for using 0.75 g (2.2 mmol) of tert-butyl [(6-amino-methylpyridin-2-yl)tert-butoxycarbonylamino] acetate obtained in the same manner as in Reference example 1-(e), and using 0.41 g (2.2 mmol) of thiophen-2-ylsulfonyl chloride in place of 3-pyridylsulfonyl chloride to obtain 0.68 g of the title compound as yellow oil. (Yield: 64%)
Mass Spectrum (FAB, m/z): 484 ($M^{+}$+1).
$^{1}$H-NMR Spectrum ($CDCl_3$, δ ppm): 7.71 (d, J=8.2 Hz, 1H), 7.57 (dd, J=3.7, 1.3 Hz, 1H), 7.56 (dd, J=8.2, 7.4 Hz, 1H), 7.50 (dd, J=5.1, 1.3 Hz, 1H), 7.01 (dd, J=5.1, 3.7 Hz, 1H), 6.83 (dd, J=7.4, 0.6 Hz, 1H), 5.68 (t, J=5.2 Hz, 1H), 4.45 (s, 2H), 4.27 (d, J=5.2 Hz, 2H), 1.53 (s, 9H), 1.47 (s, 9H).

[Reference example 36]

tert-Butyl [tert-butoxycarbonyl(6-{[4-1,1-dimethylpentyl)benzyl]aminomethyl}-pyridin-2-yl)amino] acetate

To 5 ml of a methylene chloride solution containing 0.48 g (1.4 mmol) of tert-butyl [(6-aminomethylpyridin-2-yl)tert-butoxycarbonylamino] acetate obtained in the same manner as in Reference example 1-(e) was added 244 mg (1.19 mmol) of 4-(1,1-dimethylpentyl)benzaldehyde obtained in the same manner as in Reference example 29-(a), and the mixture was stirred at room temperature for 18 hours. Then, 0.38 g (1.8 mmol) of triacetoxy potassium borohydride was added to the mixture, and the resulting mixture was stirred at room temperature for 18 hours. After completion of the reaction, to the reaction mixture was added a saturated aqueous sodium hydrogen carbonate solution, and the resulting mixture was extracted with methylene chloride. The organic layer was dried over anhydrous magnesium sulfate and concentrated under reduced pressure. The residue was applied to silica gel column chromatography (eluent; n-hexane:ethyl acetate=4:1→1:1 (V/V)), and the fractions containing the objective material were concentrated under reduced pressure to obtain 0.41 g of the title compound as yellow oil. (Yield: 66%)
Mass Spectrum (CI, m/z): 526 ($M^{+}$+1).
$^{1}$H-NMR Spectrum (DMSO-$d_6$, δ ppm): 7.72 (dd, J=8.3, 7.3 Hz, 1H), 7.59 (d, J=8.3 Hz, 1H), 7.32-7.18 (m, 4H), 7.16 (d, J=7.3 Hz, 1H), 4.46 (s, 2H), 3.68 (s, 2H), 3.66 (s, 2H), 1.60-1.51 (m, 2H), 1.46 (s, 9H), 1.35 (s, 9H), 1.23 (s, 6H), 1.21-1.12 (m, 2H), 1.03-0.90 (m, 2H), 0.78 (t, J=7.2 Hz, 3H).

[Reference example 37]

4-(1,1-Dimethylbutyl)benzyl bromide

To 2 ml of a chlorobenzene solution containing 164 mg (0.930 mmol) of 1-methyl-4-(1,1-dimethylbutyl)benzene (see Journal of the American Chemical Society, 74, 5163(1952)) were added 164 mg (0.921 mmol) of NBS and 25.7 mg (0.103 mmol) of 2,2'-azobis(2,4-dimethylvaleronitrile), and the mixture was stirred at 90°C for 1.5 hours. After completion of the reaction, to the reaction mixture was added a saturated aqueous sodium hydrogen carbonate solution, and the resulting mixture was extracted with toluene. The organic layer was washed with a saturated aqueous sodium hydrogen carbonate solution, dried over anhydrous magnesium sulfate and concentrated under reduced pressure to obtain 280 mg of the title compound as pale yellow oil substantially quantitatively.
Mass Spectrum (EI, m/z): 254, 256 ($M^{+}$).
$^{1}$H-NMR Spectrum ($CDCl_3$, δ ppm): 7.37-7.19 (m, 4H), 4.49 (s, 2H), 1.61-1.48 (m, 2H), 1.28 (s, 6H), 1.15-0.98 (m, 2H), 0.81 (t, J=7.3 Hz, 3H).

[Reference example 38]

4-(5,5,5-Trifluoro-1,1-dimethylpentyl)benzyl alcohol

38-(a): 1-Bromo-4-(5,5,5-trifluoro-1,1-dimethylpentyl)benzene

To 25 ml of a methylene chloride solution containing 1.0 ml (9.1 mmol) of titanium tetrachloride was added dropwise 4.56 ml (9.1 mmol) of 2.0M dimethyl-zinc/toluene solution at -45°C over 15 minutes, and the mixture was stirred at the same temperature for 30 minutes. Then, to the mixture was added dropwise 5 ml of a methylene chloride solution containing 1.18 g (4.00 mmol) of 1-(4-bromophenyl)-5,5,5-trifluoropentan-1-one (see US2009/0202478A) at -45°C over 20 minutes. After completion of the dropwise addition, the temperature of the mixture was gradually raised to room temperature. After completion of the reaction, water was added to the reaction mixture, and the resulting mixture was extracted with methylene chloride. The organic layer was washed successively with water and a saturated aqueous sodium chloride solution, dried over anhydrous magnesium sulfate and concentrated under reduced pressure. The residue was applied to silica gel column chromatography (eluent; n-hexane), and the fractions containing the objective material were concentrated under reduced pressure to obtain 757 mg of the title compound as colorless oily product.
(Yield: 61 %)
Mass Spectrum (EI, m/z): 308, 310 ($M^+$).
$^1$H-NMR Spectrum ($CDCl_3$, $\delta$ ppm): 7.45-7.40 (m, 2H), 7.20-7.16 (m, 2H), 2.05-1.90 (m, 2H), 1.67-1.60 (m, 2H), 1.35-1.23 (m, 2H), 1.29 (s, 6H).

38-(b): 4-(5,5,5-Trifluoro-1,1-dimethylpentyl)benzaldehyde

Reaction and post-treatment were carried out in the similar manner as in Reference example 29-(a) except for using 754 mg (2.44 mmol) of 1-bromo-4-(5,5,5-trifluoro-1,1-dimethylpentyl)benzene obtained in 38-(a) in place of 1-bromo-4-(1,1-dimethylpentyl)benzene, and using 1.84 ml (2.9 mmol) of 1.6M n-butyl lithium/n-hexane solution and 0.57 ml (7.4 mmol) of N,N-dimethylformamide to obtain 528 mg of the title compound as colorless oily product. (Yield: 84%)
Mass Spectrum (CI, m/z): 259 ($M^+$+1).
$^1$H-NMR Spectrum ($CDCl_3$, $\delta$ ppm): 9.99 (s, 1H), 7.85-7.82 (m, 2H), 7.51-7.48 (m, 2H), 2.04-1.91 (m, 2H), 1.75-1.66 (m, 2H), 1.37-1.27 (m, 8H).

38-(c): 4-(5,5,5-Trifluoro-1,1-dimethylpentyl)benzyl alcohol

Reaction and post-treatment were carried out in the similar manner as in Reference example 23-(d) except for using 520 mg (2.01 mmol) of 4-(5,5,5-trifluoro-1,1-dimethylpentyl)benzaldehyde obtained in Reference example 38-(b) in place of 4-(1-ethyl-1-methylpropyl)benzaldehyde and using 38.0 mg (1.00 mmol) of potassium borohydride to obtain 240 mg of the title compound as pale yellow oil. (Yield: 46%) Mass Spectrum (EI, m/z): 260 ($M^+$).
$^1$H-NMR Spectrum ($CDCl_3$, $\delta$ ppm): 7.33 (s, 4H), 4.68 (s, 2H), 2.03-1.89 (m, 2H), 1.70-1.62 (m, 2H), 1.37-1.26 (m, 2H), 1.32 (s, 6H).

[Reference example 39]

3-Fluoro-4-(1,1-dimethylpentyl)benzyl alcohol

39-(a): 3-Fluoro-4-(1,1-dimethylpentyl)benzaldehyde

Reaction and post-treatment were carried out in the similar manner as in Reference example 29-(a) except for using 880 mg (3.22 mmol) of 1-bromo-3-fluoro-4-(1,1-dimethylpentyl)benzene (see Molecular Crystals and Liquid Crystals, 195, 221 (1991)) in place of 1-bromo-4-(1,1-dimethylpentyl)benzene, and using 2.06 ml (3.3 mmol) of 1.6M n-butyl lithium/n-hexane solution and 0.75 ml (9.7 mmol) of N,N-dimethylformamide to obtain 418 mg of the title compound as pale yellow oil. (Yield: 58%)
Mass Spectrum (CI, m/z): 223 ($M^+$+1).
$^1$H-NMR Spectrum ($CDCl_3$, $\delta$ ppm): 9.95 (d, J=1.7 Hz, 1H), 7.59 (dd, J=7.8, 1.7 Hz, 1H), 7.49 (dd, J=12.5, 1.7 Hz, 1H), 7.42 (dd, J=7.8, 7.8 Hz, 1H), 1.81-1.71 (m, 2H), 1.38 (s, 3H), 1.38 (s, 3H), 1.31-1.16 (m, 2H), 1.06-0.92 (m, 2H), 0.82 (t, J=7.3 Hz, 3H).

39-(b): 3-Fluoro-4-(1,1-dimethylpentyl)benzyl alcohol

Reaction and post-treatment were carried out in the similar manner as in Reference example 23-(d) except for using 412 mg (1.85 mmol) of 3-fluoro-4-(1,1-dimethylpentyl)benzaldehyde obtained in Reference example 39-(a) in place of 4-(1-ethyl-1-methylpropyl)benzaldehyde and using 35.0 mg (0.925 mmol) of potassium borohydride to obtain 393 mg of the title compound as colorless oily product. (Yield: 95%)
Mass Spectrum (EI, m/z): 224 ($M^+$).
$^1$H-NMR Spectrum ($CDCl_3$, $\delta$ ppm): 7.25-7.18 (m, 1H), 7.07-6.97 (m, 2H), 4.66 (d, J=5.9 Hz, 2H), 1.76-1.67 (m, 2H), 1.66 (t, J=5.9 Hz, 1H), 1.34 (s, 3H), 1.34 (s, 3H), 1.30-1.16 (m, 2H), 1.06-0.93 (m, 2H), 0.82 (t, J=7.3 Hz, 3H).

[Reference example 40]

2-Fluoro-4-(1,1-dimethylpentyl)benzyl alcohol

40-(a): 1-(4-Bromo-3-fluorophenyl)pentan-1-one

Reaction and post-treatment were carried out in the similar manner as in Reference example 31-(c) except for using 2.48 g (9.46 mmol) of 4-bromo-3-fluoro-N-methoxy-N-methylbenzamide (see US2008/39457A) in place of N-methoxy-4-(4-methoxybenzyloxymethyl)-N-methylbenzamide, and using 240 mg (9.87 mmol) of magnesium and 1.43 g (10.4 mmol) of 1-bromobutane to obtain 1.44 g of the title compound as colorless oily product. (Yield: 59%)
Mass Spectrum (EI, m/z): 258, 260 ($M^+$).
$^1$H-NMR Spectrum ($CDCl_3$, $\delta$ ppm): 7.72-7.59 (m, 3H), 2.92 (t, J=7.3 Hz, 2H), 1.78-1.65 (m, 2H), 1.47-1.34 (m, 2H), 0.95 (t, J=7.3 Hz, 3H).

40-(b): 1-Bromo-2-fluoro-4-(1,1-dimethylpentyl)benzene

Reaction and post-treatment were carried out in the similar manner as in Reference example 38-(a) except for using 1.43 g (5.52 mmol) of 1-(4-bromo-3-fluorophenyl)pentan-1-one obtained in Reference example 40-(a) in place of 1-(4-bromophenyl)-5,5,5-trifluoropentan-1-one, and using 1.27 ml (11.6 mmol) of titanium tetrachloride and 5.80 ml (11.6 mmol) of 2.0M dimethylzinc/toluene solution to obtain 1.10 g of the title compound as colorless oily product. (Yield: 73 %)
Mass Spectrum (EI, m/z): 272,274 ($M^+$).
$^1$H-NMR Spectrum ($CDCl_3$, $\delta$ ppm): 7.44 (dd, J=8.5, 7.4 Hz, 1H), 7.07 (dd, J=11.0, 2.2 Hz, 1H), 6.98 (ddd, J=8.5, 2.2, 0.5 Hz, 1H), 1.60-1.52 (m, 2H), 1.27 (s, 6H), 1.26-1.14 (m, 2H), 1.07-0.96 (m, 2H), 0.82 (t, J=7.3 Hz, 3H).

40-(c): 2-Fluoro-4-(1,1-dimethylpentyl)benzaldehyde

Reaction and post-treatment were carried out in the similar manner as in Reference example 29-(a) except for using 981 mg (3.59 mmol) of 1-bromo-2-fluoro-4-(1,1-dimethylpentyl)benzene obtained in Reference example 40-(b) in place of 1-bromo-4-(1,1-dimethylpentyl)benzene, and using 2.28 ml (3.6 mmol) of 1.6M n-butyl lithium/n-hexane solution and 0.83 ml (11 mmol) of N,N-dimethylformamide to obtain 296 mg of the title compound as pale yellow oil. (Yield: 37%)
Mass Spectrum (CI, m/z): 223 ($M^++1$).
$^1$H-NMR Spectrum ($CDCl_3$, $\delta$ ppm): 10.32 (d, J=0.5 Hz, 1H), 7.80 (dd, J=8.1, 7.9 Hz, 1H), 7.23 (ddd, J=8.1, 1.7, 0.7 Hz, 1H), 7.11 (dd, J=12.7, 1.7 Hz, 1H), 1.65-1.57 (m, 2H), 1.30 (s, 6H), 1.29-1.16 (m, 2H), 1.07-0.94 (m, 2H), 0.83 (t, J=7.3 Hz, 3H).

40-(d): 2-Fluoro-4-(1,1-dimethylpentyl)benzyl alcohol

Reaction and post-treatment were carried out in the similar manner as in Reference example 23-(d) except for using 287 mg (1.29 mmol) of 2-fluoro-4-(1,1-dimethylpentyl)benzaldehyde obtained in Reference example 40-(c) in place of 4-(1-ethyl-1-methylpropyl)benzaldehyde and using 24.4 mg (0.645 mmol) of potassium borohydride to obtain 130 mg of the title compound as colorless oily product. (Yield: 45%)
Mass Spectrum (EI, m/z): 224 ($M^+$).
$^1$H-NMR Spectrum ($CDCl_3$, $\delta$ ppm): 7.32 (dd, J=8.1, 8.0 Hz, 1H), 7.10 (dd, J=8.0, 1.9 Hz, 1H), 7.01 (dd, J=12.5, 1.9 Hz, 1H), 4.73 (d, J=6.0 Hz, 2H), 1.71 (t, J=6.0 Hz, 1H), 1.61-1.53 (m, 2H), 1.30-1.15 (m, 2H), 1.27 (s, 6H), 1.08-0.97 (m, 2H), 0.82 (t, J=7.2 Hz, 3H).

[Reference example 41]

3-Dimethylamino-4-pentylbenzyl alcohol

41-(a): Ethyl 3-nitro-4-pentylbenzoate

To 30 ml of a methylene chloride solution containing 1.55 g (6.53 mmol) of 3-nitro-4-pentylbenzoic acid (see Journal of the Chemical Society, 4519 (1952)) were added 30 μl (0.39 mmol) of N,N-dimethylformamide and 845 μl (9.99 mmol) of oxalyl chloride, and the mixture was stirred at room temperature for 2.5 hours. Then, 7.0 ml (120 mmol) of ethanol was added to the mixture, and the resulting mixture was stirred at room temperature for 21.5 hours. After completion of the reaction, the reaction mixture was concentrated under reduced pressure. To the residue was added a saturated aqueous sodium hydrogen carbonate solution, and the resulting mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=1:0→7:3 (V/V)), and the fractions containing the objective material were concentrated under reduced pressure to obtain 1.69 g of the title compound as colorless oily product. (Yield: 98%)
Mass Spectrum (CI, m/z): 266 ($M^{+}$+1).
$^{1}$H-NMR Spectrum ($CDCl_3$, δ ppm): 8.50 (d, J=1.8 Hz, 1H), 8.15 (dd, J=8.1, 1.8 Hz, 1H), 7.43 (d, J=8.1 Hz, 1H), 4.41 (q, J=7.1 Hz, 2H), 2.98-2.86 (m, 2H), 1.72-1.58 (m, 2H), 1.41 (t, J=7.1 Hz, 3H), 1.40-1.30 (m, 4H), 0.96-0.84 (m, 3H).

41-(b): Ethyl 3-amino-4-pentylbenzoate

To 30 ml of an ethanol solution containing 1.69 g (6.37 mmol) of ethyl 3-nitro-4-pentylbenzoate obtained in Reference example 41-(a) was added 180 mg of 5% palladium-active carbon (55% hydrate), and the mixture was stirred under hydrogen atmosphere at 1 atm at 50°C for 2 hours. After completion of the reaction, the reaction mixture was filtered, and the filtrate was concentrated under reduced pressure to obtain 1.48 g of the title compound as pale yellow oil. (Yield: 99%)
Mass Spectrum (CI, m/z): 236 ($M^{+}$+1).
$^{1}$H-NMR Spectrum ($CDCl_3$, δ ppm): 7.40 (dd, J=7.8, 1.7 Hz, 1H), 7.35 (d, J=1.7 Hz, 1H), 7.09 (d, J=7.8 Hz, 1H), 4.34 (q, J=7.1 Hz, 2H), 3.71 (s, 2H), 2.51 (t, J=7.8 Hz, 2H), 1.71-1.52 (m, 2H), 1.44-1.28 (m, 4H), 1.37 (t, J=7.1 Hz, 3H), 0.96-0.85 (m, 3H).

41-(c): Ethyl 3-dimethylamino-4-pentylbenzoate

To 811 mg (3.45 mmol) of ethyl 3-amino-4-pentylbenzoate obtained in Reference example 41-(b) were added 1.2 ml of water, 2.4 ml of formic acid and 1.2 ml of 30% aqueous formaldehyde solution, and the mixture was stirred at room temperature for 1 hour, and then, at 100°C for 3 hours. After completion of the reaction, a saturated aqueous sodium hydrogen carbonate solution was added to the reaction mixture, and the mixture was extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=1:0→3:2 (V/V)), and the fractions containing the objective material were concentrated under reduced pressure to obtain 288 mg of the title compound as oily product. (Yield: 32%)
Mass Spectrum (CI, m/z): 264 ($M^{+}$+1).
$^{1}$H-NMR Spectrum ($CDCl_3$, δ ppm): 7.75 (d, J=1.6 Hz, 1H), 7.67 (dd, J=8.0, 1.6 Hz, 1H), 7.24 (d, J=8.0 Hz, 1H), 4.36 (q, J=7.1 Hz, 2H), 2.76-2.66 (m, 2H), 2.70 (s, 6H), 1.71-1.57 (m, 2H), 1.43-1.31 (m, 4H), 1.39 (t, J=7.1 Hz, 3H), 0.94-0.86 (m, 3H).

41-(d): 3-Dimethylamino-4-pentylbenzyl alcohol

Reaction and post-treatment were carried out in the similar manner as in Reference example 32-(b) except for using 348 mg (1.32 mmol) of ethyl 3-dimethylamino-4-pentylbenzoate obtained in Reference example 41-(c) in place of ethyl 3-butylbenzoate and using 1.1 ml (1.1 mmol) of 1.0M lithium aluminum hydride/tetrahydrofuran solution to obtain 287 mg of the title compound as colorless oily product.
(Yield: 98%)
Mass Spectrum (EI, m/z): 221 ($M^{+}$).
$^{1}$H-NMR Spectrum ($CDCl_3$, δ ppm): 7.19 (d, J=7.7 Hz, 1H), 7.10 (d, J=1.6 Hz, 1H), 6.99 (dd, J=7.7, 1.6 Hz, 1H), 4.64 (d, J=5.9 Hz, 2H), 2.70-2.63 (m, 2H), 2.68 (s, 6H), 1.70-1.54 (m, 2H), 1.41-1.32 (m, 4H), 0.94-0.86 (m, 3H).

[Reference example 42]

4-(2,2-Dimethylpentyl)benzyl alcohol

42-(a): 1-(4-Bromophenyl)-2,2-dimethylpentan-1-one

To 80 ml of a tetrahydrofuran solution containing 3.98 g (91 mmol) of sodium hydride (mineral oil 55% dispersed product) was added dropwise 10.0 g (41.5 mmol) of 1-(4-bromophenyl)pentan-1-one over 1 minute, and the mixture was stirred at room temperature for 30 minutes. Then, to the mixture was added dropwise 5.7 ml (92 mmol) of iodomethane over 3 minutes, and the mixture was stirred at room temperature for 1 hour, and then, refluxed for 2 hours. After completion of the reaction, the reaction mixture was poured into a saturated aqueous ammonium chloride solution, and extracted with ethyl acetate. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=1:0→79:21 (V/V)), and the fractions containing the objective material were concentrated under reduced pressure to obtain 9.93 g of the title compound as pale yellow oil. (Yield: 89%)
Mass Spectrum (EI, m/z): 268, 270 ($M^+$).
$^1$H-NMR Spectrum ($CDCl_3$, δ ppm): 7.60-7.50 (m, 4H), 1.75-1.65 (m, 2H), 1.36-1.15 (m, 2H), 1.29 (s, 6H), 0.86 (t, J=7.2 Hz, 3H).

42-(b): 1-Bromo-4-(2,2-dimethylpentyl)benzene

To 18 ml of a diethylene glycol solution containing 4.74 g (17.6 mmol) of 1-(4-bromophenyl)-2,2-dimethylpentan-1-one obtained in Reference example 42-(a) were added 7.92 ml (163 mmol) of hydrazine monohydrate and 2.98 g (53.1 mmol) of potassium hydroxide, and the mixture was stirred at 120°C for 1 hour, and then, at 190°C for 1.5 hours. After completion of the reaction, water was added to the reaction mixture, and the resulting mixture was extracted with n-hexane. The organic layer was dried over anhydrous magnesium sulfate and concentrated under reduced pressure. The residue was applied to silica gel column chromatography (eluent; n-hexane), and the fractions containing the objective material were concentrated under reduced pressure to obtain 2.20 g of the title compound as colorless oily product. (Yield: 49%) Mass Spectrum (EI, m/z): 254, 256 ($M^+$).
$^1$H-NMR Spectrum ($CDCl_3$, δ ppm): 7.41-7.33 (m, 2H), 7.01-6.95 (m, 2H), 2.44 (s, 2H), 1.41-1.25 (m, 2H), 1.19-1.10 (m, 2H), 0.89 (t, J=7.2 Hz, 3H), 0.82 (s, 6H).

42-(c): 4-(2,2-Dimethylpentyl)benzaldehyde

Reaction and post-treatment were carried out in the similar manner as in Reference example 29-(a) except for using 500 mg (1.96 mmol) of 1-bromo-4-(2,2-dimethylpentyl)benzene obtained in Reference example 42-(b) in place of 1-bromo-4-(1,1-dimethylpentyl)benzene, and using 1.4 ml (2.3 mmol) of 1.67M n-butyl lithium/n-hexane solution and 0.46 ml (5.9 mmol) of N,N-dimethylformamide to obtain 342 mg of the title compound as colorless oily product. (Yield: 85%)
Mass Spectrum (EI, m/z): 205 ($M^+$+1).
$^1$H-NMR Spectrum ($CDCl_3$, δ ppm): 9.99 (s, 1H), 7.83-7.74 (m, 2H), 7.32-7.24 (m, 2H), 2.58 (s, 2H), 1.41-1.24 (m, 2H), 1.22-1.13 (m, 2H), 0.91 (t, J=7.1 Hz, 3H), 0.86 (s, 6H).

42-(d): 4-(2,2-Dimethylpentyl)benzyl alcohol

Reaction and post-treatment were carried out in the similar manner as in Reference example 23-(d) except for using 337 mg (1.65 mmol) of 4-(2,2-dimethylpentyl)benzaldehyde obtained in Reference example 42-(c) in place of 4-(1-ethyl-1-methylpropyl)benzaldehyde and using 32.6 mg (0.861 mmol) of potassium borohydride to obtain 309 mg of the title compound as pale yellow oil. (Yield: 91%)
Mass Spectrum (EI, m/z): 206 ($M^+$).
$^1$H-NMR Spectrum ($CDCl_3$, δ ppm): 7.30-7.23 (m, 2H), 7.15-7.08 (m, 2H), 4.67 (d, J=5.9 Hz, 2H), 2.49 (s, 2H), 1.42-1.27 (m, 2H), 1.20-1.13 (m, 2H), 0.90 (t, J=7.2 Hz, 3H), 0.84 (s, 6H).

[Reference example 43]

4-(1,1-Dimethylhexyl)benzyl bromide

Reaction and post-treatment were carried out in the similar manner as in Reference example 37 except for using 135 mg (0.661 mmol) of 1-methyl-4-(1,1-dimethylhexyl)benzene (see Neftekhimiya, 6, 186 (1966)) in place of

1-methyl-4-(1,1-dimethylbutyl)benzene, and using 140 mg (0.785 mmol) of NBS and 20.0 mg (0.0805 mmol) of 2,2'-azobis(2,4-dimethylvaleronitrile) to obtain 215 mg of the title compound as pale yellow oil substantially quantitatively.
Mass Spectrum (EI, m/z): 282,284 ($M^+$).
$^1$H-NMR Spectrum ($CDCl_3$, δ ppm): 7.35-7.26 (m, 4H), 4.50 (s, 2H), 1.62-1.51 (m, 2H), 1.27 (s, 6H), 1.26-1.10 (m, 4H), 1.10-0.99 (m, 2H), 0.82 (t, J=6.8 Hz, 3H).

[Reference example 44]

4-(1,1,4-Trimethylpentyl)benzyl alcohol

44-(a): 4-(1,1,4-Trimethylpentyl)benzaldehyde

Reaction and post-treatment were carried out in the similar manner as in Reference example 29-(a) except for using 835 mg (3.10 mmol) of 1-bromo-4-(1,1,4-trimethylpentyl)benzene (see US2008/267892A) in place of 1-bromo-4-(1,1-dimethylpentyl)benzene, and using 2.23 ml (3.6 mmol) of 1.6M n-butyl lithium/n-hexane solution and 715 μl (9.2 mmol) of N,N-dimethylformamide to obtain 0.53 g of the title compound as oily product. (Yield: 78%)
Mass Spectrum (CI, m/z): 219 ($M^+$+1).
$^1$H-NMR Spectrum ($CDCl_3$, δ ppm): 9.98 (s, 1H), 7.84-7.79 (m, 2H), 7.51-7.46 (m, 2H), 1.68-1.58 (m, 2H), 1.48-1.34 (m, 1H), 1.32 (s, 6H), 0.96-0.85 (m, 2H), 0.80 (d, J=6.6 Hz, 6H).

44-(b): 4-(1,1,4-Trimethylpentyl)benzyl alcohol

Reaction and post-treatment were carried out in the similar manner as in Reference example 23-(d) except for using 0.53 g (2.4 mmol) of 4-(1,1,4-trimethylpentyl)benzaldehyde obtained in Reference example 44-(a) in place of 4-(1-ethyl-1-methylpropyl)benzaldehyde and using 137 mg (3.62 mmol) of potassium borohydride to obtain 570 mg of the title compound as oily product substantially quantitatively.
Mass Spectrum (EI, m/z): 220 ($M^+$).
$^1$H-NMR Spectrum ($CDCl_3$, δ ppm): 7.35-7.27 (m, 4H), 4.67 (d, J=5.9 Hz, 2H), 1.64-1.55 (m, 2H), 1.47-1.32 (m, 1H), 1.28 (s, 6H), 0.98-0.88 (m, 2H), 0.81 (d, J=6.6 Hz, 6H).

[Reference example 45]

4-(3-Difluoromethoxy-1,1-dimethylpropyl)benzyl alcohol

45-(a): 1-(3-Difluoromethoxy-1,1-dimethylpropyl)-4-methylbenzene

To 6.3 ml of an acetonitrile solution containing 2.25 g (12.6 mmol) of 3-methyl-3-(4-toluyl)butan-1-ol (see Indian Journal of Chemistry, 12, 476 (1974)) and 1.20 g of anhydrous sodium sulfate was added 2,2-difluoro-2-(fluorosulfonyl)acetic acid 521 μl (5.04 mmol) at 50°C, and the mixture was stirred at the same temperature for 2 hours. After completion of the reaction, water was added to the reaction mixture, and the resulting mixture was extracted with diethyl ether. The organic layer was washed with a saturated aqueous sodium chloride solution, dried over anhydrous magnesium sulfate and concentrated under reduced pressure. The residue was applied to silica gel column chromatography (eluent; n-hexane:ethyl acetate=4:1 (V/V)), and the fractions containing the objective material were concentrated under reduced pressure to obtain 781 mg of the title compound as colorless oily product. (Yield: 68%)
$^1$H-NMR Spectrum ($CDCl_3$, δ ppm): 7.26-7.20 (m, 2H), 7.16-7.10 (m, 2H), 6.08 (t, J=75.3 Hz, 1H), 3.64 (t, J=7.7 Hz, 2H), 2.32 (s, 3H), 2.00 (t, J=7.7 Hz, 2H), 1.33 (s, 6H).

45-(b): 4-(3-Difluoromethoxy-1,1-dimethylpropyl)benzyl bromide

Reaction and post-treatment were carried out in the similar manner as in Reference example 37 except for using 1.04 g (4.56 mmol) of 1-(3-difluoromethoxy-1,1-dimethylpropyl)-4-methylbenzene obtained in the same manner as in Reference example 45-(a) in place of 1-methyl-4-(1,1-dimethylbutyl)benzene, and using 965 mg (5.42 mmol) of NBS and 138 mg (0.556 mmol) of 2,2'-azobis(2,4-dimethylvaleronitrile) to obtain 1.52 g of the title compound as pale yellow oilsubstantially quantitatively.
Mass Spectrum (EI, m/z): 306, 308 ($M^+$).
$^1$H-NMR Spectrum ($CDCl_3$, δ ppm): 7.40-7.27 (m, 4H), 6.08 (t, J=75.2 Hz, 1H), 4.49 (s, 2H), 3.64 (t, J=7.6 Hz, 2H), 2.01 (t, J=7.6 Hz, 2H), 1.35 (s, 6H).

[Test example 1]

Measurement of EP2 receptor binding affinity

Measurement of EP2 receptor binding affinity was carried out according to the method of Abramovitz et al. (Biochimica et Biophysica Acta, 1483, 285 (2000)). A test compound dissolved in dimethylsulfoxide and $[^3H]PGE_2$ (NET-428, available from PerkinElmer Inc.) (final concentration: 10 nM) were added to a buffer solution (10 mM MES-KOH (pH 6.0), 10 mM $MgCl_2$, 1 mM EDTA) in which 10 μg of a membrane fraction (ES-562-M, available from Euroscreen S.A.) of HEK293 cells expressing human EP2 receptor had been suspended, followed by incubation at 30°C for 60 minutes. The membrane fraction was recovered on glass fiber filter paper (GF/B, available from Whatman PLC) using a cell harvester (M30R, available from Brandel Inc.), and after washing with a buffer solution (10 mM MES-KOH (pH 6.0), 10 mM $MgCl_2$), radioactivity was measured with a liquid scintillation analyzer (2000CA, available from Packard). The concentration ($IC_{50}$ value) of the test compound necessary for substituting 50% of the $[^3H]PGE_2$ bound to the receptor was calculated by using EXSAS (version 7.1.6, available from Arm Systex Co., Ltd.), and the inhibition constant (Ki value) was obtained from the following formula. The dissociation constant (Kd value) was calculated by Scatchard analysis.

$$Ki = IC_{50}/(1+([^3H]PGE_2 \text{ concentration}/Kd))$$

The test results are shown in Table 2. Incidentally, Compound A shown in the table is a sodium salt of {3-[(4-tert-butylbenzyl)(pyridin-3-ylsulfonyl)aminomethyl]phenoxy} acetic acid (CP-533,536), which is a compound of Example 14e of WO 99/19300A, and is a control compound having EP2 receptor binding affinity.

[Table 2]

| Test compound Example No. | Ki value of EP2 receptor binding affinity (nM) |
|---|---|
| Example 1 | 2.1 |
| Example 2 | 1.9 |
| Example 3 | 1.8 |
| Example 4 | 3.4 |
| Example 7 | 8.3 |
| Example 9 | 2.9 |
| Example 10 | 11 |
| Example 16 | 6.1 |
| Example 18 | 5.5 |
| Example 28 | 9.8 |
| Example 30 | 8.3 |
| Example 31 | 8.6 |
| Example 33 | 4.4 |
| Example 37 | 1.8 |
| Example 40 | 5.3 |
| Example 43 | 3.6 |
| Example 46 | 3.3 |
| Example 47 | 3.2 |
| Example 48 | 6.4 |
| Example 53 | 8.7 |
| Example 54 | 3.4 |

(continued)

| Test compound Example No. | Ki value of EP2 receptor binding affinity (nM) |
|---|---|
| Example 55 | 2.1 |
| Compound A | 16 |

In this test, compounds of the present invention showed excellent EP2 receptor binding affinity as compared to Control compound.

[Test example 2]

Measurement of EP2 agonist activity

Measurement of EP2 agonist activity was carried out according to the method of Wilson et al. (European Journal of Pharmacology, 501, 49 (2004)). HEK 293 cells (ES-562-C, available from Euroscreen S.A.) expressed human EP2 receptor were cultured in a MEM medium containing 10% FBS and seeded at 2 x $10^4$ cells per each well of a 96-well plate. On the next day, the medium was replaced with serum-free MEM medium containing 3-isobutyl -1-methylxanthine (final concentration: 500 $\mu$M) and after culturing for 30 minutes, a test compound dissolved in dimethylsulfoxide was added and the medium was allowed to stand in a carbon dioxide gas incubator. After 30 minutes, an amount of cAMP in the cells was measured with a cAMP Biotrak EIA System kit (available from GE Healthcare Bioscience). The concentration ($EC_{50}$ value) of the test compound necessary for increasing the amount of cAMP to 50% of the maximum increased amount was calculated by non-linear regression of the concentration of the test compound and the amount of cAMP using EXSAS.
The test results are shown in Table 3.
[Table 3]

| Test compound Example No. | $EC_{50}$ value of EP2 agonist activity (nM) |
|---|---|
| Example 1 | 9.5 |
| Example 2 | 3.6 |
| Example 3 | 6.0 |
| Example 4 | 7.0 |
| Example 5 | 2.6 |
| Example 6 | 5.4 |
| Example 7 | 4.5 |
| Example 9 | 4.9 |
| Example 10 | 4.2 |
| Example 14 | 8.0 |
| Example 16 | 6.3 |
| Example 18 | 3.0 |
| Example 21 | 4.3 |
| Example 23 | 2.7 |
| Example 24 | 4.5 |
| Example 27 | 6.9 |
| Example 28 | 6.4 |
| Example 29 | 5.0 |
| Example 30 | 2.7 |

(continued)

| Test compound Example No. | $EC_{50}$ value of EP2 agonist activity (nM) |
|---|---|
| Example 31 | 6.1 |
| Example 33 | 4.4 |
| Example 35 | 2.8 |
| Example 37 | 6.1 |
| Example 40 | 2.8 |
| Example 47 | 7.1 |
| Example 51 | 8.2 |
| Example 53 | 4.1 |
| Example 54 | 3.4 |
| Example 55 | 7.0 |
| Example 60 | 5.5 |
| Compound A | 17 |

In the present test, compounds of the present invention showed excellent EP2 agonist activity as compared with that of the control compound.

[Test example 3]

Measurement of $PGE_2$ receptor selectivity

With regard to EP1, EP3 and EP4 receptors, measurements of the receptor binding affinity were carried out in the same manner as in Test example 1 according to the following methods.

1) Measurement of EP1 receptor binding affinity

Measurement of EP1 receptor binding affinity was carried out according to the method of Abramovitz et al. (Biochimica et Biophysica Acta, 1483, 285 (2000)). A test compound dissolved in dimethylsulfoxide and $[^3H]PGE_2$ (NET-428, available from PerkinElmer Inc.) (final concentration: 10 nM) were added to a buffer solution (10 mM MES-KOH (pH 6.0), 10 mM $MgCl_2$ 1 mM EDTA) in which 10 μg of a membrane fraction (HTS099M, available from Millipore Corp) of Chem-1 cells expressing human EP1 receptor had been suspended, followed by incubation at 30°C for 60 minutes. The membrane fraction was recovered on glass fiber filter paper (GF/B, available from Whatman PLC) pretreated with 0.3% polyethyleneimine by using a cell harvester (M30R, available from Brandel Inc.), and after washing with a buffer solution (10 mM MES-KOH (pH 6.0), 10 mM $MgCl_2$), radioactivity was measured with a liquid scintillation analyzer (2000CA, available from Packard). The concentration ($IC_{50}$ value) of the test compound necessary for substituting 50% of the $[^3H]PGE_2$ bound to the receptor was calculated by using EXSAS (version 7.1.6, available from Arm Systex Co., Ltd.), and the inhibition constant (Ki value) was obtained from the following formula. The dissociation constant (Kd value) was calculated by Scatchard analysis. $Ki=IC_{50}/(1+([^3H]PGE_2\ concentration/Kd))$

2) Measurement of EP3 receptor binding affinity

Measurement of EP3 receptor binding affinity was carried out according to the method described in the data sheet of Millipore Corp. The test compound dissolved in dimethylsulfoxide and $[^3H]PGE_2$ (NET-428, available from PerkinElmer Inc.) (final concentration: 2 nM) were added to a buffer solution (50 mM Tris-HCl (pH 7.4), 10 mM $MgCl_2$, 1 mM EDTA) in which 10 μg of a membrane fraction (HTS092M, available from Millipore Corp.) of Chem-1 cells expressing human EP3 receptor had been suspended, followed by incubation at 30°C for 60 minutes. The membrane fraction was recovered on glass fiber filter paper (GF/B, available from Whatman PLC) pretreated with 0.3% polyethyleneimine by using a cell harvester (M30R, available from Brandel Inc.), and after washing with a buffer solution (50 mM Tris-HCl (pH 7.4)), radioactivity was measured with a liquid scintillation analyzer (2000CA, available from Packard). A concentration ($IC_{50}$ value) of the test compound necessary for substituting 50% of the $[^3H]PGE_2$ bound to the receptor was calculated

by using EXSAS (version 7.1.6, available from Arm Systex Co., Ltd.), and the inhibition constant (Ki value) was obtained by the following formula. The dissociation constant (Kd value) was calculated by Scatchard analysis.

$$Ki=IC_{50}/(1+([^3H]PGE_2 \text{ concentration } /Kd))$$

3) Measurement of EP4 receptor binding affinity

Measurement of EP4 receptor binding affinity was carried out according to the method described in the data sheet of Millipore Corp. The test compound dissolved in dimethylsulfoxide and $[^3H]PGE_2$ (NET-428, available from PerkinElmer Inc.) (final concentration: 1 nM) were added to a buffer solution (50 mM HEPES-NaOH (pH 7.4), 5 mM $MgCl_2$, 1 mM $CaCl_2$, 0.2% BSA) in which 20 μg of a membrane fraction (HTS142M, available from Millipore Corp.) of Chem-1 cells expressing human EP4 receptor had been suspended, followed by incubation at 30°C for 60 minutes. The membrane fraction was recovered on glass fiber filter paper (GF/B, available from Whatman PLC) pretreated with 0.3% polyethyleneimine by using a cell harvester (M30R, available from Brandel Inc.), and after washing with a buffer solution (50 mM HEPES-NaOH (pH 7.4), 500 mM NaCl, 0.1% BSA), radioactivity was measured with a liquid scintillation analyzer (2000CA, available from Packard). A concentration ($IC_{50}$ value) of the test compound necessary for substituting 50% of the $[^3H]PGE_2$ bound to the receptor was calculated by using EXSAS (version 7.1.6, available from Arm Systex Co., Ltd.), and the inhibition constant (Ki value) was obtained by the following formula. The dissociation constant (Kd value) was calculated by Scatchard analysis. $Ki=IC_{50}/(1+([^3H]PGE_2 \text{ concentration } /Kd))$

4) $PGE_2$ receptor selectivity

According to the receptor binding affinity measurement tests of [Test example 1], and 1) to 3) of [Test example 3], $PGE_2$ receptor selectivities of the representative compounds of the present invention are shown in Table 4. Incidentally, as a comparative control, $PGE_2$ which is an endogenous ligand was used.

[Table 4]

| Test compound Example No. | EP 1 receptor binding affinity Ki value (nM) | EP2 receptor binding affinity Ki value (nM) | EP3 receptor binding affinity Ki value (nM) | EP4 receptor binding affinity Ki value (nM) |
|---|---|---|---|---|
| Example 1 | >3000 | 2.1 | >3000 | >3000 |
| Example 2 | >3000 | 1.9 | >3000 | >3000 |
| Example 9 | >3000 | 2.9 | >3000 | >3000 |
| Example 18 | >3000 | 5.5 | >3000 | >3000 |
| Example 37 | >3000 | 1.8 | >3000 | >3000 |
| Example 47 | >3000 | 3.2 | >3000 | >3000 |
| Example 53 | >3000 | 8.7 | >3000 | >3000 |
| Example 54 | >3000 | 3.4 | >3000 | >3000 |
| $PGE_2$ | 9.0 | 7.1 | 2.8 | 1.4 |

Compounds of the present invention are weaker in binding affinities to EP1, EP3 and EP4 receptors as compared to that of $PGE_2$, and showed selective EP2 receptor binding affinity.

[Test example 4]

Acetylcholine-induced airway contraction test under no anesthesia

Airway contraction reaction was measured by modifying the method of Pennock, et al. (J. Appl. Physiology, 46, 399-406 (1979)). Guinea pigs (Hartley, male, 7 weeks-old, supplier: Nippon SLC) were fixed in a double-chamber respiratory funciton measurement box (manufactured by Buxco Electronics), and respiratory function thereof were measured by a respiratory function analyzer (Biosystem XA, Buxco Electronics) using a double-chamber plethysmograph

method. As an index of airway contraction, a specific airway resistance (hereinafter abbreviated to as sRaw) was used. Airway contraction reaction was induced by making inhalation of acetylcholine (1 mg/ml, available from Sigma) for 1 minute. At the time of completion of acetylcholine inhalation, change in sRaw with a lapse of time was measured for 6 minutes continuously, and an area under the curve (AUC) was calculated.
The test compound was dissolved in a phosphate-buffered saline to prepare a 10mM solution. The test compound or a phosphate-buffered saline was administered to guinea pigs by being inhaled for 30 minutes using a compressor type nebulizer (NE-C13, manufactured by Omron Corporation) and a nose-only exposure system (manufactured by M·I·P·S Technologies). After 8 minutes from the completion of the administration by inhalation and after 1 hour from the same, airway contraction reaction was induced by inhalation of acetylcholine, and an airway contraction inhibiting ratio of the test compound at the respective times was calculated from the formula shown below.

Inhibiting ratio (%)=(1-average value of sRaw (AUC) of test compound-inhaled group/average value of sRaw (AUC) of phosphate-buffered saline-inhaled group)x100

[Table 5]

| Test compound Example No. | Inhibition of airway contraction (%) | |
|---|---|---|
| | After 8 minutes | After 1 hour |
| Example 1 | 100 | 91 |
| Example 51 | 91 | 90 |
| Example 54 | 81 | 85 |
| Compound A | 91 | 9 |

In the present test, compounds of the present invention showed excellent continuity in trachea contraction inhibiting action as compared to the control compound.

Preparation example

(Preparation example 1) (Hard capsules)

50 mg of the compound of Example 2, 128.7 mg of lactose, 70 mg of cellulose and 1.3 mg of magnesium stearate were mixed, the mixture was passed through 60 mesh sieve, and then, 250 mg of the powder was placed in No. 3 gelatin capsule to prepare a capsule.

(Preparation example 2) (Tablets)

50 mg of the compound of Example 2, 124 mg of lactose, 25 mg of cellulose and 1 mg of magnesium stearate were mixed, the mixture was tableted by a tableting machine to prepare a tablet with 200 mg per one tablet. Sugar coating can be applied to this tablet, if necessary.

UTILIZABILITY IN INDUSTRY

The aminopyridine compound represented by the formula (I) or a pharmaceutically acceptable salt thereof of the present invention demonstrates potent EP2 selective agonistic action, and also has superior properties in terms of tissue distribution, bioavailability (BA), fast-acting pharmaceutically effect, sustained pharmaceutically effect, solubility, physical stability, drug interaction, toxicity and the like. Thus, the present invention is able to provide a novel compound having superior properties as a therapeutic and/or prophylactic agent for respiratory diseases (such as asthma, COPD, bronchitis, emphysema, pulmonary fibrosis, acute respiratory distress syndrome (ARDS), cystic fibrosis and pulmonary hypertension) or eye diseases (in particular, glaucoma). Moreover, the compound represented by the formula (I) of the present invention is also useful as a therapeutic and/or prophylactic agent for diseases for which EP2 agonistic action

is thought to be useful (such as bone diseases, gastric ulcer, hypertension, etc).

## Claims

1. An aminopyridine compound represented by the formula (I):

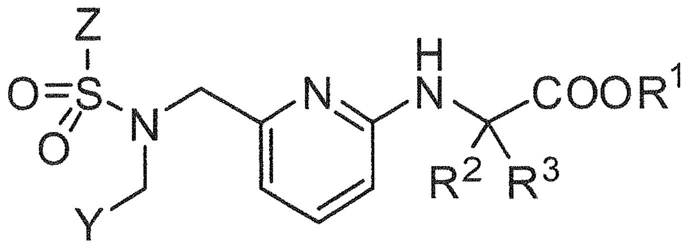

(I)

[wherein
$R^1$, $R^2$ and $R^3$ are each independently represent a hydrogen atom or a $C_1$-$C_6$ alkyl group,
Y represents a formula (II):

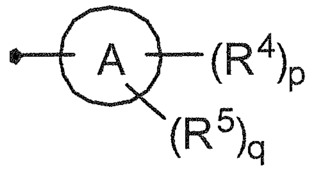

(II)

(wherein Ring A represents an aromatic ring group or a 5- to 6-membered heteroaromatic ring group, $R^4$ represents a $C_1$-$C_{12}$ alkyl group, a halogeno-$C_1$-$C_8$ alkyl group, a $C_1$-$C_6$ alkoxy group, a halogeno-$C_1$-$C_6$ alkoxy group, a halogeno-$C_1$-$C_6$ alkoxy-$C_1$-$C_6$ alkyl group, a $C_3$-$C_6$ cycloalkyl-$C_1$-$C_6$ alkyl group, a $C_2$-$C_6$ alkenyl group or the formula (III):

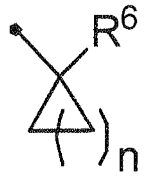

(III)

(wherein $R^6$ represents a $C_1$-$C_{12}$ alkyl group, a $C_1$-$C_6$ alkoxy group, a $C_7$-$C_{18}$ aralkyl group, a $C_1$-$C_6$ alkoxy-$C_1$-$C_6$ alkyl group or a $C_7$-$C_{18}$ aralkyloxy-$C_1$-$C_6$ alkyl group, and n is an integer of 1 to 4.), p is an integer of 1 to 3, $R^5$ represents a halogeno group or an amino group which may be substituted by a $C_1$-$C_6$ alkyl group, and q is an integer of 0 to 2.),
Z represents an aromatic ring group or a 5- to 6-membered heteroaromatic ring group each of which may be substituted by a group(s) selected from the group consisting of a halogeno group, a $C_1$-$C_6$ alkyl group, a halogeno-$C_1$-$C_6$ alkyl group, a $C_1$-$C_6$ alkoxy group and a halogeno-$C_1$-$C_6$ alkoxy group.]
or a pharmaceutically acceptable salt thereof.

2. The aminopyridine compound or a pharmaceutically acceptable salt thereof according to Claim 1, wherein $R^2$ and $R^3$ each independently represent a hydrogen atom or a $C_1$-$C_4$ alkyl group.

3. The aminopyridine compound or a pharmaceutically acceptable salt thereof according to Claim 1 or 2, wherein Y represents the formula (II):

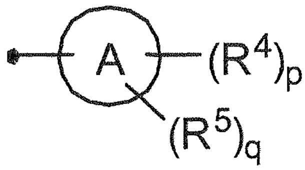

(II)

(wherein Ring A represents a phenyl group, a thienyl group, a thiazolyl group, a pyridyl group or a pyrimidinyl group, $R^4$ represents a $C_1$-$C_8$ alkyl group, a fluoro-$C_1$-$C_8$ alkyl group, a $C_1$-$C_4$ alkoxy group, a halogeno-$C_1$-$C_4$ alkoxy group, a fluoro-$C_1$-$C_4$ alkoxy-$C_3$-$C_6$ alkyl group, a $C_3$-$C_4$ cycloalkyl-$C_3$-$C_6$ alkyl group, a $C_3$-$C_6$ alkenyl group or the formula (III):

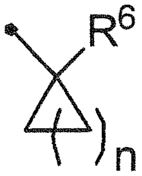

(III)

(wherein $R^6$ represents a $C_1$-$C_{10}$ alkyl group, a $C_1$-$C_4$ alkoxy group, a $C_7$-$C_{12}$ aralkyl group, a $C_1$-$C_4$ alkoxy-$C_1$-$C_4$ alkyl group or a $C_7$-$C_{12}$ aralkyloxy-$C_1$-$C_4$ alkyl group, and n is an integer of 1 to 3.), p is an integer of 1 or 2, $R^5$ represents a fluoro group, a chloro group or an amino group which may be substituted by a $C_1$-$C_3$ alkyl group(s), and q is an integer of 0 or 1.).

**4.** The aminopyridine compound or a pharmaceutically acceptable salt thereof according to any one of Claims 1 to 3, wherein Z represents a phenyl group, a furyl group, a thienyl group, an imidazolyl group, a thiazolyl group, a pyridyl group or a pyrimidinyl group each of which may be substituted by a group(s) selected from the group consisting of a halogeno group, a $C_1$-$C_4$ alkyl group, halogeno-$C_1$-$C_4$ alkyl group, a $C_1$-$C_4$ alkoxy group and a halogeno-$C_1$-$C_4$ alkoxy group.

**5.** The aminopyridine compound or a pharmaceutically acceptable salt thereof according to Claim 3, wherein Y represents the formula (II):

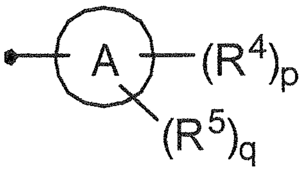

(II)

(wherein Ring A represents a phenyl group, a thienyl group, a thiazolyl group, a pyridyl group or a pyrimidinyl group, $R^4$ represents an ethyl group, a propyl group, an isopropyl group, a butyl group, a sec-butyl group, a tert-butyl group, a pentyl group, an isopentyl group, a neopentyl group, a tert-pentyl group, a 1-methylbutyl group, a 1-ethylpropyl group, a hexyl group, a 1-methylpentyl group, a 1-ethylbutyl group, a 1,1-dimethylbutyl group, a 2,2-dimethylbutyl group, a 1-ethyl-1-methylpropyl group, a 1-methylhexyl group, a 1,1-dimethylpentyl group, a 2,2-dimethylpentyl group, a 1,1-dimethylhexyl group, a 1,1,3,3-tetramethylbutyl group, a 1,1,4-trimethylpentyl group, a trifluoromethyl group, a 4,4,4-trifluorobutyl group, a 5,5,5-trifluoropentyl group, a 6,6,6-trifluorohexyl group, a 5,5,5-trifluoro-1-methylpentyl group, a 4,4,4-trifluoro-1,1-dimethylbutyl group, a 5,5,5-trifluoro-1,1-dimethylpentyl group, a 5,5,5-trifluoro-2,2-dimethylpentyl group, a propoxy group, a butoxy group, an isobutoxy group, a trifluoromethoxy group, a difluoromethoxy group, a trichloromethoxy group, a dichloromethoxy group, a 3-difluoromethoxypropyl group, a 3-difluoromethoxy-1-methylpropyl group, a 3-difluoromethoxy-1,1-dimethylpropyl group, a 4-difluoromethoxy-1,1-dimethylbutyl group, a 3-cyclopropylpropyl group, a 3-cyclopropyl-1-methylpropyl group, a 3-cyclopropyl-1,1-dimethylpropyl group, a 4-cyclopropyl-1,1-dimethylbutyl group, an isopropenyl group, a 1-methyl-1-propenyl group, a 1-methyl-1-butenyl group, a 1-methyl-1-pentenyl group or the formula (III):

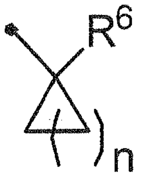

(III)

(wherein $R^6$ represents a methyl group, an ethyl group, a propyl group, an isopropyl group, a butyl group, an isobutyl group, a sec-butyl group, a tert-butyl group, a pentyl group, a hexyl group, a heptyl group, an octyl group, a nonyl group, a decyl group, a methoxy group, an ethoxy group, a propoxy group, an isopropoxy group, a benzyl group, a 1-phenylethyl group, a 2-phenylethyl group, a naphthalen-1-ylmethyl group, a naphthalen-2-ylmethyl group, an ethoxymethyl group, a propoxymethyl group, a butoxymethyl group, a 2-ethoxyethyl group, a 2-propoxyethyl group, a benzyloxymethyl group, a 2-benzyloxyethyl group, a 1-phenylethoxymethyl group, a 2-phenylethoxymethyl group, a 1-(1-phenylethoxy)ethyl group or a 2-(2-phenylethoxy)ethyl group, and n is an integer of 1 or 2.), p is an integer of 1 or 2, $R^5$ represents a fluoro group, a chloro group, an amino group, a methylamino group, a dimethylamino group or an ethylamino group, and q is an integer of 0 or 1.).

**6.** The aminopyridine compound or a pharmaceutically acceptable salt thereof according to Claim 3, wherein Y represents a 4-ethylphenyl group, a 3,4-diethylphenyl group, a 4-propylphenyl group, a 4-isopropylphenyl group, a 3-butylphenyl group, a 4-butylphenyl group, a 3-tert-butylphenyl group, a 4-tert-butylphenyl group, a 5-tert-butylthiophen-2-yl group, a 3-pentylphenyl group, a 4-pentylphenyl group, a 3-dimethylamino-4-pentylphenyl group, a 4-isopentylphenyl group, a 4-neopentylphenyl group, a 4-tert-pentylphenyl group, a 5-tert-pentylthiophen-2-yl group, a 4-(1-ethylpropyl)phenyl group, a 4-(1-methylbutyl)phenyl group, a 3-hexylphenyl group, a 4-(1-ethylbutyl)phenyl group, a 4-(1-methylpentyl)phenyl group, a 5-(1-methylpentyl)thiophen-2-yl group, a 3-(1,1-dimethylbutyl)phenyl group, a 4-(1,1-dimethylbutyl)phenyl group, a 2-fluoro-4-(1,1-dimethylbutyl)phenyl group, a 3-fluoro-4-(1,1-dimethylbutyl)-phenyl group, a 4-(2,2-dimethylbutyl)phenyl group, a 5-(1,1-dimethylbutyl)thiophen-2-yl group, a 4-(1-ethyl-1-methylpropyl)phenyl group, a 3-(1,1-dimethylpentyl)phenyl group, a 4-(1,1-dimethylpentyl)phenyl group, a 2-fluoro-4-(1,1-dimethylpentyl)phenyl group, a 3-fluoro-4-(1,1-dimethylpentyl)phenyl group, a 3-amino-4-(1,1-dimethylpentyl)phenyl group, a 3-methylamino-4-(1,1-dimethylpentyl)phenyl group, a 3-dimethylamino-4-(1,1-dimethylpentyl)phenyl group, a 4-(1,1-dimethylpentyl)thiophen-2-yl group, a 5-(1,1-dimethylpentyl)thiophen-2-yl group, a 5-(1,1-dimethylpentyl)-thiazol-2-yl group, a 5-(1,1-dimethylpentyl)pyridin-2-yl group, a 6-(1,1-dimethylpentyl)pyridin-3-yl group, a 5-(1,1-dimethylpentyl)pyrimidin-2-yl group, a 2-(1,1-dimethylpentyl)pyrimidin-5-yl group, a 3-(2,2-dimethylpentyl)phenyl group, a 4-(2,2-dimethylpentyl)phenyl group, a 4-(1-methylhexyl)phenyl group, a 4-(1,1,3,3-tetramethylbutyl)phenyl group, a 4-(1,1,4-trimethylpentyl)phenyl group, a 4-(1,1-dimethylhexyl)phenyl group, a 4-trifluoromethylphenyl group, a 4-(4,4,4-trifluorobutyl)phenyl group, a 4-(5,5,5-trifluoropentyl)phenyl group, a 4-(6,6,6-trifluorohexyl)phenyl group, a 4-(5,5,5-trifluoro-1-methylpentyl)phenyl group, a 4-(4,4,4-trifluoro-1,1-dimethylbutyl)-phenyl group, a 4-(5,5,5-trifluoro-1,1-dimethylpentyl)phenyl group, a 4-(5,5,5-trifluoro-2,2-dimethylpentyl)phenyl group, a 5-(5,5,5-trifluoro-1,1-dimethylpentyl)thiophen-2-yl group, a 4-propoxyphenyl group, a 4-butoxyphenyl group, a 4-isobutoxyphenyl group, a 4-difluoromethoxyphenyl group, a 4-trifluoromethoxyphenyl group, a 4-(3-difluoromethoxy-1-methylpropyl)phenyl group, a 4-(3-difluoromethoxy-1,1-dimethylpropyl)-phenyl group, a 4-(3-cyclopropyl-1-methylpropyl)phenyl group, a 4-(3-cyclopropyl-1,1-dimethylpropyl)phenyl group, a 4-isopropenylphenyl group, a 4-(1-methyl-1-propenyl)-phenyl group, a 4-(1-methyl-1-butenyl)phenyl group, a 4-(1-methyl-1-pentenyl)phenyl group, a 4-(1-methylcyclopropyl)phenyl group, a 4-(1-ethylcyclopropyl)phenyl group, a 4-(1-propylcyclopropyl)phenyl group, a 4-(1-isopropylcyclopropyl)phenyl group, a 3-(1-butylcyclopropyl)phenyl group, a 4-(1-butylcyclopropyl)phenyl group, a 2-fluoro-4-(1-butylcyclopropyl)phenyl group, a 3-fluoro-4-(1-butylcyclopropyl)phenyl group, a 3-dimethylamino-4-(1-butylcyclopropyl)phenyl group, a 5-(1-butylcyclopropyl)thiophen-2-yl group, a 4-(1-hexylcyclopropyl)phenyl group, a 4-(1-nonylcyclopropyl)phenyl group, a 4-(1-methoxycyclopropyl)phenyl group, a 4-(1-ethoxycyclopropyl)phenyl group, a 4-(1-propoxycyclopropyl)phenyl group, a 4-(1-benzylcyclopropyl)phenyl group, a 4-{1-(1-phenylethyl)cyclopropyl}phenyl group, a 4-{1-(2-phenylethyl)cyclopropyl}phenyl group, a 4-(1-ethoxymathylcyclopropyl)phenyl group, a 4-(1-propoxymethylcyclopropyl)phenyl group, a 5-(1-propoxymethylcyclopropyl)thiophen-2-yl group, a 4-{1-(2-ethoxyethyl)cyclopropyl}phenyl group, a 4-{1-(2-propoxyethyl)-cyclopropyl}phenyl group, a 4-(1-butoxymethylcyclopropyl)phenyl group, a 4-(1-benzyloxymethylcyclopropyl)phenyl group, a 4- {1-(2-benzyloxyethyl)cyclopropyl}-phenyl group, a 4-{1-(1-phenylethoxymethyl)cyclopropyl}phenyl group, a 4-{1-(2-phenylethoxymethyl)cyclopropyl}phenyl group, a 4-(1-ethylcyclobutyl)phenyl group, a 4-(1-butylcyclobutyl)phenyl group or a 5-(1-butylcyclobutyl)thiophen-2-yl group.

**7.** The aminopyridine compound or a pharmaceutically acceptable salt thereof according to Claim 3, wherein Y represents a 3,4-diethylphenyl group, a 3-butylphenyl group, a 4-butylphenyl group, a 3-tert-butylphenyl group, a 4-tert-butylphenyl group, a 5-tert-butylthiophen-2-yl group, a 4-pentylphenyl group, a 3-dimethylamino-4-pentylphenyl group, a 4-isopentylphenyl group, a 4-neopentylphenyl group, a 4-tert-pentylphenyl group, a 5-tert-pentylthiophen-2-yl group, a 4-(1-ethylpropyl)phenyl group, a 4-(1-methylbutyl)phenyl group, a 3-hexylphenyl group, a 4-(1-methylpentyl)phenyl group, a 5-(1-methylpentyl)thiophen-2-yl group, a 3-(1,1-dimethylbutyl)phenyl group, a 4-(1,1-dimethylbutyl)phenyl group, a 2-fluoro-4-(1,1-dimethylbutyl)phenyl group, a 3-fluoro-4-(1,1-dimethylbutyl)phenyl group, a 5-(1,1-dimethylbutyl)thiophen-2-yl group, a 4-(1-ethyl-1-methylpropyl)phenyl group, a 3-(1,1-dimethylpentyl)phenyl group, a 4-(1,1-dimethylpentyl)phenyl group, a 2-fluoro-4-(1,1-dimethylpentyl)phenyl group, a 3-fluoro-4-(1,1-dimethylpentyl)phenyl group, a 3-dimethylamino-4-(1,1-dimethylpentyl)-phenyl group, a 5-(1,1-dimethylpentyl)thiophen-2-yl group, a 4-(2,2-dimethylpentyl)-phenyl group, a 4-(1,1,3,3-tetramethylbutyl)phenyl group, a 4-(1,1,4-trimethylpentyl)-phenyl group, a 4-(1,1-dimethylhexyl)phenyl group, a 4-trifluoromethylphenyl group, a 4-(4,4,4-trifluoro-1,1-dimethylbutyl)phenyl group, a 4-(5,5,5-trifluoro-1,1-dimethylpentyl)phenyl group, a 5-(5,5,5-trifluoro-1,1-dimethylpentyl)thiophen-2-yl group, a 4-butoxyphenyl group, a 4-difluoromethoxyphenyl group, a 4-(3-difluoromethoxy-1,1-dimethylpropyl)phenyl group, a 4-(3-cyclopropyl-1,1-dimethylpropyl)phenyl group, a 4-(1-methyl-1-butenyl)phenyl group, a 4-(1-methyl-1-pentenyl)phenyl group, a 4-(1-methylcyclopropyl)phenyl group, a 4-(1-ethylcyclopropyl)phenyl group, a 4-(1-propylcyclopropyl)phenyl group, a 4-(1-isopropylcyclopropyl)phenyl group, a 4-(1-butylcyclopropyl)phenyl group, a 2-fluoro-4-(1-butylcyclopropyl)phenyl group, a 3-fluoro-4-(1-butylcyclopropyl)phenyl group, a 5-(1-butylcyclopropyl)thiophen-2-yl group, a 4-(1-nonylcyclopropyl)phenyl group, a 4-(1-methoxycyclopropyl)phenyl group, a 4-(1-benzylcyclopropyl)phenyl group, a 4-{1-(2-phenylethyl)cyclopropyl}-phenyl group, a 4-(1-propoxymethylcyclopropyl)phenyl group, a 5-(1-propoxymethylcyclopropyl)thiophen-2-yl group, a 4-(1-benzyloxymethylcyclopropyl)phenyl group, a 4-(1-ethylcyclobutyl)phenyl group or a 4-(1-butylcyclobutyl)phenyl group.

8. The aminopyridine compound or a pharmaceutically acceptable salt thereof according to Claim 3, wherein Y represents a 3,4-diethylphenyl group, a 3-butylphenyl group, a 3-tert-butylphenyl group, a 4-tert-butylphenyl group, a 4-pentylphenyl group, a 3-dimethylamino-4-pentylphenyl group, a 4-neopentylphenyl group, a 4-tert-pentylphenyl group, a 5-tert-pentylthiophen-2-yl group, a 4-(1-ethylpropyl)phenyl group, a 3-hexylphenyl group, a 4-(1-methylpentyl)phenyl group, a 4-(1,1-dimethylbutyl)phenyl group, a 4-(1-ethyl-1-methylpropyl)phenyl group, a 4-(1,1-dimethylpentyl)phenyl group, a 2-fluoro-4-(1,1-dimethylpentyl)phenyl group, a 3 -fluoro-4-(1,1-dimethylpentyl)phenyl group, a 5-(1,1-dimethylpentyl)thiophen-2-yl group, a 4-(2,2-dimethylpentyl)phenyl group, a 4-(1,1,3,3-tetramethylbutyl)phenyl group, a 4-(1,1,4-trimethylpentyl)phenyl group, a 4-(1,1-dimethylhexyl)phenyl group, a 4-trifluoromethylphenyl group, a 4-(5,5,5-trifluoro-1,1-dimethylpentyl)phenyl group, a 4-butoxyphenyl group, a 4-difluoromethoxyphenyl group, a 4-(3-difluoromethoxy-1,1-dimethylpropyl)phenyl group, a 4-(1-methyl-1-pentenyl)phenyl group, a 4-(1-methylcyclopropyl)phenyl group, a 4-(1-ethylcyclopropyl)phenyl group, a 4-(1-isopropylcyclopropyl)phenyl group, a 4-(1-butylcyclopropyl)phenyl group, a 4-(1-nonylcyclopropyl)phenyl group, a 4-(1-methoxycyclopropyl)phenyl group, a 4-(1-benzylcyclopropyl)phenyl group, a 4-(1-propoxymethylcyclopropyl)phenyl group, a 4-(1-benzyloxymethylcyclopropyl)phenyl group, a 4-(1-ethylcyclobutyl)phenyl group or a 4-(1-butylcyclobutyl)phenyl group.

9. The aminopyridine compound or a pharmaceutically acceptable salt thereof according to Claim 4, wherein Z represents a phenyl group, a furyl group, a thienyl group, an imidazolyl group, a thiazolyl group, a pyridyl group or a pyrimidinyl group each of which may be substituted by a group(s) selected from the group consisting of a fluoro group, a chloro group, a bromo group, a methyl group, an ethyl group, a propyl group, an isopropyl group, a tert-butyl group, a trifluoromethyl group, a difluoromethyl group, a trichloromethyl group, a dichloromethyl group, a 2,2,2-trifluoroethyl group, a 2,2,2-trichloroethyl group, a methoxy group, an ethoxy group, a propoxy group, an isopropoxy group, a trifluoromethoxy group, a difluoromethoxy group, a trichloromethoxy group and a dichloromethoxy group.

10. The aminopyridine compound or a pharmaceutically acceptable salt thereof according to Claim 4, wherein Z represents a phenyl group, a 2-fluorophenyl group, a 3-fluorophenyl group, a 4-fluorophenyl group, a 3,4-difluorophenyl group, a 3,5-difluorophenyl group, a 3,4,5-trifluorophenyl group, a 2-chlorophenyl group, a 3-chlorophenyl group, a 4-chlorophenyl group, a 2,6-dichlorophenyl group, a 4-chloro-3-fluorophenyl group, a 4-chloro-3,5-difluorophenyl group, a 4-bromophenyl group, a 4-methylphenyl group, a 3-fluoro-4-methylphenyl group, a 4-ethylphenyl group, a 4-ethyl-3-fluorophenyl group, a 4-propylphenyl group, a 4-isopropylphenyl group, a 4-tert-butylphenyl group, a 4-trifluoromethylphenyl group, a 3-fluoro-4-trifluoromethylphenyl group, a 4-difluoromethylphenyl group, a 4-trichloromethylphenyl group, a 4-dichloromethylphenyl group, a 4-(2,2,2-trifluoroethyl)phenyl group, a 4-(2,2,2-trichloroethyl)phenyl group, a 4-methoxyphenyl group, a 3-fluoro-4-methoxyphenyl group, a 4-ethoxyphenyl group, a 4-propoxyphenyl group, a 4-isopropoxyphenyl group, a 4-trifluoromethoxyphenyl group, a 4-difluoromethoxyphenyl group, a 4-difluoromethoxy-3-fluorophenyl group, a 4-trichloromethoxyphenyl group, a 4-dichloromethoxyphenyl group, a furan-2-yl group, a 5-methylfuran-2-yl group, a thiophen-2-yl group, a thiophen-3-yl group, a 5-chlorothiophen-2-yl

group, a 1-methyl-1H-imidazol-4-yl group, a thiazol-2-yl group, a pyridin-2-yl group, a 5-fluoropyridin-2-yl group, a 5-chloropyridin-2-yl group, a 5-methylpyridin-2-yl group, a 5-ethylpyridin-2-yl group, a 5-trifluoromethylpyridin-2-yl group, a 5-methoxypyridin-2-yl group, a 5-difluoromethoxypyridin-2-yl group, a pyridin-3-yl group, a 6-fluoropyridin-3-yl group, a 6-chloropyridin-3-yl group, a 6-methylpyridin-3-yl group, a 6-ethylpyridin-3-yl group, a 6-trifluoromethylpyridin-3-yl group, a 6-methoxypyridin-3-yl group, a 6-difluoromethoxypyridin-3-yl group, a pyridin-4-yl group or a pyrimidin-2-yl group.

**11.** The aminopyridine compound or a pharmaceutically acceptable salt thereof according to Claim 4, wherein Z represents a phenyl group, a 2-fluorophenyl group, a 3-fluorophenyl group, a 4-fluorophenyl group, a 3,4-difluorophenyl group, a 3,5-difluorophenyl group, a 2-chlorophenyl group, a 3-chlorophenyl group, a 4-chlorophenyl group, a 2,6-dichlorophenyl group, a 4-chloro-3-fluorophenyl group, a 4-methylphenyl group, a 3-fluoro-4-methylphenyl group, a 4-trifluoromethylphenyl group, a 3-fluoro-4-trifluoromethylphenyl group, a 4-methoxyphenyl group, a 3-fluoro-4-methoxyphenyl group, a 4-difluoromethoxyphenyl group, a 4-difluoromethoxy-3-fluorophenyl group, a furan-2-yl group, a 5-methylfuran-2-yl group, a thiophen-2-yl group, a thiophen-3-yl group, a 1-methyl-1H-imidazol-4-yl group, a thiazol-2-yl group, a pyridin-2-yl group, a 5-fluoropyridin-2-yl group, a 5-chloropyridin-2-yl group, a 5-methoxypyridin-2-yl group, a pyridin-3-yl group, a 6-fluoropyridin-3-yl group, a 6-chloropyridin-3-yl group, a 6-methoxypyridin-3-yl group or a pyridin-4-yl group.

**12.** The aminopyridine compound or a pharmaceutically acceptable salt thereof according to Claim 4, wherein Z represents a phenyl group, a 2-fluorophenyl group, a 3-fluorophenyl group, a 4-fluorophenyl group, a 4-chlorophenyl group, a 4-methoxyphenyl group, a furan-2-yl group, a 5-methylfuran-2-yl group, a thiophen-2-yl group, a 1-methyl-1H-imidazol-4-yl group, a thiazol-2-yl group, a pyridin-2-yl group, a pyridin-3-yl group or a pyridin-4-yl group.

**13.** The aminopyridine compound or a pharmaceutically acceptable salt thereof according to Claim 1, wherein $R^1$ represents a hydrogen atom, a methyl group, an ethyl group, a propyl group, an isopropyl group or a tert-butyl group,
$R^2$ and $R^3$ each independently represent a hydrogen atom or methyl group,
Y represents the formula (II):

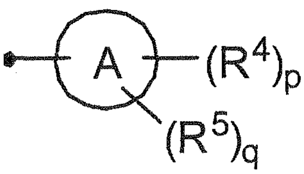 (II)

(wherein Ring A represents a phenyl group, a thienyl group, a thiazolyl group, a pyridyl group or a pyrimidinyl group, $R^4$ represents a $C_1$-$C_8$ alkyl group, a fluoro-$C_1$-$C_8$ alkyl group, a $C_1$-$C_4$ alkoxy group, a halogeno-$C_1$-$C_4$ alkoxy group, a fluoro-$C_1$-$C_4$ alkoxy-$C_3$-$C_6$ alkyl group, a $C_3$-$C_4$ cycloalkyl-$C_3$-$C_6$ alkyl group, a $C_3$-$C_6$ alkenyl group or the formula (III):

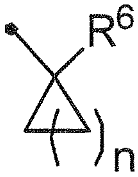 (III)

(wherein $R^6$ represents a $C_1$-$C_{10}$ alkyl group, a $C_1$-$C_4$ alkoxy group, a $C_7$-$C_{12}$ aralkyl group, a $C_1$-$C_4$ alkoxy-$C_1$-$C_4$ alkyl group or a $C_7$-$C_{12}$ aralkyloxy-$C_1$-$C_4$ alkyl group, and n is an integer of 1 to 3.), p is an integer of 1 or 2, $R^5$ represents a fluoro group, a chloro group or an amino group which may be substituted by a $C_1$-$C_3$ alkyl group, and q is an integer of 0 or 1.), and
Z represents a phenyl group, a furyl group, a thienyl group, an imidazolyl group, a thiazolyl group, a pyridyl group or a pyrimidinyl group each of which may be substituted by a group(s) selected from the group consisting of a halogeno group, a $C_1$-$C_4$ alkyl group, a halogeno-$C_1$-$C_4$ alkyl group, a $C_1$-$C_4$ alkoxy group and a halogeno-$C_1$-$C_4$ alkoxy group.

**14.** The aminopyridine compound or a pharmaceutically acceptable salt thereof according to Claim 1, wherein $R^1$ rep-

resents a a hydrogen atom, a methyl group, an ethyl group, a propyl group, an isopropyl group or a tert-butyl group, $R^2$ and $R^3$ each independently represent a hydrogen atom or a methyl group, Y represents the formula (II):

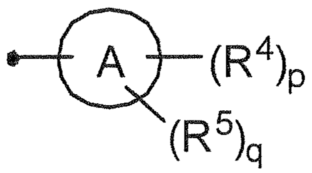

(II)

(wherein Ring A represents a phenyl group, a thienyl group, a thiazolyl group, a pyridyl group or a pyrimidinyl group, $R^4$ represents an ethyl group, a propyl group, an isopropyl group, a butyl group, a sec-butyl group, a tert-butyl group, a pentyl group, an isopentyl group, a neopentyl group, a tert-pentyl group, a 1-methylbutyl group, a 1-ethylpropyl group, a hexyl group, a 1-methylpentyl group, a 1-ethylbutyl group, a 1,1-dimethylbutyl group, a 2,2-dimethylbutyl group, a 1-ethyl-1-methylpropyl group, a 1-methylhexyl group, a 1,1-dimethylpentyl group, a 2,2-dimethylpentyl group, a 1,1-dimethylhexyl group, a 1,1,3,3-tetramethylbutyl group, a 1,1,4-trimethylpentyl group, a trifluoromethyl group, a 4,4,4-trifluorobutyl group, a 5,5,5-trifluoropentyl group, a 6,6,6-trifluorohexyl group, a 5,5,5-trifluoro-1-methylpentyl group, a 4,4,4-trifluoro-1,1-dimethylbutyl group, a 5,5,5-trifluoro-1,1-dimethylpentyl group, a 5,5,5-trifluoro-2,2-dimethylpentyl group, a propoxy group, a butoxy group, an isobutoxy group, a trifluoromethoxy group, a difluoromethoxy group, a trichloromethoxy group, a dichloromethoxy group, a 3-difluoromethoxypropyl group, a 3-difluoromethoxy-1-methylpropyl group, a 3-difluoromethoxy-1,1-dimethylpropyl group, a 4-difluoromethoxy-1,1-dimethylbutyl group, a 3-cyclopropylpropyl group, a 3-cyclopropyl-1-methylpropyl group, a 3-cyclopropyl-1,1-dimethylpropyl group, a 4-cyclopropyl-1,1-dimethylbutyl group, an isopropenyl group, a 1-methyl-1-propenyl group, a 1-methyl-1-butenyl group, a 1-methyl-1-pentenyl group or the formula (III):

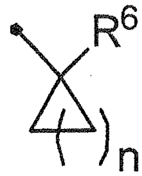

(III)

(wherein $R^6$ represents a methyl group, an ethyl group, a propyl group, an isopropyl group, a butyl group, an isobutyl group, a sec-butyl group, a tert-butyl group, a pentyl group, a hexyl group, a heptyl group, an octyl group, a nonyl group, a decyl group, a methoxy group, an ethoxy group, a propoxy group, an isopropoxy group, a benzyl group, a 1-phenylethyl group, a 2-phenylethyl group, a naphthalen-1-ylmethyl group, a naphthalen-2-ylmethyl group, an ethoxymethyl group, a propoxymethyl group, a butoxymethyl group, a 2-ethoxyethyl group, a 2-propoxyethyl group, a benzyloxymethyl group, a 2-benzyloxyethyl group, a 1-phenylethoxymethyl group, a 2-phenylethoxymethyl group, a 1-(1-phenylethoxy)ethyl group or a 2-(2-phenylethoxy)ethyl group, and n is an integer of 1 or 2.), p is an integer of 1 or 2, $R^5$ represents a fluoro group, a chloro group, an amino group, a methylamino group, a dimethylamino group or an ethylamino group, and q is an integer of 0 or 1.),

Z represents a phenyl group, a furyl group, a thienyl group, an imidazolyl group, a thiazolyl group, a pyridyl group or a pyrimidinyl group each of which may be substituted by a group(s) selected from the group consisting of a fluoro group, a chloro group, a bromo group, a methyl group, an ethyl group, a propyl group, an isopropyl group, a tert-butyl group, a trifluoromethyl group, a difluoromethyl group, a trichloromethyl group, a dichloromethyl group, a 2,2,2-trifluoroethyl group, a 2,2,2-trichloroethyl group, a methoxy group, an ethoxy group, a propoxy group, an isopropoxy group, a trifluoromethoxy group, a difluoromethoxy group, a trichloromethoxy group and a dichloromethoxy group.

**15.** The aminopyridine compound or a pharmaceutically acceptable salt thereof according to Claim 1, wherein $R^1$ represents a hydrogen atom, methyl group, an ethyl group or an isopropyl group,
$R^2$ and $R^3$ are both hydrogen atom,
Y represents a 4-ethylphenyl group, a 3,4-diethylphenyl group, a 4-propylphenyl group, a 4-isopropylphenyl group, a 3-butylphenyl group, a 4-butylphenyl group, a 3-tert-butylphenyl group, a 4-tert-butylphenyl group, a 5-tert-butylthiophen-2-yl group, a 3-pentylphenyl group, a 4-pentylphenyl group, a 3-dimethylamino-4-pentylphenyl group, a 4-isopentylphenyl group, a 4-neopentylphenyl group, a 4-tert-pentylphenyl group, a 5-tert-pentylthiophen-2-yl group, a 4-(1-ethylpropyl)phenyl group, a 4-(1-methylbutyl)phenyl group, a 3-hexylphenyl group, a 4-(1-ethylbutyl)phenyl group, a 4-(1-methylpentyl)phenyl group, a 5-(1-methylpentyl)thiophen-2-yl group, a 3-(1,1-dimethylbutyl)phenyl

group, a 4-(1,1-dimethylbutyl)phenyl group, a 2-fluoro-4-(1,1-dimethylbutyl)phenyl group, a 3-fluoro-4-(1,1-dimethylbutyl)phenyl group, a 4-(2,2-dimethylbutyl)phenyl group, a 5-(1,1-dimethylbutyl)thiophen-2-yl group, a 4-(1-ethyl-1-methylpropyl)phenyl group, a 3-(1,1-dimethylpentyl)phenyl group, a 4-(1, 1-dimethylpentyl)phenyl group, a 2-fluoro-4-(1, 1-dimethylpentyl)phenyl group, a 3-fluoro-4-(1,1-dimethylpentyl)phenyl group, a 3-amino-4-(1,1-dimethylpentyl)phenyl group, a 3-methylamino-4-(1,1-dimethylpentyl)phenyl group, a 3-dimethylamino-4-(1,1-dimethylpentyl)phenyl group, a 4-(1,1-dimethylpentyl)thiophen-2-yl group, a 5-(1,1-dimethylpentyl)thiophen-2-yl group, a 5-(1,1-dimethylpentyl)thiazol-2-yl group, a 5-(1,1-dimethylpentyl)pyridin-2-yl group, a 6-(1,1-dimethylpentyl)pyridin-3-yl group, a 5-(1,1-dimethylpentyl)pyrimidin-2-yl group, a 2-(1,1-dimethylpentyl)pyrimidin-5-yl group, a 3-(2,2-dimethylpentyl)phenyl group, a 4-(2,2-dimethylpentyl)phenyl group, a 4-(1-methylhexyl)phenyl group, a 4-(1,1,3,3-tetramethylbutyl)phenyl group, a 4-(1,1,4-trimethylpentyl)phenyl group, a 4-(1,1-dimethylhexyl)phenyl group, a 4-trifluoromethylphenyl group, a 4-(4,4,4-trifluorobutyl)phenyl group, a 4-(5,5,5-trifluoropentyl)-phenyl group, a 4-(6,6,6-trifluorohexyl)phenyl group, a 4-(5,5,5-trifluoro-1-methylpentyl)phenyl group, a 4-(4,4,4-trifluoro-1,1-dimethylbutyl)phenyl group, a 4-(5,5,5-trifluoro-1,1-dimethylpentyl)phenyl group, a 4-(5,5,5-trifluoro-2,2-dimethylpentyl)-phenyl group, a 5-(5,5,5-trifluoro-1,1-dimethylpentyl)thiophen-2-yl group, a 4-propoxyphenyl group, a 4-butoxyphenyl group, a 4-isobutoxyphenyl group, a 4-difluoromethoxyphenyl group, a 4-trifluoromethoxyphenyl group, a 4-(3-difluoromethoxy-1-methylpropyl)phenyl group, a 4-(3-difluoromethoxy-1,1-dimethylpropyl)phenyl group, a 4-(3-cyclopropyl-1-methylpropyl)phenyl group, a 4-(3-cyclopropyl-1,1-dimethylpropyl)phenyl group, a 4-isopropenylphenyl group, a 4-(1-methyl-1-propenyl)phenyl group, a 4-(1-methyl-1-butenyl)phenyl group, a 4-(1-methyl-1-pentenyl)phenyl group, a 4-(1-methylcyclopropyl)phenyl group, a 4-(1-ethylcyclopropyl)phenyl group, a 4-(1-propylcyclopropyl)phenyl group, a 4-(1-isopropylcyclopropyl)phenyl group, a 3-(1-butylcyclopropyl)phenyl group, a 4-(1-butylcyclopropyl)phenyl group, a 2-fluoro-4-(1-butylcyclopropyl)phenyl group, a 3-fluoro-4-(1-butylcyclopropyl)phenyl group, a 3-dimethylamino-4-(1-butylcyclopropyl)phenyl group, a 5-(1-butylcyclopropyl)thiophen-2-yl group, a 4-(1-hexylcyclopropyl)phenyl group, a 4-(1-nonylcyclopropyl)phenyl group, a 4-(1-methoxycyclopropyl)phenyl group, a 4-(1-ethoxycyclopropyl)phenyl group, a 4-(1-propoxycyclopropyl)phenyl group, a 4-(1-benzylcyclopropyl)phenyl group, a 4-{1-(1-phenylethyl)cyclopropyl}phenyl group, a 4-{1-(2-phenylethyl)cyclopropyl}phenyl group, a 4-(1-ethoxymethylcyclopropyl)phenyl group, a 4-(1-propoxymethylcyclopropyl)phenyl group, a 5-(1-propoxymethylcyclopropyl)thiophen-2-yl group, a 4-{1-(2-ethoxyethyl)cyclopropyl}phenyl group, a 4-{1-(2-propoxyethyl)-cyclopropyl}phenyl group, a 4-(1-butoxymethylcyclopropyl)phenyl group, a 4-(1-benzyloxymethylcyclopropyl)phenyl group, a 4-{1-(2-benzyloxyethyl)cyclopropyl}-phenyl group, a 4-{1-(1-phenylethoxymethyl)cyclopropyl}phenyl group, a 4- {1-(2-phenylethoxymethyl)cyclopropyl}phenyl group, a 4-(1-ethylcyclobutyl)phenyl group, a 4-(1-butylcyclobutyl)phenyl group or a 5-(1-butylcyclobutyl)thiophen-2-yl group,
Z represents a phenyl group, a 2-fluorophenyl group, a 3-fluorophenyl group, a 4-fluorophenyl group, a 3,4-difluorophenyl group, a 3,5-difluorophenyl group, a 3,4,5-trifluorophenyl group, a 2-chlorophenyl group, a 3-chlorophenyl group, a 4-chlorophenyl group, a 2,6-dichlorophenyl group, a 4-chloro-3-fluorophenyl group, a 4-chloro-3,5-difluorophenyl group, a 4-bromophenyl group, a 4-methylphenyl group, a 3-fluoro-4-methylphenyl group, a 4-ethylphenyl group, a 4-ethyl-3-fluorophenyl group, a 4-propylphenyl group, a 4-isopropylphenyl group, a 4-tert-butylphenyl group, a 4-trifluoromethylphenyl group, a 3-fluoro-4-trifluoromethylphenyl group, a 4-difluoromethylphenyl group, a 4-trichloromethylphenyl group, a 4-dichloromethylphenyl group, a 4-(2,2,2-trifluoroethyl)phenyl group, a 4-(2,2,2-trichloroethyl)phenyl group, a 4-methoxyphenyl group, a 3-fluoro-4-methoxyphenyl group, a 4-ethoxyphenyl group, a 4-propoxyphenyl group, a 4-isopropoxyphenyl group, a 4-trifluoromethoxyphenyl group, a 4-difluoromethoxyphenyl group, a 4-difluoromethoxy-3-fluorophenyl group, a 4-trichloromethoxyphenyl group, a 4-dichloromethoxyphenyl group, a furan-2-yl group, a 5-methylfuran-2-yl group, a thiophen-2-yl group, a thiophen-3-yl group, a 5-chlorothiophen-2-yl group, a 1-methyl-1H-imidazol-4-yl group, a thiazol-2-yl group, a pyridin-2-yl group, a 5-fluoropyridin-2-yl group, a 5-chloropyridin-2-yl group, a 5-methylpyridin-2-yl group, a 5-ethylpyridin-2-yl group, a 5-trifluoromethylpyridin-2-yl group, a 5-methoxypyridin-2-yl group, a 5-difluoromethoxypyridin-2-yl group, a pyridin-3-yl group, a 6-fluoropyridin-3-yl group, a 6-chloropyridin-3-yl group, a 6-methylpyridin-3-yl group, a 6-ethylpyridin-3-yl group, a 6-trifluoromethylpyridin-3-yl group, a 6-methoxypyridin-3-yl group, a 6-difluoromethoxypyridin-3-yl group, a pyridin-4-yl group or a pyrimidin-2-yl group.

**16.** The aminopyridine compound or a pharmaceutically acceptable salt thereof according to Claim 1, wherein $R^1$ represents a hydrogen atom, a methyl group, an ethyl group or an isopropyl group,
$R^2$ and $R^3$ are both hydrogen atom,
Y represents a 3,4-diethylphenyl group, a 3-butylphenyl group, a 4-butylphenyl group, a 3-tert-butylphenyl group, a 4-tert-butylphenyl group, a 5-tert-butylthiophen-2-yl group, a 4-pentylphenyl group, a 3-dimethylamino-4-pentylphenyl group, a 4-isopentylphenyl group, a 4-neopentylphenyl group, a 4-tert-pentylphenyl group, a 5-tert-pentylthiophen-2-yl group, a 4-(1-ethylpropyl)phenyl group, a 4-{1-methylbutyl}phenyl group, a 3-hexylphenyl group, a 4-(1-methylpentyl)phenyl group, a 5-(1-methylpentyl)-thiophen-2-yl group, a 3-(1,1-dimethylbutyl)phenyl group, a 4-(1,1-dimethylbutyl)-phenyl group, a 2-fluoro-4-(1,1-dimethylbutyl)phenyl group, a 3-fluoro-4-(1,1-dimethylbutyl)phenyl group, a 5-(1,1-dimethylbutyl)thiophen-2-yl group, a 4-(1-ethyl-1-methylpropyl)phenyl group, a 3-(1,1-dimethylpentyl)phenyl group, a 4-(1,1-dimethylpentyl)phenyl group, a 2-fluoro-4-(1,1-dimethylpentyl)phenyl group, a 3-fluoro-4-(1,1-dimethylpentyl)phenyl group, a 3-dimethylamino-4-(1,1-dimethylpentyl)phenyl group, a 5-(1,1-dimethylpentyl)thiophen-2-yl group, a 4-(2,2-dimethylpentyl)phenyl group, a 4-(1,1,3,3-tetramethylbutyl)phenyl group, a 4-(1,1,4-trimethylpentyl)phenyl group, a 4-(1,1-dimethylhexyl)phenyl group, a 4-trifluoromethylphenyl group, a 4-(4,4,4-trifluoro-1,1-dimethylbutyl)phenyl group, a 4-(5,5,5-trifluoro-1,1-dimethylpentyl)phenyl group, a 5-(5,5,5-trifluoro-1,1-dimethylpentyl)thiophen-2-yl group, a 4-butoxyphenyl group, a 4-difluoromethoxyphenyl group, a 4-(3-difluoromethoxy-1,1-dimethylpropyl)phenyl group, a 4-(3-cyclopropyl-1,1-dimethylpropyl)phenyl group, a 4-(1-methyl-1-butenyl)-phenyl group, a 4-(1-methyl-1-pentenyl)phenyl group, a 4-(1-methylcyclopropyl)phenyl group, a 4-(1-ethylcyclopropyl)phenyl group, a 4-(1-propylcyclopropyl)phenyl group, a 4-(1-isopropylcyclopropyl)phenyl group, a 4-(1-butylcyclopropyl)phenyl group, a 2-fluoro-4-(1-butylcyclopropyl)phenyl group, a 3-fluoro-4-(1-butylcyclopropyl)phenyl group, a 5-(1-butylcyclopropyl)thiophen-2-yl group, a 4-(1-nonylcyclopropyl)phenyl group, a 4-(1-methoxycyclopropyl)phenyl group, a 4-(1-benzylcyclopropyl)phenyl group, a 4-{1-(2-phenylethyl)cyclopropyl}phenyl group, a 4-(1-propoxymethylcyclopropyl)phenyl group, a 5-(1-propoxymethylcyclopropyl)thiophen-2-yl group, a 4-(1-benzyloxymethylcyclopropyl)phenyl group, a 4-(1-ethylcyclobutyl)phenyl group or a 4-(1-butylcyclobutyl)phenyl group,

Z represents a phenyl group, a 2-fluorophenyl group, a 3-fluorophenyl group, a 4-fluorophenyl group, a 3,4-difluorophenyl group, a 3,5-difluorophenyl group, a 2-chlorophenyl group, a 3-chlorophenyl group, a 4-chlorophenyl group, a 2,6-dichlorophenyl group, a 4-chloro-3-fluorophenyl group, a 4-methylphenyl group, a 3-fluoro-4-methylphenyl group, a 4-trifluoromethylphenyl group, a 3-fluoro-4-trifluoromethylphenyl group, a 4-methoxyphenyl group, a 3-fluoro-4-methoxyphenyl group, a 4-difluoromethoxyphenyl group, a 4-difluoromethoxy-3-fluorophenyl group, a furan-2-yl group, a 5-methylfuran-2-yl group, a thiophen-2-yl group, a thiophen-3-yl group, a 1-methyl-1H-imidazol-4-yl group, a thiazol-2-yl group, a pyridin-2-yl group, a 5-fluoropyridin-2-yl group, a 5-chloropyridin-2-yl group, a 5-methoxypyridin-2-yl group, a pyridin-3-yl group, a 6-fluoropyridin-3-yl group, a 6-chloropyridin-3-yl group, a 6-methoxypyridin-3-yl group or a pyridin-4-yl group.

**17.** The aminopyridine compound or a pharmaceutically acceptable salt thereof according to Claim 1, wherein $R^1$ represents a hydrogen atom, a methyl group, an ethyl group or an isopropyl group,
$R^2$ and $R^3$ are both hydrogen atom,
Y represents a 3,4-diethylphenyl group, a 3-butylphenyl group, a 3-tert-butylphenyl group, a 4-tert-butylphenyl group, a 4-pentylphenyl group, a 3-dimethylamino-4-pentylphenyl group, a 4-neopentylphenyl group, a 4-tert-pentylphenyl group, a 5-tert-pentylthiophen-2-yl group, a 4-(1-ethylpropyl)phenyl group, a 3-hexylphenyl group, a 4-(1-methylpentyl)phenyl group, a 4-(1,1-dimethylbutyl)phenyl group, a 4-(1-ethyl-1-methylpropyl)phenyl group, a 4-(1,1-dimethylpentyl)phenyl group, a 2-fluoro-4-(1,1-dimethylpentyl)phenyl group, a 3-fluoro-4-(1,1-dimethylpentyl)phenyl group, a 5-(1,1-dimethylpentyl)thiophen-2-yl group, a 4-(2,2-dimethylpentyl)phenyl group, a 4-(1,1,3,3-tetramethylbutyl)phenyl group, a 4-(1,1,4-trimethylpentyl)phenyl group, a 4-(1,1-dimethylhexyl)phenyl group, a 4-trifluoromethylphenyl group, a 4-(5,5,5-trifluoro-1,1-dimethylpentyl)phenyl group, a 4-butoxyphenyl group, a 4-difluoromethoxyphenyl group, a 4-(3-difluoromethoxy-1,1-dimethylpropyl)phenyl group, a 4-(1-methyl-1-pentenyl)phenyl group, a 4-(1-methylcyclopropyl)phenyl group, a 4-(1-ethylcyclopropyl)phenyl group, a 4-(1-isopropylcyclopropyl)phenyl group, a 4-(1-butylcyclopropyl)phenyl group, a 4-(1-nonylcyclopropyl)phenyl group, a 4-(1-methoxycyclopropyl)phenyl group, a 4-(1-benzylcyclopropyl)phenyl group, a 4-(1-propoxymethylcyclopropyl)phenyl group, a 4-(1-benzyloxymethylcyclopropyl)phenyl group, a 4-(1-ethylcyclobutyl)phenyl group or a 4-(2-hutylcyclobutyl)phenyl group,
Z represents a phenyl group, a 2-fluorophenyl group, a 3-fluorophenyl group, a 4-fluorophenyl group, a 4-chlorophenyl group, a 4-methoxyphenyl group, a furan-2-yl group, a 5-methylfuran-2-yl group, a thiophen-2-yl group, a 1-methyl-1H-imidazol-4-yl group, a thiazol-2-yl group, a pyridin-2-yl group, a pyridin-3-yl group or a pyridin-4-yl group.

**18.** The aminopyridine compound or a pharmaceutically acceptable salt thereof according to Claim 1, wherein the aminopyridine compound is {6-[(3,4-diethylbenzyl)(pyridin-2-ylsulfonyl)aminomethyl]pyridin-2-ylamino}acetic acid,
{6-[(3-butylbenzyl){pyridin-2-ylsulfonyl)aminomethyl]pyridin-2-ylamino} acetic acid,
{6-[(3-tert-butylbenzyl)(pyridin-3-ylsulfonyl)aminomethyl]pyridin-2-ylaminol}acetic acid,
{6-[(benzenesulfonyl)(4-tert-butylbenzyl)aminomethyl]pyridin-2-ylamino} acetic acid,
{6-[(4-tert-butylbenzyl)(2-fluorobenzenesulfonyl)aminomethyl]pyridin-2-ylamino}-acetic acid,
{6-[(4-tert-butylbenzyl)(3-fluorobenzenesulfonyl)aminomethyl]pyridin-2-ylamino}-acetic acid,
{6-[(4-tert-butylbenzyl)(4-fluorobenzenesulfonyl)aminomethyl]pyridin-2-ylamino}-acetic acid,
{6-[(4-tert-butylbenzyl)(4-chlorobenzenesulfonyl)aminomethyl]pyridin-2-ylamino}-acetic acid,
{6-[(4-tert-butylbenzyl)(4-methoxybenzenesulfonyl)aminomethyl]pyridin-2-ylamino}-acetic acid,
{6-[(4-tert-butylbenzyl)(pyridin-2-ylsulfonyl)aminomethyl]pyridin-2-ylamino}acetic acid,
{6-[(4-tert-butylbenzyl)(pyridin-3-ylsulfonyl)aminomethyl]pyridin-2-ylamino} acetic acid,

{6-[(4-pentylbenzyl)(pyridin-2-ylsulfonyl)aminomethyl]pyridin-2-ylamino}acetic acid,
{6-[(3-dimethylamino-4-pentylbenzyl)(pyridin-2-ylsulfonyl)aminomethyl]pyridin-2-ylamino}acetic acid,
{6-[(4-neopentylbenzyl)(pyridin-2-ylsulfonyl)aminomethyl]pyridin-2-ylamino}acetic acid,
{6-[(4-tert-pentylbenzyl)(pyridin-2-ylsulfonyl)aminomethyl]pyridin-2-ylamino}acetic acid,
{6-[(5-tert-pentylthiophen-2-ylmethyl)(pyridin-2-ylsulfonyl)aminomethyl]pyridin-2-ylamino} acetic acid,
(6-{[4-(1-ethylpropyl)benzyl](pyridin-3-ylsulfonyl)aminomethyl}pyridin-2-ylamino)-acetic acid,
{6-[(3-hexylbenzyl)(pyridin-2-ylsulfonyl)ammomethyl]pyridin-2-ylamino} acetic acid,
(6-{[4-(1-methylpentyl)benzyl](pyridin-2-ylsulfonyl)aminomethyl} pyridin-2-ylamino)-acetic acid,
(6-{[4-(1,1-dimethylbutyl)benzyl]{pyridin-2-ylsulfonyl)aminomethyl}pyridin-2-ylamino)acetic acid,
(6-{[4-(1-ethyl-1-methylpropyl)benzyl](pyridin-3-ylsulfonyl)aminomethyl}pyridin-2-ylamino)acetic acid,
(6-{(benzenesulfonyl)[4-(1,1-dimethylpentyl)benzyl]aminomethyl}pyridin-2-ylamino)-acetic acid,
(6-{(2-fluorobenzenesulfonyl)[4-(1,1-dimethylpentyl)benzyl]aminomethyl}pyridin-2-ylamino)acetic acid,
(6-{(3-fluorobenzenesulfonyl)[4-(1,1-dimethylpentyl)benzyl]aminomethyl}pyridin-2-ylamino)acetic acid,
(6-{(4-fluorobenzenesulfonyl)[4-(1,1-dimethylpentyl)benzyl]aminomethyl}pyridin-2-ylamino)acetic acid,
(6-{(4-chlorobenzenesulfonyl)[4-(1,1-dimethylpentyl)benzyl]aminomethyl}pyridin-2-ylamino)acetic acid,
(6-{(4-methoxybenzenesulfonyl)[4-(1,1-dimethylpentyl)benzyl]aminomethyl}pyridin-2-ylamino)acetic acid,
(6-{(furan-2-ylsulfonyl)[4-(1,1-dimethylpentyl)benzyl]aminomethyl}pyridin-2-ylamino)acetic acid,
(6-{[4-(1,1-dimethylpentyl)benzyl](thiophen-2-ylsulfonyl)aminomethyl}pyridin-2-ylamino)acetic acid,
(6-{[4-(1,1-dimethylpentyl)benzyl](thiazol-2-ylsulfonyl)aminomethyl}pyridin-2-ylamino)acetic acid,
(6-{[4-(1,1-dimethylpentyl)benzyl](pyridin-2-ylsulfonyl)aminomethyl}pyridin-2-ylamino)acetic acid,
(6-{[4-(1,1-dimethylpentyl)benzyl](pyridin-3-ylsulfonyl)aminomethyl}pyridin-2-ylamino)acetic acid,
(6-{[4-(1,1-dimethylpentyl)benzyl](pyridin-4-ylsulfonyl)aminomethyl}pyridin-2-ylamino)acetic acid,
(6-{[2-fluoro-4-(1,1-dimethylpentyl)benzyl](pyridin-2-ylsulfonyl)aminomethyl}-pyridin-2-ylamino)acetic acid,
(6-{[3-fluoro-4-(1,1-dimethylpentyl)benzyl](pyridin-2-ylsulfonyl)aminomethyl}-pyridin-2-ylamino)acetic acid,
(6-{(4-fluorobenzenesulfonyl)[5-(1,1-dimethylpentyl)thiophen-2-ylmethyl]aminomethyl}pyridin-2-ylamino)acetic acid,
(6-{[5-(1,1-dimethylpentyl)thiophen-2-ylmethyl](pyridin-2-ylsulfonyl)aminomethyl}-pyridin-2-ylamino)acetic acid,
(6-{[5-(1,1-dimethylpentyl)thiophen-2-ylmethyl](pyridin-3-ylsulfonyl)aminomethyl}-pyridin-2-ylamino)acetic acid,
(6-{[4-(2,2-dimethylpentyl)benzyl](pyridin-2-ylsulfonyl)aminomethyl}pyridin-2-ylamino)acetic acid,
(6-{[4-(1,1,3,3-tetramethylbutyl)benzyl](pyridin-2-ylsulfonyl)aminomethyl}pyridin-2-ylamino)acetic acid,
(6-{[4-(1,1,4-trimethylpentyl)benzyl](pyridin-2-ylsulfonyl)aminomethyl}pyridin-2-ylamino)acetic acid,
(6-{[4-(1,1-dimethylhexyl)benzyl](pyridin-2-ylsulfonyl)aminomethyl}pyridin-2-ylamino)acetic acid,
{6-[(pyridin-3-ylsulfonyl)(4-trifluoromethylbenzyl)aminomethyl]pyridin-2-ylamino}-acetic acid,
(6-{[4-(5,5,5-trifluoro-1,1-dimethylpentyl)benzyl](pyridin-2-ylsulfonyl)aminomethyl}-pyridin-2-ylamino)acetic acid,
{6-[(4-butoxybenzyl)(pyridin-2-ylsulfonyl)aminomethyl]pyridin-2-ylaminoacetic acid,
{6-[(4-difluoromethoxybenzyl)(pyridin-3-ylsulfonyl)aminomethyl]pyridin-2-ylamino}-acetic acid,
(6-{[4-(3-difluoromethoxy-1,1-dimethylpropyl)benzyl]{pyridin-2-ylsulfonyl)aminomethyl}pyridin-2-ylamino)acetic acid,
(6-{[4-(3-difluoromethoxy-1,1-dimethylpropyl)benzyl](pyridin-2-ylsulfonyl)aminomethyl}pyridin-2-ylamino)ethyl acetate,
(6-{[4-(1-methyl-1-pentenyl)benzyl](pyridin-2-ylsulfonyl)aminomethyl}pyridin-2-ylamino)acetic acid,
{6-{[4-(1-methylcyclopropyl)benzyl](pyridin-3-ylsulfonyl)aminomethyl}pyridin-2-ylamino)acetic acid,
(6-{[4-(1-ethylcyclopropyl)benzyl](4-fluorobenzenesulfonyl)aminomethyl}pyridin-2-ylamino)acetic acid,
(6-{[4-(1-ethylcyclopropyl)benzyl](pyridin-2-ylsulfonyl)aminomethyl}pyridin-2-ylamino)acetic acid,
(6-{[4-(1-ethylcyclopropyl)benzyl](pyridin-3-ylsulfonyl)aminomethyl}pyridin-2-ylamino)acetic acid,
(6-{[4-(1-isopropylcyclopropyl)benzyl](pyridin-3-ylsulfonyl)aminomethyl}pyridin-2-ylamino)acetic acid,
(6-{[4-(1-butylcyclopropyl)benzyl](1-methyl-1H-imidazol-4-ylsulfonyl)aminomethyl}-pyridin-2-ylamino)acetic acid,
(6-{[4-(1-butylcyclopropyl)benzyl](pyridin-2-ylsulfonyl)aminomethyl}pyridin-2-ylamino)acetic acid,
(6-{[4-(1-butylcyclopropyl)benzyl](pyridin-3-ylsulfonyl)aminomethyl}pyridin-2-ylamino)acetic acid,
(6-{[4-(1-butylcyclopropyl)benzyl](pyridin-4-ylsulfonyl)aminomethyl}pyridin-2-ylamino)acetic acid,
(6-{[4-(1-nonylcyclopropyl)benzyl]{pyridin-3-ylsulfonyl)aminomethyl}pyridin-2-ylamino)acetic acid,
(6-{[4-(1-methoxycyclopropyl)benzyl](pyridin-2-ylsulfonyl)aminomethyl}pyridin-2-ylamino)acetic acid,
(6-{[4-(1-benzylcyclopropyl)benzyl](pyridin-2-ylsulfonyl)aminomethyl}pyridin-2-ylamino)acetic acid,
(6-[4-(1-propoxymethylcyclopropyl)benzyl](pyridin-2-ylsulfonyl)aminomethyl}-pyridin-2-ylamino)acetic acid,
(6-{[4-(1-benzyloxymethylcyclopropyl)benzyl](pyridin-2-ylsulfonyl)aminomethyl} - pyridin-2-ylamino)acetic acid,
(6-{[4-(1-ethylcyclobutyl)benzyl](pyridin-3-ylsulfonyl)aminomethyl}pyridin-2-ylamino)acetic acid,
(6-{[4-(1-butylcyclobutyl)benzyl](pyridin-2-ylsulfonyl)aminomethyl}pyridin-2-ylamino)acetic acid, or
(6-{(5-methylfuran-2-ylsulfonyl)[4-(1,1-dimethylpentyl)benzyl]aminomethyl}pyridin-2-ylamino)acetic acid.

**19.** A medical composition comprising the aminopyridine compound or a pharmaceutically acceptable salt thereof according to any one of Claims 1 to 18 as an effective ingredient.

**20.** The medical composition according to Claim 19, which is for the prophylaxis or treatment of respiratory diseases.

**21.** The medical composition according to Claim 19, which is for the prophylaxis or treatment of glaucoma.

**INTERNATIONAL SEARCH REPORT**

International application No.
PCT/JP2010/073274

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D213/74, A61K31/44, A61K31/4427, A61K31/4436, A61K31/4439, A61K31/444, A61P11/00, A61P11/06, A61P11/08, A61P27/06, A61P43/00, C07D401/12, C07D405/12, C07D409/12, C07D409/14, C07D417/12

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | WO 2009/113600 A1 (Ube Industries, Ltd.), 17 September 2009 (17.09.2009), entire text; particularly, claims; examples; test examples & AU 2009224329 A | 1-21 |
| Y | JP 2006-519250 A (Pfizer Products Inc.), 24 August 2006 (24.08.2006), entire text; particularly, claims; examples & WO 2004/078169 A1 & EP 1601351 A1 & CA 2518193 A & NZ 541828 A & KR 10-2005-0105511 A & CN 1859903 A & PL 378748 A & CL 4122004 A & ZA 200506532 A | 1-21 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 March, 2011 (03.03.11) | 15 March, 2011 (15.03.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2010/073274

C (Continuation). DOCUMENTS CONSIDERED TO BE RELEVANT

| Category* | Citation of document, with indication, where appropriate, of the relevant passages | Relevant to claim No. |
|---|---|---|
| P,Y | WO 2010/113957 A1 (Ube Industries, Ltd.), 07 October 2010 (07.10.2010), entire text (Family: none) | 1-21 |

Form PCT/ISA/210 (continuation of second sheet) (July 2009)

INTERNATIONAL SEARCH REPORT

International application No.
PCT/JP2010/073274

Continuation of A. CLASSIFICATION OF SUBJECT MATTER
(International Patent Classification (IPC))

*C07D213/74*(2006.01)i, *A61K31/44*(2006.01)i, *A61K31/4427*(2006.01)i, *A61K31/4436*(2006.01)i, *A61K31/4439*(2006.01)i, *A61K31/444*(2006.01)i, *A61P11/00*(2006.01)i, *A61P11/06*(2006.01)i, *A61P11/08*(2006.01)i, *A61P27/06*(2006.01)i, *A61P43/00*(2006.01)i, *C07D401/12*(2006.01)i, *C07D405/12*(2006.01)i, *C07D409/12*(2006.01)i, *C07D409/14*(2006.01)i, *C07D417/12*(2006.01)i

(According to International Patent Classification (IPC) or to both national classification and IPC)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9828264 A
- WO 9919300 A
- WO 2004078169 A
- WO 2008015517 A
- WO 2009113600 A
- WO 2010113957 A
- WO 2002059077 A
- WO 2004037811 A
- WO 2006074884 A
- WO 2004069792 A
- WO 2006013048 A
- WO 2005100292 A
- US 6369261 B1
- WO 2008018827 A
- WO 2010047982 A
- US 200766820 A
- WO 2003080578 A
- WO 2005035503 A
- WO 2008033455 A
- JP 54046735 A
- US 20090202478 A
- US 200839457 A
- US 2008267892 A


### Non-patent literature cited in the description


- *American Journal of Respiratory and Critical Care Medicine,* 1999, vol. 159, 31
- *American Journal of Physiology-Lung Cellular and Molecular Physiology,* 2003, vol. 284, L599
- *Prostaglandins, Leukotrienes and Essential Fatty Acids,* 2004, vol. 71, 277
- *Invest. Ophthalmol. Vis. Sci.,* 2002, vol. 43, 1475
- *Invest. Ophthalmol. Vis. Sci.,* 1983, vol. 24, 312
- *Proceedings of the National Academy of Sciences of the United States of America,* 2003, vol. 100, 6736
- **T.W.GREENE ; P.G.M. WUTS.** Protective Groups in Organic Synthesis. John Wiley & Sons, Inc, 582, 725
- **T.W.GREENE ; P.G.M.WUTS.** Protective Groups in Organic Synthesis. John Wiley & Sons, Inc, 538
- **T.W.GREENE ; P.G.M.WUTS.** Protective Groups in Organic Synthesis. John Wiley & Sons, Inc, 917
- *Bioorganic and Medicinal Chemistry,* 2006, vol. 14, 7121
- *Tetrahedron,* 2001, vol. 57, 5757
- *Synthesis,* 2005, vol. 4, 547
- *Journal of the American Chemical Society,* 1952, vol. 74, 5163
- *Molecular Crystals and Liquid Crystals,* 1991, vol. 195, 221
- *Journal of the Chemical Society,* 1952, 4519
- *Neftekhimiya,* 1966, vol. 6, 186
- *Indian Journal of Chemistry,* 1974, vol. 12, 476
- **ABRAMOVITZ et al.** *Biochimica et Biophysica Acta,* 2000, vol. 1483, 285
- **WILSON et al.** *European Journal of Pharmacology,* 2004, vol. 501, 49
- **PENNOCK et al.** *J. Appl. Physiology,* 1979, vol. 46, 399-406